# EUROPÄISCHE PATENTANMELDUNG

(11) **EP 2 813 575 A1**
(43) Veröffentlichungstag der Anmeldung: **17.12.2014**
(21) Anmeldenummer: 13172030.2
(22) Anmeldetag: 14.06.2013
(51) Int. Cl.: C12P 5/02, C12P 7/04, C12P 7/16, C12P 7/30, C12P 7/42, C12P 7/52, C12R 1/01

(54) **Verfahren zur Herstellung von organischen Verbindungen aus Knallgas und CO2 über Acetoacetyl-CoA als Zwischenprodukt**

(71) Anmelder: Evonik Industries AG, 45128 Essen (DE)
(72) Erfinder: Wittmann, Eva Maria, 83301 Traunreut (DE); Haas, Thomas, 48161 Münster (DE); Schaffer, Steffen, 45699 Herten (DE); Pötter, Markus, 48149 Münster (DE); Schiemann, Yvonne, 45357 Essen (DE); Kirchner, Nicole, 45665 Recklinghausen (DE)

(57) **Zusammenfassung**

Gegenstand der vorliegenden Erfindung ist ein Verfahren zur Herstellung von organischen Verbindungen umfassend die Verfahrensschritte
A) Bereitstellen eines Knallgasbakteriums mit einer verglichen zu seinem Wildtyp gesteigerten Aktivität eines Enzyms E₁, welches die Umsetzung
2 Acetyl-CoA zu Acetoacetyl-CoA und CoA
zu katalysieren vermag, in einem wässrigen Medium;
B) in Kontakt Bringen des wässrigen Mediums mit einem Gas enthaltend H₂, CO₂ und O₂
in einem Gewichtsverhältnis von 20 bis 70 zu 10 bis 45 zu 5 bis 35
und gegebenenfalls
C) Aufreinigen der organischen Verbindung.

SEQUENCE LISTING

<110>  Evonik Industries

<120>  Verfahren zur Herstellung von organischen Verbindungen aus
       Knallgas und CO2 über Acetoacetyl-CoA als Zwischenprodukt

<130>  201200133

<160>  88

<170>  PatentIn version 3.5

<210>  1
<211>  17
<212>  DNA
<213>  Ralstonia eutropha

<400>  1
tttcaggaga ttcaaga                                                    17


<210>  2
<211>  13042
<212>  DNA
<213>  Artificial Sequence

<220>
<223>  vector

<400>  2
ctcgggccgt ctcttgggct tgatcggcct tcttgcgcat ctcacgcgct cctgcggcgg      60

cctgtagggc aggctcatac ccctgccgaa ccgcttttgt cagccggtcg gccacggctt     120

ccggcgtctc aacgcgcttt gagattccca gcttttcggc caatccctgc ggtgcatagg     180

cgcgtggctc gaccgcttgc gggctgatgg tgacgtggcc cactggtggc cgctccaggg     240

cctcgtagaa cgcctgaatg cgcgtgtgac gtgccttgct gccctcgatg ccccgttgca     300

gccctagatc ggccacagcg gccgcaaacg tggtctggtc gcgggtcatc tgcgctttgt     360

tgccgatgaa ctccttggcc gacagcctgc cgtcctgcgt cagcggcacc acgaacgcgg     420

tcatgtgcgg gctggtttcg tcacggtgga tgctggccgt cacgatgcga tccgccccgt     480

acttgtccgc cagccacttg tgcgccttct cgaagaacgc cgcctgctgt tcttggctgg     540

ccgacttcca ccattccggg ctggccgtca tgacgtactc gaccgccaac acagcgtcct     600

tgcgccgctt ctctggcagc aactcgcgca gtcggcccat cgcttcatcg gtgctgctgg     660

ccgcccagtg ctcgttctct ggcgtcctgc tggcgtcagc gttgggcgtc tcgcgctcgc     720

ggtaggcgtg cttgagactg gccgccacgt tgcccatttt cgccagcttc ttgcatcgca     780

tgatcgcgta tgccgccatg cctgcccctc ccttttggtg tccaaccggc tcgacggggg     840

cagcgcaagg cggtgcctcc ggcgggccac tcaatgcttg agtatactca ctagactttg     900

cttcgcaaag tcgtgaccgc ctacggcggc tgcggcgccc tacgggcttg ctctccgggc     960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaattttct ctggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag gggccacttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatcccaat attaaaaaaa taagagttac catttaaggt aactcttatt 3300

tttattagtc ggacgcgcgc tccggcaccg ggagattcac ccactccttg tagaaggcct 3360

cgatgtccgg ctcgaagtcg tggatcacgg gataccacgg ggtaggcgct tcgacgtcgt 3420

gcatggtgcc gcacgatggg cagtagtatt cgcgatacac ctgccactgg gtgtcggggg 3480

ccatcagctt cggatacacc tctgtcatcg cctcctcggt gtctcgcacg tagatcgcgg 3540

catgcagctt ccagttctcg cggtaatcac agaactcgtg accgcagtcg cacttgacta 3600

cccacttctt gctcttgacc gactgcacga taaacatgtg cgggctgagc ggtagcacga 3660

tgcggtcagg aaagttcacc ttggcctgca aggctgacag gtactgcgcg aaccggctct 3720

cgtccttggg catcgagagc atgcggaagg tggtctccca atcgagggaa ccatcgacga 3780

ggtgggcgac ctgttcattg gtataggttg acattcttga atctcctgaa attactcttc 3840

gacctgaacg acggtggtga catcgggcag cagggagagg tccatgcggt gcttggcgcc 3900

gtaggatggc acgcccagct cttcttcacg cacctgccag tcggcgggca ggctccagaa 3960

ttccttgaac tcccgcgtga acttctcaga cagggcaaag ctggtggcga acatgtgctg 4020

cacctggacg gacgcgtgct tgttgagaat gcgttcgcgc tcttccttca tccattcccg 4080

cgtcggcagc gcccgggcca ggcgttcctt gcgaacttcg gcacggcgtg cttgggtctt 4140

gacggcatcc acggtaaaga cgcccttgtc gtcttgggtg aagacagccc catatacctt 4200

ctgcgcgtag gccggcagca ggaacttctg gttcaggtcc gcttcgatgg cttgcgggtc 4260

ccggtcgaga gggtcgccaa agcccgggcc accgcgcagg tagttcaggt acagatcatg 4320

gttggcgtag cagtcctccg tcgtgatgca ctgcttgtcg cgcttgacct cggcggtggc 4380

gtcgatgtgg cgttcgaagt cggggttact cggatcgatg tcgccgccca gcggcaggga 4440

gtcgccgatg gcgatgcgct ggtcgaggcc ggtcttgtgc gcctcgaagc gatagccggt 4500

agcggaggga tagccgccca tcatgcccca gtcgctgttc atgaagccgt tgcccatgaa 4560

gaacatggtc cagtcctggg cgttccacac catgcgcagc gtctcgaacc cgcaaccgcc 4620

gcggtacttg ccgtagccgc cggaattggc cttgacgttg cggccaaggt agagcagcgg 4680

ctcagccatc tcccagattt cgatgtcgcc catatcgcct tccggattcc agatggcggc 4740

agcgtggttg agaccatcct ttacggcgca ggcgccggtg ccacaggaac tggcctcgaa 4800

gctgttgacc gcatggatct ctccgtcctg gttgatgccg ccgccctgca gccagttgga 4860

ggtgttggcg ttgccggcgt tgacctcttc caggtagccg cggctgaagt aggactgcga 4920

caggccgcgc cacagcgccg cccagccgga gaccaggaag tgccaggcat aggcatggcc 4980

ggtgcggcga tcatcggggt tgcaccacgt gcccttgggc aagcggaact cggtagcgaa 5040

atacgccccg tcgttgatac gctgggtcgg cacaagggtc tggcacatca tcacccagat 5100

tccggaagtg aaggccacct ggtgggcgtt gaaggtatgc cagccccagc ggctcgcgcc 5160

ctcaaagtcg aggcgccact ttccgtccga cttgatggtc atctccaccg gcgaatgcat 5220

gatggagtcg agcttggcga aggcgttgga gacctgcacg tcgtcatgct tgtagggcac 5280

gtcgacaaag gaaaccttgc ggtacttgcc aggcagggtc atcgccttga tgcggctctg 5340

cagtccgcgg cggccttctt cgatgacctc ataggcgaac ttctcgtagg cctcgaggcc 5400

gtcggcacga atcacttctt cgaccagttc gcggatcatg tggcagccag cgatccgggt 5460

tttctcatcg aggatccagt atttcggcgt gcgcaccgag cgctgcgact cgtgcagcca 5520

gtcgcgcagc ggctcgtcgt tcacgccggt cttgcggcag gtgatcatgt agccgtcgcc 5580

aaagcgctgc gtctgcccgg ttgacatcga gcccggcgtt accgagccgg tgtcgatgac 5640

gtgggtgacg ccgcctaccc agccgatcag cttcccctcc cagaagatcg gcacgatggt 5700

ggcgatgtcg caggggtgca cgttgccgat ggagcagtcg ttgttggtga acatgtcacc 5760

gggattgatg ccggggttgg cctcccagtt gttctcgatc atgtacttga tcgccgcccc 5820

catggtgccg acgtggatga tgatgccggt cgaggtcagc acgcagtcac ccacagcgtt 5880

gtagagcgtg aagcagagtt cgccttcctg ctcgacgatg gggctggcgg caattttctt 5940

cgcggtttcg cgggcatgca ccaagccgcc gcgcagcttg gagaacagct tctcatagcc 6000

gatcgggtcg ctgtcgcgga actccagttt gtcgaggccg ttgtagtggc cggacgcctg 6060

agtgcgagcg atgatcgcgt cgcggagctc cttgagggtc ttgccgttct gcagcagatt 6120

gcccaggccc acttccttgt tgctcatcat attcatatct tgaatctcct gaaattactc 6180

ttcgacctga acgacggtgg tgacatcggg cagcagggag aggtccatgc ggtgcttggc 6240

gccgtaggat ggcacgccca gctcttcttc acgcacctgc cagtcggcgg gcaggctcca 6300

gaattccttg aactcccgcg tgaacttctc agacagggca aagctggtgg cgaacatgtg 6360

ctgcacctgg acggacgcgt gcttgttgag aatgcgttcg cgctcttcct tcatccattc 6420

ccgcgtcggc agcgcccggg ccaggcgttc cttgcgaact tcggcacggc gtgcttgggt 6480

cttgacggca tccacggtaa agacgccctt gtcgtcttgg gtgaagacag ccccatatac 6540

cttctgcgcg taggccggca gcaggaactt ctggttcagg tccgcttcga tggcttgcgg 6600

gtcccggtcg agagggtcgc caaagcccgg gccaccgcgc aggtagttca ggtacagatc 6660

atggttggcg tagcagtcct ccgtcgtgat gcactgcttg tcgcgcttga cctcggcggt 6720

ggcgtcgatg tggcgttcga agtcggggtt actcggatcg atgtcgccgc ccagcggcag 6780

ggagtcgccg atggcgatgc gctggtcgag gccggtcttg tgcgcctcga agcgatagcc 6840

ggtagcggag ggatagccgc ccatcatgcc ccagtcgctg ttcatgaagc cgttgcccat 6900

gaagaacatg gtccagtcct gggcgttcca caccatgcgc agcgtctcga acccgcaacc 6960

gccgcggtac ttgccgtagc cgccggaatt ggccttgacg ttgcggccaa ggtagagcag 7020

cggctcagcc atctcccaga tttcgatgtc gcccatatcg ccttccggat tccagatggc 7080

ggcagcgtgg ttgagaccat cctttacggc gcaggcgccg gtgccacagg aactggcctc 7140

gaagctgttg accgcatgga tctctccgtc ctggttgatg ccgccgccct gcagccagtt 7200

ggaggtgttg gcgttgccgg cgttgacctc ttccaggtag ccgcggctga agtaggactg 7260

cgacaggccg cgccacagcg ccgcccagcc ggagaccagg aagtgccagg cataggcatg 7320

gccggtgcgg cgatcatcgg ggttgcacca cgtgcccttg ggcaagcgga actcggtagc 7380

gaaatacgcc ccgtcgttga tacgctgggt cggcacaagg gtctggcaca tcatcaccca 7440

gattccggaa gtgaaggcca cctggtgggc gttgaaggta tgccagcccc agcggctcgc 7500

gccctcaaag tcgaggcgcc actttccgtc cgacttgatg gtcatctcca ccggcgaatg 7560

catgatggag tcgagcttgg cgaaggcgtt ggagacctgc acgtcgtcat gcttgtaggg 7620

cacgtcgaca aaggaaacct tgcggtactt gccaggcagg gtcatcgcct tgatgcggct 7680

ctgcagtccg cggcggcctt cttcgatgac ctcataggcg aacttctcgt aggcctcgag 7740

gccgtcggca cgaatcactt cttcgaccag ttcgcggatc atgtggcagc cagcgatccg 7800

ggttttctca tcgaggatcc agtatttcgg cgtgcgcacc gagcgctgcg actcgtgcag 7860

ccagtcgcgc agcggctcgt cgttcacgcc ggtcttgcgg caggtgatca tgtagccgtc 7920

gccaaagcgc tgcgtctgcc cggttgacat cgagcccggc gttaccgagc cggtgtcgat 7980

gacgtgggtg acgccgccta cccagccgat cagcttcccc tcccagaaga tcggcacgat 8040

ggtggcgatg tcgcaggggt gcacgttgcc gatggagcag tcgttgttgg tgaacatgtc 8100

accgggattg atgccggggt tggcctccca gttgttctcg atcatgtact tgatcgccgc 8160

ccccatggtg ccgacgtgga tgatgatgcc ggtcgaggtc agcacgcagt cacccacagc 8220

gttgtagagc gtgaagcaga gttcgccttc ctgctcgacg atggggctgg cggcaatttt 8280

cttcgcggtt tcgcgggcat gcaccaagcc gccgcgcagc ttggagaaca gcttctcata 8340

gccgatcggg tcgctgtcgc ggaactccag tttgtcgagg ccgttgtagt ggccggacgc 8400

ctgagtgcga gcgatgatcg cgtcgcggag ctccttgagg gtcttgccgt tctgcagcag 8460

attgcccagg cccacttcct tgttgctcat catattcatt cttgaatctc ctgaaatgtg 8520

aaattgttat ccgctcacaa ttccacacaa catacgagcc ggaagcataa agtgtaaagc 8580

ctggggaagc ttattatctt aagtaataaa aataagagtt accttaaatg gtaactctta 8640

tttttttaat attgtttcat agtatttctt tctacacggc catcgggcgc agctcattgc 8700

tgatgagcag gtcggcggcc gtcagcgagc gaatttcgtc aatggtggtg ttcttgttga 8760

tttcggtgag gaggaggccg tcgttaatca cttcgatcac ccccagttcg gtcacaatca 8820

ggttcgcctg cgatttggcg gtcagcggca gggtgcactt cttcaggatc ttcggttggc 8880

ccttgttggt gtggcgcatg gcaatgatga ccttcttggc cccgttcacc agatccatgg 8940

cgccgcccat ccccgacagc atcttgcccg gcacgatcca gttggcgata ttgcccttct 9000

cgtccacctg cagcgcgccc agcaccgtga catccacatg cccgccccga atgagcgaga 9060

acgacaccga cgagtcgaaa aaggtgccgt ccggcagcac ggtggtatag tcgccccccg 9120

cattcaccac gtccttgtcc gcctcattga ttttcgggct ggcccccatg cccacaatgc 9180

cattttccga ctggaaggtg atcttgaaat tcttcgggat gtagtcggcc accatcgtgg 9240

gcaggcccac gcccagattg accagctgcc cgtttttcag ttcgcgggcc acgcgcttcg 9300

caataatctc cttggccagg tttttgtcat taatcatttt aggcgggctc cttcacgatg 9360

tagttgatga gcacgcccgg cgtcatggcc ttttccttct ccagcttctc gcaggagacc 9420

aggttttcgg cttccacgat cacggttttg gcggccatcg ccatgtacgg gttgaagttc 9480

ttggtggtgc ccttgtagaa ggtgttgccg gcttcgtcga caatcgagcc tttaatcagg 9540

gccacgtcgg cggtcagcgg cagctccagg aggtattcgg tcccgttgat ggagatcttc 9600

tttttgcctt tttcgatgag ggtccccagg ccggtcttgg tcaggacccc cccgaggccc 9660

gagccccccg cgcgaatgcg ctccaccagc gtgccctgcg gcgacagttc cacttccagc 9720

tcattgttga acagtttttt ccccgtgtcc ggattcgagc caatgtacga ggcgatcagt 9780

ttcttcacct ggttgttcga gatgagcttg ccgatgccgg tgttcgggta gcaggtatcg 9840

ttggagataa tggtgagatt cttgatgttc aggttcacca gaaaatcaat cagcttggtc 9900

ggggtgccgc agttcagaaa gcccccaatc ataatcgtca tgccgtcctt gaagaacgag 9960

cggaggttct caaagcggat gatcttcgag ttcattttaa tccctccttt taaactttct 10020

agcacttttc cagcaggatc gcggtgccct ggccgccgcc gatgcacagg gtcgccagcc 10080

ccttcttggc gtcgcgcttc tgcatcgcgt gcaccagggt caccaggatg cgggcgcccg 10140

aggcgccgat cgggtgcccc agggcgatgg cgccgccatt cacgttcact ttgttcatgt 10200

cgaacttcag gtccttggcg acggccagcg actgggcggc aaaggcctcg ttcgactcga 10260

tcaggtccag ctcgtcgacc gtccagccgg ctttctcgat cgccgccttg gtggcgtaga 10320

acgggccgta gcccatgatg gccgggtcca cgccggccga gccatacgac acgatcttcg 10380

ccagcggttt cacgcccagc tccttggcct tttcggccga catgatcacc agcacggccg 10440

cgcagtcgtt caggcccgag gcgttgcccg cggtcacggt gccgtccttc ttgaaggccg 10500

gcttcagctt ggccaggccc tcgatcgtcg acccgaagcg cgggtgctcg tccgtgtcca 10560

ccacggtctc gcccttgcgg cccttaatca ccaccggcac gatctcgtcc ttgaactggc 10620

ccgacttgat ggcttcctcc gccttctttt gcgaggccag ggcgaactcg tcctgctcct 10680

cgcgcgagat gttccagcgc tcggcgatgt tctcggcggt gatgcccatg tggtagtcgt 10740

tgaaggcgtc ccacaggccg tcggtgatca tctcgtccac gaacttggcg ttccccatgc 10800

ggtagcccca gcgggcgttg ttggccaggt acggggcgcg cgacatgttt tccatgccgc 10860

cggcaatgat cacgtcggcg tcgccggcct tgatgatctg ggccgccagc gacacggtgc 10920

gcaggcccga gccgcacacc ttgttgatgg tcatggcggg gatctccacc gggaggccgg 10980

ctttgaagct cgcctggcgg gccgggttct ggccgagccc ggcctgcagc acgttgccca 11040

ggatcacctc gttcacgtcc tccggcttga tgccggcctt cttcacggcc tccttaatgg 11100

cggtggcgcc caggtccacg gcgggcacgt ctttcaggct cttgccgtac gagccgatcg 11160

cggtgcgcac ggccgaggca atcaccacct ccttcattct tgaatctcct gaaaggtacc 11220

cagcttttgt tccctttagt gagggttaat tgcgcgcttg gcgtaatcat ggtcatagct 11280

gtttcctgtg tgaaattgtt atccgctcac aattccacac aacatacgag ccggaagcat 11340

aaagtgtaaa gcctggggtg cctaatgagt gagctaactc acattaattg cgttgcgctc 11400

actgcccgct ttccagtcgg gaaacctgtc gtgccagctg cattaatgaa tcggccaacg 11460

cgcggggaga ggcggtttgc gtattgggcg catgcataaa aactgttgta attcattaag 11520

cattctgccg acatggaagc catcacaaac ggcatgatga acctgaatcg ccagcggcat 11580

cagcaccttg tcgccttgcg tataatattt gcccatgggg gtgggcgaag aactccagca 11640

tgagatcccc gcgctggagg atcatccagc cggcgtcccg gaaaacgatt ccgaagccca 11700

acctttcata gaaggcggcg gtggaatcga aatctcgtga tggcaggttg ggcgtcgctt 11760

ggtcggtcat ttcgaacccc agagtcccgc tcagaagaac tcgtcaagaa ggcgatagaa 11820

ggcgatgcgc tgcgaatcgg gagcggcgat accgtaaagc acgaggaagc ggtcagccca 11880

ttcgccgcca agctcttcag caatatcacg ggtagccaac gctatgtcct gatagcggtc 11940

cgccacaccc agccggccac agtcgatgaa tccagaaaag cggccatttt ccaccatgat 12000

attcggcaag caggcatcgc catgggtcac gacgagatcc tcgccgtcgg gcatgcgcgc 12060

cttgagcctg gcgaacagtt cggctggcgc gagcccctga tgctcttcgt ccagatcatc 12120

ctgatcgaca agaccggctt ccatccgagt acgtgctcgc tcgatgcgat gtttcgcttg 12180

gtggtcgaat gggcaggtag ccggatcaag cgtatgcagc cgccgcattg catcagccat 12240

gatggatact ttctcggcag gagcaaggtg agatgacagg agatcctgcc ccggcacttc 12300

gcccaatagc agccagtccc ttcccgcttc agtgacaacg tcgagcacag ctgcgcaagg 12360

aacgcccgtc gtggccagcc acgatagccg cgctgcctcg tcctgcagtt cattcagggc 12420

accggacagg tcggtcttga caaaaagaac cgggcgcccc tgcgctgaca gccggaacac 12480

ggcggcatca gagcagccga ttgtctgttg tgcccagtca tagccgaata gcctctccac 12540

ccaagcggcc ggagaacctg cgtgcaatcc atcttgttca atcatgcgaa acgatcctca 12600

tcctgtctct tgatcagatc ttgatcccct gcgccatcag atccttggcg gcaagaaagc 12660

catccagttt actttgcagg gcttcccaac cttaccagag ggcgccccag ctggcaattc 12720

cggttcgctt gctgtccata aaaccgccca gtctagctat cgccatgtaa gcccactgca 12780

agctacctgc tttctctttg cgcttgcgtt ttcccttgtc cagatagccc agtagctgac 12840

attcatccca ggtggcactt ttcggggaaa tgtgcgcgcc cgcgttcctg ctggcgctgg 12900

gcctgtttct ggcgctggac ttcccgctgt tccgtcagca gcttttcgcc cacggccttg 12960

atgatcgcgg cggccttggc ctgcatatcc cgattcaacg gccccagggc gtccagaacg 13020

ggcttcaggc gctcccgaag gt 13042

<210> 3
<211> 13050
<212> DNA
<213> Artificial Sequence

<220>
<223> vector

<400> 3
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaattttct ctggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag gggccacttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatcccaat attaaaaaaa taagagttac catttaaggt aactcttatt 3300

tttattagtc ggacgcgcgc tccggcaccg ggagattcac ccactccttg tagaaggcct 3360

cgatgtccgg ctcgaagtcg tggatcacgg gataccacgg ggtaggcgct tcgacgtcgt 3420

gcatggtgcc gcacgatggg cagtagtatt cgcgatacac ctgccactgg gtgtcggggg 3480

ccatcagctt cggatacacc tctgtcatcg cctcctcggt gtctcgcacg tagatcgcgg 3540

catgcagctt ccagttctcg cggtaatcac agaactcgtg accgcagtcg cacttgacta 3600

cccacttctt gctcttgacc gactgcacga taaacatgtg cgggctgagc ggtagcacga 3660

tgcggtcagg aaagttcacc ttggcctgca aggctgacag gtactgcgcg aaccggctct 3720

cgtccttggg catcgagagc atgcggaagg tggtctccca atcgagggaa ccatcgacga 3780

ggtgggcgac ctgttcattg gtataggttg acattcttga atctcctgaa attactcttc 3840

gacctgaacg acggtggtga catcgggcag cagggagagg tccatgcggt gcttggcgcc 3900

gtaggatggc acgcccagct cttcttcacg cacctgccag tcggcgggca ggctccagaa 3960

ttccttgaac tcccgcgtga acttctcaga cagggcaaag ctggtggcga acatgtgctg 4020

cacctggacg gacgcgtgct tgttgagaat gcgttcgcgc tcttccttca tccattcccg 4080

cgtcggcagc gcccgggcca ggcgttcctt gcgaacttcg gcacggcgtg cttgggtctt 4140

gacggcatcc acggtaaaga cgcccttgtc gtcttgggtg aagacagccc catatacctt 4200

ctgcgcgtag gccggcagca ggaacttctg gttcaggtcc gcttcgatgg cttgcgggtc 4260

ccggtcgaga gggtcgccaa agcccgggcc accgcgcagg tagttcaggt acagatcatg 4320

gttggcgtag cagtcctccg tcgtgatgca ctgcttgtcg cgcttgacct cggcggtggc 4380

gtcgatgtgg cgttcgaagt cggggttact cggatcgatg tcgccgccca gcggcaggga 4440

gtcgccgatg gcgatgcgct ggtcgaggcc ggtcttgtgc gcctcgaagc gatagccggt 4500

agcggaggga tagccgccca tcatgcccca gtcgctgttc atgaagccgt tgcccatgaa 4560

gaacatggtc cagtcctggg cgttccacac catgcgcagc gtctcgaacc cgcaaccgcc 4620

gcggtacttg ccgtagccgc cggaattggc cttgacgttg cggccaaggt agagcagcgg 4680

ctcagccatc tcccagattt cgatgtcgcc catatcgcct tccggattcc agatggcggc 4740

agcgtggttg agaccatcct ttacggcgca ggcgccggtg ccacaggaac tggcctcgaa 4800

gctgttgacc gcatggatct ctccgtcctg gttgatgccg ccgccctgca gccagttgga 4860

ggtgttggcg ttgccggcgt tgacctcttc caggtagccg cggctgaagt aggactgcga 4920

caggccgcgc cacagcgccg cccagccgga gaccaggaag tgccaggcat aggcatggcc 4980

ggtgcggcga tcatcggggt tgcaccacgt gcccttgggc aagcggaact cggtagcgaa 5040

atacgccccg tcgttgatac gctgggtcgg cacaagggtc tggcacatca tcacccagat 5100

tccggaagtg aaggccacct ggtgggcgtt gaaggtatgc cagccccagc ggctcgcgcc 5160

ctcaaagtcg aggcgccact ttccgtccga cttgatggtc atctccaccg gcgaatgcat 5220

gatggagtcg agcttggcga aggcgttgga gacctgcacg tcgtcatgct tgtagggcac 5280

gtcgacaaag gaaaccttgc ggtacttgcc aggcagggtc atcgccttga tgcggctctg 5340

cagtccgcgg cggccttctt cgatgacctc ataggcgaac ttctcgtagg cctcgaggcc 5400

gtcggcacga atcacttctt cgaccagttc gcggatcatg tggcagccag cgatccgggt 5460

tttctcatcg aggatccagt atttcggcgt gcgcaccgag cgctgcgact cgtgcagcca 5520

gtcgcgcagc ggctcgtcgt tcacgccggt cttgcggcag gtgatcatgt agccgtcgcc 5580

aaagcgctgc gtctgcccgg ttgacatcga gcccggcgtt accgagccgg tgtcgatgac 5640

gtgggtgacg ccgcctaccc agccgatcag cttcccctcc cagaagatcg gcacgatggt 5700

ggcgatgtcg caggggtgca cgttgccgat ggagcagtcg ttgttggtga acatgtcacc 5760

gggattgatg ccggggttgg cctcccagtt gttctcgatc atgtacttga tcgccgcccc 5820

catggtgccg acgtggatga tgatgccggt cgaggtcagc acgcagtcac ccacagcgtt 5880

gtagagcgtg aagcagagtt cgccttcctg ctcgacgatg gggctggcgg caatttcttt 5940

cgcggtttcg cgggcatgca ccaagccgcc gcgcagcttg gagaacagct tctcatagcc 6000

gatcgggtcg ctgtcgcgga actccagttt gtcgaggccg ttgtagtggc cggacgcctg 6060

agtgcgagcg atgatcgcgt cgcggagctc cttgagggtc ttgccgttct gcagcagatt 6120

gcccaggccc acttccttgt tgctcatcat attcatatct tgaatctcct gaaattactc 6180

ttcgacctga acgacggtgg tgacatcggg cagcagggag aggtccatgc ggtgcttggc 6240

gccgtaggat ggcacgccca gctcttcttc acgcacctgc cagtcggcgg gcaggctcca 6300

gaattccttg aactcccgcg tgaacttctc agacagggca aagctggtgg cgaacatgtg 6360

ctgcacctgg acggacgcgt gcttgttgag aatgcgttcg cgctcttcct tcatccattc 6420

ccgcgtcggc agcgcccggg ccaggcgttc cttgcgaact tcggcacggc gtgcttgggt 6480

cttgacggca tccacggtaa agacgccctt gtcgtcttgg gtgaagacag ccccatatac 6540

cttctgcgcg taggccggca gcaggaactt ctggttcagg tccgcttcga tggcttgcgg 6600

gtcccggtcg agagggtcgc caaagcccgg gccaccgcgc aggtagttca ggtacagatc 6660

atggttggcg tagcagtcct ccgtcgtgat gcactgcttg tcgcgcttga cctcggcggt 6720

ggcgtcgatg tggcgttcga agtcggggtt actcggatcg atgtcgccgc ccagcggcag 6780

ggagtcgccg atggcgatgc gctggtcgag gccggtcttg tgcgcctcga agcgatagcc 6840

ggtagcggag ggatagccgc ccatcatgcc ccagtcgctg ttcatgaagc cgttgcccat 6900

gaagaacatg gtccagtcct gggcgttcca caccatgcgc agcgtctcga acccgcaacc 6960

gccgcggtac ttgccgtagc cgccggaatt ggccttgacg ttgcggccaa ggtagagcag 7020

cggctcagcc atctcccaga tttcgatgtc gcccatatcg ccttccggat tccagatggc 7080

ggcagcgtgg ttgagaccat cctttacggc gcaggcgccg gtgccacagg aactggcctc 7140

gaagctgttg accgcatgga tctctccgtc ctggttgatg ccgccgccct gcagccagtt 7200

ggaggtgttg gcgttgccgg cgttgacctc ttccaggtag ccgcggctga agtaggactg 7260

cgacaggccg cgccacagcg ccgcccagcc ggagaccagg aagtgccagg cataggcatg 7320

gccggtgcgg cgatcatcgg ggttgcacca cgtgcccttg ggcaagcgga actcggtagc 7380

gaaatacgcc ccgtcgttga tacgctgggt cggcacaagg gtctggcaca tcatcaccca 7440

gattccggaa gtgaaggcca cctggtgggc gttgaaggta tgccagcccc agcggctcgc 7500

gccctcaaag tcgaggcgcc actttccgtc cgacttgatg gtcatctcca ccggcgaatg 7560

catgatggag tcgagcttgg cgaaggcgtt ggagacctgc acgtcgtcat gcttgtaggg 7620

cacgtcgaca aaggaaacct tgcggtactt gccaggcagg gtcatcgcct tgatgcggct 7680

ctgcagtccg cggcggcctt cttcgatgac ctcataggcg aacttctcgt aggcctcgag 7740

gccgtcggca cgaatcactt cttcgaccag ttcgcggatc atgtggcagc cagcgatccg 7800

ggttttctca tcgaggatcc agtatttcgg cgtgcgcacc gagcgctgcg actcgtgcag 7860

ccagtcgcgc agcggctcgt cgttcacgcc ggtcttgcgg caggtgatca tgtagccgtc 7920

gccaaagcgc tgcgtctgcc cggttgacat cgagcccggc gttaccgagc cggtgtcgat 7980

gacgtgggtg acgccgccta cccagccgat cagcttcccc tcccagaaga tcggcacgat 8040

ggtggcgatg tcgcaggggt gcacgttgcc gatggagcag tcgttgttgg tgaacatgtc 8100

accgggattg atgccggggt tggcctccca gttgttctcg atcatgtact tgatcgccgc 8160

ccccatggtg ccgacgtgga tgatgatgcc ggtcgaggtc agcacgcagt cacccacagc 8220

gttgtagagc gtgaagcaga gttcgccttc ctgctcgacg atggggctgg cggcaatttt 8280

cttcgcggtt tcgcgggcat gcaccaagcc gccgcgcagc ttggagaaca gcttctcata 8340

gccgatcggg tcgctgtcgc ggaactccag tttgtcgagg ccgttgtagt ggccggacgc 8400

ctgagtgcga gcgatgatcg cgtcgcggag ctccttgagg gtcttgccgt tctgcagcag 8460

attgcccagg cccacttcct tgttgctcat catattcatt cttgaatctc ctgaaatgtg 8520

aaattgttat ccgctcacaa ttccacacaa catacgagcc ggaagcataa agtgtaaagc 8580

ctggggaagc ttattatctt aagtaataaa aataagagtt accttaaatg gtaactctta 8640

tttttttaat attgtttcat agtatttctt tctacacggc catcgggcgc agctcattgc 8700

tgatgagcag gtcggcggcc gtcagcgagc gaatttcgtc aatggtggtg ttcttgttga 8760

tttcggtgag gaggaggccg tcgttaatca cttcgatcac ccccagttcg gtcacaatca 8820

ggttcgcctg cgatttggcg gtcagcggca gggtgcactt cttcaggatc ttcggttggc 8880

ccttgttggt gtggcgcatg gcaatgatga ccttcttggc cccgttcacc agatccatgg 8940

cgccgcccat ccccgacagc atcttgcccg gcacgatcca gttggcgata ttgcccttct 9000

cgtccacctg cagcgcgccc agcaccgtga catccacatg cccgccccga atgagcgaga 9060

acgacaccga cgagtcgaaa aaggtgccgt ccggcagcac ggtggtatag tcgccccccg 9120

cattcaccac gtccttgtcc gcctcattga ttttcgggct ggcccccatg cccacaatgc 9180

cattttccga ctggaaggtg atcttgaaat tcttcgggat gtagtcggcc accatcgtgg 9240

gcaggcccac gcccagattg accagctgcc cgtttttcag ttcgcgggcc acgcgcttcg 9300

caataatctc cttggccagg tttttgtcat taatcatttt aggcgggctc cttcacgatg 9360

tagttgatga gcacgcccgg cgtcatggcc ttttccttct ccagcttctc gcaggagacc 9420

aggttttcgg cttccacgat cacggttttg gcggccatcg ccatgtacgg gttgaagttc 9480

ttggtggtgc ccttgtagaa ggtgttgccg gcttcgtcga caatcgagcc tttaatcagg 9540

gccacgtcgg cggtcagcgg cagctccagg aggtattcgg tcccgttgat ggagatcttc 9600

tttttgcctt tttcgatgag ggtccccagg ccggtcttgg tcaggacccc cccgaggccc 9660

gagccccccg cgcgaatgcg ctccaccagc gtgccctgcg gcgacagttc cacttccagc 9720

tcattgttga acagtttttt ccccgtgtcc ggattcgagc caatgtacga ggcgatcagt 9780

ttcttcacct ggttgttcga gatgagcttg ccgatgccgg tgttcgggta gcaggtatcg 9840

ttggagataa tggtgagatt cttgatgttc aggttcacca gaaaatcaat cagcttggtc 9900

ggggtgccgc agttcagaaa gcccccaatc ataatcgtca tgccgtcctt gaagaacgag 9960

cggaggttct caaagcggat gatcttcgag ttcattttaa tccctccttt taaattcctt 10020

atttgcgctc gactgccagc gccacgccca tgccgccgcc gatgcacagc gaggccaggc 10080

ccttcttcgc gtcacggcgc ttcatctcgt gcagcagcgt caccaggata cggcagcccg 10140

acgcgccgat cgggtggccg atggcgatgg cgccgccgtt cacattgacc ttggaggtgt 10200

cccagcccat ctgctggtgc accgccagcg cctgcgcggc aaaggcctcg ttgatctcca 10260

tcaggtccag gtcttgcggg gtccactcgg cgcgcgacag ggcgcgcttg gaggccggca 10320

ccgggcccat gcccatcacc ttgggatcga caccggcgtt ggcatagctc ttgatcgtgg 10380

ccagcggggt caggcccagt tccttggcct tggccgccga catcaccacc accgcggcgg 10440

cgccgtcgtt caggcccgag gcgttggccg cggtcaccgt gccggccttg tcgaaggcgg 10500

gcttgaggcc ggacatgctg tccagcgtgg cgccctggcg cacgaactcg tcggtcttga 10560

aggccaccgg gtcgcccttg cgctgcggga tcagcaccgg gacgatctct tcgtcaaact 10620

tgccggcctt ctgcgcggct tcggccttgt tctgcgagcc gacggcgaac tcatcctgcg 10680

cctcgcgtgt gatgccgtat tccttggcca cgttctcggc ggtgatgccc atgtggtact 10740

ggttgtacac gtcccacagg ccgtcgacga tcatggtgtc gaccagcttg gcatcgccca 10800

tgcggaaacc atcgcgcgag cccggcagca cgtgcggggc ggcgctcatg ttttcctggc 10860

cgccggccac cacgatctcg gcgtcgcccg ccatgatcgc gttggcggcc agcatcacgg 10920

ccttcaggcc cgagccgcac accttgttga tggtcatggc cggcaccatc gccggcaggc 10980

cggccttgat cgcggcctgg cgtgcggggt tctggcccga accggcggtc agcacctggc 11040

ccatgatgac ttcgctcacc tgctccggct tgacgccggc gcgctccagc gcggccttga 11100

tgaccacggc acccagttcc ggtgccggga tcttggccag cgagccgcca aacttgccga 11160

ccgcggtgcg ggcggcggat acgatgacaa cgtcagtcat tgtgtagtcc tttcaatgga 11220

aaggtaccca gcttttgttc cctttagtga gggttaattg cgcgcttggc gtaatcatgg 11280

tcatagctgt ttcctgtgtg aaattgttat ccgctcacaa ttccacacaa catacgagcc 11340

ggaagcataa agtgtaaagc ctggggtgcc taatgagtga gctaactcac attaattgcg 11400

ttgcgctcac tgcccgcttt ccagtcggga aacctgtcgt gccagctgca ttaatgaatc 11460

ggccaacgcg cggggagagg cggtttgcgt attgggcgca tgcataaaaa ctgttgtaat 11520

tcattaagca ttctgccgac atggaagcca tcacaaacgg catgatgaac ctgaatcgcc 11580

agcggcatca gcaccttgtc gccttgcgta taatatttgc ccatgggggt gggcgaagaa 11640

ctccagcatg agatccccgc gctggaggat catccagccg gcgtcccgga aaacgattcc 11700

gaagcccaac ctttcataga aggcggcggt ggaatcgaaa tctcgtgatg gcaggttggg 11760

cgtcgcttgg tcggtcattt cgaaccccag agtcccgctc agaagaactc gtcaagaagg 11820

cgatagaagg cgatgcgctg cgaatcggga gcggcgatac cgtaaagcac gaggaagcgg 11880

tcagcccatt cgccgccaag ctcttcagca atatcacggg tagccaacgc tatgtcctga 11940

tagcggtccg ccacacccag ccggccacag tcgatgaatc cagaaaagcg gccattttcc 12000

accatgatat tcggcaagca ggcatcgcca tgggtcacga cgagatcctc gccgtcgggc 12060

atgcgcgcct tgagcctggc gaacagttcg gctggcgcga gcccctgatg ctcttcgtcc 12120

agatcatcct gatcgacaag accggcttcc atccgagtac gtgctcgctc gatgcgatgt 12180

ttcgcttggt ggtcgaatgg gcaggtagcc ggatcaagcg tatgcagccg ccgcattgca 12240

tcagccatga tggatacttt ctcggcagga gcaaggtgag atgacaggag atcctgcccc 12300

ggcacttcgc ccaatagcag ccagtccctt cccgcttcag tgacaacgtc gagcacagct 12360

gcgcaaggaa cgcccgtcgt ggccagccac gatagccgcg ctgcctcgtc ctgcagttca 12420

ttcagggcac cggacaggtc ggtcttgaca aaaagaaccg ggcgcccctg cgctgacagc 12480

cggaacacgg cggcatcaga gcagccgatt gtctgttgtg cccagtcata gccgaatagc 12540

ctctccaccc aagcggccgg agaacctgcg tgcaatccat cttgttcaat catgcgaaac 12600

gatcctcatc ctgtctcttg atcagatctt gatcccctgc gccatcagat ccttggcggc 12660

aagaaagcca tccagtttac tttgcagggc ttcccaacct taccagaggg cgccccagct 12720

ggcaattccg gttcgcttgc tgtccataaa accgcccagt ctagctatcg ccatgtaagc 12780

ccactgcaag ctacctgctt tctctttgcg cttgcgtttt cccttgtcca gatagcccag 12840

tagctgacat tcatcccagg tggcactttt cggggaaatg tgcgcgcccg cgttcctgct 12900

ggcgctgggc ctgtttctgg cgctggactt cccgctgttc cgtcagcagc ttttcgccca 12960

cggccttgat gatcgcggcg gccttggcct gcatatcccg attcaacggc cccagggcgt 13020

ccagaacggg cttcaggcgc tcccgaaggt 13050

<210> 4
<211> 13020
<212> DNA
<213> Artificial Sequence

<220>
<223> vector

<400> 4
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaattttct ctggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag gggccacttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatggccca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatcccaat attaaaaaaa taagagttac catttaaggt aactcttatt 3300

tttattagtc ggacgcgcgc tccggcaccg ggagattcac ccactccttg tagaaggcct 3360

cgatgtccgg ctcgaagtcg tggatcacgg gataccacgg ggtaggcgct tcgacgtcgt 3420

gcatggtgcc gcacgatggg cagtagtatt cgcgatacac ctgccactgg gtgtcggggg 3480

ccatcagctt cggatacacc tctgtcatcg cctcctcggt gtctcgcacg tagatcgcgg 3540

catgcagctt ccagttctcg cggtaatcac agaactcgtg accgcagtcg cacttgacta 3600

cccacttctt gctcttgacc gactgcacga taaacatgtg cgggctgagc ggtagcacga 3660

tgcggtcagg aaagttcacc ttggcctgca aggctgacag gtactgcgcg aaccggctct 3720

cgtccttggg catcgagagc atgcggaagg tggtctccca atcgagggaa ccatcgacga 3780

ggtgggcgac ctgttcattg gtataggttg acattcttga atctcctgaa attactcttc 3840

gacctgaacg acggtggtga catcgggcag cagggagagg tccatgcggt gcttggcgcc 3900

gtaggatggc acgcccagct cttcttcacg cacctgccag tcggcgggca ggctccagaa 3960

ttccttgaac tcccgcgtga acttctcaga cagggcaaag ctggtggcga acatgtgctg 4020

cacctggacg gacgcgtgct tgttgagaat gcgttcgcgc tcttccttca tccattcccg 4080

cgtcggcagc gcccgggcca ggcgttcctt gcgaacttcg gcacggcgtg cttgggtctt 4140

gacggcatcc acggtaaaga cgcccttgtc gtcttgggtg aagacagccc catatacctt 4200

ctgcgcgtag gccggcagca ggaacttctg gttcaggtcc gcttcgatgg cttgcgggtc 4260

ccggtcgaga gggtcgccaa agcccgggcc accgcgcagg tagttcaggt acagatcatg 4320

gttggcgtag cagtcctccg tcgtgatgca ctgcttgtcg cgcttgacct cggcggtggc 4380

gtcgatgtgg cgttcgaagt cggggttact cggatcgatg tcgccgccca gcggcaggga 4440

gtcgccgatg gcgatgcgct ggtcgaggcc ggtcttgtgc gcctcgaagc gatagccggt 4500

agcggaggga tagccgccca tcatgcccca gtcgctgttc atgaagccgt tgcccatgaa 4560

gaacatggtc cagtcctggg cgttccacac catgcgcagc gtctcgaacc cgcaaccgcc 4620

gcggtacttg ccgtagccgc cggaattggc cttgacgttg cggccaaggt agagcagcgg 4680

ctcagccatc tcccagattt cgatgtcgcc catatcgcct tccggattcc agatggcggc 4740

agcgtggttg agaccatcct ttacggcgca ggcgccggtg ccacaggaac tggcctcgaa 4800

gctgttgacc gcatggatct ctccgtcctg gttgatgccg ccgccctgca gccagttgga 4860

ggtgttggcg ttgccggcgt tgacctcttc caggtagccg cggctgaagt aggactgcga 4920

caggccgcgc cacagcgccg cccagccgga gaccaggaag tgccaggcat aggcatggcc 4980

ggtgcggcga tcatcggggt tgcaccacgt gcccttgggc aagcggaact cggtagcgaa 5040

atacgccccg tcgttgatac gctgggtcgg cacaagggtc tggcacatca tcacccagat 5100

tccggaagtg aaggccacct ggtgggcgtt gaaggtatgc cagccccagc ggctcgcgcc 5160

ctcaaagtcg aggcgccact ttccgtccga cttgatggtc atctccaccg gcgaatgcat 5220

gatggagtcg agcttggcga aggcgttgga gacctgcacg tcgtcatgct tgtagggcac 5280

gtcgacaaag gaaaccttgc ggtacttgcc aggcagggtc atcgccttga tgcggctctg 5340

cagtccgcgg cggccttctt cgatgacctc ataggcgaac ttctcgtagg cctcgaggcc 5400

gtcggcacga atcacttctt cgaccagttc gcggatcatg tggcagccag cgatccgggt 5460

tttctcatcg aggatccagt atttcggcgt gcgcaccgag cgctgcgact cgtgcagcca 5520

gtcgcgcagc ggctcgtcgt tcacgccggt cttgcggcag gtgatcatgt agccgtcgcc 5580

aaagcgctgc gtctgcccgg ttgacatcga gcccggcgtt accgagccgg tgtcgatgac 5640

gtgggtgacg ccgcctaccc agccgatcag cttcccctcc cagaagatcg gcacgatggt 5700

ggcgatgtcg caggggtgca cgttgccgat ggagcagtcg ttgttggtga acatgtcacc 5760

gggattgatg ccggggttgg cctcccagtt gttctcgatc atgtacttga tcgccgcccc 5820

catggtgccg acgtggatga tgatgccggt cgaggtcagc acgcagtcac ccacagcgtt 5880

gtagagcgtg aagcagagtt cgccttcctg ctcgacgatg gggctggcgg caattttctt 5940

cgcggtttcg cgggcatgca ccaagccgcc gcgcagcttg gagaacagct tctcatagcc 6000

gatcgggtcg ctgtcgcgga actccagttt gtcgaggccg ttgtagtggc cggacgcctg 6060

agtgcgagcg atgatcgcgt cgcggagctc cttgagggtc ttgccgttct gcagcagatt 6120

gcccaggccc acttccttgt tgctcatcat attcatatct tgaatctcct gaaattactc 6180

ttcgacctga acgacggtgg tgacatcggg cagcagggag aggtccatgc ggtgcttggc 6240

gccgtaggat ggcacgccca gctcttcttc acgcacctgc cagtcggcgg gcaggctcca 6300

gaattccttg aactcccgcg tgaacttctc agacagggca aagctggtgg cgaacatgtg 6360

ctgcacctgg acggacgcgt gcttgttgag aatgcgttcg cgctcttcct tcatccattc 6420

ccgcgtcggc agcgcccggg ccaggcgttc cttgcgaact tcggcacggc gtgcttgggt 6480

cttgacggca tccacggtaa agacgccctt gtcgtcttgg gtgaagacag ccccatatac 6540

cttctgcgcg taggccggca gcaggaactt ctggttcagg tccgcttcga tggcttgcgg 6600

gtcccggtcg agagggtcgc caaagcccgg gccaccgcgc aggtagttca ggtacagatc 6660

atggttggcg tagcagtcct ccgtcgtgat gcactgcttg tcgcgcttga cctcggcggt 6720

ggcgtcgatg tggcgttcga agtcggggtt actcggatcg atgtcgccgc ccagcggcag 6780

ggagtcgccg atggcgatgc gctggtcgag gccggtcttg tgcgcctcga agcgatagcc 6840

ggtagcggag ggatagccgc ccatcatgcc ccagtcgctg ttcatgaagc cgttgcccat 6900

gaagaacatg gtccagtcct gggcgttcca caccatgcgc agcgtctcga acccgcaacc 6960

gccgcggtac ttgccgtagc cgccggaatt ggccttgacg ttgcggccaa ggtagagcag 7020

cggctcagcc atctcccaga tttcgatgtc gcccatatcg ccttccggat tccagatggc 7080

ggcagcgtgg ttgagaccat cctttacggc gcaggcgccg gtgccacagg aactggcctc 7140

gaagctgttg accgcatgga tctctccgtc ctggttgatg ccgccgccct gcagccagtt 7200

ggaggtgttg gcgttgccgg cgttgacctc ttccaggtag ccgcggctga agtaggactg 7260

cgacaggccg cgccacagcg ccgcccagcc ggagaccagg aagtgccagg cataggcatg 7320

gccggtgcgg cgatcatcgg ggttgcacca cgtgcccttg ggcaagcgga actcggtagc 7380

gaaatacgcc ccgtcgttga tacgctgggt cggcacaagg gtctggcaca tcatcaccca 7440

gattccggaa gtgaaggcca cctggtgggc gttgaaggta tgccagcccc agcggctcgc 7500

gccctcaaag tcgaggcgcc actttccgtc cgacttgatg gtcatctcca ccggcgaatg 7560

catgatggag tcgagcttgg cgaaggcgtt ggagacctgc acgtcgtcat gcttgtaggg 7620

cacgtcgaca aaggaaacct tgcggtactt gccaggcagg gtcatcgcct tgatgcggct 7680

ctgcagtccg cggcggcctt cttcgatgac ctcataggcg aacttctcgt aggcctcgag 7740

gccgtcggca cgaatcactt cttcgaccag ttcgcggatc atgtggcagc cagcgatccg 7800

ggttttctca tcgaggatcc agtatttcgg cgtgcgcacc gagcgctgcg actcgtgcag 7860

ccagtcgcgc agcggctcgt cgttcacgcc ggtcttgcgg caggtgatca tgtagccgtc 7920

gccaaagcgc tgcgtctgcc cggttgacat cgagcccggc gttaccgagc cggtgtcgat 7980

gacgtgggtg acgccgccta cccagccgat cagcttcccc tcccagaaga tcggcacgat 8040

ggtggcgatg tcgcaggggt gcacgttgcc gatggagcag tcgttgttgg tgaacatgtc 8100

accgggattg atgccggggt tggcctccca gttgttctcg atcatgtact tgatcgccgc 8160

ccccatggtg ccgacgtgga tgatgatgcc ggtcgaggtc agcacgcagt cacccacagc 8220

gttgtagagc gtgaagcaga gttcgccttc ctgctcgacg atggggctgg cggcaatttt 8280

cttcgcggtt tcgcgggcat gcaccaagcc gccgcgcagc ttggagaaca gcttctcata 8340

gccgatcggg tcgctgtcgc ggaactccag tttgtcgagg ccgttgtagt ggccggacgc 8400

ctgagtgcga gcgatgatcg cgtcgcggag ctccttgagg gtcttgccgt tctgcagcag 8460

attgcccagg cccacttcct tgttgctcat catattcatt cttgaatctc ctgaaatgtg 8520

aaattgttat ccgctcacaa ttccacacaa catacgagcc ggaagcataa agtgtaaagc 8580

ctggggaagc tttcgatgtt caagaaaaca cccgataact ttcgctatcg ggtgttttta 8640

ttgattagtt gaggcgttcg atcaccatgg cgatgccctg gcccccgccg atgcacaggg 8700

tggccaggcc cagcgtctta tcccgggcct gcatggcgtg cagcagggtc accaggatgc 8760

gcgcgcccga ggcgccgatc ggatggccca gggcgatcgc gccgccgttc acattcacct 8820

tctccgagtc gaagcccagg ttcttgccca ccgccaggaa ctgggcggcg aaggcctcgt 8880

tggcctcgat caggtcgatg tccgccagtt gcaggcccgc cagctgcagg gccttctggg 8940

tggccggcac cgggcccatg cccatcaggg ccggcggcac cccgcccgag gcgtacgact 9000

tgatgcgggc cagcggggtc agccccgcgg ccagcgcggc cgactcctcc atgatcacca 9060

gcgccgcggc gccgtcgttg atgcccgagg cgttgcccgc ggtcacggtg ccggctttgt 9120

cgaaggccgg gcgcagggcc cccagggcct cggcggtcga gttggccttc gggaactcgt 9180

cctgcgagaa cacgaaggtc ttcttgcggg tcaccacgtt caccggcacg atctcggccg 9240

tgaaggcgcc cgactcgatg gcggcggcgg ccttgcgctg cgagtgcagg gccagctcgt 9300

cctgcatctc gcgggtgatg ccgtactcct tggccacgtt ctcggcggtg atgcccatgt 9360

ggtagccgtg ggtggcgcac atgaggccgt cgcggaggat cacgtcgtac acctggccgt 9420

cccccagccg atagcccgag cgggccttgg cgtccagcag gtacggggcc agcgacatgt 9480

tctccatgcc gccggccacg atcgattggg cttgcccggc ttggatggct tgggcggcga 9540

gggcgaccga cttcaggccg gagccgcaca ccttgttcac ggtgaagccg cacacggtct 9600

cggcgaggcc cgacttcagg agggcctgcc gggccgggtt ctggcccagc ccggcttgca 9660

gcacgttgcc catgatcacc tcgtccacgt gttgcgagtc gatcttggcg cgctcgatgg 9720

ccgccttgat cacggtggcc cccaggtcga tggccgaggt cgaggcgagc gagccgttga 9780

acgagccgat cgcggtgcgg acggccgaca cgatcacgca gttcttcatt ttatattcct 9840

tctgtttgta ggggtgccgt cacaggtcgc cgcgctgggt gttcaggtcg gcggccacct 9900

cgaagcgggc ctcggtcttg gcgcgcacgg tcgcgaggtc gcagccgtcg gcgatctcgg 9960

tcagccacat cttgccgtcg atgaagcgga acacggccag ttcggtcacc agcatgtgca 10020

cggcgtgctg ggccgtcagc ggcatggtgc agcggcgcag gatcttggcc gagccatcct 10080

tggcgcagtg ctccatcgca atgatcacct tgcgcgagcc ggtcacgagg tccatggccc 10140

cccccatgcc cgggaccatt ttgcccggca cgacccagtt ggccaggttg gcctcctcgt 10200

ccacctggag cccgccgagg acgcaggcgt cgatgtgccc cccccggatc agggcgaacg 10260

acatggccga gtcgaacatc gcggcgcccg gcagcacccc gcacggctgg ccccccgcgt 10320

tcaccaggtc cgggtgggcg gtggtcaccg ggcccaggcc caggaagccg ttctccgact 10380

gcagggtgat gtggatgccc tccggcagat agttggccac catggtcggc aggccgatgc 10440

ccaggttcac gatgtcgccg tcgcgcagtt cctgcgcgac gcggcgggcg atgcgctgct 10500

tggcgtccat tacttcgact cttgggagac gatgatatgg tcgatcaccg cgccgggggt 10560

cacgatatga tccggttgca gctccccggt ttcgacgagt tcgtccggtt ccacgagggt 10620

gatatccgcg gccagcgcaa tcagcgggtt gaagttccgg gcggacagct ggtacgtcag 10680

gttgcccagg gtgtcgcagc gatgggcgcg aatcagggcc agatcggcgc gcagcgggcg 10740

ttccagcagc caggtcttcc cgtccagggt cagcgtctgc ttgccttcct cgaccacggt 10800

gcccacgccg gtcggcgtca ggaacccgcc gaggcccgcc ccgccgcacc ggatttgctc 10860

aatgagggtc ccctgcggga ccagcaccac atccatctcg cccgagatca tccggcggcc 10920

ggtctccggg ttcgtgccga tgtggctggc gatcactttg cggacgcggc cgttgacaat 10980

cagggggcca atgccggtgt ccacgaaggc ggtgtcgttg gcaatgagcg tcagatcgcg 11040

cacccccgac tccaggaggg cttcgaccag ccgcgacggc gtcccgatgc ccatgaagcc 11100

ccccaccatg atggtcatgc catcgcggaa aaagccggtg gcgtcctgca gcgtcatgag 11160

cttggtcttc attttttatc cctcttgcat acggtaccca gcttttgttc cctttagtga 11220

gggttaattg cgcgcttggc gtaatcatgg tcatagctgt ttcctgtgtg aaattgttat 11280

ccgctcacaa ttccacacaa catacgagcc ggaagcataa agtgtaaagc ctggggtgcc 11340

taatgagtga gctaactcac attaattgcg ttgcgctcac tgcccgcttt ccagtcggga 11400

aacctgtcgt gccagctgca ttaatgaatc ggccaacgcg cggggagagg cggtttgcgt 11460

attgggcgca tgcataaaaa ctgttgtaat tcattaagca ttctgccgac atggaagcca 11520

tcacaaacgg catgatgaac ctgaatcgcc agcggcatca gcaccttgtc gccttgcgta 11580

taatatttgc ccatgggggt gggcgaagaa ctccagcatg agatccccgc gctggaggat 11640

catccagccg gcgtcccgga aaacgattcc gaagcccaac ctttcataga aggcggcggt 11700

ggaatcgaaa tctcgtgatg gcaggttggg cgtcgcttgg tcggtcattt cgaaccccag 11760

agtcccgctc agaagaactc gtcaagaagg cgatagaagg cgatgcgctg cgaatcggga 11820

gcggcgatac cgtaaagcac gaggaagcgg tcagcccatt cgccgccaag ctcttcagca 11880

atatcacggg tagccaacgc tatgtcctga tagcggtccg ccacacccag ccggccacag 11940

tcgatgaatc cagaaaagcg gccattttcc accatgatat tcggcaagca ggcatcgcca 12000

tgggtcacga cgagatcctc gccgtcgggc atgcgcgcct tgagcctggc gaacagttcg 12060

gctggcgcga gcccctgatg ctcttcgtcc agatcatcct gatcgacaag accggcttcc 12120

atccgagtac gtgctcgctc gatgcgatgt ttcgcttggt ggtcgaatgg gcaggtagcc 12180

ggatcaagcg tatgcagccg ccgcattgca tcagccatga tggatacttt ctcggcagga 12240

gcaaggtgag atgacaggag atcctgcccc ggcacttcgc ccaatagcag ccagtccctt 12300

cccgcttcag tgacaacgtc gagcacagct gcgcaaggaa cgcccgtcgt ggccagccac 12360

gatagccgcg ctgcctcgtc ctgcagttca ttcagggcac cggacaggtc ggtcttgaca 12420

aaaagaaccg ggcgcccctg cgctgacagc cggaacacgg cggcatcaga gcagccgatt 12480

gtctgttgtg cccagtcata gccgaatagc ctctccaccc aagcggccgg agaacctgcg 12540

tgcaatccat cttgttcaat catgcgaaac gatcctcatc ctgtctcttg atcagatctt 12600

gatcccctgc gccatcagat ccttggcggc aagaaagcca tccagtttac tttgcagggc 12660

ttcccaacct taccagaggg cgccccagct ggcaattccg gttcgcttgc tgtccataaa 12720

accgcccagt ctagctatcg ccatgtaagc ccactgcaag ctacctgctt tctctttgcg 12780

cttgcgtttt cccttgtcca gatagcccag tagctgacat tcatcccagg tggcactttt 12840

cggggaaatg tgcgcgcccg cgttcctgct ggcgctgggc ctgtttctgg cgctggactt 12900

cccgctgttc cgtcagcagc ttttcgccca cggccttgat gatcgcggcg gccttggcct 12960

gcatatcccg attcaacggc cccagggcgt ccagaacggg cttcaggcgc tcccgaaggt 13020

<210> 5
<211> 10031
<212> DNA
<213> Artificial Sequence

<220>
<223> vector

<400> 5
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaattttct ctggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag ggccactttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gttagtcgga 1800

cgcgcgctcc ggcaccggga gattcaccca ctccttgtag aaggcctcga tgtccggctc 1860

gaagtcgtgg atcacgggat accacggggt aggcgcttcg acgtcgtgca tggtgccgca 1920

cgatgggcag tagtattcgc gatacacctg ccactgggtg tcgggggcca tcagcttcgg 1980

atacacctct gtcatcgcct cctcggtgtc tcgcacgtag atcgcggcat gcagcttcca 2040

gttctcgcgg taatcacaga actcgtgacc gcagtcgcac ttgactaccc acttcttgct 2100

cttgaccgac tgcacgataa acatgtgcgg gctgagcggt agcacgatgc ggtcaggaaa 2160

gttcaccttg gcctgcaagg ctgacaggta ctgcgcgaac cggctctcgt ccttgggcat 2220

cgagagcatg cggaaggtgg tctcccaatc gagggaacca tcgacgaggt gggcgacctg 2280

ttcattggta taggttgaca ttcttgaatc tcctgaaatt actcttcgac ctgaacgacg 2340

gtggtgacat cgggcagcag ggagaggtcc atgcggtgct tggcgccgta ggatggcacg 2400

cccagctctt cttcacgcac ctgccagtcg gcgggcaggc tccagaattc cttgaactcc 2460

cgcgtgaact tctcagacag ggcaaagctg gtggcgaaca tgtgctgcac ctggacggac 2520
gcgtgcttgt tgagaatgcg ttcgcgctct tccttcatcc attcccgcgt cggcagcgcc 2580
cgggccaggc gttccttgcg aacttcggca cggcgtgctt gggtcttgac ggcatccacg 2640
gtaaagacgc ccttgtcgtc ttgggtgaag acagccccat ataccttctg cgcgtaggcc 2700
ggcagcagga acttctggtt caggtccgct tcgatggctt gcgggtcccg gtcgagaggg 2760
tcgccaaagc ccgggccacc gcgcaggtag ttcaggtaca gatcatggtt ggcgtagcag 2820
tcctccgtcg tgatgcactg cttgtcgcgc ttgacctcgg cggtggcgtc gatgtggcgt 2880
tcgaagtcgg ggttactcgg atcgatgtcg ccgcccagcg gcagggagtc gccgatggcg 2940
atgcgctggt cgaggccggt cttgtgcgcc tcgaagcgat agccggtagc ggagggatag 3000
ccgcccatca tgccccagtc gctgttcatg aagccgttgc ccatgaagaa catggtccag 3060
tcctgggcgt tccacaccat gcgcagcgtc tcgaacccgc aaccgccgcg gtacttgccg 3120
tagccgccgg aattggcctt gacgttgcgg ccaaggtaga gcagcggctc agccatctcc 3180
cagatttcga tgtcgcccat atcgccttcc ggattccaga tggcggcagc gtggttgaga 3240
ccatccttta cggcgcaggc gccggtgcca caggaactgg cctcgaagct gttgaccgca 3300
tggatctctc cgtcctggtt gatgccgccg ccctgcagcc agttggaggt gttggcgttg 3360
ccggcgttga cctcttccag gtagccgcgg ctgaagtagg actgcgacag gccgcgccac 3420
agcgccgccc agccggagac caggaagtgc caggcatagg catggccggt gcggcgatca 3480
tcggggttgc accacgtgcc cttgggcaag cggaactcgg tagcgaaata cgccccgtcg 3540
ttgatacgct gggtcggcac aagggtctgg cacatcatca cccagattcc ggaagtgaag 3600
gccacctggt gggcgttgaa ggtatgccag ccccagcggc tcgcgccctc aaagtcgagg 3660
cgccactttc cgtccgactt gatggtcatc tccaccggcg aatgcatgat ggagtcgagc 3720
ttggcgaagg cgttggagac ctgcacgtcg tcatgcttgt agggcacgtc gacaaaggaa 3780
accttgcggt acttgccagg cagggtcatc gccttgatgc ggctctgcag tccgcggcgg 3840
ccttcttcga tgacctcata ggcgaacttc tcgtaggcct cgaggccgtc ggcacgaatc 3900
acttcttcga ccagttcgcg gatcatgtgg cagccagcga tccgggtttt ctcatcgagg 3960
atccagtatt tcggcgtgcg caccgagcgc tgcgactcgt gcagccagtc gcgcagcggc 4020
tcgtcgttca cgccggtctt gcggcaggtg atcatgtagc cgtcgccaaa gcgctgcgtc 4080
tgcccggttg acatcgagcc cggcgttacc gagccggtgt cgatgacgtg ggtgacgccg 4140
cctacccagc cgatcagctt cccctcccag aagatcggca cgatggtggc gatgtcgcag 4200
gggtgcacgt tgccgatgga gcagtcgttg ttggtgaaca tgtcaccggg attgatgccg 4260
gggttggcct cccagttgtt ctcgatcatg tacttgatcg ccgcccccat ggtgccgacg 4320
tggatgatga tgccggtcga ggtcagcacg cagtcaccca cagcgttgta gagcgtgaag 4380

cagagttcgc cttcctgctc gacgatgggg ctggcggcaa ttttcttcgc ggtttcgcgg 4440

gcatgcacca agccgccgcg cagcttggag aacagcttct catagccgat cgggtcgctg 4500

tcgcggaact ccagtttgtc gaggccgttg tagtggccgg acgcctgagt gcgagcgatg 4560

atcgcgtcgc ggagctcctt gagggtcttg ccgttctgca gcagattgcc caggcccact 4620

tccttgttgc tcatcatatt catatcttga atctcctgaa attactcttc gacctgaacg 4680

acggtggtga catcgggcag cagggagagg tccatgcggt gcttggcgcc gtaggatggc 4740

acgcccagct cttcttcacg cacctgccag tcggcgggca ggctccagaa ttccttgaac 4800

tcccgcgtga acttctcaga cagggcaaag ctggtggcga acatgtgctg cacctggacg 4860

gacgcgtgct tgttgagaat gcgttcgcgc tcttccttca tccattcccg cgtcggcagc 4920

gcccgggcca ggcgttcctt gcgaacttcg gcacggcgtg cttgggtctt gacggcatcc 4980

acggtaaaga cgcccttgtc gtcttgggtg aagacagccc catatacctt ctgcgcgtag 5040

gccggcagca ggaacttctg gttcaggtcc gcttcgatgg cttgcgggtc ccggtcgaga 5100

gggtcgccaa agcccgggcc accgcgcagg tagttcaggt acagatcatg gttggcgtag 5160

cagtcctccg tcgtgatgca ctgcttgtcg cgcttgacct cggcggtggc gtcgatgtgg 5220

cgttcgaagt cggggttact cggatcgatg tcgccgccca gcggcaggga gtcgccgatg 5280

gcgatgcgct ggtcgaggcc ggtcttgtgc gcctcgaagc gatagccggt agcggaggga 5340

tagccgccca tcatgcccca gtcgctgttc atgaagccgt tgcccatgaa gaacatggtc 5400

cagtcctggg cgttccacac catgcgcagc gtctcgaacc cgcaaccgcc gcggtacttg 5460

ccgtagccgc cggaattggc cttgacgttg cggccaaggt agagcagcgg ctcagccatc 5520

tcccagattt cgatgtcgcc catatcgcct tccggattcc agatggcggc agcgtggttg 5580

agaccatcct ttacggcgca ggcgccggtg ccacaggaac tggcctcgaa gctgttgacc 5640

gcatggatct ctccgtcctg gttgatgccg ccgccctgca gccagttgga ggtgttggcg 5700

ttgccggcgt tgacctcttc caggtagccg cggctgaagt aggactgcga caggccgcgc 5760

cacagcgccg cccagccgga gaccaggaag tgccaggcat aggcatggcc ggtgcggcga 5820

tcatcggggt tgcaccacgt gcccttgggc aagcggaact cggtagcgaa atacgccccg 5880

tcgttgatac gctgggtcgg cacaagggtc tggcacatca tcacccagat tccggaagtg 5940

aaggccacct ggtgggcgtt gaaggtatgc cagccccagc ggctcgcgcc ctcaaagtcg 6000

aggcgccact ttccgtccga cttgatggtc atctccaccg gcgaatgcat gatggagtcg 6060

agcttggcga aggcgttgga gacctgcacg tcgtcatgct tgtagggcac gtcgacaaag 6120

gaaaccttgc ggtacttgcc aggcagggtc atcgccttga tgcggctctg cagtccgcgg 6180

cggccttctt cgatgacctc ataggcgaac ttctcgtagg cctcgaggcc gtcggcacga 6240

atcacttctt cgaccagttc gcggatcatg tggcagccag cgatccgggt tttctcatcg 6300

aggatccagt atttcggcgt gcgcaccgag cgctgcgact cgtgcagcca gtcgcgcagc 6360

ggctcgtcgt tcacgccggt cttgcggcag gtgatcatgt agccgtcgcc aaagcgctgc 6420

gtctgcccgg ttgacatcga gcccggcgtt accgagccgg tgtcgatgac gtgggtgacg 6480

ccgcctaccc agccgatcag cttcccctcc cagaagatcg gcacgatggt ggcgatgtcg 6540

caggggtgca cgttgccgat ggagcagtcg ttgttggtga acatgtcacc gggattgatg 6600

ccggggttgg cctcccagtt gttctcgatc atgtacttga tcgccgcccc catggtgccg 6660

acgtggatga tgatgccggt cgaggtcagc acgcagtcac ccacagcgtt gtagagcgtg 6720

aagcagagtt cgccttcctg ctcgacgatg gggctggcgg caattttctt cgcggtttcg 6780

cgggcatgca ccaagccgcc gcgcagcttg gagaacagct tctcatagcc gatcgggtcg 6840

ctgtcgcgga actccagttt gtcgaggccg ttgtagtggc cggacgcctg agtgcgagcg 6900

atgatcgcgt cgcggagctc cttgagggtc ttgccgttct gcagcagatt gcccaggccc 6960

acttccttgt tgctcatcat attcattctt gaatctcctg aaactttcta gcacttttcc 7020

agcaggatcg cggtgccctg gccgccgccg atgcacaggg tcgccagccc cttcttggcg 7080

tcgcgcttct gcatcgcgtg caccagggtc accaggatgc gggcgcccga ggcgccgatc 7140

gggtgcccca gggcgatggc gccgccattc acgttcactt tgttcatgtc gaacttcagg 7200

tccttggcga cggccagcga ctgggcggca aaggcctcgt tcgactcgat caggtccagc 7260

tcgtcgaccg tccagccggc tttctcgatc gccgccttgg tggcgtagaa cgggccgtag 7320

cccatgatgg ccgggtccac gccggccgag ccatacgaca cgatcttcgc cagcggtttc 7380

acgcccagct ccttggcctt ttcggccgac atgatcacca gcacggccgc gcagtcgttc 7440

aggcccgagg cgttgcccgc ggtcacggtg ccgtccttct tgaaggccgg cttcagcttg 7500

gccaggccct cgatcgtcga cccgaagcgc gggtgctcgt ccgtgtccac cacggtctcg 7560

cccttgcggc ccttaatcac caccggcacg atctcgtcct tgaactggcc cgacttgatg 7620

gcttcctccg ccttcttttg cgaggccagg gcgaactcgt cctgctcctc gcgcgagatg 7680

ttccagcgct cggcgatgtt ctcggcggtg atgcccatgt ggtagtcgtt gaaggcgtcc 7740

cacaggccgt cggtgatcat ctcgtccacg aacttggcgt tccccatgcg gtagccccag 7800

cgggcgttgt tggccaggta cggggcgcgc gacatgtttt ccatgccgcc ggcaatgatc 7860

acgtcggcgt cgccggcctt gatgatctgg gccgccagcg acacggtgcg caggcccgag 7920

ccgcacacct tgttgatggt catggcgggg atctccaccg ggaggccggc tttgaagctc 7980

gcctggcggg ccgggttctg gccgagcccg gcctgcagca cgttgcccag gatcacctcg 8040

ttcacgtcct ccggcttgat gccggccttc ttcacggcct ccttaatggc ggtggcgccc 8100

aggtccacgg cgggcacgtc tttcaggctc ttgccgtacg agccgatcgc ggtgcgcacg 8160

gccgaggcaa tcaccacctc cttcattctt gaatctcctg aaaggtaccc agcttttgtt 8220

ccctttagtg agggttaatt gcgcgcttgg cgtaatcatg gtcatagctg tttcctgtgt 8280

gaaattgtta tccgctcaca attccacaca acatacgagc cggaagcata aagtgtaaag 8340

cctggggtgc ctaatgagtg agctaactca cattaattgc gttgcgctca ctgcccgctt 8400

tccagtcggg aaacctgtcg tgccagctgc attaatgaat cggccaacgc gcggggagag 8460

gcggtttgcg tattgggcgc atgcataaaa actgttgtaa ttcattaagc attctgccga 8520

catggaagcc atcacaaacg gcatgatgaa cctgaatcgc cagcggcatc agcaccttgt 8580

cgccttgcgt ataatatttg cccatggggg tgggcgaaga actccagcat gagatccccg 8640

cgctggagga tcatccagcc ggcgtcccgg aaaacgattc cgaagcccaa cctttcatag 8700

aaggcggcgg tggaatcgaa atctcgtgat ggcaggttgg gcgtcgcttg gtcggtcatt 8760

tcgaacccca gagtcccgct cagaagaact cgtcaagaag gcgatagaag gcgatgcgct 8820

gcgaatcggg agcggcgata ccgtaaagca cgaggaagcg gtcagcccat tcgccgccaa 8880

gctcttcagc aatatcacgg gtagccaacg ctatgtcctg atagcggtcc gccacaccca 8940

gccggccaca gtcgatgaat ccagaaaagc ggccattttc caccatgata ttcggcaagc 9000

aggcatcgcc atgggtcacg acgagatcct cgccgtcggg catgcgcgcc ttgagcctgg 9060

cgaacagttc ggctggcgcg agcccctgat gctcttcgtc cagatcatcc tgatcgacaa 9120

gaccggcttc catccgagta cgtgctcgct cgatgcgatg tttcgcttgg tggtcgaatg 9180

ggcaggtagc cggatcaagc gtatgcagcc gccgcattgc atcagccatg atggatactt 9240

tctcggcagg agcaaggtga gatgacagga gatcctgccc cggcacttcg cccaatagca 9300

gccagtccct tcccgcttca gtgacaacgt cgagcacagc tgcgcaagga acgcccgtcg 9360

tggccagcca cgatagccgc gctgcctcgt cctgcagttc attcagggca ccggacaggt 9420

cggtcttgac aaaaagaacc gggcgcccct gcgctgacag ccggaacacg gcggcatcag 9480

agcagccgat tgtctgttgt gcccagtcat agccgaatag cctctccacc caagcggccg 9540

gagaacctgc gtgcaatcca tcttgttcaa tcatgcgaaa cgatcctcat cctgtctctt 9600

gatcagatct tgatcccctg cgccatcaga tccttggcgg caagaaagcc atccagttta 9660

ctttgcaggg cttcccaacc ttaccagagg gcgccccagc tggcaattcc ggttcgcttg 9720

ctgtccataa aaccgcccag tctagctatc gccatgtaag cccactgcaa gctacctgct 9780

ttctctttgc gcttgcgttt tcccttgtcc agatagccca gtagctgaca ttcatcccag 9840

gtggcacttt tcggggaaat gtgcgcgccc gcgttcctgc tggcgctggg cctgtttctg 9900

gcgctggact tcccgctgtt ccgtcagcag cttttcgccc acggccttga tgatcgcggc 9960

ggccttggcc tgcatatccc gattcaacgg ccccagggcg tccagaacgg gcttcaggcg 10020

ctcccgaagg t                                                          10031

<210>  6
<211>  5330
<212>  DNA
<213>  Artificial Sequence

<220>
<223>  vector

<400>  6
ccccaggctt tacactttat gcttccggct cgtatgttgt gtggaattgt gagcggataa      60

caatttcaca tttcaggaga ttcaagaatg aatatgatga gcaacaagga agtgggcctg     120

ggcaatctgc tgcagaacgg caagaccctc aaggagctcc gcgacgcgat catcgctcgc     180

actcaggcgt ccggccacta caacggcctc gacaaactgg agttccgcga cagcgacccg     240

atcggctatg agaagctgtt ctccaagctg cgcggcggct tggtgcatgc ccgcgaaacc     300

gcgaagaaaa ttgccgccag ccccatcgtc gagcaggaag gcgaactctg cttcacgctc     360

tacaacgctg tgggtgactg cgtgctgacc tcgaccggca tcatcatcca cgtcggcacc     420

atgggggcgg cgatcaagta catgatcgag aacaactggg aggccaaccc cggcatcaat     480

cccggtgaca tgttcaccaa caacgactgc tccatcggca acgtgcaccc ctgcgacatc     540

gccaccatcg tgccgatctt ctgggagggg aagctgatcg gctgggtagg cggcgtcacc     600

cacgtcatcg acaccggctc ggtaacgccg ggctcgatgt caaccgggca gacgcagcgc     660

tttggcgacg gctacatgat cacctgccgc aagaccggcg tgaacgacga gccgctgcgc     720

gactggctgc acgagtcgca gcgctcggtg cgcacgccga aatactggat cctcgatgag     780

aaaacccgga tcgctggctg ccacatgatc cgcgaactgg tcgaagaagt gattcgtgcc     840

gacggcctcg aggcctacga gaagttcgcc tatgaggtca tcgaagaagg ccgccgcgga     900

ctgcagagcc gcatcaaggc gatgaccctg cctggcaagt accgcaaggt ttcctttgtc     960

gacgtgccct acaagcatga cgacgtgcag gtctccaacg ccttcgccaa gctcgactcc    1020

atcatgcatt cgccggtgga gatgaccatc aagtcggacg gaaagtggcg cctcgacttt    1080

gagggcgcga gccgctgggg ctggcatacc ttcaacgccc accaggtggc cttcacttcc    1140

ggaatctggg tgatgatgtg ccagaccctt gtgccgaccc agcgtatcaa cgacggggcg    1200

tatttcgcta ccgagttccg cttgcccaag ggcacgtggt gcaaccccga tgatcgccgc    1260

accggccatg cctatgcctg gcacttcctg gtctccggct gggcggcgct gtggcgcggc    1320

ctgtcgcagt cctacttcag ccgcggctac ctggaagagg tcaacgccgg caacgccaac    1380

acctccaact ggctgcaggg cggcggcatc aaccaggacg gagagatcca tgcggtcaac    1440

agcttcgagg ccagttcctg tggcaccggc gcctgcgccg taaaggatgg tctcaaccac    1500

gctgccgcca tctggaatcc ggaaggcgat atgggcgaca tcgaaatctg ggagatggct    1560

gagccgctgc tctaccttgg ccgcaacgtc aaggccaatt ccggcggcta cggcaagtac 1620

cgcggcggtt gcgggttcga gacgctgcgc atggtgtgga acgcccagga ctggaccatg 1680

ttcttcatgg gcaacggctt catgaacagc gactggggca tgatgggcgg ctatccctcc 1740

gctaccggct atcgcttcga ggcgcacaag accggcctcg accagcgcat cgccatcggc 1800

gactccctgc cgctgggcgg cgacatcgat ccgagtaacc ccgacttcga acgccacatc 1860

gacgccaccg ccgaggtcaa gcgcgacaag cagtgcatca cgacggagga ctgctacgcc 1920

aaccatgatc tgtacctgaa ctacctgcgc ggtggcccgg gctttggcga ccctctcgac 1980

cgggacccgc aagccatcga agcggacctg aaccagaagt tcctgctgcc ggcctacgcg 2040

cagaaggtat atggggctgt cttcacccaa gacgacaagg gcgtctttac cgtggatgcc 2100

gtcaagaccc aagcacgccg tgccgaagtt cgcaaggaac gcctggcccg ggcgctgccg 2160

acgcgggaat ggatgaagga agagcgcgaa cgcattctca acaagcacgc gtccgtccag 2220

gtgcagcaca tgttcgccac cagctttgcc ctgtctgaga agttcacgcg ggagttcaag 2280

gaattctgga gcctgcccgc cgactggcag gtgcgtgaag aagagctggg cgtgccatcc 2340

tacggcgcca agcaccgcat ggacctctcc ctgctgcccg atgtcaccac cgtcgttcag 2400

gtcgaagagt aatttcagga gattcaagat atgaatatga tgagcaacaa ggaagtgggc 2460

ctgggcaatc tgctgcagaa cggcaagacc ctcaaggagc tccgcgacgc gatcatcgct 2520

cgcactcagg cgtccggcca ctacaacggc ctcgacaaac tggagttccg cgacagcgac 2580

ccgatcggct atgagaagct gttctccaag ctgcgcggcg gcttggtgca tgcccgcgaa 2640

accgcgaaga aaattgccgc cagccccatc gtcgagcagg aaggcgaact ctgcttcacg 2700

ctctacaacg ctgtgggtga ctgcgtgctg acctcgaccg gcatcatcat ccacgtcggc 2760

accatggggg cggcgatcaa gtacatgatc gagaacaact gggaggccaa ccccggcatc 2820

aatcccggtg acatgttcac caacaacgac tgctccatcg gcaacgtgca cccctgcgac 2880

atcgccacca tcgtgccgat cttctgggag gggaagctga tcggctgggt aggcggcgtc 2940

acccacgtca tcgacaccgg ctcggtaacg ccgggctcga tgtcaaccgg gcagacgcag 3000

cgctttggcg acggctacat gatcacctgc cgcaagaccg gcgtgaacga cgagccgctg 3060

cgcgactggc tgcacgagtc gcagcgctcg gtgcgcacgc cgaaatactg gatcctcgat 3120

gagaaaaccc ggatcgctgg ctgccacatg atccgcgaac tggtcgaaga agtgattcgt 3180

gccgacggcc tcgaggccta cgagaagttc gcctatgagg tcatcgaaga aggccgccgc 3240

ggactgcaga gccgcatcaa ggcgatgacc ctgcctggca agtaccgcaa ggtttccttt 3300

gtcgacgtgc cctacaagca tgacgacgtg caggtctcca acgccttcgc caagctcgac 3360

tccatcatgc attcgccggt ggagatgacc atcaagtcgg acggaaagtg gcgcctcgac 3420

tttgagggcg cgagccgctg gggctggcat accttcaacg cccaccaggt ggccttcact 3480
tccggaatct gggtgatgat gtgccagacc cttgtgccga cccagcgtat caacgacggg 3540
gcgtatttcg ctaccgagtt ccgcttgccc aagggcacgt ggtgcaaccc cgatgatcgc 3600
cgcaccggcc atgcctatgc ctggcacttc ctggtctccg gctgggcggc gctgtggcgc 3660
ggcctgtcgc agtcctactt cagccgcggc tacctggaag aggtcaacgc cggcaacgcc 3720
aacacctcca actggctgca gggcggcggc atcaaccagg acggagagat ccatgcggtc 3780
aacagcttcg aggccagttc ctgtggcacc ggcgcctgcg ccgtaaagga tggtctcaac 3840
cacgctgccg ccatctggaa tccggaaggc gatatgggcg acatcgaaat ctgggagatg 3900
gctgagccgc tgctctacct tggccgcaac gtcaaggcca attccggcgg ctacggcaag 3960
taccgcggcg gttgcgggtt cgagacgctg cgcatggtgt ggaacgccca ggactggacc 4020
atgttcttca tgggcaacgg cttcatgaac agcgactggg gcatgatggg cggctatccc 4080
tccgctaccg gctatcgctt cgaggcgcac aagaccggcc tcgaccagcg catcgccatc 4140
ggcgactccc tgccgctggg cggcgacatc gatccgagta accccgactt cgaacgccac 4200
atcgacgcca ccgccgaggt caagcgcgac aagcagtgca tcacgacgga ggactgctac 4260
gccaaccatg atctgtacct gaactacctg cgcggtggcc cgggctttgg cgaccctctc 4320
gaccgggacc cgcaagccat cgaagcggac ctgaaccaga agttcctgct gccggcctac 4380
gcgcagaagg tatatggggc tgtcttcacc caagacgaca agggcgtctt taccgtggat 4440
gccgtcaaga cccaagcacg ccgtgccgaa gttcgcaagg aacgcctggc ccgggcgctg 4500
ccgacgcggg aatggatgaa ggaagagcgc gaacgcattc tcaacaagca cgcgtccgtc 4560
caggtgcagc acatgttcgc caccagcttt gccctgtctg agaagttcac gcgggagttc 4620
aaggaattct ggagcctgcc cgccgactgg caggtgcgtg aagaagagct gggcgtgcca 4680
tcctacggcg ccaagcaccg catggacctc tccctgctgc ccgatgtcac caccgtcgtt 4740
caggtcgaag agtaatttca ggagattcaa gaatgtcaac ctataccaat gaacaggtcg 4800
cccacctcgt cgatggttcc ctcgattggg agaccacctt ccgcatgctc tcgatgccca 4860
aggacgagag ccggttcgcg cagtacctgt cagccttgca ggccaaggtg aactttcctg 4920
accgcatcgt gctaccgctc agcccgcaca tgtttatcgt gcagtcggtc aagagcaaga 4980
agtgggtagt caagtgcgac tgcggtcacg agttctgtga ttaccgcgag aactggaagc 5040
tgcatgccgc gatctacgtg cgagacaccg aggaggcgat gacagaggtg tatccgaagc 5100
tgatggcccc cgacacccag tggcaggtgt atcgcgaata ctactgccca tcgtgcggca 5160
ccatgcacga cgtcgaagcg cctaccccgt ggtatcccgt gatccacgac ttcgagccgg 5220
acatcgaggc cttctacaag gagtgggtga atctcccggt gccggagcgc gcgtccgact 5280
aataaaaata agagttacct taaatggtaa ctcttatttt tttaatattg 5330

<210>  7
<211>  5329
<212>  DNA
<213>  Artificial Sequence

<220>
<223>  expression construct

<400>  7
ccccaggctt tacactttat gcttccggct cgtatgttgt gtggaattgt gagcggataa      60

caatttcaca tttcaggaga ttcaagaatg aatatgatga gcaacaagga agtgggcctg     120

ggcaatctgc tgcagaacgg caagaccctc aaggagctcc gcgacgcgat catcgctcgc     180

actcaggcgt ccggccacta caacggcctc gacaaactgg agttccgcga cagcgacccg     240

atcggctatg agaagctgtt ctccaagctg cgcggcggct tggtgcatgc ccgcgaaacc     300

gcgaagaaaa ttgccgccag ccccatcgtc gagcaggaag gcgaactctg cttcacgctc     360

tacaacgctg tgggtgactg cgtgctgacc tcgaccggca tcatcatcca cgtcggcacc     420

atgggggcgg cgatcaagta catgatcgag aacaactggg aggccaaccc cggcatcaat     480

cccggtgaca tgttcaccaa caacgactgc tccatcggca acgtgcaccc ctgcgacatc     540

gccaccatcg tgccgatctt ctgggagggg aagctgatcg gctgggtagg cggcgtcacc     600

cacgtcatcg acaccggctc ggtaacgccg ggctcgatgt caaccgggca gacgcagcgc     660

tttggcgacg gctacatgat cacctgccgc aagaccggcg tgaacgacga gccgctgcgc     720

gactggctgc acgagtcgca gcgctcggtg cgcacgccga aatactggat cctcgatgag     780

aaaacccgga tcgctggctg ccacatgatc cgcgaactgg tcgaagaagt gattcgtgcc     840

gacggcctcg aggcctacga gaagttcgcc tatgaggtca tcgaagaagg ccgccgcgga     900

ctgcagagcc gcatcaaggc gatgaccctg cctggcaagt accgcaaggt ttcctttgtc     960

gacgtgccct acaagcatga cgacgtgcag gtctccaacg ccttcgccaa gctcgactcc    1020

atcatgcatt cgccggtgga gatgaccatc aagtcggacg gaaagtggcg cctcgacttt    1080

gagggcgcga gccgctgggg ctggcatacc ttcaacgccc accaggtggc cttcacttcc    1140

ggaatctggg tgatgatgtg ccagaccctt gtgccgaccc agcgtatcaa cgacggggcg    1200

tatttcgcta ccgagttccg cttgcccaag ggcacgtggt gcaaccccga tgatcgccgc    1260

accggccatg cctatgcctg gcacttcctg gtctccggct gggcggcgct gtggcgcggc    1320

ctgtcgcagt cctacttcag ccgcggctac ctggaagagg tcaacgccgg caacgccaac    1380

acctccaact ggctgcaggg cggcggcatc aaccaggacg gagagatcca tgcggtcaac    1440

agcttcgagg ccagttcctg tggcaccggc gcctgcgccg taaaggatgg tctcaaccac    1500

gctgccgcca tctggaatcc ggaaggcgat atgggcgaca tcgaaatctg ggagatggct    1560

gagccgctgc tctaccttgg ccgcaacgtc aaggccaatt ccggcggcta cggcaagtac 1620
cgcggcggtt gcgggttcga gacgctgcgc atggtgtgga acgcccagga ctggaccatg 1680
ttcttcatgg gcaacggctt catgaacagc gactggggca tgatgggcgg ctatccctcc 1740
gctaccggct atcgcttcga ggcgcacaag accggcctcg accagcgcat cgccatcggc 1800
gactccctgc cgctgggcgg cgacatcgat ccgagtaacc ccgacttcga acgccacatc 1860
gacgccaccg ccgaggtcaa gcgcgacaag cagtgcatca cgacggagga ctgctacgcc 1920
aaccatgatc tgtacctgaa ctacctgcgc ggtggcccgg gctttggcga ccctctcgac 1980
cgggacccgc aagccatcga agcggacctg aaccagaagt tcctgctgcc ggcctacgcg 2040
cagaaggtat atggggctgt cttcacccaa gacgacaagg gcgtctttac cgtggatgcc 2100
gtcaagaccc aagcacgccg tgccgaagtt cgcaaggaac gcctggcccg ggcgctgccg 2160
acgcgggaat ggatgaagga agagcgcgaa cgcattctca acaagcacgc gtccgtccag 2220
gtgcagcaca tgttcgccac cagctttgcc ctgtctgaga agttcacgcg ggagttcaag 2280
gaattctgga gcctgcccgc cgactggcag gtgcgtgaag aagagctggg cgtgccatcc 2340
tacggcgcca agcaccgcat ggacctctcc ctgctgcccg atgtcaccac cgtcgttcag 2400
gtcgaagagt aatttcagga gattcaagat atgaatatga tgagcaacaa ggaagtgggc 2460
ctgggcaatc tgctgcagaa cggcaagacc ctcaaggagc tccgcgacgc gatcatcgct 2520
cgcactcagg cgtccggcca ctacaacggc ctcgacaaac tggagttccg cgacagcgac 2580
ccgatcggct atgagaagct gttctccaag ctgcgcggcg gcttggtgca tgcccgcgaa 2640
accgcgaaga aaattgccgc cagccccatc gtcgagcagg aaggcgaact ctgcttcacg 2700
ctctacaacg ctgtgggtga ctgcgtgctg acctcgaccg gcatcatcat ccacgtcggc 2760
accatggggg cggcgatcaa gtacatgatc gagaacaact gggaggccaa ccccggcatc 2820
aatcccggtg acatgttcac caacaacgac tgctccatcg gcaacgtgca cccctgcgac 2880
atcgccacca tcgtgccgat cttctgggag gggaagctga tcggctgggt aggcggcgtc 2940
acccacgtca tcgacaccgg ctcggtaacg ccgggctcga tgtcaaccgg gcagacgcag 3000
cgctttggcg acggctacat gatcacctgc cgcaagaccg gcgtgaacga cgagccgctg 3060
cgcgactggc tgcacgagtc gcagcgctcg gtgcgcacgc cgaaatactg gatcctcgat 3120
gagaaaaccc ggatcgctgg ctgccacatg atccgcgaac tggtcgaaga agtgattcgt 3180
gccgacggcc tcgaggccta cgagaagttc gcctatgagg tcatcgaaga aggccgccgc 3240
ggactgcaga gccgcatcaa ggcgatgacc ctgcctggca agtaccgcaa ggtttccttt 3300
gtcgacgtgc cctacaagca tgacgacgtg caggtctcca acgccttcgc caagctcgac 3360
tccatcatgc attcgccggt ggagatgacc atcaagtcgg acggaaagtg gcgcctcgac 3420
tttgagggcg cgagccgctg gggctggcat accttcaacg cccaccaggt ggccttcact 3480

tccggaatct gggtgatgat gtgccagacc cttgtgccga cccagcgtat caacgacggg 3540

gcgtatttcg ctaccgagtt ccgcttgccc aagggcacgt ggtgcaaccc cgatgatcgc 3600

cgcaccggcc atgcctatgc ctggcacttc ctggtctccg gctgggcggc gctgtggcgc 3660

ggcctgtcgc agtcctactt cagccgcggc tacctggaag aggtcaacgc cggcaacgcc 3720

aacacctcca actggctgca gggcggcggc atcaaccagg acggagagat ccatgcggtc 3780

aacagcttcg aggccagttc ctgtggcacc ggcgcctgcg ccgtaaagga tggtctcaac 3840

cacgctgccg ccatctggaa tccggaaggc gatatgggcg acatcgaaat ctgggagatg 3900

gctgagccgc tgctctacct tggccgcaac gtcaaggcca attccggcgg ctacggcaag 3960

taccgcggcg gttgcgggtt cgagacgctg cgcatggtgt ggaacgccca ggactggacc 4020

atgttcttca tgggcaacgg cttcatgaac agcgactggg gcatgatggg cggctatccc 4080

tccgctaccg gctatcgctt cgaggcgcac aagaccggcc tcgaccagcg catcgccatc 4140

ggcgactccc tgccgctggg cggcgacatc gatccgagta accccgactt cgaacgccac 4200

atcgacgcca ccgccgaggt caagcgcgac aagcagtgca tcacgacgga ggactgctac 4260

gccaaccatg atctgtacct gaactacctg cgcggtggcc cgggctttgg cgaccctctc 4320

gaccgggacc cgcaagccat cgaagcggac ctgaaccaga agttcctgct gccggcctac 4380

gcgcagaagg tatatggggc tgtcttcacc caagacgaca agggcgtctt taccgtggat 4440

gccgtcaaga cccaagcacg ccgtgccgaa gttcgcaagg aacgcctggc ccgggcgctg 4500

ccgacgcggg aatggatgaa ggaagagcgc gaacgcattc tcaacaagca cgcgtccgtc 4560

caggtgcagc acatgttcgc caccagcttt gccctgtctg agaagttcac gcgggagttc 4620

aaggaattct ggagcctgcc cgccgactgg caggtgcgtg aagaagagct gggcgtgcca 4680

tcctacggcg ccaagcaccg catggacctc tccctgctgc ccgatgtcac caccgtcgtt 4740

caggtcgaag agtaatttca ggagattcaa gaatgtcaac ctataccaat gaacaggtcg 4800

cccacctcgt cgatggttcc ctcgattggg agaccacctt ccgcatgctc tcgatgccca 4860

aggacgagag ccggttcgcg cagtacctgt cagccttgca ggccaaggtg aactttcctg 4920

accgcatcgt gctaccgctc agcccgcaca tgtttatcgt gcagtcggtc aagagcaaga 4980

agtgggtagt caagtgcgac tgcggtcacg agttctgtga ttaccgcgag aactggaagc 5040

tgcatgccgc gatctacgtg cgagacaccg aggaggcgat gacagaggtg tatccgaagc 5100

tgatggcccc cgacacccag tggcaggtgt atcgcgaata ctactgccca tcgtgcggca 5160

ccatgcacga cgtcgaagcg cctaccccgt ggtatcccgt gatccacgac ttcgagccgg 5220

acatcgaggc cttctacaag gagtgggtga atctcccggt gccggagcgc gcgtccgact 5280

aaaaaaataa gagttacctt aaatggtaac tcttattttt ttaatattg 5329

<210>  8
<211>  2145
<212>  DNA
<213>  Ralstonia eutropha

<400>  8
atgcaaccgc agtccaccgt acaggtgatg ggcatcgatg ctggtggcac gatgaccgac      60

acattcttcg tgcgctcgga tggccgtttc gtggtcggca aggcgcaaag caatcccggc     120

gatgaatccc ttgccatcta taactcgtcc gaagatgcgc tcgcacactg ggaccggaca     180

gtggatgacg tttatccgga actggtgacc tgcgtctatt ccggtacggc catgctcaat     240

cgcatcctga tgcgcaaagg ccttgatgtt ggcctgattt gcaatcgagg cttcgagcag     300

atccattcga tggggcgcgc gctgcagagc tacttgggtt atgccctgga agaccgcatt     360

cacttgaata cgcatcgcta cgatgagccc ctggtgcccg tttcacgcac ccgtggtgtc     420

accgagcgca ccgacgtgca agggaaggta gtcatcccgc tgcgggaaga cgaagtccgc     480

cagtccaccc gcgaactggt ggaggccggc tcgcaagcca tcgttatttg cctgctgcaa     540

tcccacaaga acgaatcgag cgagcaacgc gcccgggaca tcgtgcgtga ggaactgaag     600

aggctgaagg cggatattcc cgtcttcgcc tcggtggact actacccgtc gcgcaaggaa     660

agccaccgga tgaacaccac catccttgag gcttacggcg cagagccttc gcgccagacg     720

ctgaagaagg ttagcgaccg cttcaggaag cacggggcca agttcgatct gcgcgtcatg     780

gctacgcacg gcggcaccat tagctggaag gccaaggaac tcgcgcgcac gattgtttcc     840

ggcccgattg gtggcgtgat cggctcgaaa ctgctcgggg aggccttggg cgacgagaac     900

attgcatgct ccgatattgg cggcaccagc ttcgacgtgg cgttgatcac caagggcaac     960

ttcgctatca agtccgatcc ggacatggct cgcctcgtgc tctctctacc gctagtcgcc    1020

atggactcgg tcggcgcggg tgccggcagc ttcgtgcgtc tggatcccta cagcaagtcc    1080

atcaagcttg gcccggactc tgctggctat cgcgtcggca cctgctggcc ggaaagcggt    1140

ctcgacaccg tttcggtttc cgactgccac gtggtgctgg gctacctgaa tccggacaac    1200

tttctcggtg gcgccatcaa gctcgatgtg gaacgcgccc gccagcatat caaggcgcag    1260

atcgccgatc cgctcggcct gtcggtggaa gatgccgcgg ccggcgtcat cgagttgctg    1320

gacctgacgc tgtccgaata cctgcgcgcc aatatcagcg ccaagggcta caacccggcg    1380

gagttcacgt gcttctccta tggtggcgcc ggccccgtgc atacctacgg ttatacggag    1440

ggggtaggtt tcaaggacgt ggtcgtgccc gcctgggcgg ccggcttctc ggccttcggc    1500

tgtgcctgcg ccgacttcga atatcgctat gacaagtcgg tcgacgtcgg tgtcgcacaa    1560

ctcgcgccgg acggcgataa ggctgccgcc tgcaagacct tgcaggaagc ctggtccgaa    1620

ttggccatca aggttatcga cgagtttgtc ctcaacggct acaagcccga agacgtgctg    1680

ctgatccccg gctacaagat gcagtacatg ggccaactga acgaccttga aatcgtatcg 1740

ccggtgacca gtgctgccac cgcagccgac tgggacaaga tcgtcgaggc cttcgagacc 1800

acctatggcc gggtgtacgc cagttccgcg cgctcgccgg aactgggctt ctcgatcacc 1860

ggcgcgatcc tgcgcggcac cgtcgttacc cagaagccgg tactgccgga agacccggat 1920

gcgggcccat tgccgcccaa ggaagcctat ctcggcaccc gccccttcta tcgccacaag 1980

aagtgggtgg atgccgcact gtggaagatg gaggcgctca aggccggcaa ccacatcgtg 2040

ggccccgcca tcatcgaatc cgatgccacc accttcgtcg tgccggatgg tttcgagaca 2100

acggtcgaca agcatcgcct gttccacctc aaggaagtga agtaa 2145

<210> 9
<211> 2325
<212> DNA
<213> Ralstonia eutropha

<400> 9
atgaatatga tgagcaacaa ggaagtgggc ctgggcaatc tgctgcagaa cggcaagacc 60

ctcaaggagc tccgcgacgc gatcatcgct cgcactcagg cgtccggcca ctacaacggc 120

ctcgacaaac tggagttccg cgacagcgac ccgatcggct atgagaagct gttctccaag 180

ctgcgcggcg gcttggtgca tgcccgcgaa accgcgaaga aaattgccgc cagccccatc 240

gtcgagcagg aaggcgaact ctgcttcacg ctctacaacg ctgtgggtga ctgcgtgctg 300

acctcgaccg gcatcatcat ccacgtcggc accatggggg cggcgatcaa gtacatgatc 360

gagaacaact gggaggccaa ccccggcatc aatcccggtg acatgttcac caacaacgac 420

tgctccatcg gcaacgtgca cccctgcgac atcgccacca tcgtgccgat cttctgggag 480

gggaagctga tcggctgggt aggcggcgtc acccacgtca tcgacaccgg ctcggtaacg 540

ccgggctcga tgtcaaccgg gcagacgcag cgctttggcg acggctacat gatcacctgc 600

cgcaagaccg gcgtgaacga cgagccgctg cgcgactggc tgcacgagtc gcagcgctcg 660

gtgcgcacgc cgaaatactg gatcctcgat gagaaaaccc ggatcgctgg ctgccacatg 720

atccgcgaac tggtcgaaga agtgattcgt gccgacggcc tcgaggccta cgagaagttc 780

gcctatgagg tcatcgaaga aggccgccgc ggactgcaga gccgcatcaa ggcgatgacc 840

ctgcctggca agtaccgcaa ggtttccttt gtcgacgtgc cctacaagca tgacgacgtg 900

caggtctcca acgccttcgc caagctcgac tccatcatgc attcgccggt ggagatgacc 960

atcaagtcgg acggaaagtg gcgcctcgac tttgagggcg cgagccgctg gggctggcat 1020

accttcaacg cccaccaggt ggccttcact tccggaatct gggtgatgat gtgccagacc 1080

cttgtgccga cccagcgtat caacgacggg gcgtatttcg ctaccgagtt ccgcttgccc 1140

aagggcacgt ggtgcaaccc cgatgatcgc ccgcaccggc atgcctatgc ctggcacttc 1200

ctggtctccg gctgggcggc gctgtggcgc ggcctgtcgc agtcctactt cagccgcggc 1260

tacctggaag aggtcaacgc cggcaacgcc aacacctcca actggctgca gggcggcggc 1320

atcaaccagg acggagagat ccatgcggtc aacagcttcg aggccagttc ctgtggcacc 1380

ggcgcctgcg ccgtaaagga tggtctcaac cacgctgccg ccatctggaa tccggaaggc 1440

gatatgggcg acatcgaaat ctgggagatg gctgagccgc tgctctacct tggccgcaac 1500

gtcaaggcca attccggcgg ctacggcaag taccgcggcg gttgcgggtt cgagacgctg 1560

cgcatggtgt ggaacgccca ggactggacc atgttcttca tgggcaacgg cttcatgaac 1620

agcgactggg gcatgatggg cggctatccc tccgctaccg gctatcgctt cgaggcgcac 1680

aagaccggcc tcgaccagcg catcgccatc ggcgactccc tgccgctggg cggcgacatc 1740

gatccgagta accccgactt cgaacgccac atcgacgcca ccgccgaggt caagcgcgac 1800

aagcagtgca tcacgacgga ggactgctac gccaaccatg atctgtacct gaactacctg 1860

cgcggtggcc cgggctttgg cgaccctctc gaccgggacc cgcaagccat cgaagcggac 1920

ctgaaccaga agttcctgct gccggcctac gcgcagaagg tatatggggc tgtcttcacc 1980

caagacgaca agggcgtctt taccgtggat gccgtcaaga cccaagcacg ccgtgccgaa 2040

gttcgcaagg aacgcctggc ccgggcgctg ccgacgcggg aatggatgaa ggaagagcgc 2100

gaacgcattc tcaacaagca cgcgtccgtc caggtgcagc acatgttcgc caccagcttt 2160

gccctgtctg agaagttcac gcgggagttc aaggaattct ggagcctgcc cgccgactgg 2220

caggtgcgtg aagaagagct gggcgtgcca tcctacggcg ccaagcaccg catggacctc 2280

tccctgctgc ccgatgtcac caccgtcgtt caggtcgaag agtaa 2325

<210> 10
<211> 5144
<212> DNA
<213> Artificial Sequence

<220>
<223> vector

<400> 10
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaattttct ctggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag gggccacttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatcccccg ggctgcagga attcgatatc aagcttatcg ataccgtcga 3300

cctcgagggg gggcccggta cccagctttt gttcccttta gtgagggtta attgcgcgct 3360

tggcgtaatc atggtcatag ctgtttcctg tgtgaaattg ttatccgctc acaattccac 3420

acaacatacg agccggaagc ataaagtgta aagcctgggg tgcctaatga gtgagctaac 3480

tcacattaat tgcgttgcgc tcactgcccg ctttccagtc gggaaacctg tcgtgccagc 3540

tgcattaatg aatcggccaa cgcgcgggga gaggcggttt gcgtattggg cgcatgcata 3600

aaaactgttg taattcatta agcattctgc cgacatggaa gccatcacaa acggcatgat 3660

gaacctgaat cgccagcggc atcagcacct tgtcgccttg cgtataatat ttgcccatgg 3720

gggtgggcga agaactccag catgagatcc ccgcgctgga ggatcatcca gccggcgtcc 3780

cggaaaacga ttccgaagcc caacctttca tagaaggcgg cggtggaatc gaaatctcgt 3840

gatggcaggt tgggcgtcgc ttggtcggtc atttcgaacc ccagagtccc gctcagaaga 3900

actcgtcaag aaggcgatag aaggcgatgc gctgcgaatc gggagcggcg ataccgtaaa 3960

gcacgaggaa gcggtcagcc cattcgccgc caagctcttc agcaatatca cgggtagcca 4020

acgctatgtc ctgatagcgg tccgccacac ccagccggcc acagtcgatg aatccagaaa 4080

agcggccatt ttccaccatg atattcggca agcaggcatc gccatgggtc acgacgagat 4140

cctcgccgtc gggcatgcgc gccttgagcc tggcgaacag ttcggctggc gcgagcccct 4200

gatgctcttc gtccagatca tcctgatcga caagaccggc ttccatccga gtacgtgctc 4260

gctcgatgcg atgtttcgct tggtggtcga atgggcaggt agccggatca agcgtatgca 4320

gccgccgcat tgcatcagcc atgatggata ctttctcggc aggagcaagg tgagatgaca 4380

ggagatcctg ccccggcact tcgcccaata gcagccagtc ccttcccgct tcagtgacaa 4440

cgtcgagcac agctgcgcaa ggaacgcccg tcgtggccag ccacgatagc cgcgctgcct 4500

cgtcctgcag ttcattcagg gcaccggaca ggtcggtctt gacaaaaaga accgggcgcc 4560

cctgcgctga cagccggaac acggcggcat cagagcagcc gattgtctgt tgtgcccagt 4620

catagccgaa tagcctctcc acccaagcgg ccggagaacc tgcgtgcaat ccatcttgtt 4680

caatcatgcg aaacgatcct catcctgtct cttgatcaga tcttgatccc ctgcgccatc 4740

agatccttgg cggcaagaaa gccatccagt ttactttgca gggcttccca accttaccag 4800

agggcgcccc agctggcaat tccggttcgc ttgctgtcca taaaaccgcc cagtctagct 4860

atcgccatgt aagcccactg caagctacct gctttctctt tgcgcttgcg ttttcccttg 4920

tccagatagc ccagtagctg acattcatcc caggtggcac ttttcgggga aatgtgcgcg 4980

cccgcgttcc tgctggcgct gggcctgttt ctggcgctgg acttcccgct gttccgtcag 5040

cagcttttcg cccacggcct tgatgatcgc ggcggccttg gcctgcatat cccgattcaa 5100

cggccccagg gcgtccagaa cgggcttcag gcgctcccga aggt 5144

<210> 11
<211> 351
<212> PRT
<213> Clostridium beijerinckii

<400> 11

Met Lys Gly Phe Ala Met Leu Gly Ile Asn Lys Leu Gly Trp Ile Glu
1               5                   10                  15

Lys Glu Arg Pro Val Ala Gly Ser Tyr Asp Ala Ile Val Arg Pro Leu
            20                  25                  30

Ala Val Ser Pro Cys Thr Ser Asp Ile His Thr Val Phe Glu Gly Ala
        35                  40                  45

Leu Gly Asp Arg Lys Asn Met Ile Leu Gly His Glu Ala Val Gly Glu
    50                  55                  60

Val Val Glu Val Gly Ser Glu Val Lys Asp Phe Lys Pro Gly Asp Arg
65                  70                  75                  80

Val Ile Val Pro Cys Thr Thr Pro Asp Trp Arg Ser Leu Glu Val Gln
                85                  90                  95

Ala Gly Phe Gln Gln His Ser Asn Gly Met Leu Ala Gly Trp Lys Phe
        100                 105                 110

Ser Asn Phe Lys Asp Gly Val Phe Gly Glu Tyr Phe His Val Asn Asp
    115                 120                 125

Ala Asp Met Asn Leu Ala Ile Leu Pro Lys Asp Met Pro Leu Glu Asn
    130                 135                 140

Ala Val Met Ile Thr Asp Met Met Thr Thr Gly Phe His Gly Ala Glu
145                 150                 155                 160

Leu Ala Asp Ile Gln Met Gly Ser Ser Val Val Val Ile Gly Ile Gly
                165                 170                 175

Ala Val Gly Leu Met Gly Ile Ala Gly Ala Lys Leu Arg Gly Ala Gly
        180                 185                 190

Arg Ile Ile Gly Val Gly Ser Arg Pro Ile Cys Val Glu Ala Ala Lys
    195                 200                 205

Phe Tyr Gly Ala Thr Asp Ile Leu Asn Tyr Lys Asn Gly His Ile Val
    210                 215                 220

Asp Gln Val Met Lys Leu Thr Asn Gly Lys Gly Val Asp Arg Val Ile
225                 230                 235                 240

Met Ala Gly Gly Gly Ser Glu Thr Leu Ser Gln Ala Val Ser Met Val
                245                 250                 255

Lys Pro Gly Gly Ile Ile Ser Asn Ile Asn Tyr His Gly Ser Gly Asp
        260                 265                 270

Ala Leu Leu Ile Pro Arg Val Glu Trp Gly Cys Gly Met Ala His Lys
    275                 280                 285

Thr Ile Lys Gly Gly Leu Cys Pro Gly Gly Arg Leu Arg Ala Glu Met
    290                 295                 300

Leu Arg Asp Met Val Val Tyr Asn Arg Val Asp Leu Ser Lys Leu Val
305                 310                 315                 320

Thr His Val Tyr His Gly Phe Asp His Ile Glu Glu Ala Leu Leu Leu
                325                 330                 335

Met Lys Asp Lys Pro Lys Asp Leu Ile Lys Ala Val Val Ile Leu
        340                 345                 350

<210>  12
<211>  1301
<212>  DNA
<213>  Artificial Sequence

<220>
<223>  expression cassette

<400>  12
aacccgcatc acacccgcgt cttgaaatgc ccctaccccg tccctataat tagcactcgt      60

caggggtgag tgctaacagc ctcctgcgac acctgaacat ttctgacggc cgtcgcctcg     120

gtagccggcc gatttgtgat caaaaactca ctttttcagg agattcaaga atgaagggct      180

tcgccatgct gggcatcaac aaactgggct ggatcgagaa ggagcgcccg gtggccggct     240

cgtatgacgc catcgtgcgc ccgctcgccg tgtcgccgtg tacgtcggac atccacaccg     300

tgtttgaggg cgcgctgggg gaccgcaaga atatgatcct ggggcacgag gccgtgggcg     360

aggtggtgga ggtggggtcg gaggtgaagg actttaagcc cggcgaccgg gtgatcgtgc     420

cgtgcaccac ccccgactgg cgctcgctgg aggtccaggc cgggttccag cagcattcga     480

acggcatgct cgccgggtgg aagttctcga acttcaagga cggcgtgttc ggggagtact     540

ttcatgtgaa cgacgccgac atgaacctgg ccatcctgcc gaaagatatg ccgctggaaa     600

acgcggtgat gatcaccgat atgatgacca cggggttcca cggggccgag ctggcggata     660

tccagatggg cagctcggtc gtcgtgatcg gcatcggggc ggtggggctc atgggcatcg     720

cgggcgcgaa gctgcggggg gcgggccgca tcatcggcgt gggctcgcgg ccgatctgcg     780

tggaggcggc caagttctac ggcgccaccg acatcctgaa ctacaagaac ggccatattg     840

tggaccaggt catgaagctg accaacggca agggcgtgga ccgcgtcatc atggccgggg     900

gggggtccga aacgctgtcg caggccgtct cgatggtgaa gccgggcggg atcatttcga     960

acatcaacta ccacggctcg ggcgacgcgc tgctcatccc ccgcgtggaa tggggctgcg    1020

gcatggccca taagaccatt aagggcggcc tgtgccccgg cgggcggctg cgcgcggaga    1080

tgctgcgcga catggtggtg tataatcgcg tcgacctgtc gaaactggtg acgcacgtgt    1140

accatggctt cgaccacatc gaggaggccc tcctgctgat gaaagacaag ccgaaggacc    1200

tgatcaaggc cgtcgtcatc ctgtaagtgg cctgacagtc gccggaaaga aaaggcggcg    1260

ccccgcgggg ccgccctttt tgcttatcga ggcagcctgc t                        1301


<210>  13
<211>  220
<212>  PRT
<213>  Escherichia coli

<400>  13

Met Lys Thr Lys Leu Met Thr Leu Gln Asp Ala Thr Gly Phe Phe Arg

1               5                   10                  15

Asp Gly Met Thr Ile Met Val Gly Gly Phe Met Gly Ile Gly Thr Pro
            20                  25                  30

Ser Arg Leu Val Glu Ala Leu Leu Glu Ser Gly Val Arg Asp Leu Thr
        35                  40                  45

Leu Ile Ala Asn Asp Thr Ala Phe Val Asp Thr Gly Ile Gly Pro Leu
    50                  55                  60

Ile Val Asn Gly Arg Val Arg Lys Val Ile Ala Ser His Ile Gly Thr
65                  70                  75                  80

Asn Pro Glu Thr Gly Arg Arg Met Ile Ser Gly Glu Met Asp Val Val
                85                  90                  95

Leu Val Pro Gln Gly Thr Leu Ile Glu Gln Ile Arg Cys Gly Gly Ala
            100                 105                 110

Gly Leu Gly Gly Phe Leu Thr Pro Thr Gly Val Gly Thr Val Val Glu
        115                 120                 125

Glu Gly Lys Gln Thr Leu Thr Leu Asp Gly Lys Thr Trp Leu Leu Glu
    130                 135                 140

Arg Pro Leu Arg Ala Asp Leu Ala Leu Ile Arg Ala His Arg Cys Asp
145                 150                 155                 160

Thr Leu Gly Asn Leu Thr Tyr Gln Leu Ser Ala Arg Asn Phe Asn Pro
                165                 170                 175

Leu Ile Ala Leu Ala Ala Asp Ile Thr Leu Val Glu Pro Asp Glu Leu
            180                 185                 190

Val Glu Thr Gly Glu Leu Gln Pro Asp His Ile Val Thr Pro Gly Ala
        195                 200                 205

Val Ile Asp His Ile Ile Val Ser Gln Glu Ser Lys
    210                 215                 220

<210>  14
<211>  216
<212>  PRT
<213>  Escherichia coli

<400>  14

Met Asp Ala Lys Gln Arg Ile Ala Arg Arg Val Ala Gln Glu Leu Arg

1                   5                   10                  15

Asp Gly Asp Ile Val Asn Leu Gly Ile Gly Leu Pro Thr Met Val Ala
            20                  25                  30

Asn Tyr Leu Pro Glu Gly Ile His Ile Thr Leu Gln Ser Glu Asn Gly
        35                  40                  45

Phe Leu Gly Leu Gly Pro Val Thr Thr Ala His Pro Asp Leu Val Asn
    50                  55                  60

Ala Gly Gly Gln Pro Cys Gly Val Leu Pro Gly Ala Ala Met Phe Asp
65                  70                  75                  80

Ser Ala Met Ser Phe Ala Leu Ile Arg Gly Gly His Ile Asp Ala Cys
                85                  90                  95

Val Leu Gly Gly Leu Gln Val Asp Glu Glu Ala Asn Leu Ala Asn Trp
            100                 105                 110

Val Val Pro Gly Lys Met Val Pro Gly Met Gly Gly Ala Met Asp Leu
        115                 120                 125

Val Thr Gly Ser Arg Lys Val Ile Ile Ala Met Glu His Cys Ala Lys
    130                 135                 140

Asp Gly Ser Ala Lys Ile Leu Arg Arg Cys Thr Met Pro Leu Thr Ala
145                 150                 155                 160

Gln His Ala Val His Met Leu Val Thr Glu Leu Ala Val Phe Arg Phe
                165                 170                 175

Ile Asp Gly Lys Met Trp Leu Thr Glu Ile Ala Asp Gly Cys Asp Leu
            180                 185                 190

Ala Thr Val Arg Ala Lys Thr Glu Ala Arg Phe Glu Val Ala Ala Asp
        195                 200                 205

Leu Asn Thr Gln Arg Gly Asp Leu
    210                 215

<210>  15
<211>  394
<212>  PRT
<213>  Escherichia blattae

<400>  15

Met Lys Asn Cys Val Ile Val Ser Ala Val Arg Thr Ala Ile Gly Ser

1               5                   10                  15

Phe Asn Gly Ser Leu Ala Ser Thr Ser Ala Ile Asp Leu Gly Ala Thr
            20                  25                  30

Val Ile Lys Ala Ala Ile Glu Arg Ala Lys Ile Asp Ser Gln His Val
        35                  40                  45

Asp Glu Val Ile Met Gly Asn Val Leu Gln Ala Gly Leu Gly Gln Asn
    50                  55                  60

Pro Ala Arg Gln Ala Leu Leu Lys Ser Gly Leu Ala Glu Thr Val Cys
65                  70                  75                  80

Gly Phe Thr Val Asn Lys Val Cys Gly Ser Gly Leu Lys Ser Val Ala
                85                  90                  95

Leu Ala Ala Gln Ala Ile Gln Ala Gly Gln Ala Gln Ser Ile Val Ala
            100                 105                 110

Gly Gly Met Glu Asn Met Ser Leu Ala Pro Tyr Leu Leu Asp Ala Lys
        115                 120                 125

Ala Arg Ser Gly Tyr Arg Leu Gly Asp Gly Gln Val Tyr Asp Val Ile
    130                 135                 140

Leu Arg Asp Gly Leu Met Cys Ala Thr His Gly Tyr His Met Gly Ile
145                 150                 155                 160

Thr Ala Glu Asn Val Ala Lys Glu Tyr Gly Ile Thr Arg Glu Met Gln
                165                 170                 175

Asp Glu Leu Ala Leu His Ser Gln Arg Lys Ala Ala Ala Ala Ile Glu
            180                 185                 190

Ser Gly Ala Phe Thr Ala Glu Ile Val Pro Val Asn Val Val Thr Arg
        195                 200                 205

Lys Lys Thr Phe Val Phe Ser Gln Asp Glu Phe Pro Lys Ala Asn Ser
    210                 215                 220

Thr Ala Glu Ala Leu Gly Ala Leu Arg Pro Ala Phe Asp Lys Ala Gly
225                 230                 235                 240

Thr Val Thr Ala Gly Asn Ala Ser Gly Ile Asn Asp Gly Ala Ala Ala
                245                 250                 255

Leu Val Ile Met Glu Glu Ser Ala Ala Leu Ala Ala Gly Leu Thr Pro
            260                 265                 270

Leu Ala Arg Ile Lys Ser Tyr Ala Ser Gly Gly Val Pro Pro Ala Leu
        275                 280                 285

Met Gly Met Gly Pro Val Pro Ala Thr Gln Lys Ala Leu Gln Leu Ala
    290                 295                 300

Gly Leu Gln Leu Ala Asp Ile Asp Leu Ile Glu Ala Asn Glu Ala Phe
305                 310                 315                 320

Ala Ala Gln Phe Leu Ala Val Gly Lys Asn Leu Gly Phe Asp Ser Glu
                325                 330                 335

Lys Val Asn Val Asn Gly Gly Ala Ile Ala Leu Gly His Pro Ile Gly
            340                 345                 350

Ala Ser Gly Ala Arg Ile Leu Val Thr Leu Leu His Ala Met Gln Ala
        355                 360                 365

Arg Asp Lys Thr Leu Gly Leu Ala Thr Leu Cys Ile Gly Gly Gly Gln
    370                 375                 380

Gly Ile Ala Met Val Ile Glu Arg Leu Asn
385                 390

<210>  16
<211>  2612
<212>  DNA
<213>  Artificial Sequence

<220>
<223>  codonoptimized gen

<400>  16
ggtaccgtat gcaagaggga taaaaaatga agaccaagct catgacgctg caggacgcca      60

ccggcttttt ccgcgatggc atgaccatca tggtgggggg cttcatgggc atcgggacgc     120

cgtcgcggct ggtcgaagcc ctcctggagt cgggggtgcg cgatctgacg ctcattgcca     180

acgacaccgc cttcgtggac accggcattg gccccctgat tgtcaacggc cgcgtccgca     240

aagtgatcgc cagccacatc ggcacgaacc cggagaccgg ccgccggatg atctcgggcg     300

agatggatgt ggtgctggtc ccgcagggga ccctcattga gcaaatccgg tgcggcgggg     360

cgggcctcgg cgggttcctg acgccgaccg gcgtgggcac cgtggtcgag gaaggcaagc     420

agacgctgac cctggacggg aagacctggc tgctggaacg cccgctgcgc gccgatctgg     480

ccctgattcg cgcccatcgc tgcgacaccc tgggcaacct gacgtaccag ctgtccgccc     540

ggaacttcaa cccgctgatt gcgctggccg cggatatcac cctcgtggaa ccggacgaac 600

tcgtcgaaac cggggagctg caaccggatc atatcgtgac ccccggcgcg gtgatcgacc 660

atatcatcgt ctcccaagag tcgaagtaat ggacgccaag cagcgcatcg cccgccgcgt 720

cgcgcaggaa ctgcgcgacg gcgacatcgt gaacctgggc atcggcctgc cgaccatggt 780

ggccaactat ctgccggagg gcatccacat caccctgcag tcggagaacg gcttcctggg 840

cctgggcccg gtgaccaccg cccacccgga cctggtgaac gcggggggcc agccgtgcgg 900

ggtgctgccg ggcgccgcga tgttcgactc ggccatgtcg ttcgccctga tccggggggg 960

gcacatcgac gcctgcgtcc tcggcgggct ccaggtggac gaggaggcca acctggccaa 1020

ctgggtcgtg ccgggcaaaa tggtcccggg catggggggg gccatggacc tcgtgaccgg 1080

ctcgcgcaag gtgatcattg cgatggagca ctgcgccaag gatggctcgg ccaagatcct 1140

gcgccgctgc accatgccgc tgacggccca gcacgccgtg cacatgctgg tgaccgaact 1200

ggccgtgttc cgcttcatcg acggcaagat gtggctgacc gagatcgccg acggctgcga 1260

cctcgcgacc gtgcgcgcca agaccgaggc ccgcttcgag gtggccgccg acctgaacac 1320

ccagcgcggc gacctgtgac ggcaccccta caaacagaag gaatataaaa tgaagaactg 1380

cgtgatcgtg tcggccgtcc gcaccgcgat cggctcgttc aacggctcgc tcgcctcgac 1440

ctcggccatc gacctggggg ccaccgtgat caaggcggcc atcgagcgcg ccaagatcga 1500

ctcgcaacac gtggacgagg tgatcatggg caacgtgctg caagccgggc tgggccagaa 1560

cccggcccgg caggccctcc tgaagtcggg cctcgccgag accgtgtgcg gcttcaccgt 1620

gaacaaggtg tgcggctccg gcctgaagtc ggtcgccctc gccgcccaag ccatccaagc 1680

cgggcaagcc caatcgatcg tggccggcgg catggagaac atgtcgctgg ccccgtacct 1740

gctggacgcc aaggcccgct cgggctatcg gctgggggac ggccaggtgt acgacgtgat 1800

cctccgcgac ggcctcatgt gcgccaccca cggctaccac atgggcatca ccgccgagaa 1860

cgtggccaag gagtacggca tcacccgcga gatgcaggac gagctggccc tgcactcgca 1920

gcgcaaggcc gccgccgcca tcgagtcggg cgccttcacg gccgagatcg tgccggtgaa 1980

cgtggtgacc cgcaagaaga ccttcgtgtt ctcgcaggac gagttcccga aggccaactc 2040

gaccgccgag gccctggggg ccctgcgccc ggccttcgac aaagccggca ccgtgaccgc 2100

gggcaacgcc tcgggcatca acgacggcgc cgcggcgctg gtgatcatgg aggagtcggc 2160

cgcgctggcc gcggggctga ccccgctggc ccgcatcaag tcgtacgcct cgggcggggt 2220

gccgccggcc ctgatgggca tgggcccggt gccggccacc cagaaggccc tgcagctggc 2280

gggcctgcaa ctggcggaca tcgacctgat cgaggccaac gaggccttcg ccgcccagtt 2340

cctggcggtg ggcaagaacc tgggcttcga ctcggagaag gtgaatgtga acggcggcgc 2400

gatcgccctg ggccatccga tcggcgcctc gggcgcgcgc atcctggtga ccctgctgca 2460

cgccatgcag gcccgggata agacgctggg cctggccacc ctgtgcatcg gcgggggcca     2520

gggcatcgcc atggtgatcg aacgcctcaa ctaatcaata aaaacacccg atagcgaaag     2580

ttatcgggtg ttttcttgaa catcgaaagc tt                                   2612

<210>  17
<211>  393
<212>  PRT
<213>  Escherichia coli

<400>  17

Met Thr Asp Val Val Ile Val Ser Ala Ala Arg Thr Ala Val Gly Lys
1               5                   10                  15

Phe Gly Gly Ser Leu Ala Lys Ile Pro Ala Pro Glu Leu Gly Ala Val
            20                  25                  30

Val Ile Lys Ala Ala Leu Glu Arg Ala Gly Val Lys Pro Glu Gln Val
        35                  40                  45

Ser Glu Val Ile Met Gly Gln Val Leu Thr Ala Gly Ser Gly Gln Asn
    50                  55                  60

Pro Ala Arg Gln Ala Ala Ile Lys Ala Gly Leu Pro Ala Met Val Pro
65                  70                  75                  80

Ala Met Thr Ile Asn Lys Val Cys Gly Ser Gly Leu Lys Ala Val Met
                85                  90                  95

Leu Ala Ala Asn Ala Ile Met Ala Gly Asp Ala Glu Ile Val Val Ala
            100                 105                 110

Gly Gly Gln Glu Asn Met Ser Ala Ala Pro His Val Leu Pro Gly Ser
        115                 120                 125

Arg Asp Gly Phe Arg Met Gly Asp Ala Lys Leu Val Asp Thr Met Ile
    130                 135                 140

Val Asp Gly Leu Trp Asp Val Tyr Asn Gln Tyr His Met Gly Ile Thr
145                 150                 155                 160

Ala Glu Asn Val Ala Lys Glu Tyr Gly Ile Thr Arg Glu Ala Gln Asp
                165                 170                 175

Glu Phe Ala Val Gly Ser Gln Asn Lys Ala Glu Ala Ala Gln Lys Ala
            180                 185                 190

Gly Lys Phe Asp Glu Glu Ile Val Pro Val Leu Ile Pro Gln Arg Lys
        195                 200                 205

Gly Asp Pro Val Ala Phe Lys Thr Asp Glu Phe Val Arg Gln Gly Ala
    210                 215                 220

Thr Leu Asp Ser Met Ser Gly Leu Lys Pro Ala Phe Asp Lys Ala Gly
225                 230                 235                 240

Thr Val Thr Ala Ala Asn Ala Ser Gly Leu Asn Asp Gly Ala Ala Ala
                245                 250                 255

Val Val Val Met Ser Ala Ala Lys Ala Lys Glu Leu Gly Leu Thr Pro
            260                 265                 270

Leu Ala Thr Ile Lys Ser Tyr Ala Asn Ala Gly Val Asp Pro Lys Val
        275                 280                 285

Met Gly Met Gly Pro Val Pro Ala Ser Lys Arg Ala Leu Ser Arg Ala
    290                 295                 300

Glu Trp Thr Pro Gln Asp Leu Asp Leu Met Glu Ile Asn Glu Ala Phe
305                 310                 315                 320

Ala Ala Gln Ala Leu Ala Val His Gln Gln Met Gly Trp Asp Thr Ser
                325                 330                 335

Lys Val Asn Val Asn Gly Gly Ala Ile Ala Ile Gly His Pro Ile Gly
            340                 345                 350

Ala Ser Gly Cys Arg Ile Leu Val Thr Leu Leu His Glu Met Lys Arg
        355                 360                 365

Arg Asp Ala Lys Lys Gly Leu Ala Ser Leu Cys Ile Gly Gly Gly Met
    370                 375                 380

Gly Val Ala Leu Ala Val Glu Arg Lys
385                 390

<210> 18
<211> 218
<212> PRT
<213> Clostridium acetobutylicum

<400> 18

Met Asn Ser Lys Ile Ile Arg Phe Glu Asn Leu Arg Ser Phe Phe Lys
1               5                   10                  15

Asp Gly Met Thr Ile Met Ile Gly Gly Phe Leu Asn Cys Gly Thr Pro
            20                  25                  30

Thr Lys Leu Ile Asp Phe Leu Val Asn Leu Asn Ile Lys Asn Leu Thr
        35                  40                  45

Ile Ile Ser Asn Asp Thr Cys Tyr Pro Asn Thr Gly Ile Gly Lys Leu
    50                  55                  60

Ile Ser Asn Asn Gln Val Lys Lys Leu Ile Ala Ser Tyr Ile Gly Ser
65                  70                  75                  80

Asn Pro Asp Thr Gly Lys Lys Leu Phe Asn Asn Glu Leu Glu Val Glu
                85                  90                  95

Leu Ser Pro Gln Gly Thr Leu Val Glu Arg Ile Arg Ala Gly Gly Ser
            100                 105                 110

Gly Leu Gly Gly Val Leu Thr Lys Thr Gly Leu Gly Thr Leu Ile Glu
        115                 120                 125

Lys Gly Lys Lys Lys Ile Ser Ile Asn Gly Thr Glu Tyr Leu Leu Glu
    130                 135                 140

Leu Pro Leu Thr Ala Asp Val Ala Leu Ile Lys Gly Ser Ile Val Asp
145                 150                 155                 160

Glu Ala Gly Asn Thr Phe Tyr Lys Gly Thr Thr Lys Asn Phe Asn Pro
                165                 170                 175

Tyr Met Ala Met Ala Ala Lys Thr Val Ile Val Glu Ala Glu Asn Leu
            180                 185                 190

Val Ser Cys Glu Lys Leu Glu Lys Glu Lys Ala Met Thr Pro Gly Val
        195                 200                 205

Leu Ile Asn Tyr Ile Val Lys Glu Pro Ala
    210                 215

<210> 19
<211> 221
<212> PRT
<213> Escherichia coli

<400> 19

Met Ile Asn Asp Lys Asn Leu Ala Lys Glu Ile Ile Ala Lys Arg Val
1               5                   10                  15

Ala Arg Glu Leu Lys Asn Gly Gln Leu Val Asn Leu Gly Val Gly Leu
            20                  25                  30

Pro Thr Met Val Ala Asp Tyr Ile Pro Lys Asn Phe Lys Ile Thr Phe
        35                  40                  45

Gln Ser Glu Asn Gly Ile Val Gly Met Gly Ala Ser Pro Lys Ile Asn
    50                  55                  60

Glu Ala Asp Lys Asp Val Val Asn Ala Gly Gly Asp Tyr Thr Thr Val
65                  70                  75                  80

Leu Pro Asp Gly Thr Phe Phe Asp Ser Ser Val Ser Phe Ser Leu Ile
                85                  90                  95

Arg Gly Gly His Val Asp Val Thr Val Leu Gly Ala Leu Gln Val Asp
            100                 105                 110

Glu Lys Gly Asn Ile Ala Asn Trp Ile Val Pro Gly Lys Met Leu Ser
        115                 120                 125

Gly Met Gly Gly Ala Met Asp Leu Val Asn Gly Ala Lys Lys Val Ile
    130                 135                 140

Ile Ala Met Arg His Thr Asn Lys Gly Gln Pro Lys Ile Leu Lys Lys
145                 150                 155                 160

Cys Thr Leu Pro Leu Thr Ala Lys Ser Gln Ala Asn Leu Ile Val Thr
                165                 170                 175

Glu Leu Gly Val Ile Glu Val Ile Asn Asp Gly Leu Leu Leu Thr Glu
            180                 185                 190

Ile Asn Lys Asn Thr Thr Ile Asp Glu Ile Arg Ser Leu Thr Ala Ala
        195                 200                 205

Asp Leu Leu Ile Ser Asn Glu Leu Arg Pro Met Ala Val
    210                 215                 220

<210>  20
<211>  2642
<212>  DNA
<213>  Artificial Sequence

<220>
<223>  codonoptimized gen

<400>  20
ggtacctttc cattgaaagg actacacaat gactgacgtt gtcatcgtat ccgccgcccg        60

caccgcggtc ggcaagtttg gcggctcgct ggccaagatc ccggcaccgg aactgggtgc 120

cgtggtcatc aaggccgcgc tggagcgcgc cggcgtcaag ccggagcagg tgagcgaagt 180

catcatgggc caggtgctga ccgccggttc gggccagaac cccgcacgcc aggccgcgat 240

caaggccggc ctgccggcga tggtgccggc catgaccatc aacaaggtgt gcggctcggg 300

cctgaaggcc gtgatgctgg ccgccaacgc gatcatggcg ggcgacgccg agatcgtggt 360

ggccggcggc caggaaaaca tgagcgccgc cccgcacgtg ctgccgggct cgcgcgatgg 420

tttccgcatg ggcgatgcca agctggtcga caccatgatc gtcgacggcc tgtgggacgt 480

gtacaaccag taccacatgg gcatcaccgc cgagaacgtg gccaaggaat acggcatcac 540

acgcgaggcg caggatgagt tcgccgtcgg ctcgcagaac aaggccgaag ccgcgcagaa 600

ggccggcaag tttgacgaag agatcgtccc ggtgctgatc ccgcagcgca agggcgaccc 660

ggtggccttc aagaccgacg agttcgtgcg ccagggcgcc acgctggaca gcatgtccgg 720

cctcaagccc gccttcgaca aggccggcac ggtgaccgcg gccaacgcct cgggcctgaa 780

cgacggcgcc gccgcggtgg tggtgatgtc ggcggccaag gccaaggaac tgggcctgac 840

cccgctggcc acgatcaaga gctatgccaa cgccggtgtc gatcccaagg tgatgggcat 900

gggcccggtg ccggcctcca agcgcgccct gtcgcgcgcc gagtggaccc cgcaagacct 960

ggacctgatg gagatcaacg aggcctttgc cgcgcaggcg ctggcggtgc accagcagat 1020

gggctgggac acctccaagg tcaatgtgaa cggcggcgcc atcgccatcg gccacccgat 1080

cggcgcgtcg ggctgccgta tcctggtgac gctgctgcac gagatgaagc gccgtgacgc 1140

gaagaagggc ctggcctcgc tgtgcatcgg cggcggcatg ggcgtggcgc tggcagtcga 1200

gcgcaaataa ggaatttaaa aggagggatt aaaatgaact cgaagatcat ccgctttgag 1260

aacctccgct cgttcttcaa ggacggcatg acgattatga ttgggggctt tctgaactgc 1320

ggcaccccga ccaagctgat tgattttctg gtgaacctga acatcaagaa tctcaccatt 1380

atctccaacg atacctgcta cccgaacacc ggcatcggca agctcatctc gaacaaccag 1440

gtgaagaaac tgatcgcctc gtacattggc tcgaatccgg acacggggaa aaaactgttc 1500

aacaatgagc tggaagtgga actgtcgccg cagggcacgc tggtggagcg cattcgcgcg 1560

gggggctcgg gcctcggggg ggtcctgacc aagaccggcc tggggaccct catcgaaaaa 1620

ggcaaaaaga agatctccat caacggaacc gaatacctcc tggagctgcc gctgaccgcc 1680

gacgtggccc tgattaaagg ctcgattgtc gacgaagccg gcaacacctt ctacaagggc 1740

accaccaaga acttcaaccc gtacatggcg atggccgcca aaaccgtgat cgtggaagcc 1800

gaaaacctgg tctcctgcga gaagctggag aaggaaaagg ccatgacgcc gggcgtgctc 1860

atcaactaca tcgtgaagga gcccgcctaa aatgattaat gacaaaaacc tggccaagga 1920

gattattgcg aagcgcgtgg cccgcgaact gaaaaacggg cagctggtca atctgggcgt 1980

gggcctgccc acgatggtgg ccgactacat cccgaagaat ttcaagatca ccttccagtc 2040

ggaaaatggc attgtgggca tgggggccag cccgaaaatc aatgaggcgg acaaggacgt 2100

ggtgaatgcg gggggcgact ataccaccgt gctgccggac ggcacctttt tcgactcgtc 2160

ggtgtcgttc tcgctcattc ggggcgggca tgtggatgtc acggtgctgg gcgcgctgca 2220

ggtggacgag aagggcaata tcgccaactg gatcgtgccg ggcaagatgc tgtcggggat 2280

gggcggcgcc atggatctgg tgaacggggc caagaaggtc atcattgcca tgcgccacac 2340

caacaagggc caaccgaaga tcctgaagaa gtgcaccctg ccgctgaccg ccaaatcgca 2400

ggcgaacctg attgtgaccg aactgggggt gatcgaagtg attaacgacg gcctcctcct 2460

caccgaaatc aacaagaaca ccaccattga cgaaattcgc tcgctgacgg ccgccgacct 2520

gctcatcagc aatgagctgc gcccgatggc cgtgtagaaa gaaatactat gaaacaatat 2580

taaaaaaata agagttacca tttaaggtaa ctcttatttt tattacttaa gataataagc 2640

tt 2642

<210> 21
<211> 392
<212> PRT
<213> Clostridium acetobutylicum

<400> 21

Met Lys Glu Val Val Ile Ala Ser Ala Val Arg Thr Ala Ile Gly Ser
1               5                   10                  15

Tyr Gly Lys Ser Leu Lys Asp Val Pro Ala Val Asp Leu Gly Ala Thr
            20                  25                  30

Ala Ile Lys Glu Ala Val Lys Lys Ala Gly Ile Lys Pro Glu Asp Val
        35                  40                  45

Asn Glu Val Ile Leu Gly Asn Val Leu Gln Ala Gly Leu Gly Gln Asn
    50                  55                  60

Pro Ala Arg Gln Ala Ser Phe Lys Ala Gly Leu Pro Val Glu Ile Pro
65                  70                  75                  80

Ala Met Thr Ile Asn Lys Val Cys Gly Ser Gly Leu Arg Thr Val Ser
                85                  90                  95

Leu Ala Ala Gln Ile Ile Lys Ala Gly Asp Ala Asp Val Ile Ile Ala
            100                 105                 110

Gly Gly Met Glu Asn Met Ser Arg Ala Pro Tyr Leu Ala Asn Asn Ala
        115                 120                 125

Arg Trp Gly Tyr Arg Met Gly Asn Ala Lys Phe Val Asp Glu Met Ile
    130                 135                 140

Thr Asp Gly Leu Trp Asp Ala Phe Asn Asp Tyr His Met Gly Ile Thr
145                 150                 155                 160

Ala Glu Asn Ile Ala Glu Arg Trp Asn Ile Ser Arg Glu Glu Gln Asp
                165                 170                 175

Glu Phe Ala Leu Ala Ser Gln Lys Lys Ala Glu Glu Ala Ile Lys Ser
            180                 185                 190

Gly Gln Phe Lys Asp Glu Ile Val Pro Val Val Ile Lys Gly Arg Lys
        195                 200                 205

Gly Glu Thr Val Val Asp Thr Asp Glu His Pro Arg Phe Gly Ser Thr
    210                 215                 220

Ile Glu Gly Leu Ala Lys Leu Lys Pro Ala Phe Lys Lys Asp Gly Thr
225                 230                 235                 240

Val Thr Ala Gly Asn Ala Ser Gly Leu Asn Asp Cys Ala Ala Val Leu
                245                 250                 255

Val Ile Met Ser Ala Glu Lys Ala Lys Glu Leu Gly Val Lys Pro Leu
            260                 265                 270

Ala Lys Ile Val Ser Tyr Gly Ser Ala Gly Val Asp Pro Ala Ile Met
        275                 280                 285

Gly Tyr Gly Pro Phe Tyr Ala Thr Lys Ala Ala Ile Glu Lys Ala Gly
    290                 295                 300

Trp Thr Val Asp Glu Leu Asp Leu Ile Glu Ser Asn Glu Ala Phe Ala
305                 310                 315                 320

Ala Gln Ser Leu Ala Val Ala Lys Asp Leu Lys Phe Asp Met Asn Lys
                325                 330                 335

Val Asn Val Asn Gly Gly Ala Ile Ala Leu Gly His Pro Ile Gly Ala
            340                 345                 350

Ser Gly Ala Arg Ile Leu Val Thr Leu Val His Ala Met Gln Lys Arg
        355                 360                 365

Asp Ala Lys Lys Gly Leu Ala Thr Leu Cys Ile Gly Gly Gly Gln Gly

370                 375                 380

Thr Ala Ile Leu Leu Glu Lys Cys
385                 390

<210>  22
<211>  2634
<212>  DNA
<213>  Artificial Sequence

<220>
<223>  codonoptimized gen

<400>  22
ggtacctttc aggagattca agaatgaagg aggtggtgat tgcctcggcc gtgcgcaccg      60

cgatcggctc gtacggcaag agcctgaaag acgtgcccgc cgtggacctg ggcgccaccg     120

ccattaagga ggccgtgaag aaggccggca tcaagccgga ggacgtgaac gaggtgatcc     180

tgggcaacgt gctgcaggcc gggctcggcc agaacccggc ccgccaggcg agcttcaaag     240

ccggcctccc ggtggagatc cccgccatga ccatcaacaa ggtgtgcggc tcgggcctgc     300

gcaccgtgtc gctggcggcc cagatcatca aggccggcga cgccgacgtg atcattgccg     360

gcggcatgga aaacatgtcg cgcgccccgt acctggccaa caacgcccgc tggggctacc     420

gcatggggaa cgccaagttc gtggacgaga tgatcaccga cggcctgtgg gacgccttca     480

acgactacca catgggcatc accgccgaga acatcgccga gcgctggaac atctcgcgcg     540

aggagcagga cgagttcgcc ctggcctcgc aaaagaaggc ggaggaagcc atcaagtcgg     600

gccagttcaa ggacgagatc gtgccggtgg tgattaaggg ccgcaagggc gagaccgtgg     660

tggacacgga cgagcacccg cgcttcgggt cgacgatcga gggcctggcc aagctgaagc     720

cggccttcaa gaaggacggc accgtgaccg cgggcaacgc ctcgggcctg aacgactgcg     780

cggccgtgct ggtgatcatg tcggccgaaa aggccaagga gctgggcgtg aaaccgctgg     840

cgaagatcgt gtcgtatggc tcggccggcg tggacccggc catcatgggc tacggcccgt     900

tctacgccac caaggcggcg atcgagaaag ccggctggac ggtcgacgag ctggacctga     960

tcgagtcgaa cgaggccttt gccgcccagt cgctggccgt cgccaaggac ctgaagttcg    1020

acatgaacaa agtgaacgtg aatggcggcg ccatcgccct ggggcacccg atcggcgcct    1080

cgggcgcccg catcctggtg accctggtgc acgcgatgca gaagcgcgac gccaagaagg    1140

ggctggcgac cctgtgcatc ggcggcggcc agggcaccgc gatcctgctg gaaaagtgct    1200

agaaagttta aaaggaggga ttaaaatgaa ctcgaagatc atccgctttg agaacctccg    1260

ctcgttcttc aaggacggca tgacgattat gattgggggc tttctgaact gcggcacccc    1320

gaccaagctg attgattttc tggtgaacct gaacatcaag aatctcacca ttatctccaa    1380

cgatacctgc tacccgaaca ccggcatcgg caagctcatc tcgaacaacc aggtgaagaa    1440

actgatcgcc tcgtacattg gctcgaatcc ggacacgggg aaaaaactgt tcaacaatga 1500

gctggaagtg gaactgtcgc cgcagggcac gctggtggag cgcattcgcg cggggggctc 1560

gggcctcggg ggggtcctga ccaagaccgg cctggggacc ctcatcgaaa aaggcaaaaa 1620

gaagatctcc atcaacggga ccgaatacct cctggagctg ccgctgaccg ccgacgtggc 1680

cctgattaaa ggctcgattg tcgacgaagc cggcaacacc ttctacaagg gcaccaccaa 1740

gaacttcaac ccgtacatgg cgatggccgc caaaaccgtg atcgtggaag ccgaaaacct 1800

ggtctcctgc gagaagctgg agaaggaaaa ggccatgacg ccgggcgtgc tcatcaacta 1860

catcgtgaag gagcccgcct aaaatgatta atgacaaaaa cctggccaag gagattattg 1920

cgaagcgcgt ggcccgcgaa ctgaaaaacg ggcagctggt caatctgggc gtgggcctgc 1980

ccacgatggt ggccgactac atcccgaaga atttcaagat caccttccag tcggaaaatg 2040

gcattgtggg catgggggcc agcccgaaaa tcaatgaggc ggacaaggac gtggtgaatg 2100

cggggggcga ctataccacc gtgctgccgg acggcacctt tttcgactcg tcggtgtcgt 2160

tctcgctcat tcggggcggg catgtggatg tcacggtgct gggcgcgctg caggtggacg 2220

agaagggcaa tatcgccaac tggatcgtgc cgggcaagat gctgtcgggg atgggcggcg 2280

ccatggatct ggtgaacggg gccaagaagg tcatcattgc catgcgccac accaacaagg 2340

gccaaccgaa gatcctgaag aagtgcaccc tgccgctgac cgccaaatcg caggcgaacc 2400

tgattgtgac cgaactgggg gtgatcgaag tgattaacga cggcctcctc ctcaccgaaa 2460

tcaacaagaa caccaccatt gacgaaattc gctcgctgac ggccgccgac ctgctcatca 2520

gcaatgagct gcgcccgatg gccgtgtaga aagaaatact atgaaacaat attaaaaaaa 2580

taagagttac catttaaggt aactcttatt tttattactt aagataataa gctt 2634

<210> 23
<211> 136
<212> PRT
<213> Haemophilus influenzae

<400> 23

Met Leu Gly Asn Cys Phe Ser Phe Pro Val Arg Val Tyr Tyr Glu Asp
1               5                   10                  15

Thr Asp Ala Gly Gly Val Val Tyr His Ala Arg Tyr Leu His Phe Phe
            20                  25                  30

Glu Arg Ala Arg Thr Glu Tyr Leu Arg Ile Leu Asn Phe Thr Gln Gln
        35                  40                  45

Thr Leu Leu Glu Glu Gln Gln Leu Ala Phe Val Val Lys Ser Leu Ala
    50                  55                  60

Ile Asp Tyr Cys Val Ala Ala Lys Leu Asp Asp Leu Leu Met Val Glu
65                  70                  75                  80

Thr Glu Val Ser Glu Val Lys Gly Ala Thr Ile Leu Phe Glu Gln Arg
                85                  90                  95

Leu Met Arg Asn Thr Leu Met Leu Ser Lys Ala Thr Val Lys Val Ala
            100                 105                 110

Cys Val Asp Leu Gly Lys Met Lys Pro Val Ala Leu Pro Lys Glu Val
        115                 120                 125

Lys Ala Ala Phe Asn His Leu Lys
    130                 135

<210>  24
<211>  244
<212>  PRT
<213>  Clostridium acetobutylicum

<400>  24

Met Leu Lys Asp Glu Val Ile Lys Gln Ile Ser Thr Pro Leu Thr Ser
1               5                   10                  15

Pro Ala Phe Pro Arg Gly Pro Tyr Lys Phe His Asn Arg Glu Tyr Phe
            20                  25                  30

Asn Ile Val Tyr Arg Thr Asp Met Asp Ala Leu Arg Lys Val Val Pro
        35                  40                  45

Glu Pro Leu Glu Ile Asp Glu Pro Leu Val Arg Phe Glu Ile Met Ala
    50                  55                  60

Met His Asp Thr Ser Gly Leu Gly Cys Tyr Thr Glu Ser Gly Gln Ala
65                  70                  75                  80

Ile Pro Val Ser Phe Asn Gly Val Lys Gly Asp Tyr Leu His Met Met
                85                  90                  95

Tyr Leu Asp Asn Glu Pro Ala Ile Ala Val Gly Arg Glu Leu Ser Ala
            100                 105                 110

Tyr Pro Lys Lys Leu Gly Tyr Pro Lys Leu Phe Val Asp Ser Asp Thr
        115                 120                 125

Leu Val Gly Thr Leu Asp Tyr Gly Lys Leu Arg Val Ala Thr Ala Thr
    130                 135                 140

Met Gly Tyr Lys His Lys Ala Leu Asp Ala Asn Glu Ala Lys Asp Gln
145                 150                 155                 160

Ile Cys Arg Pro Asn Tyr Met Leu Lys Ile Ile Pro Asn Tyr Asp Gly
                165                 170                 175

Ser Pro Arg Ile Cys Glu Leu Ile Asn Ala Lys Ile Thr Asp Val Thr
            180                 185                 190

Val His Glu Ala Trp Thr Gly Pro Thr Arg Leu Gln Leu Phe Asp His
        195                 200                 205

Ala Met Ala Pro Leu Asn Asp Leu Pro Val Lys Glu Ile Val Ser Ser
    210                 215                 220

Ser His Ile Leu Ala Asp Ile Ile Leu Pro Arg Ala Glu Val Ile Tyr
225                 230                 235                 240

Asp Tyr Leu Lys

<210>  25
<211>  2612
<212>  DNA
<213>  Artificial Sequence

<220>
<223>  codonoptimized gen

<400>  25
ggtaccgtat gcaagaggga taaaaaatga agaccaagct catgacgctg caggacgcca      60

ccggcttttt ccgcgatggc atgaccatca tggtgggggg cttcatgggc atcgggacgc     120

cgtcgcggct ggtcgaagcc ctcctggagt cgggggtgcg cgatctgacg ctcattgcca     180

acgacaccgc cttcgtggac accggcattg gccccctgat tgtcaacggc cgcgtccgca     240

aagtgatcgc cagccacatc ggcacgaacc cggagaccgg ccgccggatg atctcgggcg     300

agatggatgt ggtgctggtc ccgcagggga ccctcattga gcaaatccgg tgcggcgggg     360

cgggcctcgg cgggttcctg acgccgaccg gcgtgggcac cgtggtcgag gaaggcaagc     420

agacgctgac cctggacggg aagacctggc tgctggaacg cccgctgcgc gccgatctgg     480

ccctgattcg cgcccatcgc tgcgacaccc tgggcaacct gacgtaccag ctgtccgccc     540

ggaacttcaa cccgctgatt gcgctggccg cggatatcac cctcgtggaa ccggacgaac     600

tcgtcgaaac cggggagctg caaccggatc atatcgtgac ccccggcgcg gtgatcgacc     660

atatcatcgt ctcccaagag tcgaagtaat ggacgccaag cagcgcatcg cccgccgcgt     720

cgcgcaggaa ctgcgcgacg gcgacatcgt gaacctgggc atcggcctgc cgaccatggt 780

ggccaactat ctgccggagg gcatccacat caccctgcag tcggagaacg gcttcctggg 840

cctgggcccg gtgaccaccg cccacccgga cctggtgaac gcggggggcc agccgtgcgg 900

ggtgctgccg ggcgccgcga tgttcgactc ggccatgtcg ttcgccctga tccggggggg 960

gcacatcgac gcctgcgtcc tcggcgggct ccaggtggac gaggaggcca acctggccaa 1020

ctgggtcgtg ccgggcaaaa tggtcccggg catggggggg gccatggacc tcgtgaccgg 1080

ctcgcgcaag gtgatcattg cgatggagca ctgcgccaag gatggctcgg ccaagatcct 1140

gcgccgctgc accatgccgc tgacggccca gcacgccgtg cacatgctgg tgaccgaact 1200

ggccgtgttc cgcttcatcg acggcaagat gtggctgacc gagatcgccg acggctgcga 1260

cctcgcgacc gtgcgcgcca agaccgaggc ccgcttcgag gtggccgccg acctgaacac 1320

ccagcgcggc gacctgtgac ggcaccccta caaacagaag gaatataaaa tgaagaactg 1380

cgtgatcgtg tcggccgtcc gcaccgcgat cggctcgttc aacggctcgc tcgcctcgac 1440

ctcggccatc gacctggggg ccaccgtgat caaggcggcc atcgagcgcg ccaagatcga 1500

ctcgcaacac gtggacgagg tgatcatggg caacgtgctg caagccgggc tgggccagaa 1560

cccggcccgg caggccctcc tgaagtcggg cctcgccgag accgtgtgcg gcttcaccgt 1620

gaacaaggtg tgcggctccg gcctgaagtc ggtcgccctc gccgcccaag ccatccaagc 1680

cgggcaagcc caatcgatcg tggccggcgg catggagaac atgtcgctgg ccccgtacct 1740

gctggacgcc aaggcccgct cgggctatcg gctgggggac ggccaggtgt acgacgtgat 1800

cctccgcgac ggcctcatgt gcgccaccca cggctaccac atgggcatca ccgccgagaa 1860

cgtggccaag gagtacggca tcacccgcga gatgcaggac gagctggccc tgcactcgca 1920

gcgcaaggcc gccgccgcca tcgagtcggg cgccttcacg gccgagatcg tgccggtgaa 1980

cgtggtgacc cgcaagaaga ccttcgtgtt ctcgcaggac gagttcccga aggccaactc 2040

gaccgccgag gccctggggg ccctgcgccc ggccttcgac aaagccggca ccgtgaccgc 2100

gggcaacgcc tcgggcatca acgacggcgc cgcggcgctg gtgatcatgg aggagtcggc 2160

cgcgctggcc gcggggctga ccccgctggc ccgcatcaag tcgtacgcct cgggcggggt 2220

gccgccggcc ctgatgggca tgggcccggt gccggccacc cagaaggccc tgcagctggc 2280

gggcctgcaa ctggcggaca tcgacctgat cgaggccaac gaggccttcg ccgcccagtt 2340

cctggcggtg ggcaagaacc tgggcttcga ctcggagaag gtgaatgtga acggcggcgc 2400

gatcgccctg ggccatccga tcggcgcctc gggcgcgcgc atcctggtga ccctgctgca 2460

cgccatgcag gcccgggata agacgctggg cctggccacc ctgtgcatcg gcgggggcca 2520

gggcatcgcc atggtgatcg aacgcctcaa ctaatcaata aaaacacccg atagcgaaag 2580

ttatcgggtg ttttcttgaa catcgaaagc tt 2612

<210>  26
<211>  70
<212>  DNA
<213>  Escherichia coli

<400>  26
ccccaggctt tacactttat gcttccggct cgtatgttgt gtggaattgt gagcggataa      60

caatttcaca                                                              70


<210>  27
<211>  882
<212>  DNA
<213>  Artificial Sequence

<220>
<223>  codonoptimized gen

<400>  27
aagcttcccc aggctttaca ctttatgctt ccggctcgta tgttgtgtgg aattgtgagc      60

ggataacaat ttcacatttc aggagattca agaatgctca aggacgaagt catcaaacaa     120

atcagcaccc cgctgacgtc ccccgccttt ccgcgggggc cctacaagtt ccacaaccgc     180

gagtacttca acatcgtgta ccgcaccgac atggatgccc tccgcaaggt cgtcccggaa     240

ccgctggaga tcgatgagcc cctggtgcgc ttcgagatta tggcgatgca tgacacctcc     300

ggcctgggct gctataccga aagcggccag gcgattccgg tctcgtttaa tggcgtgaag     360

ggggattacc tgcacatgat gtacctggac aacgaaccgg ccattgccgt gggccgcgag     420

ctgtccgcct accccaagaa gctgggctat ccgaagctgt ttgtggattc ggacacgctg     480

gtcggcaccc tcgactacgg caaactgcgc gtcgccaccg ccaccatggg ctacaagcac     540

aaggcgctgg acgcgaatga agcgaaggac cagatttgcc gccccaacta catgctgaaa     600

atcatcccca attacgacgg ctccccgcgc atttgcgaac tgatcaacgc gaagatcacc     660

gacgtgaccg tgcatgaagc ctggacgggc cccacgcgcc tgcagctctt cgaccatgcg     720

atggcgcccc tgaatgacct gccggtgaaa gaaatcgtgt cgtcctcgca tatcctcgcc     780

gatattatcc tgccccgggc ggaggtcatc tacgactacc tgaagtaata aaaataagag     840

ttaccttaaa tggtaactct tattttttta atattgggat cc                        882


<210>  28
<211>  7714
<212>  DNA
<213>  Artificial Sequence

<220>
<223>  vector

<400>  28
ctcgggccgt ctcttgggct tgatcggcct tcttgcgcat ctcacgcgct cctgcggcgg      60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaattttct ctggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag gggccacttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatcccccg ggctgcagga attcgatatc aagctttcga tgttcaagaa 3300

aacacccgat aactttcgct atcgggtgtt tttattgatt agttgaggcg ttcgatcacc 3360

atggcgatgc cctggccccc gccgatgcac agggtggcca ggcccagcgt cttatcccgg 3420

gcctgcatgg cgtgcagcag ggtcaccagg atgcgcgcgc ccgaggcgcc gatcggatgg 3480

cccagggcga tcgcgccgcc gttcacattc accttctccg agtcgaagcc caggttcttg 3540

cccaccgcca ggaactgggc ggcgaaggcc tcgttggcct cgatcaggtc gatgtccgcc 3600

agttgcaggc ccgccagctg cagggccttc tgggtggccg gcaccgggcc catgcccatc 3660

agggccggcg gcaccccgcc cgaggcgtac gacttgatgc gggccagcgg ggtcagcccc 3720

gcggccagcg cggccgactc ctccatgatc accagcgccg cggcgccgtc gttgatgccc 3780

gaggcgttgc ccgcggtcac ggtgccggct ttgtcgaagg ccgggcgcag ggcccccagg 3840

gcctcggcgg tcgagttggc cttcgggaac tcgtcctgcg agaacacgaa ggtcttcttg 3900

cgggtcacca cgttcaccgg cacgatctcg gccgtgaagg cgcccgactc gatggcggcg 3960

gcggccttgc gctgcgagtg cagggccagc tcgtcctgca tctcgcgggt gatgccgtac 4020

tccttggcca cgttctcggc ggtgatgccc atgtggtagc cgtgggtggc gcacatgagg 4080

ccgtcgcgga ggatcacgtc gtacacctgg ccgtccccca gccgatagcc cgagcgggcc 4140

ttggcgtcca gcaggtacgg ggccagcgac atgttctcca tgccgccggc cacgatcgat 4200

tgggcttgcc cggcttggat ggcttgggcg gcgagggcga ccgacttcag gccggagccg 4260

cacaccttgt tcacggtgaa gccgcacacg gtctcggcga ggcccgactt caggagggcc 4320

tgccgggccg ggttctggcc cagcccggct tgcagcacgt tgcccatgat cacctcgtcc 4380

acgtgttgcg agtcgatctt ggcgcgctcg atggccgcct tgatcacggt ggcccccagg 4440

tcgatggccg aggtcgaggc gagcgagccg ttgaacgagc cgatcgcggt gcggacggcc 4500

gacacgatca cgcagttctt cattttatat tccttctgtt tgtaggggtg ccgtcacagg 4560

tcgccgcgct gggtgttcag gtcggcggcc acctcgaagc gggcctcggt cttggcgcgc 4620

acggtcgcga ggtcgcagcc gtcggcgatc tcggtcagcc acatcttgcc gtcgatgaag 4680

cggaacacgg ccagttcggt caccagcatg tgcacggcgt gctgggccgt cagcggcatg 4740

gtgcagcggc gcaggatctt ggccgagcca tccttggcgc agtgctccat cgcaatgatc 4800

accttgcgcg agccggtcac gaggtccatg gcccccccca tgcccgggac cattttgccc 4860

ggcacgaccc agttggccag gttggcctcc tcgtccacct ggagcccgcc gaggacgcag 4920

gcgtcgatgt gccccccccg gatcagggcg aacgacatgg ccgagtcgaa catcgcggcg 4980

cccggcagca ccccgcacgg ctggcccccc gcgttcacca ggtccgggtg ggcggtggtc 5040

accgggccca ggcccaggaa gccgttctcc gactgcaggg tgatgtggat gccctccggc 5100

agatagttgg ccaccatggt cggcaggccg atgcccaggt tcacgatgtc gccgtcgcgc 5160

agttcctgcg cgacgcggcg ggcgatgcgc tgcttggcgt ccattacttc gactcttggg 5220

agacgatgat atggtcgatc accgcgccgg gggtcacgat atgatccggt tgcagctccc 5280

cggtttcgac gagttcgtcc ggttccacga gggtgatatc cgcggccagc gcaatcagcg 5340

ggttgaagtt ccgggcggac agctggtacg tcaggttgcc cagggtgtcg cagcgatggg 5400

cgcgaatcag ggccagatcg gcgcgcagcg ggcgttccag cagccaggtc ttcccgtcca 5460

gggtcagcgt ctgcttgcct tcctcgacca cggtgcccac gccggtcggc gtcaggaacc 5520

cgccgaggcc cgccccgccg caccggattt gctcaatgag ggtcccctgc gggaccagca 5580

ccacatccat ctcgcccgag atcatccggc ggccggtctc cgggttcgtg ccgatgtggc 5640

tggcgatcac tttgcggacg cggccgttga caatcagggg gccaatgccg gtgtccacga 5700

aggcggtgtc gttggcaatg agcgtcagat cgcgcacccc cgactccagg agggcttcga 5760

ccagccgcga cggcgtcccg atgcccatga agccccccac catgatggtc atgccatcgc 5820

ggaaaaagcc ggtggcgtcc tgcagcgtca tgagcttggt cttcattttt tatccctctt 5880

gcatacggta cccagctttt gttcccttta gtgagggtta attgcgcgct tggcgtaatc 5940

atggtcatag ctgtttcctg tgtgaaattg ttatccgctc acaattccac acaacatacg 6000

agccggaagc ataaagtgta aagcctgggg tgcctaatga gtgagctaac tcacattaat 6060

tgcgttgcgc tcactgcccg ctttccagtc gggaaacctg tcgtgccagc tgcattaatg 6120

aatcggccaa cgcgcgggga gaggcggttt gcgtattggg cgcatgcata aaaactgttg 6180

taattcatta agcattctgc cgacatggaa gccatcacaa acggcatgat gaacctgaat 6240

cgccagcggc atcagcacct tgtcgccttg cgtataatat ttgcccatgg gggtgggcga 6300

agaactccag catgagatcc ccgcgctgga ggatcatcca gccggcgtcc cggaaaacga 6360

ttccgaagcc caacctttca tagaaggcgg cggtggaatc gaaatctcgt gatggcaggt 6420

tgggcgtcgc ttggtcggtc atttcgaacc ccagagtccc gctcagaaga actcgtcaag 6480

aaggcgatag aaggcgatgc gctgcgaatc gggagcggcg ataccgtaaa gcacgaggaa 6540

gcggtcagcc cattcgccgc caagctcttc agcaatatca cgggtagcca acgctatgtc 6600

ctgatagcgg tccgccacac ccagccggcc acagtcgatg aatccagaaa agcggccatt 6660

ttccaccatg atattcggca agcaggcatc gccatgggtc acgacgagat cctcgccgtc 6720

gggcatgcgc gccttgagcc tggcgaacag ttcggctggc gcgagcccct gatgctcttc 6780

gtccagatca tcctgatcga caagaccggc ttccatccga gtacgtgctc gctcgatgcg 6840

atgtttcgct tggtggtcga atgggcaggt agccggatca agcgtatgca gccgccgcat 6900

tgcatcagcc atgatggata ctttctcggc aggagcaagg tgagatgaca ggagatcctg 6960

ccccggcact tcgcccaata gcagccagtc ccttcccgct tcagtgacaa cgtcgagcac 7020

agctgcgcaa ggaacgcccg tcgtggccag ccacgatagc cgcgctgcct cgtcctgcag 7080

ttcattcagg gcaccggaca ggtcggtctt gacaaaaaga accgggcgcc cctgcgctga 7140

cagccggaac acggcggcat cagagcagcc gattgtctgt tgtgcccagt catagccgaa 7200

tagcctctcc acccaagcgg ccggagaacc tgcgtgcaat ccatcttgtt caatcatgcg 7260

aaacgatcct catcctgtct cttgatcaga tcttgatccc ctgcgccatc agatccttgg 7320

cggcaagaaa gccatccagt ttactttgca gggcttccca accttaccag agggcgcccc 7380

agctggcaat tccggttcgc ttgctgtcca taaaaccgcc cagtctagct atcgccatgt 7440

aagcccactg caagctacct gctttctctt tgcgcttgcg ttttcccttg tccagatagc 7500

ccagtagctg acattcatcc caggtggcac ttttcgggga aatgtgcgcg cccgcgttcc 7560

tgctggcgct gggcctgttt ctggcgctgg acttcccgct gttccgtcag cagcttttcg 7620

cccacggcct tgatgatcgc ggcggccttg gcctgcatat cccgattcaa cggccccagg 7680

gcgtccagaa cgggcttcag gcgctcccga aggt 7714

<210> 29
<211> 7744
<212> DNA
<213> Artificial Sequence

<220>
<223> vector

<400> 29
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaattttct ctggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag gggccacttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatcccccg ggctgcagga attcgatatc aagcttatta tcttaagtaa 3300

taaaaataag agttacctta aatggtaact cttatttttt taatattgtt tcatagtatt 3360

tctttctaca cggccatcgg gcgcagctca ttgctgatga gcaggtcggc ggccgtcagc 3420

gagcgaattt cgtcaatggt ggtgttcttg ttgatttcgg tgaggaggag gccgtcgtta 3480

atcacttcga tcacccccag ttcggtcaca atcaggttcg cctgcgattt ggcggtcagc 3540

ggcagggtgc acttcttcag gatcttcggt tggcccttgt tggtgtggcg catggcaatg 3600

atgaccttct tggccccgtt caccagatcc atggcgccgc ccatccccga cagcatcttg 3660

cccggcacga tccagttggc gatattgccc ttctcgtcca cctgcagcgc gcccagcacc 3720

gtgacatcca catgcccgcc ccgaatgagc gagaacgaca ccgacgagtc gaaaaaggtg 3780

ccgtccggca gcacggtggt atagtcgccc cccgcattca ccacgtcctt gtccgcctca 3840

ttgattttcg ggctggcccc catgcccaca atgccatttt ccgactggaa ggtgatcttg 3900

aaattcttcg ggatgtagtc ggccaccatc gtgggcaggc ccacgcccag attgaccagc 3960

tgcccgtttt tcagttcgcg ggccacgcgc ttcgcaataa tctccttggc caggtttttg 4020

tcattaatca ttttaggcgg gctccttcac gatgtagttg atgagcacgc ccggcgtcat 4080

ggccttttcc ttctccagct tctcgcagga gaccaggttt tcggcttcca cgatcacggt 4140

tttggcggcc atcgccatgt acgggttgaa gttcttggtg gtgcccttgt agaaggtgtt 4200

gccggcttcg tcgacaatcg agcctttaat cagggccacg tcggcggtca gcggcagctc 4260

caggaggtat tcggtcccgt tgatggagat cttctttttg cctttttcga tgagggtccc 4320

caggccggtc ttggtcagga cccccccgag gcccgagccc cccgcgcgaa tgcgctccac 4380

cagcgtgccc tgcggcgaca gttccacttc cagctcattg ttgaacagtt ttttccccgt 4440

gtccggattc gagccaatgt acgaggcgat cagtttcttc acctggttgt tcgagatgag 4500

cttgccgatg ccggtgttcg ggtagcaggt atcgttggag ataatggtga gattcttgat 4560

gttcaggttc accagaaaat caatcagctt ggtcggggtg ccgcagttca gaaagccccc 4620

aatcataatc gtcatgccgt ccttgaagaa cgagcggagg ttctcaaagc ggatgatctt 4680

cgagttcatt ttaatccctc cttttaaatt ccttatttgc gctcgactgc cagcgccacg 4740

cccatgccgc cgccgatgca cagcgaggcc aggcccttct tcgcgtcacg gcgcttcatc 4800

tcgtgcagca gcgtcaccag gatacggcag cccgacgcgc cgatcgggtg gccgatggcg 4860

atggcgccgc cgttcacatt gaccttggag gtgtcccagc ccatctgctg gtgcaccgcc 4920

agcgcctgcg cggcaaaggc ctcgttgatc tccatcaggt ccaggtcttg cggggtccac 4980

tcggcgcgcg acagggcgcg cttggaggcc ggcaccgggc ccatgcccat caccttggga 5040

tcgacaccgg cgttggcata gctcttgatc gtggccagcg gggtcaggcc cagttccttg 5100

gccttggccg ccgacatcac caccaccgcg gcggcgccgt cgttcaggcc cgaggcgttg 5160

gccgcggtca ccgtgccggc cttgtcgaag gcgggcttga ggccggacat gctgtccagc 5220

gtggcgccct ggcgcacgaa ctcgtcggtc ttgaaggcca ccgggtcgcc cttgcgctgc 5280

gggatcagca ccgggacgat ctcttcgtca aacttgccgg ccttctgcgc ggcttcggcc 5340

ttgttctgcg agccgacggc gaactcatcc tgcgcctcgc gtgtgatgcc gtattccttg 5400

gccacgttct cggcggtgat gcccatgtgg tactggttgt acacgtccca caggccgtcg 5460

acgatcatgg tgtcgaccag cttggcatcg cccatgcgga aaccatcgcg cgagcccggc 5520

agcacgtgcg gggcggcgct catgttttcc tggccgccgg ccaccacgat ctcggcgtcg 5580

cccgccatga tcgcgttggc ggccagcatc acggccttca ggcccgagcc gcacaccttg 5640

ttgatggtca tggccggcac catcgccggc aggccggcct tgatcgcggc ctggcgtgcg 5700

gggttctggc ccgaaccggc ggtcagcacc tggcccatga tgacttcgct cacctgctcc 5760

ggcttgacgc cggcgcgctc cagcgcggcc ttgatgacca cggcacccag ttccggtgcc 5820

gggatcttgg ccagcgagcc gccaaacttg ccgaccgcgg tgcgggcggc ggatacgatg 5880

acaacgtcag tcattgtgta gtcctttcaa tggaaaggta cccagctttt gttcccttta 5940

gtgagggtta attgcgcgct tggcgtaatc atggtcatag ctgtttcctg tgtgaaattg 6000

ttatccgctc acaattccac acaacatacg agccggaagc ataaagtgta aagcctgggg 6060

tgcctaatga gtgagctaac tcacattaat tgcgttgcgc tcactgcccg ctttccagtc 6120

gggaaacctg tcgtgccagc tgcattaatg aatcggccaa cgcgcgggga gaggcggttt 6180

gcgtattggg cgcatgcata aaaactgttg taattcatta agcattctgc cgacatggaa 6240

gccatcacaa acggcatgat gaacctgaat cgccagcggc atcagcacct tgtcgccttg 6300

cgtataatat ttgcccatgg gggtgggcga agaactccag catgagatcc ccgcgctgga 6360

ggatcatcca gccggcgtcc cggaaaacga ttccgaagcc caacctttca tagaaggcgg 6420

cggtggaatc gaaatctcgt gatggcaggt tgggcgtcgc ttggtcggtc atttcgaacc 6480

ccagagtccc gctcagaaga actcgtcaag aaggcgatag aaggcgatgc gctgcgaatc 6540

gggagcggcg ataccgtaaa gcacgaggaa gcggtcagcc cattcgccgc caagctcttc 6600

agcaatatca cgggtagcca acgctatgtc ctgatagcgg tccgccacac ccagccggcc 6660

acagtcgatg aatccagaaa agcggccatt ttccaccatg atattcggca agcaggcatc 6720

gccatgggtc acgacgagat cctcgccgtc gggcatgcgc gccttgagcc tggcgaacag 6780

ttcggctggc gcgagcccct gatgctcttc gtccagatca tcctgatcga caagaccggc 6840

ttccatccga gtacgtgctc gctcgatgcg atgtttcgct tggtggtcga atgggcaggt 6900

agccggatca agcgtatgca gccgccgcat tgcatcagcc atgatggata ctttctcggc 6960

aggagcaagg tgagatgaca ggagatcctg ccccggcact tcgcccaata gcagccagtc 7020

ccttcccgct tcagtgacaa cgtcgagcac agctgcgcaa ggaacgcccg tcgtggccag 7080

ccacgatagc cgcgctgcct cgtcctgcag ttcattcagg gcaccggaca ggtcggtctt 7140

gacaaaaaga accgggcgcc cctgcgctga cagccggaac acggcggcat cagagcagcc 7200

gattgtctgt tgtgcccagt catagccgaa tagcctctcc acccaagcgg ccggagaacc 7260

tgcgtgcaat ccatcttgtt caatcatgcg aaacgatcct catcctgtct cttgatcaga 7320

tcttgatccc ctgcgccatc agatccttgg cggcaagaaa gccatccagt ttactttgca 7380

gggcttccca accttaccag agggcgcccc agctggcaat tccggttcgc ttgctgtcca 7440

taaaaccgcc cagtctagct atcgccatgt aagcccactg caagctacct gctttctctt 7500

tgcgcttgcg ttttcccttg tccagatagc ccagtagctg acattcatcc caggtggcac 7560

ttttcgggga aatgtgcgcg cccgcgttcc tgctggcgct gggcctgttt ctggcgctgg 7620

acttcccgct gttccgtcag cagcttttcg cccacggcct tgatgatcgc ggcggccttg 7680

gcctgcatat cccgattcaa cggccccagg gcgtccagaa cgggcttcag gcgctcccga 7740

aggt 7744

<210> 30
<211> 7736
<212> DNA
<213> Artificial Sequence

<220>
<223> vector

<400> 30
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960
ttcgccctgc gcggtcgctg cgctcccttg ccagcccgtg gatatgtgga cgatggccgc 1020
gagcggccac cggctggctc gcttcgctcg gcccgtggac aaccctgctg gacaagctga 1080
tggacaggct gcgcctgccc acgagcttga ccacagggat tgcccaccgg ctacccagcc 1140
ttcgaccaca tacccaccgg ctccaactgc gcggcctgcg gccttgcccc atcaattttt 1200
ttaattttct ctggggaaaa gcctccggcc tgcggcctgc gcgcttcgct tgccggttgg 1260
acaccaagtg gaaggcgggt caaggctcgc gcagcgaccg cgcagcggct tggccttgac 1320
gcgcctggaa cgacccaagc ctatgcgagt gggggcagtc gaaggcgaag cccgcccgcc 1380
tgccccccga gcctcacggc ggcgagtgcg ggggttccaa gggggcagcg ccaccttggg 1440
caaggccgaa ggccgcgcag tcgatcaaca agccccggag gggccacttt ttgccggagg 1500
gggagccgcg ccgaaggcgt gggggaaccc cgcaggggtg cccttctttg ggcaccaaag 1560
aactagatat agggcgaaat gcgaaagact taaaaatcaa caacttaaaa aaggggggta 1620
cgcaacagct cattgcggca ccccccgcaa tagctcattg cgtaggttaa agaaaatctg 1680
taattgactg ccacttttac gcaacgcata attgttgtcg cgctgccgaa aagttgcagc 1740
tgattgcgca tggtgccgca accgtgcggc accctaccgc atggagataa gcatggccac 1800
gcagtccaga gaaatcggca ttcaagccaa gaacaagccc ggtcactggg tgcaaacgga 1860
acgcaaagcg catgaggcgt gggccgggct tattgcgagg aaacccacgg cggcaatgct 1920
gctgcatcac ctcgtggcgc agatgggcca ccagaacgcc gtggtggtca gccagaagac 1980
actttccaag ctcatcggac gttctttgcg gacggtccaa tacgcagtca aggacttggt 2040
ggccgagcgc tggatctccg tcgtgaagct caacggcccc ggcaccgtgt cggcctacgt 2100
ggtcaatgac cgcgtggcgt ggggccagcc ccgcgaccag ttgcgcctgt cggtgttcag 2160
tgccgccgtg gtggttgatc acgacgacca ggacgaatcg ctgttggggc atggcgacct 2220
gcgccgcatc ccgaccctgt atccgggcga gcagcaacta ccgaccggcc ccggcgagga 2280
gccgcccagc cagcccggca ttccgggcat ggaaccagac ctgccagcct tgaccgaaac 2340
ggaggaatgg gaacggcgcg ggcagcagcg cctgccgatg cccgatgagc cgtgttttct 2400
ggacgatggc gagccgttgg agccgccgac acgggtcacg ctgccgcgcc ggtagcactt 2460
gggttgcgca gcaacccgta agtgcgctgt tccagactat cggctgtagc cgcctcgccg 2520
ccctatacct tgtctgcctc cccgcgttgc gtcgcggtgc atggagccgg gccacctcga 2580
cctgaatgga agccggcggc acctcgctaa cggattcacc gtttttatca ggctctggga 2640
ggcagaataa atgatcatat cgtcaattat tacctccacg gggagagcct gagcaaactg 2700
gcctcaggca tttgagaagc acacggtcac actgcttccg gtagtcaata aaccggtaaa 2760
ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatcccccg ggctgcagga attcgatatc aagcttatta tcttaagtaa 3300

taaaaataag agttacctta aatggtaact cttatttttt taatattgtt tcatagtatt 3360

tctttctaca cggccatcgg gcgcagctca ttgctgatga gcaggtcggc ggccgtcagc 3420

gagcgaattt cgtcaatggt ggtgttcttg ttgatttcgg tgaggaggag gccgtcgtta 3480

atcacttcga tcacccccag ttcggtcaca atcaggttcg cctgcgattt ggcggtcagc 3540

ggcagggtgc acttcttcag gatcttcggt tggcccttgt tggtgtggcg catggcaatg 3600

atgaccttct tggccccgtt caccagatcc atggcgccgc ccatccccga cagcatcttg 3660

cccggcacga tccagttggc gatattgccc ttctcgtcca cctgcagcgc gcccagcacc 3720

gtgacatcca catgcccgcc ccgaatgagc gagaacgaca ccgacgagtc gaaaaaggtg 3780

ccgtccggca gcacggtggt atagtcgccc cccgcattca ccacgtcctt gtccgcctca 3840

ttgattttcg ggctggcccc catgcccaca atgccatttt ccgactggaa ggtgatcttg 3900

aaattcttcg ggatgtagtc ggccaccatc gtgggcaggc ccacgcccag attgaccagc 3960

tgcccgtttt tcagttcgcg ggccacgcgc ttcgcaataa tctccttggc caggtttttg 4020

tcattaatca ttttaggcgg gctccttcac gatgtagttg atgagcacgc ccggcgtcat 4080

ggccttttcc ttctccagct tctcgcagga gaccaggttt tcggcttcca cgatcacggt 4140

tttggcggcc atcgccatgt acgggttgaa gttcttggtg gtgcccttgt agaaggtgtt 4200

gccggcttcg tcgacaatcg agcctttaat cagggccacg tcggcggtca gcggcagctc 4260

caggaggtat tcggtcccgt tgatggagat cttctttttg cctttttcga tgagggtccc 4320

caggccggtc ttggtcagga cccccccgag gcccgagccc cccgcgcgaa tgcgctccac 4380

cagcgtgccc tgcggcgaca gttccacttc cagctcattg ttgaacagtt ttttccccgt 4440

gtccggattc gagccaatgt acgaggcgat cagtttcttc acctggttgt tcgagatgag 4500

cttgccgatg ccggtgttcg ggtagcaggt atcgttggag ataatggtga gattcttgat 4560

gttcaggttc accagaaaat caatcagctt ggtcggggtg ccgcagttca gaaagccccc 4620

aatcataatc gtcatgccgt ccttgaagaa cgagcggagg ttctcaaagc ggatgatctt 4680

cgagttcatt ttaatccctc cttttaaact ttctagcact tttccagcag gatcgcggtg 4740
ccctggccgc cgccgatgca cagggtcgcc agccccttct tggcgtcgcg cttctgcatc 4800
gcgtgcacca gggtcaccag gatgcgggcg cccgaggcgc cgatcgggtg ccccagggcg 4860
atggcgccgc cattcacgtt cactttgttc atgtcgaact tcaggtcctt ggcgacggcc 4920
agcgactggg cggcaaaggc ctcgttcgac tcgatcaggt ccagctcgtc gaccgtccag 4980
ccggctttct cgatcgccgc cttggtggcg tagaacgggc cgtagcccat gatggccggg 5040
tccacgccgg ccgagccata cgacacgatc ttcgccagcg gtttcacgcc cagctccttg 5100
gccttttcgg ccgacatgat caccagcacg gccgcgcagt cgttcaggcc cgaggcgttg 5160
cccgcggtca cggtgccgtc cttcttgaag gccggcttca gcttggccag gccctcgatc 5220
gtcgacccga agcgcgggtg ctcgtccgtg tccaccacgg tctcgccctt gcggccctta 5280
atcaccaccg gcacgatctc gtccttgaac tggcccgact tgatggcttc ctccgccttc 5340
ttttgcgagg ccagggcgaa ctcgtcctgc tcctcgcgcg agatgttcca gcgctcggcg 5400
atgttctcgg cggtgatgcc catgtggtag tcgttgaagg cgtcccacag gccgtcggtg 5460
atcatctcgt ccacgaactt ggcgttcccc atgcggtagc cccagcgggc gttgttggcc 5520
aggtacgggg cgcgcgacat gttttccatg ccgccggcaa tgatcacgtc ggcgtcgccg 5580
gccttgatga tctgggccgc cagcgacacg gtgcgcaggc ccgagccgca caccttgttg 5640
atggtcatgg cggggatctc caccgggagg ccggctttga agctcgcctg gcgggccggg 5700
ttctggccga gcccggcctg cagcacgttg cccaggatca cctcgttcac gtcctccggc 5760
ttgatgccgg ccttcttcac ggcctcctta atggcggtgg cgcccaggtc cacggcgggc 5820
acgtctttca ggctcttgcc gtacgagccg atcgcggtgc gcacggccga ggcaatcacc 5880
acctccttca ttcttgaatc tcctgaaagg tacccagctt ttgttccctt tagtgagggt 5940
taattgcgcg cttggcgtaa tcatggtcat agctgtttcc tgtgtgaaat tgttatccgc 6000
tcacaattcc acacaacata cgagccggaa gcataaagtg taaagcctgg ggtgcctaat 6060
gagtgagcta actcacatta attgcgttgc gctcactgcc cgctttccag tcgggaaacc 6120
tgtcgtgcca gctgcattaa tgaatcggcc aacgcgcggg gagaggcggt ttgcgtattg 6180
ggcgcatgca taaaaactgt tgtaattcat taagcattct gccgacatgg aagccatcac 6240
aaacggcatg atgaacctga atcgccagcg gcatcagcac cttgtcgcct tgcgtataat 6300
atttgcccat gggggtgggc gaagaactcc agcatgagat ccccgcgctg gaggatcatc 6360
cagccggcgt cccggaaaac gattccgaag cccaaccttt catagaaggc ggcggtggaa 6420
tcgaaatctc gtgatggcag gttgggcgtc gcttggtcgg tcatttcgaa ccccagagtc 6480
ccgctcagaa gaactcgtca agaaggcgat agaaggcgat gcgctgcgaa tcgggagcgg 6540
cgataccgta aagcacgagg aagcggtcag cccattcgcc gccaagctct tcagcaatat 6600

cacgggtagc caacgctatg tcctgatagc ggtccgccac acccagccgg ccacagtcga 6660

tgaatccaga aaagcggcca ttttccacca tgatattcgg caagcaggca tcgccatggg 6720

tcacgacgag atcctcgccg tcgggcatgc gcgccttgag cctggcgaac agttcggctg 6780

gcgcgagccc ctgatgctct tcgtccagat catcctgatc gacaagaccg gcttccatcc 6840

gagtacgtgc tcgctcgatg cgatgtttcg cttggtggtc gaatgggcag gtagccggat 6900

caagcgtatg cagccgccgc attgcatcag ccatgatgga tactttctcg gcaggagcaa 6960

ggtgagatga caggagatcc tgccccggca cttcgcccaa tagcagccag tcccttcccg 7020

cttcagtgac aacgtcgagc acagctgcgc aaggaacgcc cgtcgtggcc agccacgata 7080

gccgcgctgc ctcgtcctgc agttcattca gggcaccgga caggtcggtc ttgacaaaaa 7140

gaaccgggcg cccctgcgct gacagccgga acacggcggc atcagagcag ccgattgtct 7200

gttgtgccca gtcatagccg aatagcctct ccacccaagc ggccggagaa cctgcgtgca 7260

atccatcttg ttcaatcatg cgaaacgatc ctcatcctgt ctcttgatca gatcttgatc 7320

ccctgcgcca tcagatcctt ggcggcaaga aagccatcca gtttactttg cagggcttcc 7380

caaccttacc agagggcgcc ccagctggca attccggttc gcttgctgtc cataaaaccg 7440

cccagtctag ctatcgccat gtaagcccac tgcaagctac ctgctttctc tttgcgcttg 7500

cgttttccct tgtccagata gcccagtagc tgacattcat cccaggtggc acttttcggg 7560

gaaatgtgcg cgcccgcgtt cctgctggcg ctgggcctgt ttctggcgct ggacttcccg 7620

ctgttccgtc agcagctttt cgcccacggc cttgatgatc gcggcggcct tggcctgcat 7680

atcccgattc aacggcccca gggcgtccag aacgggcttc aggcgctccc gaaggt 7736

<210> 31
<211> 8560
<212> DNA
<213> Artificial Sequence

<220>
<223> vector

<400> 31
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480
acttgtccgc cagccacttg tgcgccttct cgaagaacgc cgcctgctgt tcttggctgg 540
ccgacttcca ccattccggg ctggccgtca tgacgtactc gaccgccaac acagcgtcct 600
tgcgccgctt ctctggcagc aactcgcgca gtcggcccat cgcttcatcg gtgctgctgg 660
ccgcccagtg ctcgttctct ggcgtcctgc tggcgtcagc gttgggcgtc tcgcgctcgc 720
ggtaggcgtg cttgagactg gccgccacgt tgcccatttt cgccagcttc ttgcatcgca 780
tgatcgcgta tgccgccatg cctgcccctc ccttttggtg tccaaccggc tcgacggggg 840
cagcgcaagg cggtgcctcc ggcgggccac tcaatgcttg agtatactca ctagactttg 900
cttcgcaaag tcgtgaccgc ctacggcggc tgcggcgccc tacgggcttg ctctccgggc 960
ttcgccctgc gcggtcgctg cgctcccttg ccagcccgtg gatatgtgga cgatggccgc 1020
gagcggccac cggctggctc gcttcgctcg gcccgtggac aaccctgctg gacaagctga 1080
tggacaggct gcgcctgccc acgagcttga ccacagggat tgcccaccgg ctacccagcc 1140
ttcgaccaca tacccaccgg ctccaactgc gcggcctgcg gccttgcccc atcaattttt 1200
ttaattttct ctggggaaaa gcctccggcc tgcggcctgc gcgcttcgct tgccggttgg 1260
acaccaagtg gaaggcgggt caaggctcgc gcagcgaccg cgcagcggct tggccttgac 1320
gcgcctggaa cgacccaagc ctatgcgagt gggggcagtc gaaggcgaag cccgcccgcc 1380
tgccccccga gcctcacggc ggcgagtgcg ggggttccaa gggggcagcg ccaccttggg 1440
caaggccgaa ggccgcgcag tcgatcaaca agccccggag gggccacttt ttgccggagg 1500
gggagccgcg ccgaaggcgt gggggaaccc cgcaggggtg cccttctttg ggcaccaaag 1560
aactagatat agggcgaaat gcgaaagact taaaaatcaa caacttaaaa aaggggggta 1620
cgcaacagct cattgcggca ccccccgcaa tagctcattg cgtaggttaa agaaaatctg 1680
taattgactg ccacttttac gcaacgcata attgttgtcg cgctgccgaa aagttgcagc 1740
tgattgcgca tggtgccgca accgtgcggc accctaccgc atggagataa gcatggccac 1800
gcagtccaga gaaatcggca ttcaagccaa gaacaagccc ggtcactggg tgcaaacgga 1860
acgcaaagcg catgaggcgt gggccgggct tattgcgagg aaacccacgg cggcaatgct 1920
gctgcatcac ctcgtggcgc agatgggcca ccagaacgcc gtggtggtca gccagaagac 1980
actttccaag ctcatcggac gttctttgcg gacggtccaa tacgcagtca aggacttggt 2040
ggccgagcgc tggatctccg tcgtgaagct caacggcccc ggcaccgtgt cggcctacgt 2100
ggtcaatgac cgcgtggcgt ggggccagcc ccgcgaccag ttgcgcctgt cggtgttcag 2160
tgccgccgtg gtggttgatc acgacgacca ggacgaatcg ctgttggggc atggcgacct 2220
gcgccgcatc ccgaccctgt atccgggcga gcagcaacta ccgaccggcc ccggcgagga 2280
gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400
ggacgatggc gagccgttgg agccgccgac acgggtcacg ctgccgcgcc ggtagcactt 2460
gggttgcgca gcaacccgta agtgcgctgt tccagactat cggctgtagc cgcctcgccg 2520
ccctatacct tgtctgcctc cccgcgttgc gtcgcggtgc atggagccgg gccacctcga 2580
cctgaatgga agccggcggc acctcgctaa cggattcacc gtttttatca ggctctggga 2640
ggcagaataa atgatcatat cgtcaattat tacctccacg gggagagcct gagcaaactg 2700
gcctcaggca tttgagaagc acacggtcac actgcttccg gtagtcaata aaccggtaaa 2760
ccagcaatag acataagcgg ctatttaacg accctgccct gaaccgacga ccgggtcgaa 2820
tttgctttcg aatttctgcc attcatccgc ttattatcac ttattcaggc gtagcaccag 2880
gcgtttaagg gcaccaataa ctgccttaaa aaaattacgc cccgccctgc cactcatcgc 2940
agtcggccta ttggttaaaa aatgagctga tttaacaaaa atttaacgcg aattttaaca 3000
aaatattaac gcttacaatt tccattcgcc attcaggctg cgcaactgtt gggaagggcg 3060
atcggtgcgg gcctcttcgc tattacgcca gctggcgaaa gggggatgtg ctgcaaggcg 3120
attaagttgg gtaacgccag ggttttccca gtcacgacgt tgtaaaacga cggccagtga 3180
gcgcgcgtaa tacgactcac tatagggcga attggagctc caccgcggtg gcggccgctc 3240
tagaactagt ggatcccaat attaaaaaaa taagagttac catttaaggt aactcttatt 3300
tttattactt caggtagtcg tagatgacct ccgcccgggg caggataata tcggcgagga 3360
tatgcgagga cgacacgatt tctttcaccg gcaggtcatt caggggcgcc atcgcatggt 3420
cgaagagctg caggcgcgtg gggcccgtcc aggcttcatg cacggtcacg tcggtgatct 3480
tcgcgttgat cagttcgcaa atgcgcgggg agccgtcgta attggggatg attttcagca 3540
tgtagttggg gcggcaaatc tggtccttcg cttcattcgc gtccagcgcc ttgtgcttgt 3600
agcccatggt ggcggtggcg acgcgcagtt tgccgtagtc gagggtgccg accagcgtgt 3660
ccgaatccac aaacagcttc ggatagccca gcttcttggg gtaggcggac agctcgcggc 3720
ccacggcaat ggccggttcg ttgtccaggt acatcatgtg caggtaatcc cccttcacgc 3780
cattaaacga gaccggaatc gcctggccgc tttcggtata gcagcccagg ccggaggtgt 3840
catgcatcgc cataatctcg aagcgcacca ggggctcatc gatctccagc ggttccggga 3900
cgaccttgcg gagggcatcc atgtcggtgc ggtacacgat gttgaagtac tcgcggttgt 3960
ggaacttgta gggcccccgc ggaaaggcgg gggacgtcag cggggtgctg atttgtttga 4020
tgacttcgtc cttgagcatt cttgaatctc ctgaaatgtg aaattgttat ccgctcacaa 4080
ttccacacaa catacgagcc ggaagcataa agtgtaaagc ctggggaagc tttcgatgtt 4140
caagaaaaca cccgataact ttcgctatcg ggtgttttta ttgattagtt gaggcgttcg 4200

atcaccatgg cgatgccctg gcccccgccg atgcacaggg tggccaggcc cagcgtctta 4260

tcccgggcct gcatggcgtg cagcagggtc accaggatgc gcgcgcccga ggcgccgatc 4320

ggatggccca gggcgatcgc gccgccgttc acattcacct tctccgagtc gaagcccagg 4380

ttcttgccca ccgccaggaa ctgggcggcg aaggcctcgt tggcctcgat caggtcgatg 4440

tccgccagtt gcaggcccgc cagctgcagg gccttctggg tggccggcac cgggcccatg 4500

cccatcaggg ccggcggcac cccgcccgag gcgtacgact tgatgcgggc cagcggggtc 4560

agccccgcgg ccagcgcggc cgactcctcc atgatcacca gcgccgcggc gccgtcgttg 4620

atgcccgagg cgttgcccgc ggtcacggtg ccggctttgt cgaaggccgg gcgcagggcc 4680

cccagggcct cggcggtcga gttggccttc gggaactcgt cctgcgagaa cacgaaggtc 4740

ttcttgcggg tcaccacgtt caccggcacg atctcggccg tgaaggcgcc cgactcgatg 4800

gcggcggcgg ccttgcgctg cgagtgcagg gccagctcgt cctgcatctc gcgggtgatg 4860

ccgtactcct tggccacgtt ctcggcggtg atgcccatgt ggtagccgtg ggtggcgcac 4920

atgaggccgt cgcggaggat cacgtcgtac acctggccgt cccccagccg atagcccgag 4980

cgggccttgg cgtccagcag gtacggggcc agcgacatgt tctccatgcc gccggccacg 5040

atcgattggg cttgcccggc ttggatggct tgggcggcga gggcgaccga cttcaggccg 5100

gagccgcaca ccttgttcac ggtgaagccg cacacggtct cggcgaggcc cgacttcagg 5160

agggcctgcc gggccgggtt ctggcccagc ccggcttgca gcacgttgcc catgatcacc 5220

tcgtccacgt gttgcgagtc gatcttggcg cgctcgatgg ccgccttgat cacggtggcc 5280

cccaggtcga tggccgaggt cgaggcgagc gagccgttga acgagccgat cgcggtgcgg 5340

acggccgaca cgatcacgca gttcttcatt ttatattcct tctgtttgta ggggtgccgt 5400

cacaggtcgc cgcgctgggt gttcaggtcg gcggccacct cgaagcgggc ctcggtcttg 5460

gcgcgcacgg tcgcgaggtc gcagccgtcg gcgatctcgg tcagccacat cttgccgtcg 5520

atgaagcgga acacggccag ttcggtcacc agcatgtgca cggcgtgctg ggccgtcagc 5580

ggcatggtgc agcggcgcag gatcttggcc gagccatcct tggcgcagtg ctccatcgca 5640

atgatcacct tgcgcgagcc ggtcacgagg tccatggccc cccccatgcc cgggaccatt 5700

ttgcccggca cgacccagtt ggccaggttg gcctcctcgt ccacctggag cccgccgagg 5760

acgcaggcgt cgatgtgccc cccccggatc agggcgaacg acatggccga gtcgaacatc 5820

gcggcgcccg gcagcacccc gcacggctgg ccccccgcgt tcaccaggtc cgggtgggcg 5880

gtggtcaccg ggcccaggcc caggaagccg ttctccgact gcagggtgat gtggatgccc 5940

tccggcagat agttggccac catggtcggc aggccgatgc ccaggttcac gatgtcgccg 6000

tcgcgcagtt cctgcgcgac gcggcgggcg atgcgctgct tggcgtccat tacttcgact 6060

cttgggagac gatgatatgg tcgatcaccg cgccgggggt cacgatatga tccggttgca 6120

gctccccggt ttcgacgagt tcgtccggtt ccacgagggt gatatccgcg gccagcgcaa 6180

tcagcgggtt gaagttccgg gcggacagct ggtacgtcag gttgcccagg gtgtcgcagc 6240

gatgggcgcg aatcagggcc agatcggcgc gcagcgggcg ttccagcagc caggtcttcc 6300

cgtccagggt cagcgtctgc ttgccttcct cgaccacggt gcccacgccg gtcggcgtca 6360

ggaacccgcc gaggcccgcc ccgccgcacc ggatttgctc aatgagggtc ccctgcggga 6420

ccagcaccac atccatctcg cccgagatca tccggcggcc ggtctccggg ttcgtgccga 6480

tgtggctggc gatcactttg cggacgcggc cgttgacaat cagggggcca atgccggtgt 6540

ccacgaaggc ggtgtcgttg gcaatgagcg tcagatcgcg cacccccgac tccaggaggg 6600

cttcgaccag ccgcgacggc gtcccgatgc ccatgaagcc ccccaccatg atggtcatgc 6660

catcgcggaa aaagccggtg gcgtcctgca gcgtcatgag cttggtcttc attttttatc 6720

cctcttgcat acggtaccca gcttttgttc cctttagtga gggttaattg cgcgcttggc 6780

gtaatcatgg tcatagctgt ttcctgtgtg aaattgttat ccgctcacaa ttccacacaa 6840

catacgagcc ggaagcataa agtgtaaagc ctggggtgcc taatgagtga gctaactcac 6900

attaattgcg ttgcgctcac tgcccgcttt ccagtcggga aacctgtcgt gccagctgca 6960

ttaatgaatc ggccaacgcg cggggagagg cggtttgcgt attgggcgca tgcataaaaa 7020

ctgttgtaat tcattaagca ttctgccgac atggaagcca tcacaaacgg catgatgaac 7080

ctgaatcgcc agcggcatca gcaccttgtc gccttgcgta taatatttgc ccatgggggt 7140

gggcgaagaa ctccagcatg agatccccgc gctggaggat catccagccg gcgtcccgga 7200

aaacgattcc gaagcccaac ctttcataga aggcggcggt ggaatcgaaa tctcgtgatg 7260

gcaggttggg cgtcgcttgg tcggtcattt cgaaccccag agtcccgctc agaagaactc 7320

gtcaagaagg cgatagaagg cgatgcgctg cgaatcggga gcggcgatac cgtaaagcac 7380

gaggaagcgg tcagcccatt cgccgccaag ctcttcagca atatcacggg tagccaacgc 7440

tatgtcctga tagcggtccg ccacacccag ccggccacag tcgatgaatc cagaaaagcg 7500

gccattttcc accatgatat tcggcaagca ggcatcgcca tgggtcacga cgagatcctc 7560

gccgtcgggc atgcgcgcct tgagcctggc gaacagttcg gctggcgcga gcccctgatg 7620

ctcttcgtcc agatcatcct gatcgacaag accggcttcc atccgagtac gtgctcgctc 7680

gatgcgatgt ttcgcttggt ggtcgaatgg gcaggtagcc ggatcaagcg tatgcagccg 7740

ccgcattgca tcagccatga tggatacttt ctcggcagga gcaaggtgag atgacaggag 7800

atcctgcccc ggcacttcgc ccaatagcag ccagtccctt cccgcttcag tgacaacgtc 7860

gagcacagct gcgcaaggaa cgcccgtcgt ggccagccac gatagccgcg ctgcctcgtc 7920

ctgcagttca ttcagggcac cggacaggtc ggtcttgaca aaaagaaccg ggcgcccctg 7980

cgctgacagc cggaacacgg cggcatcaga gcagccgatt gtctgttgtg cccagtcata 8040

gccgaatagc ctctccaccc aagcggccgg agaacctgcg tgcaatccat cttgttcaat 8100

catgcgaaac gatcctcatc ctgtctcttg atcagatctt gatcccctgc gccatcagat 8160

ccttggcggc aagaaagcca tccagtttac tttgcagggc ttcccaacct taccagaggg 8220

cgccccagct ggcaattccg gttcgcttgc tgtccataaa accgcccagt ctagctatcg 8280

ccatgtaagc ccactgcaag ctacctgctt tctctttgcg cttgcgtttt cccttgtcca 8340

gatagcccag tagctgacat tcatccaggg tggcactttt cggggaaatg tgcgcgcccg 8400

cgttcctgct ggcgctgggc ctgtttctgg cgctggactt cccgctgttc cgtcagcagc 8460

ttttcgccca cggccttgat gatcgcggcg gccttggcct gcatatcccg attcaacggc 8520

cccagggcgt ccagaacggg cttcaggcgc tcccgaaggt 8560

<210> 32
<211> 8590
<212> DNA
<213> Artificial Sequence

<220>
<223> vector

<400> 32
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaattttct ctggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag gggccacttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatcccaat attaaaaaaa taagagttac catttaaggt aactcttatt 3300

tttattactt caggtagtcg tagatgacct ccgcccgggg caggataata tcggcgagga 3360

tatgcgagga cgacacgatt tctttcaccg gcaggtcatt caggggcgcc atcgcatggt 3420

cgaagagctg caggcgcgtg gggcccgtcc aggcttcatg cacggtcacg tcggtgatct 3480

tcgcgttgat cagttcgcaa atgcgcgggg agccgtcgta attggggatg attttcagca 3540

tgtagttggg gcggcaaatc tggtccttcg cttcattcgc gtccagcgcc ttgtgcttgt 3600

agcccatggt ggcggtggcg acgcgcagtt tgccgtagtc gagggtgccg accagcgtgt 3660

ccgaatccac aaacagcttc ggatagccca gcttcttggg gtaggcggac agctcgcggc 3720

ccacggcaat ggccggttcg ttgtccaggt acatcatgtg caggtaatcc cccttcacgc 3780

cattaaacga gaccggaatc gcctggccgc tttcggtata gcagcccagg ccggaggtgt 3840

catgcatcgc cataatctcg aagcgcacca ggggctcatc gatctccagc ggttccggga 3900

cgaccttgcg gagggcatcc atgtcggtgc ggtacacgat gttgaagtac tcgcggttgt 3960

ggaacttgta gggcccccgc ggaaaggcgg gggacgtcag cggggtgctg atttgtttga 4020

tgacttcgtc cttgagcatt cttgaatctc ctgaaatgtg aaattgttat ccgctcacaa 4080

ttccacacaa catacgagcc ggaagcataa agtgtaaagc ctggggaagc ttattatctt 4140

aagtaataaa aataagagtt accttaaatg gtaactctta tttttttaat attgtttcat 4200

agtatttctt tctacacggc catcgggcgc agctcattgc tgatgagcag gtcggcggcc 4260

gtcagcgagc gaatttcgtc aatggtggtg ttcttgttga tttcggtgag gaggaggccg 4320

tcgttaatca cttcgatcac ccccagttcg gtcacaatca ggttcgcctg cgatttggcg 4380

gtcagcggca gggtgcactt cttcaggatc ttcggttggc ccttgttggt gtggcgcatg 4440

gcaatgatga ccttcttggc cccgttcacc agatccatgg cgccgcccat ccccgacagc 4500

atcttgcccg gcacgatcca gttggcgata ttgcccttct cgtccacctg cagcgcgccc 4560

agcaccgtga catccacatg cccgccccga atgagcgaga acgacaccga cgagtcgaaa 4620

aaggtgccgt ccggcagcac ggtggtatag tcgccccccg cattcaccac gtccttgtcc 4680

gcctcattga ttttcgggct ggcccccatg cccacaatgc cattttccga ctggaaggtg 4740

atcttgaaat tcttcgggat gtagtcggcc accatcgtgg gcaggcccac gcccagattg 4800

accagctgcc cgtttttcag ttcgcgggcc acgcgcttcg caataatctc cttggccagg 4860

tttttgtcat taatcatttt aggcgggctc cttcacgatg tagttgatga gcacgcccgg 4920

cgtcatggcc ttttccttct ccagcttctc gcaggagacc aggttttcgg cttccacgat 4980

cacggttttg gcggccatcg ccatgtacgg gttgaagttc ttggtggtgc ccttgtagaa 5040

ggtgttgccg gcttcgtcga caatcgagcc tttaatcagg gccacgtcgg cggtcagcgg 5100

cagctccagg aggtattcgg tcccgttgat ggagatcttc tttttgcctt tttcgatgag 5160

ggtccccagg ccggtcttgg tcaggacccc cccgaggccc gagccccccg cgcgaatgcg 5220

ctccaccagc gtgccctgcg gcgacagttc cacttccagc tcattgttga acagtttttt 5280

ccccgtgtcc ggattcgagc caatgtacga ggcgatcagt ttcttcacct ggttgttcga 5340

gatgagcttg ccgatgccgg tgttcgggta gcaggtatcg ttggagataa tggtgagatt 5400

cttgatgttc aggttcacca gaaaatcaat cagcttggtc ggggtgccgc agttcagaaa 5460

gcccccaatc ataatcgtca tgccgtcctt gaagaacgag cggaggttct caaagcggat 5520

gatcttcgag ttcattttaa tccctccttt taaattcctt atttgcgctc gactgccagc 5580

gccacgccca tgccgccgcc gatgcacagc gaggccaggc ccttcttcgc gtcacggcgc 5640

ttcatctcgt gcagcagcgt caccaggata cggcagcccg acgcgccgat cgggtggccg 5700

atggcgatgg cgccgccgtt cacattgacc ttggaggtgt cccagcccat ctgctggtgc 5760

accgccagcg cctgcgcggc aaaggcctcg ttgatctcca tcaggtccag gtcttgcggg 5820

gtccactcgg cgcgcgacag ggcgcgcttg gaggccggca ccgggcccat gcccatcacc 5880

ttgggatcga caccggcgtt ggcatagctc ttgatcgtgg ccagcggggt caggcccagt 5940

tccttggcct tggccgccga catcaccacc accgcggcgg cgccgtcgtt caggcccgag 6000

gcgttggccg cggtcaccgt gccggccttg tcgaaggcgg gcttgaggcc ggacatgctg 6060

tccagcgtgg cgccctggcg cacgaactcg tcggtcttga aggccaccgg gtcgcccttg 6120

cgctgcggga tcagcaccgg gacgatctct tcgtcaaact tgccggcctt ctgcgcggct 6180

tcggccttgt tctgcgagcc gacggcgaac tcatcctgcg cctcgcgtgt gatgccgtat 6240

tccttggcca cgttctcggc ggtgatgccc atgtggtact ggttgtacac gtcccacagg 6300

ccgtcgacga tcatggtgtc gaccagcttg gcatcgccca tgcggaaacc atcgcgcgag 6360

cccggcagca cgtgcggggc ggcgctcatg ttttcctggc cgccggccac cacgatctcg 6420

gcgtcgcccg ccatgatcgc gttggcggcc agcatcacgg ccttcaggcc cgagccgcac 6480

accttgttga tggtcatggc cggcaccatc gccggcaggc cggccttgat cgcggcctgg 6540

cgtgcggggt tctggcccga accggcggtc agcacctggc ccatgatgac ttcgctcacc 6600

tgctccggct tgacgccggc gcgctccagc gcggccttga tgaccacggc acccagttcc 6660

ggtgccggga tcttggccag cgagccgcca aacttgccga ccgcggtgcg ggcggcggat 6720

acgatgacaa cgtcagtcat tgtgtagtcc tttcaatgga aaggtaccca gcttttgttc 6780

cctttagtga gggttaattg cgcgcttggc gtaatcatgg tcatagctgt ttcctgtgtg 6840

aaattgttat ccgctcacaa ttccacacaa catacgagcc ggaagcataa agtgtaaagc 6900

ctggggtgcc taatgagtga gctaactcac attaattgcg ttgcgctcac tgcccgcttt 6960

ccagtcggga aacctgtcgt gccagctgca ttaatgaatc ggccaacgcg cggggagagg 7020

cggtttgcgt attgggcgca tgcataaaaa ctgttgtaat tcattaagca ttctgccgac 7080

atggaagcca tcacaaacgg catgatgaac ctgaatcgcc agcggcatca gcaccttgtc 7140

gccttgcgta taatatttgc ccatgggggt gggcgaagaa ctccagcatg agatccccgc 7200

gctggaggat catccagccg gcgtcccgga aaacgattcc gaagcccaac ctttcataga 7260

aggcggcggt ggaatcgaaa tctcgtgatg gcaggttggg cgtcgcttgg tcggtcattt 7320

cgaaccccag agtcccgctc agaagaactc gtcaagaagg cgatagaagg cgatgcgctg 7380

cgaatcggga gcggcgatac cgtaaagcac gaggaagcgg tcagcccatt cgccgccaag 7440

ctcttcagca atatcacggg tagccaacgc tatgtcctga tagcggtccg ccacacccag 7500

ccggccacag tcgatgaatc cagaaaagcg gccattttcc accatgatat tcggcaagca 7560

ggcatcgcca tgggtcacga cgagatcctc gccgtcgggc atgcgcgcct tgagcctggc 7620

gaacagttcg gctggcgcga gcccctgatg ctcttcgtcc agatcatcct gatcgacaag 7680

accggcttcc atccgagtac gtgctcgctc gatgcgatgt ttcgcttggt ggtcgaatgg 7740

gcaggtagcc ggatcaagcg tatgcagccg ccgcattgca tcagccatga tggatacttt 7800

ctcggcagga gcaaggtgag atgacaggag atcctgcccc ggcacttcgc ccaatagcag 7860

ccagtccctt cccgcttcag tgacaacgtc gagcacagct gcgcaaggaa cgcccgtcgt 7920

ggccagccac gatagccgcg ctgcctcgtc ctgcagttca ttcagggcac cggacaggtc 7980

ggtcttgaca aaaagaaccg ggcgcccctg cgctgacagc cggaacacgg cggcatcaga 8040

gcagccgatt gtctgttgtg cccagtcata gccgaatagc ctctccaccc aagcggccgg 8100

agaacctgcg tgcaatccat cttgttcaat catgcgaaac gatcctcatc ctgtctcttg 8160

atcagatctt gatcccctgc gccatcagat ccttggcggc aagaaagcca tccagtttac 8220

tttgcagggc ttcccaacct taccagaggg cgccccagct ggcaattccg gttcgcttgc 8280

tgtccataaa accgcccagt ctagctatcg ccatgtaagc ccactgcaag ctacctgctt 8340

tctctttgcg cttgcgtttt cccttgtcca gatagcccag tagctgacat tcatcccagg 8400

tggcactttt cggggaaatg tgcgcgcccg cgttcctgct ggcgctgggc ctgtttctgg 8460

cgctggactt cccgctgttc cgtcagcagc ttttcgccca cggccttgat gatcgcggcg 8520

gccttggcct gcatatcccg attcaacggc cccagggcgt ccagaacggg cttcaggcgc 8580

tcccgaaggt                                                          8590

<210>  33
<211>  8590
<212>  DNA
<213>  Artificial Sequence

<220>
<223>  vector

<400>  33
ctcgggccgt ctcttgggct tgatcggcct tcttgcgcat ctcacgcgct cctgcggcgg      60

cctgtagggc aggctcatac ccctgccgaa ccgcttttgt cagccggtcg gccacggctt     120

ccggcgtctc aacgcgcttt gagattccca gcttttcggc caatccctgc ggtgcatagg     180

cgcgtggctc gaccgcttgc gggctgatgg tgacgtggcc cactggtggc cgctccaggg     240

cctcgtagaa cgcctgaatg cgcgtgtgac gtgccttgct gccctcgatg ccccgttgca     300

gccctagatc ggccacagcg gccgcaaacg tggtctggtc gcgggtcatc tgcgctttgt     360

tgccgatgaa ctccttggcc gacagcctgc cgtcctgcgt cagcggcacc acgaacgcgg     420

tcatgtgcgg gctggtttcg tcacggtgga tgctggccgt cacgatgcga tccgccccgt     480

acttgtccgc cagccacttg tgcgccttct cgaagaacgc cgcctgctgt tcttggctgg     540

ccgacttcca ccattccggg ctggccgtca tgacgtactc gaccgccaac acagcgtcct     600

tgcgccgctt ctctggcagc aactcgcgca gtcggcccat cgcttcatcg gtgctgctgg     660

ccgcccagtg ctcgttctct ggcgtcctgc tggcgtcagc gttgggcgtc tcgcgctcgc     720

ggtaggcgtg cttgagactg gccgccacgt tgcccatttt cgccagcttc ttgcatcgca     780

tgatcgcgta tgccgccatg cctgcccctc ccttttggtg tccaaccggc tcgacggggg     840

cagcgcaagg cggtgcctcc ggcgggccac tcaatgcttg agtatactca ctagactttg     900

cttcgcaaag tcgtgaccgc ctacggcggc tgcggcgccc tacgggcttg ctctccgggc     960

ttcgccctgc gcggtcgctg cgctcccttg ccagcccgtg gatatgtgga cgatggccgc    1020

gagcggccac cggctggctc gcttcgctcg gcccgtggac aaccctgctg gacaagctga    1080

tggacaggct gcgcctgccc acgagcttga ccacagggat tgcccaccgg ctacccagcc    1140

ttcgaccaca tacccaccgg ctccaactgc gcggcctgcg gccttgcccc atcaattttt    1200

ttaattttct ctggggaaaa gcctccggcc tgcggcctgc gcgcttcgct tgccggttgg    1260

acaccaagtg gaaggcgggt caaggctcgc gcagcgaccg cgcagcggct tggccttgac    1320

gcgcctggaa cgacccaagc ctatgcgagt gggggcagtc gaaggcgaag cccgcccgcc    1380

tgccccccga gcctcacggc ggcgagtgcg ggggttccaa gggggcagcg ccaccttggg    1440

caaggccgaa ggccgcgcag tcgatcaaca agccccggag gggccacttt ttgccggagg    1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatcccaat attaaaaaaa taagagttac catttaaggt aactcttatt 3300

tttattactt gaggtagtcg tagatgactt cggcgcgcgg gaggatgatg tccgccagga 3360

tatgcgacga cgacacaatc tctttcacgg ggaggtcgtt gagcggcgcc atcgcatgat 3420

cgaacagctg caggcgggtg gggccggtcc acgcttcatg cacggtcacg tcggtaatct 3480

tggcgttgat cagttcgcag atgcgcggcg agccgtcata attcggaata atcttcagca 3540

tgtagttcgg ccggcagatt tggtccttgg cctcattcgc gtcgagcgcc ttgtgcttgt 3600

agcccatggt ggccgtcgcc acgcggagct tgccatagtc cagggtcccg accagcgtgt 3660

ccgagtccac gaagagtttc ggatagccca gtttcttcgg gtaggccgac agctcgcgcc 3720

ccacggcaat cgcgggttcg ttgtccagat acatcatgtg caggtaatcg cccttcacgc 3780

cgttaaagct caccggaatg gcttggcccg attcggtgta gcagcccagg cccgaggtat 3840

cgtgcatcgc catgatctcg aagcgcacca ggggctcgtc gatctccagc ggctcgggca 3900

ccaccttgcg cagggcgtcc atgtcggtgc gatacacgat gttgaagtat tcccggttgt 3960

ggaacttgta gggcccccgc ggaaaggcgg gcgaggtcag cggggtcgaa atttgcttaa 4020

tcacctcatc tttgagcatt cttgaatctc ctgaaatgtg aaattgttat ccgctcacaa 4080

ttccacacaa catacgagcc ggaagcataa agtgtaaagc ctggggaagc ttattatctt 4140

aagtaataaa aataagagtt accttaaatg gtaactctta tttttttaat attgtttcat 4200

agtatttctt tctacacggc catcgggcgc agctcattgc tgatgagcag gtcggcggcc 4260

gtcagcgagc gaatttcgtc aatggtggtg ttcttgttga tttcggtgag gaggaggccg 4320

tcgttaatca cttcgatcac ccccagttcg gtcacaatca ggttcgcctg cgatttggcg 4380

gtcagcggca gggtgcactt cttcaggatc ttcggttggc ccttgttggt gtggcgcatg 4440

gcaatgatga ccttcttggc cccgttcacc agatccatgg cgccgcccat ccccgacagc 4500

atcttgcccg gcacgatcca gttggcgata ttgcccttct cgtccacctg cagcgcgccc 4560

agcaccgtga catccacatg cccgccccga atgagcgaga acgacaccga cgagtcgaaa 4620

aaggtgccgt ccggcagcac ggtggtatag tcgccccccg cattcaccac gtccttgtcc 4680

gcctcattga ttttcgggct ggcccccatg cccacaatgc cattttccga ctggaaggtg 4740

atcttgaaat tcttcgggat gtagtcggcc accatcgtgg gcaggcccac gcccagattg 4800

accagctgcc cgtttttcag ttcgcgggcc acgcgcttcg caataatctc cttggccagg 4860

tttttgtcat taatcatttt aggcgggctc cttcacgatg tagttgatga gcacgcccgg 4920

cgtcatggcc ttttccttct ccagcttctc gcaggagacc aggttttcgg cttccacgat 4980

cacggttttg gcggccatcg ccatgtacgg gttgaagttc ttggtggtgc ccttgtagaa 5040

ggtgttgccg gcttcgtcga caatcgagcc tttaatcagg gccacgtcgg cggtcagcgg 5100

cagctccagg aggtattcgg tcccgttgat ggagatcttc tttttgcctt tttcgatgag 5160

ggtccccagg ccggtcttgg tcaggacccc cccgaggccc gagccccccg cgcgaatgcg 5220

ctccaccagc gtgccctgcg gcgacagttc cacttccagc tcattgttga acagtttttt 5280

ccccgtgtcc ggattcgagc caatgtacga ggcgatcagt ttcttcacct ggttgttcga 5340

gatgagcttg ccgatgccgg tgttcgggta gcaggtatcg ttggagataa tggtgagatt 5400

cttgatgttc aggttcacca gaaaatcaat cagcttggtc ggggtgccgc agttcagaaa 5460

gcccccaatc ataatcgtca tgccgtcctt gaagaacgag cggaggttct caaagcggat 5520

gatcttcgag ttcattttaa tccctccttt taaattcctt atttgcgctc gactgccagc 5580

gccacgccca tgccgccgcc gatgcacagc gaggccaggc ccttcttcgc gtcacggcgc 5640

ttcatctcgt gcagcagcgt caccaggata cggcagcccg acgcgccgat cgggtggccg 5700

atggcgatgg cgccgccgtt cacattgacc ttggaggtgt cccagcccat ctgctggtgc 5760

accgccagcg cctgcgcggc aaaggcctcg ttgatctcca tcaggtccag gtcttgcggg 5820

gtccactcgg cgcgcgacag ggcgcgcttg gaggccggca ccgggcccat gcccatcacc 5880

ttgggatcga caccggcgtt ggcatagctc ttgatcgtgg ccagcggggt caggcccagt 5940

tccttggcct tggccgccga catcaccacc accgcggcgg cgccgtcgtt caggcccgag 6000

gcgttggccg cggtcaccgt gccggccttg tcgaaggcgg gcttgaggcc ggacatgctg 6060

tccagcgtgg cgccctggcg cacgaactcg tcggtcttga aggccaccgg gtcgcccttg 6120

cgctgcggga tcagcaccgg gacgatctct tcgtcaaact tgccggcctt ctgcgcggct 6180

tcggccttgt tctgcgagcc gacggcgaac tcatcctgcg cctcgcgtgt gatgccgtat 6240

tccttggcca cgttctcggc ggtgatgccc atgtggtact ggttgtacac gtcccacagg 6300

ccgtcgacga tcatggtgtc gaccagcttg gcatcgccca tgcggaaacc atcgcgcgag 6360

cccggcagca cgtgcggggc ggcgctcatg ttttcctggc cgccggccac cacgatctcg 6420

gcgtcgcccg ccatgatcgc gttggcggcc agcatcacgg ccttcaggcc cgagccgcac 6480

accttgttga tggtcatggc cggcaccatc gccggcaggc cggccttgat cgcggcctgg 6540

cgtgcggggt tctggcccga accggcggtc agcacctggc ccatgatgac ttcgctcacc 6600

tgctccggct tgacgccggc gcgctccagc gcggccttga tgaccacggc acccagttcc 6660

ggtgccggga tcttggccag cgagccgcca aacttgccga ccgcggtgcg ggcggcggat 6720

acgatgacaa cgtcagtcat tgtgtagtcc tttcaatgga aaggtaccca gcttttgttc 6780

cctttagtga gggttaattg cgcgcttggc gtaatcatgg tcatagctgt ttcctgtgtg 6840

aaattgttat ccgctcacaa ttccacacaa catacgagcc ggaagcataa agtgtaaagc 6900

ctggggtgcc taatgagtga gctaactcac attaattgcg ttgcgctcac tgcccgcttt 6960

ccagtcggga aacctgtcgt gccagctgca ttaatgaatc ggccaacgcg cggggagagg 7020

cggtttgcgt attgggcgca tgcataaaaa ctgttgtaat tcattaagca ttctgccgac 7080

atggaagcca tcacaaacgg catgatgaac ctgaatcgcc agcggcatca gcaccttgtc 7140

gccttgcgta taatatttgc ccatgggggt gggcgaagaa ctccagcatg agatccccgc 7200

gctggaggat catccagccg gcgtcccgga aaacgattcc gaagcccaac ctttcataga 7260

aggcggcggt ggaatcgaaa tctcgtgatg gcaggttggg cgtcgcttgg tcggtcattt 7320

cgaaccccag agtcccgctc agaagaactc gtcaagaagg cgatagaagg cgatgcgctg 7380

cgaatcggga gcggcgatac cgtaaagcac gaggaagcgg tcagcccatt cgccgccaag 7440

ctcttcagca atatcacggg tagccaacgc tatgtcctga tagcggtccg ccacacccag 7500

ccggccacag tcgatgaatc cagaaaagcg gccattttcc accatgatat tcggcaagca 7560

ggcatcgcca tgggtcacga cgagatcctc gccgtcgggc atgcgcgcct tgagcctggc 7620

gaacagttcg gctggcgcga gcccctgatg ctcttcgtcc agatcatcct gatcgacaag 7680

accggcttcc atccgagtac gtgctcgctc gatgcgatgt ttcgcttggt ggtcgaatgg 7740

gcaggtagcc ggatcaagcg tatgcagccg ccgcattgca tcagccatga tggatacttt 7800

ctcggcagga gcaaggtgag atgacaggag atcctgcccc ggcacttcgc ccaatagcag 7860

ccagtccctt cccgcttcag tgacaacgtc gagcacagct gcgcaaggaa cgcccgtcgt 7920

ggccagccac gatagccgcg ctgcctcgtc ctgcagttca ttcagggcac cggacaggtc 7980

ggtcttgaca aaaagaaccg ggcgcccctg cgctgacagc cggaacacgg cggcatcaga 8040

gcagccgatt gtctgttgtg cccagtcata gccgaatagc ctctccaccc aagcggccgg 8100

agaacctgcg tgcaatccat cttgttcaat catgcgaaac gatcctcatc ctgtctcttg 8160

atcagatctt gatcccctgc gccatcagat ccttggcggc aagaaagcca tccagtttac 8220

tttgcagggc ttcccaacct taccagaggg cgccccagct ggcaattccg gttcgcttgc 8280

tgtccataaa accgcccagt ctagctatcg ccatgtaagc ccactgcaag ctacctgctt 8340

tctctttgcg cttgcgtttt cccttgtcca gatagcccag tagctgacat tcatcccagg 8400

tggcactttt cggggaaatg tgcgcgcccg cgttcctgct ggcgctgggc ctgtttctgg 8460

cgctggactt cccgctgttc cgtcagcagc ttttcgccca cggccttgat gatcgcggcg 8520

gccttggcct gcatatcccg attcaacggc cccagggcgt ccagaacggg cttcaggcgc 8580

tcccgaaggt 8590

<210> 34
<211> 8582
<212> DNA
<213> Artificial Sequence

<220>
<223> vector

<400> 34
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaattttct ctggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag ggccactttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatggccca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatcccaat attaaaaaaa taagagttac catttaaggt aactcttatt 3300

tttattactt caggtagtcg tagatgacct ccgcccgggg caggataata tcggcgagga 3360

tatgcgagga cgacacgatt tctttcaccg gcaggtcatt caggggcgcc atcgcatggt 3420

cgaagagctg caggcgcgtg gggcccgtcc aggcttcatg cacggtcacg tcggtgatct 3480

tcgcgttgat cagttcgcaa atgcgcgggg agccgtcgta attggggatg attttcagca 3540

tgtagttggg gcggcaaatc tggtccttcg cttcattcgc gtccagcgcc ttgtgcttgt 3600

agcccatggt ggcggtggcg acgcgcagtt tgccgtagtc gagggtgccg accagcgtgt 3660

ccgaatccac aaacagcttc ggatagccca gcttcttggg gtaggcggac agctcgcggc 3720

ccacggcaat ggccggttcg ttgtccaggt acatcatgtg caggtaatcc cccttcacgc 3780

cattaaacga gaccggaatc gcctggccgc tttcggtata gcagcccagg ccggaggtgt 3840

catgcatcgc cataatctcg aagcgcacca ggggctcatc gatctccagc ggttccggga 3900

cgaccttgcg gagggcatcc atgtcggtgc ggtacacgat gttgaagtac tcgcggttgt 3960
ggaacttgta gggcccccgc ggaaaggcgg gggacgtcag cggggtgctg atttgtttga 4020
tgacttcgtc cttgagcatt cttgaatctc ctgaaatgtg aaattgttat ccgctcacaa 4080
ttccacacaa catacgagcc ggaagcataa agtgtaaagc ctggggaagc ttattatctt 4140
aagtaataaa aataagagtt accttaaatg gtaactctta tttttttaat attgtttcat 4200
agtatttctt tctacacggc catcgggcgc agctcattgc tgatgagcag gtcggcggcc 4260
gtcagcgagc gaatttcgtc aatggtggtg ttcttgttga tttcggtgag gaggaggccg 4320
tcgttaatca cttcgatcac ccccagttcg gtcacaatca ggttcgcctg cgatttggcg 4380
gtcagcggca gggtgcactt cttcaggatc ttcggttggc ccttgttggt gtggcgcatg 4440
gcaatgatga ccttcttggc cccgttcacc agatccatgg cgccgcccat ccccgacagc 4500
atcttgcccg gcacgatcca gttggcgata ttgcccttct cgtccacctg cagcgcgccc 4560
agcaccgtga catccacatg cccgccccga atgagcgaga acgacaccga cgagtcgaaa 4620
aaggtgccgt ccggcagcac ggtggtatag tcgccccccg cattcaccac gtccttgtcc 4680
gcctcattga ttttcgggct ggcccccatg cccacaatgc cattttccga ctggaaggtg 4740
atcttgaaat tcttcgggat gtagtcggcc accatcgtgg gcaggcccac gcccagattg 4800
accagctgcc cgtttttcag ttcgcgggcc acgcgcttcg caataatctc cttggccagg 4860
tttttgtcat taatcatttt aggcgggctc cttcacgatg tagttgatga gcacgcccgg 4920
cgtcatggcc ttttccttct ccagcttctc gcaggagacc aggttttcgg cttccacgat 4980
cacggttttg gcggccatcg ccatgtacgg gttgaagttc ttggtggtgc ccttgtagaa 5040
ggtgttgccg gcttcgtcga caatcgagcc tttaatcagg gccacgtcgg cggtcagcgg 5100
cagctccagg aggtattcgg tcccgttgat ggagatcttc tttttgcctt tttcgatgag 5160
ggtccccagg ccggtcttgg tcaggacccc cccgaggccc gagccccccg cgcgaatgcg 5220
ctccaccagc gtgccctgcg gcgacagttc cacttccagc tcattgttga acagtttttt 5280
ccccgtgtcc ggattcgagc caatgtacga ggcgatcagt ttcttcacct ggttgttcga 5340
gatgagcttg ccgatgccgg tgttcgggta gcaggtatcg ttggagataa tggtgagatt 5400
cttgatgttc aggttcacca gaaaatcaat cagcttggtc ggggtgccgc agttcagaaa 5460
gcccccaatc ataatcgtca tgccgtcctt gaagaacgag cggaggttct caaagcggat 5520
gatcttcgag ttcattttaa tccctccttt taaactttct agcacttttc cagcaggatc 5580
gcggtgccct ggccgccgcc gatgcacagg gtcgccagcc ccttcttggc gtcgcgcttc 5640
tgcatcgcgt gcaccagggt caccaggatg cgggcgcccg aggcgccgat cgggtgcccc 5700
agggcgatgg cgccgccatt cacgttcact ttgttcatgt cgaacttcag gtccttggcg 5760
acggccagcg actgggcggc aaaggcctcg ttcgactcga tcaggtccag ctcgtcgacc 5820

gtccagccgg ctttctcgat cgccgccttg gtggcgtaga acgggccgta gcccatgatg 5880

gccgggtcca cgccggccga gccatacgac acgatcttcg ccagcggttt cacgcccagc 5940

tccttggcct tttcggccga catgatcacc agcacggccg cgcagtcgtt caggcccgag 6000

gcgttgcccg cggtcacggt gccgtccttc ttgaaggccg gcttcagctt ggccaggccc 6060

tcgatcgtcg acccgaagcg cgggtgctcg tccgtgtcca ccacggtctc gcccttgcgg 6120

cccttaatca ccaccggcac gatctcgtcc ttgaactggc ccgacttgat ggcttcctcc 6180

gccttctttt gcgaggccag ggcgaactcg tcctgctcct cgcgcgagat gttccagcgc 6240

tcggcgatgt tctcggcggt gatgcccatg tggtagtcgt tgaaggcgtc ccacaggccg 6300

tcggtgatca tctcgtccac gaacttggcg ttccccatgc ggtagcccca gcgggcgttg 6360

ttggccaggt acggggcgcg cgacatgttt tccatgccgc cggcaatgat cacgtcggcg 6420

tcgccggcct tgatgatctg ggccgccagc gacacggtgc gcaggcccga gccgcacacc 6480

ttgttgatgg tcatggcggg gatctccacc gggaggccgg ctttgaagct cgcctggcgg 6540

gccgggttct ggccgagccc ggcctgcagc acgttgccca ggatcacctc gttcacgtcc 6600

tccggcttga tgccggcctt cttcacggcc tccttaatgg cggtggcgcc caggtccacg 6660

gcgggcacgt ctttcaggct cttgccgtac gagccgatcg cggtgcgcac ggccgaggca 6720

atcaccacct ccttcattct tgaatctcct gaaaggtacc cagcttttgt tccctttagt 6780

gagggttaat tgcgcgcttg gcgtaatcat ggtcatagct gtttcctgtg tgaaattgtt 6840

atccgctcac aattccacac aacatacgag ccggaagcat aaagtgtaaa gcctggggtg 6900

cctaatgagt gagctaactc acattaattg cgttgcgctc actgcccgct ttccagtcgg 6960

gaaacctgtc gtgccagctg cattaatgaa tcggccaacg cgcggggaga ggcggtttgc 7020

gtattgggcg catgcataaa aactgttgta attcattaag cattctgccg acatggaagc 7080

catcacaaac ggcatgatga acctgaatcg ccagcggcat cagcaccttg tcgccttgcg 7140

tataatattt gcccatgggg gtgggcgaag aactccagca tgagatcccc gcgctggagg 7200

atcatccagc cggcgtcccg gaaaacgatt ccgaagccca acctttcata gaaggcggcg 7260

gtggaatcga aatctcgtga tggcaggttg ggcgtcgctt ggtcggtcat ttcgaacccc 7320

agagtcccgc tcagaagaac tcgtcaagaa ggcgatagaa ggcgatgcgc tgcgaatcgg 7380

gagcggcgat accgtaaagc acgaggaagc ggtcagccca ttcgccgcca agctcttcag 7440

caatatcacg ggtagccaac gctatgtcct gatagcggtc cgccacaccc agccggccac 7500

agtcgatgaa tccagaaaag cggccatttt ccaccatgat attcggcaag caggcatcgc 7560

catgggtcac gacgagatcc tcgccgtcgg gcatgcgcgc cttgagcctg gcgaacagtt 7620

cggctggcgc gagcccctga tgctcttcgt ccagatcatc ctgatcgaca agaccggctt 7680

ccatccgagt acgtgctcgc tcgatgcgat gtttcgcttg gtggtcgaat gggcaggtag 7740

ccggatcaag cgtatgcagc cgccgcattg catcagccat gatggatact ttctcggcag 7800

gagcaaggtg agatgacagg agatcctgcc ccggcacttc gcccaatagc agccagtccc 7860

ttcccgcttc agtgacaacg tcgagcacag ctgcgcaagg aacgcccgtc gtggccagcc 7920

acgatagccg cgctgcctcg tcctgcagtt cattcagggc accggacagg tcggtcttga 7980

caaaaagaac cgggcgcccc tgcgctgaca gccggaacac ggcggcatca gagcagccga 8040

ttgtctgttg tgcccagtca tagccgaata gcctctccac ccaagcggcc ggagaacctg 8100

cgtgcaatcc atcttgttca atcatgcgaa acgatcctca tcctgtctct tgatcagatc 8160

ttgatcccct gcgccatcag atccttggcg gcaagaaagc catccagttt actttgcagg 8220

gcttcccaac cttaccagag ggcgccccag ctggcaattc cggttcgctt gctgtccata 8280

aaaccgccca gtctagctat cgccatgtaa gcccactgca agctacctgc tttctctttg 8340

cgcttgcgtt ttcccttgtc cagatagccc agtagctgac attcatccca ggtggcactt 8400

ttcggggaaa tgtgcgcgcc cgcgttcctg ctggcgctgg gcctgtttct ggcgctggac 8460

ttcccgctgt tccgtcagca gcttttcgcc cacggccttg atgatcgcgg cggccttggc 8520

ctgcatatcc cgattcaacg gccccagggc gtccagaacg ggcttcaggc gctcccgaag 8580

gt 8582

<210> 35
<211> 246
<212> PRT
<213> Ralstonia eutropha

<400> 35

Met Thr Gln Arg Ile Ala Tyr Val Thr Gly Gly Met Gly Gly Ile Gly
1               5                   10                  15

Thr Ala Ile Cys Gln Arg Leu Ala Lys Asp Gly Phe Arg Val Val Ala
            20                  25                  30

Gly Cys Gly Pro Asn Ser Pro Arg Arg Glu Lys Trp Leu Glu Gln Gln
        35                  40                  45

Lys Ala Leu Gly Phe Asp Phe Ile Ala Ser Glu Gly Asn Val Ala Asp
    50                  55                  60

Trp Asp Ser Thr Lys Thr Ala Phe Asp Lys Val Lys Ser Glu Val Gly
65                  70                  75                  80

Glu Val Asp Val Leu Ile Asn Asn Ala Gly Ile Thr Arg Asp Val Val
                85                  90                  95

Phe Arg Lys Met Thr Arg Ala Asp Trp Asp Ala Val Ile Asp Thr Asn
            100                 105                 110

Leu Thr Ser Leu Phe Asn Val Thr Lys Gln Val Ile Asp Gly Met Ala
        115                 120                 125

Asp Arg Gly Trp Gly Arg Ile Val Asn Ile Ser Ser Val Asn Gly Gln
    130                 135                 140

Lys Gly Gln Phe Gly Gln Thr Asn Tyr Ser Thr Ala Lys Ala Gly Leu
145                 150                 155                 160

His Gly Phe Thr Met Ala Leu Ala Gln Glu Val Ala Thr Lys Gly Val
                165                 170                 175

Thr Val Asn Thr Val Ser Pro Gly Tyr Ile Ala Thr Asp Met Val Lys
            180                 185                 190

Ala Ile Arg Gln Asp Val Leu Asp Lys Ile Val Ala Thr Ile Pro Val
        195                 200                 205

Lys Arg Leu Gly Leu Pro Glu Glu Ile Ala Ser Ile Cys Ala Trp Leu
    210                 215                 220

Ser Ser Glu Glu Ser Gly Phe Ser Thr Gly Ala Asp Phe Ser Leu Asn
225                 230                 235                 240

Gly Gly Leu His Met Gly
                245

<210> 36
<211> 134
<212> PRT
<213> Ralstonia eutropha

<400> 36

Met Ser Ala Gln Ser Leu Glu Val Gly Gln Lys Ala Arg Leu Ser Lys
1               5                   10                  15

Arg Phe Gly Ala Ala Glu Val Ala Ala Phe Ala Ala Leu Ser Glu Asp
            20                  25                  30

Phe Asn Pro Leu His Leu Asp Pro Ala Phe Ala Ala Thr Thr Ala Phe
        35                  40                  45

Glu Arg Pro Ile Val His Gly Met Leu Leu Ala Ser Leu Phe Ser Gly
    50                  55                  60

Leu Leu Gly Gln Gln Leu Pro Gly Lys Gly Ser Ile Tyr Leu Gly Gln
65                  70                  75                  80

Ser Leu Ser Phe Lys Leu Pro Val Phe Val Gly Asp Glu Val Thr Ala
                85                  90                  95

Glu Val Glu Val Thr Ala Leu Arg Glu Asp Lys Pro Ile Ala Thr Leu
            100                 105                 110

Thr Thr Arg Ile Phe Thr Gln Gly Gly Ala Leu Ala Val Thr Gly Glu
        115                 120                 125

Ala Val Val Lys Leu Pro
    130

<210>  37
<211>  858
<212>  PRT
<213>  Clostridium acetobutylicum

<400>  37

Met Lys Val Thr Asn Gln Lys Glu Leu Lys Gln Lys Leu Asn Glu Leu
1               5                   10                  15

Arg Glu Ala Gln Lys Lys Phe Ala Thr Tyr Thr Gln Glu Gln Val Asp
            20                  25                  30

Lys Ile Phe Lys Gln Cys Ala Ile Ala Ala Ala Lys Glu Arg Ile Asn
        35                  40                  45

Leu Ala Lys Leu Ala Val Glu Glu Thr Gly Ile Gly Leu Val Glu Asp
    50                  55                  60

Lys Ile Ile Lys Asn His Phe Ala Ala Glu Tyr Ile Tyr Asn Lys Tyr
65                  70                  75                  80

Lys Asn Glu Lys Thr Cys Gly Ile Ile Asp His Asp Asp Ser Leu Gly
                85                  90                  95

Ile Thr Lys Val Ala Glu Pro Ile Gly Ile Val Ala Ala Ile Val Pro
            100                 105                 110

Thr Thr Asn Pro Thr Ser Thr Ala Ile Phe Lys Ser Leu Ile Ser Leu
        115                 120                 125

Lys Thr Arg Asn Ala Ile Phe Phe Ser Pro His Pro Arg Ala Lys Lys
    130                 135                 140

Ser Thr Ile Ala Ala Ala Lys Leu Ile Leu Asp Ala Ala Val Lys Ala
145                 150                 155                 160

Gly Ala Pro Lys Asn Ile Ile Gly Trp Ile Asp Glu Pro Ser Ile Glu
                165                 170                 175

Leu Ser Gln Asp Leu Met Ser Glu Ala Asp Ile Ile Leu Ala Thr Gly
            180                 185                 190

Gly Pro Ser Met Val Lys Ala Ala Tyr Ser Ser Gly Lys Pro Ala Ile
        195                 200                 205

Gly Val Gly Ala Gly Asn Thr Pro Ala Ile Ile Asp Glu Ser Ala Asp
    210                 215                 220

Ile Asp Met Ala Val Ser Ser Ile Ile Leu Ser Lys Thr Tyr Asp Asn
225                 230                 235                 240

Gly Val Ile Cys Ala Ser Glu Gln Ser Ile Leu Val Met Asn Ser Ile
                245                 250                 255

Tyr Glu Lys Val Lys Glu Glu Phe Val Lys Arg Gly Ser Tyr Ile Leu
            260                 265                 270

Asn Gln Asn Glu Ile Ala Lys Ile Lys Glu Thr Met Phe Lys Asn Gly
        275                 280                 285

Ala Ile Asn Ala Asp Ile Val Gly Lys Ser Ala Tyr Ile Ile Ala Lys
    290                 295                 300

Met Ala Gly Ile Glu Val Pro Gln Thr Thr Lys Ile Leu Ile Gly Glu
305                 310                 315                 320

Val Gln Ser Val Glu Lys Ser Glu Leu Phe Ser His Glu Lys Leu Ser
                325                 330                 335

Pro Val Leu Ala Met Tyr Lys Val Lys Asp Phe Asp Glu Ala Leu Lys
            340                 345                 350

Lys Ala Gln Arg Leu Ile Glu Leu Gly Gly Ser Gly His Thr Ser Ser
        355                 360                 365

Leu Tyr Ile Asp Ser Gln Asn Asn Lys Asp Lys Val Lys Glu Phe Gly
    370                 375                 380

Leu Ala Met Lys Thr Ser Arg Thr Phe Ile Asn Met Pro Ser Ser Gln
385                 390                 395                 400

Gly Ala Ser Gly Asp Leu Tyr Asn Phe Ala Ile Ala Pro Ser Phe Thr
                405                 410                 415

Leu Gly Cys Gly Thr Trp Gly Gly Asn Ser Val Ser Gln Asn Val Glu
            420                 425                 430

Pro Lys His Leu Leu Asn Ile Lys Ser Val Ala Glu Arg Arg Glu Asn
        435                 440                 445

Met Leu Trp Phe Lys Val Pro Gln Lys Ile Tyr Phe Lys Tyr Gly Cys
    450                 455                 460

Leu Arg Phe Ala Leu Lys Glu Leu Lys Asp Met Asn Lys Lys Arg Ala
465                 470                 475                 480

Phe Ile Val Thr Asp Lys Asp Leu Phe Lys Leu Gly Tyr Val Asn Lys
                485                 490                 495

Ile Thr Lys Val Leu Asp Glu Ile Asp Ile Lys Tyr Ser Ile Phe Thr
            500                 505                 510

Asp Ile Lys Ser Asp Pro Thr Ile Asp Ser Val Lys Lys Gly Ala Lys
        515                 520                 525

Glu Met Leu Asn Phe Glu Pro Asp Thr Ile Ile Ser Ile Gly Gly Gly
    530                 535                 540

Ser Pro Met Asp Ala Ala Lys Val Met His Leu Leu Tyr Glu Tyr Pro
545                 550                 555                 560

Glu Ala Glu Ile Glu Asn Leu Ala Ile Asn Phe Met Asp Ile Arg Lys
                565                 570                 575

Arg Ile Cys Asn Phe Pro Lys Leu Gly Thr Lys Ala Ile Ser Val Ala
            580                 585                 590

Ile Pro Thr Thr Ala Gly Thr Gly Ser Glu Ala Thr Pro Phe Ala Val
        595                 600                 605

Ile Thr Asn Asp Glu Thr Gly Met Lys Tyr Pro Leu Thr Ser Tyr Glu
    610                 615                 620

Leu Thr Pro Asn Met Ala Ile Ile Asp Thr Glu Leu Met Leu Asn Met
625                 630                 635                 640

Pro Arg Lys Leu Thr Ala Ala Thr Gly Ile Asp Ala Leu Val His Ala

645                 650                 655

Ile Glu Ala Tyr Val Ser Val Met Ala Thr Asp Tyr Thr Asp Glu Leu
            660                 665                 670

Ala Leu Arg Ala Ile Lys Met Ile Phe Lys Tyr Leu Pro Arg Ala Tyr
        675                 680                 685

Lys Asn Gly Thr Asn Asp Ile Glu Ala Arg Glu Lys Met Ala His Ala
    690                 695                 700

Ser Asn Ile Ala Gly Met Ala Phe Ala Asn Ala Phe Leu Gly Val Cys
705                 710                 715                 720

His Ser Met Ala His Lys Leu Gly Ala Met His His Val Pro His Gly
                725                 730                 735

Ile Ala Cys Ala Val Leu Ile Glu Glu Val Ile Lys Tyr Asn Ala Thr
            740                 745                 750

Asp Cys Pro Thr Lys Gln Thr Ala Phe Pro Gln Tyr Lys Ser Pro Asn
        755                 760                 765

Ala Lys Arg Lys Tyr Ala Glu Ile Ala Glu Tyr Leu Asn Leu Lys Gly
    770                 775                 780

Thr Ser Asp Thr Glu Lys Val Thr Ala Leu Ile Glu Ala Ile Ser Lys
785                 790                 795                 800

Leu Lys Ile Asp Leu Ser Ile Pro Gln Asn Ile Ser Ala Ala Gly Ile
                805                 810                 815

Asn Lys Lys Asp Phe Tyr Asn Thr Leu Asp Lys Met Ser Glu Leu Ala
            820                 825                 830

Phe Asp Asp Gln Cys Thr Thr Ala Asn Pro Arg Tyr Pro Leu Ile Ser
        835                 840                 845

Glu Leu Lys Asp Ile Tyr Ile Lys Ser Phe
    850                 855

<210>  38
<211>  50
<212>  DNA
<213>  Acinetobacter caviae

<400>  38
ggcacgcagg cacaatcagc ccggcccctg ccgggctgat tgttctcccc                50

<210>  39
<211>  5106
<212>  DNA
<213>  Artificial Sequence

<220>
<223>  codonoptimized gen

<400>  39
ggtacctttc cattgaaagg actacacaat gactgacgtt gtcatcgtat ccgccgcccg      60

caccgcggtc ggcaagtttg gcggctcgct ggccaagatc ccggcaccgg aactgggtgc     120

cgtggtcatc aaggccgcgc tggagcgcgc cggcgtcaag ccggagcagg tgagcgaagt     180

catcatgggc caggtgctga ccgccggttc gggccagaac cccgcacgcc aggccgcgat     240

caaggccggc ctgccggcga tggtgccggc catgaccatc aacaaggtgt gcggctcggg     300

cctgaaggcc gtgatgctgg ccgccaacgc gatcatggcg ggcgacgccg agatcgtggt     360

ggccggcggc caggaaaaca tgagcgccgc cccgcacgtg ctgccgggct cgcgcgatgg     420

tttccgcatg ggcgatgcca agctggtcga caccatgatc gtcgacggcc tgtgggacgt     480

gtacaaccag taccacatgg gcatcaccgc cgagaacgtg gccaaggaat acggcatcac     540

acgcgaggcg caggatgagt tcgccgtcgg ctcgcagaac aaggccgaag ccgcgcagaa     600

ggccggcaag tttgacgaag agatcgtccc ggtgctgatc ccgcagcgca agggcgaccc     660

ggtggccttc aagaccgacg agttcgtgcg ccagggcgcc acgctggaca gcatgtccgg     720

cctcaagccc gccttcgaca aggccggcac ggtgaccgcg gccaacgcct cgggcctgaa     780

cgacggcgcc gccgcggtgg tggtgatgtc ggcggccaag gccaaggaac tgggcctgac     840

cccgctggcc acgatcaaga gctatgccaa cgccggtgtc gatcccaagg tgatgggcat     900

gggcccggtg ccggcctcca agcgcgccct gtcgcgcgcc gagtggaccc cgcaagacct     960

ggacctgatg gagatcaacg aggcctttgc cgcgcaggcg ctggcggtgc accagcagat    1020

gggctgggac acctccaagg tcaatgtgaa cggcggcgcc atcgccatcg gccacccgat    1080

cggcgcgtcg ggctgccgta tcctggtgac gctgctgcac gagatgaagc gccgtgacgc    1140

gaagaagggc ctggcctcgc tgtgcatcgg cggcggcatg ggcgtggcgc tggcagtcga    1200

gcgcaaataa ggaaggggtt ttccggggcc gcgcgcggtt ggcgcggacc cggcgacgat    1260

aacgaagcca atcaaggagt ggacatgact cagcgcattg cgtatgtgac cggcggcatg    1320

ggtggtatcg gaaccgccat ttgccagcgg ctggccaagg atggctttcg tgtggtggcc    1380

ggttgcggcc ccaactcgcc gcgccgcgaa aagtggctgg agcagcagaa ggccctgggc    1440

ttcgatttca ttgcctcgga aggcaatgtg gctgactggg actcgaccaa gaccgcattc    1500

gacaaggtca agtccgaggt cggcgaggtt gatgtgctga tcaacaacgc cggtatcacc    1560

cgcgacgtgg tgttccgcaa gatgacccgc gccgactggg atgcggtgat cgacaccaac    1620

ctgacctcgc tgttcaacgt caccaagcag gtgatcgacg gcatggccga ccgtggctgg 1680

ggccgcatcg tcaacatctc gtcggtgaac gggcagaagg gccagttcgg ccagaccaac 1740

tactccaccg ccaaggccgg cctgcatggc ttcaccatgg cactggcgca ggaagtggcg 1800

accaagggcg tgaccgtcaa cacggtctct ccgggctata tcgccaccga catggtcaag 1860

gcgatccgcc aggacgtgct cgacaagatc gtcgcgacga tcccggtcaa gcgcctgggc 1920

ctgccggaag agatcgcctc gatctgcgcc tggttgtcgt cggaggagtc cggtttctcg 1980

accggcgccg acttctcgct caacggcggc ctgcatatgg gctgaaacag aggaggacgc 2040

cgcatgtcgg cccagtcgct ggaggtcggg caaaaagccc ggctgtccaa gcggtttggc 2100

gcggcggagg tggcggcctt tgcggcgctc tccgaggact tcaacccgct gcacctggac 2160

cccgcgtttg ccgcgaccac cgcgttcgag cgccccatcg tgcatggcat gctgctcgcc 2220

tcgctgttct cgggcctcct cggccagcag ctccccggca aggggagcat ctacctgggc 2280

caatcgctct cgttcaaact gccggtgttc gtgggggacg aagtgaccgc cgaggtggaa 2340

gtcaccgcgc tgcgcgagga caaaccgatc gccacgctga cgacccggat ctttacccaa 2400

ggcggggccc tggccgtcac cggggaagcc gtggtcaagc tgccgtaagc accggctttc 2460

aggagattca agaatgaagg tgaccaacca gaaggagctg aagcagaagc tgaacgagct 2520

gcgcgaggcc cagaagaagt tcgccaccta cacccaggag caggtggaca agatcttcaa 2580

gcagtgcgcc attgccgccg ccaaggagcg catcaacctg gccaagctgg ccgtggagga 2640

gaccggcatc ggcctggtgg aggacaagat catcaagaac cacttcgccg ccgagtacat 2700

ctacaacaag tacaagaacg agaagacctg cggcattatc gaccacgacg actcgctggg 2760

catcaccaag gtggccgagc cgatcggcat cgtggccgcc atcgtgccga ccacgaaccc 2820

gacctcgacc gccatcttca agtcgctgat ctcgctgaag acccgcaacg ccatcttctt 2880

ctcgccgcac ccgcgcgcca agaagtcgac catcgccgcc gccaagctga tcctggacgc 2940

cgccgtgaag gccggggccc cgaagaacat catcggctgg attgacgagc cgtcgatcga 3000

gctgtcgcag gacctgatgt cggaggccga tatcatcctg gccaccggcg gcccgtcgat 3060

ggtgaaggcc gcctactcgt cgggcaagcc ggccatcggc gtgggcgcgg gcaacacccc 3120

ggccatcatc gacgagtccg ccgacatcga tatggccgtg tcgtcgatca tcctgtcgaa 3180

gacctacgac aacggcgtga tctgcgcctc ggagcagtcg atcctggtga tgaactccat 3240

ctacgagaag gtgaaggagg agttcgtgaa gcgcggctcg tacatcctga accagaacga 3300

gatcgccaag atcaaggaga cgatgttcaa gaacggcgcc atcaacgccg acatcgtggg 3360

caagtcggcc tacatcatcg ccaagatggc cggcatcgag gtgccgcaga ccaccaagat 3420

cctcatcggc gaggtgcagt cggtggagaa gtcggagctg ttctcgcacg agaagctgtc 3480

gccggtgctg gccatgtaca aggtgaagga cttcgatgag gccctcaaga aggcccagcg 3540

cctgatcgag ctgggcggct cgggccacac ctcgtcgctg tacatcgact cgcagaacaa 3600

caaggacaag gtgaaggagt tcggcctggc catgaagacc tcgcgcacct tcatcaacat 3660

gccgtcgtcg cagggcgcct cgggcgacct gtacaacttc gccatcgccc cgtcgttcac 3720

cctgggctgc gggacctggg gcggcaactc ggtgtcgcag aacgtggagc cgaagcacct 3780

gctgaacatc aagtcggtgg ccgagcgccg cgagaatatg ctgtggttca aggtgccgca 3840

gaagatctac ttcaagtacg gctgcctgcg cttcgccctg aaggagctga aggacatgaa 3900

caagaagcgc gccttcatcg tgaccgacaa ggacctgttc aagctgggct acgtgaacaa 3960

gatcaccaag gtgctggacg agatcgacat caagtactcg atcttcaccg atatcaagtc 4020

ggacccgacc attgactcgg tgaagaaggg cgccaaggag atgctgaact tcgagccgga 4080

caccatcatc tcgatcggcg gcggctcgcc gatggacgcc gccaaggtga tgcacctgct 4140

gtacgagtac ccggaggcgg agatcgagaa cctcgccatc aatttcatgg acatccgcaa 4200

gcgcatctgc aacttcccga agctgggcac caaggccatc tcggtggcca tcccgaccac 4260

cgccggcacc ggctcggagg ccaccccgtt cgccgtgatc accaacgacg agaccggcat 4320

gaagtacccg ctgacctcgt acgaactgac cccgaacatg gccatcattg acaccgagct 4380

gatgctgaac atgccgcgca agctgaccgc cgccaccggc attgatgccc tggtgcacgc 4440

catcgaggcc tacgtgtcgg tgatggccac ggactacacc gacgagctgg ccctgcgcgc 4500

catcaagatg atcttcaagt acctgccgcg cgcctacaag aacggcacca acgacatcga 4560

ggcccgcgag aagatggccc acgcctcgaa catcgccggc atggccttcg ccaacgcctt 4620

cctgggcgtg tgccactcga tggcccacaa gctgggcgcc atgcaccacg tgccgcacgg 4680

catcgcctgc gccgtgctga tcgaggaggt gatcaagtac aacgccaccg actgcccgac 4740

caagcagacc gccttcccgc agtacaagtc gccgaacgcc aagcgcaagt acgccgagat 4800

cgccgagtac ctgaacctga agggcacctc ggacacggag aaggtgaccg ccctgatcga 4860

ggccatctcg aagctgaaga tcgacctgtc gatcccgcag aacatctcgg ccgccggcat 4920

caacaagaag gatttctaca acaccctgga caagatgtcg gagctggcct tcgacgacca 4980

gtgcaccacc gccaacccgc gctacccgct gatctcggag ctgaaggaca tctacatcaa 5040

gtcgttctaa ggcacgcagg cacaatcagc ccggcccctg ccgggctgat tgttctcccc 5100

aagctt 5106

<210> 40
<211> 2512
<212> DNA
<213> Artificial Sequence

<220>

<223>  codonoptimized gen

<400>  40
ggtacctttc cattgaaagg actacacaat gactgacgtt gtcatcgtat ccgccgcccg      60

caccgcggtc ggcaagtttg gcggctcgct ggccaagatc ccggcaccgg aactgggtgc     120

cgtggtcatc aaggccgcgc tggagcgcgc cggcgtcaag ccggagcagg tgagcgaagt     180

catcatgggc caggtgctga ccgccggttc gggccagaac cccgcacgcc aggccgcgat     240

caaggccggc ctgccggcga tggtgccggc catgaccatc aacaaggtgt gcggctcggg     300

cctgaaggcc gtgatgctgg ccgccaacgc gatcatggcg ggcgacgccg agatcgtggt     360

ggccggcggc caggaaaaca tgagcgccgc cccgcacgtg ctgccgggct cgcgcgatgg     420

tttccgcatg ggcgatgcca agctggtcga caccatgatc gtcgacggcc tgtgggacgt     480

gtacaaccag taccacatgg gcatcaccgc cgagaacgtg gccaaggaat acggcatcac     540

acgcgaggcg caggatgagt tcgccgtcgg ctcgcagaac aaggccgaag ccgcgcagaa     600

ggccggcaag tttgacgaag agatcgtccc ggtgctgatc ccgcagcgca agggcgaccc     660

ggtggccttc aagaccgacg agttcgtgcg ccagggcgcc acgctggaca gcatgtccgg     720

cctcaagccc gccttcgaca aggccggcac ggtgaccgcg gccaacgcct cgggcctgaa     780

cgacggcgcc gccgcggtgg tggtgatgtc ggcggccaag gccaaggaac tgggcctgac     840

cccgctggcc acgatcaaga gctatgccaa cgccggtgtc gatcccaagg tgatgggcat     900

gggcccggtg ccggcctcca agcgcgccct gtcgcgcgcc gagtggaccc cgcaagacct     960

ggacctgatg gagatcaacg aggcctttgc cgcgcaggcg ctggcggtgc accagcagat    1020

gggctgggac acctccaagg tcaatgtgaa cggcggcgcc atcgccatcg gccacccgat    1080

cggcgcgtcg ggctgccgta tcctggtgac gctgctgcac gagatgaagc gccgtgacgc    1140

gaagaagggc ctggcctcgc tgtgcatcgg cggcggcatg ggcgtggcgc tggcagtcga    1200

gcgcaaataa ggaaggggtt ttccggggcc gcgcgcggtt ggcgcggacc cggcgacgat    1260

aacgaagcca atcaaggagt ggacatgact cagcgcattg cgtatgtgac cggcggcatg    1320

ggtggtatcg gaaccgccat ttgccagcgg ctggccaagg atggctttcg tgtggtggcc    1380

ggttgcggcc ccaactcgcc gcgccgcgaa aagtggctgg agcagcagaa ggccctgggc    1440

ttcgatttca ttgcctcgga aggcaatgtg gctgactggg actcgaccaa gaccgcattc    1500

gacaaggtca agtccgaggt cggcgaggtt gatgtgctga tcaacaacgc cggtatcacc    1560

cgcgacgtgg tgttccgcaa gatgacccgc gccgactggg atgcggtgat cgacaccaac    1620

ctgacctcgc tgttcaacgt caccaagcag gtgatcgacg gcatggccga ccgtggctgg    1680

ggccgcatcg tcaacatctc gtcggtgaac gggcagaagg gccagttcgg ccagaccaac    1740

tactccaccg ccaaggccgg cctgcatggc ttcaccatgg cactggcgca ggaagtggcg    1800

accaagggcg tgaccgtcaa cacggtctct ccgggctata tcgccaccga catggtcaag 1860

gcgatccgcc aggacgtgct cgacaagatc gtcgcgacga tcccggtcaa gcgcctgggc 1920

ctgccggaag agatcgcctc gatctgcgcc tggttgtcgt cggaggagtc cggtttctcg 1980

accggcgccg acttctcgct caacggcggc ctgcatatgg gctgaaacag aggaggacgc 2040

cgcatgtcgg cccagtcgct ggaggtcggg caaaaagccc ggctgtccaa gcggtttggc 2100

gcggcggagg tggcggcctt tgcggcgctc tccgaggact tcaacccgct gcacctggac 2160

cccgcgtttg ccgcgaccac cgcgttcgag cgccccatcg tgcatggcat gctgctcgcc 2220

tcgctgttct cgggcctcct cggccagcag ctccccggca aggggagcat ctacctgggc 2280

caatcgctct cgttcaaact gccggtgttc gtgggggacg aagtgaccgc cgaggtggaa 2340

gtcaccgcgc tgcgcgagga caaaccgatc gccacgctga cgacccggat ctttacccaa 2400

ggcggggccc tggccgtcac cggggaagcc gtggtcaagc tgccgtaagc accggcggca 2460

cgcaggcaca atcagcccgg cccctgccgg gctgattgtt ctccccaagc tt 2512

<210> 41
<211> 282
<212> PRT
<213> Clostridium acetobutylicum

<400> 41

Met Lys Lys Val Cys Val Ile Gly Ala Gly Thr Met Gly Ser Gly Ile
1               5                   10                  15

Ala Gln Ala Phe Ala Ala Lys Gly Phe Glu Val Val Leu Arg Asp Ile
            20                  25                  30

Lys Asp Glu Phe Val Asp Arg Gly Leu Asp Phe Ile Asn Lys Asn Leu
        35                  40                  45

Ser Lys Leu Val Lys Lys Gly Lys Ile Glu Glu Ala Thr Lys Val Glu
    50                  55                  60

Ile Leu Thr Arg Ile Ser Gly Thr Val Asp Leu Asn Met Ala Ala Asp
65                  70                  75                  80

Cys Asp Leu Val Ile Glu Ala Ala Val Glu Arg Met Asp Ile Lys Lys
                85                  90                  95

Gln Ile Phe Ala Asp Leu Asp Asn Ile Cys Lys Pro Glu Thr Ile Leu
            100                 105                 110

Ala Ser Asn Thr Ser Ser Leu Ser Ile Thr Glu Val Ala Ser Ala Thr
        115                 120                 125

Lys Thr Asn Asp Lys Val Ile Gly Met His Phe Phe Asn Pro Ala Pro
    130                 135                 140

Val Met Lys Leu Val Glu Val Ile Arg Gly Ile Ala Thr Ser Gln Glu
145                 150                 155                 160

Thr Phe Asp Ala Val Lys Glu Thr Ser Ile Ala Ile Gly Lys Asp Pro
                165                 170                 175

Val Glu Val Ala Glu Ala Pro Gly Phe Val Val Asn Arg Ile Leu Ile
            180                 185                 190

Pro Met Ile Asn Glu Ala Val Gly Ile Leu Ala Glu Gly Ile Ala Ser
        195                 200                 205

Val Glu Asp Ile Asp Lys Ala Met Lys Leu Gly Ala Asn His Pro Met
    210                 215                 220

Gly Pro Leu Glu Leu Gly Asp Phe Ile Gly Leu Asp Ile Cys Leu Ala
225                 230                 235                 240

Ile Met Asp Val Leu Tyr Ser Glu Thr Gly Asp Ser Lys Tyr Arg Pro
                245                 250                 255

His Thr Leu Leu Lys Lys Tyr Val Arg Ala Gly Trp Leu Gly Arg Lys
            260                 265                 270

Ser Gly Lys Gly Phe Tyr Asp Tyr Ser Lys
        275                 280

<210>  42
<211>  66
<212>  DNA
<213>  Clostridium acetobutylicum

<400>  42
actatgaaac aatattaaaa aaataagagt taccatttaa ggtaactctt atttttatta      60

cttaag                                                                  66

<210>  43
<211>  4742
<212>  DNA
<213>  Artificial Sequence

<220>
<223>  codonoptimized gen

<400>  43
ggtacctttc aggagattca agaatgaagg aggtggtgat tgcctcggcc gtgcgcaccg      60

cgatcggctc gtacggcaag agcctgaaag acgtgcccgc cgtggacctg ggcgccaccg 120

ccattaagga ggccgtgaag aaggccggca tcaagccgga ggacgtgaac gaggtgatcc 180

tgggcaacgt gctgcaggcc gggctcggcc agaacccggc ccgccaggcg agcttcaaag 240

ccggcctccc ggtggagatc cccgccatga ccatcaacaa ggtgtgcggc tcgggcctgc 300

gcaccgtgtc gctggcggcc cagatcatca aggccggcga cgccgacgtg atcattgccg 360

gcggcatgga aaacatgtcg cgcgccccgt acctggccaa caacgcccgc tggggctacc 420

gcatggggaa cgccaagttc gtggacgaga tgatcaccga cggcctgtgg gacgccttca 480

acgactacca catgggcatc accgccgaga acatcgccga gcgctggaac atctcgcgcg 540

aggagcagga cgagttcgcc ctggcctcgc aaaagaaggc ggaggaagcc atcaagtcgg 600

gccagttcaa ggacgagatc gtgccggtgg tgattaaggg ccgcaagggc gagaccgtgg 660

tggacacgga cgagcacccg cgcttcgggt cgacgatcga gggcctggcc aagctgaagc 720

cggccttcaa gaaggacggc accgtgaccg cgggcaacgc ctcgggcctg aacgactgcg 780

cggccgtgct ggtgatcatg tcggccgaaa aggccaagga gctgggcgtg aaaccgctgg 840

cgaagatcgt gtcgtatggc tcggccggcg tggacccggc catcatgggc tacggcccgt 900

tctacgccac caaggcggcg atcgagaaag ccggctggac ggtcgacgag ctggacctga 960

tcgagtcgaa cgaggccttt gccgcccagt cgctggccgt cgccaaggac ctgaagttcg 1020

acatgaacaa agtgaacgtg aatggcggcg ccatcgccct ggggcacccg atcggcgcct 1080

cgggcgcccg catcctggtg accctggtgc acgcgatgca gaagcgcgac gccaagaagg 1140

ggctggcgac cctgtgcatc ggcggcggcc agggcaccgc gatcctgctg gaaaagtgct 1200

agtttcagga gattcaagaa tgaagaaggt gtgcgtcatt ggcgcgggga cgatggggtc 1260

cggcatcgcg caggccttcg ccgccaaagg cttcgaggtg gtcctccggg acatcaagga 1320

tgaattcgtc gatcgcggcc tggatttcat taacaagaac ctgtcgaaac tggtcaaaaa 1380

aggcaagatc gaagaggcca ccaaggtgga gattctcacc cgcatcagcg ggaccgtgga 1440

cctgaacatg gccgcggact gcgacctcgt gattgaagcc gccgtcgagc gcatggacat 1500

taagaaacag atctttgcgg atctcgacaa catctgcaaa ccggagacga ttctcgcctc 1560

caacaccagc tcgctctcga tcacggaggt ggcgagcgcg accaagacca acgacaaagt 1620

gatcggcatg catttcttta atcccgcccc cgtcatgaaa ctcgtggagg tcattcgcgg 1680

cattgccacg tcgcaggaaa ccttcgacgc ggtgaaagag acgtcgattg ccattggcaa 1740

ggaccccgtg gaagtcgccg aggcgccggg gttcgtcgtc aaccgcattc tgatcccgat 1800

gatcaatgaa gcggtgggca tcctcgccga agggattgcc tcggtcgagg atattgataa 1860

ggccatgaag ctgggggcga accacccgat gggcccgctg gaactggggg actttatcgg 1920

cctcgacatt tgcctggcca tcatggacgt gctgtactcg gaaaccggcg attcgaagta 1980

tcgcccgcac acgctgctga aaaagtatgt ccgggcgggc tggctgggcc gcaagagcgg 2040

caagggcttt tacgactact cgaaataaaa agaaattttc aggagattca agaatgaagg 2100

tgaccaacca gaaggagctg aagcagaagc tgaacgagct gcgcgaggcc cagaagaagt 2160

tcgccaccta cacccaggag caggtggaca agatcttcaa gcagtgcgcc attgccgccg 2220

ccaaggagcg catcaacctg gccaagctgg ccgtggagga gaccggcatc ggcctggtgg 2280

aggacaagat catcaagaac cacttcgccg ccgagtacat ctacaacaag tacaagaacg 2340

agaagacctg cggcattatc gaccacgacg actcgctggg catcaccaag gtggccgagc 2400

cgatcggcat cgtggccgcc atcgtgccga ccacgaaccc gacctcgacc gccatcttca 2460

agtcgctgat ctcgctgaag acccgcaacg ccatcttctt ctcgccgcac ccgcgcgcca 2520

agaagtcgac catcgccgcc gccaagctga tcctggacgc cgccgtgaag gccggggccc 2580

cgaagaacat catcggctgg attgacgagc cgtcgatcga gctgtcgcag gacctgatgt 2640

cggaggccga tatcatcctg gccaccggcg gcccgtcgat ggtgaaggcc gcctactcgt 2700

cgggcaagcc ggccatcggc gtgggcgcgg gcaacacccc ggccatcatc gacgagtccg 2760

ccgacatcga tatggccgtg tcgtcgatca tcctgtcgaa gacctacgac aacggcgtga 2820

tctgcgcctc ggagcagtcg atcctggtga tgaactccat ctacgagaag gtgaaggagg 2880

agttcgtgaa gcgcggctcg tacatcctga accagaacga gatcgccaag atcaaggaga 2940

cgatgttcaa gaacggcgcc atcaacgccg acatcgtggg caagtcggcc tacatcatcg 3000

ccaagatggc cggcatcgag gtgccgcaga ccaccaagat cctcatcggc gaggtgcagt 3060

cggtggagaa gtcggagctg ttctcgcacg agaagctgtc gccggtgctg gccatgtaca 3120

aggtgaagga cttcgatgag gccctcaaga aggcccagcg cctgatcgag ctgggcggct 3180

cgggccacac ctcgtcgctg tacatcgact cgcagaacaa caaggacaag gtgaaggagt 3240

tcggcctggc catgaagacc tcgcgcacct tcatcaacat gccgtcgtcg cagggcgcct 3300

cgggcgacct gtacaacttc gccatcgccc cgtcgttcac cctgggctgc gggacctggg 3360

gcggcaactc ggtgtcgcag aacgtggagc cgaagcacct gctgaacatc aagtcggtgg 3420

ccgagcgccg cgagaatatg ctgtggttca aggtgccgca gaagatctac ttcaagtacg 3480

gctgcctgcg cttcgccctg aaggagctga aggacatgaa caagaagcgc gccttcatcg 3540

tgaccgacaa ggacctgttc aagctgggct acgtgaacaa gatcaccaag gtgctggacg 3600

agatcgacat caagtactcg atcttcaccg atatcaagtc ggacccgacc attgactcgg 3660

tgaagaaggg cgccaaggag atgctgaact tcgagccgga caccatcatc tcgatcggcg 3720

gcggctcgcc gatggacgcc gccaaggtga tgcacctgct gtacgagtac ccggaggcgg 3780

agatcgagaa cctcgccatc aatttcatgg acatccgcaa gcgcatctgc aacttcccga 3840

agctgggcac caaggccatc tcggtggcca tcccgaccac cgccggcacc ggctcggagg 3900

ccaccccgtt cgccgtgatc accaacgacg agaccggcat gaagtacccg ctgacctcgt 3960

acgaactgac cccgaacatg gccatcattg acaccgagct gatgctgaac atgccgcgca 4020

agctgaccgc cgccaccggc attgatgccc tggtgcacgc catcgaggcc tacgtgtcgg 4080

tgatggccac ggactacacc gacgagctgg ccctgcgcgc catcaagatg atcttcaagt 4140

acctgccgcg cgcctacaag aacggcacca acgacatcga ggcccgcgag aagatggccc 4200

acgcctcgaa catcgccggc atggccttcg ccaacgcctt cctgggcgtg tgccactcga 4260

tggcccacaa gctgggcgcc atgcaccacg tgccgcacgg catcgcctgc gccgtgctga 4320

tcgaggaggt gatcaagtac aacgccaccg actgcccgac caagcagacc gccttcccgc 4380

agtacaagtc gccgaacgcc aagcgcaagt acgccgagat cgccgagtac ctgaacctga 4440

agggcacctc ggacacggag aaggtgaccg ccctgatcga ggccatctcg aagctgaaga 4500

tcgacctgtc gatcccgcag aacatctcgg ccgccggcat caacaagaag gatttctaca 4560

acaccctgga caagatgtcg gagctggcct tcgacgacca gtgcaccacc gccaacccgc 4620

gctacccgct gatctcggag ctgaaggaca tctacatcaa gtcgttctaa actatgaaac 4680

aatattaaaa aaataagagt taccatttaa ggtaactctt atttttatta cttaagaagc 4740

tt 4742

<210> 44
<211> 2148
<212> DNA
<213> Artificial Sequence

<220>
<223> codonoptimized gen

<400> 44
ggtacctttc aggagattca agaatgaagg aggtggtgat tgcctcggcc gtgcgcaccg 60

cgatcggctc gtacggcaag agcctgaaag acgtgcccgc cgtggacctg ggcgccaccg 120

ccattaagga ggccgtgaag aaggccggca tcaagccgga ggacgtgaac gaggtgatcc 180

tgggcaacgt gctgcaggcc gggctcggcc agaacccggc ccgccaggcg agcttcaaag 240

ccggcctccc ggtggagatc cccgccatga ccatcaacaa ggtgtgcggc tcgggcctgc 300

gcaccgtgtc gctggcggcc cagatcatca aggccggcga cgccgacgtg atcattgccg 360

gcggcatgga aaacatgtcg cgcgccccgt acctggccaa caacgcccgc tggggctacc 420

gcatggggaa cgccaagttc gtggacgaga tgatcaccga cggcctgtgg gacgccttca 480

acgactacca catgggcatc accgccgaga acatcgccga gcgctggaac atctcgcgcg 540

aggagcagga cgagttcgcc ctggcctcgc aaaagaaggc ggaggaagcc atcaagtcgg 600

gccagttcaa ggacgagatc gtgccggtgg tgattaaggg ccgcaagggc gagaccgtgg 660

tggacacgga cgagcacccg cgcttcgggt cgacgatcga gggcctggcc aagctgaagc 720

cggccttcaa gaaggacggc accgtgaccg cgggcaacgc ctcgggcctg aacgactgcg 780

cggccgtgct ggtgatcatg tcggccgaaa aggccaagga gctgggcgtg aaaccgctgg 840

cgaagatcgt gtcgtatggc tcggccggcg tggacccggc catcatgggc tacggcccgt 900

tctacgccac caaggcggcg atcgagaaag ccggctggac ggtcgacgag ctggacctga 960

tcgagtcgaa cgaggccttt gccgcccagt cgctggccgt cgccaaggac ctgaagttcg 1020

acatgaacaa agtgaacgtg aatggcggcg ccatcgccct ggggcacccg atcggcgcct 1080

cgggcgcccg catcctggtg accctggtgc acgcgatgca gaagcgcgac gccaagaagg 1140

ggctggcgac cctgtgcatc ggcggcggcc agggcaccgc gatcctgctg gaaaagtgct 1200

agtttcagga gattcaagaa tgaagaaggt gtgcgtcatt ggcgcgggga cgatggggtc 1260

cggcatcgcg caggccttcg ccgccaaagg cttcgaggtg gtcctccggg acatcaagga 1320

tgaattcgtc gatcgcggcc tggatttcat taacaagaac ctgtcgaaac tggtcaaaaa 1380

aggcaagatc gaagaggcca ccaaggtgga gattctcacc cgcatcagcg ggaccgtgga 1440

cctgaacatg gccgcggact gcgacctcgt gattgaagcc gccgtcgagc gcatggacat 1500

taagaaacag atctttgcgg atctcgacaa catctgcaaa ccggagacga ttctcgcctc 1560

caacaccagc tcgctctcga tcacggaggt ggcgagcgcg accaagacca acgacaaagt 1620

gatcggcatg catttcttta atcccgcccc cgtcatgaaa ctcgtggagg tcattcgcgg 1680

cattgccacg tcgcaggaaa ccttcgacgc ggtgaaagag acgtcgattg ccattggcaa 1740

ggaccccgtg gaagtcgccg aggcgccggg gttcgtcgtc aaccgcattc tgatcccgat 1800

gatcaatgaa gcggtgggca tcctcgccga agggattgcc tcggtcgagg atattgataa 1860

ggccatgaag ctgggggcga accacccgat gggcccgctg gaactggggg actttatcgg 1920

cctcgacatt tgcctggcca tcatggacgt gctgtactcg gaaaccggcg attcgaagta 1980

tcgcccgcac acgctgctga aaaagtatgt ccgggcgggc tggctgggcc gcaagagcgg 2040

caagggcttt tacgactact cgaaataaaa agaaatacta tgaaacaata ttaaaaaaat 2100

aagagttacc atttaaggta actcttattt ttattactta agaagctt 2148

<210> 45
<211> 249
<212> PRT
<213> Ralstonia eutropha

<400> 45

Met Lys Val Leu Val Ala Val Lys Arg Val Val Asp Tyr Asn Val Lys
1               5                   10                  15

Val Arg Val Lys Ala Asp Gly Ser Gly Val Asp Leu Ala Asn Val Lys
            20                  25                  30

Met Ser Met Asn Pro Phe Asp Glu Ile Ala Val Glu Glu Ala Val Arg
        35                  40                  45

Leu Lys Glu Ala Gly Val Val Thr Glu Val Ile Ala Val Ser Cys Gly
    50                  55                  60

Val Thr Gln Cys Gln Glu Thr Leu Arg Thr Ala Met Ala Ile Gly Ala
65                  70                  75                  80

Asp Arg Gly Ile Leu Val Glu Ser Asn Glu Asp Leu Gln Pro Leu Ala
                85                  90                  95

Val Ala Lys Leu Leu Lys Ala Leu Ile Asp Lys Glu Gln Pro Gln Leu
            100                 105                 110

Val Ile Leu Gly Lys Gln Ala Ile Asp Asp Asp Ser Asn Gln Thr Gly
        115                 120                 125

Gln Met Val Ala Ala Leu Ala Gly Leu Pro Gln Ala Thr Phe Ala Ser
    130                 135                 140

Lys Val Val Val Ala Asp Gly Lys Ala Ser Val Thr Arg Glu Val Asp
145                 150                 155                 160

Gly Gly Leu Glu Thr Leu Ser Leu Lys Leu Pro Ala Val Val Thr Thr
                165                 170                 175

Asp Leu Arg Leu Asn Glu Pro Arg Tyr Val Thr Leu Pro Asn Ile Met
            180                 185                 190

Lys Ala Lys Lys Lys Gln Leu Asp Ile Val Lys Pro Glu Asp Leu Gly
        195                 200                 205

Val Asp Val Lys Pro Arg Leu Ser Thr Leu Lys Val Val Glu Pro Ala
    210                 215                 220

Lys Arg Ser Ala Gly Val Met Val Pro Asp Val Ala Thr Leu Val Gln
225                 230                 235                 240

Lys Leu Lys Asn Glu Ala Lys Val Ile
                245

<210> 46
<211> 311
<212> PRT

<213>  Ralstonia eutropha

<400>  46

Met Thr Ala Leu Val Ile Ala Glu His Asp Asn Gln Ser Ile Lys Ala
1               5                   10                  15

Ala Thr Leu Asn Thr Val Thr Ala Ala Ala Gln Cys Gly Gly Asp Val
            20                  25                  30

His Val Leu Val Ala Gly Ala Asn Ala Lys Ala Ala Ala Asp Ala Ala
        35                  40                  45

Ala Lys Ile Ala Gly Val Thr Lys Val Leu Leu Ala Asp Ala Pro Tyr
    50                  55                  60

Phe Gly Asp Gly Leu Ala Glu Asn Val Ala Glu Gln Ala Leu Ala Ile
65                  70                  75                  80

Ala Asn Asp Tyr Ser His Ile Leu Ala Pro Ala Thr Pro Tyr Gly Lys
                85                  90                  95

Asn Ile Leu Pro Arg Val Ala Ala Lys Leu Asp Val Ala Gln Ile Ser
            100                 105                 110

Glu Ile Ser Lys Val Asp Ala Pro Asp Thr Phe Glu Arg Pro Ile Tyr
        115                 120                 125

Ala Gly Asn Ala Ile Ala Thr Val Lys Ser Glu Asp Lys Ile Lys Val
    130                 135                 140

Ile Thr Val Arg Gly Thr Ala Phe Asp Ala Ala Ala Ala Glu Gly Gly
145                 150                 155                 160

Ser Ala Ala Val Glu Thr Leu Pro Ala Val Ala Asp Ala Gly Val Ser
                165                 170                 175

Gln Phe Val Ser Arg Glu Val Ala Lys Ser Asp Arg Pro Glu Leu Thr
            180                 185                 190

Ala Ala Lys Ile Ile Val Ser Gly Gly Arg Gly Val Gly Ser Gly Glu
        195                 200                 205

Asn Tyr Thr Lys Val Leu Thr Pro Leu Ala Asp Lys Leu Gly Ala Ala
    210                 215                 220

Leu Gly Ala Ser Arg Ala Ala Val Asp Ala Gly Phe Val Pro Asn Asp
225                 230                 235                 240

Tyr Gln Val Gly Gln Thr Gly Lys Ile Val Ala Pro Gln Leu Tyr Ile
                245                 250                 255

Ala Val Gly Ile Ser Gly Ala Ile Gln His Leu Ala Gly Met Lys Asp
            260                 265                 270

Ser Lys Val Ile Val Ala Ile Asn Lys Asp Ala Glu Ala Pro Ile Phe
        275                 280                 285

Ser Val Ala Asp Tyr Gly Leu Val Gly Asp Leu Asn Thr Val Val Pro
    290                 295                 300

Glu Leu Val Ala Ala Leu Gly
305                 310

<210> 47
<211> 594
<212> PRT
<213> Ralstonia eutropha

<400> 47

Met Thr Tyr Arg Ala Pro Ile Lys Asp Met Leu Phe Val Met Asn Glu
1               5                   10                  15

Leu Ala Gly Leu Glu Ala Val Ser Lys Leu Pro Gly Phe Glu Glu Ala
            20                  25                  30

Thr Pro Glu Thr Ala Glu Ala Val Leu Asp Glu Ala Ala Lys Phe Asn
        35                  40                  45

Glu Gln Val Val Ala Pro Leu Asn Arg Ala Gly Asp Leu Asp Pro Ser
    50                  55                  60

Ser Trp Lys Asp Gly Val Val Thr Thr Thr Pro Gly Phe Lys Glu Ala
65                  70                  75                  80

Phe Arg Gln Phe Gly Glu Gly Gly Trp Gln Gly Val Leu His Pro Gln
                85                  90                  95

Glu Phe Gly Gly Gln Gly Leu Pro Lys Leu Val Ala Thr Ala Cys Asn
            100                 105                 110

Glu Met Leu Asn Thr Ala Asn Leu Ser Phe Ala Leu Cys Pro Leu Leu
        115                 120                 125

Thr Asp Gly Ala Ile Glu Ala Leu Leu Thr Ala Gly Thr Asp Glu Gln
    130                 135                 140

Lys Ala Thr Phe Leu Pro Lys Leu Ile Ser Gly Glu Trp Thr Gly Thr
145                 150                 155                 160

Met Asn Leu Thr Glu Pro Gln Ala Gly Ser Asp Leu Ala Ala Val Arg
                165                 170                 175

Thr Arg Ala Glu Pro Gln Gly Asp Gly Thr Tyr Lys Val Phe Gly Thr
            180                 185                 190

Lys Ile Phe Ile Thr Tyr Gly Glu His Asp Met Ala Lys Asn Ile Val
        195                 200                 205

His Leu Val Leu Ala Arg Thr Pro Thr Ala Pro Glu Gly Val Lys Gly
    210                 215                 220

Ile Ser Leu Phe Ile Val Pro Lys Phe Met Val Asn Ala Asp Gly Ser
225                 230                 235                 240

Thr Gly Glu Arg Asn Asp Val His Cys Val Ser Ile Glu His Lys Leu
                245                 250                 255

Gly Ile Lys Ala Ser Pro Thr Ala Val Leu Gln Phe Gly Asp His Gly
            260                 265                 270

Gly Ala Ile Gly Thr Leu Val Gly Glu Glu Asn Arg Gly Leu Glu Tyr
        275                 280                 285

Met Phe Ile Met Met Asn Ser Ala Arg Phe Ser Val Gly Met Gln Gly
    290                 295                 300

Ile Ala Val Ser Glu Arg Ala Tyr Gln Gln Ala Val Ala Phe Ala Arg
305                 310                 315                 320

Glu Arg Val Gln Ser Arg Pro Val Asp Gly Ser Ala Arg Glu Ala Val
                325                 330                 335

Thr Ile Ile His His Pro Asp Val Lys Arg Met Leu Met Thr Met Arg
            340                 345                 350

Ala Leu Thr Glu Gly Ala Arg Ala Val Ala Tyr Val Ala Ala Ala Ala
        355                 360                 365

Ser Asp Ala Ala His Gln His Pro Asp Glu Ala Val Arg Gln Gln Asn
    370                 375                 380

Gln Ala Phe Tyr Glu Phe Leu Val Pro Val Val Lys Gly Trp Ser Thr
385                 390                 395                 400

Glu Leu Ser Ile Asp Val Thr Ser Leu Gly Val Gln Val His Gly Gly
                405                 410                 415

Met Gly Phe Ile Glu Glu Thr Gly Ala Ala Gln His Tyr Arg Asp Ala
            420                 425                 430

Arg Ile Leu Pro Ile Tyr Glu Gly Thr Thr Ala Ile Gln Ala Asn Asp
        435                 440                 445

Leu Ile Gly Arg Lys Thr Val Arg Asp Gly Gly Ala Val Ala Arg Ala
    450                 455                 460

Ile Cys Ala Gln Ile Ala Glu Thr Glu Ala Ala Leu Gly Asn His Ser
465                 470                 475                 480

Cys Ala Gly Phe Thr Ala Val Gln Ala Gln Leu Ala Lys Gly Arg Ala
                485                 490                 495

Ala Leu Glu Asp Val Val Ala Phe Val Val Ala Asn Ala Lys Ser Asp
            500                 505                 510

Pro Asn Ala Val Phe Ala Gly Ser Val Pro Tyr Leu Lys Leu Cys Gly
        515                 520                 525

Ile Val Phe Ser Gly Trp Gln Phe Gly Arg Ala Met Leu Ala Ala Asp
    530                 535                 540

Ala Lys Arg Ala Gly Asp Pro Gly Phe Tyr Asp Ala Lys Ile Ala Thr
545                 550                 555                 560

Ala His Phe Phe Ala Glu His Ile Leu Ser Gln Ala Ser Ala Leu Arg
                565                 570                 575

Asp Ala Ile Val Ser Gly Ala Ala Pro Val Asn Ala Met Thr Ala Glu
            580                 585                 590

Gln Phe

<210>  48
<211>  75
<212>  DNA
<213>  Ralstonia eutropha

<400>  48
gtggcctgac agtcgccgga aagaaaaagg gcggccccgc ggggccgccc tttttgctta      60

tcgaggcagc ctgct                                                       75

<210>  49
<211>  3762
<212>  DNA
<213>  Artificial Sequence

<220>
<223>  synthetic gen

<400>  49
aagcttcccc aggctttaca ctttatgctt ccggctcgta tgttgtgtgg aattgtgagc      60

ggataacaat ttcacatatc agtggagtga gcgcatgaaa gtactcgtcg cagtcaagcg     120

ggtggtggat tacaacgtca aggtccgcgt caaggcggac ggcagcggcg tcgatctggc     180

caacgtgaag atgagcatga accccttcga cgaaatcgcc gtggaagagg ccgtgcgcct     240

gaaggaagcc ggtgttgtca ccgaagtgat cgccgtctcg tgcggtgtca cgcagtgcca     300

ggaaaccctg cgcaccgcga tggccatcgg tgccgaccgc ggcatcctgg tggaatcgaa     360

cgaagacctg caaccgctgg ccgtggccaa gctgctgaag gcgctgatcg acaaggaaca     420

gccgcaactg gtgatcctgg gcaagcaggc catcgacgac gactccaacc agaccggcca     480

gatggtggcc gcgctggctg gcctgccgca agccacgttc gcctccaagg tggtggttgc     540

cgacggcaag gcctcggtga cgcgtgaagt cgatggcggc ctggaaacgc tgtcgctcaa     600

gctgccggcc gtggtgacga ccgacctgcg cctgaacgag ccgcgctacg tgacgctgcc     660

gaacatcatg aaggccaaga agaagcagct cgatatcgtc aagccggaag acctcggcgt     720

cgacgtcaag ccgcgcctgt cgaccctgaa ggtggtcgag cctgccaagc gcagcgcggg     780

tgtgatggtg ccggacgttg cgacgctggt gcagaagctg aagaacgaag ccaaggttat     840

ctgagcgcgg gggagaaata acatgactgc actcgtcatt gctgaacacg acaatcaatc     900

catcaaggcc gccacgctga acaccgtgac tgccgctgcc cagtgcggcg gcgacgtgca     960

cgtgctggtg gctggtgcca acgccaaggc cgcggccgat gcggccgcca agatcgccgg    1020

cgtgaccaag gtcctgctgg ccgacgcccc gtacttcggt gacggcctgg ccgagaacgt    1080

ggccgagcag gcgctggcca tcgccaacga ctactcgcat atcctggctc cggccacccc    1140

gtacggcaag aacatcctgc cgcgcgtggc tgccaagctg gacgtggccc agatctcgga    1200

aatctccaag gtcgacgccc cggacacgtt cgagcgcccg atctacgccg gcaacgccat    1260

cgccacggtc aagtcggaag acaagatcaa ggtcatcacc gtgcgcggca ccgcctttga    1320

cgccgccgcc gccgaaggtg gctcggctgc cgtcgagacc ctgccggccg tggccgacgc    1380

aggcgtttcg cagtttgttt cgcgcgaagt ggccaagagc gaccgtccgg aactgaccgc    1440

cgccaagatc atcgtctcgg gtggccgtgg cgtgggttcg ggcgagaact acaccaaggt    1500

gctgacgccg ctggccgaca agctgggcgc cgcgctgggt gcctcgcgcg ccgccgtgga    1560

cgccggcttc gtgccgaacg actaccaggt cggccagacc ggcaagatcg tcgcgccgca 1620

gctgtatatc gccgtcggta tctccggcgc gatccagcac ctggccggca tgaaggactc 1680

caaggtgatc gttgcgatca acaaggatgc cgaagccccg atcttctccg tggccgacta 1740

cggcctggta ggcgacctga acaccgtggt gccggagctg gtggcagcac tgggctgatg 1800

ccttgccggc cgccaggcgg ccggcactgg tagcaagtac gagccgttcc gggggtgacc 1860

cgcgaacggc tttttcatac atgttgggag acatcgatga cttaccgtgc gccgatcaag 1920

gacatgctgt tcgtcatgaa cgagctggcc gggctagagg ccgtgagcaa actgccgggc 1980

ttcgaggaag ccacgccgga aacggccgag gcagtgctgg acgaggccgc caagttcaac 2040

gagcaggtcg tcgcgccgct gaaccgcgcc ggcgacctgg acccgagcag ctggaaggac 2100

ggcgtggtca ccaccacgcc cggctttaag gaggccttcc gccagttcgg cgagggcggc 2160

tggcagggcg tgctgcatcc gcaggagttt ggcggccagg gcctgcccaa gctggtcgcc 2220

accgcctgca acgagatgct gaacaccgcg aacctgtcgt tcgcgctgtg cccgctgctg 2280

accgatggtg ccatcgaagc gctgctgacg gccggcacgg atgagcagaa ggccaccttc 2340

ctgcccaagc tgatctcggg cgagtggacc ggcaccatga acctgaccga gccgcaggcc 2400

ggctcggatc tggccgcagt gcgcacccgt gccgagccgc agggcgacgg cacctacaaa 2460

gtgttcggca ccaagatctt catcacctac ggcgagcacg acatggcgaa gaacatcgtc 2520

catctcgtgc tggcccgcac gcccaccgcg cccgagggcg tcaagggcat ctcgctgttc 2580

atcgtgccga agttcatggt caacgccgac ggcagcaccg gtgagcgcaa cgacgtgcat 2640

tgcgtctcga tcgagcacaa gctgggcatc aaggcgagcc ccacggccgt gctgcagttc 2700

ggcgaccatg gcggcgccat cggcacgctg gtcggcgaag agaaccgtgg cctcgagtac 2760

atgttcatca tgatgaactc ggcacgcttc tcggtcggca tgcagggcat cgccgtatcc 2820

gaacgcgcct accagcaggc ggtggccttt gcgcgcgagc gcgtgcagag ccgcccggtc 2880

gatggttcgg cgcgcgaggc ggtgaccatc atccaccacc ctgacgtcaa gcgcatgctg 2940

atgaccatgc gcgcgctgac cgaaggcgcg cgcgccgtgg cctacgtggc cgcggccgcc 3000

agcgacgcgg cgcaccagca tcccgacgaa gcggtgcgcc agcagaacca ggccttctac 3060

gagttcctgg tgccggtggt gaaaggctgg agcaccgagc tgtcgatcga cgtcaccagc 3120

ctgggtgtgc aggtgcacgg cggcatgggc tttatcgagg agaccggcgc ggcgcagcac 3180

taccgcgacg cgcgtatcct gccgatctac gaaggcacca cggcaatcca ggccaacgac 3240

ctgatcggcc gcaagaccgt gcgcgacggc ggcgccgtgg cgcgtgcgat ctgcgcgcag 3300

atcgccgaga ccgaggctgc gctgggcaat cacagctgcg ccgggttcac cgcggtacag 3360

gcgcaactgg ccaaggtcgc gcggcgctg gaagacgtgg tggcgtttgt cgtggccaat 3420

gccaagtccg acccgaacgc cgtctttgcc ggcagcgtgc cttacctgaa gctgtgcggc 3480

atcgtgttct ccggctggca gttcggccgt gcgatgctgg ccgccgatgc gaagcgggcc 3540

ggggacccgg gcttttatga cgccaagatc gccaccgcgc atttctttgc ggagcacatc 3600

ctgtcgcagg cgtcggcttt gcgcgatgcc atcgtcagtg gtgcggcacc ggtgaatgcg 3660

atgacggccg agcagttctg agtggcctga cagtcgccgg aaagaaaaag ggcggccccg 3720

cggggccgcc ctttttgctt atcgaggcag cctgctggat cc 3762

<210> 50
<211> 261
<212> PRT
<213> Clostridium acetobutylicum

<400> 50

Met Glu Leu Asn Asn Val Ile Leu Glu Lys Glu Gly Lys Val Ala Val
1               5                   10                  15

Val Thr Ile Asn Arg Pro Lys Ala Leu Asn Ala Leu Asn Ser Asp Thr
            20                  25                  30

Leu Lys Glu Met Asp Tyr Val Ile Gly Glu Ile Glu Asn Asp Ser Glu
        35                  40                  45

Val Leu Ala Val Ile Leu Thr Gly Ala Gly Glu Lys Ser Phe Val Ala
    50                  55                  60

Gly Ala Asp Ile Ser Glu Met Lys Glu Met Asn Thr Ile Glu Gly Arg
65                  70                  75                  80

Lys Phe Gly Ile Leu Gly Asn Lys Val Phe Arg Arg Leu Glu Leu Leu
                85                  90                  95

Glu Lys Pro Val Ile Ala Ala Val Asn Gly Phe Ala Leu Gly Gly Gly
            100                 105                 110

Cys Glu Ile Ala Met Ser Cys Asp Ile Arg Ile Ala Ser Ser Asn Ala
        115                 120                 125

Arg Phe Gly Gln Pro Glu Val Gly Leu Gly Ile Thr Pro Gly Phe Gly
    130                 135                 140

Gly Thr Gln Arg Leu Ser Arg Leu Val Gly Met Gly Met Ala Lys Gln
145                 150                 155                 160

Leu Ile Phe Thr Ala Gln Asn Ile Lys Ala Asp Glu Ala Leu Arg Ile
                165                 170                 175

Gly Leu Val Asn Lys Val Val Glu Pro Ser Glu Leu Met Asn Thr Ala
            180                 185                 190

Lys Glu Ile Ala Asn Lys Ile Val Ser Asn Ala Pro Val Ala Val Lys
        195                 200                 205

Leu Ser Lys Gln Ala Ile Asn Arg Gly Met Gln Cys Asp Ile Asp Thr
    210                 215                 220

Ala Leu Ala Phe Glu Ser Glu Ala Phe Gly Glu Cys Phe Ser Thr Glu
225                 230                 235                 240

Asp Gln Lys Asp Ala Met Thr Ala Phe Ile Glu Lys Arg Lys Ile Glu
                245                 250                 255

Gly Phe Lys Asn Arg
            260

<210>  51
<211>  252
<212>  PRT
<213>  Clostridium acetobutylicum

<400>  51

Met Asn Ile Val Val Cys Leu Lys Gln Val Pro Asp Thr Ala Glu Val
1               5                   10                  15

Arg Ile Asp Pro Val Lys Gly Thr Leu Ile Arg Glu Gly Val Pro Ser
            20                  25                  30

Ile Ile Asn Pro Asp Asp Lys Asn Ala Leu Glu Glu Ala Leu Val Leu
        35                  40                  45

Lys Asp Asn Tyr Gly Ala His Val Thr Val Ile Ser Met Gly Pro Pro
    50                  55                  60

Gln Ala Lys Asn Ala Leu Val Glu Ala Leu Ala Met Gly Ala Asp Glu
65                  70                  75                  80

Ala Val Leu Leu Thr Asp Arg Ala Phe Gly Gly Ala Asp Thr Leu Ala
                85                  90                  95

Thr Ser His Thr Ile Ala Ala Gly Ile Lys Lys Leu Lys Tyr Asp Ile
            100                 105                 110

Val Phe Ala Gly Arg Gln Ala Ile Asp Gly Asp Thr Ala Gln Val Gly
        115                 120                 125

Pro Glu Ile Ala Glu His Leu Gly Ile Pro Gln Val Thr Tyr Val Glu
    130                 135                 140

Lys Val Glu Val Asp Gly Asp Thr Leu Lys Ile Arg Lys Ala Trp Glu
145                 150                 155                 160

Asp Gly Tyr Glu Val Val Glu Val Lys Thr Pro Val Leu Leu Thr Ala
                165                 170                 175

Ile Lys Glu Leu Asn Val Pro Arg Tyr Met Ser Val Glu Lys Ile Phe
            180                 185                 190

Gly Ala Phe Asp Lys Glu Val Lys Met Trp Thr Ala Asp Asp Ile Asp
        195                 200                 205

Val Asp Lys Ala Asn Leu Gly Leu Lys Gly Ser Pro Thr Lys Val Lys
    210                 215                 220

Lys Ser Ser Thr Lys Glu Val Lys Gly Gln Gly Glu Val Ile Asp Lys
225                 230                 235                 240

Pro Val Lys Glu Ala Ala Asp Met Leu Ser Gln Asn
                245                 250

<210>  52
<211>  337
<212>  PRT
<213>  Clostridium acetobutylicum

<400>  52

Met Asn Lys Ala Asp Tyr Lys Gly Val Trp Val Phe Ala Glu Gln Arg
1               5                   10                  15

Asp Gly Glu Leu Gln Lys Val Ser Leu Glu Leu Leu Gly Lys Gly Lys
            20                  25                  30

Glu Met Ala Glu Lys Leu Gly Val Glu Leu Thr Ala Val Leu Leu Gly
        35                  40                  45

His Asn Thr Glu Lys Met Ser Lys Asp Leu Leu Ser His Gly Ala Asp
    50                  55                  60

Lys Val Leu Ala Ala Asp Asn Glu Leu Leu Ala His Phe Ser Thr Asp
65                  70                  75                  80

Gly Tyr Ala Lys Val Ile Cys Asp Leu Val Asn Glu Arg Lys Pro Glu
                85                  90                  95

Ile Leu Phe Ile Gly Ala Thr Phe Ile Gly Arg Asp Leu Gly Pro Arg
            100                 105                 110

Ile Ala Ala Arg Leu Ser Thr Gly Leu Thr Ala Asp Cys Thr Ser Leu
        115                 120                 125

Asp Ile Asp Val Glu Asn Arg Asp Leu Leu Ala Thr Arg Pro Ala Phe
    130                 135                 140

Gly Gly Asn Leu Ile Ala Thr Ile Val Cys Ser Asp His Arg Pro Gln
145                 150                 155                 160

Met Ala Thr Val Arg Pro Gly Val Phe Phe Glu Lys Leu Pro Val Asn
                165                 170                 175

Asp Ala Asn Val Ser Asp Asp Lys Ile Glu Lys Val Ala Ile Lys Leu
            180                 185                 190

Thr Ala Ser Asp Ile Arg Thr Lys Val Ser Lys Val Val Lys Leu Ala
        195                 200                 205

Lys Asp Ile Ala Asp Ile Gly Glu Ala Lys Val Leu Val Ala Gly Gly
    210                 215                 220

Arg Gly Val Gly Ser Lys Glu Asn Phe Glu Lys Leu Glu Glu Leu Ala
225                 230                 235                 240

Ser Leu Leu Gly Gly Thr Ile Ala Ala Ser Arg Ala Ala Ile Glu Lys
                245                 250                 255

Glu Trp Val Asp Lys Asp Leu Gln Val Gly Gln Thr Gly Lys Thr Val
            260                 265                 270

Arg Pro Thr Leu Tyr Ile Ala Cys Gly Ile Ser Gly Ala Ile Gln His
        275                 280                 285

Leu Ala Gly Met Gln Asp Ser Asp Tyr Ile Ile Ala Ile Asn Lys Asp
    290                 295                 300

Val Glu Ala Pro Ile Met Lys Val Ala Asp Leu Ala Ile Val Gly Asp
305                 310                 315                 320

Val Asn Lys Val Val Pro Glu Leu Ile Ala Gln Val Lys Ala Ala Asn
                325                 330                 335

Asn

<210>  53
<211>  379
<212>  PRT
<213>  Clostridium acetobutylicum

<400>  53

Met Asp Phe Asn Leu Thr Arg Glu Gln Glu Leu Val Arg Gln Met Val
1               5                   10                  15

Arg Glu Phe Ala Glu Asn Glu Val Lys Pro Ile Ala Ala Glu Ile Asp
            20                  25                  30

Glu Thr Glu Arg Phe Pro Met Glu Asn Val Lys Lys Met Gly Gln Tyr
        35                  40                  45

Gly Met Met Gly Ile Pro Phe Ser Lys Glu Tyr Gly Gly Ala Gly Gly
    50                  55                  60

Asp Val Leu Ser Tyr Ile Ile Ala Val Glu Glu Leu Ser Lys Val Cys
65                  70                  75                  80

Gly Thr Thr Gly Val Ile Leu Ser Ala His Thr Ser Leu Cys Ala Ser
                85                  90                  95

Leu Ile Asn Glu His Gly Thr Glu Glu Gln Lys Gln Lys Tyr Leu Val
            100                 105                 110

Pro Leu Ala Lys Gly Glu Lys Ile Gly Ala Tyr Gly Leu Thr Glu Pro
        115                 120                 125

Asn Ala Gly Thr Asp Ser Gly Ala Gln Gln Thr Val Ala Val Leu Glu
    130                 135                 140

Gly Asp His Tyr Val Ile Asn Gly Ser Lys Ile Phe Ile Thr Asn Gly
145                 150                 155                 160

Gly Val Ala Asp Thr Phe Val Ile Phe Ala Met Thr Asp Arg Thr Lys
                165                 170                 175

Gly Thr Lys Gly Ile Ser Ala Phe Ile Ile Glu Lys Gly Phe Lys Gly
            180                 185                 190

Phe Ser Ile Gly Lys Val Glu Gln Lys Leu Gly Ile Arg Ala Ser Ser
        195                 200                 205

Thr Thr Glu Leu Val Phe Glu Asp Met Ile Val Pro Val Glu Asn Met
    210                 215                 220

Ile Gly Lys Glu Gly Lys Gly Phe Pro Ile Ala Met Lys Thr Leu Asp
225                 230                 235                 240

Gly Gly Arg Ile Gly Ile Ala Ala Gln Ala Leu Gly Ile Ala Glu Gly
                245                 250                 255

Ala Phe Asn Glu Ala Arg Ala Tyr Met Lys Glu Arg Lys Gln Phe Gly
            260                 265                 270

Arg Ser Leu Asp Lys Phe Gln Gly Leu Ala Trp Met Met Ala Asp Met
        275                 280                 285

Asp Val Ala Ile Glu Ser Ala Arg Tyr Leu Val Tyr Lys Ala Ala Tyr
    290                 295                 300

Leu Lys Gln Ala Gly Leu Pro Tyr Thr Val Asp Ala Ala Arg Ala Lys
305                 310                 315                 320

Leu His Ala Ala Asn Val Ala Met Asp Val Thr Thr Lys Ala Val Gln
                325                 330                 335

Leu Phe Gly Gly Tyr Gly Tyr Thr Lys Asp Tyr Pro Val Glu Arg Met
            340                 345                 350

Met Arg Asp Ala Lys Ile Thr Glu Ile Tyr Glu Gly Thr Ser Glu Val
        355                 360                 365

Gln Lys Leu Val Ile Ser Gly Lys Ile Phe Arg
    370                 375

<210>  54
<211>  3941
<212>  DNA
<213>  Artificial Sequence

<220>
<223>  synthetic gen

<400>  54
aagcttcccc aggctttaca ctttatgctt ccggctcgta tgttgtgtgg aattgtgagc      60

ggataacaat ttcacatttt aggaggatta gtcatggaac tcaacaatgt catcctggag     120

aaagaaggca aggtcgcggt ggtgaccatc aaccgcccga aggccctcaa cgccctgaac     180

tcggacaccc tgaaagagat ggactacgtg atcggcgaaa tcgagaacga ctcggaggtg     240

ctcgccgtga tcctgacggg cgcgggcgag aagagcttcg tcgccggggc ggacatctcg     300

gagatgaaag aaatgaatac catcgagggg cgcaagttcg gcattctggg gaacaaagtg     360

tttcggcggc tggagctgct ggaaaaaccg gtgattgcgg ccgtgaatgg gttcgccctg     420

ggcggcgggt gcgaaattgc gatgtcctgc gatatccgca tcgcctcctc gaacgcccgc 480

ttcggccagc cggaagtggg cctgggcatc accccgggct ttggcggcac ccagcggctg 540

tcgcgcctcg tcggcatggg catggccaag cagctgattt tcaccgcgca gaacatcaag 600

gccgatgagg ccctgcgcat tggcctggtg aacaaggtgg tggagccctc ggaactgatg 660

aacaccgcca aggagatcgc caacaaaatc gtgagcaatg ccccggtggc cgtcaaactg 720

tcgaagcaag ccattaaccg cggcatgcag tgcgacattg acacggccct cgccttcgag 780

tcggaagcgt tcggggaatg cttctcgacc gaggaccaga aagacgccat gaccgccttc 840

attgagaagc gcaagatcga agggttcaag aaccgctagg aggtaagttt atatggactt 900

caacctgacg cgcgagcaag agctggtgcg ccagatggtc cgggagttcg ccgagaacga 960

ggtgaagccg attgccgcgg agatcgacga gacggagcgg ttcccgatgg agaacgtgaa 1020

gaagatgggc cagtacggca tgatgggcat cccgttctcg aaggagtacg gcggggccgg 1080

cggcgacgtg ctgtcgtaca tcatcgccgt ggaggagctg tcgaaggtgt gcggcaccac 1140

cggcgtgatc ctgtcggccc acacctcgct gtgcgcctcg ctgattaacg agcacggcac 1200

cgaggagcag aagcagaagt acctggtgcc gctggccaag ggggagaaga tcggcgccta 1260

cggcctgacc gagccgaacg ccggcaccga ttcgggcgcc cagcagaccg tggccgtgct 1320

ggagggcgac cactacgtga tcaacggctc gaaaatcttc atcaccaacg gcggcgtcgc 1380

cgacaccttc gtcatcttcg ccatgaccga ccgcaccaag gggaccaagg gcatctcggc 1440

ctttatcatc gagaagggct tcaagggctt ctcgatcggc aaggtggagc aaaagctggg 1500

catccgcgcg tcgtcgacca ccgagctggt gttcgaggac atgatcgtgc cggtggagaa 1560

catgattggc aaggagggca agggcttccc gatcgccatg aagaccctgg acgggggccg 1620

catcggcatc gcggcccagg ccctgggcat cgccgaaggc gcctttaacg aggcccgcgc 1680

gtacatgaag gaacgcaagc agttcggccg ctcgctggac aagttccagg gcctggcctg 1740

gatgatggcc gacatggacg tggccatcga gtcggcccgc tacctggtgt acaaggccgc 1800

ctacctgaag caggcgggcc tgccgtacac cgtggatgcc gcccgcgcca agctgcatgc 1860

cgccaacgtg gccatggacg tgaccaccaa ggccgtgcag ctgttcggcg gctatggcta 1920

caccaaggac taccccgtgg agcgcatgat gcgcgacgcc aagatcacgg agatctacga 1980

gggcacctcg gaggtgcaga agctggtgat ctcgggcaag atcttccgct aatttaagga 2040

ggttaagagg atgaatattg tggtctgcct caaacaagtc cccgacaccg ccgaggtccg 2100

cattgatccc gtcaaaggca ccctgattcg ggagggcgtc ccgtccatca ttaaccccga 2160

cgacaaaaac gcgctcgagg aagccctcgt gctgaaagat aactatgggg cgcacgtcac 2220

ggtcatctcg atgggccccc cccaagccaa gaacgccctg gtcgaagcgc tggccatggg 2280

ggccgatgag gcggtgctgc tcacggaccg ggcgtttggg ggcgcggaca ccctcgccac 2340

ctcgcatacc attgccgccg gcattaagaa gctgaaatat gacattgtct ttgccggccg 2400

ccaggccatc gacggcgata cggcgcaggt cgggccggag attgcggaac atctcgggat 2460

tccccaagtc acctacgtgg aaaaagtgga ggtggatggg gacacgctca agatccggaa 2520

ggcctgggag gatgggtacg aggtcgtgga agtcaagacc ccggtgctcc tgacggccat 2580

taaggaactg aacgtcccgc ggtacatgtc ggtggagaaa atcttcggcg cgttcgacaa 2640

ggaggtgaaa atgtggaccg cggatgatat cgacgtggat aaagccaatc tgggcctgaa 2700

ggggtcgccg accaaagtga agaagtcgag cacgaaagaa gtgaaaggcc agggcgaggt 2760

cattgataaa ccggtgaagg aagcggccga tatgctctcg cagaactaaa agaagaacac 2820

atatttaagt taggagggat ttttcaatga acaaggccga ctacaagggc gtgtgggtgt 2880

ttgcggagca gcgcgacggc gagctgcaga aggtgtcgct ggagctgctg ggcaagggca 2940

aggagatggc cgagaagctg ggggtggaac tgaccgccgt gctgctgggc cacaacaccg 3000

agaagatgtc gaaggacctg ctgagccacg gcgcggataa ggtgctcgcg gccgacaatg 3060

aactcctcgc ccacttctcg accgacggct acgcgaaggt gatctgcgac ctcgtgaacg 3120

agcgcaagcc ggagatcctg ttcattggcg ccacgttcat cgggcgggac ctgggcccgc 3180

ggatcgcggc gcgcctgtcg accggcctga ccgccgactg cacctcgctg gacatcgacg 3240

tggagaaccg ggacctcctg gcgacccgcc cggcgttcgg cggcaacctg atcgccacca 3300

tcgtctgctc ggaccaccgc ccgcagatgg ccacggtccg ccccggcgtc ttcttcgaga 3360

agctgccggt gaacgacgcc aacgtgtccg acgataagat cgagaaagtc gccatcaagc 3420

tgaccgcgtc cgacatccgc accaaagtgt cgaaagtggt gaagctggcc aaagacatcg 3480

cggacatcgg cgaggccaag gtgctcgtgg ccgggggccg cggcgtgggc tcgaaggaga 3540

acttcgaaaa actggaggag ctggcctcgc tcctgggcgg caccatcgcc gcctcgcgcg 3600

cggccatcga gaaagagtgg gtggacaaag acctccaggt gggccagacc ggcaagaccg 3660

tgcgcccgac cctgtacatc gcctgcggca tctcgggggc gatccagcac ctggccggca 3720

tgcaggactc ggactacatc atcgccatca ataaggacgt ggaggccccg attatgaagg 3780

tggccgacct ggcgatcgtg ggcgacgtga acaaagtggt cccggagctg atcgcgcagg 3840

tgaaggccgc caacaactga gtggcctgac agtcgccgga aagaaaaagg gcggccccgc 3900

ggggccgccc tttttgctta tcgaggcagc ctgctggatc c 3941

<210> 55
<211> 3145
<212> DNA
<213> Artificial Sequence

<220>

<223>  synthetic gen

<400>  55
aagcttcccc aggctttaca ctttatgctt ccggctcgta tgttgtgtgg aattgtgagc      60

ggataacaat ttcacataga taggaggtaa gtttatatgg acttcaacct gacccgcgag     120

caggagctgg tgcgccagat ggtgcgcgag ttcgcggaaa acgaggtgaa gcccattgcc     180

gcggagatcg acgagaccga gcgcttcccg atggagaacg tgaagaagat gggccagtat     240

ggcatgatgg ggattccgtt ctcgaaggag tacggcgggg cgggggggga cgtgctgtcg     300

tacatcatcg ccgtggagga gctgtccaag gtgtgcggca ccaccggggt gatcctgtcg     360

gcccacacct ccctctgcgc ctcgctgatc aacgagcacg ggaccgagga gcagaaacag     420

aagtacctgg tcccgctggc caaaggggag aagatcggcg cgtacggcct gacggaaccg     480

aacgccggca ccgactcggg cgcgcagcag accgtggccg tcctggaagg cgaccactac     540

gtgatcaacg gctccaaaat ctttattacc aacggcggcg tggcggacac gttcgtgatc     600

ttcgcgatga ccgaccgcac caagggcacc aaaggcatct cggcctttat cattgagaaa     660

gggtttaagg gcttctcgat cggcaaggtg gagcaaaagc tgggcatccg cgcgtcgtcg     720

accaccgaac tggtgttcga ggacatgatc gtgccggtgg aaaacatgat tggcaaagag     780

gggaaggggt tcccgattgc gatgaaaacc ctggatgggg ggcggatcgg cattgcggcc     840

caagccctgg ggatcgcgga gggcgccttc aacgaagccc gggcctacat gaaggagcgc     900

aagcagttcg gccgctcgct ggacaagttt cagggcctgg cctggatgat ggccgatatg     960

gacgtggcca ttgagtccgc ccggtatctg gtctataagg ccgcctacct gaagcaggcg    1020

ggcctgccgt acaccgtgga cgccgcgcgc gccaagctgc atgccgccaa cgtcgccatg    1080

gacgtgacca ccaaagcggt gcaactgttc ggcggctacg gctacacgaa ggactacccg    1140

gtcgagcgca tgatgcgcga cgccaagatt accgagatct acgagggcac gtcggaagtg    1200

cagaagctgg tgattagcgg caagattttc cgctaattta aggaggttaa gaggatgaac    1260

atcgtggtgt gtctgaagca ggtgccggac accgccgagg tgcgcatcga tccggtgaag    1320

gggaccctga tccgcgaggg cgtgccgtcg atcatcaacc cggacgacaa gaatgcgctg    1380

gaggaagcgc tggtcctgaa agacaactac ggggcccatg tgaccgtgat ctcgatgggc    1440

cccccgcagg ccaagaacgc cctggtggag gccctggcca tgggggcgga cgaggcggtg    1500

ctgctgaccg accgcgcctt cgggggggcg gataccctgg cgacctcgca caccatcgcc    1560

gcgggcatca agaagctgaa atatgatatc gtgtttgccg ggcgccaggc cattgacggc    1620

gacacggccc aggtcgggcc ggagatcgcc gagcatctgg gcatccccca ggtgacctac    1680

gtggagaaag tcgaggtgga tggcgatacg ctgaaaatcc gcaaggcctg ggaggacggc    1740

tacgaggtcg tggaggtgaa gaccccggtc ctgctgacgg ccatcaagga gctgaacgtg    1800

ccgcgctaca tgtcggtgga aaagattttc ggcgcgttcg acaaggaggt caagatgtgg 1860

accgcggatg acatcgacgt cgacaaggcc aacctggggc tgaagggctc gccgaccaag 1920

gtgaaaaagt cgtcgaccaa agaagtcaag ggccaaggcg aagtgatcga taagcccgtg 1980

aaggaggcgg ccgatatgct gtcgcagaac taaaagaaga acacatattt aagttaggag 2040

ggatttttca atgaacaaag ccgactacaa gggcgtgtgg gtgttcgccg agcaacgcga 2100

cggcgagctg cagaaggtgt cgctggaact gctggggaag ggcaaggaaa tggccgagaa 2160

actgggcgtg gagctgacgg ccgtgctgct ggggcacaac accgagaaga tgtcgaagga 2220

cctgctgtcg cacggcgcgg acaaggtcct ggccgccgac aacgagctgc tggcccattt 2280

cagcaccgac ggctacgcca aggtgatctg tgacctggtg aacgagcgca agccggagat 2340

cctgttcatc ggcgccacgt tcatcggccg cgacctcggc ccgcggatcg ccgcccggct 2400

gtcgaccggc ctgaccgccg actgcacctc gctggacatc gacgtggaga accgcgatct 2460

gctcgcgacc cgcccggcct tcggcggcaa cctgatcgcc accatcgtgt gctcggacca 2520

ccgcccgcag atggccacgg tgcggcccgg cgtgttcttc gagaagctgc cggtgaacga 2580

cgccaacgtg tcggacgaca agatcgagaa agtggccatc aagctgaccg cctcggacat 2640

ccgcaccaag gtctcgaaag tggtgaagct cgccaaggat attgccgata tcggcgaggc 2700

caaggtgctc gtggccgggg gccgcggcgt gggctcgaag gagaactttg agaagctgga 2760

ggagctggcc tccctcctcg gcggcaccat cgccgcctcg cgcgccgcca tcgaaaagga 2820

gtgggtggat aaggacctcc aagtggggca gaccggcaag accgtgcgcc cgaccctgta 2880

catcgcctgc ggcatctcgg gcgccatcca gcacctggcc ggcatgcagg actcggacta 2940

catcatcgcg atcaacaagg acgtggaagc cccgatcatg aaggtggccg acctggccat 3000

tgtgggcgat gtgaacaagg tggtgcccga gctgatcgcc caagtcaagg ccgccaacaa 3060

ctgagtggcc tgacagtcgc cggaaagaaa aagggcggcc ccgcggggcc gccctttttg 3120

cttatcgagg cagcctgctg gatcc 3145

<210> 56
<211> 10208
<212> DNA
<213> Artificial Sequence

<220>
<223> vector

<400> 56
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaatttctc tggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag gggccacttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatcccccg ggctgcagga attcgatatc aagcttgggg agaacaatca 3300

gcccggcagg ggccgggctg attgtgcctg cgtgccttag aacgacttga tgtagatgtc 3360

cttcagctcc gagatcagcg ggtagcgcgg gttggcggtg gtgcactggt cgtcgaaggc 3420

cagctccgac atcttgtcca gggtgttgta gaaatccttc ttgttgatgc cggcggccga 3480

gatgttctgc gggatcgaca ggtcgatctt cagcttcgag atggcctcga tcagggcggt 3540

caccttctcc gtgtccgagg tgcccttcag gttcaggtac tcggcgatct cggcgtactt 3600

gcgcttggcg ttcggcgact tgtactgcgg gaaggcggtc tgcttggtcg ggcagtcggt 3660

ggcgttgtac ttgatcacct cctcgatcag cacggcgcag gcgatgccgt gcggcacgtg 3720

gtgcatggcg cccagcttgt gggccatcga gtggcacacg cccaggaagg cgttggcgaa 3780

ggccatgccg gcgatgttcg aggcgtgggc catcttctcg cgggcctcga tgtcgttggt 3840

gccgttcttg taggcgcgcg gcaggtactt gaagatcatc ttgatggcgc gcagggccag 3900

ctcgtcggtg tagtccgtgg ccatcaccga cacgtaggcc tcgatggcgt gcaccagggc 3960

atcaatgccg gtggcggcgg tcagcttgcg cggcatgttc agcatcagct cggtgtcaat 4020

gatggccatg ttcggggtca gttcgtacga ggtcagcggg tacttcatgc cggtctcgtc 4080

gttggtgatc acggcgaacg gggtggcctc cgagccggtg ccggcggtgg tcgggatggc 4140

caccgagatg gccttggtgc ccagcttcgg gaagttgcag atgcgcttgc ggatgtccat 4200

gaaattgatg gcgaggttct cgatctccgc ctccgggtac tcgtacagca ggtgcatcac 4260

cttggcggcg tccatcggcg agccgccgcc gatcgagatg atggtgtccg gctcgaagtt 4320

cagcatctcc ttggcgccct tcttcaccga gtcaatggtc gggtccgact tgatatcggt 4380

gaagatcgag tacttgatgt cgatctcgtc cagcaccttg gtgatcttgt tcacgtagcc 4440

cagcttgaac aggtccttgt cggtcacgat gaaggcgcgc ttcttgttca tgtccttcag 4500

ctccttcagg gcgaagcgca ggcagccgta cttgaagtag atcttctgcg gcaccttgaa 4560

ccacagcata ttctcgcggc gctcggccac cgacttgatg ttcagcaggt gcttcggctc 4620

cacgttctgc gacaccgagt tgccgcccca ggtcccgcag cccagggtga acgacggggc 4680

gatggcgaag ttgtacaggt cgcccgaggc gccctgcgac gacggcatgt tgatgaaggt 4740

gcgcgaggtc ttcatggcca ggccgaactc cttcaccttg tccttgttgt tctgcgagtc 4800

gatgtacagc gacgaggtgt ggcccgagcc gcccagctcg atcaggcgct gggccttctt 4860

gagggcctca tcgaagtcct tcaccttgta catggccagc accggcgaca gcttctcgtg 4920

cgagaacagc tccgacttct ccaccgactg cacctcgccg atgaggatct tggtggtctg 4980

cggcacctcg atgccggcca tcttggcgat gatgtaggcc gacttgccca cgatgtcggc 5040

gttgatggcg ccgttcttga acatcgtctc cttgatcttg gcgatctcgt tctggttcag 5100

gatgtacgag ccgcgcttca cgaactcctc cttcaccttc tcgtagatgg agttcatcac 5160

caggatcgac tgctccgagg cgcagatcac gccgttgtcg taggtcttcg acaggatgat 5220

cgacgacacg gccatatcga tgtcggcgga ctcgtcgatg atggccgggg tgttgcccgc 5280

gcccacgccg atggccggct tgcccgacga gtaggcggcc ttcaccatcg acgggccgcc 5340

ggtggccagg atgatatcgg cctccgacat caggtcctgc gacagctcga tcgacggctc 5400

gtcaatccag ccgatgatgt tcttcggggc cccggccttc acggcggcgt ccaggatcag 5460

cttggcggcg gcgatggtcg acttcttggc gcgcgggtgc ggcgagaaga agatggcgtt 5520

gcgggtcttc agcgagatca gcgacttgaa gatggcggtc gaggtcgggt tcgtggtcgg 5580

cacgatggcg gccacgatgc cgatcggctc ggccaccttg gtgatgccca gcgagtcgtc 5640

gtggtcgata atgccgcagg tcttctcgtt cttgtacttg ttgtagatgt actcggcggc 5700

gaagtggttc ttgatgatct tgtcctccac caggccgatg ccggtctcct ccacggccag 5760

cttggccagg ttgatgcgct ccttggcggc ggcaatggcg cactgcttga agatcttgtc 5820

cacctgctcc tgggtgtagg tggcgaactt cttctgggcc tcgcgcagct cgttcagctt 5880

ctgcttcagc tccttctggt tggtcacctt cattcttgaa tctcctgaaa gccggtgctt 5940

acggcagctt gaccacggct tccccggtga cggccagggc cccgccttgg gtaaagatcc 6000

gggtcgtcag cgtggcgatc ggtttgtcct cgcgcagcgc ggtgacttcc acctcggcgg 6060

tcacttcgtc ccccacgaac accggcagtt tgaacgagag cgattggccc aggtagatgc 6120

tccccttgcc ggggagctgc tggccgagga ggcccgagaa cagcgaggcg agcagcatgc 6180

catgcacgat ggggcgctcg aacgcggtgg tcgcggcaaa cgcggggtcc aggtgcagcg 6240

ggttgaagtc ctcggagagc gccgcaaagg ccgccacctc cgccgcgcca aaccgcttgg 6300

acagccgggc tttttgcccg acctccagcg actgggccga catgcggcgt cctcctctgt 6360

ttcagcccat atgcaggccg ccgttgagcg agaagtcggc gccggtcgag aaaccggact 6420

cctccgacga caaccaggcg cagatcgagg cgatctcttc cggcaggccc aggcgcttga 6480

ccgggatcgt cgcgacgatc ttgtcgagca cgtcctggcg gatcgccttg accatgtcgg 6540

tggcgatata gcccggagag accgtgttga cggtcacgcc cttggtcgcc acttcctgcg 6600

ccagtgccat ggtgaagcca tgcaggccgg ccttggcggt ggagtagttg gtctggccga 6660

actggccctt ctgcccgttc accgacgaga tgttgacgat gcggccccag ccacggtcgg 6720

ccatgccgtc gatcacctgc ttggtgacgt tgaacagcga ggtcaggttg gtgtcgatca 6780

ccgcatccca gtcggcgcgg gtcatcttgc ggaacaccac gtcgcgggtg ataccggcgt 6840

tgttgatcag cacatcaacc tcgccgacct cggacttgac cttgtcgaat gcggtcttgg 6900

tcgagtccca gtcagccaca ttgccttccg aggcaatgaa atcgaagccc agggccttct 6960

gctgctccag ccacttttcg cggcgcggcg agttggggcc gcaaccggcc accacacgaa 7020

agccatcctt ggccagccgc tggcaaatgg cggttccgat accacccatg ccgccggtca 7080

catacgcaat gcgctgagtc atgtccactc cttgattggc ttcgttatcg tcgccgggtc 7140

cgcgccaacc gcgcgcggcc ccggaaaacc ccttccttat ttgcgctcga ctgccagcgc 7200

cacgcccatg ccgccgccga tgcacagcga ggccaggccc ttcttcgcgt cacggcgctt 7260

catctcgtgc agcagcgtca ccaggatacg gcagcccgac gcgccgatcg ggtggccgat 7320

ggcgatggcg ccgccgttca cattgacctt ggaggtgtcc cagcccatct gctggtgcac 7380

cgccagcgcc tgcgcggcaa aggcctcgtt gatctccatc aggtccaggt cttgcggggt 7440

ccactcggcg cgcgacaggg cgcgcttgga ggccggcacc gggcccatgc ccatcacctt 7500

gggatcgaca ccggcgttgg catagctctt gatcgtggcc agcggggtca ggcccagttc 7560

cttggccttg gccgccgaca tcaccaccac cgcggcggcg ccgtcgttca ggcccgaggc 7620

gttggccgcg gtcaccgtgc cggccttgtc gaaggcgggc ttgaggccgg acatgctgtc 7680

cagcgtggcg ccctggcgca cgaactcgtc ggtcttgaag gccaccgggt cgcccttgcg 7740

ctgcgggatc agcaccggga cgatctcttc gtcaaacttg ccggccttct gcgcggcttc 7800

ggccttgttc tgcgagccga cggcgaactc atcctgcgcc tcgcgtgtga tgccgtattc 7860

cttggccacg ttctcggcgg tgatgcccat gtggtactgg ttgtacacgt cccacaggcc 7920

gtcgacgatc atggtgtcga ccagcttggc atcgcccatg cggaaaccat cgcgcgagcc 7980

cggcagcacg tgcggggcgg cgctcatgtt ttcctggccg ccggccacca cgatctcggc 8040

gtcgcccgcc atgatcgcgt tggcggccag catcacggcc ttcaggcccg agccgcacac 8100

cttgttgatg gtcatggccg gcaccatcgc cggcaggccg gccttgatcg cggcctggcg 8160

tgcggggttc tggcccgaac cggcggtcag cacctggccc atgatgactt cgctcacctg 8220

ctccggcttg acgccggcgc gctccagcgc ggccttgatg accacggcac ccagttccgg 8280

tgccgggatc ttggccagcg agccgccaaa cttgccgacc gcggtgcggg cggcggatac 8340

gatgacaacg tcagtcattg tgtagtcctt tcaatggaaa ggtacccagc ttttgttccc 8400

tttagtgagg gttaattgcg cgcttggcgt aatcatggtc atagctgttt cctgtgtgaa 8460

attgttatcc gctcacaatt ccacacaaca tacgagccgg aagcataaag tgtaaagcct 8520

ggggtgccta atgagtgagc taactcacat taattgcgtt gcgctcactg cccgctttcc 8580

agtcgggaaa cctgtcgtgc cagctgcatt aatgaatcgg ccaacgcgcg gggagaggcg 8640

gtttgcgtat tgggcgcatg cataaaaact gttgtaattc attaagcatt ctgccgacat 8700

ggaagccatc acaaacggca tgatgaacct gaatcgccag cggcatcagc accttgtcgc 8760

cttgcgtata atatttgccc atgggggtgg gcgaagaact ccagcatgag atccccgcgc 8820

tggaggatca tccagccggc gtcccggaaa acgattccga agcccaacct ttcatagaag 8880

gcggcggtgg aatcgaaatc tcgtgatggc aggttgggcg tcgcttggtc ggtcatttcg 8940

aaccccagag tcccgctcag aagaactcgt caagaaggcg atagaaggcg atgcgctgcg 9000

aatcgggagc ggcgataccg taaagcacga ggaagcggtc agcccattcg ccgccaagct 9060

cttcagcaat atcacgggta gccaacgcta tgtcctgata gcggtccgcc acacccagcc 9120

ggccacagtc gatgaatcca gaaaagcggc cattttccac catgatattc ggcaagcagg 9180

catcgccatg ggtcacgacg agatcctcgc cgtcgggcat gcgcgccttg agcctggcga 9240

acagttcggc tggcgcgagc ccctgatgct cttcgtccag atcatcctga tcgacaagac 9300

cggcttccat ccgagtacgt gctcgctcga tgcgatgttt cgcttggtgg tcgaatgggc 9360

aggtagccgg atcaagcgta tgcagccgcc gcattgcatc agccatgatg gatactttct 9420

cggcaggagc aaggtgagat gacaggagat cctgccccgg cacttcgccc aatagcagcc 9480

agtcccttcc cgcttcagtg acaacgtcga gcacagctgc gcaaggaacg cccgtcgtgg 9540

ccagccacga tagccgcgct gcctcgtcct gcagttcatt cagggcaccg gacaggtcgg 9600

tcttgacaaa aagaaccggg cgcccctgcg ctgacagccg gaacacggcg gcatcagagc 9660

agccgattgt ctgttgtgcc cagtcatagc cgaatagcct ctccacccaa gcggccggag 9720

aacctgcgtg caatccatct tgttcaatca tgcgaaacga tcctcatcct gtctcttgat 9780

cagatcttga tcccctgcgc catcagatcc ttggcggcaa gaaagccatc cagtttactt 9840

tgcagggctt cccaacctta ccagagggcg ccccagctgg caattccggt tcgcttgctg 9900

tccataaaac cgcccagtct agctatcgcc atgtaagccc actgcaagct acctgctttc 9960

tctttgcgct tgcgttttcc cttgtccaga tagcccagta gctgacattc atcccaggtg 10020

gcacttttcg gggaaatgtg cgcgcccgcg ttcctgctgg cgctgggcct gtttctggcg 10080

ctggacttcc cgctgttccg tcagcagctt ttcgcccacg gccttgatga tcgcggcggc 10140

cttggcctgc atatcccgat tcaacggccc cagggcgtcc agaacgggct tcaggcgctc 10200

ccgaaggt 10208

<210> 57
<211> 7614
<212> DNA
<213> Artificial Sequence

<220>
<223> vector

<400> 57
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaattttct ctggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag gggccacttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatcccccg ggctgcagga attcgatatc aagcttgggg agaacaatca 3300

gcccggcagg ggccgggctg attgtgcctg cgtgccgccg gtgcttacgg cagcttgacc 3360

acggcttccc cggtgacggc cagggccccg ccttgggtaa agatccgggt cgtcagcgtg 3420

gcgatcggtt tgtcctcgcg cagcgcggtg acttccacct cggcggtcac ttcgtccccc 3480

acgaacaccg gcagtttgaa cgagagcgat tggcccaggt agatgctccc cttgccgggg 3540

agctgctggc cgaggaggcc cgagaacagc gaggcgagca gcatgccatg cacgatgggg 3600

cgctcgaacg cggtggtcgc ggcaaacgcg gggtccaggt gcagcgggtt gaagtcctcg 3660

gagagcgccg caaaggccgc cacctccgcc gcgccaaacc gcttggacag ccgggctttt 3720

tgcccgacct ccagcgactg ggccgacatg cggcgtcctc ctctgtttca gcccatatgc 3780

aggccgccgt tgagcgagaa gtcggcgccg gtcgagaaac cggactcctc cgacgacaac 3840

caggcgcaga tcgaggcgat ctcttccggc aggcccaggc gcttgaccgg gatcgtcgcg 3900

acgatcttgt cgagcacgtc ctggcggatc gccttgacca tgtcggtggc gatatagccc 3960

ggagagaccg tgttgacggt cacgcccttg gtcgccactt cctgcgccag tgccatggtg 4020

aagccatgca ggccggcctt ggcggtggag tagttggtct ggccgaactg gcccttctgc 4080

ccgttcaccg acgagatgtt gacgatgcgg ccccagccac ggtcggccat gccgtcgatc 4140

acctgcttgg tgacgttgaa cagcgaggtc aggttggtgt cgatcaccgc atcccagtcg 4200

gcgcgggtca tcttgcggaa caccacgtcg cgggtgatac cggcgttgtt gatcagcaca 4260

tcaacctcgc cgacctcgga cttgaccttg tcgaatgcgg tcttggtcga gtcccagtca 4320

gccacattgc cttccgaggc aatgaaatcg aagcccaggg ccttctgctg ctccagccac 4380

ttttcgcggc gcggcgagtt ggggccgcaa ccggccacca cacgaaagcc atccttggcc 4440

agccgctggc aaatggcggt tccgatacca cccatgccgc cggtcacata cgcaatgcgc 4500

tgagtcatgt ccactccttg attggcttcg ttatcgtcgc cgggtccgcg ccaaccgcgc 4560

gcggccccgg aaaacccctt ccttatttgc gctcgactgc cagcgccacg cccatgccgc 4620

cgccgatgca cagcgaggcc aggcccttct tcgcgtcacg gcgcttcatc tcgtgcagca 4680

gcgtcaccag gatacggcag cccgacgcgc cgatcgggtg gccgatggcg atggcgccgc 4740

cgttcacatt gaccttggag gtgtcccagc ccatctgctg gtgcaccgcc agcgcctgcg 4800

cggcaaaggc ctcgttgatc tccatcaggt ccaggtcttg cggggtccac tcggcgcgcg 4860

acagggcgcg cttggaggcc ggcaccgggc ccatgcccat caccttggga tcgacaccgg 4920

cgttggcata gctcttgatc gtggccagcg gggtcaggcc cagttccttg gccttggccg 4980

ccgacatcac caccaccgcg gcggcgccgt cgttcaggcc cgaggcgttg gccgcggtca 5040

ccgtgccggc cttgtcgaag gcgggcttga ggccggacat gctgtccagc gtggcgccct 5100

ggcgcacgaa ctcgtcggtc ttgaaggcca ccgggtcgcc cttgcgctgc gggatcagca 5160

ccgggacgat ctcttcgtca aacttgccgg ccttctgcgc ggcttcggcc ttgttctgcg 5220

agccgacggc gaactcatcc tgcgcctcgc gtgtgatgcc gtattccttg gccacgttct 5280

cggcggtgat gcccatgtgg tactggttgt acacgtccca caggccgtcg acgatcatgg 5340

tgtcgaccag cttggcatcg cccatgcgga aaccatcgcg cgagcccggc agcacgtgcg 5400

gggcggcgct catgttttcc tggccgccgg ccaccacgat ctcggcgtcg cccgccatga 5460

tcgcgttggc ggccagcatc acggccttca ggcccgagcc gcacaccttg ttgatggtca 5520

tggccggcac catcgccggc aggccggcct tgatcgcggc ctggcgtgcg gggttctggc 5580

ccgaaccggc ggtcagcacc tggcccatga tgacttcgct cacctgctcc ggcttgacgc 5640

cggcgcgctc cagcgcggcc ttgatgacca cggcacccag ttccggtgcc gggatcttgg 5700

ccagcgagcc gccaaacttg ccgaccgcgg tgcgggcggc ggatacgatg acaacgtcag 5760

tcattgtgta gtcctttcaa tggaaaggta cccagctttt gttcccttta gtgagggtta 5820

attgcgcgct tggcgtaatc atggtcatag ctgtttcctg tgtgaaattg ttatccgctc 5880

acaattccac acaacatacg agccggaagc ataaagtgta aagcctgggg tgcctaatga 5940

gtgagctaac tcacattaat tgcgttgcgc tcactgcccg ctttccagtc gggaaacctg 6000

tcgtgccagc tgcattaatg aatcggccaa cgcgcgggga gaggcggttt gcgtattggg 6060

cgcatgcata aaaactgttg taattcatta agcattctgc cgacatggaa gccatcacaa 6120

acggcatgat gaacctgaat cgccagcggc atcagcacct tgtcgccttg cgtataatat 6180

ttgcccatgg gggtgggcga agaactccag catgagatcc ccgcgctgga ggatcatcca 6240

gccggcgtcc cggaaaacga ttccgaagcc caacctttca tagaaggcgg cggtggaatc 6300

gaaatctcgt gatggcaggt tgggcgtcgc ttggtcggtc atttcgaacc ccagagtccc 6360

gctcagaaga actcgtcaag aaggcgatag aaggcgatgc gctgcgaatc gggagcggcg 6420

ataccgtaaa gcacgaggaa gcggtcagcc cattcgccgc caagctcttc agcaatatca 6480

cgggtagcca acgctatgtc ctgatagcgg tccgccacac ccagccggcc acagtcgatg 6540

aatccagaaa agcggccatt ttccaccatg atattcggca agcaggcatc gccatgggtc 6600

acgacgagat cctcgccgtc gggcatgcgc gccttgagcc tggcgaacag ttcggctggc 6660

gcgagcccct gatgctcttc gtccagatca tcctgatcga caagaccggc ttccatccga 6720

gtacgtgctc gctcgatgcg atgtttcgct tggtggtcga atgggcaggt agccggatca 6780

agcgtatgca gccgccgcat tgcatcagcc atgatggata ctttctcggc aggagcaagg 6840

tgagatgaca ggagatcctg ccccggcact tcgcccaata gcagccagtc ccttcccgct 6900

tcagtgacaa cgtcgagcac agctgcgcaa ggaacgcccg tcgtggccag ccacgatagc 6960

cgcgctgcct cgtcctgcag ttcattcagg gcaccggaca ggtcggtctt gacaaaaaga 7020

accgggcgcc cctgcgctga cagccggaac acggcggcat cagagcagcc gattgtctgt 7080

tgtgcccagt catagccgaa tagcctctcc acccaagcgg ccggagaacc tgcgtgcaat 7140

ccatcttgtt caatcatgcg aaacgatcct catcctgtct cttgatcaga tcttgatccc 7200

ctgcgccatc agatccttgg cggcaagaaa gccatccagt ttactttgca gggcttccca 7260

accttaccag agggcgcccc agctggcaat tccggttcgc ttgctgtcca taaaaccgcc 7320

cagtctagct atcgccatgt aagcccactg caagctacct gctttctctt tgcgcttgcg 7380

ttttcccttg tccagatagc ccagtagctg acattcatcc caggtggcac ttttcgggga 7440

aatgtgcgcg cccgcgttcc tgctggcgct gggcctgttt ctggcgctgg acttcccgct 7500

gttccgtcag cagcttttcg cccacggcct tgatgatcgc ggcggccttg gcctgcatat 7560

cccgattcaa cggccccagg gcgtccagaa cgggcttcag gcgctcccga aggt 7614

<210> 58
<211> 9844
<212> DNA
<213> Artificial Sequence

<220>
<223> vector

<400> 58
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaattttct ctggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag gggccacttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatcccccg ggctgcagga attcgatatc aagcttctta agtaataaaa 3300

ataagagtta ccttaaatgg taactcttat ttttttaata ttgtttcata gtttagaacg 3360

acttgatgta gatgtccttc agctccgaga tcagcgggta gcgcgggttg gcggtggtgc 3420

actggtcgtc gaaggccagc tccgacatct tgtccagggt gttgtagaaa tccttcttgt 3480

tgatgccggc ggccgagatg ttctgcggga tcgacaggtc gatcttcagc ttcgagatgg 3540

cctcgatcag ggcggtcacc ttctccgtgt ccgaggtgcc cttcaggttc aggtactcgg 3600

cgatctcggc gtacttgcgc ttggcgttcg gcgacttgta ctgcgggaag gcggtctgct 3660

tggtcgggca gtcggtggcg ttgtacttga tcacctcctc gatcagcacg gcgcaggcga 3720

tgccgtgcgg cacgtggtgc atggcgccca gcttgtgggc catcgagtgg cacacgccca 3780

ggaaggcgtt ggcgaaggcc atgccggcga tgttcgaggc gtgggccatc ttctcgcggg 3840

cctcgatgtc gttggtgccg ttcttgtagg cgcgcggcag gtacttgaag atcatcttga 3900

tggcgcgcag ggccagctcg tcggtgtagt ccgtggccat caccgacacg taggcctcga 3960

tggcgtgcac cagggcatca atgccggtgg cggcggtcag cttgcgcggc atgttcagca 4020

tcagctcggt gtcaatgatg gccatgttcg gggtcagttc gtacgaggtc agcgggtact 4080

tcatgccggt ctcgtcgttg gtgatcacgg cgaacggggt ggcctccgag ccggtgccgg 4140

cggtggtcgg gatggccacc gagatggcct tggtgcccag cttcgggaag ttgcagatgc 4200

gcttgcggat gtccatgaaa ttgatggcga ggttctcgat ctccgcctcc gggtactcgt 4260

acagcaggtg catcaccttg gcggcgtcca tcggcgagcc gccgccgatc gagatgatgg 4320

tgtccggctc gaagttcagc atctccttgg cgcccttctt caccgagtca atggtcgggt 4380

ccgacttgat atcggtgaag atcgagtact tgatgtcgat ctcgtccagc accttggtga 4440

tcttgttcac gtagcccagc ttgaacaggt ccttgtcggt cacgatgaag gcgcgcttct 4500

tgttcatgtc cttcagctcc ttcagggcga agcgcaggca gccgtacttg aagtagatct 4560

tctgcggcac cttgaaccac agcatattct cgcggcgctc ggccaccgac ttgatgttca 4620

gcaggtgctt cggctccacg ttctgcgaca ccgagttgcc gccccaggtc ccgcagccca 4680

gggtgaacga cggggcgatg gcgaagttgt acaggtcgcc cgaggcgccc tgcgacgacg 4740

gcatgttgat gaaggtgcgc gaggtcttca tggccaggcc gaactccttc accttgtcct 4800

tgttgttctg cgagtcgatg tacagcgacg aggtgtggcc cgagccgccc agctcgatca 4860

ggcgctgggc cttcttgagg gcctcatcga agtccttcac cttgtacatg gccagcaccg 4920

gcgacagctt ctcgtgcgag aacagctccg acttctccac cgactgcacc tcgccgatga 4980

ggatcttggt ggtctgcggc acctcgatgc cggccatctt ggcgatgatg taggccgact 5040

tgcccacgat gtcggcgttg atggcgccgt tcttgaacat cgtctccttg atcttggcga 5100

tctcgttctg gttcaggatg tacgagccgc gcttcacgaa ctcctccttc accttctcgt 5160

agatggagtt catcaccagg atcgactgct ccgaggcgca gatcacgccg ttgtcgtagg 5220

tcttcgacag gatgatcgac gacacggcca tatcgatgtc ggcggactcg tcgatgatgg 5280

ccggggtgtt gcccgcgccc acgccgatgg ccggcttgcc cgacgagtag gcggccttca 5340

ccatcgacgg gccgccggtg gccaggatga tatcggcctc cgacatcagg tcctgcgaca 5400

gctcgatcga cggctcgtca atccagccga tgatgttctt cggggccccg gccttcacgg 5460

cggcgtccag gatcagcttg gcggcggcga tggtcgactt cttggcgcgc gggtgcggcg 5520

agaagaagat ggcgttgcgg gtcttcagcg agatcagcga cttgaagatg gcggtcgagg 5580

tcgggttcgt ggtcggcacg atggcggcca cgatgccgat cggctcggcc accttggtga 5640

tgcccagcga gtcgtcgtgg tcgataatgc cgcaggtctt ctcgttcttg tacttgttgt 5700

agatgtactc ggcggcgaag tggttcttga tgatcttgtc ctccaccagg ccgatgccgg 5760

tctcctccac ggccagcttg gccaggttga tgcgctcctt ggcggcggca atggcgcact 5820

gcttgaagat cttgtccacc tgctcctggg tgtaggtggc gaacttcttc tgggcctcgc 5880

gcagctcgtt cagcttctgc ttcagctcct tctggttggt caccttcatt cttgaatctc 5940

ctgaaaattt ctttttattt cgagtagtcg taaaagccct tgccgctctt gcggcccagc 6000

cagcccgccc ggacatactt tttcagcagc gtgtgcgggc gatacttcga atcgccggtt 6060

tccgagtaca gcacgtccat gatggccagg caaatgtcga ggccgataaa gtcccccagt 6120

tccagcgggc ccatcgggtg gttcgccccc agcttcatgg ccttatcaat atcctcgacc 6180

gaggcaatcc cttcggcgag gatgcccacc gcttcattga tcatcgggat cagaatgcgg 6240

ttgacgacga accccggcgc ctcggcgact tccacggggt ccttgccaat ggcaatcgac 6300

gtctctttca ccgcgtcgaa ggtttcctgc gacgtggcaa tgccgcgaat gacctccacg 6360

agtttcatga cgggggcggg attaaagaaa tgcatgccga tcactttgtc gttggtcttg 6420

gtcgcgctcg ccacctccgt gatcgagagc gagctggtgt tggaggcgag aatcgtctcc 6480

ggtttgcaga tgttgtcgag atccgcaaag atctgtttct taatgtccat gcgctcgacg 6540

gcggcttcaa tcacgaggtc gcagtccgcg gccatgttca ggtccacggt cccgctgatg 6600

cgggtgagaa tctccacctt ggtggcctct tcgatcttgc cttttttgac cagtttcgac 6660

aggttcttgt taatgaaatc caggccgcga tcgacgaatt catccttgat gtcccggagg 6720

accacctcga agcctttggc ggcgaaggcc tgcgcgatgc cggaccccat cgtccccgcg 6780

ccaatgacgc acaccttctt cattcttgaa tctcctgaaa ctagcacttt tccagcagga 6840

tcgcggtgcc ctggccgccg ccgatgcaca gggtcgccag ccccttcttg gcgtcgcgct 6900

tctgcatcgc gtgcaccagg gtcaccagga tgcgggcgcc cgaggcgccg atcgggtgcc 6960

ccagggcgat ggcgccgcca ttcacgttca ctttgttcat gtcgaacttc aggtccttgg 7020

cgacggccag cgactgggcg gcaaaggcct cgttcgactc gatcaggtcc agctcgtcga 7080

ccgtccagcc ggctttctcg atcgccgcct tggtggcgta gaacgggccg tagcccatga 7140

tggccgggtc cacgccggcc gagccatacg acacgatctt cgccagcggt ttcacgccca 7200

gctccttggc cttttcggcc gacatgatca ccagcacggc cgcgcagtcg ttcaggcccg 7260

aggcgttgcc cgcggtcacg gtgccgtcct tcttgaaggc cggcttcagc ttggccaggc 7320

cctcgatcgt cgacccgaag cgcgggtgct cgtccgtgtc caccacggtc tcgcccttgc 7380

ggcccttaat caccaccggc acgatctcgt ccttgaactg gcccgacttg atggcttcct 7440

ccgccttctt ttgcgaggcc agggcgaact cgtcctgctc ctcgcgcgag atgttccagc 7500

gctcggcgat gttctcggcg gtgatgccca tgtggtagtc gttgaaggcg tcccacaggc 7560

cgtcggtgat catctcgtcc acgaacttgg cgttccccat gcggtagccc cagcgggcgt 7620

tgttggccag gtacggggcg cgcgacatgt tttccatgcc gccggcaatg atcacgtcgg 7680

cgtcgccggc cttgatgatc tgggccgcca gcgacacggt gcgcaggccc gagccgcaca 7740

ccttgttgat ggtcatggcg gggatctcca ccgggaggcc ggctttgaag ctcgcctggc 7800

gggccgggtt ctggccgagc ccggcctgca gcacgttgcc caggatcacc tcgttcacgt 7860

cctccggctt gatgccggcc ttcttcacgg cctccttaat ggcggtggcg cccaggtcca 7920

cggcgggcac gtctttcagg ctcttgccgt acgagccgat cgcggtgcgc acggccgagg 7980

caatcaccac ctccttcatt cttgaatctc ctgaaaggta cccagctttt gttcccttta 8040

gtgagggtta attgcgcgct tggcgtaatc atggtcatag ctgtttcctg tgtgaaattg 8100

ttatccgctc acaattccac acaacatacg agccggaagc ataaagtgta aagcctgggg 8160

tgcctaatga gtgagctaac tcacattaat tgcgttgcgc tcactgcccg ctttccagtc 8220

gggaaacctg tcgtgccagc tgcattaatg aatcggccaa cgcgcgggga gaggcggttt 8280

gcgtattggg cgcatgcata aaaactgttg taattcatta agcattctgc cgacatggaa 8340

gccatcacaa acggcatgat gaacctgaat cgccagcggc atcagcacct tgtcgccttg 8400

cgtataatat ttgcccatgg gggtgggcga agaactccag catgagatcc ccgcgctgga 8460

ggatcatcca gccggcgtcc cggaaaacga ttccgaagcc caacctttca tagaaggcgg 8520

cggtggaatc gaaatctcgt gatggcaggt tgggcgtcgc ttggtcggtc atttcgaacc 8580

ccagagtccc gctcagaaga actcgtcaag aaggcgatag aaggcgatgc gctgcgaatc 8640

gggagcggcg ataccgtaaa gcacgaggaa gcggtcagcc cattcgccgc caagctcttc 8700

agcaatatca cgggtagcca acgctatgtc ctgatagcgg tccgccacac ccagccggcc 8760

acagtcgatg aatccagaaa agcggccatt ttccaccatg atattcggca agcaggcatc 8820

gccatgggtc acgacgagat cctcgccgtc gggcatgcgc gccttgagcc tggcgaacag 8880

ttcggctggc gcgagcccct gatgctcttc gtccagatca tcctgatcga caagaccggc 8940

ttccatccga gtacgtgctc gctcgatgcg atgtttcgct tggtggtcga atgggcaggt 9000

agccggatca agcgtatgca gccgccgcat tgcatcagcc atgatggata ctttctcggc 9060

aggagcaagg tgagatgaca ggagatcctg ccccggcact tcgcccaata gcagccagtc 9120

ccttcccgct tcagtgacaa cgtcgagcac agctgcgcaa ggaacgcccg tcgtggccag 9180

ccacgatagc cgcgctgcct cgtcctgcag ttcattcagg gcaccggaca ggtcggtctt 9240

gacaaaaaga accgggcgcc cctgcgctga cagccggaac acggcggcat cagagcagcc 9300

gattgtctgt tgtgcccagt catagccgaa tagcctctcc acccaagcgg ccggagaacc 9360

tgcgtgcaat ccatcttgtt caatcatgcg aaacgatcct catcctgtct cttgatcaga 9420

tcttgatccc ctgcgccatc agatccttgg cggcaagaaa gccatccagt ttactttgca 9480

gggcttccca accttaccag agggcgcccc agctggcaat tccggttcgc ttgctgtcca 9540

taaaaccgcc cagtctagct atcgccatgt aagcccactg caagctacct gctttctctt 9600

tgcgcttgcg ttttcccttg tccagatagc ccagtagctg acattcatcc caggtggcac 9660

ttttcgggga aatgtgcgcg cccgcgttcc tgctggcgct gggcctgttt ctggcgctgg 9720

acttcccgct gttccgtcag cagcttttcg cccacggcct tgatgatcgc ggcggccttg 9780

gcctgcatat cccgattcaa cggccccagg gcgtccagaa cgggcttcag gcgctcccga 9840

aggt 9844

<210> 59
<211> 7250
<212> DNA
<213> Artificial Sequence

<220>
<223>  vector

<400>  59
ctcgggccgt ctcttgggct tgatcggcct tcttgcgcat ctcacgcgct cctgcggcgg      60

cctgtagggc aggctcatac ccctgccgaa ccgcttttgt cagccggtcg gccacggctt     120

ccggcgtctc aacgcgcttt gagattccca gcttttcggc caatccctgc ggtgcatagg     180

cgcgtggctc gaccgcttgc gggctgatgg tgacgtggcc cactggtggc cgctccaggg     240

cctcgtagaa cgcctgaatg cgcgtgtgac gtgccttgct gccctcgatg ccccgttgca     300

gccctagatc ggccacagcg gccgcaaacg tggtctggtc gcgggtcatc tgcgctttgt     360

tgccgatgaa ctccttggcc gacagcctgc cgtcctgcgt cagcggcacc acgaacgcgg     420

tcatgtgcgg gctggtttcg tcacggtgga tgctggccgt cacgatgcga tccgccccgt     480

acttgtccgc cagccacttg tgcgccttct cgaagaacgc cgcctgctgt tcttggctgg     540

ccgacttcca ccattccggg ctggccgtca tgacgtactc gaccgccaac acagcgtcct     600

tgcgccgctt ctctggcagc aactcgcgca gtcggcccat cgcttcatcg gtgctgctgg     660

ccgcccagtg ctcgttctct ggcgtcctgc tggcgtcagc gttgggcgtc tcgcgctcgc     720

ggtaggcgtg cttgagactg gccgccacgt tgcccatttt cgccagcttc ttgcatcgca     780

tgatcgcgta tgccgccatg cctgcccctc ccttttggtg tccaaccggc tcgacggggg     840

cagcgcaagg cggtgcctcc ggcgggccac tcaatgcttg agtatactca ctagactttg     900

cttcgcaaag tcgtgaccgc ctacggcggc tgcggcgccc tacgggcttg ctctccgggc     960

ttcgccctgc gcggtcgctg cgctcccttg ccagcccgtg gatatgtgga cgatggccgc    1020

gagcggccac cggctggctc gcttcgctcg gcccgtggac aaccctgctg gacaagctga    1080

tggacaggct gcgcctgccc acgagcttga ccacagggat tgcccaccgg ctacccagcc    1140

ttcgaccaca tacccaccgg ctccaactgc gcggcctgcg gccttgcccc atcaattttt    1200

ttaattttct ctggggaaaa gcctccggcc tgcggcctgc gcgcttcgct tgccggttgg    1260

acaccaagtg gaaggcgggt caaggctcgc gcagcgaccg cgcagcggct tggccttgac    1320

gcgcctggaa cgacccaagc ctatgcgagt gggggcagtc gaaggcgaag cccgcccgcc    1380

tgccccccga gcctcacggc ggcgagtgcg ggggttccaa gggggcagcg ccaccttggg    1440

caaggccgaa ggccgcgcag tcgatcaaca agccccggag gggccacttt ttgccggagg    1500

gggagccgcg ccgaaggcgt gggggaaccc cgcaggggtg cccttctttg ggcaccaaag    1560

aactagatat agggcgaaat gcgaaagact taaaaatcaa caacttaaaa aaggggggta    1620

cgcaacagct cattgcggca ccccccgcaa tagctcattg cgtaggttaa agaaaatctg    1680

taattgactg ccacttttac gcaacgcata attgttgtcg cgctgccgaa aagttgcagc    1740

tgattgcgca tggtgccgca accgtgcggc accctaccgc atggagataa gcatggccac    1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatcccccg ggctgcagga attcgatatc aagcttctta agtaataaaa 3300

ataagagtta ccttaaatgg taactcttat ttttttaata ttgtttcata gtatttcttt 3360

ttatttcgag tagtcgtaaa agcccttgcc gctcttgcgg cccagccagc ccgcccggac 3420

atactttttc agcagcgtgt gcgggcgata cttcgaatcg ccggtttccg agtacagcac 3480

gtccatgatg gccaggcaaa tgtcgaggcc gataaagtcc cccagttcca gcgggcccat 3540

cgggtggttc gcccccagct tcatggcctt atcaatatcc tcgaccgagg caatcccttc 3600

ggcgaggatg cccaccgctt cattgatcat cgggatcaga atgcggttga cgacgaaccc 3660

cggcgcctcg gcgacttcca cggggtcctt gccaatggca atcgacgtct ctttcaccgc 3720
gtcgaaggtt tcctgcgacg tggcaatgcc gcgaatgacc tccacgagtt tcatgacggg 3780
ggcgggatta aagaaatgca tgccgatcac tttgtcgttg gtcttggtcg cgctcgccac 3840
ctccgtgatc gagagcgagc tggtgttgga ggcgagaatc gtctccggtt tgcagatgtt 3900
gtcgagatcc gcaaagatct gtttcttaat gtccatgcgc tcgacggcgg cttcaatcac 3960
gaggtcgcag tccgcggcca tgttcaggtc cacggtcccg ctgatgcggg tgagaatctc 4020
caccttggtg gcctcttcga tcttgccttt tttgaccagt ttcgacaggt tcttgttaat 4080
gaaatccagg ccgcgatcga cgaattcatc cttgatgtcc cggaggacca cctcgaagcc 4140
tttggcggcg aaggcctgcg cgatgccgga ccccatcgtc cccgcgccaa tgacgcacac 4200
cttcttcatt cttgaatctc ctgaaactag cacttttcca gcaggatcgc ggtgccctgg 4260
ccgccgccga tgcacagggt cgccagcccc ttcttggcgt cgcgcttctg catcgcgtgc 4320
accagggtca ccaggatgcg ggcgcccgag gcgccgatcg ggtgccccag ggcgatggcg 4380
ccgccattca cgttcacttt gttcatgtcg aacttcaggt ccttggcgac ggccagcgac 4440
tgggcggcaa aggcctcgtt cgactcgatc aggtccagct cgtcgaccgt ccagccggct 4500
ttctcgatcg ccgccttggt ggcgtagaac gggccgtagc ccatgatggc cgggtccacg 4560
ccggccgagc catacgacac gatcttcgcc agcggtttca cgcccagctc cttggccttt 4620
tcggccgaca tgatcaccag cacggccgcg cagtcgttca ggcccgaggc gttgcccgcg 4680
gtcacggtgc cgtccttctt gaaggccggc ttcagcttgg ccaggccctc gatcgtcgac 4740
ccgaagcgcg ggtgctcgtc cgtgtccacc acggtctcgc ccttgcggcc cttaatcacc 4800
accggcacga tctcgtcctt gaactggccc gacttgatgg cttcctccgc cttcttttgc 4860
gaggccaggg cgaactcgtc ctgctcctcg cgcgagatgt tccagcgctc ggcgatgttc 4920
tcggcggtga tgcccatgtg gtagtcgttg aaggcgtccc acaggccgtc ggtgatcatc 4980
tcgtccacga acttggcgtt ccccatgcgg tagccccagc gggcgttgtt ggccaggtac 5040
ggggcgcgcg acatgtttcc catgccgccg gcaatgatca cgtcggcgtc gccggccttg 5100
atgatctggg ccgccagcga cacggtgcgc aggcccgagc cgcacacctt gttgatggtc 5160
atggcgggga tctccaccgg gaggccggct ttgaagctcg cctggcgggc cgggttctgg 5220
ccgagcccgg cctgcagcac gttgcccagg atcacctcgt tcacgtcctc cggcttgatg 5280
ccggccttct tcacggcctc cttaatggcg gtggcgccca ggtccacggc gggcacgtct 5340
ttcaggctct tgccgtacga gccgatcgcg gtgcgcacgg ccgaggcaat caccacctcc 5400
ttcattcttg aatctcctga aaggtaccca gcttttgttc cctttagtga gggttaattg 5460
cgcgcttggc gtaatcatgg tcatagctgt ttcctgtgtg aaattgttat ccgctcacaa 5520
ttccacacaa catacgagcc ggaagcataa agtgtaaagc ctggggtgcc taatgagtga 5580

gctaactcac attaattgcg ttgcgctcac tgcccgcttt ccagtcggga aacctgtcgt 5640

gccagctgca ttaatgaatc ggccaacgcg cggggagagg cggtttgcgt attgggcgca 5700

tgcataaaaa ctgttgtaat tcattaagca ttctgccgac atggaagcca tcacaaacgg 5760

catgatgaac ctgaatcgcc agcggcatca gcaccttgtc gccttgcgta taatatttgc 5820

ccatgggggt gggcgaagaa ctccagcatg agatccccgc gctggaggat catccagccg 5880

gcgtcccgga aaacgattcc gaagcccaac ctttcataga aggcggcggt ggaatcgaaa 5940

tctcgtgatg gcaggttggg cgtcgcttgg tcggtcattt cgaaccccag agtcccgctc 6000

agaagaactc gtcaagaagg cgatagaagg cgatgcgctg cgaatcggga gcggcgatac 6060

cgtaaagcac gaggaagcgg tcagcccatt cgccgccaag ctcttcagca atatcacggg 6120

tagccaacgc tatgtcctga tagcggtccg ccacacccag ccggccacag tcgatgaatc 6180

cagaaaagcg gccattttcc accatgatat tcggcaagca ggcatcgcca tgggtcacga 6240

cgagatcctc gccgtcgggc atgcgcgcct tgagcctggc gaacagttcg gctggcgcga 6300

gcccctgatg ctcttcgtcc agatcatcct gatcgacaag accggcttcc atccgagtac 6360

gtgctcgctc gatgcgatgt ttcgcttggt ggtcgaatgg gcaggtagcc ggatcaagcg 6420

tatgcagccg ccgcattgca tcagccatga tggatacttt ctcggcagga gcaaggtgag 6480

atgacaggag atcctgcccc ggcacttcgc ccaatagcag ccagtccctt cccgcttcag 6540

tgacaacgtc gagcacagct gcgcaaggaa cgcccgtcgt ggccagccac gatagccgcg 6600

ctgcctcgtc ctgcagttca ttcagggcac cggacaggtc ggtcttgaca aaaagaaccg 6660

ggcgcccctg cgctgacagc cggaacacgg cggcatcaga gcagccgatt gtctgttgtg 6720

cccagtcata gccgaatagc ctctccaccc aagcggccgg agaacctgcg tgcaatccat 6780

cttgttcaat catgcgaaac gatcctcatc ctgtctcttg atcagatctt gatcccctgc 6840

gccatcagat ccttggcggc aagaaagcca tccagtttac tttgcagggc ttcccaacct 6900

taccagaggg cgccccagct ggcaattccg gttcgcttgc tgtccataaa accgcccagt 6960

ctagctatcg ccatgtaagc ccactgcaag ctacctgctt tctctttgcg cttgcgtttt 7020

cccttgtcca gatagcccag tagctgacat tcatcccagg tggcactttt cggggaaatg 7080

tgcgcgcccg cgttcctgct ggcgctgggc ctgtttctgg cgctggactt cccgctgttc 7140

cgtcagcagc ttttcgccca cggccttgat gatcgcggcg gccttggcct gcatatcccg 7200

attcaacggc cccagggcgt ccagaacggg cttcaggcgc tcccgaaggt 7250

<210> 60
<211> 14113
<212> DNA
<213> Artificial Sequence

<220>
<223>  vector

<400>  60
ctcgggccgt ctcttgggct tgatcggcct tcttgcgcat ctcacgcgct cctgcggcgg      60

cctgtagggc aggctcatac ccctgccgaa ccgcttttgt cagccggtcg gccacggctt     120

ccggcgtctc aacgcgcttt gagattccca gcttttcggc caatccctgc ggtgcatagg     180

cgcgtggctc gaccgcttgc gggctgatgg tgacgtggcc cactggtggc cgctccaggg     240

cctcgtagaa cgcctgaatg cgcgtgtgac gtgccttgct gccctcgatg ccccgttgca     300

gccctagatc ggccacagcg gccgcaaacg tggtctggtc gcgggtcatc tgcgctttgt     360

tgccgatgaa ctccttggcc gacagcctgc cgtcctgcgt cagcggcacc acgaacgcgg     420

tcatgtgcgg gctggtttcg tcacggtgga tgctggccgt cacgatgcga tccgccccgt     480

acttgtccgc cagccacttg tgcgccttct cgaagaacgc cgcctgctgt tcttggctgg     540

ccgacttcca ccattccggg ctggccgtca tgacgtactc gaccgccaac acagcgtcct     600

tgcgccgctt ctctggcagc aactcgcgca gtcggcccat cgcttcatcg gtgctgctgg     660

ccgcccagtg ctcgttctct ggcgtcctgc tggcgtcagc gttgggcgtc tcgcgctcgc     720

ggtaggcgtg cttgagactg gccgccacgt tgcccatttt cgccagcttc ttgcatcgca     780

tgatcgcgta tgccgccatg cctgcccctc ccttttggtg tccaaccggc tcgacggggg     840

cagcgcaagg cggtgcctcc ggcgggccac tcaatgcttg agtatactca ctagactttg     900

cttcgcaaag tcgtgaccgc ctacggcggc tgcggcgccc tacgggcttg ctctccgggc     960

ttcgccctgc gcggtcgctg cgctcccttg ccagcccgtg gatatgtgga cgatggccgc    1020

gagcggccac cggctggctc gcttcgctcg gcccgtggac aaccctgctg gacaagctga    1080

tggacaggct gcgcctgccc acgagcttga ccacagggat tgcccaccgg ctacccagcc    1140

ttcgaccaca tacccaccgg ctccaactgc gcggcctgcg gccttgcccc atcaattttt    1200

ttaattttct ctggggaaaa gcctccggcc tgcggcctgc gcgcttcgct tgccggttgg    1260

acaccaagtg gaaggcgggt caaggctcgc gcagcgaccg cgcagcggct tggccttgac    1320

gcgcctggaa cgacccaagc ctatgcgagt gggggcagtc gaaggcgaag cccgcccgcc    1380

tgccccccga gcctcacggc ggcgagtgcg ggggttccaa gggggcagcg ccaccttggg    1440

caaggccgaa ggccgcgcag tcgatcaaca agccccggag gggccacttt ttgccggagg    1500

gggagccgcg ccgaaggcgt gggggaaccc cgcaggggtg cccttctttg ggcaccaaag    1560

aactagatat agggcgaaat gcgaaagact taaaaatcaa caacttaaaa aaggggggta    1620

cgcaacagct cattgcggca ccccccgcaa tagctcattg cgtaggttaa agaaaatctg    1680

taattgactg ccacttttac gcaacgcata attgttgtcg cgctgccgaa aagttgcagc    1740

tgattgcgca tggtgccgca accgtgcggc accctaccgc atggagataa gcatggccac    1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatccagca ggctgcctcg ataagcaaaa agggcggccc cgcggggccg 3300

ccctttttct ttccggcgac tgtcaggcca ctcagttgtt ggcggccttc acctgcgcga 3360

tcagctccgg gaccactttg ttcacgtcgc ccacgatcgc caggtcggcc accttcataa 3420

tcggggcctc cacgtcctta ttgatggcga tgatgtagtc cgagtcctgc atgccggcca 3480

ggtgctggat cgcccccgag atgccgcagg cgatgtacag ggtcgggcgc acggtcttgc 3540

cggtctggcc cacctggagg tctttgtcca cccactcttt ctcgatggcc gcgcgcgagg 3600

cggcgatggt gccgcccagg agcgaggcca gctcctccag tttttcgaag ttctccttcg 3660

agcccacgcc gcggcccccg gccacgagca ccttggcctc gccgatgtcc gcgatgtctt 3720

tggccagctt caccactttc gacactttgg tgcggatgtc ggacgcggtc agcttgatgg 3780

cgactttctc gatcttatcg tcggacacgt tggcgtcgtt caccggcagc ttctcgaaga 3840

agacgccggg gcggaccgtg gccatctgcg ggcggtggtc cgagcagacg atggtggcga 3900

tcaggttgcc gccgaacgcc gggcgggtcg ccaggaggtc ccggttctcc acgtcgatgt 3960

ccagcgaggt gcagtcggcg gtcaggccgg tcgacaggcg cgccgcgatc cgcgggccca 4020

ggtcccgccc gatgaacgtg gcgccaatga acaggatctc cggcttgcgc tcgttcacga 4080

ggtcgcagat caccttcgcg tagccgtcgg tcgagaagtg ggcgaggagt tcattgtcgg 4140

ccgcgagcac cttatccgcg ccgtggctca gcaggtcctt cgacatcttc tcggtgttgt 4200

ggcccagcag cacggcggtc agttccaccc ccagcttctc ggccatctcc ttgcccttgc 4260

ccagcagctc cagcgacacc ttctgcagct cgccgtcgcg ctgctccgca aacacccaca 4320

cgcccttgta gtcggccttg ttcattgaaa aatccctcct aacttaaata tgtgttcttc 4380

ttttagttct gcgagagcat atcggccgct tccttcaccg gtttatcaat gacctcgccc 4440

tgccctttca cttctttcgt gctcgacttc ttcactttgg tcggcgaccc cttcaggccc 4500

agattggctt tatccacgtc gatatcatcc gcggtccaca ttttcacctc cttgtcgaac 4560

gcgccgaaga ttttctccac cgacatgtac cgcgggacgt tcagttcctt aatggccgtc 4620

aggagcaccg gggtcttgac ttccacgacc tcgtacccat cctcccaggc cttccggatc 4680

ttgagcgtgt ccccatccac ctccactttt tccacgtagg tgacttgggg aatcccgaga 4740

tgttccgcaa tctccggccc gacctgcgcc gtatcgccgt cgatggcctg gcggccggca 4800

aagacaatgt catatttcag cttcttaatg ccggcggcaa tggtatgcga ggtggcgagg 4860

gtgtccgcgc ccccaaacgc ccggtccgtg agcagcaccg cctcatcggc ccccatggcc 4920

agcgcttcga ccagggcgtt cttggcttgg gggggcccca tcgagatgac cgtgacgtgc 4980

gccccatagt tatctttcag cacgagggct tcctcgagcg cgtttttgtc gtcggggtta 5040

atgatggacg ggacgccctc ccgaatcagg gtgcctttga cgggatcaat gcggacctcg 5100

gcggtgtcgg ggacttgttt gaggcagacc acaatattca tcctcttaac ctccttaaat 5160

tagcggaaga tcttgcccga gatcaccagc ttctgcacct ccgaggtgcc ctcgtagatc 5220

tccgtgatct tggcgtcgcg catcatgcgc tccacggggt agtccttggt gtagccatag 5280

ccgccgaaca gctgcacggc cttggtggtc acgtccatgg ccacgttggc ggcatgcagc 5340

ttggcgcggg cggcatccac ggtgtacggc aggcccgcct gcttcaggta ggcggccttg 5400

tacaccaggt agcgggccga ctcgatggcc acgtccatgt cggccatcat ccaggccagg 5460

ccctggaact tgtccagcga gcggccgaac tgcttgcgtt ccttcatgta cgcgcgggcc 5520

tcgttaaagg cgccttcggc gatgcccagg gcctgggccg cgatgccgat gcggcccccg 5580

tccagggtct tcatggcgat cgggaagccc ttgccctcct tgccaatcat gttctccacc 5640

ggcacgatca tgtcctcgaa caccagctcg gtggtcgacg acgcgcggat gcccagcttt 5700

tgctccacct tgccgatcga gaagcccttg aagcccttct cgatgataaa ggccgagatg 5760

cccttggtcc ccttggtgcg gtcggtcatg gcgaagatga cgaaggtgtc ggcgacgccg 5820

ccgttggtga tgaagatttt cgagccgttg atcacgtagt ggtcgccctc cagcacggcc 5880

acggtctgct gggcgcccga atcggtgccg gcgttcggct cggtcaggcc gtaggcgccg 5940

atcttctccc ccttggccag cggcaccagg tacttctgct tctgctcctc ggtgccgtgc 6000

tcgttaatca gcgaggcgca cagcgaggtg tgggccgaca ggatcacgcc ggtggtgccg 6060

cacaccttcg acagctcctc cacggcgatg atgtacgaca gcacgtcgcc gccggccccg 6120

ccgtactcct tcgagaacgg gatgcccatc atgccgtact ggcccatctt cttcacgttc 6180

tccatcggga accgctccgt ctcgtcgatc tccgcggcaa tcggcttcac ctcgttctcg 6240

gcgaactccc ggaccatctg gcgcaccagc tcttgctcgc gcgtcaggtt gaagtccata 6300

taaacttacc tcctagcggt tcttgaaccc ttcgatcttg cgcttctcaa tgaaggcggt 6360

catggcgtct ttctggtcct cggtcgagaa gcattccccg aacgcttccg actcgaaggc 6420

gagggccgtg tcaatgtcgc actgcatgcc gcggttaatg gcttgcttcg acagtttgac 6480

ggccaccggg gcattgctca cgattttgtt ggcgatctcc ttggcggtgt tcatcagttc 6540

cgagggctcc accaccttgt tcaccaggcc aatgcgcagg gcctcatcgg ccttgatgtt 6600

ctgcgcggtg aaaatcagct gcttggccat gcccatgccg acgaggcgcg acagccgctg 6660

ggtgccgcca aagcccgggg tgatgcccag gcccacttcc ggctggccga agcgggcgtt 6720

cgaggaggcg atgcggatat cgcaggacat cgcaatttcg cacccgccgc ccagggcgaa 6780

cccattcacg gccgcaatca ccggtttttc cagcagctcc agccgccgaa acactttgtt 6840

ccccagaatg ccgaacttgc gcccctcgat ggtattcatt tctttcatct ccgagatgtc 6900

cgccccggcg acgaagctct tctcgcccgc gcccgtcagg atcacggcga gcacctccga 6960

gtcgttctcg atttcgccga tcacgtagtc catctctttc agggtgtccg agttcagggc 7020

gttgagggcc ttcgggcggt tgatggtcac caccgcgacc ttgccttctt tctccaggat 7080

gacattgttg agttccatga ctaatcctcc taaaatgtga aattgttatc cgctcacaat 7140

tccacacaac atacgagccg gaagcataaa gtgtaaagcc tggggaagct tggggagaac 7200

aatcagcccg gcaggggccg ggctgattgt gcctgcgtgc cttagaacga cttgatgtag 7260

atgtccttca gctccgagat cagcgggtag cgcgggttgg cggtggtgca ctggtcgtcg 7320

aaggccagct ccgacatctt gtccagggtg ttgtagaaat ccttcttgtt gatgccggcg 7380

gccgagatgt tctgcgggat cgacaggtcg atcttcagct tcgagatggc ctcgatcagg 7440

gcggtcacct tctccgtgtc cgaggtgccc ttcaggttca ggtactcggc gatctcggcg 7500

tacttgcgct tggcgttcgg cgacttgtac tgcgggaagg cggtctgctt ggtcgggcag 7560

tcggtggcgt tgtacttgat cacctcctcg atcagcacgg cgcaggcgat gccgtgcggc 7620

acgtggtgca tggcgcccag cttgtgggcc atcgagtggc acacgcccag gaaggcgttg 7680

gcgaaggcca tgccggcgat gttcgaggcg tgggccatct tctcgcgggc ctcgatgtcg 7740

ttggtgccgt tcttgtaggc gcgcggcagg tacttgaaga tcatcttgat ggcgcgcagg 7800

gccagctcgt cggtgtagtc cgtggccatc accgacacgt aggcctcgat ggcgtgcacc 7860

agggcatcaa tgccggtggc ggcggtcagc ttgcgcggca tgttcagcat cagctcggtg 7920

tcaatgatgg ccatgttcgg ggtcagttcg tacgaggtca gcgggtactt catgccggtc 7980

tcgtcgttgg tgatcacggc gaacggggtg gcctccgagc cggtgccggc ggtggtcggg 8040

atggccaccg agatggcctt ggtgcccagc ttcgggaagt tgcagatgcg cttgcggatg 8100

tccatgaaat tgatggcgag gttctcgatc tccgcctccg ggtactcgta cagcaggtgc 8160

atcaccttgg cggcgtccat cggcgagccg ccgccgatcg agatgatggt gtccggctcg 8220

aagttcagca tctccttggc gcccttcttc accgagtcaa tggtcgggtc cgacttgata 8280

tcggtgaaga tcgagtactt gatgtcgatc tcgtccagca ccttggtgat cttgttcacg 8340

tagcccagct tgaacaggtc cttgtcggtc acgatgaagg cgcgcttctt gttcatgtcc 8400

ttcagctcct tcagggcgaa gcgcaggcag ccgtacttga agtagatctt ctgcggcacc 8460

ttgaaccaca gcatattctc gcggcgctcg gccaccgact tgatgttcag caggtgcttc 8520

ggctccacgt tctgcgacac cgagttgccg ccccaggtcc cgcagcccag ggtgaacgac 8580

ggggcgatgg cgaagttgta caggtcgccc gaggcgccct gcgacgacgg catgttgatg 8640

aaggtgcgcg aggtcttcat ggccaggccg aactccttca ccttgtcctt gttgttctgc 8700

gagtcgatgt acagcgacga ggtgtggccc gagccgccca gctcgatcag gcgctggccc 8760

ttcttgaggg cctcatcgaa gtccttcacc ttgtacatgg ccagcaccgg cgacagcttc 8820

tcgtgcgaga acagctccga cttctccacc gactgcacct cgccgatgag gatcttggtg 8880

gtctgcggca cctcgatgcc ggccatcttg gcgatgatgt aggccgactt gcccacgatg 8940

tcggcgttga tggcgccgtt cttgaacatc gtctccttga tcttggcgat ctcgttctgg 9000

ttcaggatgt acgagccgcg cttcacgaac tcctccttca ccttctcgta gatggagttc 9060

atcaccagga tcgactgctc cgaggcgcag atcacgccgt tgtcgtaggt cttcgacagg 9120

atgatcgacg acacggccat atcgatgtcg gcggactcgt cgatgatggc cggggtgttg 9180

cccgcgccca cgccgatggc cggcttgccc gacgagtagg cggccttcac catcgacggg 9240

ccgccggtgg ccaggatgat atcggcctcc gacatcaggt cctgcgacag ctcgatcgac 9300

ggctcgtcaa tccagccgat gatgttcttc ggggccccgg ccttcacggc ggcgtccagg 9360

atcagcttgg cggcggcgat ggtcgacttc ttggcgcgcg ggtgcggcga gaagaagatg 9420

gcgttgcggg tcttcagcga gatcagcgac ttgaagatgg cggtcgaggt cgggttcgtg 9480

gtcggcacga tggcggccac gatgccgatc ggctcggcca ccttggtgat gcccagcgag 9540

tcgtcgtggt cgataatgcc gcaggtcttc tcgttcttgt acttgttgta gatgtactcg 9600

gcggcgaagt ggttcttgat gatcttgtcc tccaccaggc cgatgccggt ctcctccacg 9660

gccagcttgg ccaggttgat gcgctccttg gcggcggcaa tggcgcactg cttgaagatc 9720

ttgtccacct gctcctgggt gtaggtggcg aacttcttct gggcctcgcg cagctcgttc 9780

agcttctgct tcagctcctt ctggttggtc accttcattc ttgaatctcc tgaaagccgg 9840

tgcttacggc agcttgacca cggcttcccc ggtgacggcc agggccccgc cttgggtaaa 9900

gatccgggtc gtcagcgtgg cgatcggttt gtcctcgcgc agcgcggtga cttccacctc 9960

ggcggtcact tcgtccccca cgaacaccgg cagtttgaac gagagcgatt ggcccaggta 10020

gatgctcccc ttgccgggga gctgctggcc gaggaggccc gagaacagcg aggcgagcag 10080

catgccatgc acgatggggc gctcgaacgc ggtggtcgcg gcaaacgcgg ggtccaggtg 10140

cagcgggttg aagtcctcgg agagcgccgc aaaggccgcc acctccgccg cgccaaaccg 10200

cttggacagc cgggcttttt gcccgacctc cagcgactgg gccgacatgc ggcgtcctcc 10260

tctgtttcag cccatatgca ggccgccgtt gagcgagaag tcggcgccgg tcgagaaacc 10320

ggactcctcc gacgacaacc aggcgcagat cgaggcgatc tcttccggca ggcccaggcg 10380

cttgaccggg atcgtcgcga cgatcttgtc gagcacgtcc tggcggatcg ccttgaccat 10440

gtcggtggcg atatagcccg gagagaccgt gttgacggtc acgcccttgg tcgccacttc 10500

ctgcgccagt gccatggtga agccatgcag gccggccttg gcggtggagt agttggtctg 10560

gccgaactgg cccttctgcc cgttcaccga cgagatgttg acgatgcggc cccagccacg 10620

gtcggccatg ccgtcgatca cctgcttggt gacgttgaac agcgaggtca ggttggtgtc 10680

gatcaccgca tcccagtcgg cgcgggtcat cttgcggaac accacgtcgc gggtgatacc 10740

ggcgttgttg atcagcacat caacctcgcc gacctcggac ttgaccttgt cgaatgcggt 10800

cttggtcgag tcccagtcag ccacattgcc ttccgaggca atgaaatcga agcccagggc 10860

cttctgctgc tccagccact tttcgcggcg cggcgagttg gggccgcaac cggccaccac 10920

acgaaagcca tccttggcca gccgctggca aatggcggtt ccgataccac ccatgccgcc 10980

ggtcacatac gcaatgcgct gagtcatgtc cactccttga ttggcttcgt tatcgtcgcc 11040

gggtccgcgc caaccgcgcg cggccccgga aaaccccttc cttatttgcg ctcgactgcc 11100

agcgccacgc ccatgccgcc gccgatgcac agcgaggcca ggcccttctt cgcgtcacgg 11160

cgcttcatct cgtgcagcag cgtcaccagg atacggcagc ccgacgcgcc gatcgggtgg 11220

ccgatggcga tggcgccgcc gttcacattg accttggagg tgtcccagcc catctgctgg 11280

tgcaccgcca gcgcctgcgc ggcaaaggcc tcgttgatct ccatcaggtc caggtcttgc 11340

ggggtccact cggcgcgcga cagggcgcgc ttggaggccg gcaccgggcc catgcccatc 11400

accttgggat cgacaccggc gttggcatag ctcttgatcg tggccagcgg ggtcaggccc 11460

agttccttgg ccttggccgc cgacatcacc accaccgcgg cggcgccgtc gttcaggccc 11520

gaggcgttgg ccgcggtcac cgtgccggcc ttgtcgaagg cgggcttgag gccggacatg 11580

ctgtccagcg tggcgccctg gcgcacgaac tcgtcggtct tgaaggccac cgggtcgccc 11640

ttgcgctgcg ggatcagcac cgggacgatc tcttcgtcaa acttgccggc cttctgcgcg 11700

gcttcggcct tgttctgcga gccgacggcg aactcatcct gcgcctcgcg tgtgatgccg 11760

tattccttgg ccacgttctc ggcggtgatg cccatgtggt actggttgta cacgtcccac 11820

aggccgtcga cgatcatggt gtcgaccagc ttggcatcgc ccatgcggaa accatcgcgc 11880

gagcccggca gcacgtgcgg ggcggcgctc atgttttcct ggccgccggc caccacgatc 11940

tcggcgtcgc ccgccatgat cgcgttggcg gccagcatca cggccttcag gcccgagccg 12000

cacaccttgt tgatggtcat ggccggcacc atcgccggca ggccggcctt gatcgcggcc 12060

tggcgtgcgg ggttctggcc cgaaccggcg gtcagcacct ggcccatgat gacttcgctc 12120

acctgctccg gcttgacgcc ggcgcgctcc agcgcggcct tgatgaccac ggcacccagt 12180

tccggtgccg ggatcttggc cagcgagccg ccaaacttgc cgaccgcggt gcgggcggcg 12240

gatacgatga caacgtcagt cattgtgtag tcctttcaat ggaaaggtac ccagcttttg 12300

ttccctttag tgagggttaa ttgcgcgctt ggcgtaatca tggtcatagc tgtttcctgt 12360

gtgaaattgt tatccgctca caattccaca caacatacga gccggaagca taaagtgtaa 12420

agcctggggt gcctaatgag tgagctaact cacattaatt gcgttgcgct cactgcccgc 12480

tttccagtcg ggaaacctgt cgtgccagct gcattaatga atcggccaac gcgcggggag 12540

aggcggtttg cgtattgggc gcatgcataa aaactgttgt aattcattaa gcattctgcc 12600

gacatggaag ccatcacaaa cggcatgatg aacctgaatc gccagcggca tcagcacctt 12660

gtcgccttgc gtataatatt tgcccatggg ggtgggcgaa gaactccagc atgagatccc 12720

cgcgctggag gatcatccag ccggcgtccc ggaaaacgat tccgaagccc aacctttcat 12780

agaaggcggc ggtggaatcg aaatctcgtg atggcaggtt gggcgtcgct tggtcggtca 12840

tttcgaaccc cagagtcccg ctcagaagaa ctcgtcaaga aggcgataga aggcgatgcg 12900

ctgcgaatcg ggagcggcga taccgtaaag cacgaggaag cggtcagccc attcgccgcc 12960

aagctcttca gcaatatcac gggtagccaa cgctatgtcc tgatagcggt ccgccacacc 13020

cagccggcca cagtcgatga atccagaaaa gcggccattt tccaccatga tattcggcaa 13080

gcaggcatcg ccatgggtca cgacgagatc ctcgccgtcg ggcatgcgcg ccttgagcct 13140

ggcgaacagt tcggctggcg cgagcccctg atgctcttcg tccagatcat cctgatcgac 13200

aagaccggct tccatccgag tacgtgctcg ctcgatgcga tgtttcgctt ggtggtcgaa 13260

tgggcaggta gccggatcaa gcgtatgcag ccgccgcatt gcatcagcca tgatggatac 13320

tttctcggca ggagcaaggt gagatgacag gagatcctgc cccggcactt cgcccaatag 13380

cagccagtcc cttcccgctt cagtgacaac gtcgagcaca gctgcgcaag gaacgcccgt 13440

cgtggccagc cacgatagcc gcgctgcctc gtcctgcagt tcattcaggg caccggacag 13500

gtcggtcttg acaaaaagaa ccgggcgccc ctgcgctgac agccggaaca cggcggcatc 13560

agagcagccg attgtctgtt gtgcccagtc atagccgaat agcctctcca cccaagcggc 13620

cggagaacct gcgtgcaatc catcttgttc aatcatgcga aacgatcctc atcctgtctc 13680

ttgatcagat cttgatcccc tgcgccatca gatccttggc ggcaagaaag ccatccagtt 13740

tactttgcag ggcttcccaa ccttaccaga gggcgcccca gctggcaatt ccggttcgct 13800

tgctgtccat aaaaccgccc agtctagcta tcgccatgta agcccactgc aagctacctg 13860

ctttctcttt gcgcttgcgt tttcccttgt ccagatagcc cagtagctga cattcatccc 13920

aggtggcact tttcggggaa atgtgcgcgc ccgcgttcct gctggcgctg ggcctgtttc 13980

tggcgctgga cttcccgctg ttccgtcagc agcttttcgc ccacggcctt gatgatcgcg 14040

gcggccttgg cctgcatatc ccgattcaac ggccccaggg cgtccagaac gggcttcagg 14100

cgctcccgaa ggt 14113

<210> 61
<211> 11340
<212> DNA
<213> Artificial Sequence

<220>
<223> vector

<400> 61
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaattttct ctggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag ggccactttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatggccca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatccagca ggctgcctcg ataagcaaaa agggcggccc cgcggggccg 3300

ccctttttct ttccggcgac tgtcaggcca ctcagaactg ctcggccgtc atcgcattca 3360

ccggtgccgc accactgacg atggcatcgc gcaaagccga cgcctgcgac aggatgtgct 3420

ccgcaaagaa atgcgcggtg gcgatcttgg cgtcataaaa gcccgggtcc ccggcccgct 3480

tcgcatcggc ggccagcatc gcacggccga actgccagcc ggagaacacg atgccgcaca 3540

gcttcaggta aggcacgctg ccggcaaaga cggcgttcgg gtcggacttg gcattggcca 3600

cgacaaacgc caccacgtct tccagcgccg cgcgaccctt ggccagttgc gcctgtaccg 3660

cggtgaaccc ggcgcagctg tgattgccca gcgcagcctc ggtctcggcg atctgcgcgc 3720

agatcgcacg cgccacggcg ccgccgtcgc gcacggtctt gcggccgatc aggtcgttgg 3780

cctggattgc cgtggtgcct tcgtagatcg gcaggatacg cgcgtcgcgg tagtgctgcg 3840

ccgcgccggt ctcctcgata aagcccatgc cgccgtgcac ctgcacaccc aggctggtga 3900

cgtcgatcga cagctcggtg ctccagcctt tcaccaccgg caccaggaac tcgtagaagg 3960

cctggttctg ctggcgcacc gcttcgtcgg gatgctggtg cgccgcgtcg ctggcggccg 4020

cggccacgta ggccacggcg cgcgcgcctt cggtcagcgc gcgcatggtc atcagcatgc 4080

gcttgacgtc agggtggtgg atgatggtca ccgcctcgcg cgccgaacca tcgaccgggc 4140

ggctctgcac gcgctcgcgc gcaaaggcca ccgcctgctg gtaggcgcgt tcggatacgg 4200

cgatgccctg catgccgacc gagaagcgtg ccgagttcat catgatgaac atgtactcga 4260

ggccacggtt ctcttcgccg accagcgtgc cgatggcgcc gccatggtcg ccgaactgca 4320

gcacggccgt ggggctcgcc ttgatgccca gcttgtgctc gatcgagacg caatgcacgt 4380

cgttgcgctc accggtgctg ccgtcggcgt tgaccatgaa cttcggcacg atgaacagcg 4440

agatgccctt gacgccctcg ggcgcggtgg gcgtgcgggc cagcacgaga tggacgatgt 4500

tcttcgccat gtcgtgctcg ccgtaggtga tgaagatctt ggtgccgaac actttgtagg 4560

tgccgtcgcc ctgcggctcg gcacgggtgc gcactgcggc cagatccgag ccggcctgcg 4620

gctcggtcag gttcatggtg ccggtccact cgcccgagat cagcttgggc aggaaggtgg 4680

ccttctgctc atccgtgccg gccgtcagca gcgcttcgat ggcaccatcg gtcagcagcg 4740

ggcacagcgc gaacgacagg ttcgcggtgt tcagcatctc gttgcaggcg gtggcgacca 4800

gcttgggcag gccctggccg ccaaactcct gcggatgcag cacgccctgc cagccgccct 4860

cgccgaactg gcggaaggcc tccttaaagc cgggcgtggt ggtgaccacg ccgtccttcc 4920

agctgctcgg gtccaggtcg ccggcgcggt tcagcggcgc gacgacctgc tcgttgaact 4980

tggcggcctc gtccagcact gcctcggccg tttccggcgt ggcttcctcg aagcccggca 5040

gtttgctcac ggcctctagc ccggccagct cgttcatgac gaacagcatg tccttgatcg 5100

gcgcacggta agtcatcgat gtctcccaac atgtatgaaa aagccgttcg cgggtcaccc 5160

ccggaacggc tcgtacttgc taccagtgcc ggccgcctgg cggccggcaa ggcatcagcc 5220

cagtgctgcc accagctccg gcaccacggt gttcaggtcg cctaccaggc cgtagtcggc 5280

cacggagaag atcggggctt cggcatcctt gttgatcgca acgatcacct tggagtcctt 5340

catgccggcc aggtgctgga tcgcgccgga gataccgacg gcgatataca gctgcggcgc 5400

gacgatcttg ccggtctggc cgacctggta gtcgttcggc acgaagccgg cgtccacggc 5460

ggcgcgcgag gcacccagcg cggcgcccag cttgtcggcc agcggcgtca gcaccttggt 5520

gtagttctcg cccgaaccca cgccacggcc acccgagacg atgatcttgg cggcggtcag 5580

ttccggacgg tcgctcttgg ccacttcgcg cgaaacaaac tgcgaaacgc ctgcgtcggc 5640

cacggccggc agggtctcga cggcagccga gccaccttcg gcggcggcgg cgtcaaaggc 5700

ggtgccgcgc acggtgatga ccttgatctt gtcttccgac ttgaccgtgg cgatggcgtt 5760

gccggcgtag atcgggcgct cgaacgtgtc cggggcgtcg accttggaga tttccgagat 5820

ctgggccacg tccagcttgg cagccacgcg cggcaggatg ttcttgccgt acggggtggc 5880

cggagccagg atatgcgagt agtcgttggc gatggccagc gcctgctcgg ccacgttctc 5940

ggccaggccg tcaccgaagt acggggcgtc ggccagcagg accttggtca cgccggcgat 6000

cttggcggcc gcatcggccg cggccttggc gttggcacca gccaccagca cgtgcacgtc 6060

gccgccgcac tgggcagcgg cagtcacggt gttcagcgtg gcggccttga tggattgatt 6120

gtcgtgttca gcaatgacga gtgcagtcat gttatttctc ccccgcgctc agataacctt 6180

ggcttcgttc ttcagcttct gcaccagcgt cgcaacgtcc ggcaccatca cacccgcgct 6240

gcgcttggca ggctcgacca ccttcagggt cgacaggcgc ggcttgacgt cgacgccgag 6300

gtcttccggc ttgacgatat cgagctgctt cttcttggcc ttcatgatgt tcggcagcgt 6360

cacgtagcgc ggctcgttca ggcgcaggtc ggtcgtcacc acggccggca gcttgagcga 6420

cagcgtttcc aggccgccat cgacttcacg cgtcaccgag gccttgccgt cggcaaccac 6480

caccttggag gcgaacgtgg cttgcggcag gccagccagc gcggccacca tctggccggt 6540

ctggttggag tcgtcgtcga tggcctgctt gcccaggatc accagttgcg gctgttcctt 6600

gtcgatcagc gccttcagca gcttggccac ggccagcggt tgcaggtctt cgttcgattc 6660

caccaggatg ccgcggtcgg caccgatggc catcgcggtg cgcagggttt cctggcactg 6720

cgtgacaccg cacgagacgg cgatcacttc ggtgacaaca ccggcttcct tcaggcgcac 6780

ggcctcttcc acggcgattt cgtcgaaggg gttcatgctc atcttcacgt tggccagatc 6840

gacgccgctg ccgtccgcct tgacgcggac cttgacgttg taatccacca cccgcttgac 6900

tgcgacgagt actttcatgc gctcactcca ctgatatgtg aaattgttat ccgctcacaa 6960

ttccacacaa catacgagcc ggaagcataa agtgtaaagc ctggggaagc ttggggagaa 7020

caatcagccc ggcaggggcc gggctgattg tgcctgcgtg ccgccggtgc ttacggcagc 7080

ttgaccacgg cttccccggt gacggccagg gccccgcctt gggtaaagat ccgggtcgtc 7140

agcgtggcga tcggtttgtc ctcgcgcagc gcggtgactt ccacctcggc ggtcacttcg 7200

tcccccacga acaccggcag tttgaacgag agcgattggc ccaggtagat gctccccttg 7260

ccggggagct gctggccgag gaggcccgag aacagcgagg cgagcagcat gccatgcacg 7320

atggggcgct cgaacgcggt ggtcgcggca aacgcggggt ccaggtgcag cgggttgaag 7380

tcctcggaga gcgccgcaaa ggccgccacc tccgccgcgc caaaccgctt ggacagccgg 7440

gctttttgcc cgacctccag cgactgggcc gacatgcggc gtcctcctct gtttcagccc 7500

atatgcaggc cgccgttgag cgagaagtcg gcgccggtcg agaaaccgga ctcctccgac 7560

gacaaccagg cgcagatcga ggcgatctct tccggcaggc ccaggcgctt gaccgggatc 7620

gtcgcgacga tcttgtcgag cacgtcctgg cggatcgcct tgaccatgtc ggtggcgata 7680

tagcccggag agaccgtgtt gacggtcacg cccttggtcg ccacttcctg cgccagtgcc 7740

atggtgaagc catgcaggcc ggccttggcg gtggagtagt tggtctggcc gaactggccc 7800

ttctgcccgt tcaccgacga gatgttgacg atgcggcccc agccacggtc ggccatgccg 7860

tcgatcacct gcttggtgac gttgaacagc gaggtcaggt tggtgtcgat caccgcatcc 7920

cagtcggcgc gggtcatctt gcggaacacc acgtcgcggg tgataccggc gttgttgatc 7980

agcacatcaa cctcgccgac ctcggacttg accttgtcga atgcggtctt ggtcgagtcc 8040

cagtcagcca cattgccttc cgaggcaatg aaatcgaagc ccagggcctt ctgctgctcc 8100

agccactttt cgcggcgcgg cgagttgggg ccgcaaccgg ccaccacacg aaagccatcc 8160

ttggccagcc gctggcaaat ggcggttccg ataccaccca tgccgccggt cacatacgca 8220

atgcgctgag tcatgtccac tccttgattg gcttcgttat cgtcgccggg tccgcgccaa 8280

ccgcgcgcgg ccccggaaaa ccccttcctt atttgcgctc gactgccagc gccacgccca 8340

tgccgccgcc gatgcacagc gaggccaggc ccttcttcgc gtcacggcgc ttcatctcgt 8400

gcagcagcgt caccaggata cggcagcccg acgcgccgat cgggtggccg atggcgatgg 8460

cgccgccgtt cacattgacc ttggaggtgt cccagcccat ctgctggtgc accgccagcg 8520

cctgcgcggc aaaggcctcg ttgatctcca tcaggtccag gtcttgcggg gtccactcgg 8580

cgcgcgacag ggcgcgcttg gaggccggca ccgggcccat gcccatcacc ttgggatcga 8640

caccggcgtt ggcatagctc ttgatcgtgg ccagcggggt caggcccagt tccttggcct 8700

tggccgccga catcaccacc accgcggcgg cgccgtcgtt caggcccgag gcgttggccg 8760

cggtcaccgt gccggccttg tcgaaggcgg gcttgaggcc ggacatgctg tccagcgtgg 8820

cgccctggcg cacgaactcg tcggtcttga aggccaccgg gtcgcccttg cgctgcggga 8880

tcagcaccgg gacgatctct tcgtcaaact tgccggcctt ctgcgcggct tcggccttgt 8940

tctgcgagcc gacggcgaac tcatcctgcg cctcgcgtgt gatgccgtat tccttggcca 9000

cgttctcggc ggtgatgccc atgtggtact ggttgtacac gtcccacagg ccgtcgacga 9060

tcatggtgtc gaccagcttg gcatcgccca tgcggaaacc atcgcgcgag cccggcagca 9120

cgtgcggggc ggcgctcatg ttttcctggc cgccggccac cacgatctcg gcgtcgcccg 9180

ccatgatcgc gttggcggcc agcatcacgg ccttcaggcc cgagccgcac accttgttga 9240

tggtcatggc cggcaccatc gccggcaggc cggccttgat cgcggcctgg cgtgcggggt 9300

tctggcccga accggcggtc agcacctggc ccatgatgac ttcgctcacc tgctccggct 9360

tgacgccggc gcgctccagc gcggccttga tgaccacggc acccagttcc ggtgccggga 9420

tcttggccag cgagccgcca aacttgccga ccgcggtgcg ggcggcggat acgatgacaa 9480

cgtcagtcat tgtgtagtcc tttcaatgga aaggtaccca gcttttgttc cctttagtga 9540

gggttaattg cgcgcttggc gtaatcatgg tcatagctgt ttcctgtgtg aaattgttat 9600

ccgctcacaa ttccacacaa catacgagcc ggaagcataa agtgtaaagc ctggggtgcc 9660

taatgagtga gctaactcac attaattgcg ttgcgctcac tgcccgcttt ccagtcggga 9720

aacctgtcgt gccagctgca ttaatgaatc ggccaacgcg cggggagagg cggtttgcgt 9780

attgggcgca tgcataaaaa ctgttgtaat tcattaagca ttctgccgac atggaagcca 9840

tcacaaacgg catgatgaac ctgaatcgcc agcggcatca gcaccttgtc gccttgcgta 9900

taatatttgc ccatgggggt gggcgaagaa ctccagcatg agatccccgc gctggaggat 9960

catccagccg gcgtcccgga aaacgattcc gaagcccaac ctttcataga aggcggcggt 10020

ggaatcgaaa tctcgtgatg gcaggttggg cgtcgcttgg tcggtcattt cgaaccccag 10080

agtcccgctc agaagaactc gtcaagaagg cgatagaagg cgatgcgctg cgaatcggga 10140

gcggcgatac cgtaaagcac gaggaagcgg tcagcccatt cgccgccaag ctcttcagca 10200

atatcacggg tagccaacgc tatgtcctga tagcggtccg ccacacccag ccggccacag 10260

tcgatgaatc cagaaaagcg gccattttcc accatgatat tcggcaagca ggcatcgcca 10320

tgggtcacga cgagatcctc gccgtcgggc atgcgcgcct tgagcctggc gaacagttcg 10380

gctggcgcga gcccctgatg ctcttcgtcc agatcatcct gatcgacaag accggcttcc 10440

atccgagtac gtgctcgctc gatgcgatgt ttcgcttggt ggtcgaatgg gcaggtagcc 10500

ggatcaagcg tatgcagccg ccgcattgca tcagccatga tggatacttt ctcggcagga 10560

gcaaggtgag atgacaggag atcctgcccc ggcacttcgc ccaatagcag ccagtccctt 10620

cccgcttcag tgacaacgtc gagcacagct gcgcaaggaa cgcccgtcgt ggccagccac 10680

gatagccgcg ctgcctcgtc ctgcagttca ttcagggcac cggacaggtc ggtcttgaca 10740

aaaagaaccg ggcgcccctg cgctgacagc cggaacacgg cggcatcaga gcagccgatt 10800

gtctgttgtg cccagtcata gccgaatagc ctctccaccc aagcggccgg agaacctgcg 10860

tgcaatccat cttgttcaat catgcgaaac gatcctcatc ctgtctcttg atcagatctt 10920

gatcccctgc gccatcagat ccttggcggc aagaaagcca tccagtttac tttgcagggc 10980

ttcccaacct taccagaggg cgccccagct ggcaattccg gttcgcttgc tgtccataaa 11040

accgcccagt ctagctatcg ccatgtaagc ccactgcaag ctacctgctt tctctttgcg 11100

cttgcgtttt cccttgtcca gatagcccag tagctgacat tcatcccagg tggcactttt 11160

cggggaaatg tgcgcgcccg cgttcctgct ggcgctgggc ctgtttctgg cgctggactt 11220

cccgctgttc cgtcagcagc ttttcgccca cggccttgat gatcgcggcg gccttggcct 11280

gcatatcccg attcaacggc cccagggcgt ccagaacggg cttcaggcgc tcccgaaggt 11340

<210> 62
<211> 14113
<212> DNA
<213> Artificial Sequence

<220>
<223> vector

<400> 62
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300
gccctagatc ggccacagcg gccgcaaacg tggtctggtc gcgggtcatc tgcgctttgt 360
tgccgatgaa ctccttggcc gacagcctgc cgtcctgcgt cagcggcacc acgaacgcgg 420
tcatgtgcgg gctggtttcg tcacggtgga tgctggccgt cacgatgcga tccgccccgt 480
acttgtccgc cagccacttg tgcgccttct cgaagaacgc cgcctgctgt tcttggctgg 540
ccgacttcca ccattccggg ctggccgtca tgacgtactc gaccgccaac acagcgtcct 600
tgcgccgctt ctctggcagc aactcgcgca gtcggcccat cgcttcatcg gtgctgctgg 660
ccgcccagtg ctcgttctct ggcgtcctgc tggcgtcagc gttgggcgtc tcgcgctcgc 720
ggtaggcgtg cttgagactg gccgccacgt tgcccatttt cgccagcttc ttgcatcgca 780
tgatcgcgta tgccgccatg cctgcccctc ccttttggtg tccaaccggc tcgacggggg 840
cagcgcaagg cggtgcctcc ggcgggccac tcaatgcttg agtatactca ctagactttg 900
cttcgcaaag tcgtgaccgc ctacggcggc tgcggcgccc tacgggcttg ctctccgggc 960
ttcgccctgc gcggtcgctg cgctcccttg ccagcccgtg gatatgtgga cgatggccgc 1020
gagcggccac cggctggctc gcttcgctcg gcccgtggac aaccctgctg gacaagctga 1080
tggacaggct gcgcctgccc acgagcttga ccacagggat tgcccaccgg ctacccagcc 1140
ttcgaccaca tacccaccgg ctccaactgc gcggcctgcg gccttgcccc atcaattttt 1200
ttaattttct ctggggaaaa gcctccggcc tgcggcctgc gcgcttcgct tgccggttgg 1260
acaccaagtg gaaggcgggt caaggctcgc gcagcgaccg cgcagcggct tggccttgac 1320
gcgcctggaa cgacccaagc ctatgcgagt gggggcagtc gaaggcgaag cccgcccgcc 1380
tgccccccga gcctcacggc ggcgagtgcg ggggttccaa gggggcagcg ccaccttggg 1440
caaggccgaa ggccgcgcag tcgatcaaca agccccggag gggccacttt ttgccggagg 1500
gggagccgcg ccgaaggcgt gggggaaccc cgcaggggtg cccttctttg ggcaccaaag 1560
aactagatat agggcgaaat gcgaaagact taaaaatcaa caacttaaaa aaggggggta 1620
cgcaacagct cattgcggca ccccccgcaa tagctcattg cgtaggttaa agaaaatctg 1680
taattgactg ccacttttac gcaacgcata attgttgtcg cgctgccgaa aagttgcagc 1740
tgattgcgca tggtgccgca accgtgcggc accctaccgc atggagataa gcatggccac 1800
gcagtccaga gaaatcggca ttcaagccaa gaacaagccc ggtcactggg tgcaaacgga 1860
acgcaaagcg catgaggcgt gggccgggct tattgcgagg aaacccacgg cggcaatgct 1920
gctgcatcac ctcgtggcgc agatgggcca ccagaacgcc gtggtggtca gccagaagac 1980
actttccaag ctcatcggac gttctttgcg gacggtccaa tacgcagtca aggacttggt 2040
ggccgagcgc tggatctccg tcgtgaagct caacggcccc ggcaccgtgt cggcctacgt 2100
ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatccagca ggctgcctcg ataagcaaaa agggcggccc cgcggggccg 3300

ccctttttct ttccggcgac tgtcaggcca ctcagttgtt ggcggccttc acctgcgcga 3360

tcagctccgg gaccactttg ttcacgtcgc ccacgatcgc caggtcggcc accttcataa 3420

tcggggcctc cacgtcctta ttgatggcga tgatgtagtc cgagtcctgc atgccggcca 3480

ggtgctggat cgcccccgag atgccgcagg cgatgtacag ggtcgggcgc acggtcttgc 3540

cggtctggcc cacctggagg tctttgtcca cccactcttt ctcgatggcc gcgcgcgagg 3600

cggcgatggt gccgcccagg agcgaggcca gctcctccag tttttcgaag ttctccttcg 3660

agcccacgcc gcggcccccg gccacgagca ccttggcctc gccgatgtcc gcgatgtctt 3720

tggccagctt caccactttc gacactttgg tgcggatgtc ggacgcggtc agcttgatgg 3780

cgactttctc gatcttatcg tcggacacgt tggcgtcgtt caccggcagc ttctcgaaga 3840

agacgccggg gcggaccgtg gccatctgcg ggcggtggtc cgagcagacg atggtggcga 3900

tcaggttgcc gccgaacgcc gggcgggtcg ccaggaggtc ccggttctcc acgtcgatgt 3960

ccagcgaggt gcagtcggcg gtcaggccgg tcgacaggcg cgccgcgatc cgcgggccca 4020

ggtcccgccc gatgaacgtg gcgccaatga acaggatctc cggcttgcgc tcgttcacga 4080

ggtcgcagat caccttcgcg tagccgtcgg tcgagaagtg ggcgaggagt tcattgtcgg 4140

ccgcgagcac cttatccgcg ccgtggctca gcaggtcctt cgacatcttc tcggtgttgt 4200

ggcccagcag cacggcggtc agttccaccc ccagcttctc ggccatctcc ttgcccttgc 4260

ccagcagctc cagcgacacc ttctgcagct cgccgtcgcg ctgctccgca aacacccaca 4320

cgcccttgta gtcggccttg ttcattgaaa aatccctcct aacttaaata tgtgttcttc 4380

ttttagttct gcgagagcat atcggccgct tccttcaccg gtttatcaat gacctcgccc 4440

tgccctttca cttctttcgt gctcgacttc ttcactttgg tcggcgaccc cttcaggccc 4500

agattggctt tatccacgtc gatatcatcc gcggtccaca ttttcacctc cttgtcgaac 4560

gcgccgaaga ttttctccac cgacatgtac cgcgggacgt tcagttcctt aatggccgtc 4620

aggagcaccg gggtcttgac ttccacgacc tcgtacccat cctcccaggc cttccggatc 4680

ttgagcgtgt ccccatccac ctccactttt tccacgtagg tgacttgggg aatcccgaga 4740

tgttccgcaa tctccggccc gacctgcgcc gtatcgccgt cgatggcctg gcggccggca 4800

aagacaatgt catatttcag cttcttaatg ccggcggcaa tggtatgcga ggtggcgagg 4860

gtgtccgcgc ccccaaacgc ccggtccgtg agcagcaccg cctcatcggc ccccatggcc 4920

agcgcttcga ccagggcgtt cttggcttgg gggggcccca tcgagatgac cgtgacgtgc 4980

gccccatagt tatctttcag cacgagggct tcctcgagcg cgtttttgtc gtcggggtta 5040

atgatggacg ggacgccctc ccgaatcagg gtgcctttga cgggatcaat gcggacctcg 5100

gcggtgtcgg ggacttgttt gaggcagacc acaatattca tcctcttaac ctccttaaat 5160

tagcggaaga tcttgcccga gatcaccagc ttctgcacct ccgaggtgcc ctcgtagatc 5220

tccgtgatct tggcgtcgcg catcatgcgc tccacggggt agtccttggt gtagccatag 5280

ccgccgaaca gctgcacggc cttggtggtc acgtccatgg ccacgttggc ggcatgcagc 5340

ttggcgcggg cggcatccac ggtgtacggc aggcccgcct gcttcaggta ggcggccttg 5400

tacaccaggt agcgggccga ctcgatggcc acgtccatgt cggccatcat ccaggccagg 5460

ccctggaact tgtccagcga gcggccgaac tgcttgcgtt ccttcatgta cgcgcgggcc 5520

tcgttaaagg cgccttcggc gatgcccagg gcctgggccg cgatgccgat gcggcccccg 5580

tccagggtct tcatggcgat cgggaagccc ttgccctcct tgccaatcat gttctccacc 5640

ggcacgatca tgtcctcgaa caccagctcg gtggtcgacg acgcgcggat gcccagcttt 5700

tgctccacct tgccgatcga gaagcccttg aagcccttct cgatgataaa ggccgagatg 5760

cccttggtcc ccttggtgcg gtcggtcatg gcgaagatga cgaaggtgtc ggcgacgccg 5820

ccgttggtga tgaagatttt cgagccgttg atcacgtagt ggtcgccctc cagcacggcc 5880

acggtctgct gggcgcccga atcggtgccg gcgttcggct cggtcaggcc gtaggcgccg 5940

atcttctccc ccttggccag cggcaccagg tacttctgct tctgctcctc ggtgccgtgc 6000

tcgttaatca gcgaggcgca cagcgaggtg tgggccgaca ggatcacgcc ggtggtgccg 6060

cacaccttcg acagctcctc cacggcgatg atgtacgaca gcacgtcgcc gccggccccg 6120

ccgtactcct tcgagaacgg gatgcccatc atgccgtact ggcccatctt cttcacgttc 6180

tccatcggga accgctccgt ctcgtcgatc tccgcggcaa tcggcttcac ctcgttctcg 6240

gcgaactccc ggaccatctg gcgcaccagc tcttgctcgc gcgtcaggtt gaagtccata 6300

taaacttacc tcctagcggt tcttgaaccc ttcgatcttg cgcttctcaa tgaaggcggt 6360

catggcgtct ttctggtcct cggtcgagaa gcattccccg aacgcttccg actcgaaggc 6420

gagggccgtg tcaatgtcgc actgcatgcc gcggttaatg gcttgcttcg acagtttgac 6480

ggccaccggg gcattgctca cgattttgtt ggcgatctcc ttggcggtgt tcatcagttc 6540

cgagggctcc accaccttgt tcaccaggcc aatgcgcagg gcctcatcgg ccttgatgtt 6600

ctgcgcggtg aaaatcagct gcttggccat gcccatgccg acgaggcgcg acagccgctg 6660

ggtgccgcca aagcccgggg tgatgcccag gcccacttcc ggctggccga agcgggcgtt 6720

cgaggaggcg atgcggatat cgcaggacat cgcaatttcg cacccgccgc ccagggcgaa 6780

cccattcacg gccgcaatca ccggtttttc cagcagctcc agccgccgaa acactttgtt 6840

ccccagaatg ccgaacttgc gcccctcgat ggtattcatt tctttcatct ccgagatgtc 6900

cgccccggcg acgaagctct tctcgcccgc gcccgtcagg atcacggcga gcacctccga 6960

gtcgttctcg atttcgccga tcacgtagtc catctctttc agggtgtccg agttcagggc 7020

gttgagggcc ttcgggcggt tgatggtcac caccgcgacc ttgccttctt tctccaggat 7080

gacattgttg agttccatga ctaatcctcc taaaatgtga aattgttatc cgctcacaat 7140

tccacacaac atacgagccg gaagcataaa gtgtaaagcc tggggaagct tggggagaac 7200

aatcagcccg gcaggggccg ggctgattgt gcctgcgtgc cttagaacga cttgatgtag 7260

atgtccttca gctccgagat cagcgggtag cgcgggttgg cggtggtgca ctggtcgtcg 7320

aaggccagct ccgacatctt gtccagggtg ttgtagaaat ccttcttgtt gatgccggcg 7380

gccgagatgt tctgcgggat cgacaggtcg atcttcagct tcgagatggc ctcgatcagg 7440

gcggtcacct tctccgtgtc cgaggtgccc ttcaggttca ggtactcggc gatctcggcg 7500

tacttgcgct tggcgttcgg cgacttgtac tgcgggaagg cggtctgctt ggtcgggcag 7560

tcggtggcgt tgtacttgat cacctcctcg atcagcacgg cgcaggcgat gccgtgcggc 7620

acgtggtgca tggcgcccag cttgtgggcc atcgagtggc acacgcccag gaaggcgttg 7680

gcgaaggcca tgccggcgat gttcgaggcg tgggccatct tctcgcgggc ctcgatgtcg 7740

ttggtgccgt tcttgtaggc gcgcggcagg tacttgaaga tcatcttgat ggcgcgcagg 7800

gccagctcgt cggtgtagtc cgtggccatc accgacacgt aggcctcgat ggcgtgcacc 7860

agggcatcaa tgccggtggc ggcggtcagc ttgcgcggca tgttcagcat cagctcggtg 7920

tcaatgatgg ccatgttcgg ggtcagttcg tacgaggtca gcgggtactt catgccggtc 7980

tcgtcgttgg tgatcacggc gaacggggtg gcctccgagc cggtgccggc ggtggtcggg 8040

atggccaccg agatggcctt ggtgcccagc ttcgggaagt tgcagatgcg cttgcggatg 8100

tccatgaaat tgatggcgag gttctcgatc tccgcctccg ggtactcgta cagcaggtgc 8160

atcaccttgg cggcgtccat cggcgagccg ccgccgatcg agatgatggt gtccggctcg 8220

aagttcagca tctccttggc gcccttcttc accgagtcaa tggtcgggtc cgacttgata 8280

tcggtgaaga tcgagtactt gatgtcgatc tcgtccagca ccttggtgat cttgttcacg 8340

tagcccagct tgaacaggtc cttgtcggtc acgatgaagg cgcgcttctt gttcatgtcc 8400

ttcagctcct tcagggcgaa gcgcaggcag ccgtacttga agtagatctt ctgcggcacc 8460

ttgaaccaca gcatattctc gcggcgctcg gccaccgact tgatgttcag caggtgcttc 8520

ggctccacgt tctgcgacac cgagttgccg ccccaggtcc cgcagcccag ggtgaacgac 8580

ggggcgatgg cgaagttgta caggtcgccc gaggcgccct gcgacgacgg catgttgatg 8640

aaggtgcgcg aggtcttcat ggccaggccg aactccttca ccttgtcctt gttgttctgc 8700

gagtcgatgt acagcgacga ggtgtggccc gagccgccca gctcgatcag gcgctgggcc 8760

ttcttgaggg cctcatcgaa gtccttcacc ttgtacatgg ccagcaccgg cgacagcttc 8820

tcgtgcgaga acagctccga cttctccacc gactgcacct cgccgatgag gatcttggtg 8880

gtctgcggca cctcgatgcc ggccatcttg gcgatgatgt aggccgactt gcccacgatg 8940

tcggcgttga tggcgccgtt cttgaacatc gtctccttga tcttggcgat ctcgttctgg 9000

ttcaggatgt acgagccgcg cttcacgaac tcctccttca ccttctcgta gatggagttc 9060

atcaccagga tcgactgctc cgaggcgcag atcacgccgt tgtcgtaggt cttcgacagg 9120

atgatcgacg acacggccat atcgatgtcg gcggactcgt cgatgatggc cggggtgttg 9180

cccgcgccca cgccgatggc cggcttgccc gacgagtagg cggccttcac catcgacggg 9240

ccgccggtgg ccaggatgat atcggcctcc gacatcaggt cctgcgacag ctcgatcgac 9300

ggctcgtcaa tccagccgat gatgttcttc ggggccccgg ccttcacggc ggcgtccagg 9360

atcagcttgg cggcggcgat ggtcgacttc ttggcgcgcg ggtgcggcga gaagaagatg 9420

gcgttgcggg tcttcagcga gatcagcgac ttgaagatgg cggtcgaggt cgggttcgtg 9480

gtcggcacga tggcggccac gatgccgatc ggctcggcca ccttggtgat gcccagcgag 9540

tcgtcgtggt cgataatgcc gcaggtcttc tcgttcttgt acttgttgta gatgtactcg 9600

gcggcgaagt ggttcttgat gatcttgtcc tccaccaggc cgatgccggt ctcctccacg 9660

gccagcttgg ccaggttgat gcgctccttg gcggcggcaa tggcgcactg cttgaagatc 9720

ttgtccacct gctcctgggt gtaggtggcg aacttcttct gggcctcgcg cagctcgttc 9780

agcttctgct tcagctcctt ctggttggtc accttcattc ttgaatctcc tgaaagccgg 9840

tgcttacggc agcttgacca cggcttcccc ggtgacggcc agggccccgc cttgggtaaa 9900

gatccgggtc gtcagcgtgg cgatcggttt gtcctcgcgc agcgcggtga cttccacctc 9960

ggcggtcact tcgtccccca cgaacaccgg cagtttgaac gagagcgatt ggcccaggta 10020

gatgctcccc ttgccgggga gctgctggcc gaggaggccc gagaacagcg aggcgagcag 10080

catgccatgc acgatggggc gctcgaacgc ggtggtcgcg gcaaacgcgg ggtccaggtg 10140

cagcgggttg aagtcctcgg agagcgccgc aaaggccgcc acctccgccg cgccaaaccg 10200

cttggacagc cgggcttttt gcccgacctc cagcgactgg gccgacatgc ggcgtcctcc 10260

tctgtttcag cccatatgca ggccgccgtt gagcgagaag tcggcgccgg tcgagaaacc 10320

ggactcctcc gacgacaacc aggcgcagat cgaggcgatc tcttccggca ggcccaggcg 10380

cttgaccggg atcgtcgcga cgatcttgtc gagcacgtcc tggcggatcg ccttgaccat 10440

gtcggtggcg atatagcccg gagagaccgt gttgacggtc acgcccttgg tcgccacttc 10500

ctgcgccagt gccatggtga agccatgcag gccggccttg gcggtggagt agttggtctg 10560

gccgaactgg cccttctgcc cgttcaccga cgagatgttg acgatgcggc cccagccacg 10620

gtcggccatg ccgtcgatca cctgcttggt gacgttgaac agcgaggtca ggttggtgtc 10680

gatcaccgca tcccagtcgg cgcgggtcat cttgcggaac accacgtcgc gggtgatacc 10740

ggcgttgttg atcagcacat caacctcgcc gacctcggac ttgaccttgt cgaatgcggt 10800

cttggtcgag tcccagtcag ccacattgcc ttccgaggca atgaaatcga agcccagggc 10860

cttctgctgc tccagccact tttcgcggcg cggcgagttg gggccgcaac cggccaccac 10920

acgaaagcca tccttggcca gccgctggca aatggcggtt ccgataccac ccatgccgcc 10980

ggtcacatac gcaatgcgct gagtcatgtc cactccttga ttggcttcgt tatcgtcgcc 11040

gggtccgcgc caaccgcgcg cggccccgga aaaccccttc cttatttgcg ctcgactgcc 11100

agcgccacgc ccatgccgcc gccgatgcac agcgaggcca ggcccttctt cgcgtcacgg 11160

cgcttcatct cgtgcagcag cgtcaccagg atacggcagc ccgacgcgcc gatcgggtgg 11220

ccgatggcga tggcgccgcc gttcacattg accttggagg tgtcccagcc catctgctgg 11280

tgcaccgcca gcgcctgcgc ggcaaaggcc tcgttgatct ccatcaggtc caggtcttgc 11340

ggggtccact cggcgcgcga cagggcgcgc ttggaggccg gcaccgggcc catgcccatc 11400

accttgggat cgacaccggc gttggcatag ctcttgatcg tggccagcgg ggtcaggccc 11460

agttccttgg ccttggccgc cgacatcacc accaccgcgg cggcgccgtc gttcaggccc 11520

gaggcgttgg ccgcggtcac cgtgccggcc ttgtcgaagg cgggcttgag gccggacatg 11580

ctgtccagcg tggcgccctg gcgcacgaac tcgtcggtct tgaaggccac cgggtcgccc 11640

ttgcgctgcg ggatcagcac cgggacgatc tcttcgtcaa acttgccggc cttctgcgcg 11700

gcttcggcct tgttctgcga gccgacggcg aactcatcct gcgcctcgcg tgtgatgccg 11760

tattccttgg ccacgttctc ggcggtgatg cccatgtggt actggttgta cacgtcccac 11820

aggccgtcga cgatcatggt gtcgaccagc ttggcatcgc ccatgcggaa accatcgcgc 11880

gagcccggca gcacgtgcgg ggcggcgctc atgttttcct ggccgccggc caccacgatc 11940

tcggcgtcgc ccgccatgat cgcgttggcg gccagcatca cggccttcag gcccgagccg 12000

cacaccttgt tgatggtcat ggccggcacc atcgccggca ggccggcctt gatcgcggcc 12060

tggcgtgcgg ggttctggcc cgaaccggcg gtcagcacct ggcccatgat gacttcgctc 12120

acctgctccg gcttgacgcc ggcgcgctcc agcgcggcct tgatgaccac ggcacccagt 12180

tccggtgccg ggatcttggc cagcgagccg ccaaacttgc cgaccgcggt gcgggcggcg 12240

gatacgatga caacgtcagt cattgtgtag tcctttcaat ggaaaggtac ccagcttttg 12300

ttccctttag tgagggttaa ttgcgcgctt ggcgtaatca tggtcatagc tgtttcctgt 12360

gtgaaattgt tatccgctca caattccaca caacatacga gccggaagca taaagtgtaa 12420

agcctggggt gcctaatgag tgagctaact cacattaatt gcgttgcgct cactgcccgc 12480

tttccagtcg ggaaacctgt cgtgccagct gcattaatga atcggccaac gcgcggggag 12540

aggcggtttg cgtattgggc gcatgcataa aaactgttgt aattcattaa gcattctgcc 12600

gacatggaag ccatcacaaa cggcatgatg aacctgaatc gccagcggca tcagcacctt 12660

gtcgccttgc gtataatatt tgcccatggg ggtgggcgaa gaactccagc atgagatccc 12720

cgcgctggag gatcatccag ccggcgtccc ggaaaacgat tccgaagccc aacctttcat 12780

agaaggcggc ggtggaatcg aaatctcgtg atggcaggtt gggcgtcgct tggtcggtca 12840

tttcgaaccc cagagtcccg ctcagaagaa ctcgtcaaga aggcgataga aggcgatgcg 12900

ctgcgaatcg ggagcggcga taccgtaaag cacgaggaag cggtcagccc attcgccgcc 12960

aagctcttca gcaatatcac gggtagccaa cgctatgtcc tgatagcggt ccgccacacc 13020

cagccggcca cagtcgatga atccagaaaa gcggccattt tccaccatga tattcggcaa 13080

gcaggcatcg ccatgggtca cgacgagatc ctcgccgtcg ggcatgcgcg ccttgagcct 13140

ggcgaacagt tcggctggcg cgagcccctg atgctcttcg tccagatcat cctgatcgac 13200

aagaccggct tccatccgag tacgtgctcg ctcgatgcga tgtttcgctt ggtggtcgaa 13260

tgggcaggta gccggatcaa gcgtatgcag ccgccgcatt gcatcagcca tgatggatac 13320

tttctcggca ggagcaaggt gagatgacag gagatcctgc cccggcactt cgcccaatag 13380

cagccagtcc cttcccgctt cagtgacaac gtcgagcaca gctgcgcaag gaacgcccgt 13440

cgtggccagc cacgatagcc gcgctgcctc gtcctgcagt tcattcaggg caccggacag 13500

gtcggtcttg acaaaaagaa ccgggcgccc ctgcgctgac agccggaaca cggcggcatc 13560

agagcagccg attgtctgtt gtgcccagtc atagccgaat agcctctcca cccaagcggc 13620

cggagaacct gcgtgcaatc catcttgttc aatcatgcga aacgatcctc atcctgtctc 13680

ttgatcagat cttgatcccc tgcgccatca gatccttggc ggcaagaaag ccatccagtt 13740

tactttgcag ggcttcccaa ccttaccaga gggcgcccca gctggcaatt ccggttcgct 13800

tgctgtccat aaaaccgccc agtctagcta tcgccatgta agcccactgc aagctacctg 13860

ctttctcttt gcgcttgcgt tttcccttgt ccagatagcc cagtagctga cattcatccc 13920

aggtggcact tttcggggaa atgtgcgcgc ccgcgttcct gctggcgctg ggcctgtttc 13980

tggcgctgga cttcccgctg ttccgtcagc agcttttcgc ccacggcctt gatgatcgcg 14040

gcggccttgg cctgcatatc ccgattcaac ggccccaggg cgtccagaac gggcttcagg 14100

cgctcccgaa ggt 14113

<210> 63
<211> 11519
<212> DNA
<213> Artificial Sequence

<220>
<223> vector

<400> 63
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960
ttcgccctgc gcggtcgctg cgctcccttg ccagcccgtg gatatgtgga cgatggccgc 1020
gagcggccac cggctggctc gcttcgctcg gcccgtggac aaccctgctg gacaagctga 1080
tggacaggct gcgcctgccc acgagcttga ccacagggat tgcccaccgg ctacccagcc 1140
ttcgaccaca tacccaccgg ctccaactgc gcggcctgcg gccttgcccc atcaattttt 1200
ttaattttct ctggggaaaa gcctccggcc tgcggcctgc gcgcttcgct tgccggttgg 1260
acaccaagtg gaaggcgggt caaggctcgc gcagcgaccg cgcagcggct tggccttgac 1320
gcgcctggaa cgacccaagc ctatgcgagt gggggcagtc gaaggcgaag cccgcccgcc 1380
tgccccccga gcctcacggc ggcgagtgcg ggggttccaa gggggcagcg ccaccttggg 1440
caaggccgaa ggccgcgcag tcgatcaaca agccccggag gggccacttt ttgccggagg 1500
gggagccgcg ccgaaggcgt gggggaaccc cgcaggggtg cccttctttg ggcaccaaag 1560
aactagatat agggcgaaat gcgaaagact taaaaatcaa caacttaaaa aaggggggta 1620
cgcaacagct cattgcggca ccccccgcaa tagctcattg cgtaggttaa agaaaatctg 1680
taattgactg ccacttttac gcaacgcata attgttgtcg cgctgccgaa aagttgcagc 1740
tgattgcgca tggtgccgca accgtgcggc accctaccgc atggagataa gcatggccac 1800
gcagtccaga gaaatcggca ttcaagccaa gaacaagccc ggtcactggg tgcaaacgga 1860
acgcaaagcg catgaggcgt gggccgggct tattgcgagg aaacccacgg cggcaatgct 1920
gctgcatcac ctcgtggcgc agatgggcca ccagaacgcc gtggtggtca gccagaagac 1980
actttccaag ctcatcggac gttctttgcg gacggtccaa tacgcagtca aggacttggt 2040
ggccgagcgc tggatctccg tcgtgaagct caacggcccc ggcaccgtgt cggcctacgt 2100
ggtcaatgac cgcgtggcgt ggggccagcc ccgcgaccag ttgcgcctgt cggtgttcag 2160
tgccgccgtg gtggttgatc acgacgacca ggacgaatcg ctgttggggc atggcgacct 2220
gcgccgcatc ccgaccctgt atccgggcga gcagcaacta ccgaccggcc ccggcgagga 2280
gccgcccagc cagcccggca ttccgggcat ggaaccagac ctgccagcct tgaccgaaac 2340
ggaggaatgg gaacggcgcg ggcagcagcg cctgccgatg cccgatgagc cgtgttttct 2400
ggacgatggc gagccgttgg agccgccgac acgggtcacg ctgccgcgcc ggtagcactt 2460
gggttgcgca gcaacccgta agtgcgctgt tccagactat cggctgtagc cgcctcgccg 2520
ccctatacct tgtctgcctc cccgcgttgc gtcgcggtgc atggagccgg gccacctcga 2580
cctgaatgga agccggcggc acctcgctaa cggattcacc gtttttatca ggctctggga 2640
ggcagaataa atgatcatat cgtcaattat tacctccacg gggagagcct gagcaaactg 2700
gcctcaggca tttgagaagc acacggtcac actgcttccg gtagtcaata aaccggtaaa 2760
ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatccagca ggctgcctcg ataagcaaaa agggcggccc cgcggggccg 3300

ccctttttct ttccggcgac tgtcaggcca ctcagttgtt ggcggccttc acctgcgcga 3360

tcagctccgg gaccactttg ttcacgtcgc ccacgatcgc caggtcggcc accttcataa 3420

tcggggcctc cacgtcctta ttgatggcga tgatgtagtc cgagtcctgc atgccggcca 3480

ggtgctggat cgcccccgag atgccgcagg cgatgtacag ggtcgggcgc acggtcttgc 3540

cggtctggcc cacctggagg tctttgtcca cccactcttt ctcgatggcc gcgcgcgagg 3600

cggcgatggt gccgcccagg agcgaggcca gctcctccag tttttcgaag ttctccttcg 3660

agcccacgcc gcggcccccg gccacgagca ccttggcctc gccgatgtcc gcgatgtctt 3720

tggccagctt caccactttc gacactttgg tgcggatgtc ggacgcggtc agcttgatgg 3780

cgactttctc gatcttatcg tcggacacgt tggcgtcgtt caccggcagc ttctcgaaga 3840

agacgccggg gcggaccgtg gccatctgcg ggcggtggtc cgagcagacg atggtggcga 3900

tcaggttgcc gccgaacgcc gggcgggtcg ccaggaggtc ccggttctcc acgtcgatgt 3960

ccagcgaggt gcagtcggcg gtcaggccgg tcgacaggcg cgccgcgatc cgcgggccca 4020

ggtcccgccc gatgaacgtg gcgccaatga acaggatctc cggcttgcgc tcgttcacga 4080

ggtcgcagat caccttcgcg tagccgtcgg tcgagaagtg ggcgaggagt tcattgtcgg 4140

ccgcgagcac cttatccgcg ccgtggctca gcaggtcctt cgacatcttc tcggtgttgt 4200

ggcccagcag cacggcggtc agttccaccc ccagcttctc ggccatctcc ttgcccttgc 4260

ccagcagctc cagcgacacc ttctgcagct cgccgtcgcg ctgctccgca aacacccaca 4320

cgcccttgta gtcggccttg ttcattgaaa aatccctcct aacttaaata tgtgttcttc 4380

ttttagttct gcgagagcat atcggccgct tccttcaccg gtttatcaat gacctcgccc 4440

tgccctttca cttctttcgt gctcgacttc ttcactttgg tcggcgaccc cttcaggccc 4500

agattggctt tatccacgtc gatatcatcc gcggtccaca ttttcacctc cttgtcgaac 4560

gcgccgaaga ttttctccac cgacatgtac cgcgggacgt tcagttcctt aatggccgtc 4620

aggagcaccg gggtcttgac ttccacgacc tcgtacccat cctcccaggc cttccggatc 4680

ttgagcgtgt ccccatccac ctccactttt tccacgtagg tgacttgggg aatcccgaga 4740

tgttccgcaa tctccggccc gacctgcgcc gtatcgccgt cgatggcctg gcggccggca 4800

aagacaatgt catatttcag cttcttaatg ccggcggcaa tggtatgcga ggtggcgagg 4860

gtgtccgcgc ccccaaacgc ccggtccgtg agcagcaccg cctcatcggc ccccatggcc 4920

agcgcttcga ccagggcgtt cttggcttgg gggggcccca tcgagatgac cgtgacgtgc 4980

gccccatagt tatctttcag cacgagggct tcctcgagcg cgtttttgtc gtcggggtta 5040

atgatggacg ggacgccctc ccgaatcagg gtgcctttga cgggatcaat gcggacctcg 5100

gcggtgtcgg ggacttgttt gaggcagacc acaatattca tcctcttaac ctccttaaat 5160

tagcggaaga tcttgcccga gatcaccagc ttctgcacct ccgaggtgcc ctcgtagatc 5220

tccgtgatct tggcgtcgcg catcatgcgc tccacggggt agtccttggt gtagccatag 5280

ccgccgaaca gctgcacggc cttggtggtc acgtccatgg ccacgttggc ggcatgcagc 5340

ttggcgcggg cggcatccac ggtgtacggc aggcccgcct gcttcaggta ggcggccttg 5400

tacaccaggt agcgggccga ctcgatggcc acgtccatgt cggccatcat ccaggccagg 5460

ccctggaact tgtccagcga gcggccgaac tgcttgcgtt ccttcatgta cgcgcgggcc 5520

tcgttaaagg cgccttcggc gatgcccagg gcctgggccg cgatgccgat gcggcccccg 5580

tccagggtct tcatggcgat cgggaagccc ttgccctcct tgccaatcat gttctccacc 5640

ggcacgatca tgtcctcgaa caccagctcg gtggtcgacg acgcgcggat gcccagcttt 5700

tgctccacct tgccgatcga gaagcccttg aagcccttct cgatgataaa ggccgagatg 5760

cccttggtcc ccttggtgcg gtcggtcatg gcgaagatga cgaaggtgtc ggcgacgccg 5820

ccgttggtga tgaagatttt cgagccgttg atcacgtagt ggtcgccctc cagcacggcc 5880

acggtctgct gggcgcccga atcggtgccg gcgttcggct cggtcaggcc gtaggcgccg 5940

atcttctccc ccttggccag cggcaccagg tacttctgct tctgctcctc ggtgccgtgc 6000

tcgttaatca gcgaggcgca cagcgaggtg tgggccgaca ggatcacgcc ggtggtgccg 6060

cacaccttcg acagctcctc cacggcgatg atgtacgaca gcacgtcgcc gccggccccg 6120

ccgtactcct tcgagaacgg gatgcccatc atgccgtact ggcccatctt cttcacgttc 6180

tccatcggga accgctccgt ctcgtcgatc tccgcggcaa tcggcttcac ctcgttctcg 6240

gcgaactccc ggaccatctg gcgcaccagc tcttgctcgc gcgtcaggtt gaagtccata 6300

taaacttacc tcctagcggt tcttgaaccc ttcgatcttg cgcttctcaa tgaaggcggt 6360

catggcgtct ttctggtcct cggtcgagaa gcattccccg aacgcttccg actcgaaggc 6420

gagggccgtg tcaatgtcgc actgcatgcc gcggttaatg gcttgcttcg acagtttgac 6480

ggccaccggg gcattgctca cgattttgtt ggcgatctcc ttggcggtgt tcatcagttc 6540

cgagggctcc accaccttgt tcaccaggcc aatgcgcagg gcctcatcgg ccttgatgtt 6600

ctgcgcggtg aaaatcagct gcttggccat gcccatgccg acgaggcgcg acagccgctg 6660

ggtgccgcca aagcccgggg tgatgcccag gcccacttcc ggctggccga agcgggcgtt 6720

cgaggaggcg atgcggatat cgcaggacat cgcaatttcg cacccgccgc ccagggcgaa 6780

cccattcacg gccgcaatca ccggtttttc cagcagctcc agccgccgaa acactttgtt 6840

ccccagaatg ccgaacttgc gcccctcgat ggtattcatt tctttcatct ccgagatgtc 6900

cgccccggcg acgaagctct tctcgcccgc gcccgtcagg atcacggcga gcacctccga 6960

gtcgttctcg atttcgccga tcacgtagtc catctctttc agggtgtccg agttcagggc 7020

gttgagggcc ttcgggcggt tgatggtcac caccgcgacc ttgccttctt tctccaggat 7080

gacattgttg agttccatga ctaatcctcc taaaatgtga aattgttatc cgctcacaat 7140

tccacacaac atacgagccg gaagcataaa gtgtaaagcc tggggaagct tggggagaac 7200

aatcagcccg gcaggggccg ggctgattgt gcctgcgtgc cgccggtgct tacggcagct 7260

tgaccacggc ttccccggtg acggccaggg ccccgccttg ggtaaagatc cgggtcgtca 7320

gcgtggcgat cggtttgtcc tcgcgcagcg cggtgacttc cacctcggcg gtcacttcgt 7380

cccccacgaa caccggcagt ttgaacgaga gcgattggcc caggtagatg ctccccttgc 7440

cggggagctg ctggccgagg aggcccgaga acagcgaggc gagcagcatg ccatgcacga 7500

tggggcgctc gaacgcggtg gtcgcggcaa acgcggggtc caggtgcagc gggttgaagt 7560

cctcggagag cgccgcaaag gccgccacct ccgccgcgcc aaaccgcttg gacagccggg 7620

ctttttgccc gacctccagc gactgggccg acatgcggcg tcctcctctg tttcagccca 7680

tatgcaggcc gccgttgagc gagaagtcgg cgccggtcga gaaaccggac tcctccgacg 7740

acaaccaggc gcagatcgag gcgatctctt ccggcaggcc caggcgcttg accgggatcg 7800

tcgcgacgat cttgtcgagc acgtcctggc ggatcgcctt gaccatgtcg gtggcgatat 7860

agcccggaga gaccgtgttg acggtcacgc ccttggtcgc cacttcctgc gccagtgcca 7920

tggtgaagcc atgcaggccg gccttggcgg tggagtagtt ggtctggccg aactggccct 7980

tctgcccgtt caccgacgag atgttgacga tgcggcccca gccacggtcg gccatgccgt 8040

cgatcacctg cttggtgacg ttgaacagcg aggtcaggtt ggtgtcgatc accgcatccc 8100

agtcggcgcg ggtcatcttg cggaacacca cgtcgcgggt gataccggcg ttgttgatca 8160

gcacatcaac ctcgccgacc tcggacttga ccttgtcgaa tgcggtcttg gtcgagtccc 8220

agtcagccac attgccttcc gaggcaatga aatcgaagcc cagggccttc tgctgctcca 8280

gccacttttc gcggcgcggc gagttggggc cgcaaccggc caccacacga aagccatcct 8340

tggccagccg ctggcaaatg gcggttccga taccacccat gccgccggtc acatacgcaa 8400

tgcgctgagt catgtccact ccttgattgg cttcgttatc gtcgccgggt ccgcgccaac 8460

cgcgcgcggc cccggaaaac cccttcctta tttgcgctcg actgccagcg ccacgcccat 8520

gccgccgccg atgcacagcg aggccaggcc cttcttcgcg tcacggcgct tcatctcgtg 8580

cagcagcgtc accaggatac ggcagcccga cgcgccgatc gggtggccga tggcgatggc 8640

gccgccgttc acattgacct tggaggtgtc ccagcccatc tgctggtgca ccgccagcgc 8700

ctgcgcggca aaggcctcgt tgatctccat caggtccagg tcttgcgggg tccactcggc 8760

gcgcgacagg gcgcgcttgg aggccggcac cgggcccatg cccatcacct tgggatcgac 8820

accggcgttg gcatagctct tgatcgtggc cagcggggtc aggcccagtt ccttggcctt 8880

ggccgccgac atcaccacca ccgcggcggc gccgtcgttc aggcccgagg cgttggccgc 8940

ggtcaccgtg ccggccttgt cgaaggcggg cttgaggccg gacatgctgt ccagcgtggc 9000

gccctggcgc acgaactcgt cggtcttgaa ggccaccggg tcgcccttgc gctgcgggat 9060

cagcaccggg acgatctctt cgtcaaactt gccggccttc tgcgcggctt cggccttgtt 9120

ctgcgagccg acggcgaact catcctgcgc ctcgcgtgtg atgccgtatt ccttggccac 9180

gttctcggcg gtgatgccca tgtggtactg gttgtacacg tcccacaggc cgtcgacgat 9240

catggtgtcg accagcttgg catcgcccat gcggaaacca tcgcgcgagc ccggcagcac 9300

gtgcggggcg gcgctcatgt tttcctggcc gccggccacc acgatctcgg cgtcgcccgc 9360

catgatcgcg ttggcggcca gcatcacggc cttcaggccc gagccgcaca ccttgttgat 9420

ggtcatggcc ggcaccatcg ccggcaggcc ggccttgatc gcggcctggc gtgcggggtt 9480

ctggcccgaa ccggcggtca gcacctggcc catgatgact tcgctcacct gctccggctt 9540

gacgccggcg cgctccagcg cggccttgat gaccacggca cccagttccg gtgccgggat 9600

cttggccagc gagccgccaa acttgccgac cgcggtgcgg gcggcggata cgatgacaac 9660

gtcagtcatt gtgtagtcct ttcaatggaa aggtacccag cttttgttcc ctttagtgag 9720

ggttaattgc gcgcttggcg taatcatggt catagctgtt tcctgtgtga aattgttatc 9780

cgctcacaat tccacacaac atacgagccg gaagcataaa gtgtaaagcc tggggtgcct 9840

aatgagtgag ctaactcaca ttaattgcgt tgcgctcact gcccgctttc cagtcgggaa 9900

acctgtcgtg ccagctgcat taatgaatcg gccaacgcgc ggggagaggc ggtttgcgta 9960

ttgggcgcat gcataaaaac tgttgtaatt cattaagcat tctgccgaca tggaagccat 10020

cacaaacggc atgatgaacc tgaatcgcca gcggcatcag caccttgtcg ccttgcgtat 10080

aatatttgcc catgggggtg ggcgaagaac tccagcatga gatccccgcg ctggaggatc 10140

atccagccgg cgtcccggaa aacgattccg aagcccaacc tttcatagaa ggcggcggtg 10200

gaatcgaaat ctcgtgatgg caggttgggc gtcgcttggt cggtcatttc gaaccccaga 10260

gtcccgctca gaagaactcg tcaagaaggc gatagaaggc gatgcgctgc gaatcgggag 10320

cggcgatacc gtaaagcacg aggaagcggt cagcccattc gccgccaagc tcttcagcaa 10380

tatcacgggt agccaacgct atgtcctgat agcggtccgc cacacccagc cggccacagt 10440

cgatgaatcc agaaaagcgg ccattttcca ccatgatatt cggcaagcag gcatcgccat 10500

gggtcacgac gagatcctcg ccgtcgggca tgcgcgcctt gagcctggcg aacagttcgg 10560

ctggcgcgag cccctgatgc tcttcgtcca gatcatcctg atcgacaaga ccggcttcca 10620

tccgagtacg tgctcgctcg atgcgatgtt tcgcttggtg gtcgaatggg caggtagccg 10680

gatcaagcgt atgcagccgc cgcattgcat cagccatgat ggatactttc tcggcaggag 10740

caaggtgaga tgacaggaga tcctgccccg gcacttcgcc caatagcagc cagtcccttc 10800

ccgcttcagt gacaacgtcg agcacagctg cgcaaggaac gcccgtcgtg gccagccacg 10860

atagccgcgc tgcctcgtcc tgcagttcat tcagggcacc ggacaggtcg gtcttgacaa 10920

aaagaaccgg gcgcccctgc gctgacagcc ggaacacggc ggcatcagag cagccgattg 10980

tctgttgtgc ccagtcatag ccgaatagcc tctccaccca agcggccgga gaacctgcgt 11040

gcaatccatc ttgttcaatc atgcgaaacg atcctcatcc tgtctcttga tcagatcttg 11100

atcccctgcg ccatcagatc cttggcggca agaaagccat ccagtttact ttgcagggct 11160

tcccaacctt accagagggc gccccagctg gcaattccgg ttcgcttgct gtccataaaa 11220

ccgcccagtc tagctatcgc catgtaagcc cactgcaagc tacctgcttt ctctttgcgc 11280

ttgcgttttc ccttgtccag atagcccagt agctgacatt catcccaggt ggcacttttc 11340

ggggaaatgt gcgcgcccgc gttcctgctg gcgctgggcc tgtttctggc gctggacttc 11400

ccgctgttcc gtcagcagct tttcgcccac ggccttgatg atcgcggcgg ccttggcctg 11460

catatcccga ttcaacggcc ccagggcgtc cagaacgggc ttcaggcgct cccgaaggt 11519

<210> 64
<211> 13749
<212> DNA
<213> Artificial Sequence

<220>
<223> vector

<400> 64
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480
acttgtccgc cagccacttg tgcgccttct cgaagaacgc cgcctgctgt tcttggctgg 540
ccgacttcca ccattccggg ctggccgtca tgacgtactc gaccgccaac acagcgtcct 600
tgcgccgctt ctctggcagc aactcgcgca gtcggcccat cgcttcatcg gtgctgctgg 660
ccgcccagtg ctcgttctct ggcgtcctgc tggcgtcagc gttgggcgtc tcgcgctcgc 720
ggtaggcgtg cttgagactg gccgccacgt tgcccatttt cgccagcttc ttgcatcgca 780
tgatcgcgta tgccgccatg cctgcccctc ccttttggtg tccaaccggc tcgacggggg 840
cagcgcaagg cggtgcctcc ggcgggccac tcaatgcttg agtatactca ctagactttg 900
cttcgcaaag tcgtgaccgc ctacggcggc tgcggcgccc tacgggcttg ctctccgggc 960
ttcgccctgc gcggtcgctg cgctcccttg ccagcccgtg gatatgtgga cgatggccgc 1020
gagcggccac cggctggctc gcttcgctcg gcccgtggac aaccctgctg gacaagctga 1080
tggacaggct gcgcctgccc acgagcttga ccacagggat tgcccaccgg ctacccagcc 1140
ttcgaccaca tacccaccgg ctccaactgc gcggcctgcg gccttgcccc atcaattttt 1200
ttaattttct ctggggaaaa gcctccggcc tgcggcctgc gcgcttcgct tgccggttgg 1260
acaccaagtg gaaggcgggt caaggctcgc gcagcgaccg cgcagcggct tggccttgac 1320
gcgcctggaa cgacccaagc ctatgcgagt gggggcagtc gaaggcgaag cccgcccgcc 1380
tgccccccga gcctcacggc ggcgagtgcg ggggttccaa gggggcagcg ccaccttggg 1440
caaggccgaa ggccgcgcag tcgatcaaca agccccggag gggccacttt ttgccggagg 1500
gggagccgcg ccgaaggcgt gggggaaccc cgcaggggtg cccttctttg ggcaccaaag 1560
aactagatat agggcgaaat gcgaaagact taaaaatcaa caacttaaaa aaggggggta 1620
cgcaacagct cattgcggca ccccccgcaa tagctcattg cgtaggttaa agaaaatctg 1680
taattgactg ccacttttac gcaacgcata attgttgtcg cgctgccgaa aagttgcagc 1740
tgattgcgca tggtgccgca accgtgcggc accctaccgc atggagataa gcatggccac 1800
gcagtccaga gaaatcggca ttcaagccaa gaacaagccc ggtcactggg tgcaaacgga 1860
acgcaaagcg catgaggcgt gggccgggct tattgcgagg aaacccacgg cggcaatgct 1920
gctgcatcac ctcgtggcgc agatgggcca ccagaacgcc gtggtggtca gccagaagac 1980
actttccaag ctcatcggac gttctttgcg gacggtccaa tacgcagtca aggacttggt 2040
ggccgagcgc tggatctccg tcgtgaagct caacggcccc ggcaccgtgt cggcctacgt 2100
ggtcaatgac cgcgtggcgt ggggccagcc ccgcgaccag ttgcgcctgt cggtgttcag 2160
tgccgccgtg gtggttgatc acgacgacca ggacgaatcg ctgttggggc atggcgacct 2220
gcgccgcatc ccgaccctgt atccgggcga gcagcaacta ccgaccggcc ccggcgagga 2280
gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400
ggacgatggc gagccgttgg agccgccgac acgggtcacg ctgccgcgcc ggtagcactt 2460
gggttgcgca gcaacccgta agtgcgctgt tccagactat cggctgtagc cgcctcgccg 2520
ccctatacct tgtctgcctc cccgcgttgc gtcgcggtgc atggagccgg gccacctcga 2580
cctgaatgga agccggcggc acctcgctaa cggattcacc gtttttatca ggctctggga 2640
ggcagaataa atgatcatat cgtcaattat tacctccacg gggagagcct gagcaaactg 2700
gcctcaggca tttgagaagc acacggtcac actgcttccg gtagtcaata aaccggtaaa 2760
ccagcaatag acataagcgg ctatttaacg accctgccct gaaccgacga ccgggtcgaa 2820
tttgctttcg aatttctgcc attcatccgc ttattatcac ttattcaggc gtagcaccag 2880
gcgtttaagg gcaccaataa ctgccttaaa aaaattacgc cccgccctgc cactcatcgc 2940
agtcggccta ttggttaaaa aatgagctga tttaacaaaa atttaacgcg aattttaaca 3000
aaatattaac gcttacaatt tccattcgcc attcaggctg cgcaactgtt gggaagggcg 3060
atcggtgcgg gcctcttcgc tattacgcca gctggcgaaa gggggatgtg ctgcaaggcg 3120
attaagttgg gtaacgccag ggttttccca gtcacgacgt tgtaaaacga cggccagtga 3180
gcgcgcgtaa tacgactcac tatagggcga attggagctc caccgcggtg gcggccgctc 3240
tagaactagt ggatccagca ggctgcctcg ataagcaaaa agggcggccc cgcggggccg 3300
ccctttttct ttccggcgac tgtcaggcca ctcagttgtt ggcggccttc acctgcgcga 3360
tcagctccgg gaccactttg ttcacgtcgc ccacgatcgc caggtcggcc accttcataa 3420
tcggggcctc cacgtcctta ttgatggcga tgatgtagtc cgagtcctgc atgccggcca 3480
ggtgctggat cgcccccgag atgccgcagg cgatgtacag ggtcgggcgc acggtcttgc 3540
cggtctggcc cacctggagg tctttgtcca cccactcttt ctcgatggcc gcgcgcgagg 3600
cggcgatggt gccgcccagg agcgaggcca gctcctccag tttttcgaag ttctccttcg 3660
agcccacgcc gcggcccccg gccacgagca ccttggcctc gccgatgtcc gcgatgtctt 3720
tggccagctt caccactttc gacactttgg tgcggatgtc ggacgcggtc agcttgatgg 3780
cgactttctc gatcttatcg tcggacacgt tggcgtcgtt caccggcagc ttctcgaaga 3840
agacgccggg gcggaccgtg gccatctgcg ggcggtggtc cgagcagacg atggtggcga 3900
tcaggttgcc gccgaacgcc gggcgggtcg ccaggaggtc ccggttctcc acgtcgatgt 3960
ccagcgaggt gcagtcggcg gtcaggccgg tcgacaggcg cgccgcgatc cgcgggccca 4020
ggtcccgccc gatgaacgtg gcgccaatga acaggatctc cggcttgcgc tcgttcacga 4080
ggtcgcagat caccttcgcg tagccgtcgg tcgagaagtg ggcgaggagt tcattgtcgg 4140
ccgcgagcac cttatccgcg ccgtggctca gcaggtcctt cgacatcttc tcggtgttgt 4200

ggcccagcag cacggcggtc agttccaccc ccagcttctc ggccatctcc ttgcccttgc 4260

ccagcagctc cagcgacacc ttctgcagct cgccgtcgcg ctgctccgca aacacccaca 4320

cgcccttgta gtcggccttg ttcattgaaa aatccctcct aacttaaata tgtgttcttc 4380

ttttagttct gcgagagcat atcggccgct tccttcaccg gtttatcaat gacctcgccc 4440

tgccctttca cttctttcgt gctcgacttc ttcactttgg tcggcgaccc cttcaggccc 4500

agattggctt tatccacgtc gatatcatcc gcggtccaca ttttcacctc cttgtcgaac 4560

gcgccgaaga ttttctccac cgacatgtac cgcgggacgt tcagttcctt aatggccgtc 4620

aggagcaccg gggtcttgac ttccacgacc tcgtacccat cctcccaggc cttccggatc 4680

ttgagcgtgt ccccatccac ctccactttt tccacgtagg tgacttgggg aatcccgaga 4740

tgttccgcaa tctccggccc gacctgcgcc gtatcgccgt cgatggcctg gcggccggca 4800

aagacaatgt catatttcag cttcttaatg ccggcggcaa tggtatgcga ggtggcgagg 4860

gtgtccgcgc ccccaaacgc ccggtccgtg agcagcaccg cctcatcggc ccccatggcc 4920

agcgcttcga ccagggcgtt cttggcttgg gggggcccca tcgagatgac cgtgacgtgc 4980

gccccatagt tatctttcag cacgagggct tcctcgagcg cgtttttgtc gtcggggtta 5040

atgatggacg ggacgccctc ccgaatcagg gtgcctttga cgggatcaat gcggacctcg 5100

gcggtgtcgg ggacttgttt gaggcagacc acaatattca tcctcttaac ctccttaaat 5160

tagcggaaga tcttgcccga gatcaccagc ttctgcacct ccgaggtgcc ctcgtagatc 5220

tccgtgatct tggcgtcgcg catcatgcgc tccacggggt agtccttggt gtagccatag 5280

ccgccgaaca gctgcacggc cttggtggtc acgtccatgg ccacgttggc ggcatgcagc 5340

ttggcgcggg cggcatccac ggtgtacggc aggcccgcct gcttcaggta ggcggccttg 5400

tacaccaggt agcgggccga ctcgatggcc acgtccatgt cggccatcat ccaggccagg 5460

ccctggaact tgtccagcga gcggccgaac tgcttgcgtt ccttcatgta cgcgcgggcc 5520

tcgttaaagg cgccttcggc gatgcccagg gcctgggccg cgatgccgat gcggcccccg 5580

tccagggtct tcatggcgat cgggaagccc ttgccctcct tgccaatcat gttctccacc 5640

ggcacgatca tgtcctcgaa caccagctcg gtggtcgacg acgcgcggat gcccagcttt 5700

tgctccacct tgccgatcga gaagcccttg aagcccttct cgatgataaa ggccgagatg 5760

cccttggtcc ccttggtgcg gtcggtcatg gcgaagatga cgaaggtgtc ggcgacgccg 5820

ccgttggtga tgaagatttt cgagccgttg atcacgtagt ggtcgccctc cagcacggcc 5880

acggtctgct gggcgcccga atcggtgccg gcgttcggct cggtcaggcc gtaggcgccg 5940

atcttctccc ccttggccag cggcaccagg tacttctgct tctgctcctc ggtgccgtgc 6000

tcgttaatca gcgaggcgca cagcgaggtg tgggccgaca ggatcacgcc ggtggtgccg 6060

cacaccttcg acagctcctc cacggcgatg atgtacgaca gcacgtcgcc gccggccccg 6120

ccgtactcct tcgagaacgg gatgcccatc atgccgtact ggcccatctt cttcacgttc 6180

tccatcggga accgctccgt ctcgtcgatc tccgcggcaa tcggcttcac ctcgttctcg 6240

gcgaactccc ggaccatctg gcgcaccagc tcttgctcgc gcgtcaggtt gaagtccata 6300

taaacttacc tcctagcggt tcttgaaccc ttcgatcttg cgcttctcaa tgaaggcggt 6360

catggcgtct ttctggtcct cggtcgagaa gcattccccg aacgcttccg actcgaaggc 6420

gagggccgtg tcaatgtcgc actgcatgcc gcggttaatg gcttgcttcg acagtttgac 6480

ggccaccggg gcattgctca cgattttgtt ggcgatctcc ttggcggtgt tcatcagttc 6540

cgagggctcc accaccttgt tcaccaggcc aatgcgcagg gcctcatcgg ccttgatgtt 6600

ctgcgcggtg aaaatcagct gcttggccat gcccatgccg acgaggcgcg acagccgctg 6660

ggtgccgcca aagcccgggg tgatgcccag gcccacttcc ggctggccga agcgggcgtt 6720

cgaggaggcg atgcggatat cgcaggacat cgcaatttcg cacccgccgc ccagggcgaa 6780

cccattcacg gccgcaatca ccggtttttc cagcagctcc agccgccgaa acactttgtt 6840

ccccagaatg ccgaacttgc gcccctcgat ggtattcatt tctttcatct ccgagatgtc 6900

cgccccggcg acgaagctct tctcgcccgc gcccgtcagg atcacggcga gcacctccga 6960

gtcgttctcg atttcgccga tcacgtagtc catctctttc agggtgtccg agttcagggc 7020

gttgagggcc ttcgggcggt tgatggtcac caccgcgacc ttgccttctt tctccaggat 7080

gacattgttg agttccatga ctaatcctcc taaaatgtga aattgttatc cgctcacaat 7140

tccacacaac atacgagccg gaagcataaa gtgtaaagcc tggggaagct tcttaagtaa 7200

taaaaataag agttacctta aatggtaact cttatttttt taatattgtt tcatagttta 7260

gaacgacttg atgtagatgt ccttcagctc cgagatcagc gggtagcgcg ggttggcggt 7320

ggtgcactgg tcgtcgaagg ccagctccga catcttgtcc agggtgttgt agaaatcctt 7380

cttgttgatg ccggcggccg agatgttctg cgggatcgac aggtcgatct tcagcttcga 7440

gatggcctcg atcagggcgg tcaccttctc cgtgtccgag gtgcccttca ggttcaggta 7500

ctcggcgatc tcggcgtact tgcgcttggc gttcggcgac ttgtactgcg ggaaggcggt 7560

ctgcttggtc gggcagtcgg tggcgttgta cttgatcacc tcctcgatca gcacggcgca 7620

ggcgatgccg tgcggcacgt ggtgcatggc gcccagcttg tgggccatcg agtggcacac 7680

gcccaggaag gcgttggcga aggccatgcc ggcgatgttc gaggcgtggg ccatcttctc 7740

gcgggcctcg atgtcgttgg tgccgttctt gtaggcgcgc ggcaggtact tgaagatcat 7800

cttgatggcg cgcagggcca gctcgtcggt gtagtccgtg gccatcaccg acacgtaggc 7860

ctcgatggcg tgcaccaggg catcaatgcc ggtggcggcg gtcagcttgc gcggcatgtt 7920

cagcatcagc tcggtgtcaa tgatggccat gttcggggtc agttcgtacg aggtcagcgg 7980

gtacttcatg ccggtctcgt cgttggtgat cacggcgaac ggggtggcct ccgagccggt 8040

gccggcggtg gtcgggatgg ccaccgagat ggccttggtg cccagcttcg ggaagttgca 8100

gatgcgcttg cggatgtcca tgaaattgat ggcgaggttc tcgatctccg cctccgggta 8160

ctcgtacagc aggtgcatca ccttggcggc gtccatcggc gagccgccgc cgatcgagat 8220

gatggtgtcc ggctcgaagt tcagcatctc cttggcgccc ttcttcaccg agtcaatggt 8280

cgggtccgac ttgatatcgg tgaagatcga gtacttgatg tcgatctcgt ccagcacctt 8340

ggtgatcttg ttcacgtagc ccagcttgaa caggtccttg tcggtcacga tgaaggcgcg 8400

cttcttgttc atgtccttca gctccttcag ggcgaagcgc aggcagccgt acttgaagta 8460

gatcttctgc ggcaccttga accacagcat attctcgcgg cgctcggcca ccgacttgat 8520

gttcagcagg tgcttcggct ccacgttctg cgacaccgag ttgccgcccc aggtcccgca 8580

gcccagggtg aacgacgggg cgatggcgaa gttgtacagg tcgcccgagg cgccctgcga 8640

cgacggcatg ttgatgaagg tgcgcgaggt cttcatggcc aggccgaact ccttcacctt 8700

gtccttgttg ttctgcgagt cgatgtacag cgacgaggtg tggcccgagc cgcccagctc 8760

gatcaggcgc tgggccttct tgagggcctc atcgaagtcc ttcaccttgt acatggccag 8820

caccggcgac agcttctcgt gcgagaacag ctccgacttc tccaccgact gcacctcgcc 8880

gatgaggatc ttggtggtct gcggcacctc gatgccggcc atcttggcga tgatgtaggc 8940

cgacttgccc acgatgtcgg cgttgatggc gccgttcttg aacatcgtct ccttgatctt 9000

ggcgatctcg ttctggttca ggatgtacga gccgcgcttc acgaactcct ccttcacctt 9060

ctcgtagatg gagttcatca ccaggatcga ctgctccgag gcgcagatca cgccgttgtc 9120

gtaggtcttc gacaggatga tcgacgacac ggccatatcg atgtcggcgg actcgtcgat 9180

gatggccggg gtgttgcccg cgcccacgcc gatggccggc ttgcccgacg agtaggcggc 9240

cttcaccatc gacgggccgc cggtggccag gatgatatcg gcctccgaca tcaggtcctg 9300

cgacagctcg atcgacggct cgtcaatcca gccgatgatg ttcttcgggg ccccggcctt 9360

cacggcggcg tccaggatca gcttggcggc ggcgatggtc gacttcttgg cgcgcgggtg 9420

cggcgagaag aagatggcgt tgcgggtctt cagcgagatc agcgacttga agatggcggt 9480

cgaggtcggg ttcgtggtcg gcacgatggc ggccacgatg ccgatcggct cggccacctt 9540

ggtgatgccc agcgagtcgt cgtggtcgat aatgccgcag gtcttctcgt tcttgtactt 9600

gttgtagatg tactcggcgg cgaagtggtt cttgatgatc ttgtcctcca ccaggccgat 9660

gccggtctcc tccacggcca gcttggccag gttgatgcgc tccttggcgg cggcaatggc 9720

gcactgcttg aagatcttgt ccacctgctc ctgggtgtag gtggcgaact tcttctgggc 9780

ctcgcgcagc tcgttcagct tctgcttcag ctccttctgg ttggtcacct tcattcttga 9840

atctcctgaa aatttctttt tatttcgagt agtcgtaaaa gcccttgccg ctcttgcggc 9900

ccagccagcc cgcccggaca tactttttca gcagcgtgtg cgggcgatac ttcgaatcgc 9960

cggtttccga gtacagcacg tccatgatgg ccaggcaaat gtcgaggccg ataaagtccc 10020

ccagttccag cgggcccatc gggtggttcg cccccagctt catggcctta tcaatatcct 10080

cgaccgaggc aatcccttcg gcgaggatgc ccaccgcttc attgatcatc gggatcagaa 10140

tgcggttgac gacgaacccc ggcgcctcgg cgacttccac ggggtccttg ccaatggcaa 10200

tcgacgtctc tttcaccgcg tcgaaggttt cctgcgacgt ggcaatgccg cgaatgacct 10260

ccacgagttt catgacgggg gcgggattaa agaaatgcat gccgatcact ttgtcgttgg 10320

tcttggtcgc gctcgccacc tccgtgatcg agagcgagct ggtgttggag gcgagaatcg 10380

tctccggttt gcagatgttg tcgagatccg caaagatctg tttcttaatg tccatgcgct 10440

cgacggcggc ttcaatcacg aggtcgcagt ccgcggccat gttcaggtcc acggtcccgc 10500

tgatgcgggt gagaatctcc accttggtgg cctcttcgat cttgcctttt ttgaccagtt 10560

tcgacaggtt cttgttaatg aaatccaggc cgcgatcgac gaattcatcc ttgatgtccc 10620

ggaggaccac ctcgaagcct ttggcggcga aggcctgcgc gatgccggac cccatcgtcc 10680

ccgcgccaat gacgcacacc ttcttcattc ttgaatctcc tgaaactagc acttttccag 10740

caggatcgcg gtgccctggc cgccgccgat gcacagggtc gccagcccct tcttggcgtc 10800

gcgcttctgc atcgcgtgca ccagggtcac caggatgcgg gcgcccgagg cgccgatcgg 10860

gtgccccagg gcgatggcgc cgccattcac gttcactttg ttcatgtcga acttcaggtc 10920

cttggcgacg gccagcgact gggcggcaaa ggcctcgttc gactcgatca ggtccagctc 10980

gtcgaccgtc cagccggctt tctcgatcgc cgccttggtg gcgtagaacg ggccgtagcc 11040

catgatggcc gggtccacgc cggccgagcc atacgacacg atcttcgcca gcggtttcac 11100

gcccagctcc ttggcctttt cggccgacat gatcaccagc acggccgcgc agtcgttcag 11160

gcccgaggcg ttgcccgcgg tcacggtgcc gtccttcttg aaggccggct tcagcttggc 11220

caggccctcg atcgtcgacc cgaagcgcgg gtgctcgtcc gtgtccacca cggtctcgcc 11280

cttgcggccc ttaatcacca ccggcacgat ctcgtccttg aactggcccg acttgatggc 11340

ttcctccgcc ttcttttgcg aggccagggc gaactcgtcc tgctcctcgc gcgagatgtt 11400

ccagcgctcg gcgatgttct cggcggtgat gcccatgtgg tagtcgttga aggcgtccca 11460

caggccgtcg gtgatcatct cgtccacgaa cttggcgttc cccatgcggt agccccagcg 11520

ggcgttgttg gccaggtacg gggcgcgcga catgttttcc atgccgccgg caatgatcac 11580

gtcggcgtcg ccggccttga tgatctgggc cgccagcgac acggtgcgca ggcccgagcc 11640

gcacaccttg ttgatggtca tggcggggat ctccaccggg aggccggctt tgaagctcgc 11700

ctggcgggcc gggttctggc cgagcccggc ctgcagcacg ttgcccagga tcacctcgtt 11760

cacgtcctcc ggcttgatgc cggccttctt cacggcctcc ttaatggcgg tggcgcccag 11820

gtccacggcg ggcacgtctt tcaggctctt gccgtacgag ccgatcgcgg tgcgcacggc 11880

cgaggcaatc accacctcct tcattcttga atctcctgaa aggtacccag cttttgttcc 11940

ctttagtgag ggttaattgc gcgcttggcg taatcatggt catagctgtt tcctgtgtga 12000

aattgttatc cgctcacaat tccacacaac atacgagccg gaagcataaa gtgtaaagcc 12060

tggggtgcct aatgagtgag ctaactcaca ttaattgcgt tgcgctcact gcccgctttc 12120

cagtcgggaa acctgtcgtg ccagctgcat taatgaatcg gccaacgcgc ggggagaggc 12180

ggtttgcgta ttgggcgcat gcataaaaac tgttgtaatt cattaagcat tctgccgaca 12240

tggaagccat cacaaacggc atgatgaacc tgaatcgcca gcggcatcag caccttgtcg 12300

ccttgcgtat aatatttgcc catgggggtg ggcgaagaac tccagcatga gatccccgcg 12360

ctggaggatc atccagccgg cgtcccggaa aacgattccg aagcccaacc tttcatagaa 12420

ggcggcggtg gaatcgaaat ctcgtgatgg caggttgggc gtcgcttggt cggtcatttc 12480

gaaccccaga gtcccgctca gaagaactcg tcaagaaggc gatagaaggc gatgcgctgc 12540

gaatcgggag cggcgatacc gtaaagcacg aggaagcggt cagcccattc gccgccaagc 12600

tcttcagcaa tatcacgggt agccaacgct atgtcctgat agcggtccgc cacacccagc 12660

cggccacagt cgatgaatcc agaaaagcgg ccattttcca ccatgatatt cggcaagcag 12720

gcatcgccat gggtcacgac gagatcctcg ccgtcgggca tgcgcgcctt gagcctggcg 12780

aacagttcgg ctggcgcgag cccctgatgc tcttcgtcca gatcatcctg atcgacaaga 12840

ccggcttcca tccgagtacg tgctcgctcg atgcgatgtt tcgcttggtg gtcgaatggg 12900

caggtagccg gatcaagcgt atgcagccgc cgcattgcat cagccatgat ggatactttc 12960

tcggcaggag caaggtgaga tgacaggaga tcctgccccg gcacttcgcc caatagcagc 13020

cagtcccttc ccgcttcagt gacaacgtcg agcacagctg cgcaaggaac gcccgtcgtg 13080

gccagccacg atagccgcgc tgcctcgtcc tgcagttcat tcagggcacc ggacaggtcg 13140

gtcttgacaa aaagaaccgg gcgcccctgc gctgacagcc ggaacacggc ggcatcagag 13200

cagccgattg tctgttgtgc ccagtcatag ccgaatagcc tctccaccca agcggccgga 13260

gaacctgcgt gcaatccatc ttgttcaatc atgcgaaacg atcctcatcc tgtctcttga 13320

tcagatcttg atcccctgcg ccatcagatc cttggcggca agaaagccat ccagtttact 13380

ttgcagggct tcccaacctt accagagggc gccccagctg gcaattccgg ttcgcttgct 13440

gtccataaaa ccgcccagtc tagctatcgc catgtaagcc cactgcaagc tacctgcttt 13500

ctctttgcgc ttgcgttttc ccttgtccag atagcccagt agctgacatt catcccaggt 13560

ggcacttttc ggggaaatgt gcgcgcccgc gttcctgctg gcgctgggcc tgtttctggc 13620

gctggacttc ccgctgttcc gtcagcagct tttcgcccac ggccttgatg atcgcggcgg 13680

ccttggcctg catatcccga ttcaacggcc ccagggcgtc cagaacgggc ttcaggcgct 13740

cccgaaggt 13749

<210> 65
<211> 11155
<212> DNA
<213> Artificial Sequence

<220>
<223> vector

<400> 65
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaattttct ctggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag gggccacttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatccagca ggctgcctcg ataagcaaaa agggcggccc cgcggggccg 3300

ccctttttct ttccggcgac tgtcaggcca ctcagttgtt ggcggccttc acctgcgcga 3360

tcagctccgg gaccactttg ttcacgtcgc ccacgatcgc caggtcggcc accttcataa 3420

tcggggcctc cacgtcctta ttgatggcga tgatgtagtc cgagtcctgc atgccggcca 3480

ggtgctggat cgcccccgag atgccgcagg cgatgtacag ggtcgggcgc acggtcttgc 3540

cggtctggcc cacctggagg tctttgtcca cccactcttt ctcgatggcc gcgcgcgagg 3600

cggcgatggt gccgcccagg agcgaggcca gctcctccag tttttcgaag ttctccttcg 3660

agcccacgcc gcggcccccg gccacgagca ccttggcctc gccgatgtcc gcgatgtctt 3720

tggccagctt caccactttc gacactttgg tgcggatgtc ggacgcggtc agcttgatgg 3780

cgactttctc gatcttatcg tcggacacgt tggcgtcgtt caccggcagc ttctcgaaga 3840

agacgccggg gcggaccgtg gccatctgcg ggcggtggtc cgagcagacg atggtggcga 3900

tcaggttgcc gccgaacgcc gggcgggtcg ccaggaggtc ccggttctcc acgtcgatgt 3960

ccagcgaggt gcagtcggcg gtcaggccgg tcgacaggcg cgccgcgatc cgcgggccca 4020

ggtcccgccc gatgaacgtg gcgccaatga acaggatctc cggcttgcgc tcgttcacga 4080

ggtcgcagat caccttcgcg tagccgtcgg tcgagaagtg ggcgaggagt tcattgtcgg 4140

ccgcgagcac cttatccgcg ccgtggctca gcaggtcctt cgacatcttc tcggtgttgt 4200

ggcccagcag cacggcggtc agttccaccc ccagcttctc ggccatctcc ttgcccttgc 4260

ccagcagctc cagcgacacc ttctgcagct cgccgtcgcg ctgctccgca aacacccaca 4320

cgcccttgta gtcggccttg ttcattgaaa aatccctcct aacttaaata tgtgttcttc 4380

ttttagttct gcgagagcat atcggccgct tccttcaccg gtttatcaat gacctcgccc 4440

tgccctttca cttctttcgt gctcgacttc ttcactttgg tcggcgaccc cttcaggccc 4500

agattggctt tatccacgtc gatatcatcc gcggtccaca ttttcacctc cttgtcgaac 4560

gcgccgaaga ttttctccac cgacatgtac cgcgggacgt tcagttcctt aatggccgtc 4620

aggagcaccg gggtcttgac ttccacgacc tcgtacccat cctcccaggc cttccggatc 4680

ttgagcgtgt ccccatccac ctccactttt tccacgtagg tgacttgggg aatcccgaga 4740

tgttccgcaa tctccggccc gacctgcgcc gtatcgccgt cgatggcctg gcggccggca 4800

aagacaatgt catatttcag cttcttaatg ccggcggcaa tggtatgcga ggtggcgagg 4860

gtgtccgcgc ccccaaacgc ccggtccgtg agcagcaccg cctcatcggc ccccatggcc 4920

agcgcttcga ccagggcgtt cttggcttgg gggggcccca tcgagatgac cgtgacgtgc 4980

gccccatagt tatctttcag cacgaggcct tcctcgagcg cgtttttgtc gtcggggtta 5040

atgatggacg ggacgccctc ccgaatcagg gtgcctttga cgggatcaat gcggacctcg 5100

gcggtgtcgg ggacttgttt gaggcagacc acaatattca tcctcttaac ctccttaaat 5160

tagcggaaga tcttgcccga gatcaccagc ttctgcacct ccgaggtgcc ctcgtagatc 5220

tccgtgatct tggcgtcgcg catcatgcgc tccacggggt agtccttggt gtagccatag 5280
ccgccgaaca gctgcacggc cttggtggtc acgtccatgg ccacgttggc ggcatgcagc 5340
ttggcgcggg cggcatccac ggtgtacggc aggcccgcct gcttcaggta ggcggccttg 5400
tacaccaggt agcgggccga ctcgatggcc acgtccatgt cggccatcat ccaggccagg 5460
ccctggaact tgtccagcga gcggccgaac tgcttgcgtt ccttcatgta cgcgcgggcc 5520
tcgttaaagg cgccttcggc gatgcccagg gcctgggccg cgatgccgat gcggcccccg 5580
tccagggtct tcatggcgat cgggaagccc ttgccctcct tgccaatcat gttctccacc 5640
ggcacgatca tgtcctcgaa caccagctcg gtggtcgacg acgcgcggat gcccagcttt 5700
tgctccacct tgccgatcga gaagcccttg aagcccttct cgatgataaa ggccgagatg 5760
cccttggtcc ccttggtgcg gtcggtcatg gcgaagatga cgaaggtgtc ggcgacgccg 5820
ccgttggtga tgaagatttt cgagccgttg atcacgtagt ggtcgccctc cagcacggcc 5880
acggtctgct gggcgcccga atcggtgccg gcgttcggct cggtcaggcc gtaggcgccg 5940
atcttctccc ccttggccag cggcaccagg tacttctgct tctgctcctc ggtgccgtgc 6000
tcgttaatca gcgaggcgca cagcgaggtg tgggccgaca ggatcacgcc ggtggtgccg 6060
cacaccttcg acagctcctc cacggcgatg atgtacgaca gcacgtcgcc gccggccccg 6120
ccgtactcct tcgagaacgg gatgcccatc atgccgtact ggcccatctt cttcacgttc 6180
tccatcggga accgctccgt ctcgtcgatc tccgcggcaa tcggcttcac ctcgttctcg 6240
gcgaactccc ggaccatctg gcgcaccagc tcttgctcgc gcgtcaggtt gaagtccata 6300
taaacttacc tcctagcggt tcttgaaccc ttcgatcttg cgcttctcaa tgaaggcggt 6360
catggcgtct ttctggtcct cggtcgagaa gcattccccg aacgcttccg actcgaaggc 6420
gagggccgtg tcaatgtcgc actgcatgcc gcggttaatg gcttgcttcg acagtttgac 6480
ggccaccggg gcattgctca cgattttgtt ggcgatctcc ttggcggtgt tcatcagttc 6540
cgagggctcc accaccttgt tcaccaggcc aatgcgcagg gcctcatcgg ccttgatgtt 6600
ctgcgcggtg aaaatcagct gcttggccat gcccatgccg acgaggcgcg acagccgctg 6660
ggtgccgcca aagcccgggg tgatgcccag gcccacttcc ggctggccga agcgggcgtt 6720
cgaggaggcg atgcggatat cgcaggacat cgcaatttcg cacccgccgc ccagggcgaa 6780
cccattcacg gccgcaatca ccggtttttc cagcagctcc agccgccgaa acactttgtt 6840
ccccagaatg ccgaacttgc gcccctcgat ggtattcatt tctttcatct ccgagatgtc 6900
cgccccggcg acgaagctct tctcgcccgc gcccgtcagg atcacggcga gcacctccga 6960
gtcgttctcg atttcgccga tcacgtagtc catctctttc agggtgtccg agttcagggc 7020
gttgagggcc ttcggcggtt gatggtcacc accgcgacct tgccttcttt ctccaggat 7080
gacattgttg agttccatga ctaatcctcc taaaatgtga aattgttatc cgctcacaat 7140

tccacacaac atacgagccg gaagcataaa gtgtaaagcc tggggaagct tcttaagtaa 7200

taaaaataag agttacctta aatggtaact cttatttttt taatattgtt tcatagtatt 7260

tctttttatt tcgagtagtc gtaaaagccc ttgccgctct tgcggcccag ccagcccgcc 7320

cggacatact ttttcagcag cgtgtgcggg cgatacttcg aatcgccggt ttccgagtac 7380

agcacgtcca tgatggccag gcaaatgtcg aggccgataa agtcccccag ttccagcggg 7440

cccatcgggt ggttcgcccc cagcttcatg gccttatcaa tatcctcgac cgaggcaatc 7500

ccttcggcga ggatgcccac cgcttcattg atcatcggga tcagaatgcg gttgacgacg 7560

aaccccggcg cctcggcgac ttccacgggg tccttgccaa tggcaatcga cgtctctttc 7620

accgcgtcga aggtttcctg cgacgtggca atgccgcgaa tgacctccac gagtttcatg 7680

acgggggcgg gattaaagaa atgcatgccg atcactttgt cgttggtctt ggtcgcgctc 7740

gccacctccg tgatcgagag cgagctggtg ttggaggcga gaatcgtctc cggtttgcag 7800

atgttgtcga gatccgcaaa gatctgtttc ttaatgtcca tgcgctcgac ggcggcttca 7860

atcacgaggt cgcagtccgc ggccatgttc aggtccacgg tcccgctgat gcgggtgaga 7920

atctccacct tggtggcctc ttcgatcttg ccttttttga ccagtttcga caggttcttg 7980

ttaatgaaat ccaggccgcg atcgacgaat tcatccttga tgtcccggag gaccacctcg 8040

aagcctttgg cggcgaaggc ctgcgcgatg ccggacccca tcgtccccgc gccaatgacg 8100

cacaccttct tcattcttga atctcctgaa actagcactt ttccagcagg atcgcggtgc 8160

cctggccgcc gccgatgcac agggtcgcca gccccttctt ggcgtcgcgc ttctgcatcg 8220

cgtgcaccag ggtcaccagg atgcgggcgc ccgaggcgcc gatcgggtgc cccagggcga 8280

tggcgccgcc attcacgttc actttgttca tgtcgaactt caggtccttg gcgacggcca 8340

gcgactgggc ggcaaaggcc tcgttcgact cgatcaggtc cagctcgtcg accgtccagc 8400

cggctttctc gatcgccgcc ttggtggcgt agaacgggcc gtagcccatg atggccgggt 8460

ccacgccggc cgagccatac gacacgatct tcgccagcgg tttcacgccc agctccttgg 8520

ccttttcggc cgacatgatc accagcacgg ccgcgcagtc gttcaggccc gaggcgttgc 8580

ccgcggtcac ggtgccgtcc ttcttgaagg ccggcttcag cttggccagg ccctcgatcg 8640

tcgacccgaa gcgcgggtgc tcgtccgtgt ccaccacggt ctcgcccttg cggcccttaa 8700

tcaccaccgg cacgatctcg tccttgaact ggcccgactt gatggcttcc tccgccttct 8760

tttgcgaggc cagggcgaac tcgtcctgct cctcgcgcga gatgttccag cgctcggcga 8820

tgttctcggc ggtgatgccc atgtggtagt cgttgaaggc gtcccacagg ccgtcggtga 8880

tcatctcgtc cacgaacttg gcgttcccca tgcggtagcc ccagcgggcg ttgttggcca 8940

ggtacggggc gcgcgacatg ttttccatgc cgccggcaat gatcacgtcg gcgtcgccgg 9000

ccttgatgat ctgggccgcc agcgacacgg tgcgcaggcc cgagccgcac accttgttga 9060

tggtcatggc ggggatctcc accgggaggc cggctttgaa gctcgcctgg cgggccgggt 9120

tctggccgag cccggcctgc agcacgttgc ccaggatcac ctcgttcacg tcctccggct 9180

tgatgccggc cttcttcacg gcctccttaa tggcggtggc gcccaggtcc acggcgggca 9240

cgtctttcag gctcttgccg tacgagccga tcgcggtgcg cacggccgag gcaatcacca 9300

cctccttcat tcttgaatct cctgaaaggt acccagcttt tgttcccttt agtgagggtt 9360

aattgcgcgc ttggcgtaat catggtcata gctgtttcct gtgtgaaatt gttatccgct 9420

cacaattcca cacaacatac gagccggaag cataaagtgt aaagcctggg gtgcctaatg 9480

agtgagctaa ctcacattaa ttgcgttgcg ctcactgccc gctttccagt cgggaaacct 9540

gtcgtgccag ctgcattaat gaatcggcca acgcgcgggg agaggcggtt tgcgtattgg 9600

gcgcatgcat aaaaactgtt gtaattcatt aagcattctg ccgacatgga agccatcaca 9660

aacggcatga tgaacctgaa tcgccagcgg catcagcacc ttgtcgcctt gcgtataata 9720

tttgcccatg ggggtgggcg aagaactcca gcatgagatc cccgcgctgg aggatcatcc 9780

agccggcgtc ccggaaaacg attccgaagc ccaacctttc atagaaggcg gcggtggaat 9840

cgaaatctcg tgatggcagg ttgggcgtcg cttggtcggt catttcgaac cccagagtcc 9900

cgctcagaag aactcgtcaa gaaggcgata gaaggcgatg cgctgcgaat cgggagcggc 9960

gataccgtaa agcacgagga agcggtcagc ccattcgccg ccaagctctt cagcaatatc 10020

acgggtagcc aacgctatgt cctgatagcg gtccgccaca cccagccggc cacagtcgat 10080

gaatccagaa aagcggccat tttccaccat gatattcggc aagcaggcat cgccatgggt 10140

cacgacgaga tcctcgccgt cgggcatgcg cgccttgagc ctggcgaaca gttcggctgg 10200

cgcgagcccc tgatgctctt cgtccagatc atcctgatcg acaagaccgg cttccatccg 10260

agtacgtgct cgctcgatgc gatgtttcgc ttggtggtcg aatgggcagg tagccggatc 10320

aagcgtatgc agccgccgca ttgcatcagc catgatggat actttctcgg caggagcaag 10380

gtgagatgac aggagatcct gccccggcac ttcgcccaat agcagccagt cccttcccgc 10440

ttcagtgaca acgtcgagca cagctgcgca aggaacgccc gtcgtggcca gccacgatag 10500

ccgcgctgcc tcgtcctgca gttcattcag ggcaccggac aggtcggtct tgacaaaaag 10560

aaccgggcgc ccctgcgctg acagccggaa cacggcggca tcagagcagc cgattgtctg 10620

ttgtgcccag tcatagccga atagcctctc cacccaagcg gccggagaac ctgcgtgcaa 10680

tccatcttgt tcaatcatgc gaaacgatcc tcatcctgtc tcttgatcag atcttgatcc 10740

cctgcgccat cagatccttg gcggcaagaa agccatccag tttactttgc agggcttccc 10800

aaccttacca gagggcgccc cagctggcaa ttccggttcg cttgctgtcc ataaaaccgc 10860

ccagtctagc tatcgccatg taagcccact gcaagctacc tgctttctct ttgcgcttgc 10920

gttttccctt gtccagatag cccagtagct gacattcatc ccaggtggca cttttcgggg 10980

aaatgtgcgc gcccgcgttc ctgctggcgc tgggcctgtt tctggcgctg gacttcccgc 11040

tgttccgtca gcagcttttc gcccacggcc ttgatgatcg cggcggcctt ggcctgcata 11100

tcccgattca acggccccag ggcgtccaga acgggcttca ggcgctcccg aaggt 11155

<210> 66
<211> 13317
<212> DNA
<213> Artificial Sequence

<220>
<223> vector

<400> 66
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaattttct ctggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag gggccacttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatccagca ggctgcctcg ataagcaaaa agggcggccc cgcggggccg 3300

ccctttttct ttccggcgac tgtcaggcca ctcagttgtt ggcggccttg acttgggcga 3360

tcagctcggg caccaccttg ttcacatcgc ccacaatggc caggtcggcc accttcatga 3420

tcggggcttc cacgtccttg ttgatcgcga tgatgtagtc cgagtcctgc atgccggcca 3480

ggtgctggat ggcgcccgag atgccgcagg cgatgtacag ggtcgggcgc acggtcttgc 3540

cggtctgccc cacttggagg tccttatcca cccactcctt ttcgatggcg gcgcgcgagg 3600

cggcgatggt gccgccgagg agggaggcca gctcctccag cttctcaaag ttctccttcg 3660

agcccacgcc gcggcccccg gccacgagca ccttggcctc gccgatatcg gcaatatcct 3720

tggcgagctt caccactttc gagaccttgg tgcggatgtc cgaggcggtc agcttgatgg 3780

ccactttctc gatcttgtcg tccgacacgt tggcgtcgtt caccggcagc ttctcgaaga 3840

acacgccggg ccgcaccgtg gccatctgcg ggcggtggtc cgagcacacg atggtggcga 3900

tcaggttgcc gccgaaggcc gggcgggtcg cgagcagatc gcggttctcc acgtcgatgt 3960

ccagcgaggt gcagtcggcg gtcaggccgg tcgacagccg ggcggcgatc cgcgggccga 4020

ggtcgcggcc gatgaacgtg gcgccgatga acaggatctc cggcttgcgc tcgttcacca 4080

ggtcacagat caccttggcg tagccgtcgg tgctgaaatg ggccagcagc tcgttgtcgg 4140

cggccaggac cttgtccgcg ccgtgcgaca gcaggtcctt cgacatcttc tcggtgttgt 4200

gccccagcag cacggccgtc agctccacgc ccagtttctc ggccatttcc ttgcccttcc 4260

ccagcagttc cagcgacacc ttctgcagct cgccgtcgcg ttgctcggcg aacacccaca 4320

cgcccttgta gtcggctttg ttcattgaaa aatccctcct aacttaaata tgtgttcttc 4380

ttttagttct gcgacagcat atcggccgcc tccttcacgg gcttatcgat cacttcgcct 4440

tggcccttga cttctttggt cgacgacttt ttcaccttgg tcggcgagcc cttcagcccc 4500

aggttggcct tgtcgacgtc gatgtcatcc gcggtccaca tcttgacctc cttgtcgaac 4560

gcgccgaaaa tcttttccac cgacatgtag cgcggcacgt tcagctcctt gatggccgtc 4620

agcaggaccg gggtcttcac ctccacgacc tcgtagccgt cctcccaggc cttgcggatt 4680

ttcagcgtat cgccatccac ctcgactttc tccacgtagg tcacctgggg gatgcccaga 4740

tgctcggcga tctccggccc gacctgggcc gtgtcgccgt caatggcctg gcgcccggca 4800

aacacgatat catatttcag cttcttgatg cccgcggcga tggtgtgcga ggtcgccagg 4860

gtatccgccc ccccgaaggc gcggtcggtc agcagcaccg cctcgtccgc ccccatggcc 4920

agggcctcca ccagggcgtt cttggcctgc gggggcccca tcgagatcac ggtcacatgg 4980

gccccgtagt tgtctttcag gaccagcgct tcctccagcg cattcttgtc gtccgggttg 5040

atgatcgacg gcacgccctc gcggatcagg gtccccttca ccggatcgat gcgcacctcg 5100

gcggtgtccg gcacctgctt cagacacacc acgatgttca tcctcttaac ctccttaaat 5160

tagcggaaaa tcttgccgct aatcaccagc ttctgcactt ccgacgtgcc ctcgtagatc 5220

tcggtaatct tggcgtcgcg catcatgcgc tcgaccgggt agtccttcgt gtagccgtag 5280

ccgccgaaca gttgcaccgc tttggtggtc acgtccatgg cgacgttggc ggcatgcagc 5340

ttggcgcgcg cggcgtccac ggtgtacggc aggcccgcct gcttcaggta ggcggcctta 5400

tagaccagat accgggcgga ctcaatggcc acgtccatat cggccatcat ccaggccagg 5460

ccctgaaact tgtccagcga gcggccgaac tgcttgcgct ccttcatgta ggcccgggct 5520

tcgttgaagg cgccctccgc gatccccagg gcttgggccg caatgccgat ccgcccccca 5580

tccagggttt tcatcgcaat cgggaacccc ttcccctctt tgccaatcat gttttccacc 5640

ggcacgatca tgtcctcgaa caccagttcg gtggtcgacg acgcgcggat gcccagcttt 5700

tgctccacct tgccgatcga gaagccctta aaccctttct caatgataaa ggccgagatg 5760

cctttggtgc ccttggtgcg gtcggtcatc gcgaagatca cgaacgtgtc cgccacgccg 5820

ccgttggtaa taaagatttt ggagccgttg atcacgtagt ggtcgccttc caggacggcc 5880

acggtctgct gcgcgcccga gtcggtgccg gcgttcggtt ccgtcaggcc gtacgcgccg 5940

atcttctccc ctttggccag cgggaccagg tacttctgtt tctgctcctc ggtcccgtgc 6000

tcgttgatca gcgaggcgca gagggaggtg tgggccgaca ggatcacccc ggtggtgccg 6060

cacaccttgg acagctcctc cacggcgatg atgtacgaca gcacgtcccc ccccgccccg 6120

ccgtactcct tcgagaacgg aatccccatc atgccatact ggcccatctt cttcacgttc 6180

tccatcggga agcgctcggt ctcgtcgatc tccgcggcaa tgggcttcac ctcgttttcc 6240

gcgaactcgc gcaccatctg gcgcaccagc tcctgctcgc gggtcaggtt gaagtccata 6300

taaacttacc tcctatctat gtgaaattgt tatccgctca caattccaca caacatacga 6360

gccggaagca taaagtgtaa agcctgggga agcttgggga gaacaatcag cccggcaggg 6420

gccgggctga ttgtgcctgc gtgccttaga acgacttgat gtagatgtcc ttcagctccg 6480

agatcagcgg gtagcgcggg ttggcggtgg tgcactggtc gtcgaaggcc agctccgaca 6540

tcttgtccag ggtgttgtag aaatccttct tgttgatgcc ggcggccgag atgttctgcg 6600

ggatcgacag gtcgatcttc agcttcgaga tggcctcgat cagggcggtc accttctccg 6660

tgtccgaggt gcccttcagg ttcaggtact cggcgatctc ggcgtacttg cgcttggcgt 6720

tcggcgactt gtactgcggg aaggcggtct gcttggtcgg gcagtcggtg gcgttgtact 6780

tgatcacctc ctcgatcagc acggcgcagg cgatgccgtg cggcacgtgg tgcatggcgc 6840

ccagcttgtg ggccatcgag tggcacacgc ccaggaaggc gttggcgaag gccatgccgg 6900

cgatgttcga ggcgtgggcc atcttctcgc gggcctcgat gtcgttggtg ccgttcttgt 6960

aggcgcgcgg caggtacttg aagatcatct tgatggcgcg cagggccagc tcgtcggtgt 7020

agtccgtggc catcaccgac acgtaggcct cgatggcgtg caccagggca tcaatgccgg 7080

tggcggcggt cagcttgcgc ggcatgttca gcatcagctc ggtgtcaatg atggccatgt 7140

tcggggtcag ttcgtacgag gtcagcgggt acttcatgcc ggtctcgtcg ttggtgatca 7200

cggcgaacgg ggtggcctcc gagccggtgc cggcggtggt cgggatggcc accgagatgg 7260

ccttggtgcc cagcttcggg aagttgcaga tgcgcttgcg gatgtccatg aaattgatgg 7320

cgaggttctc gatctccgcc tccgggtact cgtacagcag gtgcatcacc ttggcggcgt 7380

ccatcggcga gccgccgccg atcgagatga tggtgtccgg ctcgaagttc agcatctcct 7440

tggcgccctt cttcaccgag tcaatggtcg ggtccgactt gatatcggtg aagatcgagt 7500

acttgatgtc gatctcgtcc agcaccttgg tgatcttgtt cacgtagccc agcttgaaca 7560

ggtccttgtc ggtcacgatg aaggcgcgct tcttgttcat gtccttcagc tccttcaggg 7620

cgaagcgcag gcagccgtac ttgaagtaga tcttctgcgg caccttgaac cacagcatat 7680

tctcgcggcg ctcggccacc gacttgatgt tcagcaggtg cttcggctcc acgttctgcg 7740

acaccgagtt gccgccccag gtcccgcagc ccagggtgaa cgacggggcg atggcgaagt 7800

tgtacaggtc gcccgaggcg ccctgcgacg acggcatgtt gatgaaggtg cgcgaggtct 7860

tcatggccag gccgaactcc ttcaccttgt ccttgttgtt ctgcgagtcg atgtacagcg 7920

acgaggtgtg gcccgagccg cccagctcga tcaggcgctg ggccttcttg agggcctcat 7980

cgaagtcctt caccttgtac atggccagca ccggcgacag cttctcgtgc gagaacagct 8040

ccgacttctc caccgactgc acctcgccga tgaggatctt ggtggtctgc ggcacctcga 8100

tgccggccat cttggcgatg atgtaggccg acttgcccac gatgtcggcg ttgatggcgc 8160

cgttcttgaa catcgtctcc ttgatcttgg cgatctcgtt ctggttcagg atgtacgagc 8220

cgcgcttcac gaactcctcc ttcaccttct cgtagatgga gttcatcacc aggatcgact 8280

gctccgaggc gcagatcacg ccgttgtcgt aggtcttcga caggatgatc gacgacacgg 8340

ccatatcgat gtcggcggac tcgtcgatga tggccggggt gttgcccgcg cccacgccga 8400

tggccggctt gcccgacgag taggcggcct tcaccatcga cgggccgccg gtggccagga 8460

tgatatcggc ctccgacatc aggtcctgcg acagctcgat cgacggctcg tcaatccagc 8520

cgatgatgtt cttcgggccc ccggccttca cggcggcgtc caggatcagc ttggcggcgg 8580

cgatggtcga cttcttggcg cgcgggtgcg gcgagaagaa gatggcgttg cgggtcttca 8640

gcgagatcag cgacttgaag atggcggtcg aggtcgggtt cgtggtcggc acgatggcgg 8700

ccacgatgcc gatcggctcg gccaccttgg tgatgcccag cgagtcgtcg tggtcgataa 8760

tgccgcaggt cttctcgttc ttgtacttgt tgtagatgta ctcggcggcg aagtggttct 8820

tgatgatctt gtcctccacc aggccgatgc cggtctcctc cacggccagc ttggccaggt 8880

tgatgcgctc cttggcggcg gcaatggcgc actgcttgaa gatcttgtcc acctgctcct 8940

gggtgtaggt ggcgaacttc ttctgggcct cgcgcagctc gttcagcttc tgcttcagct 9000

ccttctggtt ggtcaccttc attcttgaat ctcctgaaag ccggtgctta cggcagcttg 9060

accacggctt ccccggtgac ggccagggcc ccgccttggg taaagatccg ggtcgtcagc 9120

gtggcgatcg gtttgtcctc gcgcagcgcg gtgacttcca cctcggcggt cacttcgtcc 9180

cccacgaaca ccggcagttt gaacgagagc gattggccca ggtagatgct ccccttgccg 9240

gggagctgct ggccgaggag gcccgagaac agcgaggcga gcagcatgcc atgcacgatg 9300

gggcgctcga acgcggtggt cgcggcaaac gcggggtcca ggtgcagcgg gttgaagtcc 9360

tcggagagcg ccgcaaaggc cgccacctcc gccgcgccaa accgcttgga cagccgggct 9420

ttttgcccga cctccagcga ctgggccgac atgcggcgtc ctcctctgtt tcagcccata 9480

tgcaggccgc cgttgagcga gaagtcggcg ccggtcgaga aaccggactc ctccgacgac 9540

aaccaggcgc agatcgaggc gatctcttcc ggcaggccca ggcgcttgac cgggatcgtc 9600

gcgacgatct tgtcgagcac gtcctggcgg atcgccttga ccatgtcggt ggcgatatag 9660

cccggagaga ccgtgttgac ggtcacgccc ttggtcgcca cttcctgcgc cagtgccatg 9720

gtgaagccat gcaggccggc cttggcggtg gagtagttgg tctggccgaa ctggcccttc 9780

tgcccgttca ccgacgagat gttgacgatg cggccccagc cacggtcggc catgccgtcg 9840

atcacctgct tggtgacgtt gaacagcgag gtcaggttgg tgtcgatcac cgcatcccag 9900

tcggcgcggg tcatcttgcg gaacaccacg tcgcgggtga taccggcgtt gttgatcagc 9960

acatcaacct cgccgacctc ggacttgacc ttgtcgaatg cggtcttggt cgagtcccag 10020

tcagccacat tgccttccga ggcaatgaaa tcgaagccca gggccttctg ctgctccagc 10080

cacttttcgc ggcgcggcga gttggggccg caaccggcca ccacacgaaa gccatccttg 10140

gccagccgct ggcaaatggc ggttccgata ccacccatgc cgccggtcac atacgcaatg 10200

cgctgagtca tgtccactcc ttgattggct tcgttatcgt cgccgggtcc gcgccaaccg 10260

cgcgcggccc cggaaaaccc cttccttatt tgcgctcgac tgccagcgcc acgcccatgc 10320

cgccgccgat gcacagcgag gccaggccct tcttcgcgtc acggcgcttc atctcgtgca 10380

gcagcgtcac caggatacgg cagcccgacg cgccgatcgg gtggccgatg gcgatggcgc 10440

cgccgttcac attgaccttg gaggtgtccc agcccatctg ctggtgcacc gccagcgcct 10500

gcgcggcaaa ggcctcgttg atctccatca ggtccaggtc ttgcggggtc cactcggcgc 10560

gcgacagggc gcgcttggag gccggcaccg ggcccatgcc catcaccttg ggatcgacac 10620

cggcgttggc atagctcttg atcgtggcca gcggggtcag gcccagttcc ttggccttgg 10680

ccgccgacat caccaccacc gcggcggcgc cgtcgttcag gcccgaggcg ttggccgcgg 10740

tcaccgtgcc ggccttgtcg aaggcgggct tgaggccgga catgctgtcc agcgtggcgc 10800

cctggcgcac gaactcgtcg gtcttgaagg ccaccgggtc gcccttgcgc tgcgggatca 10860

gcaccgggac gatctcttcg tcaaacttgc cggccttctg cgcggcttcg gccttgttct 10920

gcgagccgac ggcgaactca tcctgcgcct cgcgtgtgat gccgtattcc ttggccacgt 10980

tctcggcggt gatgcccatg tggtactggt tgtacacgtc ccacaggccg tcgacgatca 11040

tggtgtcgac cagcttggca tcgcccatgc ggaaaccatc gcgcgagccc ggcagcacgt 11100

gcggggcggc gctcatgttt tcctggccgc cggccaccac gatctcggcg tcgcccgcca 11160

tgatcgcgtt ggcggccagc atcacggcct tcaggcccga gccgcacacc ttgttgatgg 11220

tcatggccgg caccatcgcc ggcaggccgg ccttgatcgc ggcctggcgt gcggggttct 11280

ggcccgaacc ggcggtcagc acctggccca tgatgacttc gctcacctgc tccggcttga 11340

cgccggcgcg ctccagcgcg gccttgatga ccacggcacc cagttccggt gccgggatct 11400

tggccagcga gccgccaaac ttgccgaccg cggtgcgggc ggcggatacg atgacaacgt 11460

cagtcattgt gtagtccttt caatggaaag gtacccagct tttgttccct ttagtgaggg 11520

ttaattgcgc gcttggcgta atcatggtca tagctgtttc ctgtgtgaaa ttgttatccg 11580

ctcacaattc cacacaacat acgagccgga agcataaagt gtaaagcctg gggtgcctaa 11640

tgagtgagct aactcacatt aattgcgttg cgctcactgc ccgctttcca gtcgggaaac 11700

ctgtcgtgcc agctgcatta atgaatcggc caacgcgcgg ggagaggcgg tttgcgtatt 11760

gggcgcatgc ataaaaactg ttgtaattca ttaagcattc tgccgacatg gaagccatca 11820

caaacggcat gatgaacctg aatcgccagc ggcatcagca ccttgtcgcc ttgcgtataa 11880

tatttgccca tgggggtggg cgaagaactc cagcatgaga tccccgcgct ggaggatcat 11940

ccagccggcg tcccggaaaa cgattccgaa gcccaacctt tcatagaagg cggcggtgga 12000

atcgaaatct cgtgatggca ggttgggcgt cgcttggtcg gtcatttcga accccagagt 12060

cccgctcaga agaactcgtc aagaaggcga tagaaggcga tgcgctgcga atcgggagcg 12120

gcgataccgt aaagcacgag gaagcggtca gcccattcgc cgccaagctc ttcagcaata 12180

tcacgggtag ccaacgctat gtcctgatag cggtccgcca cacccagccg gccacagtcg 12240

atgaatccag aaaagcggcc attttccacc atgatattcg gcaagcaggc atcgccatgg 12300

gtcacgacga gatcctcgcc gtcgggcatg cgcgccttga gcctggcgaa cagttcggct 12360

ggcgcgagcc cctgatgctc ttcgtccaga tcatcctgat cgacaagacc ggcttccatc 12420

cgagtacgtg ctcgctcgat gcgatgtttc gcttggtggt cgaatgggca ggtagccgga 12480

tcaagcgtat gcagccgccg cattgcatca gccatgatgg atactttctc ggcaggagca 12540

aggtgagatg acaggagatc ctgccccggc acttcgccca atagcagcca gtcccttccc 12600

gcttcagtga caacgtcgag cacagctgcg caaggaacgc ccgtcgtggc cagccacgat 12660

agccgcgctg cctcgtcctg cagttcattc agggcaccgg acaggtcggt cttgacaaaa 12720

agaaccgggc gcccctgcgc tgacagccgg aacacggcgg catcagagca gccgattgtc 12780

tgttgtgccc agtcatagcc gaatagcctc tccacccaag cggccggaga acctgcgtgc 12840

aatccatctt gttcaatcat gcgaaacgat cctcatcctg tctcttgatc agatcttgat 12900

cccctgcgcc atcagatcct tggcggcaag aaagccatcc agtttacttt gcagggcttc 12960

ccaaccttac cagagggcgc cccagctggc aattccggtt cgcttgctgt ccataaaacc 13020

gcccagtcta gctatcgcca tgtaagccca ctgcaagcta cctgctttct ctttgcgctt 13080

gcgttttccc ttgtccagat agcccagtag ctgacattca tcccaggtgg cacttttcgg 13140

ggaaatgtgc gcgcccgcgt tcctgctggc gctgggcctg tttctggcgc tggacttccc 13200

gctgttccgt cagcagcttt tcgcccacgg ccttgatgat cgcggcggcc ttggcctgca 13260

tatcccgatt caacggcccc agggcgtcca gaacgggctt caggcgctcc cgaaggt 13317

<210> 67
<211> 10723
<212> DNA
<213> Artificial Sequence

<220>
<223> vector

<400> 67
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaattttct ctggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag gggccacttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt ggatccagca ggctgcctcg ataagcaaaa agggcggccc cgcggggccg 3300

ccctttttct ttccggcgac tgtcaggcca ctcagttgtt ggcggccttg acttgggcga 3360

tcagctcggg caccaccttg ttcacatcgc ccacaatggc caggtcggcc accttcatga 3420

tcggggcttc cacgtccttg ttgatcgcga tgatgtagtc cgagtcctgc atgccggcca 3480

ggtgctggat ggcgcccgag atgccgcagg cgatgtacag ggtcgggcgc acggtcttgc 3540

cggtctgccc cacttggagg tccttatcca cccactcctt ttcgatggcg gcgcgcgagg 3600

cggcgatggt gccgccgagg agggaggcca gctcctccag cttctcaaag ttctccttcg 3660

agcccacgcc gcggcccccg gccacgagca ccttggcctc gccgatatcg gcaatatcct 3720

tggcgagctt caccactttc gagaccttgg tgcggatgtc cgaggcggtc agcttgatgg 3780

ccactttctc gatcttgtcg tccgacacgt tggcgtcgtt caccggcagc ttctcgaaga 3840

acacgccggg ccgcaccgtg gccatctgcg ggcggtggtc cgagcacacg atggtggcga 3900

tcaggttgcc gccgaaggcc gggcgggtcg cgagcagatc gcggttctcc acgtcgatgt 3960

ccagcgaggt gcagtcggcg gtcaggccgg tcgacagccg ggcggcgatc cgcgggccga 4020

ggtcgcggcc gatgaacgtg gcgccgatga acaggatctc cggcttgcgc tcgttcacca 4080

ggtcacagat caccttggcg tagccgtcgg tgctgaaatg ggccagcagc tcgttgtcgg 4140

cggccaggac cttgtccgcg ccgtgcgaca gcaggtcctt cgacatcttc tcggtgttgt 4200

gccccagcag cacggccgtc agctccacgc ccagtttctc ggccatttcc ttgcccttcc 4260

ccagcagttc cagcgacacc ttctgcagct cgccgtcgcg ttgctcggcg aacacccaca 4320

cgcccttgta gtcggctttg ttcattgaaa aatccctcct aacttaaata tgtgttcttc 4380

ttttagttct gcgacagcat atcggccgcc tccttcacgg gcttatcgat cacttcgcct 4440

tggcccttga cttctttggt cgacgacttt ttcaccttgg tcggcgagcc cttcagcccc 4500

aggttggcct tgtcgacgtc gatgtcatcc gcggtccaca tcttgacctc cttgtcgaac 4560

gcgccgaaaa tcttttccac cgacatgtag cgcggcacgt tcagctcctt gatggccgtc 4620

agcaggaccg gggtcttcac ctccacgacc tcgtagccgt cctcccaggc cttgcggatt 4680

ttcagcgtat cgccatccac ctcgactttc tccacgtagg tcacctgggg gatgcccaga 4740

tgctcggcga tctccggccc gacctgggcc gtgtcgccgt caatggcctg gcgcccggca 4800

aacacgatat catatttcag cttcttgatg cccgcggcga tggtgtgcga ggtcgccagg 4860

gtatccgccc ccccgaaggc gcggtcggtc agcagcaccg cctcgtccgc ccccatggcc 4920

agggcctcca ccagggcgtt cttggcctgc gggggcccca tcgagatcac ggtcacatgg 4980

gccccgtagt tgtctttcag gaccagcgct tcctccagcg cattcttgtc gtccgggttg 5040

atgatcgacg gcacgccctc gcggatcagg gtccccttca ccggatcgat gcgcacctcg 5100

gcggtgtccg gcacctgctt cagacacacc acgatgttca tcctcttaac ctccttaaat 5160

tagcggaaaa tcttgccgct aatcaccagc ttctgcactt ccgacgtgcc ctcgtagatc 5220

tcggtaatct tggcgtcgcg catcatgcgc tcgaccgggt agtccttcgt gtagccgtag 5280

ccgccgaaca gttgcaccgc tttggtggtc acgtccatgg cgacgttggc ggcatgcagc 5340

ttggcgcgcg cggcgtccac ggtgtacggc aggcccgcct gcttcaggta ggcggcctta 5400

tagaccagat accgggcgga ctcaatggcc acgtccatat cggccatcat ccaggccagg 5460

ccctgaaact tgtccagcga gcggccgaac tgcttgcgct ccttcatgta ggcccgggct 5520

tcgttgaagg cgccctccgc gatccccagg gcttgggccg caatgccgat ccgcccccca 5580

tccagggttt tcatcgcaat cgggaacccc ttcccctctt tgccaatcat gttttccacc 5640

ggcacgatca tgtcctcgaa caccagttcg gtggtcgacg acgcgcggat gcccagcttt 5700

tgctccacct tgccgatcga gaagccctta aaccctttct caatgataaa ggccgagatg 5760

cctttggtgc ccttggtgcg gtcggtcatc gcgaagatca cgaacgtgtc cgccacgccg 5820

ccgttggtaa taaagatttt ggagccgttg atcacgtagt ggtcgccttc caggacggcc 5880

acggtctgct gcgcgcccga gtcggtgccg gcgttcggtt ccgtcaggcc gtacgcgccg 5940

atcttctccc ctttggccag cgggaccagg tacttctgtt tctgctcctc ggtcccgtgc 6000

tcgttgatca gcgaggcgca gagggaggtg tgggccgaca ggatcacccc ggtggtgccg 6060

cacaccttgg acagctcctc cacggcgatg atgtacgaca gcacgtcccc ccccgccccg 6120

ccgtactcct tcgagaacgg aatccccatc atgccatact ggcccatctt cttcacgttc 6180

tccatcggga agcgctcggt ctcgtcgatc tccgcggcaa tgggcttcac ctcgttttcc 6240

gcgaactcgc gcaccatctg gcgcaccagc tcctgctcgc gggtcaggtt gaagtccata 6300

taaacttacc tcctatctat gtgaaattgt tatccgctca caattccaca caacatacga 6360

gccggaagca taaagtgtaa agcctgggga agcttgggga gaacaatcag cccggcaggg 6420

gccgggctga ttgtgcctgc gtgccgccgg tgcttacggc agcttgacca cggcttcccc 6480

ggtgacggcc agggccccgc cttgggtaaa gatccgggtc gtcagcgtgg cgatcggttt 6540

gtcctcgcgc agcgcggtga cttccacctc ggcggtcact tcgtccccca cgaacaccgg 6600

cagtttgaac gagagcgatt ggcccaggta gatgctcccc ttgccgggga gctgctggcc 6660
gaggaggccc gagaacagcg aggcgagcag catgccatgc acgatggggc gctcgaacgc 6720
ggtggtcgcg gcaaacgcgg ggtccaggtg cagcgggttg aagtcctcgg agagcgccgc 6780
aaaggccgcc acctccgccg cgccaaaccg cttggacagc cgggcttttt gcccgacctc 6840
cagcgactgg gccgacatgc ggcgtcctcc tctgtttcag cccatatgca ggccgccgtt 6900
gagcgagaag tcggcgccgg tcgagaaacc ggactcctcc gacgacaacc aggcgcagat 6960
cgaggcgatc tcttccggca ggcccaggcg cttgaccggg atcgtcgcga cgatcttgtc 7020
gagcacgtcc tggcggatcg ccttgaccat gtcggtggcg atatagcccg gagagaccgt 7080
gttgacggtc acgcccttgg tcgccacttc ctgcgccagt gccatggtga agccatgcag 7140
gccggccttg gcggtggagt agttggtctg gccgaactgg cccttctgcc cgttcaccga 7200
cgagatgttg acgatgcggc cccagccacg gtcggccatg ccgtcgatca cctgcttggt 7260
gacgttgaac agcgaggtca ggttggtgtc gatcaccgca tcccagtcgg cgcgggtcat 7320
cttgcggaac accacgtcgc gggtgatacc ggcgttgttg atcagcacat caacctcgcc 7380
gacctcggac ttgaccttgt cgaatgcggt cttggtcgag tcccagtcag ccacattgcc 7440
ttccgaggca atgaaatcga agcccagggc cttctgctgc tccagccact tttcgcggcg 7500
cggcgagttg gggccgcaac cggccaccac acgaaagcca tccttggcca gccgctggca 7560
aatggcggtt ccgataccac ccatgccgcc ggtcacatac gcaatgcgct gagtcatgtc 7620
cactccttga ttggcttcgt tatcgtcgcc gggtccgcgc caaccgcgcg cggccccgga 7680
aaaccccttc cttatttgcg ctcgactgcc agcgccacgc ccatgccgcc gccgatgcac 7740
agcgaggcca ggcccttctt cgcgtcacgg cgcttcatct cgtgcagcag cgtcaccagg 7800
atacggcagc ccgacgcgcc gatcgggtgg ccgatggcga tggcgccgcc gttcacattg 7860
accttggagg tgtcccagcc catctgctgg tgcaccgcca gcgcctgcgc ggcaaaggcc 7920
tcgttgatct ccatcaggtc caggtcttgc ggggtccact cggcgcgcga cagggcgcgc 7980
ttggaggccg gcaccggccc catgcccatc accttgggat cgacaccggc gttggcatag 8040
ctcttgatcg tggccagcgg ggtcaggccc agttccttgg ccttggccgc cgacatcacc 8100
accaccgcgg cggcgccgtc gttcaggccc gaggcgttgg ccgcggtcac cgtgccggcc 8160
ttgtcgaagg cgggcttgag gccggacatg ctgtccagcg tggcgccctg gcgcacgaac 8220
tcgtcggtct tgaaggccac cgggtcgccc ttgcgctgcg ggatcagcac cgggacgatc 8280
tcttcgtcaa acttgccggc cttctgcgcg gcttcggcct tgttctgcga gccgacggcg 8340
aactcatcct gcgcctcgcg tgtgatgccg tattccttgg ccacgttctc ggcggtgatg 8400
cccatgtggt actggttgta cacgtcccac aggccgtcga cgatcatggt gtcgaccagc 8460
ttggcatcgc ccatgcggaa accatcgcgc gagcccggca gcacgtgcgg ggcggcgctc 8520

atgttttcct ggccgccggc caccacgatc tcggcgtcgc ccgccatgat cgcgttggcg 8580

gccagcatca cggccttcag gcccgagccg cacaccttgt tgatggtcat ggccggcacc 8640

atcgccggca ggccggcctt gatcgcggcc tggcgtgcgg ggttctggcc cgaaccggcg 8700

gtcagcacct ggcccatgat gacttcgctc acctgctccg gcttgacgcc ggcgcgctcc 8760

agcgcggcct tgatgaccac ggcacccagt tccggtgccg ggatcttggc cagcgagccg 8820

ccaaacttgc cgaccgcggt gcgggcggcg gatacgatga caacgtcagt cattgtgtag 8880

tcctttcaat ggaaaggtac ccagcttttg ttccctttag tgagggttaa ttgcgcgctt 8940

ggcgtaatca tggtcatagc tgtttcctgt gtgaaattgt tatccgctca caattccaca 9000

caacatacga gccggaagca taaagtgtaa agcctggggt gcctaatgag tgagctaact 9060

cacattaatt gcgttgcgct cactgcccgc tttccagtcg ggaaacctgt cgtgccagct 9120

gcattaatga atcggccaac gcgcggggag aggcggtttg cgtattgggc gcatgcataa 9180

aaactgttgt aattcattaa gcattctgcc gacatggaag ccatcacaaa cggcatgatg 9240

aacctgaatc gccagcggca tcagcacctt gtcgccttgc gtataatatt tgcccatggg 9300

ggtgggcgaa gaactccagc atgagatccc cgcgctggag gatcatccag ccggcgtccc 9360

ggaaaacgat tccgaagccc aacctttcat agaaggcggc ggtggaatcg aaatctcgtg 9420

atggcaggtt gggcgtcgct tggtcggtca tttcgaaccc cagagtcccg ctcagaagaa 9480

ctcgtcaaga aggcgataga aggcgatgcg ctgcgaatcg ggagcggcga taccgtaaag 9540

cacgaggaag cggtcagccc attcgccgcc aagctcttca gcaatatcac gggtagccaa 9600

cgctatgtcc tgatagcggt ccgccacacc cagccggcca cagtcgatga atccagaaaa 9660

gcggccattt tccaccatga tattcggcaa gcaggcatcg ccatgggtca cgacgagatc 9720

ctcgccgtcg ggcatgcgcg ccttgagcct ggcgaacagt tcggctggcg cgagcccctg 9780

atgctcttcg tccagatcat cctgatcgac aagaccggct tccatccgag tacgtgctcg 9840

ctcgatgcga tgtttcgctt ggtggtcgaa tgggcaggta gccggatcaa gcgtatgcag 9900

ccgccgcatt gcatcagcca tgatggatac tttctcggca ggagcaaggt gagatgacag 9960

gagatcctgc cccggcactt cgcccaatag cagccagtcc cttcccgctt cagtgacaac 10020

gtcgagcaca gctgcgcaag gaacgcccgt cgtggccagc cacgatagcc gcgctgcctc 10080

gtcctgcagt tcattcaggg caccggacag gtcggtcttg acaaaaagaa ccgggcgccc 10140

ctgcgctgac agccggaaca cggcggcatc agagcagccg attgtctgtt gtgcccagtc 10200

atagccgaat agcctctcca cccaagcggc cggagaacct gcgtgcaatc catcttgttc 10260

aatcatgcga aacgatcctc atcctgtctc ttgatcagat cttgatcccc tgcgccatca 10320

gatccttggc ggcaagaaag ccatccagtt tactttgcag ggcttcccaa ccttaccaga 10380

gggcgcccca gctggcaatt ccggttcgct tgctgtccat aaaaccgccc agtctagcta 10440

tcgccatgta agcccactgc aagctacctg ctttctcttt gcgcttgcgt tttcccttgt 10500

ccagatagcc cagtagctga cattcatccc aggtggcact tttcggggaa atgtgcgcgc 10560

ccgcgttcct gctggcgctg ggcctgtttc tggcgctgga cttcccgctg ttccgtcagc 10620

agcttttcgc ccacggcctt gatgatcgcg gcggccttgg cctgcatatc ccgattcaac 10680

ggccccaggg cgtccagaac gggcttcagg cgctcccgaa ggt 10723

<210> 68
<211> 153
<212> DNA
<213> Ralstonia eutropha

<400> 68
aacccgcatc acacccgcgt cttgaaatgc ccctaccccg tccctataat tagcactcgt 60

caggggtgag tgctaacagc ctcctgcgac acctgaacat ttctgacggc cgtcgcctcg 120

gtagccggcc gatttgtgat caaaaactca ctt 153

<210> 69
<211> 422
<212> PRT
<213> Jeotgalicoccus sp. ATCC 8456

<400> 69

Met Ala Thr Leu Lys Arg Asp Lys Gly Leu Asp Asn Thr Leu Lys Val
1               5                   10                  15

Leu Lys Gln Gly Tyr Leu Tyr Thr Thr Asn Gln Arg Asn Arg Leu Asn
            20                  25                  30

Thr Ser Val Phe Gln Thr Lys Ala Leu Gly Gly Lys Pro Phe Val Val
        35                  40                  45

Val Thr Gly Lys Glu Gly Ala Glu Met Phe Tyr Asn Asn Asp Val Val
    50                  55                  60

Gln Arg Glu Gly Met Leu Pro Lys Arg Ile Val Asn Thr Leu Phe Gly
65                  70                  75                  80

Lys Gly Ala Ile His Thr Val Asp Gly Lys Lys His Val Asp Arg Lys
                85                  90                  95

Ala Leu Phe Met Ser Leu Met Thr Glu Gly Asn Leu Asn Tyr Val Arg
            100                 105                 110

Glu Leu Thr Arg Thr Leu Trp His Ala Asn Thr Gln Arg Met Glu Ser
        115                 120                 125

Met Asp Glu Val Asn Ile Tyr Arg Glu Ser Ile Val Leu Leu Thr Lys
    130                 135                 140

Val Gly Thr Arg Trp Ala Gly Val Gln Ala Pro Pro Glu Asp Ile Glu
145                 150                 155                 160

Arg Ile Ala Thr Asp Met Asp Ile Met Ile Asp Ser Phe Arg Ala Leu
                165                 170                 175

Gly Gly Ala Phe Lys Gly Tyr Lys Ala Ser Lys Glu Ala Arg Arg Arg
            180                 185                 190

Val Glu Asp Trp Leu Glu Glu Gln Ile Ile Glu Thr Arg Lys Gly Asn
        195                 200                 205

Ile His Pro Pro Glu Gly Thr Ala Leu Tyr Glu Phe Ala His Trp Glu
    210                 215                 220

Asp Tyr Leu Gly Asn Pro Met Asp Ser Arg Thr Cys Ala Ile Asp Leu
225                 230                 235                 240

Met Asn Thr Phe Arg Pro Leu Ile Ala Ile Asn Arg Phe Val Ser Phe
                245                 250                 255

Gly Leu His Ala Met Asn Glu Asn Pro Ile Thr Arg Glu Lys Ile Lys
            260                 265                 270

Ser Glu Pro Asp Tyr Ala Tyr Lys Phe Ala Gln Glu Val Arg Arg Tyr
        275                 280                 285

Tyr Pro Phe Val Pro Phe Leu Pro Gly Lys Ala Lys Val Asp Ile Asp
    290                 295                 300

Phe Gln Gly Val Thr Ile Pro Ala Gly Val Gly Leu Ala Leu Asp Val
305                 310                 315                 320

Tyr Gly Thr Thr His Asp Glu Ser Leu Trp Asp Asp Pro Asn Glu Phe
                325                 330                 335

Arg Pro Glu Arg Phe Glu Thr Trp Asp Gly Ser Pro Phe Asp Leu Ile
            340                 345                 350

Pro Gln Gly Gly Gly Asp Tyr Trp Thr Asn His Arg Cys Ala Gly Glu
        355                 360                 365

Trp Ile Thr Val Ile Ile Met Glu Glu Thr Met Lys Tyr Phe Ala Glu

370                 375                 380

Lys Ile Thr Tyr Asp Val Pro Glu Gln Asp Leu Glu Val Asp Leu Asn
385                 390                 395                 400

Ser Ile Pro Gly Tyr Val Lys Ser Gly Phe Val Ile Lys Asn Val Arg
                405                 410                 415

Glu Val Val Asp Arg Thr
            420

<210>  70
<211>  301
<212>  PRT
<213>  Clostridium acetobutylicum

<400>  70

Met Ile Lys Ser Phe Asn Glu Ile Ile Met Lys Val Lys Ser Lys Glu
1               5                   10                  15

Met Lys Lys Val Ala Val Ala Val Ala Gln Asp Glu Pro Val Leu Glu
            20                  25                  30

Ala Val Arg Asp Ala Lys Lys Asn Gly Ile Ala Asp Ala Ile Leu Val
        35                  40                  45

Gly Asp His Asp Glu Ile Val Ser Ile Ala Leu Lys Ile Gly Met Asp
    50                  55                  60

Val Asn Asp Phe Glu Ile Val Asn Glu Pro Asn Val Lys Lys Ala Ala
65                  70                  75                  80

Leu Lys Ala Val Glu Leu Val Ser Thr Gly Lys Ala Asp Met Val Met
                85                  90                  95

Lys Gly Leu Val Asn Thr Ala Thr Phe Leu Arg Ser Val Leu Asn Lys
            100                 105                 110

Glu Val Gly Leu Arg Thr Gly Lys Thr Met Ser His Val Ala Val Phe
        115                 120                 125

Glu Thr Glu Lys Phe Asp Arg Leu Leu Phe Leu Thr Asp Val Ala Phe
    130                 135                 140

Asn Thr Tyr Pro Glu Leu Lys Glu Lys Ile Asp Ile Val Asn Asn Ser
145                 150                 155                 160

Val Lys Val Ala His Ala Ile Gly Ile Glu Asn Pro Lys Val Ala Pro

                165                 170                 175

Ile Cys Ala Val Glu Val Ile Asn Pro Lys Met Pro Ser Thr Leu Asp
            180                 185                 190

Ala Ala Met Leu Ser Lys Met Ser Asp Arg Gly Gln Ile Lys Gly Cys
        195                 200                 205

Val Val Asp Gly Pro Leu Ala Leu Asp Ile Ala Leu Ser Glu Glu Ala
    210                 215                 220

Ala His His Lys Gly Val Thr Gly Glu Val Ala Gly Lys Ala Asp Ile
225                 230                 235                 240

Phe Leu Met Pro Asn Ile Glu Thr Gly Asn Val Met Tyr Lys Thr Leu
                245                 250                 255

Thr Tyr Thr Thr Asp Ser Lys Asn Gly Gly Ile Leu Val Gly Thr Ser
            260                 265                 270

Ala Pro Val Val Leu Thr Ser Arg Ala Asp Ser His Glu Thr Lys Met
        275                 280                 285

Asn Ser Ile Ala Leu Ala Ala Leu Val Ala Gly Asn Lys
    290                 295                 300

<210> 71
<211> 355
<212> PRT
<213> Clostridium acetobutylicum

<400> 71

Met Tyr Arg Leu Leu Ile Ile Asn Pro Gly Ser Thr Ser Thr Lys Ile
1               5                   10                  15

Gly Ile Tyr Asp Asp Glu Lys Glu Ile Phe Glu Lys Thr Leu Arg His
            20                  25                  30

Ser Ala Glu Glu Ile Glu Lys Tyr Asn Thr Ile Phe Asp Gln Phe Gln
        35                  40                  45

Phe Arg Lys Asn Val Ile Leu Asp Ala Leu Lys Glu Ala Asn Ile Glu
    50                  55                  60

Val Ser Ser Leu Asn Ala Val Val Gly Arg Gly Gly Leu Leu Lys Pro
65                  70                  75                  80

Ile Val Ser Gly Thr Tyr Ala Val Asn Gln Lys Met Leu Glu Asp Leu

                85                  90                  95

Lys Val Gly Val Gln Gly Gln His Ala Ser Asn Leu Gly Gly Ile Ile
            100                 105                 110

Ala Asn Glu Ile Ala Lys Glu Ile Asn Val Pro Ala Tyr Ile Val Asp
        115                 120                 125

Pro Val Val Val Asp Glu Leu Asp Glu Val Ser Arg Ile Ser Gly Met
    130                 135                 140

Ala Asp Ile Pro Arg Lys Ser Ile Phe His Ala Leu Asn Gln Lys Ala
145                 150                 155                 160

Val Ala Arg Arg Tyr Ala Lys Glu Val Gly Lys Lys Tyr Glu Asp Leu
                165                 170                 175

Asn Leu Ile Val Val His Met Gly Gly Gly Thr Ser Val Gly Thr His
            180                 185                 190

Lys Asp Gly Arg Val Ile Glu Val Asn Asn Thr Leu Asp Gly Glu Gly
        195                 200                 205

Pro Phe Ser Pro Glu Arg Ser Gly Gly Val Pro Ile Gly Asp Leu Val
    210                 215                 220

Arg Leu Cys Phe Ser Asn Lys Tyr Thr Tyr Glu Glu Val Met Lys Lys
225                 230                 235                 240

Ile Asn Gly Lys Gly Gly Val Val Ser Tyr Leu Asn Thr Ile Asp Phe
                245                 250                 255

Lys Ala Val Val Asp Lys Ala Leu Glu Gly Asp Lys Lys Cys Ala Leu
            260                 265                 270

Ile Tyr Glu Ala Phe Thr Phe Gln Val Ala Lys Glu Ile Gly Lys Cys
        275                 280                 285

Ser Thr Val Leu Lys Gly Asn Val Asp Ala Ile Ile Leu Thr Gly Gly
    290                 295                 300

Ile Ala Tyr Asn Glu His Val Cys Asn Ala Ile Glu Asp Arg Val Lys
305                 310                 315                 320

Phe Ile Ala Pro Val Val Arg Tyr Gly Gly Glu Asp Glu Leu Leu Ala
                325                 330                 335

Leu Ala Glu Gly Gly Leu Arg Val Leu Arg Gly Glu Glu Lys Ala Lys
            340                 345                 350

Glu Tyr Lys
        355

<210>  72
<211>  94
<212>  DNA
<213>  Clostridium acetobutylicum

<400>  72
taaagtcata aataatataa tataaccagt acccatgttt ataaaacttt tgccctataa      60

acatgggtat tgtttttttt ttattttttt ctga                                  94

<210>  73
<211>  3563
<212>  DNA
<213>  Artificial Sequence

<220>
<223>  synthetic gen

<400>  73
ggatccaacc cgcatcacac ccgcgtcttg aaatgcccct accccgtccc tataattagc      60

actcgtcagg ggtgagtgct aacagcctcc tgcgacacct gaacatttct gacggccgtc     120

gcctcggtag ccggccgatt tgtgatcaaa aactcacttt ttcaggagat tcaagaatgg     180

ccaccctgaa gcgcgataag ggcctcgata acacgctgaa ggtgctgaag cagggctatc     240

tgtacacgac caatcagcgc aaccgcctca acacctcggt gttccagacc aaagcgctcg     300

gggggaagcc cttcgtcgtg gtgaccggga aggagggcgc cgagatgttc tacaacaacg     360

acgtggtgca acgcgaaggc atgctgccca agcgcattgt gaataccctg ttcggcaaag     420

gcgccatcca taccgtggac ggcaagaagc acgtggaccg caaggcgctc ttcatgtcgc     480

tgatgaccga aggcaacctc aactacgtgc gggagctgac ccgcaccctg tggcacgcca     540

acacgcagcg catggagtcg atggatgagg tgaacatcta ccgcgaatcg atcgtgctcc     600

tgaccaaggt gggcacccgc tgggccgggg tccaggcgcc cccggaggac attgagcgca     660

tcgcgaccga catggatatt atgatcgaca gctttcgcgc cctggggggg gcgtttaagg     720

gctacaaagc gtcgaaggag gcgcggcggc gcgtggagga ttggctcgag gagcagatca     780

tcgagacgcg caagggcaac atccacccgc ccgagggcac cgccctgtat gagtttgccc     840

actgggagga ctacctgggg aacccgatgg actcgcggac gtgcgcgatt gacctgatga     900

acacctttcg gccgctgatc gccatcaacc gcttcgtgtc cttcgggctg cacgcgatga     960

acgagaatcc catcacccgc gagaagatca agtcggagcc ggactacgcc tacaagttcg    1020

cgcaggaggt ccgccgctac tacccgttcg tgccgttcct cccggggaag gccaaagtgg    1080

atatcgactt ccaaggcgtg accattcccg ccggcgtggg gctggccctc gacgtgtatg 1140

gcaccaccca tgacgagagc ctgtgggacg acccgaacga atttcgcccg gagcggttcg 1200

agacctggga cggctcgccg ttcgacctca tcccgcaggg cgggggcgac tattggacca 1260

accaccgctg cgccggggag tggatcaccg tgattatcat ggaggagacc atgaagtatt 1320

ttgcggaaaa gatcacgtac gacgtcccgg agcaggacct ggaggtggat ctgaattcga 1380

ttccgggcta tgtgaagtcg gggttcgtga tcaaaaacgt gcgcgaggtg gtcgatcgca 1440

cctaatttca ggagattcaa gaatgatcaa gtcgttcaac gaaatcatca tgaaagtgaa 1500

atcgaaggag atgaagaaag tcgccgtcgc ggtcgcccag gacgaaccgg tgctggaggc 1560

cgtgcgggac gcgaagaaga acgggatcgc cgacgccatc ctggtgggcg atcacgatga 1620

aatcgtgtcc atcgccctga agattggcat ggatgtgaac gattttgaaa ttgtgaatga 1680

gcccaatgtg aaaaaggcgg cgctgaaggc ggtggaactg gtgtcgaccg gcaaggccga 1740

catggtgatg aaggggctgg tgaataccgc cacgtttctg cgctcggtgc tcaacaagga 1800

agtgggcctg cgcacgggga aaacgatgtc gcatgtggcc gtctttgaga ccgagaagtt 1860

tgaccgcctc ctgtttctca cggacgtcgc ctttaacacc tacccggagc tgaaagagaa 1920

gatcgacatt gtcaacaatt cggtgaaggt ggcccacgcg atcggcatcg aaaacccgaa 1980

agtggccccg atctgcgccg tggaggtgat caaccccaaa atgccgtcga ccctggatgc 2040

cgccatgctg agcaagatgt cggatcgcgg ccagatcaag ggctgcgtgg tggatggccc 2100

cctggccctg gacattgccc tgtccgagga agccgcgcac cataagggcg tgacggggga 2160

ggtggcgggc aaagcggata tcttcctcat gcccaacatc gagacgggca acgtgatgta 2220

taaaaccctc acctatacga cggacagcaa gaatgggggc atcctcgtgg gcaccagcgc 2280

cccggtcgtg ctgacctcgc gggcggattc gcacgagacc aagatgaatt cgattgcgct 2340

ggcggccctc gtcgccggga acaagtaaat taaagttaag tggaggaatg ttaacatgta 2400

ccgcctcctc atcatcaacc ccgggagcac ctccacgaag atcggcatct atgacgacga 2460

gaaagagatc tttgagaaaa cgctgcgcca ctcggcggag gagatcgaga agtataatac 2520

cattttcgac cagttccaat tccgcaagaa cgtcatcctg gacgcgctca aggaggccaa 2580

catcgaagtg tcgtcgctga acgccgtcgt ggggcgcggg ggcctgctga agcccatcgt 2640

gtcgggcacg tacgccgtga accaaaaaat gctggaagac ctgaaggtgg gggtgcaggg 2700

ccagcatgcc tcgaacctgg gcgggattat cgcgaacgag attgccaagg aaattaacgt 2760

gccggcctac atcgtggacc cggtggtcgt cgacgaactg gacgaggtgt cgcgcatctc 2820

gggcatggcg gacatcccgc gcaagtccat ttttcacgcc ctcaaccaga aggccgtggc 2880

ccggcgctac gccaaagaag tggggaaaaa gtacgaggat ctgaacctga tcgtggtgca 2940

catgggcggc gggacctcgg tggggaccca caaggacggc cgcgtgatcg aggtgaacaa 3000

caccctggat ggcgagggcc cgttttcgcc ggaacgctcc ggcggcgtgc ccatcggcga 3060

cctcgtgcgc ctgtgctttt cgaacaagta cacctacgaa gaggtgatga agaagatcaa 3120

tgggaagggc ggggtggtgt cgtacctgaa taccatcgac ttcaaggcgg tcgtggacaa 3180

agccctggag ggcgataaaa agtgcgccct gatttatgag gccttcacct tccaggtggc 3240

gaaagaaatt ggcaagtgct cgaccgtgct gaaggggaac gtggacgcca tcatcctcac 3300

cggcggcatc gcctacaatg agcacgtgtg caacgccatt gaagaccggg tgaagttcat 3360

cgcgcccgtg gtgcggtacg gcggggaaga cgagctgctc gccctggccg aaggcggcct 3420

gcgcgtgctc cggggcgagg aaaaggcgaa ggagtacaag taataaagtc ataaataata 3480

taatataacc agtacccatg tttataaaac ttttgcccta taaacatggg tattgttttt 3540

tttttatttt tttctgagag ctc 3563

<210> 74
<211> 14861
<212> DNA
<213> Artificial Sequence

<220>
<223> vector

<400> 74
   ctcgggccgt ctcttgggct tgatcggcct tcttgcgcat ctcacgcgct cctgcggcgg 60
   cctgtagggc aggctcatac ccctgccgaa ccgcttttgt cagccggtcg gccacggctt 120
   ccggcgtctc aacgcgcttt gagattccca gcttttcggc caatccctgc ggtgcatagg 180
   cgcgtggctc gaccgcttgc gggctgatgg tgacgtggcc cactggtggc cgctccaggg 240
   cctcgtagaa cgcctgaatg cgcgtgtgac gtgccttgct gccctcgatg ccccgttgca 300
   gccctagatc ggccacagcg gccgcaaacg tggtctggtc gcgggtcatc tgcgctttgt 360
   tgccgatgaa ctccttggcc gacagcctgc cgtcctgcgt cagcggcacc acgaacgcgg 420
   tcatgtgcgg gctggtttcg tcacggtgga tgctggccgt cacgatgcga tccgccccgt 480
   acttgtccgc cagccacttg tgcgccttct cgaagaacgc cgcctgctgt tcttggctgg 540
   ccgacttcca ccattccggg ctggccgtca tgacgtactc gaccgccaac acagcgtcct 600
   tgcgccgctt ctctggcagc aactcgcgca gtcggcccat cgcttcatcg gtgctgctgg 660
   ccgcccagtg ctcgttctct ggcgtcctgc tggcgtcagc gttgggcgtc tcgcgctcgc 720
   ggtaggcgtg cttgagactg gccgccacgt tgcccatttt cgccagcttc ttgcatcgca 780
   tgatcgcgta tgccgccatg cctgcccctc ccttttggtg tccaaccggc tcgacggggg 840
   cagcgcaagg cggtgcctcc ggcgggccac tcaatgcttg agtatactca ctagactttg 900
   cttcgcaaag tcgtgaccgc ctacggcggc tgcggcgccc tacgggcttg ctctccgggc 960
   ttcgccctgc gcggtcgctg cgctcccttg ccagcccgtg gatatgtgga cgatggccgc 1020
   gagcggccac cggctggctc gcttcgctcg gcccgtggac aaccctgctg gacaagctga 1080
   tggacaggct gcgcctgccc acgagcttga ccacagggat tgcccaccgg ctacccagcc 1140
   ttcgaccaca tacccaccgg ctccaactgc gcggcctgcg gccttgcccc atcaattttt 1200
   ttaattttct ctggggaaaa gcctccggcc tgcggcctgc gcgcttcgct tgccggttgg 1260
   acaccaagtg gaaggcgggt caaggctcgc gcagcgaccg cgcagcggct tggccttgac 1320
   gcgcctggaa cgacccaagc ctatgcgagt gggggcagtc gaaggcgaag cccgcccgcc 1380
   tgccccccga gcctcacggc ggcgagtgcg ggggttccaa gggggcagcg ccaccttggg 1440
   caaggccgaa ggccgcgcag tcgatcaaca agccccggag gggccacttt ttgccggagg 1500
   gggagccgcg ccgaaggcgt gggggaaccc cgcaggggtg cccttctttg ggcaccaaag 1560
   aactagatat agggcgaaat gcgaaagact taaaaatcaa caacttaaaa aaggggggta 1620
   cgcaacagct cattgcggca ccccccgcaa tagctcattg cgtaggttaa agaaaatctg 1680
   taattgactg ccacttttac gcaacgcata attgttgtcg cgctgccgaa aagttgcagc 1740
   tgattgcgca tggtgccgca accgtgcggc accctaccgc atggagataa gcatggccac 1800
   gcagtccaga gaaatcggca ttcaagccaa gaacaagccc ggtcactggg tgcaaacgga 1860
   acgcaaagcg catgaggcgt gggccgggct tattgcgagg aaacccacgg cggcaatgct 1920
   gctgcatcac ctcgtggcgc agatgggcca ccagaacgcc gtggtggtca gccagaagac 1980
   actttccaag ctcatcggac gttctttgcg gacggtccaa tacgcagtca aggacttggt 2040
   ggccgagcgc tggatctccg tcgtgaagct caacggcccc ggcaccgtgt cggcctacgt 2100
   ggtcaatgac cgcgtggcgt ggggccagcc ccgcgaccag ttgcgcctgt cggtgttcag 2160
   tgccgccgtg gtggttgatc acgacgacca ggacgaatcg ctgttggggc atggcgacct 2220
   gcgccgcatc ccgaccctgt atccgggcga gcagcaacta ccgaccggcc ccggcgagga 2280
   gccgcccagc cagcccggca ttccgggcat ggaaccagac ctgccagcct tgaccgaaac 2340
   ggaggaatgg gaacggcgcg ggcagcagcg cctgccgatg cccgatgagc cgtgttttct 2400
   ggacgatggc gagccgttgg agccgccgac acgggtcacg ctgccgcgcc ggtagcactt 2460
   gggttgcgca gcaacccgta agtgcgctgt tccagactat cggctgtagc cgcctcgccg 2520
   ccctatacct tgtctgcctc cccgcgttgc gtcgcggtgc atggagccgg gccacctcga 2580
   cctgaatgga agccggcggc acctcgctaa cggattcacc gtttttatca ggctctggga 2640
   ggcagaataa atgatcatat cgtcaattat tacctccacg gggagagcct gagcaaactg 2700
   gcctcaggca tttgagaagc acacggtcac actgcttccg gtagtcaata aaccggtaaa 2760
   ccagcaatag acataagcgg ctatttaacg accctgccct gaaccgacga ccgggtcgaa 2820
   tttgctttcg aatttctgcc attcatccgc ttattatcac ttattcaggc gtagcaccag 2880
   gcgtttaagg gcaccaataa ctgccttaaa aaaattacgc cccgccctgc cactcatcgc 2940
   agtcggccta ttggttaaaa aatgagctga tttaacaaaa atttaacgcg aattttaaca 3000
   aaatattaac gcttacaatt tccattcgcc attcaggctg cgcaactgtt gggaagggcg 3060
   atcggtgcgg gcctcttcgc tattacgcca gctggcgaaa gggggatgtg ctgcaaggcg 3120
   attaagttgg gtaacgccag ggttttccca gtcacgacgt tgtaaaacga cggccagtga 3180
   gcgcgcgtaa tacgactcac tatagggcga attggagctc tcagaaaaaa ataaaaaaaa 3240
   aacaataccc atgtttatag ggcaaaagtt ttataaacat gggtactggt tatattatat 3300
   tatttatgac tttattactt gtactccttc gccttttcct cgccccggag cacgcgcagg 3360
   ccgccttcgg ccagggcgag cagctcgtct tccccgccgt accgcaccac gggcgcgatg 3420
   aacttcaccc ggtcttcaat ggcgttgcac acgtgctcat tgtaggcgat gccgccggtg 3480
   aggatgatgg cgtccacgtt ccccttcagc acggtcgagc acttgccaat ttctttcgcc 3540
   acctggaagg tgaaggcctc ataaatcagg gcgcactttt tatcgccctc cagggctttg 3600
   tccacgaccg ccttgaagtc gatggtattc aggtacgaca ccaccccgcc cttcccattg 3660
   atcttcttca tcacctcttc gtaggtgtac ttgttcgaaa agcacaggcg cacgaggtcg 3720
   ccgatgggca cgccgccgga gcgttccggc gaaaacgggc cctcgccatc cagggtgttg 3780
   ttcacctcga tcacgcggcc gtccttgtgg gtccccaccg aggtcccgcc gcccatgtgc 3840
   accacgatca ggttcagatc ctcgtacttt ttccccactt ctttggcgta gcgccgggcc 3900
   acggccttct ggttgagggc gtgaaaaatg gacttgcgcg ggatgtccgc catgcccgag 3960
   atgcgcgaca cctcgtccag ttcgtcgacg accaccgggt ccacgatgta ggccggcacg 4020
   ttaatttcct tggcaatctc gttcgcgata atcccgccca ggttcgaggc atgctggccc 4080
   tgcaccccca ccttcaggtc ttccagcatt ttttggttca cggcgtacgt gcccgacacg 4140
   atgggcttca gcaggccccc gcgccccacg acggcgttca gcgacgacac ttcgatgttg 4200
   gcctccttga gcgcgtccag gatgacgttc ttgcggaatt ggaactggtc gaaaatggta 4260
   ttatacttct cgatctcctc cgccgagtgg cgcagcgttt tctcaaagat ctctttctcg 4320
   tcgtcataga tgccgatctt cgtggaggtg ctcccggggt tgatgatgag gaggcggtac 4380
   atgttaacat tcctccactt aactttaatt tacttgttcc cggcgacgag ggccgccagc 4440
   gcaatcgaat tcatcttggt ctcgtgcgaa tccgcccgcg aggtcagcac gaccggggcg 4500
   ctggtgccca cgaggatgcc cccattcttg ctgtccgtcg tataggtgag ggttttatac 4560
   atcacgttgc ccgtctcgat gttgggcatg aggaagatat ccgctttgcc cgccacctcc 4620
   cccgtcacgc ccttatggtg cgcggcttcc tcggacaggg caatgtccag ggccaggggg 4680
   ccatccacca cgcagccctt gatctggccg cgatccgaca tcttgctcag catggcggca 4740
   tccagggtcg acggcatttt ggggttgatc acctccacgg cgcagatcgg ggccactttc 4800
   gggttttcga tgccgatcgc gtgggccacc ttcaccgaat tgttgacaat gtcgatcttc 4860
   tctttcagct ccgggtaggt gttaaaggcg acgtccgtga gaaacaggag gcggtcaaac 4920
   ttctcggtct caaagacggc cacatgcgac atcgttttcc ccgtgcgcag gcccacttcc 4980
   ttgttgagca ccgagcgcag aaacgtggcg gtattcacca gccccttcat caccatgtcg 5040
   gccttgccgg tcgacaccag ttccaccgcc ttcagcgccg cctttttcac attgggctca 5100
   ttcacaattt caaaatcgtt cacatccatg ccaatcttca gggcgatgga cacgatttca 5160
   tcgtgatcgc ccaccaggat ggcgtcggcg atcccgttct tcttcgcgtc ccgcacggcc 5220
   tccagcaccg gttcgtcctg ggcgaccgcg acggcgactt tcttcatctc cttcgatttc 5280
   actttcatga tgatttcgtt gaacgacttg atcattcttg aatctcctga aattaggtgc 5340
   gatcgaccac ctcgcgcacg tttttgatca cgaaccccga cttcacatag cccggaatcg 5400
   aattcagatc cacctccagg tcctgctccg ggacgtcgta cgtgatcttt tccgcaaaat 5460
   acttcatggt ctcctccatg ataatcacgg tgatccactc cccggcgcag cggtggttgg 5520
   tccaatagtc gcccccgccc tgcgggatga ggtcgaacgg cgagccgtcc caggtctcga 5580
   accgctccgg gcgaaattcg ttcgggtcgt cccacaggct ctcgtcatgg gtggtgccat 5640
   acacgtcgag ggccagcccc acgccggcgg gaatggtcac gccttggaag tcgatatcca 5700
   ctttggcctt ccccgggagg aacggcacga acgggtagta gcggcggacc tcctgcgcga 5760
   acttgtaggc gtagtccggc tccgacttga tcttctcgcg ggtgatggga ttctcgttca 5820
   tcgcgtgcag cccgaaggac acgaagcggt tgatggcgat cagcggccga aaggtgttca 5880
   tcaggtcaat cgcgcacgtc cgcgagtcca tcgggttccc caggtagtcc tcccagtggg 5940
   caaactcata cagggcggtg ccctcgggcg ggtggatgtt gcccttgcgc gtctcgatga 6000
   tctgctcctc gagccaatcc tccacgcgcc gccgcgcctc cttcgacgct ttgtagccct 6060
   taaacgcccc ccccagggcg cgaaagctgt cgatcataat atccatgtcg gtcgcgatgc 6120
   gctcaatgtc ctccgggggc gcctggaccc cggcccagcg ggtgcccacc ttggtcagga 6180
   gcacgatcga ttcgcggtag atgttcacct catccatcga ctccatgcgc tgcgtgttgg 6240
   cgtgccacag ggtgcgggtc agctcccgca cgtagttgag gttgccttcg gtcatcagcg 6300
   acatgaagag cgccttgcgg tccacgtgct tcttgccgtc cacggtatgg atggcgcctt 6360
   tgccgaacag ggtattcaca atgcgcttgg gcagcatgcc ttcgcgttgc accacgtcgt 6420
   tgttgtagaa catctcggcg ccctccttcc cggtcaccac gacgaagggc ttccccccga 6480
   gcgctttggt ctggaacacc gaggtgttga ggcggttgcg ctgattggtc gtgtacagat 6540
   agccctgctt cagcaccttc agcgtgttat cgaggccctt atcgcgcttc agggtggcca 6600
   ttcttgaatc tcctgaaaaa gtgagttttt gatcacaaat cggccggcta ccgaggcgac 6660
   ggccgtcaga aatgttcagg tgtcgcagga ggctgttagc actcacccct gacgagtgct 6720
   aattataggg acggggtagg ggcatttcaa gacgcgggtg tgatgcgggt tggatccagc 6780
   aggctgcctc gataagcaaa aagggcggcc ccgcggggcc gccctttttc tttccggcga 6840
   ctgtcaggcc actcagaact gctcggcggt catggcgttc accggggcgg cgcccgacac 6900
   gatggcgtcg cgcagggccg aggcctgcga caggatgtgc tcggcgaaga aatgggcggt 6960
   ggcgatcttg gcgtcgtaga agcccgggtc gccggcgcgc ttggcgtcgg ccgccagcat 7020
   ggcgcggccg aactgccagc ccgagaacac gatgccgcac agcttcaggt acggcaccga 7080
   gccggcgaac acggcgttcg ggtccgactt ggcgttggcc accacgaagg cgaccacgtc 7140
   ctccagggcg gcgcggccct tggccagctg ggcctgcacg gcggtgaagc cggcgcacga 7200
   gtgattgccc agggcggcct cggtctcggc gatctgggcg cagatggcgc gggccacggc 7260
   gccgccgtcg cgcacggtct tgcggccgat caggtcgttg gcctggatcg cggtggtgcc 7320
   ctcgtagatc ggcaggatgc gggcgtcgcg gtagtgctgg gcggcgccgg tctcctcgat 7380
   gaagcccatg ccgccgtgca cctgcacgcc cagcgaggtc acgtcgatcg acagctcggt 7440
   cgaccagccc ttcaccaccg gcaccaggaa ctcgtagaag gcctggttct gctggcgcac 7500
   ggcctcgtcc gggtgctggt gggcggcgtc cgaggcggcg gcggccacgt aggccacggc 7560
   gcgggcgccc tcggtcaggg cgcgcatggt catcagcatg cgcttcacgt ccgggtggtg 7620
   gatgatggtc acggcctcgc gggccgagcc gtccaccggg cgcgactgca cgcgctcgcg 7680
   ggcgaaggcc acggcctgct ggtaggcgcg ctccgacacg gcgatcccct gcatgcccac 7740
   cgagaagcgg gccgagttca tcatgatgaa catgtactcc aggccgcggt tctcctcgcc 7800
   caccagggtg ccgatggcgc cgccgtggtc gccgaactgc agcacggcgg tcggcgaggc 7860
   cttgatgccc agcttgtgct cgatcgacac gcagtgcacg tcgttgcgct cgccggtcga 7920
   gccgtcggcg ttcaccatga acttcggcac gatgaacagc gagatgccct tcacgccctc 7980
   cggggcggtc ggggtgcggg ccagcaccag gtgcacgatg ttcttggcca tgtcgtgctc 8040
   gccgtaggtg atgaagatct tcgtgccgaa caccttgtag gtgccgtcgc cctgcggctc 8100
   ggcgcgggtg cgcacggcgg ccaggtcgga gccggcctgc ggctccgtca ggttcatggt 8160
   gccggtccac tcgcccgaga tcagcttcgg caggaaggtg gccttctgct cgtcggtgcc 8220
   ggcggtcagc agggcctcga tggcgccgtc ggtcagcagc gggcacaggg cgaacgacag 8280
   gttggcggtg ttcagcatct cgttgcaggc ggtggccacc agcttcggca ggccctggcc 8340
   gccgaactcc tgcgggtgca gcacgccctg ccagccgccc tcgccgaact ggcggaaggc 8400
   ctccttgaag cccgggtggg tggtcaccac gccgtccttc cacgacgacg ggtccaggtc 8460
   gccggcgcgg ttcagcgggg ccaccacctg ctcgttgaac ttggcggcct cgtccagcac 8520
   ggcctcggcg gtctccgggg tggcctcctc gaagcccggc agcttcgaca cggcctccag 8580
   gccggccagc tcgttcatca caaacagcat gtccttgatc ggggcgcggt aggtcatcga 8640
   tgtctcccaa catgtatgaa aaagccgttc gcgggtcacc cccggaacgg ctcgtacttg 8700
   ctaccagtgc cggccgcctg gcggccggca aggcatcagc ccagcgcggc caccagctcc 8760
   ggcaccacgg tgttcagatc gcccaccagg ccgtagtcgg ccaccgagaa gatcggggcc 8820
   tccgcgtcct tgttgatggc cacgatcacc ttgctgtcct tcatgccggc caggtgttgg 8880
   atcgcgccgc tgatgcccac ggcgatgtac agctgcgggg ccacaatctt gccggtctgg 8940
   cccacctggt agtcgttcgg cacgaagccc gcgtccacgg cggcgcgcga ggcgcccagg 9000
   gccgccccca gcttgtcggc cagcggggtc agcaccttgg tgtagttctc gcccgagccc 9060
   acgccgcggc cgcccgacac gatgatcttg gcggcggtca gctccgggcg gtccgacttg 9120
   gccacctcgc ggctcacgaa ctgcgacacg ccggcatcgg ccacggccgg cagggtctcg 9180
   accgcggccg agccgccctc ggcggcggcc gcgtcgaagg cggtgccgcg cacggtgatc 9240
   actttgatct tgtcctccga cttcacggtg gcgatggcgt tgccggcgta gatcgggcgc 9300
   tcgaaggtgt ccggggcgtc caccttcgag atctccgaga tctgggccac gtccagcttc 9360
   gcggccacgc gcggcaggat gttcttcccg tacggggtgg ccggggccag aatgtgcgag 9420
   tagtcgttgg caatggccag ggcctgctcc gccacgttct ccgcgaggcc gtcgccgaag 9480
   tacggggcat cggccagcag gaccttggtc acccccgcga tcttggcggc ggcgtccgcg 9540
   gcggctttgg cgttggcgcc ggccaccagc acgtgcacgt cgcccccgca ctgggcggcg 9600
   gccgtcacgg tgttcagggt ggcggccttg atcgactggt tgtcgtgctc ggcgatcacc 9660
   agggcggtca tgttatttct cccccgcgct caaatcacct tggcctcgtt cttgagcttc 9720
   tgcaccaggg tcgccacgtc cggcaccatc accccggccg agcgcttggc cggctccacg 9780
   actttcagcg tcgacaggcg cggtttgacg tccacgccga ggtcctccgg cttgacgatg 9840
   tccagctgtt tcttcttggc tttcatgatg ttcggcagcg tcacgtagcg cggctcgttc 9900
   aggcgcaggt cggtggtcac caccgcgggg agcttcagcg agagggtttc caggccccca 9960
   tccacctcgc gcgtcacgga ggccttgccg tcggcgacca cgactttcga ggcgaaggtg 10020
   gcctgcggca ggccggcgag ggcggccacc atctggccgg tctggttcga gtcgtcgtcg 10080
   atcgcctgct tgccgaggat caccagttgc ggttgctcct tatcgatgag ggccttcagg 10140
   agtttcgcca ccgccagcgg ctgcaggtct tcgttcgatt ccacgaggat gccccggtcc 10200
   gccccgatgg ccatcgccgt gcgcagggtc tcctggcatt gggtcacgcc gcacgacacc 10260
   gcgatgactt ccgtcacgac gcccgcttct ttgaggcgga cggcctcctc cacggcaatc 10320
   tcgtcgaacg ggttcatgct catcttcacg ttggccaggt cgacgccgct gccatccgcc 10380
   ttcacgcgca ccttgacgtt gtagtccacc acgcgcttca ccgccaccag gaccttcatg 10440
   cgctcactcc actgatatgt gaaattgtta tccgctcaca attccacaca acatacgagc 10500
   cggaagcata aagtgtaaag cctggggaag cttggggaga acaatcagcc cggcaggggc 10560
   cgggctgatt gtgcctgcgt gccgccggtg cttacggcag cttgaccacg gcctcgcccg 10620
   tcacggcgag cgcgccgcct tgcgtgaaga tccgggtggt cagggtggcg atcggcttgt 10680
   cctcgcgcag cgcggtgacc tccacttccg ccgtcacctc gtcgcccacg aacaccggga 10740
   gcttgaacga cagcgattgg cccaggtaga tcgagccctt gcccggcagt tgttggccga 10800
   gcaggcccga gaacagcgac gccagcagca tgccgtggac gatcgggcgc tcaaaggcgg 10860
   tcgtcgcggc gaaggccggg tccaggtgca gcgggttgaa gtcctccgac agggcggcaa 10920
   acgcggccac ctccgccgcg ccaaaccgct tcgacaggcg ggctttctgc cccacctcga 10980
   gcgactgggc cgacatgcgg cgtcctcctc tgtttcagcc catatgcagg ccgccgttga 11040
   gcgagaagtc ggcgccggtc gagaaaccgg actcctccga cgacaaccag gcgcagatcg 11100
   aggcgatctc ttccggcagg cccaggcgct tgaccgggat cgtcgcgacg atcttgtcga 11160
   gcacgtcctg gcggatcgcc ttgaccatgt cggtggcgat atagcccgga gagaccgtgt 11220
   tgacggtcac gcccttggtc gccacttcct gcgccagtgc catggtgaag ccatgcaggc 11280
   cggccttggc ggtggagtag ttggtctggc cgaactggcc cttctgcccg ttcaccgacg 11340
   agatgttgac gatgcggccc cagccacggt cggccatgcc gtcgatcacc tgcttggtga 11400
   cgttgaacag cgaggtcagg ttggtgtcga tcaccgcatc ccagtcggcg cgggtcatct 11460
   tgcggaacac cacgtcgcgg gtgataccgg cgttgttgat cagcacatca acctcgccga 11520
   cctcggactt gaccttgtcg aatgcggtct tggtcgagtc ccagtcagcc acattgcctt 11580
   ccgaggcaat gaaatcgaag cccagggcct tctgctgctc cagccacttt tcgcggcgcg 11640
   gcgagttggg gccgcaaccg gccaccacac gaaagccatc cttggccagc cgctggcaaa 11700
   tggcggttcc gataccaccc atgccgccgg tcacatacgc aatgcgctga gtcatgtcca 11760
   ctccttgatt ggcttcgtta tcgtcgccgg gtccgcgcca accgcgcgcg gccccggaaa 11820
   accccttcct tatttgcgct cgactgccag cgccacgccc atgccgccgc cgatgcacag 11880
   cgaggccagg cccttcttcg cgtcacggcg cttcatctcg tgcagcagcg tcaccaggat 11940
   acggcagccc gacgcgccga tcgggtggcc gatggcgatg gcgccgccgt tcacattgac 12000
   cttggaggtg tcccagccca tctgctggtg caccgccagc gcctgcgcgg caaaggcctc 12060
   gttgatctcc atcaggtcca ggtcttgcgg ggtccactcg gcgcgcgaca gggcgcgctt 12120
   ggaggccggc accgggccca tgcccatcac cttgggatcg acaccggcgt tggcatagct 12180
   cttgatcgtg gccagcgggg tcaggcccag ttccttggcc ttggccgccg acatcaccac 12240
   caccgcggcg gcgccgtcgt tcaggcccga ggcgttggcc gcggtcaccg tgccggcctt 12300
   gtcgaaggcg ggcttgaggc cggacatgct gtccagcgtg gcgccctggc gcacgaactc 12360
   gtcggtcttg aaggccaccg ggtcgccctt gcgctgcggg atcagcaccg ggacgatctc 12420
   ttcgtcaaac ttgccggcct tctgcgcggc ttcggccttg ttctgcgagc cgacggcgaa 12480
   ctcatcctgc gcctcgcgtg tgatgccgta ttccttggcc acgttctcgg cggtgatgcc 12540
   catgtggtac tggttgtaca cgtcccacag gccgtcgacg atcatggtgt cgaccagctt 12600
   ggcatcgccc atgcggaaac catcgcgcga gcccggcagc acgtgcgggg cggcgctcat 12660
   gttttcctgg ccgccggcca ccacgatctc ggcgtcgccc gccatgatcg cgttggcggc 12720
   cagcatcacg gccttcaggc ccgagccgca caccttgttg atggtcatgg ccggcaccat 12780
   cgccggcagg ccggccttga tcgcggcctg gcgtgcgggg ttctggcccg aaccggcggt 12840
   cagcacctgg cccatgatga cttcgctcac ctgctccggc ttgacgccgg cgcgctccag 12900
   cgcggccttg atgaccacgg cacccagttc cggtgccggg atcttggcca gcgagccgcc 12960
   aaacttgccg accgcggtgc gggcggcgga tacgatgaca acgtcagtca ttgtgtagtc 13020
   ctttcaatgg aaaggtaccc agcttttgtt ccctttagtg agggttaatt gcgcgcttgg 13080
   cgtaatcatg gtcatagctg tttcctgtgt gaaattgtta tccgctcaca attccacaca 13140
   acatacgagc cggaagcata aagtgtaaag cctggggtgc ctaatgagtg agctaactca 13200
   cattaattgc gttgcgctca ctgcccgctt tccagtcggg aaacctgtcg tgccagctgc 13260
   attaatgaat cggccaacgc gcggggagag gcggtttgcg tattgggcgc atgcataaaa 13320
   actgttgtaa ttcattaagc attctgccga catggaagcc atcacaaacg gcatgatgaa 13380
   cctgaatcgc cagcggcatc agcaccttgt cgccttgcgt ataatatttg cccatggggg 13440
   tgggcgaaga actccagcat gagatccccg cgctggagga tcatccagcc ggcgtcccgg 13500
   aaaacgattc cgaagcccaa cctttcatag aaggcggcgg tggaatcgaa atctcgtgat 13560
   ggcaggttgg gcgtcgcttg gtcggtcatt tcgaacccca gagtcccgct cagaagaact 13620
   cgtcaagaag gcgatagaag gcgatgcgct gcgaatcggg agcggcgata ccgtaaagca 13680
   cgaggaagcg gtcagcccat tcgccgccaa gctcttcagc aatatcacgg gtagccaacg 13740
   ctatgtcctg atagcggtcc gccacaccca gccggccaca gtcgatgaat ccagaaaagc 13800
   ggccattttc caccatgata ttcggcaagc aggcatcgcc atgggtcacg acgagatcct 13860
   cgccgtcggg catgcgcgcc ttgagcctgg cgaacagttc ggctggcgcg agcccctgat 13920
   gctcttcgtc cagatcatcc tgatcgacaa gaccggcttc catccgagta cgtgctcgct 13980
   cgatgcgatg tttcgcttgg tggtcgaatg ggcaggtagc cggatcaagc gtatgcagcc 14040
   gccgcattgc atcagccatg atggatactt tctcggcagg agcaaggtga gatgacagga 14100
   gatcctgccc cggcacttcg cccaatagca gccagtccct tcccgcttca gtgacaacgt 14160
   cgagcacagc tgcgcaagga acgcccgtcg tggccagcca cgatagccgc gctgcctcgt 14220
   cctgcagttc attcagggca ccggacaggt cggtcttgac aaaaagaacc gggcgcccct 14280
   gcgctgacag ccggaacacg gcggcatcag agcagccgat tgtctgttgt gcccagtcat 14340
   agccgaatag cctctccacc caagcggccg gagaacctgc gtgcaatcca tcttgttcaa 14400
   tcatgcgaaa cgatcctcat cctgtctctt gatcagatct tgatcccctg cgccatcaga 14460
   tccttggcgg caagaaagcc atccagttta ctttgcaggg cttcccaacc ttaccagagg 14520
   gcgccccagc tggcaattcc ggttcgcttg ctgtccataa aaccgcccag tctagctatc 14580
   gccatgtaag cccactgcaa gctacctgct ttctctttgc gcttgcgttt tcccttgtcc 14640
   agatagccca gtagctgaca ttcatcccag gtggcacttt tcggggaaat gtgcgcgccc 14700
   gcgttcctgc tggcgctggg cctgtttctg gcgctggact tcccgctgtt ccgtcagcag 14760
   cttttcgccc acggccttga tgatcgcggc ggccttggcc tgcatatccc gattcaacgg 14820
   ccccagggcg tccagaacgg gcttcaggcg ctcccgaagg t 14861

<210> 75
<211> 14244
<212> DNA
<213> Artificial Sequence

<220>
<223> vector

<400> 75
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960
ttcgccctgc gcggtcgctg cgctcccttg ccagcccgtg gatatgtgga cgatggccgc 1020
gagcggccac cggctggctc gcttcgctcg gcccgtggac aaccctgctg gacaagctga 1080
tggacaggct gcgcctgccc acgagcttga ccacagggat tgcccaccgg ctacccagcc 1140
ttcgaccaca tacccaccgg ctccaactgc gcggcctgcg gccttgcccc atcaattttt 1200
ttaattttct ctggggaaaa gcctccggcc tgcggcctgc gcgcttcgct tgccggttgg 1260
acaccaagtg gaaggcgggt caaggctcgc gcagcgaccg cgcagcggct tggccttgac 1320
gcgcctggaa cgacccaagc ctatgcgagt gggggcagtc gaaggcgaag cccgcccgcc 1380
tgccccccga gcctcacggc ggcgagtgcg ggggttccaa gggggcagcg ccaccttggg 1440
caaggccgaa ggccgcgcag tcgatcaaca agccccggag gggccacttt ttgccggagg 1500
gggagccgcg ccgaaggcgt gggggaaccc cgcaggggtg cccttctttg ggcaccaaag 1560
aactagatat agggcgaaat gcgaaagact taaaaatcaa caacttaaaa aaggggggta 1620
cgcaacagct cattgcggca ccccccgcaa tagctcattg cgtaggttaa agaaaatctg 1680
taattgactg ccacttttac gcaacgcata attgttgtcg cgctgccgaa aagttgcagc 1740
tgattgcgca tggtgccgca accgtgcggc accctaccgc atggagataa gcatggccac 1800
gcagtccaga gaaatcggca ttcaagccaa gaacaagccc ggtcactggg tgcaaacgga 1860
acgcaaagcg catgaggcgt gggccgggct tattgcgagg aaacccacgg cggcaatgct 1920
gctgcatcac ctcgtggcgc agatgggcca ccagaacgcc gtggtggtca gccagaagac 1980
actttccaag ctcatcggac gttctttgcg gacggtccaa tacgcagtca aggacttggt 2040
ggccgagcgc tggatctccg tcgtgaagct caacggcccc ggcaccgtgt cggcctacgt 2100
ggtcaatgac cgcgtggcgt ggggccagcc ccgcgaccag ttgcgcctgt cggtgttcag 2160
tgccgccgtg gtggttgatc acgacgacca ggacgaatcg ctgttggggc atggcgacct 2220
gcgccgcatc ccgaccctgt atccgggcga gcagcaacta ccgaccggcc ccggcgagga 2280
gccgcccagc cagcccggca ttccgggcat ggaaccagac ctgccagcct tgaccgaaac 2340
ggaggaatgg gaacggcgcg ggcagcagcg cctgccgatg cccgatgagc cgtgttttct 2400
ggacgatggc gagccgttgg agccgccgac acgggtcacg ctgccgcgcc ggtagcactt 2460
gggttgcgca gcaacccgta agtgcgctgt tccagactat cggctgtagc cgcctcgccg 2520
ccctatacct tgtctgcctc cccgcgttgc gtcgcggtgc atggagccgg gccacctcga 2580
cctgaatgga agccggcggc acctcgctaa cggattcacc gtttttatca ggctctggga 2640
ggcagaataa atgatcatat cgtcaattat tacctccacg gggagagcct gagcaaactg 2700
gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc tcagaaaaaa ataaaaaaaa 3240

aacaataccc atgtttatag ggcaaaagtt ttataaacat gggtactggt tatattatat 3300

tatttatgac tttattactt gtactccttc gccttttcct cgccccggag cacgcgcagg 3360

ccgccttcgg ccagggcgag cagctcgtct tccccgccgt accgcaccac gggcgcgatg 3420

aacttcaccc ggtcttcaat ggcgttgcac acgtgctcat tgtaggcgat gccgccggtg 3480

aggatgatgg cgtccacgtt ccccttcagc acggtcgagc acttgccaat ttctttcgcc 3540

acctggaagg tgaaggcctc ataaatcagg gcgcactttt tatcgccctc cagggctttg 3600

tccacgaccg ccttgaagtc gatggtattc aggtacgaca ccaccccgcc cttcccattg 3660

atcttcttca tcacctcttc gtaggtgtac ttgttcgaaa agcacaggcg cacgaggtcg 3720

ccgatgggca cgccgccgga gcgttccggc gaaaacgggc cctcgccatc cagggtgttg 3780

ttcacctcga tcacgcggcc gtccttgtgg gtccccaccg aggtcccgcc gcccatgtgc 3840

accacgatca ggttcagatc ctcgtacttt ttccccactt ctttggcgta gcgccgggcc 3900

acggccttct ggttgagggc gtgaaaaatg gacttgcgcg ggatgtccgc catgcccgag 3960

atgcgcgaca cctcgtccag ttcgtcgacg accaccgggt ccacgatgta ggccggcacg 4020

ttaatttcct tggcaatctc gttcgcgata atcccgccca ggttcgaggc atgctggccc 4080

tgcaccccca ccttcaggtc ttccagcatt ttttggttca cggcgtacgt gcccgacacg 4140

atgggcttca gcaggccccc gcgccccacg acggcgttca gcgacgacac ttcgatgttg 4200

gcctccttga gcgcgtccag gatgacgttc ttgcggaatt ggaactggtc gaaaatggta 4260

ttatacttct cgatctcctc cgccgagtgg cgcagcgttt tctcaaagat ctctttctcg 4320

tcgtcataga tgccgatctt cgtggaggtg ctcccggggt tgatgatgag gaggcggtac 4380

atgttaacat tcctccactt aactttaatt tacttgttcc cggcgacgag ggccgccagc 4440

gcaatcgaat tcatcttggt ctcgtgcgaa tccgcccgcg aggtcagcac gaccggggcg 4500

ctggtgccca cgaggatgcc cccattcttg ctgtccgtcg tataggtgag ggttttatac 4560

atcacgttgc ccgtctcgat gttgggcatg aggaagatat ccgctttgcc cgccacctcc 4620

cccgtcacgc ccttatggtg cgcggcttcc tcggacaggg caatgtccag ggccaggggg 4680

ccatccacca cgcagccctt gatctggccg cgatccgaca tcttgctcag catggcggca 4740

tccagggtcg acggcatttt ggggttgatc acctccacgg cgcagatcgg ggccactttc 4800

gggttttcga tgccgatcgc gtgggccacc ttcaccgaat tgttgacaat gtcgatcttc 4860

tctttcagct ccgggtaggt gttaaaggcg acgtccgtga gaaacaggag gcggtcaaac 4920

ttctcggtct caaagacggc cacatgcgac atcgttttcc ccgtgcgcag gcccacttcc 4980

ttgttgagca ccgagcgcag aaacgtggcg gtattcacca gccccttcat caccatgtcg 5040

gccttgccgg tcgacaccag ttccaccgcc ttcagcgccg cctttttcac attgggctca 5100

ttcacaattt caaaatcgtt cacatccatg ccaatcttca gggcgatgga cacgatttca 5160

tcgtgatcgc ccaccaggat ggcgtcggcg atcccgttct tcttcgcgtc ccgcacggcc 5220

tccagcaccg gttcgtcctg ggcgaccgcg acggcgactt tcttcatctc cttcgatttc 5280

actttcatga tgatttcgtt gaacgacttg atcattcttg aatctcctga aattaggtgc 5340

gatcgaccac ctcgcgcacg tttttgatca cgaaccccga cttcacatag cccggaatcg 5400

aattcagatc cacctccagg tcctgctccg ggacgtcgta cgtgatcttt tccgcaaaat 5460

acttcatggt ctcctccatg ataatcacgg tgatccactc cccggcgcag cggtggttgg 5520

tccaatagtc gcccccgccc tgcgggatga ggtcgaacgg cgagccgtcc caggtctcga 5580

accgctccgg gcgaaattcg ttcgggtcgt cccacaggct ctcgtcatgg gtggtgccat 5640

acacgtcgag ggccagcccc acgccggcgg gaatggtcac gccttggaag tcgatatcca 5700

ctttggcctt ccccgggagg aacggcacga acgggtagta gcggcggacc tcctgcgcga 5760

acttgtaggc gtagtccggc tccgacttga tcttctcgcg ggtgatggga ttctcgttca 5820

tcgcgtgcag cccgaaggac acgaagcggt tgatggcgat cagcggccga aaggtgttca 5880

tcaggtcaat cgcgcacgtc cgcgagtcca tcgggttccc caggtagtcc tcccagtggg 5940

caaactcata cagggcggtg ccctcgggcg ggtggatgtt gcccttgcgc gtctcgatga 6000

tctgctcctc gagccaatcc tccacgcgcc gccgcgcctc cttcgacgct ttgtagccct 6060

taaacgcccc ccccagggcg cgaaagctgt cgatcataat atccatgtcg gtcgcgatgc 6120

gctcaatgtc ctccgggggc gcctggaccc cggcccagcg ggtgcccacc ttggtcagga 6180

gcacgatcga ttcgcggtag atgttcacct catccatcga ctccatgcgc tgcgtgttgg 6240

cgtgccacag ggtgcgggtc agctcccgca cgtagttgag gttgccttcg gtcatcagcg 6300

acatgaagag cgccttgcgg tccacgtgct tcttgccgtc cacggtatgg atggcgcctt 6360

tgccgaacag ggtattcaca atgcgcttgg gcagcatgcc ttcgcgttgc accacgtcgt 6420

tgttgtagaa catctcggcg ccctccttcc cggtcaccac gacgaagggc ttccccccga 6480

gcgctttggt ctggaacacc gaggtgttga ggcggttgcg ctgattggtc gtgtacagat 6540

agccctgctt cagcaccttc agcgtgttat cgaggccctt atcgcgcttc agggtggcca 6600

ttcttgaatc tcctgaaaaa gtgagttttt gatcacaaat cggccggcta ccgaggcgac 6660

ggccgtcaga aatgttcagg tgtcgcagga ggctgttagc actcacccct gacgagtgct 6720

aattataggg acggggtagg ggcatttcaa gacgcgggtg tgatgcgggt tggatccagc 6780

aggctgcctc gataagcaaa aagggcggcc ccgcggggcc gccctttttc tttccggcga 6840

ctgtcaggcc actcagttgt tggcggcctt gacttgggcg atcagctcgg gcaccacctt 6900

gttcacatcg cccacaatgg ccaggtcggc caccttcatg atcggggctt ccacgtcctt 6960

gttgatcgcg atgatgtagt ccgagtcctg catgccggcc aggtgctgga tggcgcccga 7020

gatgccgcag gcgatgtaca gggtcggcgg cacggtcttg ccggtctgcc ccacttggag 7080

gtccttatcc acccactcct tttcgatggc ggcgcgcgag gcggcgatgg tgccgccgag 7140

gagggaggcc agctcctcca gcttctcaaa gttctccttc gagcccacgc cgcggccccc 7200

ggccacgagc accttggcct cgccgatatc ggcaatatcc ttggcgagct tcaccacttt 7260

cgagaccttg gtgcggatgt ccgaggcggt cagcttgatg gccactttct cgatcttgtc 7320

gtccgacacg ttggcgtcgt tcaccggcag cttctcgaag aacacgccgg gccgcaccgt 7380

ggccatctgc gggcggtggt ccgagcacac gatggtggcg atcaggttgc cgccgaaggc 7440

cgggcgggtc gcgagcagat cgcggttctc cacgtcgatg tccagcgagg tgcagtcggc 7500

ggtcaggccg gtcgacagcc gggcggcgat ccgcgggccg aggtcgcggc cgatgaacgt 7560

ggcgccgatg aacaggatct ccggcttgcg ctcgttcacc aggtcacaga tcaccttggc 7620

gtagccgtcg gtgctgaaat gggccagcag ctcgttgtcg gcggccagga ccttgtccgc 7680

gccgtgcgac agcaggtcct tcgacatctt ctcggtgttg tgccccagca gcacggccgt 7740

cagctccacg cccagtttct cggccatttc cttgcccttc cccagcagtt ccagcgacac 7800

cttctgcagc tcgccgtcgc gttgctcggc gaacacccac acgcccttgt agtcggcttt 7860

gttcattgaa aaatccctcc taacttaaat atgtgttctt cttttagttc tgcgacagca 7920

tatcggccgc ctccttcacg ggcttatcga tcacttcgcc ttggcccttg acttctttgg 7980

tcgacgactt tttcaccttg gtcggcgagc ccttcagccc caggttggcc ttgtcgacgt 8040

cgatgtcatc cgcggtccac atcttgacct ccttgtcgaa cgcgccgaaa atcttttcca 8100

ccgacatgta gcgcggcacg ttcagctcct tgatggccgt cagcaggacc ggggtcttca 8160

cctccacgac ctcgtagccg tcctcccagg ccttgcggat tttcagcgta tcgccatcca 8220

cctcgacttt ctccacgtag gtcacctggg ggatgcccag atgctcggcg atctccggcc 8280

cgacctgggc cgtgtcgccg tcaatggcct ggcgcccggc aaacacgata tcatatttca 8340

gcttcttgat gcccgcggcg atggtgtgcg aggtcgccag ggtatccgcc cccccgaagg 8400

cgcggtcggt cagcagcacc gcctcgtccg cccccatggc cagggcctcc accagggcgt 8460

tcttggcctg cgggggcccc atcgagatca cggtcacatg ggccccgtag ttgtctttca 8520

ggaccagcgc ttcctccagc gcattcttgt cgtccgggtt gatgatcgac ggcacgccct 8580

cgcggatcag ggtccccttc accggatcga tgcgcacctc ggcggtgtcc ggcacctgct 8640

tcagacacac cacgatgttc atcctcttaa cctccttaaa ttagcggaaa atcttgccgc 8700

taatcaccag cttctgcact tccgacgtgc cctcgtagat ctcggtaatc ttggcgtcgc 8760

gcatcatgcg ctcgaccggg tagtccttcg tgtagccgta gccgccgaac agttgcaccg 8820

ctttggtggt cacgtccatg gcgacgttgg cggcatgcag cttggcgcgc gcggcgtcca 8880

cggtgtacgg caggcccgcc tgcttcaggt aggcggcctt atagaccaga taccgggcgg 8940

actcaatggc cacgtccata tcggccatca tccaggccag gccctgaaac ttgtccagcg 9000

agcggccgaa ctgcttgcgc tccttcatgt aggcccgggc ttcgttgaag gcgccctccg 9060

cgatccccag ggcttgggcc gcaatgccga tccgcccccc atccagggtt ttcatcgcaa 9120

tcgggaaccc cttcccctct ttgccaatca tgttttccac cggcacgatc atgtcctcga 9180

acaccagttc ggtggtcgac gacgcgcgga tgcccagctt ttgctccacc ttgccgatcg 9240

agaagccctt aaaccctttc tcaatgataa aggccgagat gcctttggtg cccttggtgc 9300

ggtcggtcat cgcgaagatc acgaacgtgt ccgccacgcc gccgttggta ataaagattt 9360

tggagccgtt gatcacgtag tggtcgcctt ccaggacggc cacggtctgc tgcgcgcccg 9420

agtcggtgcc ggcgttcggt tccgtcaggc cgtacgcgcc gatcttctcc cctttggcca 9480

gcgggaccag gtacttctgt ttctgctcct cggtcccgtg ctcgttgatc agcgaggcgc 9540

agagggaggt gtgggccgac aggatcaccc cggtggtgcc gcacaccttg gacagctcct 9600

ccacggcgat gatgtacgac agcacgtccc cccccgcccc gccgtactcc ttcgagaacg 9660

gaatccccat catgccatac tggcccatct tcttcacgtt ctccatcggg aagcgctcgg 9720

tctcgtcgat ctccgcggca atgggcttca cctcgttttc cgcgaactcg cgcaccatct 9780

ggcgcaccag ctcctgctcg cgggtcaggt tgaagtccat ataaacttac ctcctatcta 9840

tgtgaaattg ttatccgctc acaattccac acaacatacg agccggaagc ataaagtgta 9900

aagcctgggg aagcttgggg agaacaatca gcccggcagg ggccgggctg attgtgcctg 9960

cgtgccgccg gtgcttacgg cagcttgacc acggcttccc cggtgacggc cagggccccg 10020

ccttgggtaa agatccgggt cgtcagcgtg gcgatcggtt tgtcctcgcg cagcgcggtg 10080

acttccacct cggcggtcac ttcgtccccc acgaacaccg gcagtttgaa cgagagcgat 10140

tggcccaggt agatgctccc cttgccgggg agctgctggc cgaggaggcc cgagaacagc 10200

gaggcgagca gcatgccatg cacgatgggg cgctcgaacg cggtggtcgc ggcaaacgcg 10260

gggtccaggt gcagcgggtt gaagtcctcg gagagcgccg caaaggccgc cacctccgcc 10320

gcgccaaacc gcttggacag ccgggctttt tgcccgacct ccagcgactg ggccgacatg 10380

cggcgtcctc ctctgtttca gcccatatgc aggccgccgt tgagcgagaa gtcggcgccg 10440

gtcgagaaac cggactcctc cgacgacaac caggcgcaga tcgaggcgat ctcttccggc 10500

aggcccaggc gcttgaccgg gatcgtcgcg acgatcttgt cgagcacgtc ctggcggatc 10560

gccttgacca tgtcggtggc gatatagccc ggagagaccg tgttgacggt cacgcccttg 10620

gtcgccactt cctgcgccag tgccatggtg aagccatgca ggccggcctt ggcggtggag 10680

tagttggtct ggccgaactg gcccttctgc ccgttcaccg acgagatgtt gacgatgcgg 10740

ccccagccac ggtcggccat gccgtcgatc acctgcttgg tgacgttgaa cagcgaggtc 10800

aggttggtgt cgatcaccgc atcccagtcg gcgcgggtca tcttgcggaa caccacgtcg 10860

cgggtgatac cggcgttgtt gatcagcaca tcaacctcgc cgacctcgga cttgaccttg 10920

tcgaatgcgg tcttggtcga gtcccagtca gccacattgc cttccgaggc aatgaaatcg 10980

aagcccaggg ccttctgctg ctccagccac ttttcgcggc gcggcgagtt ggggccgcaa 11040

ccggccacca cacgaaagcc atccttggcc agccgctggc aaatggcggt tccgatacca 11100

cccatgccgc cggtcacata cgcaatgcgc tgagtcatgt ccactccttg attggcttcg 11160

ttatcgtcgc cgggtccgcg ccaaccgcgc gcggccccgg aaaacccctt ccttatttgc 11220

gctcgactgc cagcgccacg cccatgccgc cgccgatgca cagcgaggcc aggcccttct 11280

tcgcgtcacg gcgcttcatc tcgtgcagca gcgtcaccag gatacggcag cccgacgcgc 11340

cgatcgggtg gccgatggcg atggcgccgc cgttcacatt gaccttggag gtgtcccagc 11400

ccatctgctg gtgcaccgcc agcgcctgcg cggcaaaggc ctcgttgatc tccatcaggt 11460

ccaggtcttg cggggtccac tcggcgcgcg acagggcgcg cttggaggcc ggcaccgggc 11520

ccatgcccat caccttggga tcgacaccgg cgttggcata gctcttgatc gtggccagcg 11580

gggtcaggcc cagttccttg gccttggccg ccgacatcac caccaccgcg gcggcgccgt 11640

cgttcaggcc cgaggcgttg gccgcggtca ccgtgccggc cttgtcgaag gcgggcttga 11700

ggccggacat gctgtccagc gtggcgccct ggcgcacgaa ctcgtcggtc ttgaaggcca 11760

ccgggtcgcc cttgcgctgc gggatcagca ccgggacgat ctcttcgtca aacttgccgg 11820

ccttctgcgc ggcttcggcc ttgttctgcg agccgacggc gaactcatcc tgcgcctcgc 11880

gtgtgatgcc gtattccttg gccacgttct cggcggtgat gcccatgtgg tactggttgt 11940

acacgtccca caggccgtcg acgatcatgg tgtcgaccag cttggcatcg cccatgcgga 12000

aaccatcgcg cgagcccggc agcacgtgcg gggcggcgct catgttttcc tggccgccgg 12060

ccaccacgat ctcggcgtcg cccgccatga tcgcgttggc ggccagcatc acggccttca 12120

ggcccgagcc gcacaccttg ttgatggtca tggccggcac catcgccggc aggccggcct 12180

tgatcgcggc ctggcgtgcg gggttctggc ccgaaccggc ggtcagcacc tggcccatga 12240

tgacttcgct cacctgctcc ggcttgacgc cggcgcgctc cagcgcggcc ttgatgacca 12300

cggcacccag ttccggtgcc gggatcttgg ccagcgagcc gccaaacttg ccgaccgcgg 12360

tgcgggcggc ggatacgatg acaacgtcag tcattgtgta gtcctttcaa tggaaaggta 12420

cccagctttt gttcccttta gtgagggtta attgcgcgct tggcgtaatc atggtcatag 12480

ctgtttcctg tgtgaaattg ttatccgctc acaattccac acaacatacg agccggaagc 12540

ataaagtgta aagcctgggg tgcctaatga gtgagctaac tcacattaat tgcgttgcgc 12600

tcactgcccg ctttccagtc gggaaacctg tcgtgccagc tgcattaatg aatcggccaa 12660

cgcgcgggga gaggcggttt gcgtattggg cgcatgcata aaaactgttg taattcatta 12720

agcattctgc cgacatggaa gccatcacaa acggcatgat gaacctgaat cgccagcggc 12780

atcagcacct tgtcgccttg cgtataatat ttgcccatgg gggtgggcga agaactccag 12840

catgagatcc ccgcgctgga ggatcatcca gccggcgtcc cggaaaacga ttccgaagcc 12900

caacctttca tagaaggcgg cggtggaatc gaaatctcgt gatggcaggt tgggcgtcgc 12960

ttggtcggtc atttcgaacc ccagagtccc gctcagaaga actcgtcaag aaggcgatag 13020

aaggcgatgc gctgcgaatc gggagcggcg ataccgtaaa gcacgaggaa gcggtcagcc 13080

cattcgccgc caagctcttc agcaatatca cgggtagcca acgctatgtc ctgatagcgg 13140

tccgccacac ccagccggcc acagtcgatg aatccagaaa agcggccatt ttccaccatg 13200

atattcggca agcaggcatc gccatgggtc acgacgagat cctcgccgtc gggcatgcgc 13260

gccttgagcc tggcgaacag ttcggctggc gcgagcccct gatgctcttc gtccagatca 13320

tcctgatcga caagaccggc ttccatccga gtacgtgctc gctcgatgcg atgtttcgct 13380

tggtggtcga atgggcaggt agccggatca agcgtatgca gccgccgcat tgcatcagcc 13440

atgatggata ctttctcggc aggagcaagg tgagatgaca ggagatcctg ccccggcact 13500

tcgcccaata gcagccagtc ccttcccgct tcagtgacaa cgtcgagcac agctgcgcaa 13560

ggaacgcccg tcgtggccag ccacgatagc cgcgctgcct cgtcctgcag ttcattcagg 13620

gcaccggaca ggtcggtctt gacaaaaaga accgggcgcc cctgcgctga cagccggaac 13680

acggcggcat cagagcagcc gattgtctgt tgtgcccagt catagccgaa tagcctctcc 13740

acccaagcgg ccggagaacc tgcgtgcaat ccatcttgtt caatcatgcg aaacgatcct 13800

catcctgtct cttgatcaga tcttgatccc ctgcgccatc agatccttgg cggcaagaaa 13860

gccatccagt ttactttgca gggcttccca accttaccag agggcgcccc agctggcaat 13920

tccggttcgc ttgctgtcca taaaaccgcc cagtctagct atcgccatgt aagcccactg 13980

caagctacct gctttctctt tgcgcttgcg ttttcccttg tccagatagc ccagtagctg 14040

acattcatcc caggtggcac ttttcgggga aatgtgcgcg cccgcgttcc tgctggcgct 14100

gggcctgttt ctggcgctgg acttcccgct gttccgtcag cagcttttcg cccacggcct 14160

tgatgatcgc ggcggccttg gcctgcatat cccgattcaa cggccccagg gcgtccagaa 14220

cgggcttcag gcgctcccga aggt 14244

<210> 76
<211> 14676
<212> DNA
<213> Artificial Sequence

<220>
<223> vector

<400> 76
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaattttct ctggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag gggccacttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc tcagaaaaaa ataaaaaaaa 3240

aacaataccc atgtttatag ggcaaaagtt ttataaacat gggtactggt tatattatat 3300

tatttatgac tttattactt gtactccttc gccttttcct cgccccggag cacgcgcagg 3360
ccgccttcgg ccagggcgag cagctcgtct tccccgccgt accgcaccac gggcgcgatg 3420
aacttcaccc ggtcttcaat ggcgttgcac acgtgctcat tgtaggcgat gccgccggtg 3480
aggatgatgg cgtccacgtt ccccttcagc acggtcgagc acttgccaat ttctttcgcc 3540
acctggaagg tgaaggcctc ataaatcagg gcgcactttt tatcgccctc cagggctttg 3600
tccacgaccg ccttgaagtc gatggtattc aggtacgaca ccaccccgcc cttcccattg 3660
atcttcttca tcacctcttc gtaggtgtac ttgttcgaaa agcacaggcg cacgaggtcg 3720
ccgatgggca cgccgccgga gcgttccggc gaaaacgggc cctcgccatc cagggtgttg 3780
ttcacctcga tcacgcggcc gtccttgtgg gtccccaccg aggtcccgcc gcccatgtgc 3840
accacgatca ggttcagatc ctcgtacttt ttccccactt ctttggcgta gcgccgggcc 3900
acggccttct ggttgagggc gtgaaaaatg gacttgcgcg ggatgtccgc catgcccgag 3960
atgcgcgaca cctcgtccag ttcgtcgacg accaccgggt ccacgatgta ggccggcacg 4020
ttaatttcct tggcaatctc gttcgcgata atcccgccca ggttcgaggc atgctggccc 4080
tgcaccccca ccttcaggtc ttccagcatt ttttggttca cggcgtacgt gcccgacacg 4140
atgggcttca gcaggccccc gcgccccacg acggcgttca gcgacgacac ttcgatgttg 4200
gcctccttga gcgcgtccag gatgacgttc ttgcggaatt ggaactggtc gaaaatggta 4260
ttatacttct cgatctcctc cgccgagtgg cgcagcgttt tctcaaagat ctctttctcg 4320
tcgtcataga tgccgatctt cgtggaggtg ctcccggggt tgatgatgag gaggcggtac 4380
atgttaacat tcctccactt aactttaatt tacttgttcc cggcgacgag ggccgccagc 4440
gcaatcgaat tcatcttggt ctcgtgcgaa tccgcccgcg aggtcagcac gaccggggcg 4500
ctggtgccca cgaggatgcc cccattcttg ctgtccgtcg tataggtgag ggttttatac 4560
atcacgttgc ccgtctcgat gttgggcatg aggaagatat ccgctttgcc cgccacctcc 4620
cccgtcacgc ccttatggtg cgcggcttcc tcggacaggg caatgtccag ggccaggggg 4680
ccatccacca cgcagccctt gatctggccg cgatccgaca tcttgctcag catggcggca 4740
tccagggtcg acggcatttt ggggttgatc acctccacgg cgcagatcgg ggccactttc 4800
gggttttcga tgccgatcgc gtgggccacc ttcaccgaat tgttgacaat gtcgatcttc 4860
tctttcagct ccgggtaggt gttaaaggcg acgtccgtga gaaacaggag gcggtcaaac 4920
ttctcggtct caaagacggc cacatgcgac atcgttttcc ccgtgcgcag gcccacttcc 4980
ttgttgagca ccgagcgcag aaacgtggcg gtattcacca gccccttcat caccatgtcg 5040
gccttgccgg tcgacaccag ttccaccgcc ttcagcgccg cctttttcac attgggctca 5100
ttcacaattt caaaatcgtt cacatccatg ccaatcttca gggcgatgga cacgatttca 5160
tcgtgatcgc ccaccaggat ggcgtcggcg atcccgttct tcttcgcgtc ccgcacggcc 5220

tccagcaccg gttcgtcctg ggcgaccgcg acggcgactt tcttcatctc cttcgatttc 5280

actttcatga tgatttcgtt gaacgacttg atcattcttg aatctcctga aattaggtgc 5340

gatcgaccac ctcgcgcacg tttttgatca cgaaccccga cttcacatag cccggaatcg 5400

aattcagatc cacctccagg tcctgctccg ggacgtcgta cgtgatcttt tccgcaaaat 5460

acttcatggt ctcctccatg ataatcacgg tgatccactc cccggcgcag cggtggttgg 5520

tccaatagtc gcccccgccc tgcgggatga ggtcgaacgg cgagccgtcc caggtctcga 5580

accgctccgg gcgaaattcg ttcgggtcgt cccacaggct ctcgtcatgg gtggtgccat 5640

acacgtcgag ggccagcccc acgccggcgg gaatggtcac gccttggaag tcgatatcca 5700

ctttggcctt ccccgggagg aacggcacga acgggtagta gcggcggacc tcctgcgcga 5760

acttgtaggc gtagtccggc tccgacttga tcttctcgcg ggtgatggga ttctcgttca 5820

tcgcgtgcag cccgaaggac acgaagcggt tgatggcgat cagcggccga aaggtgttca 5880

tcaggtcaat cgcgcacgtc cgcgagtcca tcgggttccc caggtagtcc tcccagtggg 5940

caaactcata cagggcggtg ccctcgggcg ggtggatgtt gcccttgcgc gtctcgatga 6000

tctgctcctc gagccaatcc tccacgcgcc gccgcgcctc cttcgacgct ttgtagccct 6060

taaacgcccc ccccagggcg cgaaagctgt cgatcataat atccatgtcg gtcgcgatgc 6120

gctcaatgtc ctccgggggc gcctggaccc cggcccagcg ggtgcccacc ttggtcagga 6180

gcacgatcga ttcgcggtag atgttcacct catccatcga ctccatgcgc tgcgtgttgg 6240

cgtgccacag ggtgcgggtc agctcccgca cgtagttgag gttgccttcg gtcatcagcg 6300

acatgaagag cgccttgcgg tccacgtgct tcttgccgtc cacggtatgg atggcgcctt 6360

tgccgaacag ggtattcaca atgcgcttgg gcagcatgcc ttcgcgttgc accacgtcgt 6420

tgttgtagaa catctcggcg ccctccttcc cggtcaccac gacgaagggc ttccccccga 6480

gcgctttggt ctggaacacc gaggtgttga ggcggttgcg ctgattggtc gtgtacagat 6540

agccctgctt cagcaccttc agcgtgttat cgaggccctt atcgcgcttc agggtggcca 6600

ttcttgaatc tcctgaaaaa gtgagttttt gatcacaaat cggccggcta ccgaggcgac 6660

ggccgtcaga aatgttcagg tgtcgcagga ggctgttagc actcacccct gacgagtgct 6720

aattataggg acggggtagg ggcatttcaa gacgcgggtg tgatgcgggt tggatccagc 6780

aggctgcctc gataagcaaa aagggcggcc ccgcggggcc gccctttttc tttccggcga 6840

ctgtcaggcc actcagttgt tggcggcctt cacctgcgcg atcagctccg ggaccacttt 6900

gttcacgtcg cccacgatcg ccaggtcggc caccttcata atcggggcct ccacgtcctt 6960

attgatggcg atgatgtagt ccgagtcctg catgccggcc aggtgctgga tcgcccccga 7020

gatgccgcag gcgatgtaca gggtcggcgc cacggtcttg ccggtctggc ccacctggag 7080

gtctttgtcc acccactctt tctcgatggc cgcgcgcgag gcggcgatgg tgccgcccag 7140

gagcgaggcc agctcctcca gtttttcgaa gttctccttc gagcccacgc cgcggccccc 7200

ggccacgagc accttggcct cgccgatgtc cgcgatgtct ttggccagct tcaccacttt 7260

cgacactttg gtgcggatgt cggacgcggt cagcttgatg gcgactttct cgatcttatc 7320

gtcggacacg ttggcgtcgt tcaccggcag cttctcgaag aagacgccgg ggcggaccgt 7380

ggccatctgc gggcggtggt ccgagcagac gatggtggcg atcaggttgc cgccgaacgc 7440

cgggcgggtc gccaggaggt cccggttctc cacgtcgatg tccagcgagg tgcagtcggc 7500

ggtcaggccg gtcgacaggc gcgccgcgat ccgcgggccc aggtcccgcc cgatgaacgt 7560

ggcgccaatg aacaggatct ccggcttgcg ctcgttcacg aggtcgcaga tcaccttcgc 7620

gtagccgtcg gtcgagaagt gggcgaggag ttcattgtcg gccgcgagca ccttatccgc 7680

gccgtggctc agcaggtcct tcgacatctt ctcggtgttg tggcccagca gcacggcggt 7740

cagttccacc cccagcttct cggccatctc cttgcccttg cccagcagct ccagcgacac 7800

cttctgcagc tcgccgtcgc gctgctccgc aaacacccac acgcccttgt agtcggcctt 7860

gttcattgaa aaatccctcc taacttaaat atgtgttctt cttttagttc tgcgagagca 7920

tatcggccgc ttccttcacc ggtttatcaa tgacctcgcc ctgccctttc acttctttcg 7980

tgctcgactt cttcactttg gtcggcgacc ccttcaggcc cagattggct ttatccacgt 8040

cgatatcatc cgcggtccac attttcacct ccttgtcgaa cgcgccgaag atttctccca 8100

ccgacatgta ccgcgggacg ttcagttcct taatggccgt caggagcacc ggggtcttga 8160

cttccacgac ctcgtaccca tcctcccagg ccttccggat cttgagcgtg tccccatcca 8220

cctccacttt ttccacgtag gtgacttggg gaatcccgag atgttccgca atctccggcc 8280

cgacctgcgc cgtatcgccg tcgatggcct ggcggccggc aaagacaatg tcatatttca 8340

gcttcttaat gccggcggca atggtatgcg aggtggcgag ggtgtccgcg cccccaaacg 8400

cccggtccgt gagcagcacc gcctcatcgg cccccatggc cagcgcttcg accagggcgt 8460

tcttggcttg gggggggccc atcgagatga ccgtgacgtg cgccccatag ttatctttca 8520

gcacgagggc ttcctcgagc gcgtttttgt cgtcggggtt aatgatggac gggacgccct 8580

cccgaatcag ggtgcctttg acgggatcaa tgcggacctc ggcggtgtcg gggacttgtt 8640

tgaggcagac cacaatattc atcctcttaa cctccttaaa ttagcggaag atcttgcccg 8700

agatcaccag cttctgcacc tccgaggtgc cctcgtagat ctccgtgatc ttggcgtcgc 8760

gcatcatgcg ctccacgggg tagtccttgg tgtagccata gccgccgaac agctgcacgg 8820

ccttggtggt cacgtccatg gccacgttgg cggcatgcag cttggcgcgg gcggcatcca 8880

cggtgtacgg caggcccgcc tgcttcaggt aggcggcctt gtacaccagg tagcgggccg 8940

actcgatggc cacgtccatg tcggccatca tccaggccag gccctggaac ttgtccagcg 9000

agcggccgaa ctgcttgcgt tccttcatgt acgcgcgggc ctcgttaaag gcgccttcgg 9060

cgatgcccag ggcctgggcc gcgatgccga tgcggccccc gtccagggtc ttcatggcga 9120

tcgggaagcc cttgccctcc ttgccaatca tgttctccac cggcacgatc atgtcctcga 9180

acaccagctc ggtggtcgac gacgcgcgga tgcccagctt ttgctccacc ttgccgatcg 9240

agaagccctt gaagcccttc tcgatgataa aggccgagat gcccttggtc cccttggtgc 9300

ggtcggtcat ggcgaagatg acgaaggtgt cggcgacgcc gccgttggtg atgaagattt 9360

tcgagccgtt gatcacgtag tggtcgccct ccagcacggc cacggtctgc tgggcgcccg 9420

aatcggtgcc ggcgttcggc tcggtcaggc cgtaggcgcc gatcttctcc cccttggcca 9480

gcggcaccag gtacttctgc ttctgctcct cggtgccgtg ctcgttaatc agcgaggcgc 9540

acagcgaggt gtgggccgac aggatcacgc cggtggtgcc gcacaccttc gacagctcct 9600

ccacggcgat gatgtacgac agcacgtcgc cgccggcccc gccgtactcc ttcgagaacg 9660

ggatgcccat catgccgtac tggcccatct tcttcacgtt ctccatcggg aaccgctccg 9720

tctcgtcgat ctccgcggca atcggcttca cctcgttctc ggcgaactcc cggaccatct 9780

ggcgcaccag ctcttgctcg cgcgtcaggt tgaagtccat ataaacttac ctcctagcgg 9840

ttcttgaacc cttcgatctt gcgcttctca atgaaggcgg tcatggcgtc tttctggtcc 9900

tcggtcgaga agcattcccc gaacgcttcc gactcgaagg cgagggccgt gtcaatgtcg 9960

cactgcatgc cgcggttaat ggcttgcttc gacagtttga cggccaccgg ggcattgctc 10020

acgattttgt tggcgatctc cttggcggtg ttcatcagtt ccgagggctc caccaccttg 10080

ttcaccaggc caatgcgcag ggcctcatcg gccttgatgt tctgcgcggt gaaaatcagc 10140

tgcttggcca tgcccatgcc gacgaggcgc gacagccgct gggtgccgcc aaagcccggg 10200

gtgatgccca ggcccacttc cggctggccg aagcgggcgt tcgaggaggc gatgcggata 10260

tcgcaggaca tcgcaatttc gcacccgccg cccagggcga acccattcac ggccgcaatc 10320

accggttttt ccagcagctc cagccgccga aacactttgt tccccagaat gccgaacttg 10380

cgcccctcga tggtattcat ttctttcatc tccgagatgt ccgccccggc gacgaagctc 10440

ttctcgcccg cgcccgtcag gatcacggcg agcacctccg agtcgttctc gatttcgccg 10500

atcacgtagt ccatctcttt cagggtgtcc gagttcaggg cgttgagggc cttcgggcgg 10560

ttgatggtca ccaccgcgac cttgccttct ttctccagga tgacattgtt gagttccatg 10620

actaatcctc ctaaaatgtg aaattgttat ccgctcacaa ttccacacaa catacgagcc 10680

ggaagcataa agtgtaaagc ctggggaagc ttcttaagta ataaaaataa gagttacctt 10740

aaatggtaac tcttattttt ttaatattgt ttcatagtat ttctttttat ttcgagtagt 10800

cgtaaaagcc cttgccgctc ttgcggccca gccagcccgc ccggacatac tttttcagca 10860

gcgtgtgcgg gcgatacttc gaatcgccgg tttccgagta cagcacgtcc atgatggcca 10920

ggcaaatgtc gaggccgata aagtccccca gttccagcgg gcccatcggg tggttcgccc 10980

ccagcttcat ggccttatca atatcctcga ccgaggcaat cccttcggcg aggatgccca 11040

ccgcttcatt gatcatcggg atcagaatgc ggttgacgac gaaccccggc gcctcggcga 11100

cttccacggg gtccttgcca atggcaatcg acgtctcttt caccgcgtcg aaggtttcct 11160

gcgacgtggc aatgccgcga atgacctcca cgagtttcat gacgggggcg ggattaaaga 11220

aatgcatgcc gatcactttg tcgttggtct tggtcgcgct cgccacctcc gtgatcgaga 11280

gcgagctggt gttggaggcg agaatcgtct ccggtttgca gatgttgtcg agatccgcaa 11340

agatctgttt cttaatgtcc atgcgctcga cggcggcttc aatcacgagg tcgcagtccg 11400

cggccatgtt caggtccacg gtcccgctga tgcgggtgag aatctccacc ttggtggcct 11460

cttcgatctt gccttttttg accagtttcg acaggttctt gttaatgaaa tccaggccgc 11520

gatcgacgaa ttcatccttg atgtcccgga ggaccacctc gaagcctttg gcggcgaagg 11580

cctgcgcgat gccggacccc atcgtccccg cgccaatgac gcacaccttc ttcattcttg 11640

aatctcctga aactagcact tttccagcag gatcgcggtg ccctggccgc cgccgatgca 11700

cagggtcgcc agccccttct tggcgtcgcg cttctgcatc gcgtgcacca gggtcaccag 11760

gatgcgggcg cccgaggcgc cgatcgggtg ccccagggcg atggcgccgc cattcacgtt 11820

cactttgttc atgtcgaact tcaggtcctt ggcgacggcc agcgactggg cggcaaaggc 11880

ctcgttcgac tcgatcaggt ccagctcgtc gaccgtccag ccggctttct cgatcgccgc 11940

cttggtggcg tagaacgggc cgtagcccat gatggccggg tccacgccgg ccgagccata 12000

cgacacgatc ttcgccagcg gtttcacgcc cagctccttg gccttttcgg ccgacatgat 12060

caccagcacg gccgcgcagt cgttcaggcc cgaggcgttg cccgcggtca cggtgccgtc 12120

cttcttgaag gccggcttca gcttggccag gccctcgatc gtcgacccga agcgcgggtg 12180

ctcgtccgtg tccaccacgg tctcgccctt gcggccctta atcaccaccg gcacgatctc 12240

gtccttgaac tggcccgact tgatggcttc ctccgccttc ttttgcgagg ccagggcgaa 12300

ctcgtcctgc tcctcgcgcg agatgttcca gcgctcggcg atgttctcgg cggtgatgcc 12360

catgtggtag tcgttgaagg cgtcccacag gccgtcggtg atcatctcgt ccacgaactt 12420

ggcgttcccc atgcggtagc cccagcgggc gttgttggcc aggtacgggg cgcgcgacat 12480

gttttccatg ccgccggcaa tgatcacgtc ggcgtcgccg gccttgatga tctgggccgc 12540

cagcgacacg gtgcgcaggc ccgagccgca caccttgttg atggtcatgg cggggatctc 12600

caccgggagg ccggctttga agctcgcctg gcgggccggg ttctggccga gcccggcctg 12660

cagcacgttg cccaggatca cctcgttcac gtcctccggc ttgatgccgg ccttcttcac 12720

ggcctcctta atggcggtgg cgcccaggtc cacggcgggc acgtctttca ggctcttgcc 12780

gtacgagccg atcgcggtgc gcacggccga ggcaatcacc acctccttca ttcttgaatc 12840

tcctgaaagg tacccagctt ttgttccctt tagtgagggt taattgcgcg cttggcgtaa 12900

tcatggtcat agctgtttcc tgtgtgaaat tgttatccgc tcacaattcc acacaacata 12960

cgagccggaa gcataaagtg taaagcctgg ggtgcctaat gagtgagcta actcacatta 13020

attgcgttgc gctcactgcc cgctttccag tcgggaaacc tgtcgtgcca gctgcattaa 13080

tgaatcggcc aacgcgcggg gagaggcggt ttgcgtattg ggcgcatgca taaaaactgt 13140

tgtaattcat taagcattct gccgacatgg aagccatcac aaacggcatg atgaacctga 13200

atcgccagcg gcatcagcac cttgtcgcct tgcgtataat atttgcccat gggggtgggc 13260

gaagaactcc agcatgagat ccccgcgctg gaggatcatc cagccggcgt cccggaaaac 13320

gattccgaag cccaaccttt catagaaggc ggcggtggaa tcgaaatctc gtgatggcag 13380

gttgggcgtc gcttggtcgg tcatttcgaa ccccagagtc ccgctcagaa gaactcgtca 13440

agaaggcgat agaaggcgat gcgctgcgaa tcgggagcgg cgataccgta aagcacgagg 13500

aagcggtcag cccattcgcc gccaagctct tcagcaatat cacgggtagc caacgctatg 13560

tcctgatagc ggtccgccac acccagccgg ccacagtcga tgaatccaga aaagcggcca 13620

ttttccacca tgatattcgg caagcaggca tcgccatggg tcacgacgag atcctcgccg 13680

tcgggcatgc gcgccttgag cctggcgaac agttcggctg gcgcgagccc ctgatgctct 13740

tcgtccagat catcctgatc gacaagaccg gcttccatcc gagtacgtgc tcgctcgatg 13800

cgatgtttcg cttggtggtc gaatgggcag gtagccggat caagcgtatg cagccgccgc 13860

attgcatcag ccatgatgga tactttctcg gcaggagcaa ggtgagatga caggagatcc 13920

tgccccggca cttcgcccaa tagcagccag tcccttcccg cttcagtgac aacgtcgagc 13980

acagctgcgc aaggaacgcc cgtcgtggcc agccacgata gccgcgctgc ctcgtcctgc 14040

agttcattca gggcaccgga caggtcggtc ttgacaaaaa gaaccgggcg cccctgcgct 14100

gacagccgga acacggcggc atcagagcag ccgattgtct gttgtgccca gtcatagccg 14160

aatagcctct ccacccaagc ggccggagaa cctgcgtgca atccatcttg ttcaatcatg 14220

cgaaacgatc ctcatcctgt ctcttgatca gatcttgatc ccctgcgcca tcagatcctt 14280

ggcggcaaga aagccatcca gtttactttg cagggcttcc caaccttacc agagggcgcc 14340

ccagctggca attccggttc gcttgctgtc cataaaaccg cccagtctag ctatcgccat 14400

gtaagcccac tgcaagctac ctgctttctc tttgcgcttg cgttttccct tgtccagata 14460

gcccagtagc tgacattcat cccaggtggc acttttcggg gaaatgtgcg cgcccgcgtt 14520

cctgctggcg ctgggcctgt ttctggcgct ggacttcccg ctgttccgtc agcagctttt 14580

cgcccacggc cttgatgatc gcggcggcct tggcctgcat atcccgattc aacggcccca 14640

gggcgtccag aacgggcttc aggcgctccc gaaggt                                14676

<210>  77
<211>  1689
<212>  DNA
<213>  Aquincola tertiaricarbonis L108

<220>
<221>  CDS
<222>  (1)..(1689)

<400>  77
atg acc tgg ctt gag ccg cag ata aag tcc caa ctc caa tcg gag cgc        48
Met Thr Trp Leu Glu Pro Gln Ile Lys Ser Gln Leu Gln Ser Glu Arg
1               5                   10                  15

aag gac tgg gaa gcg aac gaa gtc ggc gcc ttc ttg aag aag gcg ccc        96
Lys Asp Trp Glu Ala Asn Glu Val Gly Ala Phe Leu Lys Lys Ala Pro
            20                  25                  30

gag cgc aag gag cag ttc cac acg atc ggg gac ttc ccg gtc cag cgc       144
Glu Arg Lys Glu Gln Phe His Thr Ile Gly Asp Phe Pro Val Gln Arg
        35                  40                  45

acc tac acc gct gcc gac atc gcc gac acg ccg ctg gag gac atc ggt       192
Thr Tyr Thr Ala Ala Asp Ile Ala Asp Thr Pro Leu Glu Asp Ile Gly
    50                  55                  60

ctt ccg ggg cgc tac ccg ttc acg cgc ggg ccc tac ccg acg atg tac       240
Leu Pro Gly Arg Tyr Pro Phe Thr Arg Gly Pro Tyr Pro Thr Met Tyr
65                  70                  75                  80

cgc agc cgc acc tgg acg atg cgc cag atc gcc ggc ttc ggc acc ggc       288
Arg Ser Arg Thr Trp Thr Met Arg Gln Ile Ala Gly Phe Gly Thr Gly
                85                  90                  95

gag gac acc aac aag cgc ttc aag tat ctg atc gcg cag ggc cag acc       336
Glu Asp Thr Asn Lys Arg Phe Lys Tyr Leu Ile Ala Gln Gly Gln Thr
            100                 105                 110

ggc atc tcc acc gac ttc gac atg ccc acg ctg atg ggc tac gac tcc       384
Gly Ile Ser Thr Asp Phe Asp Met Pro Thr Leu Met Gly Tyr Asp Ser
        115                 120                 125

gac cac ccg atg agc gac ggc gag gtc ggc cgc gag ggc gtg gcg atc       432
Asp His Pro Met Ser Asp Gly Glu Val Gly Arg Glu Gly Val Ala Ile
    130                 135                 140

gac acg ctg gcc gac atg gag gcg ctg ctg gcc gac atc gac ctc gag       480
Asp Thr Leu Ala Asp Met Glu Ala Leu Leu Ala Asp Ile Asp Leu Glu
145                 150                 155                 160

aag atc tcg gtc tcg ttc acg atc aac ccg agc gcc tgg atc ctg ctc       528
Lys Ile Ser Val Ser Phe Thr Ile Asn Pro Ser Ala Trp Ile Leu Leu
                165                 170                 175

gcg atg tac gtg gcg ctc ggc gag aag cgc ggc tac gac ctg aac aag       576
Ala Met Tyr Val Ala Leu Gly Glu Lys Arg Gly Tyr Asp Leu Asn Lys
            180                 185                 190

ctg tcg ggc acg gtg cag gcc gac atc ctg aag gag tac atg gcg cag       624

Leu Ser Gly Thr Val Gln Ala Asp Ile Leu Lys Glu Tyr Met Ala Gln
        195                 200                 205

aag gag tac atc tac ccg atc gcg ccg tcg gtg cgc atc gtg cgc gac        672
Lys Glu Tyr Ile Tyr Pro Ile Ala Pro Ser Val Arg Ile Val Arg Asp
    210                 215                 220

atc atc acc tac agc gcg aag aac ctg aag cgc tac aac ccg atc aac        720
Ile Ile Thr Tyr Ser Ala Lys Asn Leu Lys Arg Tyr Asn Pro Ile Asn
225                 230                 235                 240

atc tcg ggc tac cac atc agc gag gcc ggc tcc tcg ccg ctc cag gag        768
Ile Ser Gly Tyr His Ile Ser Glu Ala Gly Ser Ser Pro Leu Gln Glu
                245                 250                 255

gcg gcc ttc acg ctg gcc aac ctg atc acc tac gtg aac gag gtg acg        816
Ala Ala Phe Thr Leu Ala Asn Leu Ile Thr Tyr Val Asn Glu Val Thr
            260                 265                 270

aag acc ggt atg cac gtc gac gaa ttc gcg ccg cgg ttg gcc ttc ttc        864
Lys Thr Gly Met His Val Asp Glu Phe Ala Pro Arg Leu Ala Phe Phe
        275                 280                 285

ttc gtg tcg caa ggt gac ttc ttc gag gag gtc gcg aag ttc cgc gcc        912
Phe Val Ser Gln Gly Asp Phe Phe Glu Glu Val Ala Lys Phe Arg Ala
    290                 295                 300

ctg cgc cgc tgc tac gcg aag atc atg aag gag cgc ttc ggt gca aga        960
Leu Arg Arg Cys Tyr Ala Lys Ile Met Lys Glu Arg Phe Gly Ala Arg
305                 310                 315                 320

aat ccg gaa tcg atg cgg ttg cgc ttc cac tgt cag acc gcg gcg gcg       1008
Asn Pro Glu Ser Met Arg Leu Arg Phe His Cys Gln Thr Ala Ala Ala
                325                 330                 335

acg ctg acc aag ccg cag tac atg gtc aac gtc gtg cgt acg tcg ctg       1056
Thr Leu Thr Lys Pro Gln Tyr Met Val Asn Val Val Arg Thr Ser Leu
            340                 345                 350

cag gcg ctg tcg gcc gtg ctc ggc ggc gcg cag tcg ctg cac acc aac       1104
Gln Ala Leu Ser Ala Val Leu Gly Gly Ala Gln Ser Leu His Thr Asn
        355                 360                 365

ggc tac gac gaa gcc ttc gcg atc ccg acc gag gat gcg atg aag atg       1152
Gly Tyr Asp Glu Ala Phe Ala Ile Pro Thr Glu Asp Ala Met Lys Met
    370                 375                 380

gcg ctg cgc acg cag cag atc att gcc gag gag agt ggt gtc gcc gac       1200
Ala Leu Arg Thr Gln Gln Ile Ile Ala Glu Glu Ser Gly Val Ala Asp
385                 390                 395                 400

gtg atc gac ccg ctg ggt ggc agc tac tac gtc gag gcg ctg acc acc       1248
Val Ile Asp Pro Leu Gly Gly Ser Tyr Tyr Val Glu Ala Leu Thr Thr
                405                 410                 415

gag tac gag aag aag atc ttc gag atc ctc gag gaa gtc gag aag cgc       1296
Glu Tyr Glu Lys Lys Ile Phe Glu Ile Leu Glu Glu Val Glu Lys Arg
            420                 425                 430

ggt ggc acc atc aag ctg atc gag cag ggc tgg ttc cag aag cag att       1344
Gly Gly Thr Ile Lys Leu Ile Glu Gln Gly Trp Phe Gln Lys Gln Ile
        435                 440                 445

gcg gac ttc gct tac gag acc gcg ctg cgc aag cag tcc ggc cag aag      1392
Ala Asp Phe Ala Tyr Glu Thr Ala Leu Arg Lys Gln Ser Gly Gln Lys
    450                 455                 460

ccg gtg atc ggg gtg aac cgc ttc gtc gag aac gaa gag gac gtc aag      1440
Pro Val Ile Gly Val Asn Arg Phe Val Glu Asn Glu Glu Asp Val Lys
465                 470                 475                 480

atc gag atc cac ccg tac gac aac acg acg gcc gaa cgc cag att tcc      1488
Ile Glu Ile His Pro Tyr Asp Asn Thr Thr Ala Glu Arg Gln Ile Ser
                485                 490                 495

cgc acg cgc cgc gtt cgc gcc gag cgc gac gag gcc aag gtg caa gcg      1536
Arg Thr Arg Arg Val Arg Ala Glu Arg Asp Glu Ala Lys Val Gln Ala
            500                 505                 510

atg ctc gac caa ctg gtg gct gtc gcc aag gac gag tcc cag aac ctg      1584
Met Leu Asp Gln Leu Val Ala Val Ala Lys Asp Glu Ser Gln Asn Leu
        515                 520                 525

atg ccg ctg acc atc gaa ctg gtg aag gcc ggc gca acg atg ggg gac      1632
Met Pro Leu Thr Ile Glu Leu Val Lys Ala Gly Ala Thr Met Gly Asp
    530                 535                 540

atc gtc gag aag ctg aag ggg atc tgg ggt acc tac cgc gag acg ccg      1680
Ile Val Glu Lys Leu Lys Gly Ile Trp Gly Thr Tyr Arg Glu Thr Pro
545                 550                 555                 560

gtc ttc tga                                                          1689
Val Phe

<210>  78
<211>  562
<212>  PRT
<213>  Aquincola tertiaricarbonis L108

<400>  78

Met Thr Trp Leu Glu Pro Gln Ile Lys Ser Gln Leu Gln Ser Glu Arg
1               5                   10                  15

Lys Asp Trp Glu Ala Asn Glu Val Gly Ala Phe Leu Lys Lys Ala Pro
            20                  25                  30

Glu Arg Lys Glu Gln Phe His Thr Ile Gly Asp Phe Pro Val Gln Arg
        35                  40                  45

Thr Tyr Thr Ala Ala Asp Ile Ala Asp Thr Pro Leu Glu Asp Ile Gly
    50                  55                  60

Leu Pro Gly Arg Tyr Pro Phe Thr Arg Gly Pro Tyr Pro Thr Met Tyr
65                  70                  75                  80

Arg Ser Arg Thr Trp Thr Met Arg Gln Ile Ala Gly Phe Gly Thr Gly
                85                  90                  95

Glu Asp Thr Asn Lys Arg Phe Lys Tyr Leu Ile Ala Gln Gly Gln Thr
        100                 105                 110

Gly Ile Ser Thr Asp Phe Asp Met Pro Thr Leu Met Gly Tyr Asp Ser
    115                 120                 125

Asp His Pro Met Ser Asp Gly Glu Val Gly Arg Glu Gly Val Ala Ile
    130                 135                 140

Asp Thr Leu Ala Asp Met Glu Ala Leu Leu Ala Asp Ile Asp Leu Glu
145                 150                 155                 160

Lys Ile Ser Val Ser Phe Thr Ile Asn Pro Ser Ala Trp Ile Leu Leu
                165                 170                 175

Ala Met Tyr Val Ala Leu Gly Glu Lys Arg Gly Tyr Asp Leu Asn Lys
            180                 185                 190

Leu Ser Gly Thr Val Gln Ala Asp Ile Leu Lys Glu Tyr Met Ala Gln
        195                 200                 205

Lys Glu Tyr Ile Tyr Pro Ile Ala Pro Ser Val Arg Ile Val Arg Asp
    210                 215                 220

Ile Ile Thr Tyr Ser Ala Lys Asn Leu Lys Arg Tyr Asn Pro Ile Asn
225                 230                 235                 240

Ile Ser Gly Tyr His Ile Ser Glu Ala Gly Ser Ser Pro Leu Gln Glu
                245                 250                 255

Ala Ala Phe Thr Leu Ala Asn Leu Ile Thr Tyr Val Asn Glu Val Thr
            260                 265                 270

Lys Thr Gly Met His Val Asp Glu Phe Ala Pro Arg Leu Ala Phe Phe
        275                 280                 285

Phe Val Ser Gln Gly Asp Phe Phe Glu Glu Val Ala Lys Phe Arg Ala
    290                 295                 300

Leu Arg Arg Cys Tyr Ala Lys Ile Met Lys Glu Arg Phe Gly Ala Arg
305                 310                 315                 320

Asn Pro Glu Ser Met Arg Leu Arg Phe His Cys Gln Thr Ala Ala Ala
                325                 330                 335

Thr Leu Thr Lys Pro Gln Tyr Met Val Asn Val Val Arg Thr Ser Leu
            340                 345                 350

Gln Ala Leu Ser Ala Val Leu Gly Gly Ala Gln Ser Leu His Thr Asn
        355                 360                 365

Gly Tyr Asp Glu Ala Phe Ala Ile Pro Thr Glu Asp Ala Met Lys Met
    370                 375                 380

Ala Leu Arg Thr Gln Gln Ile Ile Ala Glu Glu Ser Gly Val Ala Asp
385                 390                 395                 400

Val Ile Asp Pro Leu Gly Gly Ser Tyr Tyr Val Glu Ala Leu Thr Thr
                405                 410                 415

Glu Tyr Glu Lys Lys Ile Phe Glu Ile Leu Glu Glu Val Glu Lys Arg
            420                 425                 430

Gly Gly Thr Ile Lys Leu Ile Glu Gln Gly Trp Phe Gln Lys Gln Ile
        435                 440                 445

Ala Asp Phe Ala Tyr Glu Thr Ala Leu Arg Lys Gln Ser Gly Gln Lys
    450                 455                 460

Pro Val Ile Gly Val Asn Arg Phe Val Glu Asn Glu Glu Asp Val Lys
465                 470                 475                 480

Ile Glu Ile His Pro Tyr Asp Asn Thr Thr Ala Glu Arg Gln Ile Ser
                485                 490                 495

Arg Thr Arg Arg Val Arg Ala Glu Arg Asp Glu Ala Lys Val Gln Ala
            500                 505                 510

Met Leu Asp Gln Leu Val Ala Val Ala Lys Asp Glu Ser Gln Asn Leu
        515                 520                 525

Met Pro Leu Thr Ile Glu Leu Val Lys Ala Gly Ala Thr Met Gly Asp
    530                 535                 540

Ile Val Glu Lys Leu Lys Gly Ile Trp Gly Thr Tyr Arg Glu Thr Pro
545                 550                 555                 560

Val Phe

<210>  79
<211>  411
<212>  DNA
<213>  Aquincola tertiaricarbonis L108

<220>
<221>  CDS
<222>  (1)..(411)

<400>  79
atg gac caa atc ccg atc cgc gtt ctt ctc gcc aaa gtc ggc ctc gac          48
Met Asp Gln Ile Pro Ile Arg Val Leu Leu Ala Lys Val Gly Leu Asp
1               5                   10                  15

ggc cat gac cga ggc gtc aag gtc gtc gct cgc gcg ctg cgc gac gcc          96
Gly His Asp Arg Gly Val Lys Val Val Ala Arg Ala Leu Arg Asp Ala
            20                  25                  30

ggc atg gac gtc atc tac tcc ggc ctt cat cgc acg ccc gaa gag gtg          144
Gly Met Asp Val Ile Tyr Ser Gly Leu His Arg Thr Pro Glu Glu Val
        35                  40                  45

gtc aac acc gcc atc cag gaa gac gtg gac gtg ctg ggt gta agc ctc          192
Val Asn Thr Ala Ile Gln Glu Asp Val Asp Val Leu Gly Val Ser Leu
    50                  55                  60

ctg tcc ggc gtg cag ctc acg gtc ttc ccc aag atc ttc aag ctc ctg          240
Leu Ser Gly Val Gln Leu Thr Val Phe Pro Lys Ile Phe Lys Leu Leu
65                  70                  75                  80

gac gag aga ggc gct ggc gac ttg atc gtg atc gcc ggt ggc gtg atg          288
Asp Glu Arg Gly Ala Gly Asp Leu Ile Val Ile Ala Gly Gly Val Met
                85                  90                  95

ccg gac gag gac gcc gcg gcc atc cgc aag ctc ggc gtg cgc gag gtg          336
Pro Asp Glu Asp Ala Ala Ala Ile Arg Lys Leu Gly Val Arg Glu Val
            100                 105                 110

ctc ctg cag gac acg ccc ccg cag gcc atc atc gac tcg atc cgc tcc          384
Leu Leu Gln Asp Thr Pro Pro Gln Ala Ile Ile Asp Ser Ile Arg Ser
        115                 120                 125

ttg gtc gcc gcg cgc ggc gcc cgc tga                                      411
Leu Val Ala Ala Arg Gly Ala Arg
    130                 135


<210>  80
<211>  136
<212>  PRT
<213>  Aquincola tertiaricarbonis L108

<400>  80

Met Asp Gln Ile Pro Ile Arg Val Leu Leu Ala Lys Val Gly Leu Asp
1               5                   10                  15


Gly His Asp Arg Gly Val Lys Val Val Ala Arg Ala Leu Arg Asp Ala
            20                  25                  30


Gly Met Asp Val Ile Tyr Ser Gly Leu His Arg Thr Pro Glu Glu Val
        35                  40                  45


Val Asn Thr Ala Ile Gln Glu Asp Val Asp Val Leu Gly Val Ser Leu

50                  55                  60

Leu Ser Gly Val Gln Leu Thr Val Phe Pro Lys Ile Phe Lys Leu Leu
65                  70                  75                  80

Asp Glu Arg Gly Ala Gly Asp Leu Ile Val Ile Ala Gly Gly Val Met
                85                  90                  95

Pro Asp Glu Asp Ala Ala Ala Ile Arg Lys Leu Gly Val Arg Glu Val
            100                 105                 110

Leu Leu Gln Asp Thr Pro Pro Gln Ala Ile Ile Asp Ser Ile Arg Ser
        115                 120                 125

Leu Val Ala Ala Arg Gly Ala Arg
    130                 135

<210>  81
<211>  984
<212>  DNA
<213>  Aquincola tertiaricarbonis L108

<220>
<221>  CDS
<222>  (1)..(984)

<400>  81
atg act tac gtt ccc tca tcc gcc ttg ctc gag caa ctc cga gcc ggc      48
Met Thr Tyr Val Pro Ser Ser Ala Leu Leu Glu Gln Leu Arg Ala Gly
1               5                   10                  15

aat acc tgg gcg ctt ggc cgc ctg atc tcg cgc gcc gag gcc ggt gtg      96
Asn Thr Trp Ala Leu Gly Arg Leu Ile Ser Arg Ala Glu Ala Gly Val
            20                  25                  30

gcc gag gcg cgg cca gca ttg gcc gag gtc tat cgg cac gcc ggc tcg      144
Ala Glu Ala Arg Pro Ala Leu Ala Glu Val Tyr Arg His Ala Gly Ser
        35                  40                  45

gcg cat gtg atc ggt ctc acc ggg gtg ccg ggg agt ggc aag tcg act      192
Ala His Val Ile Gly Leu Thr Gly Val Pro Gly Ser Gly Lys Ser Thr
    50                  55                  60

ctc gtg gcg aag ctc acg gcc gcc ctg cgc aag cgt ggt gaa aag gtc      240
Leu Val Ala Lys Leu Thr Ala Ala Leu Arg Lys Arg Gly Glu Lys Val
65                  70                  75                  80

ggc atc gtc gca atc gat ccg tcg agc ccg tac tcg ggc ggt gcg atc      288
Gly Ile Val Ala Ile Asp Pro Ser Ser Pro Tyr Ser Gly Gly Ala Ile
                85                  90                  95

ctc ggc gac cgt atc cga atg acc gaa ctc gcc aac gat tcc ggc gta      336
Leu Gly Asp Arg Ile Arg Met Thr Glu Leu Ala Asn Asp Ser Gly Val
            100                 105                 110

ttc atc cgc agc atg gcc acg cgc ggc gcg acg ggg ggc atg gcg cgt      384

Phe Ile Arg Ser Met Ala Thr Arg Gly Ala Thr Gly Gly Met Ala Arg
        115                 120                 125

gcc gcc ctc gac gcc gtg gac ctg ctg gat gtc gcc ggc tat cac acc 432
Ala Ala Leu Asp Ala Val Asp Leu Leu Asp Val Ala Gly Tyr His Thr
    130                 135                 140

atc atc ctg gag act gtc gga gtc ggt cag gac gag gtg gag gtg gcg 480
Ile Ile Leu Glu Thr Val Gly Val Gly Gln Asp Glu Val Glu Val Ala
145                 150                 155                 160

cac gca tcg gac acg aca gtc gtc gta tcg gcg cca ggc ctt gga gac 528
His Ala Ser Asp Thr Thr Val Val Val Ser Ala Pro Gly Leu Gly Asp
                165                 170                 175

gag atc cag gcc atc aaa gcc ggc gtc ctg gaa atc gcc gac atc cat 576
Glu Ile Gln Ala Ile Lys Ala Gly Val Leu Glu Ile Ala Asp Ile His
            180                 185                 190

gtt gtc agc aag tgt gac cgc gac gac gcg aat cgc acg ctc acc gat 624
Val Val Ser Lys Cys Asp Arg Asp Asp Ala Asn Arg Thr Leu Thr Asp
        195                 200                 205

ctc aag cag atg ctg acg ctc ggc acc atg gtc ggg ccc aag cgc gca 672
Leu Lys Gln Met Leu Thr Leu Gly Thr Met Val Gly Pro Lys Arg Ala
    210                 215                 220

tgg gcg atc ccg gtc gtc ggt gtc agt tcg tac aca ggc gaa ggc gtc 720
Trp Ala Ile Pro Val Val Gly Val Ser Ser Tyr Thr Gly Glu Gly Val
225                 230                 235                 240

gac gac ctg ctc ggt cgc atc gcc gcc cac cgc cag gcg acg gcc gac 768
Asp Asp Leu Leu Gly Arg Ile Ala Ala His Arg Gln Ala Thr Ala Asp
                245                 250                 255

acc gaa ctc ggc cgc gaa cgg cgc cgt cgc gta gcc gaa ttc cgc ctt 816
Thr Glu Leu Gly Arg Glu Arg Arg Arg Arg Val Ala Glu Phe Arg Leu
            260                 265                 270

cag aag acc gcc gag acg ctg ctc ctg gag cga ttc acc acc gga gcg 864
Gln Lys Thr Ala Glu Thr Leu Leu Leu Glu Arg Phe Thr Thr Gly Ala
        275                 280                 285

cag ccc ttc tcg cct gcg ctc gca gac agc ctc agc aac cgt gcg tcg 912
Gln Pro Phe Ser Pro Ala Leu Ala Asp Ser Leu Ser Asn Arg Ala Ser
    290                 295                 300

gat ccc tac gcc gca gca cgc gaa ctc atc gcc cga acg atc cgc aag 960
Asp Pro Tyr Ala Ala Ala Arg Glu Leu Ile Ala Arg Thr Ile Arg Lys
305                 310                 315                 320

gag tac tcg aat gac ctg gct tga 984
Glu Tyr Ser Asn Asp Leu Ala
                325

<210> 82
<211> 327
<212> PRT
<213> Aquincola tertiaricarbonis L108

<400> 82

Met Thr Tyr Val Pro Ser Ser Ala Leu Leu Glu Gln Leu Arg Ala Gly
1               5                   10                  15

Asn Thr Trp Ala Leu Gly Arg Leu Ile Ser Arg Ala Glu Ala Gly Val
            20                  25                  30

Ala Glu Ala Arg Pro Ala Leu Ala Glu Val Tyr Arg His Ala Gly Ser
        35                  40                  45

Ala His Val Ile Gly Leu Thr Gly Val Pro Gly Ser Gly Lys Ser Thr
    50                  55                  60

Leu Val Ala Lys Leu Thr Ala Ala Leu Arg Lys Arg Gly Glu Lys Val
65                  70                  75                  80

Gly Ile Val Ala Ile Asp Pro Ser Ser Pro Tyr Ser Gly Gly Ala Ile
                85                  90                  95

Leu Gly Asp Arg Ile Arg Met Thr Glu Leu Ala Asn Asp Ser Gly Val
            100                 105                 110

Phe Ile Arg Ser Met Ala Thr Arg Gly Ala Thr Gly Gly Met Ala Arg
        115                 120                 125

Ala Ala Leu Asp Ala Val Asp Leu Leu Asp Val Ala Gly Tyr His Thr
    130                 135                 140

Ile Ile Leu Glu Thr Val Gly Val Gly Gln Asp Glu Val Glu Val Ala
145                 150                 155                 160

His Ala Ser Asp Thr Thr Val Val Val Ser Ala Pro Gly Leu Gly Asp
                165                 170                 175

Glu Ile Gln Ala Ile Lys Ala Gly Val Leu Glu Ile Ala Asp Ile His
            180                 185                 190

Val Val Ser Lys Cys Asp Arg Asp Asp Ala Asn Arg Thr Leu Thr Asp
        195                 200                 205

Leu Lys Gln Met Leu Thr Leu Gly Thr Met Val Gly Pro Lys Arg Ala
    210                 215                 220

Trp Ala Ile Pro Val Val Gly Val Ser Ser Tyr Thr Gly Glu Gly Val
225                 230                 235                 240

Asp Asp Leu Leu Gly Arg Ile Ala Ala His Arg Gln Ala Thr Ala Asp
                245                 250                 255

Thr Glu Leu Gly Arg Glu Arg Arg Arg Arg Val Ala Glu Phe Arg Leu
            260                 265                 270

Gln Lys Thr Ala Glu Thr Leu Leu Leu Glu Arg Phe Thr Thr Gly Ala
        275                 280                 285

Gln Pro Phe Ser Pro Ala Leu Ala Asp Ser Leu Ser Asn Arg Ala Ser
    290                 295                 300

Asp Pro Tyr Ala Ala Ala Arg Glu Leu Ile Ala Arg Thr Ile Arg Lys
305                 310                 315                 320

Glu Tyr Ser Asn Asp Leu Ala
                325

<210>  83
<211>  9837
<212>  DNA
<213>  Artificial Sequence

<220>
<223>  vector

<400>  83
ctcgggccgt ctcttgggct tgatcggcct tcttgcgcat ctcacgcgct cctgcggcgg      60

cctgtagggc aggctcatac ccctgccgaa ccgcttttgt cagccggtcg gccacggctt     120

ccggcgtctc aacgcgcttt gagattccca gcttttcggc caatccctgc ggtgcatagg     180

cgcgtggctc gaccgcttgc gggctgatgg tgacgtggcc cactggtggc cgctccaggg     240

cctcgtagaa cgcctgaatg cgcgtgtgac gtgccttgct gccctcgatg ccccgttgca     300

gccctagatc ggccacagcg gccgcaaacg tggtctggtc gcgggtcatc tgcgctttgt     360

tgccgatgaa ctccttggcc gacagcctgc cgtcctgcgt cagcggcacc acgaacgcgg     420

tcatgtgcgg gctggtttcg tcacggtgga tgctggccgt cacgatgcga tccgccccgt     480

acttgtccgc cagccacttg tgcgccttct cgaagaacgc cgcctgctgt tcttggctgg     540

ccgacttcca ccattccggg ctggccgtca tgacgtactc gaccgccaac acagcgtcct     600

tgcgccgctt ctctggcagc aactcgcgca gtcggcccat cgcttcatcg gtgctgctgg     660

ccgcccagtg ctcgttctct ggcgtcctgc tggcgtcagc gttgggcgtc tcgcgctcgc     720

ggtaggcgtg cttgagactg gccgccacgt tgcccatttt cgccagcttc ttgcatcgca     780

tgatcgcgta tgccgccatg cctgcccctc ccttttggtg tccaaccggc tcgacggggg     840

cagcgcaagg cggtgcctcc ggcgggccac tcaatgcttg agtatactca ctagactttg     900

cttcgcaaag tcgtgaccgc ctacggcggc tgcggcgccc tacgggcttg ctctccgggc     960

ttcgccctgc gcggtcgctg cgctcccttg ccagcccgtg gatatgtgga cgatggccgc    1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaattttct ctggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag gggccacttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc agcaggctgc ctcgataagc 3240

aaaaagggcg gccccgcggg gccgcccttt ttctttccgg cgactgtcag gccacttaca 3300

ggatgacgac ggccttgatc aggtccttcg gcttgtcttt catcagcagg agggcctcct 3360

cgatgtggtc gaagccatgg tacacgtgcg tcaccagttt cgacaggtcg acgcgattat 3420

acaccaccat gtcgcgcagc atctccgcgc gcagccgccc gccggggcac aggccgccct 3480

taatggtctt atgggccatg ccgcagcccc attccacgcg ggggatgagc agcgcgtcgc 3540

ccgagccgtg gtagttgatg ttcgaaatga tcccgcccgg cttcaccatc gagacggcct 3600

gcgacagcgt ttcggacccc cccccggcca tgatgacgcg gtccacgccc ttgccgttgg 3660

tcagcttcat gacctggtcc acaatatggc cgttcttgta gttcaggatg tcggtggcgc 3720

cgtagaactt ggccgcctcc acgcagatcg gccgcgagcc cacgccgatg atgcggcccg 3780

ccccccgcag cttcgcgccc gcgatgccca tgagccccac cgccccgatg ccgatcacga 3840

cgaccgagct gcccatctgg atatccgcca gctcggcccc gtggaacccc gtggtcatca 3900

tatcggtgat catcaccgcg ttttccagcg gcatatcttt cggcaggatg gccaggttca 3960

tgtcggcgtc gttcacatga aagtactccc cgaacacgcc gtccttgaag ttcgagaact 4020

tccacccggc gagcatgccg ttcgaatgct gctggaaccc ggcctggacc tccagcgagc 4080

gccagtcggg ggtggtgcac ggcacgatca cccggtcgcc gggcttaaag tccttcacct 4140

ccgaccccac ctccaccacc tcgcccacgg cctcgtgccc caggatcata ttcttgcggt 4200

cccccagcgc gccctcaaac acggtgtgga tgtccgacgt acacggcgac acggcgagcg 4260

ggcgcacgat ggcgtcatac gagccggcca ccgggcgctc cttctcgatc cagcccagtt 4320

tgttgatgcc cagcatggcg aagcccttca ttcttgaatc tcctgaaaaa gtgagttttt 4380

gatcacaaat cggccggcta ccgaggcgac ggccgtcaga aatgttcagg tgtcgcagga 4440

ggctgttagc actcacccct gacgagtgct aattataggg acggggtagg ggcatttcaa 4500

gacgcgggtg tgatgcgggt tactagtgga tcccaatatt aaaaaaataa gagttaccat 4560

ttaaggtaac tcttattttt attacttcag gtagtcgtag atgacctccg cccggggcag 4620

gataatatcg gcgaggatat gcgaggacga cacgatttct ttcaccggca ggtcattcag 4680

gggcgccatc gcatggtcga agagctgcag gcgcgtgggg cccgtccagg cttcatgcac 4740

ggtcacgtcg gtgatcttcg cgttgatcag ttcgcaaatg cgcggggagc cgtcgtaatt 4800

ggggatgatt ttcagcatgt agttggggcg gcaaatctgg tccttcgctt cattcgcgtc 4860

cagcgccttg tgcttgtagc ccatggtggc ggtggcgacg cgcagtttgc cgtagtcgag 4920

ggtgccgacc agcgtgtccg aatccacaaa cagcttcgga tagcccagct tcttggggta 4980

ggcggacagc tcgcggccca cggcaatggc cggttcgttg tccaggtaca tcatgtgcag 5040

gtaatccccc ttcacgccat taaacgagac cggaatcgcc tggccgcttt cggtatagca 5100

gcccaggccg gaggtgtcat gcatcgccat aatctcgaag cgcaccaggg gctcatcgat 5160

ctccagcggt tccgggacga ccttgcggag ggcatccatg tcggtgcggt acacgatgtt 5220

gaagtactcg cggttgtgga acttgtaggg cccccgcgga aaggcggggg acgtcagcgg 5280

ggtgctgatt tgtttgatga cttcgtcctt gagcattctt gaatctcctg aaatgtgaaa 5340

ttgttatccg ctcacaattc cacacaacat acgagccgga agcataaagt gtaaagcctg 5400

gggaagcttt cgatgttcaa gaaaacaccc gataactttc gctatcgggt gtttttattg 5460

attagttgag gcgttcgatc accatggcga tgccctggcc cccgccgatg cacagggtgg 5520

ccaggcccag cgtcttatcc cgggcctgca tggcgtgcag cagggtcacc aggatgcgcg 5580

cgcccgaggc gccgatcgga tggcccaggg cgatcgcgcc gccgttcaca ttcaccttct 5640

ccgagtcgaa gcccaggttc ttgcccaccg ccaggaactg ggcggcgaag gcctcgttgg 5700

cctcgatcag gtcgatgtcc gccagttgca ggcccgccag ctgcagggcc ttctgggtgg 5760

ccggcaccgg gcccatgccc atcagggccg gcggcacccc gcccgaggcg tacgacttga 5820

tgcgggccag cggggtcagc cccgcggcca gcgcggccga ctcctccatg atcaccagcg 5880

ccgcggcgcc gtcgttgatg cccgaggcgt tgcccgcggt cacggtgccg gctttgtcga 5940

aggccgggcg cagggccccc agggcctcgg cggtcgagtt ggccttcggg aactcgtcct 6000

gcgagaacac gaaggtcttc ttgcgggtca ccacgttcac cggcacgatc tcggccgtga 6060

aggcgcccga ctcgatggcg gcggcggcct tgcgctgcga gtgcagggcc agctcgtcct 6120

gcatctcgcg ggtgatgccg tactccttgg ccacgttctc ggcggtgatg cccatgtggt 6180

agccgtgggt ggcgcacatg aggccgtcgc ggaggatcac gtcgtacacc tggccgtccc 6240

ccagccgata gcccgagcgg gccttggcgt ccagcaggta cggggccagc gacatgttct 6300

ccatgccgcc ggccacgatc gattgggctt gcccggcttg gatggcttgg gcggcgaggg 6360

cgaccgactt caggccggag ccgcacacct tgttcacggt gaagccgcac acggtctcgg 6420

cgaggcccga cttcaggagg gcctgccggg ccgggttctg gcccagcccg gcttgcagca 6480

cgttgcccat gatcacctcg tccacgtgtt gcgagtcgat cttggcgcgc tcgatggccg 6540

ccttgatcac ggtggccccc aggtcgatgg ccgaggtcga ggcgagcgag ccgttgaacg 6600

agccgatcgc ggtgcggacg gccgacacga tcacgcagtt cttcatttta tattccttct 6660

gtttgtaggg gtgccgtcac aggtcgccgc gctgggtgtt caggtcggcg gccacctcga 6720

agcgggcctc ggtcttggcg cgcacggtcg cgaggtcgca gccgtcggcg atctcggtca 6780

gccacatctt gccgtcgatg aagcggaaca cggccagttc ggtcaccagc atgtgcacgg 6840

cgtgctgggc cgtcagcggc atggtgcagc ggcgcaggat cttggccgag ccatccttgg 6900

cgcagtgctc catcgcaatg atcaccttgc gcgagccggt cacgaggtcc atggcccccc 6960

ccatgcccgg gaccattttg cccggcacga cccagttggc caggttggcc tcctcgtcca 7020

cctggagccc gccgaggacg caggcgtcga tgtgcccccc ccggatcagg gcgaacgaca 7080

tggccgagtc gaacatcgcg gcgcccggca gcaccccgca cggctggccc cccgcgttca 7140

ccaggtccgg gtgggcggtg gtcaccgggc ccaggcccag gaagccgttc tccgactgca 7200

gggtgatgtg gatgccctcc ggcagatagt tggccaccat ggtcggcagg ccgatgccca 7260

ggttcacgat gtcgccgtcg cgcagttcct gcgcgacgcg gcgggcgatg cgctgcttgg 7320

cgtccattac ttcgactctt gggagacgat gatatggtcg atcaccgcgc cgggggtcac 7380

gatatgatcc ggttgcagct ccccggtttc gacgagttcg tccggttcca cgagggtgat 7440

atccgcggcc agcgcaatca gcgggttgaa gttccgggcg gacagctggt acgtcaggtt 7500

gcccagggtg tcgcagcgat gggcgcgaat cagggccaga tcggcgcgca gcgggcgttc 7560

cagcagccag gtcttcccgt ccagggtcag cgtctgcttg ccttcctcga ccacggtgcc 7620

cacgccggtc ggcgtcagga acccgccgag gcccgccccg ccgcaccgga tttgctcaat 7680

gagggtcccc tgcgggacca gcaccacatc catctcgccc gagatcatcc ggcggccggt 7740

ctccgggttc gtgccgatgt ggctggcgat cactttgcgg acgcggccgt tgacaatcag 7800

ggggccaatg ccggtgtcca cgaaggcggt gtcgttggca atgagcgtca gatcgcgcac 7860

ccccgactcc aggagggctt cgaccagccg cgacggcgtc ccgatgccca tgaagccccc 7920

caccatgatg gtcatgccat cgcggaaaaa gccggtggcg tcctgcagcg tcatgagctt 7980

ggtcttcatt ttttatccct cttgcatacg gtacccagct tttgttccct ttagtgaggg 8040

ttaattgcgc gcttggcgta atcatggtca tagctgtttc ctgtgtgaaa ttgttatccg 8100

ctcacaattc cacacaacat acgagccgga agcataaagt gtaaagcctg gggtgcctaa 8160

tgagtgagct aactcacatt aattgcgttg cgctcactgc ccgctttcca gtcgggaaac 8220

ctgtcgtgcc agctgcatta atgaatcggc caacgcgcgg ggagaggcgg tttgcgtatt 8280

gggcgcatgc ataaaaactg ttgtaattca ttaagcattc tgccgacatg gaagccatca 8340

caaacggcat gatgaacctg aatcgccagc ggcatcagca ccttgtcgcc ttgcgtataa 8400

tatttgccca tgggggtggg cgaagaactc cagcatgaga tccccgcgct ggaggatcat 8460

ccagccggcg tcccggaaaa cgattccgaa gcccaacctt tcatagaagg cggcggtgga 8520

atcgaaatct cgtgatggca ggttgggcgt cgcttggtcg gtcatttcga accccagagt 8580

cccgctcaga agaactcgtc aagaaggcga tagaaggcga tgcgctgcga atcgggagcg 8640

gcgataccgt aaagcacgag gaagcggtca gcccattcgc cgccaagctc ttcagcaata 8700

tcacgggtag ccaacgctat gtcctgatag cggtccgcca cacccagccg gccacagtcg 8760

atgaatccag aaaagcggcc attttccacc atgatattcg gcaagcaggc atcgccatgg 8820

gtcacgacga gatcctcgcc gtcgggcatg cgcgccttga gcctggcgaa cagttcggct 8880

ggcgcgagcc cctgatgctc ttcgtccaga tcatcctgat cgacaagacc ggcttccatc 8940

cgagtacgtg ctcgctcgat gcgatgtttc gcttggtggt cgaatgggca ggtagccgga 9000

tcaagcgtat gcagccgccg cattgcatca gccatgatgg atactttctc ggcaggagca 9060

aggtgagatg acaggagatc ctgccccggc acttcgccca atagcagcca gtcccttccc 9120

gcttcagtga caacgtcgag cacagctgcg caaggaacgc ccgtcgtggc cagccacgat 9180

agccgcgctg cctcgtcctg cagttcattc agggcaccgg acaggtcggt cttgacaaaa 9240

agaaccgggc gcccctgcgc tgacagccgg aacacggcgg catcagagca gccgattgtc 9300

tgttgtgccc agtcatagcc gaatagcctc tccacccaag cggccggaga acctgcgtgc 9360

aatccatctt gttcaatcat gcgaaacgat cctcatcctg tctcttgatc agatcttgat 9420

cccctgcgcc atcagatcct tggcggcaag aaagccatcc agtttacttt gcagggcttc 9480

ccaaccttac cagagggcgc cccagctggc aattccggtt cgcttgctgt ccataaaacc 9540

gcccagtcta gctatcgcca tgtaagccca ctgcaagcta cctgctttct ctttgcgctt 9600

gcgttttccc ttgtccagat agcccagtag ctgacattca tcccaggtgg cacttttcgg 9660

ggaaatgtgc gcgcccgcgt tcctgctggc gctgggcctg tttctggcgc tggacttccc 9720

gctgttccgt cagcagcttt tcgcccacgg ccttgatgat cgcggcggcc ttggcctgca 9780

tatcccgatt caacggcccc agggcgtcca gaacgggctt caggcgctcc cgaaggt 9837

<210> 84
<211> 9867
<212> DNA
<213> Artificial Sequence

<220>
<223> vector

<400> 84
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360
tgccgatgaa ctccttggcc gacagcctgc cgtcctgcgt cagcggcacc acgaacgcgg 420
tcatgtgcgg gctggtttcg tcacggtgga tgctggccgt cacgatgcga tccgccccgt 480
acttgtccgc cagccacttg tgcgccttct cgaagaacgc cgcctgctgt tcttggctgg 540
ccgacttcca ccattccggg ctggccgtca tgacgtactc gaccgccaac acagcgtcct 600
tgcgccgctt ctctggcagc aactcgcgca gtcggcccat cgcttcatcg gtgctgctgg 660
ccgcccagtg ctcgttctct ggcgtcctgc tggcgtcagc gttgggcgtc tcgcgctcgc 720
ggtaggcgtg cttgagactg gccgccacgt tgcccatttt cgccagcttc ttgcatcgca 780
tgatcgcgta tgccgccatg cctgcccctc ccttttggtg tccaaccggc tcgacggggg 840
cagcgcaagg cggtgcctcc ggcgggccac tcaatgcttg agtatactca ctagactttg 900
cttcgcaaag tcgtgaccgc ctacggcggc tgcggcgccc tacgggcttg ctctccgggc 960
ttcgccctgc gcggtcgctg cgctcccttg ccagcccgtg gatatgtgga cgatggccgc 1020
gagcggccac cggctggctc gcttcgctcg gcccgtggac aaccctgctg gacaagctga 1080
tggacaggct gcgcctgccc acgagcttga ccacagggat tgcccaccgg ctacccagcc 1140
ttcgaccaca tacccaccgg ctccaactgc gcggcctgcg gccttgcccc atcaattttt 1200
ttaattttct ctggggaaaa gcctccggcc tgcggcctgc gcgcttcgct tgccggttgg 1260
acaccaagtg gaaggcgggt caaggctcgc gcagcgaccg cgcagcggct tggccttgac 1320
gcgcctggaa cgacccaagc ctatgcgagt gggggcagtc gaaggcgaag cccgcccgcc 1380
tgccccccga gcctcacggc ggcgagtgcg ggggttccaa gggggcagcg ccaccttggg 1440
caaggccgaa ggccgcgcag tcgatcaaca agccccggag gggccacttt ttgccggagg 1500
gggagccgcg ccgaaggcgt gggggaaccc cgcaggggtg cccttctttg ggcaccaaag 1560
aactagatat agggcgaaat gcgaaagact taaaaatcaa caacttaaaa aaggggggta 1620
cgcaacagct cattgcggca ccccccgcaa tagctcattg cgtaggttaa agaaaatctg 1680
taattgactg ccacttttac gcaacgcata attgttgtcg cgctgccgaa aagttgcagc 1740
tgattgcgca tggtgccgca accgtgcggc accctaccgc atggagataa gcatggccac 1800
gcagtccaga gaaatcggca ttcaagccaa gaacaagccc ggtcactggg tgcaaacgga 1860
acgcaaagcg catgaggcgt gggccgggct tattgcgagg aaacccacgg cggcaatgct 1920
gctgcatcac ctcgtggcgc agatgggcca ccagaacgcc gtggtggtca gccagaagac 1980
actttccaag ctcatcggac gttctttgcg gacggtccaa tacgcagtca aggacttggt 2040
ggccgagcgc tggatctccg tcgtgaagct caacggcccc ggcaccgtgt cggcctacgt 2100
ggtcaatgac cgcgtggcgt ggggccagcc ccgcgaccag ttgcgcctgt cggtgttcag 2160
tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc agcaggctgc ctcgataagc 3240

aaaaagggcg gccccgcggg gccgcccttt ttctttccgg cgactgtcag gccacttaca 3300

ggatgacgac ggccttgatc aggtccttcg gcttgtcttt catcagcagg agggcctcct 3360

cgatgtggtc gaagccatgg tacacgtgcg tcaccagttt cgacaggtcg acgcgattat 3420

acaccaccat gtcgcgcagc atctccgcgc gcagccgccc gccggggcac aggccgccct 3480

taatggtctt atgggccatg ccgcagcccc attccacgcg ggggatgagc agcgcgtcgc 3540

ccgagccgtg gtagttgatg ttcgaaatga tcccgcccgg cttcaccatc gagacggcct 3600

gcgacagcgt ttcggacccc cccccggcca tgatgacgcg gtccacgccc ttgccgttgg 3660

tcagcttcat gacctggtcc acaatatggc cgttcttgta gttcaggatg tcggtggcgc 3720

cgtagaactt ggccgcctcc acgcagatcg gccgcgagcc cacgccgatg atgcggcccg 3780

ccccccgcag cttcgcgccc gcgatgccca tgagccccac cgccccgatg ccgatcacga 3840

cgaccgagct gcccatctgg atatccgcca gctcggcccc gtggaacccc gtggtcatca 3900

tatcggtgat catcaccgcg ttttccagcg gcatatcttt cggcaggatg gccaggttca 3960

tgtcggcgtc gttcacatga aagtactccc cgaacacgcc gtccttgaag ttcgagaact 4020

tccacccggc gagcatgccg ttcgaatgct gctggaaccc ggcctggacc tccagcgagc 4080

gccagtcggg ggtggtgcac ggcacgatca cccggtcgcc gggcttaaag tccttcacct 4140

ccgaccccac ctccaccacc tcgcccacgg cctcgtgccc caggatcata ttcttgcggt 4200

cccccagcgc gccctcaaac acggtgtgga tgtccgacgt acacggcgac acggcgagcg 4260

ggcgcacgat ggcgtcatac gagccggcca ccgggcgctc cttctcgatc cagcccagtt 4320

tgttgatgcc cagcatggcg aagcccttca ttcttgaatc tcctgaaaaa gtgagttttt 4380

gatcacaaat cggccggcta ccgaggcgac ggccgtcaga aatgttcagg tgtcgcagga 4440

ggctgttagc actcacccct gacgagtgct aattataggg acggggtagg ggcatttcaa 4500

gacgcgggtg tgatgcgggt tactagtgga tcccaatatt aaaaaaataa gagttaccat 4560

ttaaggtaac tcttattttt attacttcag gtagtcgtag atgacctccg cccggggcag 4620

gataatatcg gcgaggatat gcgaggacga cacgatttct ttcaccggca ggtcattcag 4680

gggcgccatc gcatggtcga agagctgcag gcgcgtgggg cccgtccagg cttcatgcac 4740

ggtcacgtcg gtgatcttcg cgttgatcag ttcgcaaatg cgcggggagc cgtcgtaatt 4800

ggggatgatt ttcagcatgt agttgggcgg gcaaatctgg tccttcgctt cattcgcgtc 4860

cagcgccttg tgcttgtagc ccatggtggc ggtggcgacg cgcagtttgc cgtagtcgag 4920

ggtgccgacc agcgtgtccg aatccacaaa cagcttcgga tagcccagct tcttggggta 4980

ggcggacagc tcgcggccca cggcaatggc cggttcgttg tccaggtaca tcatgtgcag 5040

gtaatccccc ttcacgccat taaacgagac cggaatcgcc tggccgcttt cggtatagca 5100

gcccaggccg gaggtgtcat gcatcgccat aatctcgaag cgcaccaggg gctcatcgat 5160

ctccagcggt tccgggacga ccttgcggag ggcatccatg tcggtgcggt acacgatgtt 5220

gaagtactcg cggttgtgga acttgtaggg cccccgcgga aaggcggggg acgtcagcgg 5280

ggtgctgatt tgtttgatga cttcgtcctt gagcattctt gaatctcctg aaatgtgaaa 5340

ttgttatccg ctcacaattc cacacaacat acgagccgga agcataaagt gtaaagcctg 5400

gggaagctta ttatcttaag taataaaaat aagagttacc ttaaatggta actcttattt 5460

ttttaatatt gtttcatagt atttctttct acacggccat cgggcgcagc tcattgctga 5520

tgagcaggtc ggcggccgtc agcgagcgaa tttcgtcaat ggtggtgttc ttgttgattt 5580

cggtgaggag gaggccgtcg ttaatcactt cgatcacccc cagttcggtc acaatcaggt 5640

tcgcctgcga tttggcggtc agcggcaggg tgcacttctt caggatcttc ggttggccct 5700

tgttggtgtg gcgcatggca atgatgacct tcttggcccc gttcaccaga tccatggcgc 5760

cgcccatccc cgacagcatc ttgcccggca cgatccagtt ggcgatattg cccttctcgt 5820

ccacctgcag cgcgcccagc accgtgacat ccacatgccc gccccgaatg agcgagaacg 5880

acaccgacga gtcgaaaaag gtgccgtccg gcagcacggt ggtatagtcg ccccccgcat 5940

tcaccacgtc cttgtccgcc tcattgattt tcggctggcc ccccatgccc acaatgccat 6000

tttccgactg gaaggtgatc ttgaaattct tcgggatgta gtcggccacc atcgtgggca 6060

ggcccacgcc cagattgacc agctgcccgt ttttcagttc gcgggccacg cgcttcgcaa 6120

taatctcctt ggccaggttt ttgtcattaa tcattttagg cgggctcctt cacgatgtag 6180

ttgatgagca cgcccggcgt catggccttt tccttctcca gcttctcgca ggagaccagg 6240

ttttcggctt ccacgatcac ggttttggcg gccatcgcca tgtacgggtt gaagttcttg 6300

gtggtgccct tgtagaaggt gttgccggct tcgtcgacaa tcgagccttt aatcagggcc 6360

acgtcggcgg tcagcggcag ctccaggagg tattcggtcc cgttgatgga gatcttcttt 6420

ttgccttttt cgatgagggt ccccaggccg gtcttggtca ggaccccccc gaggcccgag 6480

ccccccgcgc gaatgcgctc caccagcgtg ccctgcggcg acagttccac ttccagctca 6540

ttgttgaaca gtttttcccc cgtgtccgga ttcgagccaa tgtacgaggc gatcagtttc 6600

ttcacctggt tgttcgagat gagcttgccg atgccggtgt tcgggtagca ggtatcgttg 6660

gagataatgg tgagattctt gatgttcagg ttcaccagaa aatcaatcag cttggtcggg 6720

gtgccgcagt tcagaaagcc cccaatcata atcgtcatgc cgtccttgaa gaacgagcgg 6780

aggttctcaa agcggatgat cttcgagttc attttaatcc ctccttttaa attccttatt 6840

tgcgctcgac tgccagcgcc acgcccatgc cgccgccgat gcacagcgag gccaggccct 6900

tcttcgcgtc acggcgcttc atctcgtgca gcagcgtcac caggatacgg cagcccgacg 6960

cgccgatcgg gtggccgatg gcgatggcgc cgccgttcac attgaccttg gaggtgtccc 7020

agcccatctg ctggtgcacc gccagcgcct gcgcggcaaa ggcctcgttg atctccatca 7080

ggtccaggtc ttgcggggtc cactcggcgc gcgacagggc gcgcttggag gccggcaccg 7140

ggcccatgcc catcaccttg ggatcgacac cggcgttggc atagctcttg atcgtggcca 7200

gcggggtcag gcccagttcc ttggccttgg ccgccgacat caccaccacc gcggcggcgc 7260

cgtcgttcag gcccgaggcg ttggccgcgg tcaccgtgcc ggccttgtcg aaggcgggct 7320

tgaggccgga catgctgtcc agcgtggcgc cctggcgcac gaactcgtcg gtcttgaagg 7380

ccaccgggtc gcccttgcgc tgcgggatca gcaccgggac gatctcttcg tcaaacttgc 7440

cggccttctg cgcggcttcg gccttgttct gcgagccgac ggcgaactca tcctgcgcct 7500

cgcgtgtgat gccgtattcc ttggccacgt tctcggcggt gatgcccatg tggtactggt 7560

tgtacacgtc ccacaggccg tcgacgatca tggtgtcgac cagcttggca tcgcccatgc 7620

ggaaaccatc gcgcgagccc ggcagcacgt gcggggcggc gctcatgttt tcctggccgc 7680

cggccaccac gatctcggcg tcgcccgcca tgatcgcgtt ggcggccagc atcacggcct 7740

tcaggcccga gccgcacacc ttgttgatgg tcatggccgg caccatcgcc ggcaggccgg 7800

ccttgatcgc ggcctggcgt gcggggttct ggcccgaacc ggcggtcagc acctggccca 7860

tgatgacttc gctcacctgc tccggcttga cgccggcgcg ctccagcgcg gccttgatga 7920

ccacggcacc cagttccggt gccgggatct tggccagcga gccgccaaac ttgccgaccg 7980

cggtgcgggc ggcggatacg atgacaacgt cagtcattgt gtagtccttt caatggaaag 8040

gtacccagct tttgttccct ttagtgaggg ttaattgcgc gcttggcgta atcatggtca 8100

tagctgtttc ctgtgtgaaa ttgttatccg ctcacaattc cacacaacat acgagccgga 8160

agcataaagt gtaaagcctg gggtgcctaa tgagtgagct aactcacatt aattgcgttg 8220

cgctcactgc ccgctttcca gtcgggaaac ctgtcgtgcc agctgcatta atgaatcggc 8280

caacgcgcgg ggagaggcgg tttgcgtatt gggcgcatgc ataaaaactg ttgtaattca 8340

ttaagcattc tgccgacatg gaagccatca caaacggcat gatgaacctg aatcgccagc 8400

ggcatcagca ccttgtcgcc ttgcgtataa tatttgccca tgggggtggg cgaagaactc 8460

cagcatgaga tccccgcgct ggaggatcat ccagccggcg tcccggaaaa cgattccgaa 8520

gcccaacctt tcatagaagg cggcggtgga atcgaaatct cgtgatggca ggttgggcgt 8580

cgcttggtcg gtcatttcga accccagagt cccgctcaga agaactcgtc aagaaggcga 8640

tagaaggcga tgcgctgcga atcgggagcg gcgataccgt aaagcacgag gaagcggtca 8700

gcccattcgc cgccaagctc ttcagcaata tcacgggtag ccaacgctat gtcctgatag 8760

cggtccgcca cacccagccg gccacagtcg atgaatccag aaaagcggcc attttccacc 8820

atgatattcg gcaagcaggc atcgccatgg gtcacgacga gatcctcgcc gtcgggcatg 8880

cgcgccttga gcctggcgaa cagttcggct ggcgcgagcc cctgatgctc ttcgtccaga 8940

tcatcctgat cgacaagacc ggcttccatc cgagtacgtg ctcgctcgat gcgatgtttc 9000

gcttggtggt cgaatgggca ggtagccgga tcaagcgtat gcagccgccg cattgcatca 9060

gccatgatgg atactttctc ggcaggagca aggtgagatg acaggagatc ctgccccggc 9120

acttcgccca atagcagcca gtcccttccc gcttcagtga caacgtcgag cacagctgcg 9180

caaggaacgc ccgtcgtggc cagccacgat agccgcgctg cctcgtcctg cagttcattc 9240

agggcaccgg acaggtcggt cttgacaaaa agaaccgggc gcccctgcgc tgacagccgg 9300

aacacggcgg catcagagca gccgattgtc tgttgtgccc agtcatagcc gaatagcctc 9360

tccacccaag cggccggaga acctgcgtgc aatccatctt gttcaatcat gcgaaacgat 9420

cctcatcctg tctcttgatc agatcttgat cccctgcgcc atcagatcct tggcggcaag 9480

aaagccatcc agtttacttt gcagggcttc ccaaccttac cagagggcgc cccagctggc 9540

aattccggtt cgcttgctgt ccataaaacc gcccagtcta gctatcgcca tgtaagccca 9600

ctgcaagcta cctgctttct ctttgcgctt gcgttttccc ttgtccagat agcccagtag 9660

ctgacattca tcccaggtgg cacttttcgg ggaaatgtgc gcgcccgcgt tcctgctggc 9720

gctgggcctg tttctggcgc tggacttccc gctgttccgt cagcagcttt tcgcccacgg 9780

ccttgatgat cgcggcggcc ttggcctgca tatcccgatt caacggcccc agggcgtcca 9840

gaacgggctt caggcgctcc cgaaggt 9867

<210> 85
<211> 9859
<212> DNA
<213> Artificial Sequence

<220>
<223> vector

<400> 85
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaattttct ctggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag gggccacttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc agcaggctgc ctcgataagc 3240

aaaaagggcg gccccgcggg gccgcccttt ttctttccgg cgactgtcag gccacttaca 3300

ggatgacgac ggccttgatc aggtccttcg gcttgtcttt catcagcagg agggcctcct 3360

cgatgtggtc gaagccatgg tacacgtgcg tcaccagttt cgacaggtcg acgcgattat 3420

acaccaccat gtcgcgcagc atctccgcgc gcagccgccc gccggggcac aggccgccct 3480

taatggtctt atgggccatg ccgcagcccc attccacgcg ggggatgagc agcgcgtcgc 3540

ccgagccgtg gtagttgatg ttcgaaatga tcccgcccgg cttcaccatc gagacggcct 3600

gcgacagcgt ttcggacccc cccccggcca tgatgacgcg gtccacgccc ttgccgttgg 3660

tcagcttcat gacctggtcc acaatatggc cgttcttgta gttcaggatg tcggtggcgc 3720

cgtagaactt ggccgcctcc acgcagatcg gccgcgagcc cacgccgatg atgcggcccg 3780

ccccccgcag cttcgcgccc gcgatgccca tgagccccac cgccccgatg ccgatcacga 3840

cgaccgagct gcccatctgg atatccgcca gctcggcccc gtggaacccc gtggtcatca 3900

tatcggtgat catcaccgcg ttttccagcg gcatatcttt cggcaggatg gccaggttca 3960

tgtcggcgtc gttcacatga aagtactccc cgaacacgcc gtccttgaag ttcgagaact 4020

tccacccggc gagcatgccg ttcgaatgct gctggaaccc ggcctggacc tccagcgagc 4080

gccagtcggg ggtggtgcac ggcacgatca cccggtcgcc gggcttaaag tccttcacct 4140

ccgaccccac ctccaccacc tcgcccacgg cctcgtgccc caggatcata ttcttgcggt 4200

cccccagcgc gccctcaaac acggtgtgga tgtccgacgt acacggcgac acggcgagcg 4260

ggcgcacgat ggcgtcatac gagccggcca ccgggcgctc cttctcgatc cagcccagtt 4320

tgttgatgcc cagcatggcg aagcccttca ttcttgaatc tcctgaaaaa gtgagttttt 4380

gatcacaaat cggccggcta ccgaggcgac ggccgtcaga aatgttcagg tgtcgcagga 4440

ggctgttagc actcacccct gacgagtgct aattataggg acggggtagg ggcatttcaa 4500

gacgcgggtg tgatgcgggt tactagtgga tcccaatatt aaaaaaataa gagttaccat 4560

ttaaggtaac tcttattttt attacttcag gtagtcgtag atgacctccg cccggggcag 4620

gataatatcg gcgaggatat gcgaggacga cacgatttct ttcaccggca ggtcattcag 4680

gggcgccatc gcatggtcga agagctgcag gcgcgtgggg cccgtccagg cttcatgcac 4740

ggtcacgtcg gtgatcttcg cgttgatcag ttcgcaaatg cgcggggagc cgtcgtaatt 4800

ggggatgatt ttcagcatgt agttggggcg gcaaatctgg tccttcgctt cattcgcgtc 4860

cagcgccttg tgcttgtagc ccatggtggc ggtggcgacg cgcagtttgc cgtagtcgag 4920

ggtgccgacc agcgtgtccg aatccacaaa cagcttcgga tagcccagct tcttggggta 4980

ggcggacagc tcgcggccca cggcaatggc cggttcgttg tccaggtaca tcatgtgcag 5040

gtaatccccc ttcacgccat taaacgagac cggaatcgcc tggccgcttt cggtatagca 5100

gcccaggccg gaggtgtcat gcatcgccat aatctcgaag cgcaccaggg gctcatcgat 5160

ctccagcggt tccgggacga ccttgcggag ggcatccatg tcggtgcggt acacgatgtt 5220

gaagtactcg cggttgtgga acttgtaggg cccccgcgga aaggcggggg acgtcagcgg 5280
ggtgctgatt tgtttgatga cttcgtcctt gagcattctt gaatctcctg aaatgtgaaa 5340
ttgttatccg ctcacaattc cacacaacat acgagccgga agcataaagt gtaaagcctg 5400
gggaagctta ttatcttaag taataaaaat aagagttacc ttaaatggta actcttattt 5460
ttttaatatt gtttcatagt atttctttct acacggccat cgggcgcagc tcattgctga 5520
tgagcaggtc ggcggccgtc agcgagcgaa tttcgtcaat ggtggtgttc ttgttgattt 5580
cggtgaggag gaggccgtcg ttaatcactt cgatcacccc cagttcggtc acaatcaggt 5640
tcgcctgcga tttggcggtc agcggcaggg tgcacttctt caggatcttc ggttggccct 5700
tgttggtgtg gcgcatggca atgatgacct tcttggcccc gttcaccaga tccatggcgc 5760
cgcccatccc cgacagcatc ttgcccggca cgatccagtt ggcgatattg cccttctcgt 5820
ccacctgcag cgcgcccagc accgtgacat ccacatgccc gccccgaatg agcgagaacg 5880
acaccgacga gtcgaaaaag gtgccgtccg gcagcacggt ggtatagtcg ccccccgcat 5940
tcaccacgtc cttgtccgcc tcattgattt tcgggctggc ccccatgccc acaatgccat 6000
tttccgactg gaaggtgatc ttgaaattct tcgggatgta gtcggccacc atcgtgggca 6060
ggcccacgcc cagattgacc agctgcccgt ttttcagttc gcgggccacg cgcttcgcaa 6120
taatctcctt ggccaggttt ttgtcattaa tcattttagg cgggctcctt cacgatgtag 6180
ttgatgagca cgcccggcgt catggccttt tccttctcca gcttctcgca ggagaccagg 6240
ttttcggctt ccacgatcac ggttttggcg gccatcgcca tgtacgggtt gaagttcttg 6300
gtggtgccct tgtagaaggt gttgccggct tcgtcgacaa tcgagccttt aatcagggcc 6360
acgtcggcgg tcagcggcag ctccaggagg tattcggtcc cgttgatgga gatcttcttt 6420
ttgccttttt cgatgagggt ccccaggccg gtcttggtca ggaccccccc gaggcccgag 6480
ccccccgcgc gaatgcgctc caccagcgtg ccctgcggcg acagttccac ttccagctca 6540
ttgttgaaca gttttttccc cgtgtccgga ttcgagccaa tgtacgaggc gatcagtttc 6600
ttcacctggt tgttcgagat gagcttgccg atgccggtgt tcgggtagca ggtatcgttg 6660
gagataatgg tgagattctt gatgttcagg ttcaccagaa aatcaatcag cttggtcggg 6720
gtgccgcagt tcagaaagcc cccaatcata atcgtcatgc cgtccttgaa gaacgagcgg 6780
aggttctcaa agcggatgat cttcgagttc attttaatcc ctccttttaa actttctagc 6840
acttttccag caggatcgcg gtgccctggc cgccgccgat gcacagggtc gccagcccct 6900
tcttggcgtc gcgcttctgc atcgcgtgca ccagggtcac caggatgcgg gcgcccgagg 6960
cgccgatcgg gtgccccagg gcgatggcgc cgccattcac gttcactttg ttcatgtcga 7020
acttcaggtc cttggcgacg gccagcgact gggcggcaaa ggcctcgttc gactcgatca 7080
ggtccagctc gtcgaccgtc cagccggctt tctcgatcgc cgccttggtg gcgtagaacg 7140

ggccgtagcc catgatggcc gggtccacgc cggccgagcc atacgacacg atcttcgcca 7200

gcggtttcac gcccagctcc ttggcctttt cggccgacat gatcaccagc acggccgcgc 7260

agtcgttcag gcccgaggcg ttgcccgcgg tcacggtgcc gtccttcttg aaggccggct 7320

tcagcttggc caggccctcg atcgtcgacc cgaagcgcgg gtgctcgtcc gtgtccacca 7380

cggtctcgcc cttgcggccc ttaatcacca ccggcacgat ctcgtccttg aactggcccg 7440

acttgatggc ttcctccgcc ttcttttgcg aggccagggc gaactcgtcc tgctcctcgc 7500

gcgagatgtt ccagcgctcg gcgatgttct cggcggtgat gcccatgtgg tagtcgttga 7560

aggcgtccca caggccgtcg gtgatcatct cgtccacgaa cttggcgttc cccatgcggt 7620

agccccagcg ggcgttgttg gccaggtacg gggcgcgcga catgttttcc atgccgccgg 7680

caatgatcac gtcggcgtcg ccggccttga tgatctgggc cgccagcgac acggtgcgca 7740

ggcccgagcc gcacaccttg ttgatggtca tggcggggat ctccaccggg aggccggctt 7800

tgaagctcgc ctggcgggcc gggttctggc cgagcccggc ctgcagcacg ttgcccagga 7860

tcacctcgtt cacgtcctcc ggcttgatgc cggccttctt cacggcctcc ttaatggcgg 7920

tggcgcccag gtccacggcg ggcacgtctt tcaggctctt gccgtacgag ccgatcgcgg 7980

tgcgcacggc cgaggcaatc accacctcct tcattcttga atctcctgaa aggtacccag 8040

cttttgttcc ctttagtgag ggttaattgc gcgcttggcg taatcatggt catagctgtt 8100

tcctgtgtga aattgttatc cgctcacaat tccacacaac atacgagccg gaagcataaa 8160

gtgtaaagcc tggggtgcct aatgagtgag ctaactcaca ttaattgcgt tgcgctcact 8220

gcccgctttc cagtcgggaa acctgtcgtg ccagctgcat taatgaatcg gccaacgcgc 8280

ggggagaggc ggtttgcgta ttgggcgcat gcataaaaac tgttgtaatt cattaagcat 8340

tctgccgaca tggaagccat cacaaacggc atgatgaacc tgaatcgcca gcggcatcag 8400

caccttgtcg ccttgcgtat aatatttgcc catgggggtg ggcgaagaac tccagcatga 8460

gatccccgcg ctggaggatc atccagccgg cgtcccggaa aacgattccg aagcccaacc 8520

tttcatagaa ggcggcggtg gaatcgaaat ctcgtgatgg caggttgggc gtcgcttggt 8580

cggtcatttc gaaccccaga gtcccgctca gaagaactcg tcaagaaggc gatagaaggc 8640

gatgcgctgc gaatcgggag cggcgatacc gtaaagcacg aggaagcggt cagcccattc 8700

gccgccaagc tcttcagcaa tatcacgggt agccaacgct atgtcctgat agcggtccgc 8760

cacacccagc cggccacagt cgatgaatcc agaaaagcgg ccattttcca ccatgatatt 8820

cggcaagcag gcatcgccat gggtcacgac gagatcctcg ccgtcgggca tgcgcgcctt 8880

gagcctggcg aacagttcgg ctggcgcgag cccctgatgc tcttcgtcca gatcatcctg 8940

atcgacaaga ccggcttcca tccgagtacg tgctcgctcg atgcgatgtt tcgcttggtg 9000

gtcgaatggg caggtagccg gatcaagcgt atgcagccgc cgcattgcat cagccatgat 9060

ggatactttc tcggcaggag caaggtgaga tgacaggaga tcctgccccg gcacttcgcc 9120

caatagcagc cagtcccttc ccgcttcagt gacaacgtcg agcacagctg cgcaaggaac 9180

gcccgtcgtg gccagccacg atagccgcgc tgcctcgtcc tgcagttcat tcagggcacc 9240

ggacaggtcg gtcttgacaa aaagaaccgg gcgcccctgc gctgacagcc ggaacacggc 9300

ggcatcagag cagccgattg tctgttgtgc ccagtcatag ccgaatagcc tctccaccca 9360

agcggccgga gaacctgcgt gcaatccatc ttgttcaatc atgcgaaacg atcctcatcc 9420

tgtctcttga tcagatcttg atcccctgcg ccatcagatc cttggcggca agaaagccat 9480

ccagtttact ttgcagggct tcccaacctt accagagggc gccccagctg gcaattccgg 9540

ttcgcttgct gtccataaaa ccgcccagtc tagctatcgc catgtaagcc cactgcaagc 9600

tacctgcttt ctctttgcgc ttgcgttttc ccttgtccag atagcccagt agctgacatt 9660

catcccaggt ggcacttttc ggggaaatgt gcgcgcccgc gttcctgctg gcgctgggcc 9720

tgtttctggc gctggacttc ccgctgttcc gtcagcagct tttcgcccac ggccttgatg 9780

atcgcggcgg ccttggcctg catatcccga ttcaacggcc ccagggcgtc cagaacgggc 9840

ttcaggcgct cccgaaggt 9859

<210> 86
<211> 510
<212> DNA
<213> Ralstonia eutropha

<400> 86
atgtcaacct ataccaatga acaggtcgcc cacctcgtcg atggttccct cgattgggag 60

accaccttcc gcatgctctc gatgcccaag gacgagagcc ggttcgcgca gtacctgtca 120

gccttgcagg ccaaggtgaa ctttcctgac cgcatcgtgc taccgctcag cccgcacatg 180

tttatcgtgc agtcggtcaa gagcaagaag tgggtagtca agtgcgactg cggtcacgag 240

ttctgtgatt accgcgagaa ctggaagctg catgccgcga tctacgtgcg agacaccgag 300

gaggcgatga cagaggtgta tccgaagctg atggcccccg acacccagtg gcaggtgtat 360

cgcgaatact actgcccatc gtgcggcacc atgcacgacg tcgaagcgcc taccccgtgg 420

tatcccgtga tccacgactt cgagccggac atcgaggcct tctacaagga gtgggtgaat 480

ctcccggtgc cggagcgcgc gtccgactaa 510

<210> 87
<211> 1222
<212> DNA
<213> Escherichia coli

<400> 87
tctagatttc cattgaaagg actacacaat gactgacgtt gtcatcgtat ccgccgcccg 60

caccgcggtc ggcaagtttg gcggctcgct ggccaagatc ccggcaccgg aactgggtgc 120

cgtggtcatc aaggccgcgc tggagcgcgc cggcgtcaag ccggagcagg tgagcgaagt 180

catcatgggc caggtgctga ccgccggttc gggccagaac cccgcacgcc aggccgcgat 240

caaggccggc ctgccggcga tggtgccggc catgaccatc aacaaggtgt gcggctcggg 300

cctgaaggcc gtgatgctgg ccgccaacgc gatcatggcg ggcgacgccg agatcgtggt 360

ggccggcggc caggaaaaca tgagcgccgc cccgcacgtg ctgccgggct cgcgcgatgg 420

tttccgcatg ggcgatgcca agctggtcga caccatgatc gtcgacggcc tgtgggacgt 480

gtacaaccag taccacatgg gcatcaccgc cgagaacgtg gccaaggaat acggcatcac 540

acgcgaggcg caggatgagt tcgccgtcgg ctcgcagaac aaggccgaag ccgcgcagaa 600

ggccggcaag tttgacgaag agatcgtccc ggtgctgatc ccgcagcgca agggcgaccc 660

ggtggccttc aagaccgacg agttcgtgcg ccagggcgcc acgctggaca gcatgtccgg 720

cctcaagccc gccttcgaca aggccggcac ggtgaccgcg gccaacgcct cgggcctgaa 780

cgacggcgcc gccgcggtgg tggtgatgtc ggcggccaag gccaaggaac tgggcctgac 840

cccgctggcc acgatcaaga gctatgccaa cgccggtgtc gatcccaagg tgatgggcat 900

gggcccggtg ccggcctcca agcgcgccct gtcgcgcgcc gagtggaccc cgcaagacct 960

ggacctgatg gagatcaacg aggcctttgc cgcgcaggcg ctggcggtgc accagcagat 1020

gggctgggac acctccaagg tcaatgtgaa cggcggcgcc atcgccatcg gccacccgat 1080

cggcgcgtcg ggctgccgta tcctggtgac gctgctgcac gagatgaagc gccgtgacgc 1140

gaagaagggc ctggcctcgc tgtgcatcgg cggcggcatg ggcgtggcgc tggcagtcga 1200

gcgcaaataa ggaaggtcta ga 1222

<210> 88
<211> 8512
<212> DNA
<213> Artificial Sequence

<220>
<223> vector

<400> 88
accttcggga gcgcctgaag cccgttctgg acgccctggg gccgttgaat cgggatatgc 60

aggccaaggc cgccgcgatc atcaaggccg tgggcgaaaa gctgctgacg gaacagcggg 120

aagtccagcg ccagaaacag gcccagcgcc agcaggaacg cgggcgcgca catttccccg 180

aaaagtgcca cctgggatga atgtcagcta ctgggctatc tggacaaggg aaaacgcaag 240

cgcaaagaga aagcaggtag cttgcagtgg gcttacatgg cgatagctag actgggcggt 300

tttatggaca gcaagcgaac cggaattgcc agctggggcg ccctctggta aggttgggaa 360

gccctgcaaa gtaaactgga tggctttctt gccgccaagg atctgatggc gcaggggatc 420

aagatctgat caagagacag gatgaggatc gtttcgcatg attgaacaag atggattgca 480

cgcaggttct ccggccgctt gggtggagag gctattcggc tatgactggg cacaacagac 540

aatcggctgc tctgatgccg ccgtgttccg gctgtcagcg caggggcgcc cggttctttt 600

tgtcaagacc gacctgtccg gtgccctgaa tgaactgcag gacgaggcag cgcggctatc 660

gtggctggcc acgacgggcg ttccttgcgc agctgtgctc gacgttgtca ctgaagcggg 720

aagggactgg ctgctattgg gcgaagtgcc ggggcaggat ctcctgtcat ctcaccttgc 780

tcctgccgag aaagtatcca tcatggctga tgcaatgcgg cggctgcata cgcttgatcc 840

ggctacctgc ccattcgacc accaagcgaa acatcgcatc gagcgagcac gtactcggat 900

ggaagccggt cttgtcgatc aggatgatct ggacgaagag catcaggggc tcgcgccagc 960

cgaactgttc gccaggctca aggcgcgcat gcccgacggc gaggatctcg tcgtgaccca 1020

tggcgatgcc tgcttgccga atatcatggt ggaaaatggc cgcttttctg gattcatcga 1080

ctgtggccgg ctgggtgtgg cggaccgcta tcaggacata gcgttggcta cccgtgatat 1140

tgctgaagag cttggcggcg aatgggctga ccgcttcctc gtgctttacg gtatcgccgc 1200

tcccgattcg cagcgcatcg ccttctatcg ccttcttgac gagttcttct gagcgggact 1260

ctggggttcg aaatgaccga ccaagcgacg cccaacctgc catcacgaga tttcgattcc 1320

accgccgcct tctatgaaag gttgggcttc ggaatcgttt tccgggacgc cggctggatg 1380

atcctccagc gcggggatct catgctggag ttcttcgccc acccccatgg gcaaatatta 1440

tacgcaaggc gacaaggtgc tgatgccgct ggcgattcag gttcatcatg ccgtttgtga 1500

tggcttccat gtcggcagaa tgcttaatga attacaacag tttttatgca tgcgcccaat 1560

acgcaaaccg cctctccccg cgcgttggcc gattcattaa tgcagctggc acgacaggtt 1620

tcccgactgg aaagcgggca gtgagcgcaa cgcaattaat gtgagttagc tcactcatta 1680

ggcaccccag gctttacact ttatgcttcc ggctcgtatg ttgtgtggaa ttgtgagcgg 1740

ataacaattt cacacaggaa acagctatga ccatgattac gccaagcgcg caattaaccc 1800

tcactaaagg gaacaaaagc tgggtaccgg gccccccctc gaggtcgacg gtatcgataa 1860

gcttgatatc gaattcctgc agcccggggg atccactagt tctagatttc cattgaaagg 1920

actacacaat gactgacgtt gtcatcgtat ccgccgcccg caccgcggtc ggcaagtttg 1980

gcggctcgct ggccaagatc ccggcaccgg aactgggtgc cgtggtcatc aaggccgcgc 2040

tggagcgcgc cggcgtcaag ccggagcagg tgagcgaagt catcatgggc caggtgctga 2100

ccgccggttc gggccagaac cccgcacgcc aggccgcgat caaggccggc ctgccggcga 2160

tggtgccggc catgaccatc aacaaggtgt gcggctcggg cctgaaggcc gtgatgctgg 2220

ccgccaacgc gatcatggcg ggcgacgccg agatcgtggt ggccggcggc caggaaaaca 2280

tgagcgccgc cccgcacgtg ctgccgggct cgcgcgatgg tttccgcatg ggcgatgcca 2340

agctggtcga caccatgatc gtcgacggcc tgtgggacgt gtacaaccag taccacatgg 2400

gcatcaccgc cgagaacgtg gccaaggaat acggcatcac acgcgaggcg caggatgagt 2460

tcgccgtcgg ctcgcagaac aaggccgaag ccgcgcagaa ggccggcaag tttgacgaag 2520

agatcgtccc ggtgctgatc ccgcagcgca agggcgaccc ggtggccttc aagaccgacg 2580

agttcgtgcg ccagggcgcc acgctggaca gcatgtccgg cctcaagccc gccttcgaca 2640

aggccggcac ggtgaccgcg gccaacgcct cgggcctgaa cgacggcgcc gccgcggtgg 2700

tggtgatgtc ggcggccaag gccaaggaac tgggcctgac cccgctggcc acgatcaaga 2760

gctatgccaa cgccggtgtc gatcccaagg tgatgggcat gggcccggtg ccggcctcca 2820

agcgcgccct gtcgcgcgcc gagtggaccc cgcaagacct ggacctgatg gagatcaacg 2880

aggcctttgc cgcgcaggcg ctggcggtgc accagcagat gggctgggac acctccaagg 2940

tcaatgtgaa cggcggcgcc atcgccatcg gccacccgat cggcgcgtcg ggctgccgta 3000

tcctggtgac gctgctgcac gagatgaagc gccgtgacgc gaagaagggc ctggcctcgc 3060

tgtgcatcgg cggcggcatg ggcgtggcgc tggcagtcga gcgcaaataa ggaaggtcta 3120

gaaataattt tgtttaactt taagaaggaa ttcaggagcc cttcaccatg acctggcttg 3180

agccgcagat aaagtcccaa ctccaatcgg agcgcaagga ctgggaagcg aacgaagtcg 3240

gcgccttctt gaagaaggcc cccgagcgca aggagcagtt ccacacgatc ggggacttcc 3300

cggtccagcg cacctacacc gctgccgaca tcgccgacac gccgctggag gacatcggtc 3360

ttccggggcg ctacccgttc acgcgcgggc cctacccgac gatgtaccgc agccgcacct 3420

ggacgatgcg ccagatcgcc ggcttcggca ccggcgagga caccaacaag cgcttcaagt 3480

atctgatcgc gcagggccag accggcatct ccaccgactt cgacatgccc acgctgatgg 3540

gctacgactc cgaccacccg atgagcgacg gcgaggtcgg ccgcgagggc gtggcgatcg 3600

acacgctggc cgacatggag gcgctgctgg ccgacatcga cctcgagaag atctcggtct 3660

cgttcacgat caacccgagc gcctggatcc tgctcgcgat gtacgtggcg ctcggcgaga 3720

agcgcggcta cgacctgaac aagctgtcgg gcacggtgca ggccgacatc ctgaaggagt 3780

acatggcgca gaaggagtac atctacccga tcgcgccgtc ggtgcgcatc gtgcgcgaca 3840

tcatcaccta cagcgcgaag aacctgaagc gctacaaccc gatcaacatc tcgggctacc 3900

acatcagcga ggccggctcc tcgccgctcc aggaggcggc cttcacgctg gccaacctga 3960

tcacctacgt gaacgaggtg acgaagaccg gtatgcacgt cgacgaattc gcgccgcggt 4020

tggccttctt cttcgtgtcg caaggtgact tcttcgagga ggtcgcgaag ttccgcgccc 4080

tgcgccgctg ctacgcgaag atcatgaagg agcgcttcgg tgcaagaaat ccggaatcga 4140

tgcggttgcg cttccactgt cagaccgcgg cggcgacgct gaccaagccg cagtacatgg 4200

tcaacgtcgt gcgtacgtcg ctgcaggcgc tgtcggccgt gctcggcggc gcgcagtcgc 4260

tgcacaccaa cggctacgac gaagccttcg cgatcccgac cgaggatgcg atgaagatgg 4320

cgctgcgcac gcagcagatc attgccgagg agagtggtgt cgccgacgtg atcgacccgc 4380

tgggtggcag ctactacgtc gaggcgctga ccaccgagta cgagaagaag atcttcgaga 4440

tcctcgagga agtcgagaag cgcggtggca ccatcaagct gatcgagcag ggctggttcc 4500

agaagcagat tgcggacttc gcttacgaga ccgcgctgcg caagcagtcc ggccagaagc 4560

cggtgatcgg ggtgaaccgc ttcgtcgaga acgaagagga cgtcaagatc gagatccacc 4620

cgtacgacaa cacgacggcc gaacgccaga tttcccgcac gcgccgcgtt cgcgccgagc 4680

gcgacgaggc caaggtgcaa gcgatgctcg accaactggt ggctgtcgcc aaggacgagt 4740

cccagaacct gatgccgctg accatcgaac tggtgaaggc cggcgcaacg atgggggaca 4800

tcgtcgagaa gctgaagggg atctggggta cctaccgcga gacgccggtc ttctgagcag 4860

gaagcttccc accatggacc aaatcccgat ccgcgttctt ctcgccaaag tcggcctcga 4920

cggccatgac cgaggcgtca aggtcgtcgc tcgcgcgctg cgcgacgccg gcatggacgt 4980

catctactcc ggccttcatc gcacgcccga agaggtggtc aacaccgcca tccaggaaga 5040

cgtggacgtg ctgggtgtaa gcctcctgtc cggcgtgcag ctcacggtct tccccaagat 5100

cttcaagctc ctggacgaga gaggcgctgg cgacttgatc gtgatcgccg gtggcgtgat 5160

gccggacgag gacgccgcgg ccatccgcaa gctcggcgtg cgcgaggtgc tcctgcagga 5220

cacgcccccg caggccatca tcgactcgat ccgctccttg gtcgccgcgc gcggcgcccg 5280

ctgaaagggc gagctctcca attcgcccta tagtgagtcg tattacgcgc gctcactggc 5340

cgtcgtttta caacgtcgtg actgggaaaa ccctggcgtt acccaactta atcgccttgc 5400

agcacatccc cctttcgcca gctggcgtaa tagcgaagag gcccgcaccg atcgcccttc 5460

ccaacagttg cgcagcctga atggcgaatg gaaattgtaa gcgttaatat tttgttaaaa 5520

ttcgcgttaa atttttgtta aatcagctca ttttttaacc aataggccga ctgcgatgag 5580

tggcagggcg gggcgtaatt tttttaaggc agttattggt gcccttaaac gcctggtgct 5640

acgcctgaat aagtgataat aagcggatga atggcagaaa ttcgaaagca aattcgaccc 5700

ggtcgtcggt tcagggcagg gtcgttaaat agccgcttat gtctattgct ggtttaccgg 5760

tttattgact accggaagca gtgtgaccgt gtgcttctca aatgcctgag gccagtttgc 5820

tcaggctctc cccgtggagg taataattga cgatatgatc atttattctg cctcccagag 5880

cctgataaaa acggtgaatc cgttagcgag gtgccgccgg cttccattca ggtcgaggtg 5940

gcccggctcc atgcaccgcg acgcaacgcg gggaggcaga caaggtatag ggcggcgagg 6000

cggctacagc cgatagtctg gaacagcgca cttacgggtt gctgcgcaac ccaagtgcta 6060

ccggcgcggc agcgtgaccc gtgtcggcgg ctccaacggc tcgccatcgt ccagaaaaca 6120

cggctcatcg ggcatcggca ggcgctgctg cccgcgccgt tcccattcct ccgtttcggt 6180

caaggctggc aggtctggtt ccatgcccgg aatgccgggc tggctgggcg gctcctcgcc 6240

ggggccggtc ggtagttgct gctcgcccgg atacagggtc gggatgcggc gcaggtcgcc 6300

atgccccaac agcgattcgt cctggtcgtc gtgatcaacc accacggcgg cactgaacac 6360

cgacaggcgc aactggtcgc ggggctggcc ccacgccacg cggtcattga ccacgtaggc 6420

cgacacggtg ccggggccgt tgagcttcac gacggagatc cagcgctcgg ccaccaagtc 6480

cttgactgcg tattggaccg tccgcaaaga acgtccgatg agcttggaaa gtgtcttctg 6540

gctgaccacc acggcgttct ggtggcccat ctgcgccacg aggtgatgca gcagcattgc 6600

cgccgtgggt ttcctcgcaa taagcccggc ccacgcctca tgcgctttgc gttccgtttg 6660

cacccagtga ccgggcttgt tcttggcttg aatgccgatt tctctggact gcgtggccat 6720

gcttatctcc atgcggtagg gtgccgcacg gttgcggcac catgcgcaat cagctgcaac 6780

ttttcggcag cgcgacaaca attatgcgtt gcgtaaaagt ggcagtcaat tacagatttt 6840

ctttaaccta cgcaatgagc tattgcgggg ggtgccgcaa tgagctgttg cgtacccccc 6900

ttttttaagt tgttgatttt taagtctttc gcatttcgcc ctatatctag ttctttggtg 6960

cccaaagaag ggcacccctg cggggttccc ccacgccttc ggcgcggctc cccctccggc 7020

aaaaagtggc ccctccgggg cttgttgatc gactgcgcgg ccttcggcct tgcccaaggt 7080

ggcgctgccc ccttggaacc cccgcactcg ccgccgtgag gctcgggggg caggcgggcg 7140

ggcttcgcct tcgactgccc ccactcgcat aggcttgggt cgttccaggc gcgtcaaggc 7200

caagccgctg cgcggtcgct gcgcgagcct tgacccgcct tccacttggt gtccaaccgg 7260

caagcgaagc gcgcaggccg caggccggag gcttttcccc agagaaaatt aaaaaaattg 7320

atggggcaag gccgcaggcc gcgcagttgg agccggtggg tatgtggtcg aaggctgggt 7380

agccggtggg caatccctgt ggtcaagctc gtgggcaggc gcagcctgtc catcagcttg 7440

tccagcaggg ttgtccacgg gccgagcgaa gcgagccagc cggtggccgc tcgcggccat 7500

cgtccacata tccacgggct ggcaagggag cgcagcgacc gcgcagggcg aagcccggag 7560

agcaagcccg tagggcgccg cagccgccgt aggcggtcac gactttgcga agcaaagtct 7620

agtgagtata ctcaagcatt gagtggcccg ccggaggcac cgccttgcgc tgcccccgtc 7680

gagccggttg gacaccaaaa gggaggggca ggcatggcgg catacgcgat catgcgatgc 7740

aagaagctgg cgaaaatggg caacgtggcg gccagtctca agcacgccta ccgcgagcgc 7800

gagacgccca acgctgacgc cagcaggacg ccagagaacg agcactgggc ggccagcagc 7860

accgatgaag cgatgggccg actgcgcgag ttgctgccag agaagcggcg caaggacgct 7920

gtgttggcgg tcgagtacgt catgacggcc agcccggaat ggtggaagtc ggccagccaa 7980

gaacagcagg cggcgttctt cgagaaggcg cacaagtggc tggcggacaa gtacggggcg 8040

gatcgcatcg tgacggccag catccaccgt gacgaaacca gcccgcacat gaccgcgttc 8100

gtggtgccgc tgacgcagga cggcaggctg tcggccaagg agttcatcgg caacaaagcg 8160

cagatgaccc gcgaccagac cacgtttgcg gccgctgtgg ccgatctagg gctgcaacgg 8220

ggcatcgagg gcagcaaggc acgtcacacg cgcattcagg cgttctacga ggccctggag 8280

cggccaccag tgggccacgt caccatcagc ccgcaagcgg tcgagccacg cgcctatgca 8340

ccgcagggat tggccgaaaa gctgggaatc tcaaagcgcg ttgagacgcc ggaagccgtg 8400

gccgaccggc tgacaaaagc ggttcggcag gggtatgagc ctgccctaca ggccgccgca 8460

ggagcgcgtg agatgcgcaa gaaggccgat caagcccaag agacggcccg ag 8512

## Beschreibung

### Gebiet der Erfindung

Gegenstand der vorliegenden Erfindung ist ein Verfahren zur Herstellung von organischen Verbindungen umfassend die Verfahrensschritte
A) Bereitstellen eines Knallgasbakteriums mit einer verglichen zu seinem Wildtyp gesteigerten Aktivität eines Enzyms E₁, welches die Umsetzung
   2 Acetyl-CoA zu Acetoacetyl-CoA und CoA
   zu katalysieren vermag, in einem wässrigen Medium
B) in Kontakt Bringen des wässrigen Mediums mit einem Gas enthaltend H₂, CO₂ und O₂
   in einem Gewichtsverhältnis von 20 bis 70 zu 10 bis 45 zu 5 bis 35, und gegebenenfalls
C) Aufreinigen der organischen Verbindung.

### Stand der Technik

Biobasierte Treibstoffe und Chemikalien werden heutzutage üblicherweise aus Kohlenhydraten, wie Glucose, Dextrose oder Glycerin hergestellt. Dies hat eine Vielzahl von Nachteilen:
i) Preise für Kohlenhydrate, wie Glucose, Dextrose oder Glycerin, werden sich möglicherweise analog zu fossilen Rohstoffen entwickeln, da sie in Produkte mit hohem Brennwert umgewandelt werden können.
ii) Kohlenhydrate, wie Glucose, Dextrose oder Glycerin, unterliegen starken Preisschwankungen.
iii) Kohlenhydrate, wie Glucose, Dextrose oder Glycerin, sind als Rohstoffe nicht in allen Regionen in ausreichender Menge verfügbar.
iv) Die Verwendung von Kohlenhydraten, wie Glucose oder Dextrose, als Fermentationsrohstoffe steht in Konkurrenz zum Einsatz als Nahrungsmittel.

Technologien für die hochselektive Herstellung von organischen Verbindungen mittels Biokatalysatoren und mit H₂ und CO₂ als Rohstoffen unter energetisch günstigen, aeroben Bedingungen sind heutzutage noch nicht verfügbar, würden es jedoch ermöglichen, rohstofftechnisch und regional hochflexibel, diese Chemikalien aus kostengünstigen Rohstoffen bzw. Abfallströmen (Erdgas, kommunale Abfälle, Biomasse, Konvertergas, etc.) herzustellen.

### Beschreibung der Erfindung

Überraschenderweise wurde gefunden, dass das im Folgenden beschriebene Verfahren mindestens einen der Nachteile des Standes der Technik zu überwinden vermag.

Gegenstand der vorliegenden Erfindung ist daher das in Anspruch 1 sowie den davon abhängigen Ansprüchen beschriebene Verfahren.

Ein weiterer Gegenstand der Erfindung ist die Verwendung der im Rahmen der Erfindung offenbarten Knallgasbakterien zur Herstellung von organischen Verbindungen.

Gegenstand der vorliegenden Erfindung ist somit ein Verfahren zur Herstellung einer organischen Verbindung umfassend die Verfahrensschritte
A) Bereitstellen eines Knallgasbakteriums mit einer verglichen zu seinem Wildtyp gesteigerten Aktivität eines Enzyms E₁, welches die Umsetzung
   von 2 Acetyl-CoA zu Acetoacetyl-CoA und CoA
   zu katalysieren vermag, in einem wässrigen Medium
B) in Kontakt Bringen des wässrigen Mediums mit einem Gas enthaltend
   H₂, CO₂ und O₂
   in einem Gewichtsverhältnis von 20 bis 70 zu 10 bis 45 zu 5 bis 35, insbesondere von 30 bis 55 zu 15 bis 40 zu 10 bis 30, und gegebenenfalls
C) Aufreinigen der organischen Verbindung.

Unter dem Begriff "Knallgasbakterium" ist ein Bakterium zu verstehen, das in der Lage ist chemolithoautotroph zu wachsen und aus H₂ und CO₂ in Gegenwart von Sauerstoff Kohlenstoffgerüste mit mehr als einem C-Atom aufzubauen, wobei der Wasserstoff oxidiert und der Sauerstoff als terminaler Elektronenakzeptor benutzt wird. Es können erfindungsgemäß sowohl solche Bakterien eingesetzt werden, die von Natur aus Knallgasbakterien darstellen, oder aber auch Bakterien, welche durch gentechnische Modifikation zu Knallgasbakterien wurden, wie beispielsweise eine *E*. *coli* Zelle, die durch rekombinantes Einfügen der notwendigen Enzyme in die Lage versetzt wurde, aus H₂ und CO₂ in Gegenwart von Sauerstoff Kohlenstoffgerüste mit mehr als einem C-Atom aufzubauen, wobei der Wasserstoff oxidiert und der Sauerstoff als terminaler Elektronenakzeptor benutzt wird. Bevorzugt handelt es sich bei den im erfindungsgemäßen Verfahren eingesetzten Knallgasbakterien um solche, welche bereits als Wildtyp Knallgasbakterien darstellen.

Erfindungsgemäß bevorzugt eingesetzte Knallgasbakterien sind ausgewählt aus den Gattungen *Achromobacter, Acidithiobacillus, Acidovorax, Alcaligenes, Anabena, , Aquifex, Arthrobacter, Azospirillum, Bacillus, Bradyrhizobium, Cupriavidus, Derxia, Helicobacter, Herbaspirillum, Hydrogenobacter, Hydrogenobaculum, Hydrogenophaga" Hydrogenophilus, Hydrogenothermus, Hydrogenovibrio, Ideonella sp. O1, Kyrpidia, Metallosphaera, Methanobrevibacter, Myobacterium, Nocardia, Oligotropha, Paracoccus, Pelomonas, Polaromonas, Pseudomonas, Pseudonocardia, Rhizobium, Rhodococcus, Rhodopseudomonas, Rhodospirillum, Streptomyces, Thiocapsa, Treponema,Variovorax, Xanthobacter, Wautersia,* wobei *Cupriavidus* besonders bevorzugt ist, besonders aus den Spezies *Cupriavidus necator* (alias *Ralstonia eutropha, Wautersia eutropha, Alcaligenes eutrophus, Hydrogenomonas eutropha), Achromobacter ruhlandii, Acidithiobacillus ferrooxidans, Acidovorax facilis, Alcaligenes hydrogenophilus, Alcaligenes latus, Anabena cylindrica, Anabena oscillaroides, Anabena sp., Anabena spiroides, Aquifex aeolicus, Aquifex pyrophilus, Arthrobacter strain 11X, Bacillus schlegelii, Bradyrhizobium japonicum, Cupriavidus necator, Derxia gummosa, Escherichia coli, Heliobacter pylori, Herbaspirillum autotrophicum, Hydrogenobacter hydrogenophilus., Hydrogenobacter thermophilus, Hydrogenobaculum acidophilum, Hydrogenophaga flava, Hydrogenophaga palleronii, Hydrogenophaga pseudoflava, Hydrogenophaga taeniospiralis, Hydrogeneophilus thermoluteolus, Hydrogenothermus marinus, Hydrogenovibrio marinus, Ideonella sp. O-1, Kyrpidia tusciae, Metallosphaera sedula, Methanobrevibactercuticularis, Mycobacterium gordonae, Nocardia autotrophica, Oligotropha carboxidivorans, Paracoccus denitrificans, Pelomonas saccharophila, Polaromonas hydrogenivorans, Pseudomonas hydrogenovora, Pseudomonas thermophila, Rhizobium japonicum, Rhodococcus opacus, Rhodopseudomonas palustris, Seliberia carboxydohydrogena, Streptomyces thermoautotrophicus, Thiocapsa roseopersicina, Treponema primitia, Variovorax paradoxus, Xanthobacter autrophicus, Xanthobacter flavus,* insbesondere aus den Stämmen *Cupriavidus necator H16, Cupriavidus necator* H1 oder *Cupriavidus necator Z-1.*

Das Knallgasbakterium des erfindungsgemäßen Verfahrens weist eine verglichen zu seinem Wildtyp gesteigerten Aktivität eines Enzyms E₁ auf.

Der Begriff "gesteigerte Aktivität eines Enzyms", wie er vorstehend und in den nachfolgenden Ausführungen im Zusammenhang mit der vorliegenden Erfindung verwendet wird, ist vorzugsweise zu verstehen, dass der Wildtyp derart gentechnisch verändert wurde, dass die entsprechende Aktivitätssteigerung eintritt. Bevorzugt ist dieser Begriff als gesteigerte intrazelluläre Aktivität zu verstehen.

Die nun folgenden Ausführungen zur Erhöhung der Enzymaktivität in Zellen gelten sowohl für die Erhöhung der Aktivität des Enzyms E₁ als auch für alle nachfolgend genannten Enzyme, deren Aktivität gegebenenfalls erhöht werden kann.

Grundsätzlich lässt sich eine Steigerung der enzymatischen Aktivität dadurch erzielen, dass man die Kopienzahl der Gensequenz bzw. der Gensequenzen erhöht, welche für das Enzym kodieren, einen starken Promotor verwendet, die Kodonnutzung des Gens verändert, auf verschiedene Art und Weise die Halbwertszeit der mRNA oder des Enzyms erhöht, die Regulation der Expression des Gens modifiziert oder ein Gen oder Allel nutzt, das für ein entsprechendes Enzym mit einer gesteigerten Aktivität kodiert und gegebenenfalls diese Maßnahmen kombiniert. Erfindungsgemäß eingesetzte, gentechnisch veränderte Mikroorganismen werden beispielsweise durch Transformation, Transduktion, Konjugation oder eine Kombination dieser Methoden mit einem Vektor erzeugt, der das gewünschte Gen, ein Allel dieses Gens oder Teile davon und einen die Expression des Gens ermöglichenden Promotor enthält. Die heterologe Expression wird insbesondere durch Integration des Gens oder der Allele in das Chromosom der Zelle oder einen extrachromosomal replizierenden Vektor erzielt.

Einen Überblick über die Möglichkeiten zur Erhöhung der Enzym-Aktivität in Zellen am Beispiel der Pyruvat-Carboxylase gibt DE-A-100 31 999, die hiermit als Referenz eingeführt wird und deren Offenbarungsgehalt hinsichtlich der Möglichkeiten zur Erhöhung der Enzym-Aktivität in Zellen einen Teil der Offenbarung der vorliegenden Erfindung bildet.

Die Expression der vorstehend und aller nachfolgend genannten Enzyme bzw. Gene ist mit Hilfe von 1- und 2-dimensionaler Proteingelauftrennung und anschließender optischer Identifizierung der Proteinkonzentration mit entsprechender Auswertesoftware im Gel nachweisbar.

Wenn die Erhöhung einer Enzymaktivität ausschließlich auf einer Erhöhung der Expression des entsprechenden Gens basiert, so kann die Quantifizierung der Erhöhung der Enzymaktivität in einfacher Weise durch einen Vergleich der 1- oder 2-dimensionalen Proteinauftrennungen zwischen Wildtyp und gentechnisch veränderter Zelle bestimmt werden. Eine gebräuchliche Methode zur Präparation der Proteingele bei Bakterien und zur Identifizierung der Proteine ist die von Hermann et al. (Electrophoresis, 22: 1712-23 (2001) beschriebene Vorgehensweise. Die Proteinkonzentration kann ebenfalls durch Western-Blot-Hybridisierung mit einem für das nachzuweisende Protein spezifischen Antikörper (Sambrook et al., Molecular Cloning: a laboratory manual, 2nd Ed. Cold Spring Harbor Laboratory Press, Cold Spring Harbor, N.Y. USA, 1989) und anschließende optische Auswertung mit entsprechender Software zur Konzentrationsbestimmung (Lohaus und Meyer (1989) Biospektrum, 5: 32-39; Lottspeich (1999), Angewandte Chemie 111: 2630-2647) analysiert werden.

Diese Methode bietet sich auch immer dann an, wenn mögliche Produkte der durch die zu bestimmende Enzymaktivität katalysierten Reaktion im Mikroorganismus schnell verstoffwechselt werden können oder aber die Aktivität im Wiltyp selber zu gering ist, um die zu bestimmende Enzymaktivität anhand der Produktbildung ausreichend bestimmen zu können.

Das Enzym E₁, dessen Aktivität in dem Knallgasbakterium verglichen zu seinem Wildtyp gesteigert ist, ist bevorzugt ausgewählt aus Acetyl-CoA:Acetyl-CoA C-Acetyltransferasen aus der Enzymklasse EC 2.3.1.9.

Die im Zusammenhang mit der vorliegenden Erfindung angeführten Accession-Nummern entsprechen den ProteinBank Datenbank-Einträgen des NCBI mit Datum vom 26.06.2012; in der Regel wird vorliegend die Versionsnummer des Eintrages durch "Ziffer" wie beispielsweise "1", kenntlich gemacht.

Erfindungsgemäß bevorzugte Acetyl-CoA:Acetyl-CoA C-Acetyltransferasen sind ausgewählt aus der Liste
AAC26023.1, ABR35750.1 und ABR25255.1
sowie

Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (*cell dry weight)* [U/g CDW]) im Vergleich zur Aktivität des Biokatalysators ohne Anwesenheit des Bezugsproteins verstanden wird, wobei unter der Aktivität in diesem Zusammenhang die Umsetzung von zwei Acetyl-CoA zu Acetoacetyl-CoA und CoA verstanden wird.

Eine Methode zur Bestimmung der Aktivität wird in Middleton et al. The Biochemical journal (1972), 126(1), 27-34 beschrieben.

Das Knallgasbakterium wird in Verfahrensschritt A) in einem wässrigen Medium bereitgestellt. Das eingesetzte wässrige Medium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D. C., USA, 1981) enthalten.

Das Medium kann Stickstoffquellen enthalten, als Stickstoffquelle können organische stickstoffhaltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat, Ammoniak, Ammoniumhydroxid oder Ammoniakwasser verwendet werden. Die Stickstoffquellen können einzeln oder als Mischung verwendet werden.

Das Medium kann Phosphorquellen enthalten, als Phosphorquelle können Phosphorsäure, Kaliumdihydrogen-phosphat oder Dikaliumhydrogenphosphat oder die entsprechenden natriumhaltigen Salze verwendet werden.

Das Medium kann auch Kohlenstoffquellen enthalten, jedoch sollten diese insofern limitiert sein, als dass das Knallgasbakterium im Wesentlichen auf die Verwertung der in dem H₂, CO₂ und O₂ enthaltenden Gas enthaltenen kohlenstoffhaltigen Gase angewiesen ist.

Das Medium muss weiterhin Salze von Metallen enthalten wie z. B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Medium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe wie z. B. Antibiotika hinzugefügt werden.

In Verfahrensschritt B) wird das wässrige Medium mit einem Gas enthaltend H₂, CO₂ und O₂ in Kontakt gebracht.

Hierdurch wird dem Knallgasbakterium das H₂, CO₂ und O₂ enthaltende Gas als Kohlenstoffquelle bereitgestellt. Das Knallgasbakterium synthetisiert mindestens teilweise aus dieser Kohlenstoffquelle Acetoacetyl-CoA, welches zu weiteren organischen Verbindungen verstoffwechselt wird. Anders als in einem acetogenen Verfahrensprozess, der unter streng anaeroben Bedingungen stattfindet, wird Verfahrensschritt B) des erfindungsgemäßen Verfahrens unter aeroben Bedingungen durchgeführt.

Bevorzugt beträgt der Partialdruck des Wasserstoffes in dem H₂, CO₂ und O₂ enthaltenden Gas 0,1 bis 100 bar, bevorzugt 0,2 bis 10 bar, besonders bevorzugt 0,5 bis 4 bar.

Der Partialdruck des Kohlendioxids in dem H₂, CO₂ und O₂ enthaltenden Gas beträgt bevorzugt 0,03 bis 100 bar, besonders bevorzugt 0,05 bis 1 bar, insbesondere 0,05 bis 0,3 bar Der Partialdruck des Sauerstoffs in dem H₂, CO₂ und O₂ enthaltenden Gas beträgt bevorzugt 0,001 bis 100 bar, besonders bevorzugt 0,04 bis 1 bar, insbesondere 0,04 bis 0,5 bar.

Als Quelle für das H₂ und CO₂ in Verfahrensschritt B) ist insbesondere Synthesegas geeignet, welches mit Sauerstoff versetzt eingesetzt wird; daher ist es erfindungsgemäß bevorzugt, dass das Gas in Verfahrensschritt B) Synthesegas enthält.

Synthesegas kann z. B. aus dem Nebenprodukt der Kohlevergasung bereitgestellt werden. Das Knallgasbakterium wandelt folglich eine Substanz, die ein Abfallprodukt ist, in einen wertvollen Rohstoff um.

Alternativ kann Synthesegas durch die Vergasung von weit verfügbaren, preiswerten landwirtschaftlichen Rohstoffen für das erfindungsgemäße Verfahren bereitgestellt werden. Es gibt zahlreiche Beispiele an Rohstoffen, die in Synthesegas umgewandelt werden können, da fast alle Vegetationsformen zu diesem Zwecke genutzt werden können. Bevorzugte Rohstoffe sind ausgewählt aus der Gruppe umfassend perennierende Gräser wie Chinaschilf, Getreiderückstände, Verarbeitungsabfälle wie Sägemehl. Im Allgemeinen wird Synthesegas in einer Vergasungsapparatur aus getrockneter Biomasse gewonnen, hauptsächlich durch Pyrolyse, partielle Oxidation und Dampfreformierung, wobei die Primärprodukte CO, H₂ und CO₂ sind.

Normalerweise wird ein Teil des Produktgases aufbereitet, um Produkterträge zu optimieren und Teerbildung zu vermeiden. Das Cracken des unerwünschten Teers in Synthesegas und CO kann unter Einsatz von Kalk und/oder Dolomit durchgeführt werden. Diese Prozesse werden im Detail in z. B. Reed, 1981 beschrieben (Reed, T.B., 1981, Biomass gasification: principles and technology, Noves Data Corporation, Park Ridge, NJ.). Es können auch Mischungen verschiedener Quellen zur Generierung von Synthesegas eingesetzt werden.

Insbesondere ist es bevorzugt, dass der im Synthesegas enthaltene Kohlenstoff in Verfahrensschritt B) mindestens 50 Gew.-%, bevorzugt mindestens 70 Gew.-%, besonders bevorzugt mindestens 90 Gew.-% des Kohlenstoffs aller Kohlenstoffquellen, die dem Knallgasbakterium in Verfahrensschritt B) zur Verfügung stehen, ausmacht, wobei sich die Gewichtsprozente des Kohlestoffs auf die Kohlenstoffatome beziehen. Die restlichen Kohlenstoffquellen können beispielsweise in Form von Kohlenhydraten im wässrigen Medium enthalten sein oder aber auch CO₂ aus einer anderen Quelle als Synthesegas darstellen. Weitere CO₂-Quellen stellen Abgase wie beispielsweise Rauchgas, Erdölraffinerieabgase, durch Hefegärung oder clostridielle Gärung entstandene Gase, Abgase aus der Vergasung zellulosehaltige Materialien oder der Kohlevergasung dar.

Diese Abgase müssen nicht notwendigerweise als Nebenerscheinungen anderer Prozesse entstanden sein, sondern können extra für den Einsatz in dem erfindungsgemäßen Verfahren produziert werden.

Die mit dem erfindungsgemäßen Verfahren herzustellende organische Substanz ist bevorzugt ausgewählt aus Substanzen umfassend drei oder vier Kohlenstoffatomen, insbesondere Butanol, Buten, Propen, Buttersäure, Aceton, 2-Hydroxyisobuttersäure und 2-Propanol und 2-Hydroxyisobuttersäure.

### Erste Ausführungsform; Butanol

Eine erste Ausführungsform des erfindungemäßen Verfahrens ist dadurch gekennzeichnet, dass die organische Substanz Butanol ist, bevorzugt E₁ ausgewählt ist aus Acetyl-CoA:Acetyl-CoA C-Acetyltransferasen und bevorzugt das Knallgasbakterium eine verglichen zu seinem Wildtyp gesteigerte Aktivität eines Enzyms E₂, welches die Umsetzung
von Acetoacetyl-CoA und NADH oder NADPH zu 3-Hydroxybutyryl-CoA und NAD+ oder NADP+
zu katalysieren vermag, aufweist.

Das Enzym E₂ ist bevorzugt ausgewählt aus 3-Hydroxybutyryl-CoA Dehydrogenasen aus der Enzymklasse EC:1.1.1.157.

Erfindungsgemäß bevorzugte 3-Hydroxybutyryl-CoA Dehydrogenasen sind ausgewählt aus der Liste

NP_349314.1, YP_001307469.1 und CAQ53138.1
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₂ generell insbesondere die Umsetzung von Acetoacetyl-CoA und NADH zu 3-Hydroxybutyryl-CoA und NAD+ verstanden wird.

Eine Methode zur Bestimmung der Aktivität wird in Senior et al. Biochemical Journal (1973), 134(1), 225-38 beschrieben.

Eine bevorzugte erste Ausführungsform des erfindungemäßen Verfahrens ist dadurch gekennzeichnet, dass das Knallgasbakterium neben der gesteigerten Aktivität des Enzyms E₁ und gegebenenfalls E₂ eine verglichen zu seinem Wildtyp gesteigerte Aktivität eines Enzyms E₃, welches die Umsetzung von 3-Hydroxybutyryl-CoA zu Crotonyl-CoA und Wasser
zu katalysieren vermag, aufweist.

Das Enzym E₃ ist bevorzugt ausgewählt aus 3-Hydroxybutyryl-CoA Dehydratasen aus der Enzymklasse EC:4.2.1.55.

Erfindungsgemäß bevorzugte 3-Hydroxybutyryl-CoA Dehydratasen sind ausgewählt aus der Liste

NP_349318.1, YP_001307465.1 und CAQ53134.1
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₃ generell insbesondere die Umsetzung von 3-Hydroxybutyryl-CoA zu Crotonyl-CoA und Wasser verstanden wird.

Eine Methode zur Bestimmung der Aktivität wird in Boynton et al. Journal of bacteriology (1996), 178(11), 3015-24 beschrieben.

Eine weitere bevorzugte erste Ausführungsform des erfindungemäßen Verfahrens ist dadurch gekennzeichnet, dass das Knallgasbakterium neben der gesteigerten Aktivität des Enzyms E₁ und gegebenenfalls E₂ und /oder E₃ eine verglichen zu seinem Wildtyp gesteigerte Aktivität eines Enzyms E₄, welches die Umsetzung
von Crotonyl-CoA und NADH oder NADPH zu Butyryl-CoA und NAD+ oder NADP+ zu katalysieren vermag, aufweist.

Das Enzym E₄ ist bevorzugt ausgewählt aus Butyryl-CoA Dehydrogenasen aus der Enzymklasse EC:1.3.99.2

Erfindungsgemäß bevorzugte Butyryl-CoA Dehydrogenasen sind ausgewählt aus der Liste NP_349317.1, YP_001307466.1 und CAQ53135.1
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₄ generell insbesondere die Umsetzung von Crotonyl-CoA und NADH zu Butyryl-CoA und NAD+ verstanden wird.

Eine Methode zur Bestimmung der Aktivität wird in R. Graf Dissertation an der Universität Ulm 2002 und in Rhead et al, Proc. Nati. Acad. Sci. USA Vol. 77, No. 1, pp. 580-583, January 1980 Medical Sciences beschrieben.

Eine weitere bevorzugte erste Ausführungsform des erfindungemäßen Verfahrens ist dadurch gekennzeichnet, dass das Knallgasbakterium neben der gesteigerten Aktivität des Enzyms E₁ und gegebenenfalls E₂, E₃ und /oder E₄ eine verglichen zu seinem Wildtyp gesteigerte Aktivität eines Enzyms E₅, welches die Umsetzung
von Butyryl-CoA und NADH oder NADPH zu Butyraldehyd, NAD+ oder NADP+ und HS-CoA oder
die Umsetzungen
von Butyryl-CoA und NADH oder NADPH zu Butyraldehyd, NAD+ oder NADP+ und HS-CoA und
von Butyraldehyd und NADH oder NADPH zu n-Butanol und NAD+ oder NADPH+ zu katalysieren vermag, aufweist.

Bevorzugt ist das Enzym E₅ ausgewählt aus bifunktionalen Aldehyd/Alkohol Dehydrogenasen aus der Enzymklasse EC:1.2.1.10 oder EC:1.1.1.1 oder aus Butyraldehyd Dehydrogenasen aus der Enzymklasse EC:1.2.1.10.

Erstgenannte Enzyme katalysieren beide der vorgenannten Reaktionen, während Butyraldehyd Dehydrogenasen lediglich Butyryl-CoA umsetzen.

Erfindungsgemäß bevorzugte bifunktionalen Aldehyd/Alkohol Dehydrogenasen sind ausgewählt aus der Liste
NP_149199.1, NP_563447.1 und YP_002861217.1
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₅ im Falle einer bifunktionalen Aldehyd/Alkohol Dehydrogenase insbesondere die Umsetzung von mindestens einer der beiden Reaktionen
von Butyryl-CoA und NADH oder NADPH zu Butyraldehyd, NAD+ oder NADP+ und HS-CoA und
von Butyraldehyd und NADH oder NADPH zu n-Butanol und NAD+ oder NADPH+ verstanden wird.

Eine Methode zur Bestimmung der beiden Aktivitäten wird in in Yan et al. Appl. Environ. Microbiol. 1990 56 (9), 2591-9 beschrieben.

Erfindungsgemäß bevorzugte Butyraldehyd Dehydrogenasen sind ausgewählt aus der Liste YP_001310903.1 und CAQ57983.1
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₅ im Falle einer Butyraldehyd Dehydrogenase insbesondere die Umsetzung von Butyryl-CoA und NADH zu Butyraldehyd, NAD+ und HS-CoA verstanden wird.

Eine weitere bevorzugte erste Ausführungsform des erfindungemäßen Verfahrens ist dadurch gekennzeichnet, dass das Knallgasbakterium neben der gesteigerten Aktivität des Enzyms E₁ und gegebenenfalls E₂, E₃, E₄ und /oder E₅ eine verglichen zu seinem Wildtyp gesteigerte Aktivität eines Enzyms E₆, welches die Umsetzung
von Butyraldehyd und NAD(P)H zu n-Butanol und NAD(P)+
zu katalysieren vermag, aufweist.

Bevorzugt ist das Enzym E₆ ausgewählt aus Butanol Dehydrogenasen aus der Enzymklasse EC:1.1.1.-.

Erfindungsgemäß bevorzugte Butanol Dehydrogenasen sind ausgewählt aus der Liste YP_001310904.1, YP_001310904 und CAQ53139.1 sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₆ generell insbesondere die Umsetzung von Butyraldehyd und NAD(P)H zu n-Butanol und NAD(P)+ verstanden wird.

Eine Methode zur Bestimmung der Aktivität wird in Duerre et al, Applied Microbiology and Biotechnology (1987), 26(3), 268-72 beschrieben.

Es ist insbesondere bevorzugt, dass das Knallgasbakterium eine gesteigerte Aktivität E₆ aufweist, wenn es über eine gesteigerte Aktivität eines Enzyms E₅ verfügt, welches nur die Umsetzung von Butyryl-CoA und NADH oder NADPH zu Butyraldehyd, NAD+ oder NADP+ und HS-CoA zu katalysieren vermag und somit bevorzugt eine Butyraldehyd Dehydrogenase ist. Eine weitere bevorzugte erste Ausführungsform des erfindungemäßen Verfahrens ist dadurch gekennzeichnet, dass das Knallgasbakterium neben der gesteigerten Aktivität des Enzyms E₁ und gegebenenfalls E₂, E₃, E₄, E₅ und /oder E₆ eine verglichen zu seinem Wildtyp gesteigerte Aktivität eines Enzyms E₇ ausgewählt aus Elektrontransfer-Flavoproteinen aus der Enzymklasse EC:2.8.3.9 aufweist.

Insbesondere geeignet ist hier ein Enzym E₇, welches ein heterodimeres Enzym ist, aufgebaut aus zwei Untereinheiten, bei dem die
alpha-Untereinheit ausgewählt ist aus NP_349315.1, YP_001307468.1 und CAQ53137.1 und die
beta-Untereinheit ausgewählt ist aus NP_349316.1, YP_001307467.1 und CAQ53136.1
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen.

Hier ist es insbesondere bevorzugt, wenn alpha- und beta- Untereinheiten aus dem gleichen Organismus kombiniert werden.

Es ist erfindungsgemäß bevorzugt, dass das eingesetzte Knallgasbakterium eine gesteigerte Aktivität E₇ aufweist, wenn bereits eine gesteigerte Aktivität von E₄ vorliegt.

In besonders bevorzugten ersten Ausführungsformen des erfindungemäßen Verfahrens weist das Knallgasbakterium gesteigerte Aktivitäten in den Kombinationen
E₁E₂, E₁E₃, E₁E₄, E₁E₅, E₁E₅, E₁E₆, E₁E₇,
E₁E₂E₃, E₁E₃E₄, E₁E₅E₆, E₁E₂E₄, E₁E₂E₅, E₁E₂E₆, E₁E₃E₄, E₁E₃E₅, E₁E₃E₆, E₁E₄E₅, E₁E₄E₆, E₁E₆E₇,
E₁E₃E₄E₅E₆E₇, E₁E₂E₄E₅E₆E₇, E₁E₂E₃E₅E₆E₇, E₁E₂E₃E₄E₆E₇, E₁E₂E₃E₄E₅E₆ und E₁E₂E₃E₄E₅E₆E₇ auf, wobei E₁E₂E₃E₄E₅E₆E₇, E₁E₃E₄E₅E₆E₇, E₁E₃E₄E₅E₇, E₁E₂E₃E₄E₅E₇
besonders bevorzugt ist.

### Zweite Ausführungsform; Buten

Eine zweite Ausführungsform des erfindungemäßen Verfahrens ist dadurch gekennzeichnet, dass die organische Substanz Buten ist, bevorzugt E₁ ausgewählt ist aus Acetyl-CoA:Acetyl-CoA C-Acetyltransferasen und bevorzugt das Knallgasbakterium eine verglichen zu seinem Wildtyp gesteigerte Aktivität eines Enzyms E₂, welches die Umsetzung
von Acetoacetyl-CoA und NADH oder NADPH zu 3-Hydroxybutyryl-CoA und NAD+ oder NADP+
zu katalysieren vermag, aufweist.

Das Enzym E₂ ist bevorzugt ausgewählt aus 3-Hydroxybutyryl-CoA Dehydrogenasen aus der Enzymklasse EC:1.1.1.157.

Erfindungsgemäß bevorzugte 3-Hydroxybutyryl-CoA Dehydrogenasen sind ausgewählt aus der Liste

NP_349314.1, YP_001307469.1 und CAQ53138.1
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₂ generell insbesondere die Umsetzung von Acetoacetyl-CoA und NADH zu 3-Hydroxybutyryl-CoA und NAD+ verstanden wird.

Eine bevorzugte zweite Ausführungsform des erfindungemäßen Verfahrens ist dadurch gekennzeichnet, dass das Knallgasbakterium neben der gesteigerten Aktivität des Enzyms E₁ und gegebenenfalls E₂ eine verglichen zu seinem Wildtyp gesteigerte Aktivität eines Enzyms E₃, welches die Umsetzung
von 3-Hydroxybutyryl-CoA zu Crotonyl-CoA und Wasser
zu katalysieren vermag, aufweist.

Das Enzym E₃ ist bevorzugt ausgewählt aus 3-Hydroxybutyryl-CoA Dehydratasen aus der Enzymklasse EC:4.2.1.55.

Erfindungsgemäß bevorzugte 3-Hydroxybutyryl-CoA Dehydratasen sind ausgewählt aus der Liste

NP_349318.1, YP_001307465.1 und CAQ53134.1
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₃ generell insbesondere die Umsetzung von 3-Hydroxybutyryl-CoA zu Crotonyl-CoA und Wasser verstanden wird.

Eine weitere bevorzugte zweite Ausführungsform des erfindungemäßen Verfahrens ist dadurch gekennzeichnet, dass das Knallgasbakterium neben der gesteigerten Aktivität des Enzyms E₁ und gegebenenfalls E₂ und /oder E₃ eine verglichen zu seinem Wildtyp gesteigerte Aktivität eines Enzyms E₄, welches die Umsetzung
von Crotonyl-CoA und NADH oder NADPH zu Butyryl-CoA und NAD+ oder NADP+
zu katalysieren vermag, aufweist.

Das Enzym E₄ ist bevorzugt ausgewählt aus Butyryl-CoA Dehydrogenasen aus der Enzymklasse EC:1.3.99.2

Erfindungsgemäß bevorzugte Butyryl-CoA Dehydrogenasen sind ausgewählt aus der Liste NP_349317.1, YP_001307466.1 und CAQ53135.1
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₄ generell insbesondere die Umsetzung von Crotonyl-CoA und NADH zu Butyryl-CoA und NAD+ verstanden wird.

Eine weitere bevorzugte zweite Ausführungsform des erfindungemäßen Verfahrens ist dadurch gekennzeichnet, dass das Knallgasbakterium neben der gesteigerten Aktivität des Enzyms E₁ und gegebenenfalls E₂, E₃ und /oder E₄ eine verglichen zu seinem Wildtyp gesteigerteAktivität eines Enzyms E₅, welches die Umsetzung
von Butyryl-CoA und NADH oder NADPH zu Butyraldehyd, NAD+ oder NADP+ und HS-CoA oder
die Umsetzungen
von Butyryl-CoA und NADH oder NADPH zu Butyraldehyd, NAD+ oder NADP+ und HS-CoA und
von Butyraldehyd und NADH oder NADPH zu n-Butanol und NAD+ oder NADPH+
zu katalysieren vermag, aufweist.

Bevorzugt ist das Enzym E₅ ausgewählt aus bifunktionalen Aldehyd/Alkohol Dehydrogenasen aus der Enzymklasse EC:1.2.1.10 oder EC:1.1.1.1 oder aus Butyraldehyd Dehydrogenasen aus der Enzymklasse EC:1.2.1.10.

Erstgenannte Enzyme katalysieren beide der vorgenannten Reaktionen, während Butyraldehyd Dehydrogenasen lediglich Butyryl-CoA umsetzen.

Erfindungsgemäß bevorzugte bifunktionalen Aldehyd/Alkohol Dehydrogenasen sind ausgewählt aus der Liste

NP_149199.1, NP_563447.1 und YP_002861217.1
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₅ im Falle einer bifunktionalen Aldehyd/Alkohol Dehydrogenase insbesondere die Umsetzung von mindestens einer der beiden Reaktionen
von Butyryl-CoA und NADH oder NADPH zu Butyraldehyd, NAD+ oder NADP+ und HS-CoA und
von Butyraldehyd und NADH oder NADPH zu n-Butanol und NAD+ oder NADPH+ verstanden wird.

Erfindungsgemäß bevorzugte Butyraldehyd Dehydrogenasen sind ausgewählt aus der Liste YP_001310903.1 und CAQ57983.1
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₅ im Falle einer Butyraldehyd Dehydrogenase insbesondere die Umsetzung von Butyryl-CoA und NADH zu Butyraldehyd, NAD+ und HS-CoA verstanden wird.

Eine weitere bevorzugte zweite Ausführungsform des erfindungemäßen Verfahrens ist dadurch gekennzeichnet, dass das Knallgasbakterium neben der gesteigerten Aktivität des Enzyms E₁ und gegebenenfalls E₂, E₃, E₄ und /oder E₅ eine verglichen zu seinem Wildtyp gesteigerte Aktivität eines Enzyms E₆, welches die Umsetzung
von Butyraldehyd und NAD(P)H zu n-Butanol und NAD(P)+
zu katalysieren vermag, aufweist.

Bevorzugt ist das Enzym E₆ ausgewählt aus Butanol Dehydrogenasen aus der Enzymklasse EC:1.1.1.-.

Erfindungsgemäß bevorzugte Butanol Dehydrogenasen sind ausgewählt aus der Liste YP_001310904.1, YP_001310904 und CAQ53139.1 sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₆ generell insbesondere die Umsetzung von Butyraldehyd und NAD(P)H zu n-Butanol und NAD(P)+ verstanden wird.

Es ist insbesondere bevorzugt, dass das Knallgasbakterium eine gesteigerte Aktivität E₆ aufweist, wenn es über eine gesteigerte Aktivität eines Enzyms E₅ verfügt, welches nur die Umsetzung von Butyryl-CoA und NADH oder NADPH zu Butyraldehyd, NAD+ oder NADP+ und HS-CoA zu katalysieren vermag und somit bevorzugt eine Butyraldehyd Dehydrogenase ist. Eine weitere bevorzugte zweite Ausführungsform des erfindungemäßen Verfahrens ist dadurch gekennzeichnet, dass das Knallgasbakterium neben der gesteigerten Aktivität des Enzyms E₁ und gegebenenfalls E₂, E₃, E₄, E₅ und /oder E₆ eine verglichen zu seinem Wildtyp gesteigerte Aktivität eines Enzyms E₇ ausgewählt aus Elektrontransfer-Flavoproteinen aus der Enzymklasse EC:2.8.3.9 aufweist.

Insbesondere geeignet ist hier ein Enzym E₇, welches ein heterodimeres Enzym ist, aufgebaut aus zwei Untereinheiten, bei dem die
alpha-Untereinheit ausgewählt ist aus NP_349315.1, YP_001307468.1 und CAQ53137.1 und die
beta-Untereinheit ausgewählt ist aus NP_349316.1, YP_001307467.1 und CAQ53136.1
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen.

Hier ist es insbesondere bevorzugt, wenn alpha- und beta- Untereinheiten aus dem gleichen Organismus kombiniert werden.

Eine weitere bevorzugte zweite Ausführungsform des erfindungemäßen Verfahrens ist dadurch gekennzeichnet, dass das Knallgasbakterium neben der gesteigerten Aktivität des Enzyms E₁ und gegebenenfalls E₂, E₃, E₄, E₅, E₆ und /oder E₇ eine verglichen zu seinem Wildtyp gesteigerte Aktivität eines Enzyms E₈, welches die Umsetzung
von n-Butanol zu 1-Buten und Wasser
zu katalysieren vermag, aufweist.

Bevorzugt ist das Enzym E₈ ausgewählt aus Oleat Hydratasen aus der Enzymklasse EC:4.2.1.53, Kieviton Hydratasen aus der Enzymklasse EC:4.2.1.95 und Phaseollidin Hydratasen aus der Enzymklasse EC:4.2.1.97.

Erfindungsgemäß bevorzugte Oleat Hydratasen sind ausgewählt aus der Liste ACT54545, OLHYD_STRPZ und OLHYD_FLAME, bevorzugte Kieviton Hydratase ist AAA87627.1, sowie für beide Enzymklassen Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₈ generell insbesondere die Umsetzung von n-Butanol zu 1-Buten und Wasser verstanden wird.

Eine Methode zur Bestimmung der Aktivität wird in Cleveland et al. Physio. Plant. Pathol. 22 (1983) 129-142 beschrieben; es muss lediglich Kieviton durch Butanol als Substrat ersetzt werden.
In besonders bevorzugten zweiten Ausführungsformen des erfindungemäßen Verfahrens weist das Knallgasbakterium gesteigerte Aktivitäten in den Kombinationen E₁E₂E₈, E₁E₃E₈, E₁E₄E₈, E₁E₅E₈, E₁E₆E₈, E₁E₃E₄E₅E₆E₇E₈, E₁E₂E₄E₅E₆E₇E₈, E₁E₂E₃E₅E₆E₇E₈, E₁E₂E₃E₄E₆E₇E₈, E₁E₂E₃E₄E₅E₆E₈ und E₁E₂E₃E₄E₅E₆E₇E₈ auf,
wobei E₁E₂E₃E₄E₅E₆E₇E₈, E₁E₃E₄E₅E₆E₇E₈, E₁E₃E₄E₅E₇E₈, E₁E₂E₃E₄E₅E₇E₈ besonders bevorzugt ist.

### Dritte Ausführungsform; Propen und Buttersäure

Eine dritte Ausführungsform des erfindungemäßen Verfahrens ist, dadurch gekennzeichnet, dass die organische Substanz Propen oder Buttersäure ist, bevorzugt E₁ ausgewählt ist aus Acetyl-CoA:Acetyl-CoA C-Acetyltransferasen und bevorzugt das Knallgasbakterium eine verglichen zu seinem Wildtyp gesteigerte Aktivität eines Enzyms E₂, welches die Umsetzung von Acetoacetyl-CoA und NADH oder NADPH zu 3-Hydroxybutyryl-CoA und NAD+ oder NADP+
zu katalysieren vermag, aufweist.

Das Enzym E₂ ist bevorzugt ausgewählt aus 3-Hydroxybutyryl-CoA Dehydrogenasen aus der Enzymklasse EC:1.1.1.157.

Erfindungsgemäß bevorzugte 3-Hydroxybutyryl-CoA Dehydrogenasen sind ausgewählt aus der Liste

NP_349314.1, YP_001307469.1 und CAQ53138.1
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₂ generell insbesondere die Umsetzung von Acetoacetyl-CoA und NADH zu 3-Hydroxybutyryl-CoA und NAD+ verstanden wird.

Eine bevorzugte dritte Ausführungsform des erfindungemäßen Verfahrens ist dadurch gekennzeichnet, dass das Knallgasbakterium neben der gesteigerten Aktivität des Enzyms E₁ und gegebenenfalls E₂ eine verglichen zu seinem Wildtyp gesteigerte Aktivität eines Enzyms E₃, welches die Umsetzung
von 3-Hydroxybutyryl-CoA zu Crotonyl-CoA und Wasser
zu katalysieren vermag, aufweist.

Das Enzym E₃ ist bevorzugt ausgewählt aus 3-Hydroxybutyryl-CoA Dehydratasen aus der Enzymklasse EC:4.2.1.55.

Erfindungsgemäß bevorzugte 3-Hydroxybutyryl-CoA Dehydratasen sind ausgewählt aus der Liste

NP_349318.1, YP_001307465.1 und CAQ53134.1
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₃ generell insbesondere die Umsetzung von 3-Hydroxybutyryl-CoA zu Crotonyl-CoA und Wasser verstanden wird.

Eine weitere bevorzugte dritte Ausführungsform des erfindungemäßen Verfahrens ist dadurch gekennzeichnet, dass das Knallgasbakterium neben der gesteigerten Aktivität des Enzyms E₁ und gegebenenfalls E₂ und /oder E₃ eine verglichen zu seinem Wildtyp gesteigerte Aktivität eines Enzyms E₄, welches die Umsetzung
von Crotonyl-CoA und NADH oder NADPH zu Butyryl-CoA und NAD+ oder NADP+ zu katalysieren vermag, aufweist.

Das Enzym E₄ ist bevorzugt ausgewählt aus Butyryl-CoA Dehydrogenasen aus der Enzymklasse EC:1.3.99.2

Erfindungsgemäß bevorzugte Butyryl-CoA Dehydrogenasen sind ausgewählt aus der Liste NP_349317.1, YP_001307466.1 und CAQ53135.1
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₄ generell insbesondere die Umsetzung von Crotonyl-CoA und NADH zu Butyryl-CoA und NAD+ verstanden wird.

Eine weitere bevorzugte dritte Ausführungsform des erfindungemäßen Verfahrens ist dadurch gekennzeichnet, dass das Knallgasbakterium neben der gesteigerten Aktivität des Enzyms E₁ und gegebenenfalls E₂, E₃ und /oder E₄ eine verglichen zu seinem Wildtyp gesteigerte Aktivität eines Enzyms E₉, welches die Umsetzung
von n-Butyryl-CoA und Pᵢ zu Butyryl-Phosphat und HS-CoA
zu katalysieren vermag, aufweist.

Pᵢ steht in diesem Zusammenhang für ein anorganisches Phosphat.

Bevorzugt ist das Enzym E₉ ausgewählt aus Phosphat Butyryltransferasen aus der Enzymklasse EC:2.3.1.19.

Erfindungsgemäß bevorzugte Phosphat Butyryltransferasen sind ausgewählt aus der Liste ABR32393.1 und ZP_05394269.1
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₉ generell insbesondere die Umsetzung von n Butyryl-CoA und Pᵢ zu Butyryl-Phosphat und HS-CoA verstanden wird.

Eine Methode zur Bestimmung der Aktivität wird in Wiesenborn et al. Applied and Environmental Microbiology (1989), 55(2), 317-22 beschrieben.

Eine weitere bevorzugte dritte Ausführungsform des erfindungemäßen Verfahrens ist dadurch gekennzeichnet, dass das Knallgasbakterium neben der gesteigerten Aktivität des Enzyms E₁ und gegebenenfalls E₂, E₃, E₄ und /oder E₉ eine verglichen zu seinem Wildtyp gesteigerte Aktivität eines Enzyms E₁₀, welches die Umsetzung
von Butyryl-Phosphat und ADP zu Butyrat und ATP
zu katalysieren vermag, aufweist.

Bevorzugt ist das Enzym E₁₀ ausgewählt aus Butyratkinasen aus der Enzymklasse EC:2.7.2.7.

Erfindungsgemäß bevorzugte Butyratkinasen sind ausgewählt aus der Liste ABR32394.1 und ZP_05392467.1
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₁₀ generell insbesondere die Umsetzung von Butyryl-Phosphat und ADP zu Butyrat und ATP verstanden wird.

Eine Methode zur Bestimmung der Aktivität wird in Hartmanis J Biol Chem. 1987 Jan 15; 262(2):617-21 beschrieben.

Eine weitere bevorzugte dritte Ausführungsform des erfindungemäßen Verfahrens ist dadurch gekennzeichnet, dass das Knallgasbakterium neben der gesteigerten Aktivität des Enzyms E₁ und gegebenenfalls E₂, E₃, E₄, E₉ und /oder E₁₀ eine verglichen zu seinem Wildtyp gesteigerte Aktivität eines Enzyms E₁₁, welches die Umsetzung
von Butyrat und H₂O₂ zu Propen und H₂O
zu katalysieren vermag, aufweist.

Bevorzugt ist das Enzym E₁₁ ausgewählt aus Cytochrom P450 der CYP152-Familie. Erfindungsgemäß bevorzugte Cytochrom P450 der CYP152-Familie sind ausgewählt aus der Liste

HQ709266.1, NP_388092,1 und NP_739069.1
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₁₁ generell insbesondere die Umsetzung von Butyrat und H₂O₂ zu Propen und H₂O verstanden wird.

Eine Methode zur Bestimmung der Aktivität wird in Mathew Applied and Environmental Microbiology, Mar. 2011, p. 1718-1727 beschrieben.

In besonders bevorzugten dritten Ausführungsformen des erfindungemäßen Verfahrens weist das Knallgasbakterium gesteigerte Aktivitäten in den Kombinationen E₁E₂E₃E₄E₉, E₁E₂E₃E₄E₁₀, E₁E₂E₃E₄E₁₁, E₁E₃E₄E₉E₁₀E₁₁, E₁E₂E₄E₉E₁₀E₁₁, E₁E₂E₃E₉E₁₀E₁₁, E₁E₂E₃E₄E₁₀E₁₁, E₁E₂E₃E₄E₉E₁₁, E₁E₂E₃E₄E₉E₁₀ und E₁E₂E₃E₄E₉E₁₀E₁₁ auf,
wobei E₁E₂E₃E₄E₉E₁₀E₁₁, E₁E₃E₄E₉E₁₀E₁₁ besonders bevorzugt ist.

In der dritten Ausführungsform des erfindungsgemäßen Verfahrens kann die Kombination der Enzyme E₉E₁₀, auch in Kombination mit den oben genannten anderen Enzymen wie vorstehend beschrieben, welche in Summe die Reaktion von n-Butyryl-CoA zu Butyrat katalysiert, durch mindestens ein Enzym ersetzt werden, bei der das n-Butyryl-CoA durch eine Thioesterase oder Acyl-CoA-Synthetase oder Acyl-CoA/Acylat:CoA-Transferase zu Butyrat umgewandelt wird.

### Vierte Ausführungsform; Aceton

Eine vierte Ausführungsform des erfindungemäßen Verfahrens ist dadurch gekennzeichnet, dass die organische Substanz Aceton ist, bevorzugt E₁ ausgewählt ist aus Acetyl-CoA:Acetyl-CoA C-Acetyltransferasen, und das Knallgasbakterium eine verglichen zu seinem Wildtyp gesteigerte Aktivität eines Enzyms E₁₂, welches die Umsetzung
von Acetoacetyl-CoA zu Acetoacetat und CoA
zu katalysieren vermag, aufweist.

Bevorzugt ist das Enzym E₁₂ ausgewählt aus Acetoacetyl-CoA:Acetat/Acyl:CoA Transferasen aus der Enzymklasse EC:3.1.2.11, aus Butyrat-Acetoacetat-CoA-Transferasen aus der Enzymklasse EC 2.8.3.9 oder aus Acyl-CoA-Hydrolasen aus der Enzymklasse EC 3.1.2.20. Erfindungsgemäß bevorzugte Acetoacetyl-CoA:Acetat/Acyl:CoA Transferasen sind ausgewählt aus der Liste
aus zwei Untereinheiten aufgebaute Transferasen, bei denen
die alpha-Untereinheit ausgewählt ist aus NP_149326.1, YP_001310904.1 und CAQ57984.1 und die
beta-Untereinheit ausgewählt ist aus NP_149327.1, YP_001310905.1 und CAQ57985.1
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₁₂ generell insbesondere die Umsetzung von Acetoacetyl-CoA zu Acetoacetat und CoA verstanden wird.

Erfindungsgemäß bevorzugte Butyrat-Acetoacetat-CoA-Transferasen sind ausgewählt aus ctfA und ctfB aus *Clostridium acetobutylicum* und atoD und atoA aus *Escherichia coli* sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₁₂ generell insbesondere die Umsetzung von Acetoacetyl-CoA zu Acetoacetat und CoA verstanden wird.

Erfindungsgemäß bevorzugte Acyl-CoA Hydrolasen sind ausgewählt aus
tell aus *B*. *subtilis* bzw. ybgC aus *Heamophilus influenzae*
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₁₂ generell insbesondere die Umsetzung von Acetoacetyl-CoA zu Acetoacetat und CoA verstanden wird.

Eine Methode zur Bestimmung der Transferaseaktivität des Enzyms E₁₂ wird in Jeffrey et al, Applied and Environmental Microbiology, die der Hydrolaseaktivität des Enzyms E₁₂ in Aragon et al. Journal of Biological Chemistry (1983), 258(8), 4725-33. beschrieben.

Eine bevorzugte vierte Ausführungsform des erfindungemäßen Verfahrens ist dadurch gekennzeichnet, dass das Knallgasbakterium neben der gesteigerten Aktivität des Enzyms E₁ und gegebenenfalls E₁₂ eine verglichen zu seinem Wildtyp gesteigerten Aktivität eines Enzyms E₁₃, welches die Umsetzung
von Acetoacetat zu Aceton und CO₂
zu katalysieren vermag, aufweist.

Das Enzym E₁₃ ist bevorzugt ausgewählt aus Acetoacetat Decarboxylasen aus der Enzymklasse EC:4.1.1.4 oder aus Aceton:CO2 Ligasen aus der Enzymklasse EC 6.4.1.6. Erfindungsgemäß bevorzugte Acetoacetat Decarboxylasen sind ausgewählt aus der Liste NP_149328.1, YP_001310906.1 und CA057986.1
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₁₃ generell insbesondere die Umsetzung von Acetoacetat zu Aceton und CO₂ verstanden wird.

Eine Methode zur Bestimmung der Aktivität wird in Daniel et al. Appl. Environ. Microbiol. 1990, p. 3491-3498 Vol. 56, No. 11 beschrieben.

Erfindungsgemäß bevorzugte Aceton:CO2 Ligasen sind ausgewählt aus der Liste der oligomeren Proteine mit Sequenzen.

Eine Methode zur Bestimmung der Aktivität von Aceton:CO2 Ligasen wird Miriam K. Sluis et al in Proc Natl Acad Sci U S A. 1997 August 5; 94(16): 8456-8461 beschrieben, wobei hier als Substrate Acetoacetat, AMP und Orthophosphat verwendet werden.

In besonders bevorzugten vierten Ausführungsformen des erfindungemäßen Verfahrens weist das Knallgasbakterium gesteigerte Aktivitäten in den Kombinationen E₁E₁₂, E₁E₁₃ und E₁E₁₂E₁₃ auf,
wobei E₁E₁₂E₁₃ besonders bevorzugt ist.

### Fünfte Ausführungsform; Propan-2-ol; gegebenenfalls gefolgt von Dehydratisierung zu Propen

Eine fünfte Ausführungsform des erfindungemäßen Verfahrens ist dadurch gekennzeichnet, dass die organische Substanz Propan-2-ol ist, bevorzugt E₁ ausgewählt ist aus Acetyl-CoA:Acetyl-CoA C-Acetyltransferasen, und das Knallgasbakterium eine verglichen zu seinem Wildtyp gesteigerte Aktivität eines Enzyms E₁₂, welches die Umsetzung
von Acetoacetyl-CoA zu Acetoacetat und CoA
zu katalysieren vermag, aufweist.

Bevorzugt ist das Enzym E₁₂ ausgewählt aus Acetoacetyl-CoA:Acetat/Acyl:CoA Transferasen aus der Enzymklasse EC:3.1.2.11, aus Butyrat-Acetoacetat-CoA-Transferasen aus der Enzymklasse EC 2.8.3.9 oder aus Acyl-CoA-Hydrolasen aus der Enzymklasse EC 3.1.2.20. Erfindungsgemäß bevorzugte Acetoacetyl-CoA:Acetat/Acyl:CoA Transferasen sind ausgewählt aus der Liste
aus zwei Untereinheiten aufgebaute Transferasen, bei denen
die alpha-Untereinheit ausgewählt ist aus NP_149326.1, YP_001310904.1 und CAQ57984.1 und die
beta-Untereinheit ausgewählt ist aus NP_149327.1, YP_001310905.1 und CAQ57985.1 sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₁₂ generell insbesondere die Umsetzung von Acetoacetyl-CoA zu Acetoacetat und CoA verstanden wird.

Erfindungsgemäß bevorzugte Butyrat-Acetoacetat-CoA-Transferasen sind ausgewählt aus ctfA und ctfB aus *Clostridium acetobutylicum* und atoD und atoA aus *Escherichia coli* sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₁₂ generell insbesondere die Umsetzung von Acetoacetyl-CoA zu Acetoacetat und CoA verstanden wird.

Erfindungsgemäß bevorzugte Acyl-CoA Hydrolasen sind ausgewählt aus tell aus *B*. *subtilis* bzw. ybgC aus *Heamophilus influenzae*
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des

Biokatalysators ohne Anwesenheit des Bezugsproteins verstanden wird, wobei unter der Aktivität in diesem Zusammenhang und im Zusammenhang mit der Bestimmung der Aktivität des Enzyms E₁₂ generell insbesondere die Umsetzung von Acetoacetyl-CoA zu Acetoacetat und CoA verstanden wird.

Eine bevorzugte fünfte Ausführungsform des erfindungemäßen Verfahrens ist dadurch gekennzeichnet, dass das Knallgasbakterium neben der gesteigerten Aktivität des Enzyms E₁ und gegebenenfalls E₁₂ eine verglichen zu seinem Wildtyp gesteigerteAktivität eines Enzyms E₁₃, welches die Umsetzung
von Acetoacetat zu Aceton und CO₂
zu katalysieren vermag, aufweist.

Das Enzym E₁₃ ist bevorzugt ausgewählt aus Acetoacetat Decarboxylasen aus der Enzymklasse EC:4.1.1.4 oder aus Aceton:CO2 Ligasen aus der Enzymklasse EC 6.4.1.6. Erfindungsgemäß bevorzugte Acetoacetat Decarboxylasen sind ausgewählt aus der Liste NP_149328.1, YP_001310906.1 und CAQ57986.1
sowie Proteine mit einer Polypeptidsequenz, bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber den vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₁₃ generell insbesondere die Umsetzung von Acetoacetat zu Aceton und CO₂ verstanden wird.

Eine Methode zur Bestimmung der Aktivität wird in Daniel et al. Appl. Environ. Microbiol. 1990, p. 3491-3498 Vol. 56, No. 11 beschrieben.

Eine Methode zur Bestimmung der Aktivität von Aceton:CO2 Ligasen wird Miriam K. Sluis et al in Proc Natl Acad Sci U S A. 1997 August 5; 94(16): 8456-8461 beschrieben, wobei hier als Substrate Acetoacetat, AMP und Orthophosphat verwendet werden.

Eine weitere bevorzugte fünfte Ausführungsform des erfindungemäßen Verfahrens ist dadurch gekennzeichnet, dass das Knallgasbakterium neben der gesteigerten Aktivität des Enzyms E₁ und gegebenenfalls E₁₂, und /oder E₁₃ eine verglichen zu seinem Wildtyp gesteigerte Aktivität eines Enzyms E₁₄, welches die Umsetzung
von Aceton, NADPH und H⁺ zu Propan-2-ol + NADP⁺
zu katalysieren vermag, aufweist.

Bevorzugt ist das Enzym E₁₄ ausgewählt aus Propan-2-ol:NADP⁺ Oxidoreduktase aus der Enzymklasse EC:1.1.1.80.

Eine Methode zur Bestimmung der Aktivität von Propan-2-ol:NADP⁺ Oxidoreduktase wird in Antonio D. Uttaro et al in Molecular and Biochemical Parasitology 85 (1997) 213 219 beschrieben.

In besonders bevorzugten fünften Ausführungsformen des erfindungemäßen Verfahrens weist das Knallgasbakterium gesteigerte Aktivitäten in den Kombinationen E₁E₁₄, E₁E₁₂ E₁₄, E₁E₁₂E₁₃ E₁₄ und E₁E₁₂E₁₃E₁₄ auf,
wobei E₁E₁₂E₁₃E₁₄ besonders bevorzugt ist.

In einer besonders bevorzugten fünften Ausführungsform des erfindungemäßen Verfahrens wird in Verfahrensschritt C) das Propan-2-ol aus der wässrigen Lösung destillativ als Azeotrop isoliert. Methoden zur Isolierung von Propan-2-ol sind unter anderem in Lei Zhigang, Zhang Jinchang, Chen Biaohua Separation of aqueous isopropanol by reactive extractive distillation in Journal of Chemical Technology and Biotechnology Volume 77, Issue 11 pages 1251-1254, November 2002, Lloyd Berg et al *Separation of the propyl alcohols from water by azeotropic or extractive destillation* US5085739 und Berg, *Lloyd Separation of ethanol, isopropanol and water mixtures by azeotropic distillation* US5762765 beschrieben.

Eine besonders bevorzugte fünfte Ausführungsform des erfindungemäßen Verfahrens umfasst einen Verfahrensschritt D).

Dehydratisierung von dem in Verfahrensschritt C) isolierten Propan-2-ol zu Propen.

Bevorzugt wird das isolierte Propan-2-ol in Verfahrensschritt D) durch chemische Dehydratisierung an einem sauren Katalysator zu Propen umgesetzt. Entsprechende Verfahren sind in Maria L. Martinez et al Synthesis, characterization and catalytic activity of AISBA-3 mesoporous catalyst having variable silicon-to-aluminum ratios Microporous and Mesoporous Materials 144 (2011) 183-190 und A.S. Araujo et al Kinetic study of isopropanol dehydration over silicoaluminophosphate catalyst Reaction Kinetics and Catalysis Letters January 1999, Volume 66,Issue 1, pp 141-146 beschrieben.

### Sechste Ausführungsform; 2-Hydroxyisobuttersäure

Eine sechste Ausführungsform des erfindungemäßen Verfahrens ist dadurch gekennzeichnet, dass die organische Substanz 2-Hydroxyisobuttersäure oder ein Salz der 2-Hydroxyisobuttersäure ist, bevorzugt E₁ ausgewählt ist aus Acetyl-CoA:Acetyl-CoA C-Acetyltransferasen, das Knallgasbakterium gegebenenfalls eine verglichen zu seinem Wildtyp gesteigerte Aktivität eines Enzyms E₂, welches die Umsetzung
von Acetoacetyl-CoA und NADH oder NADPH zu 3-Hydroxybutyryl-CoA und NAD+ oder NADP+
zu katalysieren vermag, aufweist
und das Knallgasbakterium eine verglichen zu seinem Wildtyp gesteigerten Aktivität eines Enzyms E₁₅, welches die Umsetzung
von 3-Hydroxybutyryl-Coenzym A zu 2-Hydroxyisobutyryl-Coenzym A zu katalysieren vermag, aufweist.

In diesem Zusammenhang bevorzugte Enzyme E₂ sind jene, die in der ersten Ausführungsform als bevorzugte angegeben sind.

Bei dem Enzym E₁₅ handelt es sich vorzugsweise um eine Hydroxyl-Isobutyryl-CoA Mutase, um eine Isobutyryl-CoA Mutase (EC 5.4.99.13) oder um eine Methylmalonyl-CoA Mutase (EC 5.4.99.2), jeweils bevorzugt um eine Coenzym B12-abhängige Mutase.

Bei dem Enzym E₁₅ handelt es sich bevorzugt um solche Enzyme, die sich aus den Mikroorganismen *Aquincola tertiaricarbonis* L108, DSM18028, DSM18512, *Methylibium petroleiphilum* PM1, *Methylibium* sp. R8, *Xanthobacter autotrophicus* Py2, *Rhodobacter sphaeroides* (ATCC 17029), Nocardioides sp. JS614, *Marinobacter algicola* DG893, *Sinorhizobium medicae* WSM419, *Roseovarius* sp. 217, *Pyrococcus furiosus* DSM 3638 isolieren lassen.

In einer bevorzugten Ausgestaltung der sechsten Ausführungsform stellt das Enzym E₁₅ ein heterodimeres Enzyme dar, welches Sequenzen umfasst, ausgewählt aus Seq-ID-Nr. 78 und 80
sowie

Proteine mit einer Polypeptidsequenz bei der bis zu 60 %, bevorzugt bis zu 25 %, besonders bevorzugt bis zu 15 % insbesondere bis zu 10, 9, 8, 7, 6, 5, 4, 3, 2, 1% der Aminosäurereste gegenüber der jeweiligen vorgenannten Bezugssequenzen durch Deletion, Insertion, Substitution oder eine Kombination davon verändert sind und die noch mindestens 50 %, bevorzugt 65 %, besonders bevorzugt 80 %, insbesondere mehr als 90 % der Aktivität des Proteins mit der entsprechenden, vorgenannten Bezugssequenz besitzen, wobei unter 100 % Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/g CDW]) im Vergleich zur Aktivität des Biokatalysators ohne Anwesenheit des Bezugsproteins verstanden wird, wobei unter der Aktivität in diesem Zusammenhang und im Zusammenhang mit der Bestimmung der Aktivität des Enzyms E₁₅ generell insbesondere die Umsetzung von 3-Hydroxybutyryl-Coenzym A zu 2-Hydroxyisobutyryl-Coenzym A verstanden wird.

In besonders bevorzugten sechsten Ausführungsformen des erfindungemäßen Verfahrens weist das Knallgasbakterium gesteigerte Aktivitäten in den Kombinationen E₁E₂E₁₅ auf.

In der sechsten Ausführungsform des erfindungsgemäßen Verfahrens ist es bevorzugt, dass neben dem Enzym E₁₅ zusätzlich das Knallgasbakterium eine verglichen zu seinem Wildtyp gesteigerten Menge eines MeaB Proteins, insbesondere eines mit Seq-ID-Nr. 82, aufweist.

Bei dem MeaB Protein handelt es sich bevorzugt um solche ausgewählt aus Sequenz ID Nr. 82, YP_001023545.1 *(Methylibium petroleiphilum* PM1), YP_001409454.1 (*Xanthobacter autotrophicus* Py2), YP_001045518.1 (*Rhodobacter sphaeroides* ATCC 17029), YP_002520048.1 (*Rhodobacter sphaeroides*), AAL86727.1 (*Methylobacterium extorquens* AMI), CAX21841.1 (*Methylobacterium extorquens* DM4), YP_001637793.1 (*Methylobacterium extorquens* PA1), AAT28130.1 (*Aeromicrobium erythreum*), CAJ91091 (*Polyangium cellulosum*), AAM77046.1 (*Saccharopolyspora erythraea*) und NP_417393.1 (*Escherichia coli* str. K-12 substr. MG1655)
sowie

Proteine mit einer Polypeptidsequenz bei der bis zu 60 %, bevorzugt bis zu 25 %, besonders bevorzugt bis zu 15 % insbesondere bis zu 10, 9, 8, 7, 6, 5, 4, 3, 2, 1 % der Aminosäurereste gegenüber der jeweiligen vorgenannten Bezugssequenzen durch Deletion, Insertion, Substitution oder eine Kombination davon verändert sind.

Insbesondere können Enzyme E₁₅ und das MeaB Protein als Fusionsproteine expremiert werden, wie sie beispielsweise in der PCT/EP2010/065151 offenbart sind und welche besonders bevorzugt eingesetzt werden.

Eine Methode zur Bestimmung der Aktivität wird in Murthy VV et al in Biochim Biophys Acta. 1977 Aug 11; 483(2): 487-91 beschrieben

Folgende Abbildungen sind Bestandteil der Beispiele:
Abbildung 1: Exemplarische Aceton- und Isopropanol-Produktion C. necator H16 pBBR-EcatoDAB1Caadc
Abbildung 2: Exemplarische Butanol-Produktion mit C. necator H16 pBBR-RephaABJ-CaadhE2

### Beispiele:

### Ausführungsbeispiel 1: Konstruktion von Plasmiden für die Herstellung von Aceton mit Cupriavidus necator

Für die Konstruktion von Plasmiden für die Herstellung von Aceton mit C. *necatorwurden* fünf synthetische Expressionskassetten synthetisiert, die aus folgenden Komponenten bestehen:
1. dem *E. coli atoDAB*-Operon, kodierend für die β-Untereinheit der Acetyl-CoA:Acetoacetyl-CoA-Transferase AtoD (Seq-ID-Nr. 13), die α-Untereinheit der Acetyl-CoA:Acetoacetyl-CoA-Transferase AtoA (Seq-ID-Nr. 14) und die Thiolase AtoB (Seq-ID-Nr. NR. 15), incl. der *atoD-, atoA-* und atoB-Ribosomenbindestellen und dem *atoB*-Terminator, wobei die für AtoD, AtoA und AtoB kodierenden Bereiche für die Translation in C. *necator* codonoptimiert wurden (nRBS-EcatoD-nRBS-EcatoA-nRBS-EcatoB-T-EcatoB; Seq-ID-Nr. 16)
2. dem *C*. *necatorphaA-*Gen, kodierend für die Thiolase PhaA (Seq-ID-Nr. 17), incl. der *phaA-*Ribosomenbindestelle, und dem *C*. *acetobutylicum ctfAB*-Operon, kodierend für die α- und β-Untereinheit der Acetyl-/Butyryl-CoA-Acetoacetat:CoA-Transferase CtfA (Seq-ID-Nr. 18) und CtfB (Seq-ID-Nr. 19), incl. der *ctfA-* bzw. *ctfB*-Ribosomenbindestellen und dem *ctfAB-*Terminator, wobei die für CtfA und CtfB kodierenden Bereiche für die Translation in C. *necator* codonoptimiert wurden (nRBS-RephaA-nRBS-CactfA-nRBS-CactfB-T-CactfAB; Seq-ID-Nr. 20)
3. der Ribosomenbindestelle des C. *necatorgroEL-Gens* (Seq-ID-Nr. 1), dem C. *acetobutylicum thIA-*Gen, kodierend für die Thiolase ThIA (Seq-ID-Nr. 21) sowie dem C. *acetobutylicum* ctfAB-Operon, kodierend für die α- und β-Untereinheit der Acetyl-/Butyryl-CoA-Acetoacetat:CoA-Transferase CtfA (Seq-ID-Nr. 18) und CtfB (Seq-ID-Nr. 19), incl. der nativen ctfA- bzw. ctfB-Ribosomenbindestellen und dem nativen ctfAB-Terminator, wobei die für ThIA, CtfA und CtfB kodierenden Bereiche für die Translation in C. *necator* codonoptimiert wurden (RBS-RegroEL-CathlA-nRBS-CactfA-nRBS-CactfB-T-CactfAB; Seq-ID-Nr. 22)
4. der Ribosomenbindestelle des C. *necatorgroEL-Gens* (Seq-ID-Nr. 1), gefolgt vom C. *acetobutylicum thIA-*Gen, kodierend für die Thiolase ThlA (Seq-ID-Nr. 21), der Ribosomenbindestelle des C. *necatorgroEL-Gens* (Seq-ID-Nr. 1), gefolgt vom *H. influenzae ybgC*-Gen, kodierend für die Thioesterase YbgC (Seq-ID-Nr. 23) und schließlich der Ribosomenbindestelle des C. *necatorgroEL-Gens* (Seq-ID-Nr. 1), gefolgt vom C. *acetobutylicum adc*-Gen, kodierend für die Acetoacetat-Decarboxylase Adc (Seq-ID-Nr. 24), incl. dem *adc*-Terminator, wobei die für ThlA, YbgC und Adc kodierenden Bereiche für die Translation in *C*. *necator* codonoptimiert wurden (RBS-RegroEL-CathlA-RBS-RegroEL-HiybgC-RBS-RegroEL-Caadc-T-Caadc; Seq-ID-Nr. 25)
5. dem *E*. *coli lacZ*-Promotor (Seq-ID-Nr. 26), der Ribosomenbindestelle des *C*. *necator groEL-*Gens (Seq-ID-Nr. 1) sowie dem *C*. *acetobutylicum adc*-Gen, kodierend für die Acetoacetat-Decarboxylase Adc (Seq-ID-Nr. 24), incl. dem *adc*-Terminator, wobei der für Adc kodierende Bereich für die Translation in C. *necator* codonoptimiert wurde (Plac-RBS-RegroEL-Caadc-T-Caadc; Seq-ID-Nr. 27)

Die Expressionskassetten nRBS-EcatoD-nRBS-EcatoA-nRBS-EcatoB-T-EcatoB, nRBS-RephaA-nRBS-CactfA-nRBS-CactfB-T-CactfAB, RBS-RegroEL-CathlA-nRBS-CactfA-nRBS-CactfB-T-CactfAB und RBS-RegroEL-CathlA-RBS-RegroEL-HiybgC-RBS-RegroEL-Caadc-T-Caadc wurden dann über *Kpn*I/*Hind*III in den Broad Host Range Expressionsvektor pBBR1MCS-2 (Seq-ID-Nr. 10) kloniert, so dass die Expression der Gene unter Kontrolle des *E. coli lacZ*-Promotors steht. Die resultierenden Expressionsplasmide wurden als pBBR-EcatoDAB, pBBR-RephaA-CactfAB, pBBR-CathlA-ctfAB und pBBR-CathlA-HiybgC-Caadc bezeichnet und entsprechen den Seq-ID-Nrs. 28, 29, 30 und 31.

Die Expressionskassette Plac-RBS-RegroEL-Caadc-T-Caadc wurde dann über *Hind*III/*Bam*HI in die Vektoren pBBR-EcatoDAB, pBBR-RephaA-CactfAB und pBBR-CathlA-ctfAB (Seq-ID-Nr. 28, 29 und 30) kloniert. Die resultierenden Expressionsplasmide wurden als pBBR-EcatoDAB|Caadc, pBBR-RephaA-CactfAB|adc und pBBR-Cath|A-ctfAB|adc bezeichnet und entsprechen den Seq-ID-Nrs. 32, 33 und 34.

### Ausführungsbeispiel 2: Konstruktion von Plasmiden für die Herstellung von Aceton mit Cupriavidus necator

Für die Konstruktion von Plasmiden für die Herstellung von Aceton mit C. necatorwurden fünf synthetische Expressionskassetten synthetisiert, die aus folgenden Komponenten bestehen:
1. dem *E*. *coli* atoDAB-Operon, kodierend für die β-Untereinheit der Acetyl-CoA:Acetoacetyl-CoA-Transferase AtoD (Seq-ID-Nr. 13), die α-Untereinheit der Acetyl-CoA:Acetoacetyl-CoA-Transferase AtoA (Seq-ID-Nr. 14) und die Thiolase AtoB (Seq-ID-Nr. 15), incl. der *atoD-, atoA-* und *atoB*-Ribosomenbindestellen und dem *atoB-*Terminator, wobei die für AtoD, AtoA und AtoB kodierenden Bereiche für die Translation in *C. necator* codonoptimiert wurden (nRBS-EcatoD-nRBS-EcatoA-nRBS-EcatoB-T-EcatoB; Seq-ID-Nr. 16)
2. dem *C*. *necatorphaA-*Gen, kodierend für die Thiolase PhaA (Seq-ID-Nr. 17), incl. der *phaA*-Ribosomenbindestelle, und dem C. *acetobutylicum ctfAB*-Operon, kodierend für die α- und β-Untereinheit der Acetyl-/Butyryl-CoA-Acetoacetat:CoA-Transferase CtfA (Seq-ID-Nr. 18) und CtfB (Seq-ID-Nr. 19), incl. der *ctfA-* bzw. *ctfB-*Ribosomenbindestellen und dem *ctfAB*-Terminator, wobei die für CtfA und CtfB kodierenden Bereiche für die Translation in *C. necator* codonoptimiert wurden (nRBS-RephaA-nRBS-CactfA-nRBS-CactfB-T-CactfAB; Seq-ID-Nr. 20)
3. der Ribosomenbindestelle des *C. necatorgroEL-Gens* (Seq-ID-Nr. 1), dem *C. acetobutylicum thIA-Gen,* kodierend für die Thiolase ThIA (Seq-ID-Nr. 21) sowie dem *C. acetobutylicum ctfAB*-Operon, kodierend für die α- und β-Untereinheit der Acetyl/Butyryl-CoA-Acetoacetat:CoA-Transferase CtfA (Seq-ID-Nr. 18) und CtfB (Seq-ID-Nr. 19), incl. der nativen *ctfA-* bzw. *ctfB*-Ribosomenbindestellen und dem nativen *ctfAB-*Terminator, wobei die für ThIA, CtfA und CtfB kodierenden Bereiche für die Translation in C. *necator* codonoptimiert wurden (RBS-RegroEL-CathIA-nRBS-CactfA-nRBS-CactfB-T-CactfAB; Seq-ID-Nr. 22)
4. der Ribosomenbindestelle des *C. necatorgroEL-Gens* (Seq-ID-Nr. 1), gefolgt vom *C. acetobutylicum thIA-Gen,* kodierend für die Thiolase ThIA (Seq-ID-Nr. 21), der Ribosomenbindestelle des *C. necatorgroEL-Gens* (Seq-ID-Nr. 1), gefolgt vom *H. influenzae ybgC*-Gen, kodierend für die Thioesterase YbgC (Seq-ID-Nr. 23) und schließlich der Ribosomenbindestelle des *C. necatorgroEL-Gens* (Seq-ID-Nr. 1), gefolgt vom *Cupriavidus necator JMP134 acbB*-Gen, kodierend für die Aceton carboxylase beta Untereinheit AcbB (Seq-ID-Nr. 9), der Ribosomenbindestelle des *C. necatorgroEL-Gens* (Seq-ID-Nr. 1), gefolgt vom *Cupriavidus necator JMP134 acbA*-Gen, kodierend für die Aceton carboxylase alpha Untereinheit AcbA (Seq-ID-Nr. 8) und der Ribosomenbindestelle des *C. necatorgroEL-Gens* (Seq-ID-Nr. 1), gefolgt vom *Cupriavidus necator JMP134 acbC*-Gen, kodierend für die Aceton carboxylase gamma Untereinheit AcbC (Seq-ID-Nr. 86) incl. dem *adc*-Terminator, wobei die für ThIA und YbgC kodierenden Bereiche für die Translation in *C. necator* codonoptimiert wurden (RBS-RegroEL-CathIA-RBS-RegroEL-HiybgC-RBS-RegroEL-AcbB- RBS-RegroEL-AcbA- RBS-RegroEL-AcBC-T-Caadc; Seq-ID-Nr. 7)
5. dem *E. coli lacZ*-Promotor (Seq-ID-Nr. 26), der Ribosomenbindestelle des *C. necator groEL*-Gens (Seq-ID-Nr. 1) gefolgt vom *Cupriavidus necator JMP134 acbB*-Gen, kodierend für die Aceton carboxylase beta Untereinheit AcbB (Seq-ID-Nr. 9), der Ribosomenbindestelle des *C. necatorgroEL-Gens* (Seq-ID-Nr. 1), gefolgt vom *Cupriavidus necator JMP134 acbA*-Gen, kodierend für die Aceton carboxylase alpha Untereinheit AcbA (Seq-ID-Nr. 8) und der Ribosomenbindestelle des C. *necator groEL-*Gens (Seq-ID-Nr. 1), gefolgt vom *Cupriavidus necator JMP134 acbC*-Gen, kodierend für die Aceton carboxylase gamma Untereinheit AcbC (Seq-ID-Nr. 86) incl. dem *adc-*Terminator. (Plac-RBS- RegroEL-AcbB- RBS-RegroEL-AcbA- RBS-RegroEL-AcBC-T-Caadc; Seq-ID-Nr. 6)

Die Expressionskassetten nRBS-EcatoD-nRBS-EcatoA-nRBS-EcatoB-T-EcatoB, nRBS-RephaA-nRBS-CactfA-nRBS-CactfB-T-CactfAB, RBS-RegroEL-CathlA-nRBS-CactfA-nRBS-CactfB-T-CactfAB und RBS-RegroEL-CathIA-RBS-RegroEL-HiybgC-RBS-RegroEL- AcbB-RBS-RegroEL-AcbA- RBS-RegroEL-AcBC -T-Caadc wurden dann über *Kpn*I/*Hind*III in den Broad Host Range Expressionsvektor pBBR1 MCS-2 (Seq-ID-Nr. 10) kloniert, so dass die Expression der Gene unter Kontrolle des *E. coli lacZ*-Promotors steht. Die resultierenden Expressionsplasmide wurden als pBBR-EcatoDAB, pBBR-RephaA-CactfAB, pBBR-CathIA-ctfAB und pBBR-CathIA-HiybgC-ReacbBAC bezeichnet und entsprechen den Seq-ID-Nr.s 28, 29, 30 und 5.

Die Expressionskassette Plac- RBS-RegroEL- AcbB- RBS-RegroEL-AcbA- RBS-RegroEL-AcBC -T-Caadc wurde dann über *Hin*dIII/*Bam*HI in die Vektoren pBBR-EcatoDAB, pBBR-RephaA-CactfAB und pBBR-CathIA-ctfAB (Seq-ID-Nr.s 28, 29 und 30) kloniert. Die resultierenden Expressionsplasmide wurden als pBBR-EcatoDAB|ReacbBAC, pBBR-RephaA-CactfAB| ReacbBAC und pBBR-CathIA-ctfAB| ReacbBAC bezeichnet und entsprechen den Seq-ID-Nrs. 4, 3 und 2.

### Ausführungsbeispiel 3: Konstruktion von Plasmiden für die Herstellung von 1-Butanol mit Cupriavidus necator -

Es wurden sieben synthetische Expressionskassetten synthetisiert, die aus folgenden Komponenten bestehen:
1. dem *C. necator phaA-*Gen, kodierend für die Thiolase PhaA (Seq-ID-Nr. 17), incl. der *phaA-*Ribosomenbindestelle, dem *C. necator phaB1-*Gen, kodierend für die (R)-3-Hydroxybutyryl-CoA-Dehydrogenase PhaB1 (Seq-ID-Nr. 35), incl. der *phaB1*-Ribosomenbindestelle, dem *Acinetobacter caviae phaJ*-Gen, kodierend für die (R)-3-Hydroxybutyryl-CoA-Deydratase PhaJ (Seq-ID-Nr. 36), incl. der *phaJ*-Ribosomenbindestelle, der Ribosomenbindestelle des *C. necatorgroEL-Gens* (Seq-ID-Nr. 1), dem *C. acetobutylicum adhE2*-Gen, kodierend für die bifunktionale Butyryl-CoA-/Butyraldehyd-Dehydrogenase AdhE2 (Seq-ID-Nr. 37), sowie dem *A. caviae phaJ*-Terminator (Seq-ID-Nr. 38), wobei die für PhaJ und AdhE2 kodierenden Bereiche für die Translation in *C. necator* codonoptimiert wurden (nRBS-RephaA-nRBS-RephaB1-nRBS-AcphaJ-RBS-RegroEL-CaadhE2-T-AcphaJ; Seq-ID-Nr. 39)
2. dem C. *necator phaA-Gen,* kodierend für die Thiolase PhaA (Seq-ID-Nr. 17), incl. der *phaA-*Ribosomenbindestelle, dem *C. necator phaB1-*Gen, kodierend für die (R)-3-Hydroxybutyryl-CoA-Dehydrogenase PhaB1 (Seq-ID-Nr. 35), incl. der *phaB1*-Ribosomenbindestelle und dem *Acinetobacter caviae phaJ*-Gen, kodierend für die (R)-3-Hydroxybutyryl-CoA-Deydratase PhaJ (Seq-ID-Nr. 36), incl. der *phaJ*-Ribosomenbindestelle sowie dem *Acinetobacter caviae phaJ-*Terminator (Seq-ID-Nr. 38), wobei der für PhaJ kodierende Bereich für die Translation in *C. necator* codonoptimiert wurde (nRBS-RephaA-nRBS-RephaB1-nRBS-AcphaJ-T-AcphaJ; Seq-ID-Nr. 40)
3. der Ribosomenbindestelle des *C. necatorgroEL-Gens* (Seq-ID-Nr. 1), gefolgt vom *C. acetobutylicum thIA-Gen,* kodierend für die Thiolase ThIA (Seq-ID-Nr. 21), der Ribosomenbindestelle des *C. necatorgroEL-Gens* (Seq-ID-Nr. 1), gefolgt vom C. *acetobutylicum hbd*-Gen, kodierend für die (S)-3-Hydroxybutyryl-CoA-Dehydrogenase Hbd (Seq-ID-Nr. 41), der Ribosomenbindestelle des *C. necatorgroEL-Gens* (Seq-ID-Nr. 1), gefolgt vom *C. acetobutylicum adhE2*-Gen, kodierend für die bifunktionale Butyryl-CoA-/Butyraldehyd-Dehydrogenase AdhE2 (Seq-ID-Nr. 37) und dem *C. acetobutylicum ctfAB-*Terminator (Seq-ID-Nr. 42), wobei die für ThIA, Hbd und AdhE2 kodierenden Bereiche für die Translation in C. necator codonoptimiert wurden (RBS-RegroEL-CathIA-RBS-RegroEL-Cahbd-RegroEL-CaadhE2-T-CactfAB; Seq-ID-Nr. 43)
4. der Ribosomenbindestelle des *C. necatorgroEL-Gens* (Seq-ID-Nr. 1), gefolgt vom *C. acetobutylicum thIA-Gen,* kodierend für die Thiolase ThIA (Seq-ID-Nr. 21), der Ribosomenbindestelle des *C. necatorgroEL-Gens* (Seq-ID-Nr. 1), gefolgt vom *C. acetobutylicum hbd*-Gen, kodierend für die (S)-3-Hydroxybutyryl-CoA-Dehydrogenase Hbd (Seq-ID-Nr. 41) und dem *C. acetobutylicum ctfAB*-Terminator (Seq-ID-Nr. 42), wobei die für ThIA und Hbd kodierenden Bereiche für die Translation in *C. necator* codonoptimiert wurden (RBS-RegroEL-CathIA-RBS-RegroEL-Cahbd-T-CactfAB; Seq-ID-Nr. 44)
5. dem *E. coli lacZ*-Promotor (Seq-ID-Nr. 26), dem *C. necator etfBA-bcd-*Operon, kodierend für die β-Untereinheit des Elektron-Transferproteins EtfB (Seq-ID-Nr. 45), die α-Untereinheit des Elektron-Transferproteins EtfA (Seq-ID-Nr. 46) und die Butyryl-CoA-Dehydrogenase Bcd (Seq-ID-Nr. 46), incl. der *etfB-, etfA-* und *bcd*-Ribosomenbindestellen und dem *C. necatoretfBA-bcd-*Terminator (Seq-ID-Nr. 48) (Plac-nRBS-ReetfB-nRBS-ReetfA-nRBS-Rebcd-nT; Seq-ID-Nr. 49)
6. dem *E. coli lacZ*-Promotor (Seq-ID-Nr. 26), dem *C. acetobutylicum crt*-Gen, kodierend für die Crotonase Crt (Seq-ID-Nr. 50), incl. der *crt*-Ribosomenbindestelle, dem *C. acetobutylicum etfBA-bcd*-Operon, kodierend für die β-Untereinheit des Elektron-Transferproteins EtfB (Seq-ID-Nr. 51), die α-Untereinheit des Elektron-Transferproteins EtfA (Seq-ID-Nr. 52) und die Butyryl-CoA-Dehydrogenase Bcd (Seq-ID-Nr. 53), incl. der *etfB*-, *etf*A*-* und *bcd*-Ribosomenbindestellen und dem *C. necator etfBA-bcd*-Terminator (Seq-ID-Nr. 48) (Plac-nRBS-Cacrt-nRBS-CaetfB-nRBS-CaetfA-nRB-Cabcd-T-Rebcd; Seq-ID-Nr. 54)
7. dem *E. coli lacZ*-Promotor (Seq-ID-Nr. 26), dem *C. acetobutylicum etfBA-bcd*-Operon, kodierend für die β-Untereinheit des Elektron-Transferproteins EtfB (Seq-ID-Nr. 51), die α-Untereinheit des Elektron-Transferproteins EtfA (Seq-ID-Nr. 52) und die Butyryl-CoA-Dehydrogenase Bcd (Seq-ID-Nr. 53), incl. der *etfB-, etf*A*-* und *bcd*-Ribosomenbindestellen und dem *C. necatoretfBA-bcd*-Terminator (Seq-ID-Nr. 48) (Plac-nRBS-CaetfB-nRBS-CaetfA-nRB-Cabcd-T-Rebcd; Seq-ID-Nr. 55)

Die Expressionskassetten nRBS-RephaA-nRBS-RephaB1-nRBS-AcphaJ-RBS-RegroEL-CaadhE2-T-AcphaJ, nRBS-RephaA-nRBS-RephaB1-nRBS-AcphaJ-T-AcphaJ, RBS-RegroEL-CathIA-RBS-RegroEL-Cahbd-RegroEL-CaadhE2-T-CactfAB und RBS-RegroEL-CathIA-RBS-RegroEL-Cahbd-T-CactfAB wurden dann über *Kpn*I/*Hind*III in den Broad Host Range Expressionsvektor pBBR1 MCS-2 (Seq-ID-Nr. 10) kloniert, so dass die Expression der Gene unter Kontrolle des *E. coli lacZ*-Promotors steht. Die resultierenden Expressionsplasmide wurden als pBBR-RephaABJ-CaadhE2, pBBR-RephaABJ, pBBR-CathIA-hbd-adhE2-ctfAB und pBBR-CathIA-hbd-ctfAB bezeichnet und entsprechen den Seq-ID-Nrs. 56, 57, 58 und 59. Die Expressionskassette Plac-nRBS-ReetfB-nRBS-ReetfA-nRBS-Rebcd-nT wurde dann über *Hind*III/*Bam*HI in die Vektoren pBBR-RephaABJ-CaadhE2 und pBBR-RephaABJ (Seq-ID-Nr.s 56 und 57) kloniert. Die resultierenden Expressionsplasmide wurden als pBBR-RephaABJ-CaadhE2|ReetfBA-bcd und pBBR-RephaABJ|ReetfBA-bcd bezeichnet und entsprechen den Seq-ID-Nrs. 60 und 61.

Die Expressionskassette Plac-nRBS-Cacrt-nRBS-CaetfB-nRBS-CaetfA-nRB-Cabcd-T-Rebcd wurde dann über *Hind*III/*Bam*HI in die Vektoren pBBR-RephaABJ-CaadhE2, pBBR-RephaABJ, pBBR-CathIA-hbd-adhE2-ctfAB und pBBR-CathIA-hbd-ctfAB (Seq-ID-Nrs. 56, 57, 58 und 59) kloniert. Die resultierenden Expressionsplasmide wurden als pBBR-RephaABJ-CaadhE2|Cacrt-etfBA-Cabcd, pBBR-RephaABJ|Cacrt-etfBA-Cabcd, pBBR-CathIA-hbd-adhE2-ctfAB|crt etfBA-bcd und pBBR-CathIA-hbd-ctfAB1crt-etfBA-bcd bezeichnet und entsprechen den Seq-ID-Nrs. 62, 63, 64 und 65.

Die Expressionskassette Plac-nRBS-CaetfB-nRBS-CaetfA-nRB-Cabcd-T-Rebcd wurde dann über *Hind*III/*BamH*I in die Vektoren pBBR-RephaABJ-CaadhE2 und pBBR-RephaABJ (Seq-ID-Nrs. 56 und 57) kloniert. Die resultierenden Expressionsplasmide wurden als pBBR-RephaABJ-CaadhE2|etfBA-bcd und pBBR-RephaABJ|CaetfBA-bcd bezeichnet und entsprechen den Seq-ID-Nrs. 66 und 67.

### Ausführungsbeispiel 4: Konstruktion von Plasmiden für die Herstellung von Butyrat und 1-Propen mit Cupriavidus necator

Für die Konstruktion von Plasmiden für die Herstellung von Butyrat und 1-Propen mit *C. necator* wurde eine synthetische Expressionskassetten synthetisiert, die aus folgenden Komponenten besteht:
1. dem *C. necator groEL*-Promotor (Seq-ID-Nr. 68), der Ribosomenbindestelle des *C. necator groEL*-Gens (Seq-ID-Nr. 1), gefolgt vom *Jeotgalicoccus* sp. ATCC 8456 *oleT*-Gen, kodierend für die terminale Olefine bildende Fettsäure-Decarboxylase OleT (Seq-ID-Nr. 69), der Ribosomenbindestelle des *C. necatorgroEL-Gens* (Seq-ID-Nr. 1) und schließlich dem *C. acetobutylicum ptb-buk*-Operon, kodierend für die Phosphotransbutyrylase Ptb (Seq-ID-Nr. 70) und die Butyratkinase Buk (Seq-ID-Nr. 71), incl. der *buk*-Ribosomenbindestelle und dem *ptb-buk*-Terminator (Seq-ID-Nr. 72), wobei die für OleT, Ptb und Buk kodierenden Bereiche für die Translation in *C. necator* codonoptimiert wurden (PRegroESL-RBS-RegroEL-JeoleT-RBS-RegroEL-Captb-nRBS-Cabuk-T-Captb-buk; Seq-ID-Nr. 73)

Die Expressionskassette PRegroESL-RBS-RegroEL-JeoleT-RBS-RegroEL-Captb-nRBS-Cabuk-T-Captb-buk wurde dann über *Bam*HI/*Sac*I in die Vektoren pBBR-RephaABJ|ReetfBA-bcd, pBBR-RephaABJ|CaetfBA-bcd und pBBR-CathIA-hbd-ctfAB|crt-etfBA-bcd (Seq-ID-Nrs. 58, 60 und 62) kloniert. Die resultierenden Expressionsplasmide wurden als pBBR-RephaABJ|etfBA-bcd|JeoleT-Captb-buk, pBBR-RephaABJ|CaetfBA-bcd|JeoleT-Captb-buk und pBBR-CathIA-hbd-ctfAB|crt-etfBA-bcd|JeoleT-Captb-buk bezeichnet und entsprechen den Seq-ID-Nrs. 74, 75 und 76.

### Ausführungsbeispiel 5: Konstruktion von Plasmiden für die Herstellung von 2-Propanol mit Cupriavidus necator

Für die Konstruktion von Plasmiden für die Herstellung von 2-Propanol mit *C. necator* wurde eine synthetische Expressionskassette synthetisiert, die aus folgenden Komponenten besteht:
1. dem *C. necator groEL*-Promotor (Seq-ID-Nr. 68), der Ribosomenbindestelle des *C. necator groEL*-Gens (Seq-ID-Nr. 1), gefolgt vom *Clostridium beijerinckii* NRRL B593 *adh*-Gen, kodierend für eine primäre und sekundäre Alkohole oxidierende Alkoholdehydrogenase (Seq-ID-Nr. 11), wobei der für die primäre und sekundäre Alkohole oxidierende Alkoholdehydrogenase kodierende Bereich für die Translation in *C. necator* codonoptimiert wurden (PRegroESL-RBS-Cbadh-T-Rebcd; Seq-ID-Nr. 12)

Die Expressionskassette PRegroESL-RBS-Cbadh-T-Rebcd wurde dann über *Sac*I/*Spe*I in die Vektoren pBBR-EcatoDAB|Caadc, pBBR-RephaA-CactfAB|adc und pBBR-CathIA-ctfAB|adc (Seq-ID-Nrs. 32, 33 und 34) kloniert. Die resultierenden Expressionsplasmide wurden als pBBR-EcatoDAB|Caadc|Cbadh, pBBR-RephaA-CactfABladclCbadh und pBBR-CathIA-ctfABladclCbadh bezeichnet und entsprechen den Seq-ID-Nrs. 83, 84 und 85.

### Ausführungsbeispiel 6: Generierung des Vektors pBBR1MCS-2::HCM-phaA

Der Vektor pBBR1 MCS-2::HCM-phaA wird ausgehend vom Plasmid pBBR1 MCS-2::HCM (Generierung und Eigenschaften in Patentanmeldung EP12173010 beschrieben) erzeugt. Für die Konstruktion wird eine synthetische Expressionskassette synthetisiert, die aus folgenden Komponenten besteht:
1. dem *C. necator phaA-Gen,* kodierend für die Thiolase PhaA (Seq-ID-Nr. 17), incl. der *phaA*-Ribosomenbindestelle; up- und downstream der Sequenz werden Xbal Schnittstellen angefügt (nRBS-RephaA; Seq-ID-Nr. 87).

Das Plasmid pBBR1 MCS-2::HCM wird mit der Restricktionsendonuklease *XbaI* linearisiert und anschließend mit der ebenfalls mittels *Xba*I restringierten und so vorbereiteten Expressionskassette nRBS-RephaA ligiert. Alle molekularbiologischen Arbeiten erfolgen in dem Fachmann bekannter Art und Weise.

Die Expression der Gene erfolgt nach erfolgreicher Klonierung unter Kontrolle des *E. coli lacZ-*Promotors. Das resultierenden Expressionsplasmid wird als pBBR1 MCS-2::HCM-phaA bezeichnet und entspricht der Seq-ID-Nr. 88.

### Ausführungsbeispiel 7: Einbringen von Plasmiden für die Herstellung von, Aceton, 2-Propanol, 1-Butanol, Butyrat, 2-Hydroxyisobuttersäure und 1-Propen in Cupriavidus necator

Die Plasmide werden in kompetente *E. coli* S17-1-Zellen transferiert, einen Stamm, mit dem die konjugative Übertragung von Plasmiden u.a. in *Cupriavidus* necator-Stämme möglich ist. Dazu wird eine Spotmating-Konjugation (wie bei FRIEDRICH et al., 1981, Naturally occurring genetic transfer of hydrogen-oxidizing ability between strains of Alcaligenes eutrophus. J Bacteriol 147:198-205 beschrieben) mit den die jeweiligen Plasmide tragenden *E. coli* S17-1-Stämmen als Donoren und *R. eutopha* H16 (reklassifiziert als *Cupriavidus necator,* DSMZ 428) sowie *R. eutropha* PHB-4 (reklassifiziert als *Cupriavidus necator,* DSMZ 541) als Rezipienten durchgeführt.

Es werden in allen Fällen Transkonjuganten erhalten, die die jeweiligen Plasmide tragen und die entsprechenden Stämme wie folgt bezeichnet:
- *C. necator* H16 pBBR-EcatoDAB|Caadc
- *C. necator* H16 pBBR-RephaA-CactfAB|adc
- *C. necator* H16 pBBR-CathIA-ctfABladc
- *C. necator* H16 pBBR-RephaABJ-CaadhE2
- *C. necator* H16 pBBR-RephaABJ
- *C. necator* H16 pBBR-CathIA-hbd-adhE2-ctfAB
- *C. necator* H16 pBBR-CathIA-hbd-ctfAB
- *C. necator* H16 pBBR-RephaABJ-CaadhE2|ReetfBA-bcd
- *C. necator* H16 pBBR-RephaABJ|ReetfBA-bcd
- *C. necator* H16 pBBR-RephaABJ-CaadhE2|crt-etfBA-bcd
- *C. necator* H16 pBBR-RephaABJ|crt-etfBA-bcd
- *C. necator* H16 pBBR-RephaABJ-CaadhE2|etfBA-bcd
- *C. necator* H16 pBBR-RephaABJ|CaetfBA-bcd
- *C. necator* H16 pBBR-CathIA-hbd-adhE2-ctfABlcrt-etfBA-bcd
- *C. necator* H16 pBBR-CathIA-hbd-ctfAB1crt-etfBA-bcd
- *C. necator* H16 pBBR-RephaABJ|etfBA-bcd|JeoleT-Captb-buk
- *C. necator* H16 pBBR-RephaABJ|CaetfBA-bcd|JeoleT-Captb-buk
- *C. necator* H16 pBBR-CathIA-hbd-ctfAB1crt-etfBA-bcd1JeoleT-Captb-buk
- *C. necator* H16 pBBR-EcatoDAB|Caadc|Cbadh
- *C. necator* H16 pBBR-RephaA-CactfAB|adcl Cbadh
- *C. necator* H16 pBBR-CathIA-ctfABladcl Cbadh
- *C. necator H16* pBBR-EcatoDAB|ReacbBAC
- *C. necator H16* pBBR-RephaA-CactfAB| ReacbBAC
- *C. necator H16* pBBR-CathIA-ctfABI ReacbBAC
- *C. necator H16* pBBR1 MCS-2::HCM-phaA
- *C. necator* PHB-4 pBBR-EcatoDAB|Caadc
- *C. necator* PHB-4 pBBR-RephaA-CactfAB|adc
- *C. necator* PHB-4 pBBR-CathIA-ctfABladc
- *C. necator* PHB-4 pBBR-RephaABJ-CaadhE2
- *C. necator* PHB-4 pBBR-RephaABJ
- *C. necator* PHB-4 pBBR-CathIA-hbd-adhE2-ctfAB
- *C. necator* PHB-4 pBBR-CathIA-hbd-ctfAB
- *C. necator* PHB-4 pBBR-RephaABJ-CaadhE2|ReetfBA-bcd
- *C. necator* PHB-4 pBBR-RephaABJ|ReetfBA-bcd
- *C. necator* PHB-4 pBBR-RephaABJ-CaadhE2|crt-etfBA-bcd
- *C. necator* PHB-4 pBBR-RephaABJ|crt-etfBA-bcd
- *C. necator* PHB-4 pBBR-RephaABJ-CaadhE2IetfBA-bcd
- *C. necator* PHB-4 pBBR-RephaABJ|CaetfBA-bcd
- *C. necator* PHB-4 pBBR-CathIA-hbd-adhE2-ctfAB1crt-etfBA-bcd
- *C. necator* PHB-4 pBBR-CathIA-hbd-ctfAB1crt-etfBA-bcd
- *C. necator* PHB-4 pBBR-RephaABJetfBA-bcd|JeoleT-Captb-buk
- *C. necator* PHB-4 pBBR-RephaABJ|CaetfBA-bcd|JeoleT-Captb-buk
- *C. necator* PHB-4 pBBR-CathIA-hbd-ctfAB1crt-etfBA-bcd1JeoleT-Captb-buk
- *C. necator* PHB-4 pBBR-EcatoDAB|CaadclCbadh
- *C. necator* PHB-4 pBBR-RephaA-CactfAB|adc| Cbadh
- *C. necator* PHB-4 pBBR-CathIA-ctfABladcl Cbadh
- *C. necator PH8-4* pBBR-EcatoDAB|ReacbBAC
- *C. necator PHB-4* pBBR-RephaA-CactfAB| ReacbBAC
- *C. necator PH8-4* pBBR-CathIA-ctfABI ReacbBAC
- *C. necator PHB-4* pBBR1 MCS-2::HCM-phaA

### Ausführungsbeispiel 8: Quantifizierung von Aceton, 2-Propanol, 1-Butanol, 2-Hydroxyisobuttersäure, Butyrat und 1-Propen

### Butyrat und Butanol

Die Quantifizierung von Butanol und Butyrat aus einer Fermentationsbrühe erfolgt mittels HPLC/RID und DAD.

Jeweils 2 mL Fermentationsprobe werden in einem 2 mL Eppendorff-Reaktionsgefäß für 5 min bei 13000 rpm abzentrifugiert. Anschließend wird der Überstand über einen 0,2 µm Spritzenvorsatzfilter steril in ein HPLC-Vial filtriert. Gegebenenfalls müssen die Proben entsprechend dem Messbereich verdünnt werden.

Die Messung erfolgt unter den folgenden Bedingungen:

| | |
|---|---|
| • HPLC | Agilent 1200, Agilent Technologies, Waldbronn |
| • Mobile Phase: | Mobile Phase A1: H₂O (Millipore) + 5 mM wässerige H₂SO₄ |
| • Gradient: | Isokratisch |
| • Säule: | Supelcogel C-610H (9 µm Partikelgröße, L x I.D. 30 cm x 7,8 mm) |
| | Best.Nr.: 59320-U (Fa. Aldrich) |
| • Vorsäule: | Supelcogel H Guard Column (9 µm Partikelgröße, L x I.D. 5 cm x 4,6 mm) Best.Nr.: 59319 (Fa. Aldrich) |
| • Säulentemperatur: | 80 °C |
| • Fluss: | 1,0 mL/min |
| • Detektor: | RID |
| | DAD (210 nm) |
| • Injektionsvolumen: | 20 µL, "Flush Sovent" Injektor Nadel: Isopropanol/Wasser (1:1) |
| • Laufzeit: | 27 min |
| • Messbereich: | Für alle Analyten ca. 0,100 g/L - 12,0 g/L |

### Kalibrierung und Auswertung:

Standardsubstanzen von Butanol und Butyrat (~ 20 mg je Analyt) werden zusammen in einen 10 mL Messkolben eingewogen. Der Messkolben wird mit LM (Millipore Wasser) bis zum Eichstrich aufgefüllt (Lösung S1: Stammlösung). Aus der Stammlösung wird durch Verdünnen mit Millipore Wasser eine 5-Punkt Kalibrierung angesetzt. 1 mL S1 wird in einen 10 mL Messkolben pipettiert und mit LM bis zum Eichstrich aufgefüllt. In jeder Messreihe werden vor und nach den Proben jeweils die Kalibrierstandards in HPLC-Vials vermessen. Für die Auswertung werden beide Kalibrierreihen gemittelt. Die Auswertung von Butyrat erfolgt über das DAD Signal bei 210 nm. Butanol wird im RID Chromatogramm ausgewertet.

### 2-Hydroxyisobuttersäure

Die Bestimmung erfolgt mittels quantitativer ¹H-NMR-Spectroskopie. Als interner Quantifizierungsstandard dient Trimethylpropionsäure.

### Aceton, 2-Propanol und 1-Propen

Die Bestimmung der Analyten Aceton, Isopropanol und 1-Propen in der wässerigen Phase erfolgt mittels Headspace GC/FID Messung und Standardaddition. Die wichtigsten chromatographischen Parameter sind in der nachfolgenden Tabelle zusammengefasst.

| | |
|---|---|
| Säule | DB-Wax, 30 m x 250 µm, Filmdicke: 0,25 µm |
| GC System | Agilent 7890 |
| Gasfluss | He, 0,9 mL/min |
| Säulenofen | Equilibrierzeit: 0,5 min, Temperaturgradient: 0-4 min: 40 °C, 5 °C/min von 50 bis 130 °C, 30 °C/min von 130 bis 250 °C, 250 °C für 12 min |
| Detektor | FID, 250 °C |
| Injektion | Headspace, Temperatur: 90 °C, Septum Purge Flow 2 mL/min, Split 1:2 |
| Kalibrierung | Die Kalibrierung erfolgt über Standardaddition mit den entsprechenden Analyten (interne 1-Punkt Kalibrierung), linearer Messbereich 1-100 mg/L |

### Ausführungsbeispiel 9: Herstellung von Aceton, 2-Propanol, 1-Butanol, Butyrat und 1-Propen mit rekombinanten Ralstonia eutropha-Zellen

Die in Beispiel 7 beschriebenen plasmidtragenden *C. necator*-Stämme werden zur Untersuchungen der Bildung von Aceton, 2-Propanol, 1-Butanol, Butyrat und 1-Propen zur Vorkultur in 2 x 250ml Schüttelkolben mit Schikanen in 25 ml Medium nach Vollbrecht *et al.,* 1978 kultiviert. Das Medium besteht aus (NH₄)₂HPO₄ 2,0 g/l; KH₂PO₄ 2,1 g/l; MgSO₄ × 7 H₂0 0,2 g/l; FeCl₃ × 6 H₂0 6 mg/l; CaCl₂ × 2 H₂0 10 mg; Spurenelement-Lösung (Pfennig and Lippert, 1966) 0,1 ml. Die Spurenelementlösung setzt sich aus Titriplex III 10 g/l, FeSO₄ x 7 H₂O 4 g/l, ZnSO₄ x 7 H₂O 0,2 g/l, MnCl₂ x 4 H₂O 60 mg/l, H₃BO₃ 0,6 g/l, CoCl₂ x 6 H₂O 0,4 g/l, CuCl₂ x 2 H₂O 20 mg/l, NiCl₂ x 6 H₂O 40 mg/l, Na₂Mo₄ x 2 H₂O 60 mg/l zusammen. Das Medium der Vorkultur wurde zusätzlich mit Fructose 5 g/l, Kanamycin 300 µg/ml supplementiert. Die Kolben wurden 1 %ig (v/v) aus einer Cryokultur angeimpft. Die Kulturen werden auf einem Schüttler bei 30 °C und 150 rpm für 24h inkubiert. Danach werden die Kulturen vereinigt und eine OD₆₀₀ von ca. 3,5 bestimmt.

Die Hauptkultur erfolgt chemolithoautotroph in einem 2I Edelstahl - Reaktor, Biostat B von Sartorius, gefüllt mit 1-2 I Medium mit der Zusammensetzung (NH₄)₂HPO₄ 2,0 g/l, KH₂PO₄ 2,1 g/l, MgSO₄ × 7 H₂O 3 g/l, FeCl₃ × 6 H₂O 6 mg/l, CaCl₂ × 2 H₂O 10 mg, Biotin 1 mg/l, Thiamin-HCl 1mg/l, Ca-Pantothenat 1mg/l, Nicotinsäure 20mg/l, Spurenelement-Lösung 0,1 ml und Polypropylenglykol (PPG 1000 1:5 mit Wasser verdünnt).

Die Kultivierung erfolgt bei 30 °C, 500 - 1500 rpm, pH 7. Der pH wird einseitig mit 1 M NaOH geregelt. Die Begasung erfolgt mit einem Gasgemisch aus H₂ 90 %, CO₂ 6%, O₂ 4 % bei einem Überduck von 0-2 bar mit einer Begasungsrate von 0,19 vvm. Der Reaktor wird mit 0,1 % der Vorkultur angeimpft. Dazu wird das benötigte Volumen der Vorkultur in 50 ml Falcontubes 10 min, bei 20 °C und 4500 rpm abzentrifugiert und in 10 ml phosphatgepufferten Salzlösung resuspendiert. Das Waschen wird 3x wiederholt, das letzte Resuspendieren erfolgt in 10 ml Hauptkulturmedium. Die Kultivierungsdauer beträgt 76-150 h. Zur Probennahme werden über ein Septum mit einer Spritze mit steriler Kanüle jeweils 10 ml Probe entnommen. Bestimmt wird OD₆₀₀, BTM und das je nach Stamm zu erwartende Produkt nach Beispiel 7.

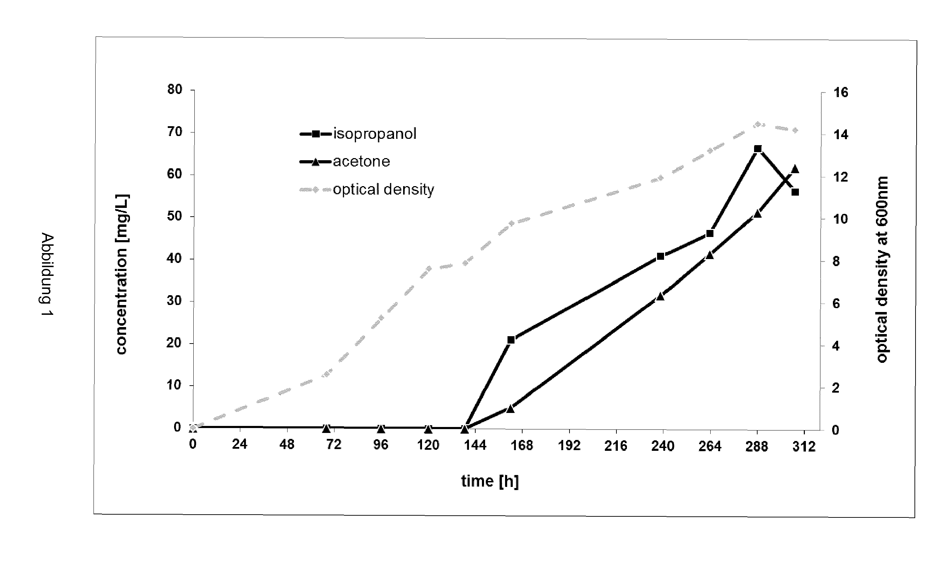

Nach Kultivierung folgender Stämme kann die Bildung von Aceton nachgewiesen werden:
- *C. necator* H16 pBBR-EcatoDAB|Caadc (siehe Abbildung 1)
- *C. necator* H16 pBBR-RephaA-CactfAB|adc
- *C. necator* H16 pBBR-CathIA-ctfABladc
- *C. necator* PHB-4 pBBR-EcatoDAB|Caadc
- *C. necator* PHB-4 pBBR-RephaA-CactfAB|adc
- *C. necator* PHB-4 pBBR-CathIA-ctfABladc

Nach Kultivierung folgender Stämme kann die Bildung von 2-Propanol nachgewiesen werden:
- *C. necator* H16 pBBR-EcatoDAB|Caadc (siehe Abbildung 1)
- *C. necator* H16 pBBR-EcatoDAB|Caadc|Cbadh
- *C. necator* H16 pBBR-RephaA-CactfAB|adc| Cbadh
- *C. necator* H16 pBBR-CathIA-ctfABladcl Cbadh
- *C. necator* PHB-4 pBBR-EcatoDAB|Caadc|Cbadh
- *C. necator* PHB-4 pBBR-RephaA-CactfAB|adc| Cbadh
- *C. necator* PHB-4 pBBR-CathIA-ctfAB|adc| Cbadh

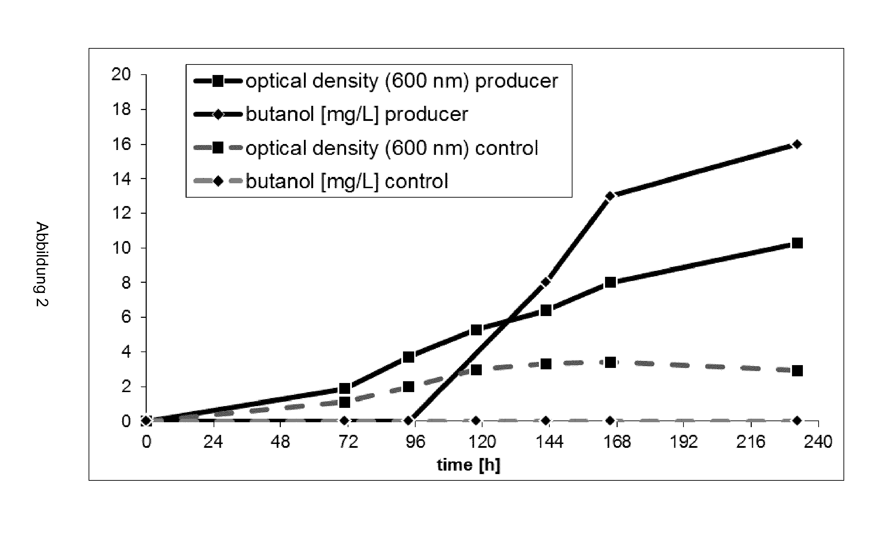

Nach Kultivierung folgender Stämme kann die Bildung von 1-Butanol nachgewiesen werden:
- *C. necator H16* pBBR-RephaABJ-CaadhE2 (siehe producer Abbildung 2)
- *C. necator* H16 pBBR-RephaABJ
- *C. necator* H16 pBBR-CathIA-hbd-adhE2-ctfAB
- *C. necator* H16 pBBR-CathIA-hbd-ctfAB
- *C. necator* H16 pBBR-RephaABJ-CaadhE2|ReetfBA-bcd
- *C. necator* H16 pBBR-RephaABJ|ReetfBA-bcd
- *C. necator* H16 pBBR-RephaABJ-CaadhE2|crt-etfBA-bcd
- *C. necator* H16 pBBR-RephaABJ|crt-etfBA-bcd
- *C. necator* H16 pBBR-RephaABJ-CaadhE2|etfBA-bcd
- *C. necator* H16 pBBR-RephaABJ|CaetfBA-bcd
- *C. necator* H16 pBBR-CathIA-hbd-adhE2-ctfAB|crt-etfBA-bcd
- *C. necator* H16 pBBR-CathIA-hbd-ctfAB|crt-etfBA-bcd
- *C. necator* PHB-4 pBBR-RephaABJ-CaadhE2
- *C. necator* PHB-4 pBBR-RephaABJ
- *C. necator* PHB-4 pBBR-CathIA-hbd-adhE2-ctfAB
- *C. necator* PHB-4 pBBR-CathIA-hbd-ctfAB
- *C. necator* PHB-4 pBBR-RephaABJ-CaadhE2|ReetfBA-bcd
- *C. necator* PHB-4 pBBR-RephaABJ|ReetfBA-bcd
- *C. necator* PHB-4 pBBR-RephaABJ-CaadhE2|crt-etfBA-bcd
- *C. necator* PHB-4 pBBR-RephaABJ|crt-etfBA-bcd
- *C. necator* PHB-4 pBBR-RephaABJ-CaadhE2|etfBA-bcd
- *C. necator* PHB-4 pBBR-RephaABJ|CaetfBA-bcd
- *C. necator* PHB-4 pBBR-CathIA-hbd-adhE2-ctfAB1crt-etfBA-bcd
- *C. necator* PHB-4 pBBR-CathIA-hbd-ctfAB1crt-etfBA-bcd

Nach Kultivierung folgender Stämme kann die Bildung von Butyrat nachgewiesen werden:
- *C. necator H16* p B B R-RephaABJ|etfBA-bcd|JeoleT-Ca ptb-buk
- *C. necator H16* pBBR-RephaABJ|CaetfBA-bcd|JeoleT-Captb-buk
- *C. necator H16* pBBR-CathlA-hbd-ctfAB|crt-etfBA-bcd]JeoleT-Captb-buk
- *C. necator* PHB-4 pBBR-RephaABJ|etfBA-bcd|JeoleT-Captb-buk
- *C. necator* PHB-4 pBBR-RephaABJ|CaetfBA-bcd|JeoleT-Captb-buk
- *C. necator* PHB-4 pBBR-CathIA-hbd-ctfAB|crt-etfBA-bcd|JeoleT-Captb-buk

Nach Kultivierung folgender Stämme kann die Bildung von 1-Propen nachgewiesen werden:
- *C. necator* H16 pBBR-RephaABJ|etfBA-bcd|JeoleT-Captb-buk
- *C. necator* H16 pBBR-RephaABJ|CaetfBA-bcd|JeoleT-Captb-buk
- *C. necator* H16 pBBR-CathIA-hbd-ctfAB|crt-etfBA-bcd|JeoleT-Captb-buk
- *C. necator* PHB-4 pBBR-RephaABJ|etfBA-bcd|JeoleT-Captb-buk
- *C. necator* PHB-4 pBBR-RephaABJ|CaetfBA-bcd|JeoleT-Captb-buk
- *C. necator* PHB-4 pBBR-CathIA-hbd-ctfAB|crt-etfBA-bcd|JeoleT-Captb-buk

Nach Kultivierung folgender Stämme kann die Bildung von 2-Hydroxyisobuttersäure nachgewiesen werden:
- *C. necator PHB-4* pBBR1MCS-2::HCM-phaA
- *C. necator H16* pBBR1MCS-2::HCM-phaA

## Patentansprüche

1. Verfahren zur Herstellung von organischen Verbindungen umfassend die Verfahrensschritte
A) Bereitstellen eines Knallgasbakteriums mit einer verglichen zu seinem Wildtyp gesteigerten Aktivität eines Enzyms E₁, welches die Umsetzung
2 Acetyl-CoA zu Acetoacetyl-CoA und CoA
zu katalysieren vermag, in einem wässrigen Medium
B) in Kontakt Bringen des wässrigen Mediums mit einem Gas enthaltend H₂, CO₂ und O₂
in einem Gewichtsverhältnis von 20 bis 70 zu 10 bis 45 zu 5 bis 35, insbesondere von 30 bis 55 zu 15 bis 40 zu 10 bis 30, und gegebenenfalls
C) Aufreinigen der organischen Verbindung.

2. Verfahren gemäß Anspruch 1,
**dadurch gekennzeichnet,**
**dass** das Knallgasbakterium ausgewählt ist aus der Liste der Gattungen *Achromobacter, Acidithiobacillus, Acidovorax, Alcaligenes, Anabena, Aquifex, Arthrobacter, Azospirillum, Bacillus, Bradyrhizobium, Cupriavidus, Derxia, Helicobacter, Herbaspirillum, Hydrogenobacter, Hydrogenobaculum, Hydrogenophaga" Hydrogenophilus, Hydrogenothermus, Hydrogenovibrio, Ideonella sp. 01, Kyrpidia, Metallosphaera, Methanobrevibacter, Myobacterium, Nocardia, Oligotropha, Paracoccus, Pelomonas, Polaromonas, Pseudomonas, Pseudonocardia, Rhizobium, Rhodococcus, Rhodopseudomonas, Rhodospirillum, Streptomyces, Treponema, Thiocapsa, Variovorax, Xanthobacter, Wautersia,* wobei *Cupriavidus* besonders bevorzugt ist.

3. Verfahren gemäß Anspruch 1 oder 2,
**dadurch gekennzeichnet,**
**dass** das Enzym E₁ ausgewählt ist aus Acetyl-CoA:Acetyl-CoA C-Acetyltransferasen aus der Enzymklasse EC 2.3.1.9.

4. Verfahren gemäß mindestens einem der vorhergehenden Ansprüche,
**dadurch gekennzeichnet,**
**dass** das Enzym E₁ ausgewählt ist aus AAC26023.1, ABR35750.1 und ABR25255.1 sowie Proteine mit einer Polypeptidsequenz, bei der bis zu 60 % der Aminosäurereste gegenüber den vorgenannten Bezugssequenzen durch Deletion, Insertion, Substitution oder eine Kombination davon verändert sind.

5. Verfahren gemäß mindestens einem der vorhergehenden Ansprüche,
**dadurch gekennzeichnet,**
**dass** das Gas in Verfahrensschritt B) Synthesegas enthält.

6. Verfahren gemäß mindestens einem der vorhergehenden Ansprüche,
**dadurch gekennzeichnet,**
**dass** das Synthesegas in Verfahrensschritt B) mindestens 50 Gew.-%, bevorzugt mindestens 70 Gew.-%, besonders bevorzugt mindestens 90 Gew.-% aller Kohlenstoffquellen, die dem Knallgasbakterium in Verfahrensschritt B) zur Verfügung stehen, ausmacht.

7. Verfahren gemäß mindestens einem der Ansprüche 1-4,
**dadurch gekennzeichnet,**
**dass** das CO₂ in Verfahrensschritt B) mindestens 50 Gew.-%, bevorzugt mindestens 70 Gew.-%, besonders bevorzugt mindestens 90 Gew.-% aller Kohlenstoffquellen, die dem Knallgasbakterium in Verfahrensschritt B) zur Verfügung stehen, ausmacht.

8. Verfahren gemäß mindestens einem der vorhergehenden Ansprüche,
**dadurch gekennzeichnet,**
**dass** die organische Substanz Butanol, Buten, Propen oder 2-Hydroxyisobuttersäure ist, und das Knallgasbakterium eine verglichen zu seinem Wildtyp gesteigerten Aktivität eines Enzyms E₂, welches die Umsetzung
von Acetoacetyl-CoA und NADH oder NADPH zu 3-Hydroxybutyryl-CoA und NAD+ oder NADP+
zu katalysieren vermag, aufweist.

9. Verfahren gemäß Anspruch 8,
**dadurch gekennzeichnet,**
**dass** das Enzym E₂ ausgewählt ist aus 3-Hydroxybutyryl-CoA Dehydrogenasen aus der Enzymklasse EC:1.1.1.157.

10. Verfahren gemäß Anspruch 9,
**dadurch gekennzeichnet,**
**dass** das Enzym E₂ ausgewählt ist aus NP_349314.1, YP_001307469.1 und CAQ53138.1 sowie Proteine mit einer Polypeptidsequenz, bei der bis zu 60 % der Aminosäurereste gegenüber den vorgenannten Bezugssequenzen durch Deletion, Insertion, Substitution oder eine Kombination davon verändert sind.

11. Verfahren gemäß mindestens einem der vorhergehenden Ansprüche,
**dadurch gekennzeichnet,**
**dass** die organische Substanz Butanol, Buten, Buttersäure oder Propen ist, und das Knallgasbakterium eine verglichen zu seinem Wildtyp gesteigerten Aktivität eines Enzyms E₃, welches die Umsetzung
von 3-Hydroxybutyryl-CoA zu Crotonyl-CoA und Wasser
zu katalysieren vermag, aufweist.

12. Verfahren gemäß Anspruch 11,
**dadurch gekennzeichnet,**
**dass** das Enzym E₃ ausgewählt ist aus 3-Hydroxybutyryl-CoA Dehydratasen aus der Enzymklasse EC:4.2.1.55.

13. Verfahren gemäß Anspruch 12,
**dadurch gekennzeichnet,**
**dass** das Enzym E₃ ausgewählt ist aus NP_349318.1, YP_001307465.1 und CAQ53134.1 sowie Proteine mit einer Polypeptidsequenz, bei der bis zu 60 % der Aminosäurereste gegenüber den vorgenannten Bezugssequenzen durch Deletion, Insertion, Substitution oder eine Kombination davon verändert sind.

14. Verfahren gemäß mindestens einem der vorhergehenden Ansprüche,
**dadurch gekennzeichnet,**
**dass** die organische Substanz Butanol, Buten, Buttersäure oder Propen ist, und das Knallgasbakterium eine verglichen zu seinem Wildtyp gesteigerten Aktivität eines Enzyms E₄, welches die Umsetzung
von Crotonyl-CoA und NADH oder NADPH zu Butyryl-CoA und NAD+ oder NADP+ zu katalysieren vermag, aufweist.

15. Verfahren gemäß Anspruch 14,
**dadurch gekennzeichnet,**
**dass** das Enzym E₄ ausgewählt ist aus Butyryl-CoA Dehydrogenasen aus der Enzymklasse EC:1.3.99.2.

16. Verfahren gemäß Anspruch 15,
**dadurch gekennzeichnet,**
**dass** das Enzym E₄ ausgewählt ist aus NP_349317.1, YP_001307466.1 und CAQ53135.1 sowie Proteine mit einer Polypeptidsequenz, bei der bis zu 60 % der Aminosäurereste gegenüber den vorgenannten Bezugssequenzen durch Deletion, Insertion, Substitution oder eine Kombination davon verändert sind.

17. Verfahren gemäß mindestens einem der vorhergehenden Ansprüche,
**dadurch gekennzeichnet,**
**dass** die organische Substanz Butanol oder Buten ist, und das Knallgasbakterium eine verglichen zu seinem Wildtyp gesteigerten Aktivität eines Enzyms E₅, welches die Umsetzung
von Butyryl-CoA und NADH oder NADPH zu Butyraldehyd, NAD+ oder NADP+ und HS-CoA oder
die Umsetzungen
von Butyryl-CoA und NADH oder NADPH zu Butyraldehyd, NAD+ oder NADP+ und HS-CoA und
von Butyraldehyd und NADH oder NADPH zu n-Butanol und NAD+ oder NADP+ zu katalysieren vermag, aufweist.

18. Verfahren gemäß Anspruch 17,
**dadurch gekennzeichnet,**
**dass** das Enzym E₅ ausgewählt ist aus bifunktionalen Aldehyd/Alkohol Dehydrogenasen aus der Enzymklasse EC:1.2.1.10 oder EC:1.1.1.1 oder aus Butyraldehyd Dehydrogenasen aus der Enzymklasse EC:1.2.1.10.

19. Verfahren gemäß Anspruch 18,
**dadurch gekennzeichnet,**
**dass** das Enzym E₅ ausgewählt ist aus NP_149199.1, NP_563447.1 und YP_002861217.1, YP_001310903.1 und CAQ57983.1 sowie Proteine mit einer Polypeptidsequenz, bei der bis zu 60 % der Aminosäurereste gegenüber den vorgenannten Bezugssequenzen durch Deletion, Insertion, Substitution oder eine Kombination davon verändert sind.

20. Verfahren gemäß mindestens einem der vorhergehenden Ansprüche,
**dadurch gekennzeichnet,**
**dass** die organische Substanz Butanol oder Buten ist, und das Knallgasbakterium eine verglichen zu seinem Wildtyp gesteigerten Aktivität eines Enzyms E₆, welches die Umsetzung
von Butyraldehyd und NAD(P)H zu n-Butanol und NAD(P)+ zu katalysieren vermag, aufweist.

21. Verfahren gemäß Anspruch 20,
**dadurch gekennzeichnet,**
**dass** das Enzym E₆ ausgewählt ist aus Butanol Dehydrogenasen aus der Enzymklasse EC:1.1.1.-.

22. Verfahren gemäß Anspruch 21,
**dadurch gekennzeichnet,**
**dass** das Enzym E₆ ausgewählt ist aus YP_001310904.1, YP_001310904 und CAQ53139.1 sowie Proteine mit einer Polypeptidsequenz, bei der bis zu 60 % der Aminosäurereste gegenüber den vorgenannten Bezugssequenzen durch Deletion, Insertion, Substitution oder eine Kombination davon verändert sind.

23. Verfahren gemäß mindestens einem der Ansprüche 20-22,
**dadurch gekennzeichnet,**
**dass** das Knallgasbakterium eine verglichen zu seinem Wildtyp gesteigerten Aktivität eines Enzyms E₇ ausgewählt aus Elektrontransfer-Flavoproteinen aus der Enzymklasse EC:2.8.3.9 aufweist.

24. Verfahren gemäß Anspruch 23,
**dadurch gekennzeichnet,**
**dass** das Enzym E₇ ein heterodimeres Enzym, aufgebaut aus zwei Untereinheiten, ist, bei dem die
alpha-Untereinheit ausgewählt ist aus NP_349315.1, YP_001307468.1 und CAQ53137.1 sowie Proteine mit einer Polypeptidsequenz, bei der bis zu 60 % der Aminosäurereste gegenüber den vorgenannten Bezugssequenzen durch Deletion, Insertion, Substitution oder eine Kombination davon verändert sind,
und die
beta-Untereinheit ausgewählt ist aus NP_349316.1, YP_001307467.1 und CAQ53136.1 sowie Proteine mit einer Polypeptidsequenz, bei der bis zu 60 % der Aminosäurereste gegenüber den vorgenannten Bezugssequenzen durch Deletion, Insertion, Substitution oder eine Kombination davon verändert sind.

25. Verfahren gemäß mindestens einem der vorhergehenden Ansprüche,
**dadurch gekennzeichnet,**
**dass** die organische Substanz Buten ist, und das Knallgasbakterium eine verglichen zu seinem Wildtyp gesteigerten Aktivität eines Enzyms E₈, welches die Umsetzung von n-Butanol zu 1-Buten und Wasser
zu katalysieren vermag, aufweist.

26. Verfahren gemäß Anspruch 25,
**dadurch gekennzeichnet,**
**dass** das Enzym E₈ ausgewählt ist aus Oleat Hydratasen aus der Enzymklasse EC:4.2.1.53, Kieviton Hydratasen aus der Enzymklasse EC:4.2.1.95 und Phaseollidin Hydratasen aus der Enzymklasse EC:4.2.1.97.

27. Verfahren gemäß Anspruch 26,
**dadurch gekennzeichnet,**
**dass** das Enzym E₈ ausgewählt ist aus ACT54545, OLHYD_STRPZ, OLHYD_FLAME und AAA87627.1, sowie Proteine mit einer Polypeptidsequenz, bei der bis zu 60 % der Aminosäurereste gegenüber den vorgenannten Bezugssequenzen durch Deletion, Insertion, Substitution oder eine Kombination davon verändert sind.

28. Verfahren gemäß mindestens einem der Ansprüche 1-16,
**dadurch gekennzeichnet,**
**dass** die organische Substanz Propen oder Buttersäure ist, und das Knallgasbakterium eine verglichen zu seinem Wildtyp gesteigerten Aktivität eines Enzyms E₉, welches die Umsetzung
von Butyryl-CoA und Pᵢ zu Butyryl-Phosphat und HS-CoA
zu katalysieren vermag, aufweist.

29. Verfahren gemäß Anspruch 28,
**dadurch gekennzeichnet,**
**dass** das Enzym E₉ ausgewählt ist aus Phosphat Butyryltransferasen aus der Enzymklasse EC:2.3.1.19.

30. Verfahren gemäß Anspruch 29,
**dadurch gekennzeichnet,**
**dass** das Enzym E₉ ausgewählt ist aus ABR32393.1 und ZP_05394269.1 sowie Proteine mit einer Polypeptidsequenz, bei der bis zu 60 % der Aminosäurereste gegenüber den vorgenannten Bezugssequenzen durch Deletion, Insertion, Substitution oder eine Kombination davon verändert sind.

31. Verfahren gemäß mindestens einem der Ansprüche 1-16 oder 28-30,
**dadurch gekennzeichnet,**
**dass** die organische Substanz Propen oder Buttersäure ist, und das Knallgasbakterium eine verglichen zu seinem Wildtyp gesteigerten Aktivität eines Enzyms E₁₀, welches die Umsetzung
von Butyryl-Phosphat und ADP zu Butyrat und ATP
zu katalysieren vermag, aufweist.

32. Verfahren gemäß Anspruch 31,
**dadurch gekennzeichnet,**
**dass** das Enzym E₁₀ ausgewählt ist aus Butyratkinasen aus der Enzymklasse EC:2.7.2.7.

33. Verfahren gemäß Anspruch 32,
**dadurch gekennzeichnet,**
**dass** das Enzym E₁₀ ausgewählt ist aus ABR32394.1 und ZP_05392467.1 sowie Proteine mit einer Polypeptidsequenz, bei der bis zu 60 % der Aminosäurereste gegenüber den vorgenannten Bezugssequenzen durch Deletion, Insertion, Substitution oder eine Kombination davon verändert sind.

34. Verfahren gemäß mindestens einem der Ansprüche 1-16 oder 28-33,
**dadurch gekennzeichnet,**
**dass** die organische Substanz Propen ist, und das Knallgasbakterium eine verglichen zu seinem Wildtyp gesteigerten Aktivität eines Enzyms E₁₁, welches die Umsetzung von Butyrat und H₂O₂ zu Propen und H₂O
zu katalysieren vermag, aufweist.

35. Verfahren gemäß Anspruch 34,
**dadurch gekennzeichnet,**
**dass** das Enzym E₁₁ ausgewählt ist aus Cytochrom P450 der CYP152-Familie.

36. Verfahren gemäß Anspruch 35,
**dadurch gekennzeichnet,**
**dass** das Enzym E₁₁ ausgewählt ist aus HQ709266.1, NP_388092,1 und NP_739069.1 sowie Proteine mit einer Polypeptidsequenz, bei der bis zu 60 % der Aminosäurereste gegenüber den vorgenannten Bezugssequenzen durch Deletion, Insertion, Substitution oder eine Kombination davon verändert sind.

37. Verfahren gemäß mindestens einem der Ansprüche 1-7,
**dadurch gekennzeichnet,**
**dass** die organische Substanz ausgewählt ist aus Aceton, 2-Propanol und Propen, und das Knallgasbakterium eine verglichen zu seinem Wildtyp gesteigerten Aktivität eines Enzyms E₁₂, welches die Umsetzung
von Acetoacetyl-CoA zu Acetoacetat und CoA
zu katalysieren vermag, aufweist.

38. Verfahren gemäß Anspruch 37,
**dadurch gekennzeichnet,**
**dass** das Enzym E₁₂ ausgewählt ist aus Acetoacetyl-CoA:Acetat/Acyl:CoA Transferasen aus der Enzymklasse EC:3.1.2.11, aus Butyrat-Acetoacetat-CoA-Transferasen aus der Enzymklasse EC:2.8.3.9 oder aus Acyl-CoA-Hydrolasen aus der Enzymklasse EC:3.1.2.20.

39. Verfahren gemäß Anspruch 38,
**dadurch gekennzeichnet,**
**dass** das Enzym E₁₂ ausgewählt ist aus
den heterodimeren Acetoacetyl-CoA:Acetat/Acyl:CoA Transferasen aufgebaut aus zwei
Untereinheiten, bei denen
die alpha-Untereinheit ausgewählt ist aus NP_149326.1, YP_001310904.1 und CAQ57984.1
und die
beta-Untereinheit ausgewählt ist aus NP_149327.1, YP_001310905.1 und CAQ57985.1
sowie Proteine mit einer Polypeptidsequenz, bei der bis zu 60 % der Aminosäurereste gegenüber den vorgenannten Bezugssequenzen durch Deletion, Insertion, Substitution oder eine Kombination davon verändert sind,
den Butyrat-Acetoacetat-CoA-Transferasen ctfA und ctfB aus *Clostridium acetobutylicum* und atoD und atoA aus *Escherichia coli* sowie Proteine mit einer Polypeptidsequenz, bei der bis zu 60 % der Aminosäurereste gegenüber den vorgenannten Bezugssequenzen durch Deletion, Insertion, Substitution oder eine Kombination davon verändert sind, und
den Acyl-CoA Hydrolasen tell aus *B. subtilis* bzw. ybgC aus *Heamophilus influenzae* sowie Proteine mit einer Polypeptidsequenz, bei der bis zu 60 % der Aminosäurereste gegenüber den vorgenannten Bezugssequenzen durch Deletion, Insertion, Substitution oder eine Kombination davon verändert sind.

40. Verfahren gemäß mindestens einem der Ansprüche 1-7 oder 37-39,
**dadurch gekennzeichnet,**
**dass** die organische Substanz ausgewählt ist aus Aceton, 2-Propanol und Propen, und das Knallgasbakterium eine verglichen zu seinem Wildtyp gesteigerten Aktivität eines Enzyms E₁₃, welches die Umsetzung
von Acetoacetat zu Aceton und CO₂
zu katalysieren vermag, aufweist.

41. Verfahren gemäß Anspruch 40,
**dadurch gekennzeichnet,**
**dass** das Enzym E₁₃ ausgewählt ist aus Acetoacetat Decarboxylasen aus der Enzymklasse EC:4.1.1.4 oder aus Aceton:CO2 Ligasen aus der Enzymklasse EC 6.4.1.6.

42. Verfahren gemäß Anspruch 41,
**dadurch gekennzeichnet,**
**dass** das Enzym E₁₃ ausgewählt ist aus NP_149328.1, YP_001310906.1 und CAQ57986.1 sowie Proteine mit einer Polypeptidsequenz, bei der bis zu 60 % der Aminosäurereste gegenüber den vorgenannten Bezugssequenzen durch Deletion, Insertion, Substitution oder eine Kombination davon verändert sind.

43. Verfahren gemäß mindestens einem der Ansprüche 1-7 oder 37-42,
**dadurch gekennzeichnet,**
**dass** die organische Substanz ausgewählt ist aus 2-Propanol und Propen, und das Knallgasbakterium eine verglichen zu seinem Wildtyp gesteigerten Aktivität eines Enzyms E₁₄, welches die Umsetzung
von Aceton, NADPH und H+ zu Propan-2-ol + NADP+
zu katalysieren vermag, aufweist.

44. Verfahren gemäß Anspruch 43,
**dadurch gekennzeichnet,**
**dass** das Enzym E₁₄ ausgewählt ist aus Propan-2-ol:NADP+ Oxidoreduktase aus der Enzymklasse EC:1.1.1.80.

45. Verfahren gemäß Anspruch 44,
**dadurch gekennzeichnet,**
**dass** das Enzym E₁₄ ausgewählt ist aus P14941.1, P35630.1, P75214.1 und P25984.1, sowie Proteine mit einer Polypeptidsequenz, bei der bis zu 60 % der Aminosäurereste gegenüber den vorgenannten Bezugssequenzen durch Deletion, Insertion, Substitution oder eine Kombination davon verändert sind.

46. Verfahren gemäß mindestens einem der Ansprüche 1-10,
**dadurch gekennzeichnet,**
**dass** die organische Substanz 2-Hydroxyisobuttersäure ist und das Knallgasbakterium eine verglichen zu seinem Wildtyp gesteigerten Aktivität eines Enzyms E₁₅, welches die Umsetzung
von 3-Hydroxybutyryl-Coenzym A zu 2-Hydroxyisobutyryl-Coenzym A zu katalysieren vermag, aufweist.

47. Verfahren gemäß Anspruch 46,
**dadurch gekennzeichnet,**
**dass** das Enzym E₁₅ ausgewählt ist aus Hydroxyl-Isobutyryl-CoA Mutasen, Isobutyryl-CoA Mutasen aus der Enzymklasse EC 5.4.99.13 und Methylmalonyl-CoA Mutasen aus der Enzymklasse EC 5.4.99.2.

48. Verfahren gemäß Anspruch 47,
**dadurch gekennzeichnet,**
**dass** das Enzym E₁₅ ausgewählt ist aus solchen, die sich aus den Mikroorganismen *Aquincola tertiaricarbonis* L108, DSM18028, DSM18512, *Methylibium petroleiphilum* PM1, *Methylibium* sp. R8, *Xanthobacter autotrophicus* Py2, *Rhodobacter sphaeroides* (ATCC 17029), Nocardioides sp. JS614, *Marinobacter algicola* DG893, *Sinorhizobium medicae* WSM419, *Roseovarius* sp. 217, *Pyrococcus furiosus* DSM 3638 isolieren lassen.

49. Verfahren gemäß Anspruch 46,
**dadurch gekennzeichnet,**
**dass** das Enzym E₁₅ ein heterodimeres Enzyme darstellt, welches Sequenzen umfasst, ausgewählt aus Seq.-ID-Nr. 78 und Seq.-ID-Nr. 80 sowie Proteine mit einer Polypeptidsequenz, bei der bis zu 60 % der Aminosäurereste gegenüber den vorgenannten Bezugssequenzen durch Deletion, Insertion, Substitution oder eine Kombination davon verändert sind.

50. Verfahren gemäß mindestens einem der Ansprüche 46-49,
**dadurch gekennzeichnet,**
**dass** das Knallgasbakterium eine verglichen zu seinem Wildtyp gesteigerten Menge eines MeaB Proteins, insbesondere eines mit Seq.-ID-Nr. 82, aufweist.
